# EUROPEAN PATENT SPECIFICATION

(11) **EP 4 313 294 B1**
(45) Date of publication and mention of the grant of the patent: **25.02.2026**
(21) Application number: 22733954.6
(22) Date of filing: 15.06.2022
(51) Int. Cl.: A61P 3/00, A61P 9/00, A61P 29/00, A61P 35/00, A61P 37/02, C07D 471/04, C07D 519/00, A61K 31/437, A61K 31/5377, A61K 31/496

(54) **3-SUBSTITUTED 1H-PYRROLO[2,3-B]PYRIDINE AS GRK5 MODULATORS**
3-SUBSTITUIERTE 1H-PYRROLO[2,3-B]PYRIDINE ALS GRK5-MODULATOREN
1H-PYRROLO[2,3-B]PYRIDINE 3-SUBSTITUÉE COMME MODULATEURS DE GRK5

(30) Priority: 15.06.2021 EP 21179666
(43) Date of publication of application: 07.02.2024
(73) Proprietor: Lead Discovery Center GmbH, 44227 Dortmund (DE)
(72) Inventor: ZHANG, Jianmin, Toronto, Ontario M2J 2Z5 (CA); ENGEL, Julian, 44269 Dortmund (DE); BAUMANN, Matthias, 44267 Dortmund (DE); NUSSBAUMER, Peter, 44227 Dortmund (DE); KLEBL, Bert, 44229 Dortmund (DE); SCHULTZ-FADEMRECHT, Carsten, 44309 Dortmund (DE)
(74) Representative: Arth, Hans-Lothar
(86) International application number: PCT/EP2022/066372
(87) International publication number: WO 2022/263548

(56) References cited:
- EP-A1- 2 070 928
- US-A1- 2015 079 108

## Description

The present invention relates to certain 3-substituted 1H-pyrrolo[2,3-b]pyridine compounds of the formula (I) and pharmaceutically acceptable salts thereof. These compounds are useful in the treatment or prevention of a disease or medical condition mediated through GRK5 selected from heart disease, inflammatory and immunological disease, metabolic disease and cancer.

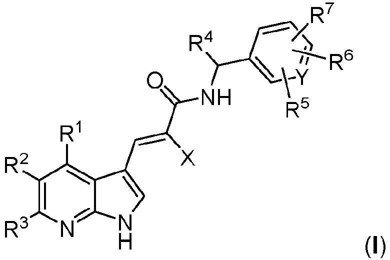

### Background of the invention

G protein-coupled receptor kinases (GRKs) constitute a family of seven mammalian serine/threonine protein kinases that based on sequence homology can be divided into the three subfamilies "GRK1" (composed of GRK1 and GRK7), "GRK2" (composed of GRK2 and GRK3) and "GRK4" (composed of GRK4, GRK5, and GRK6). Whereas GRK1, GRK4, and GRK7 are expressed in limited tissues such as retina (GRK1 and GRK7) and testis (GRK4), GRK2, GRK3, GRK5, and GRK6 are expressed ubiquitously throughout the body.

GRKs were originally identified by their ability to phosphorylate and desensitize agonist-bound G protein-coupled receptors (GPCRs). GPCRs represent the largest superfamily of transmembrane receptor proteins and facilitate cell sensitivity to a diverse array of external stimuli. As a consequence, GPCR dysregulation is a critical contributor to many diseases such as neurodegeneration, cancer and autoimmune diseases (Hauser et al., Nat Rev Drug Discov. 2017 Dec;16(12):829-842). Upon ligand binding, GPCRs become activated resulting in downstream intracellular signaling via heterotrimeric G proteins. The phosphorylation of agonist-bound GPCR by GRKs leads to the translocation and binding of β-arrestins to the receptors, inhibiting further G protein activation by blocking receptor-G protein coupling and promoting the internalization of agonist-bound GPCR. Thus, GRK-catalyzed phosphorylation and binding of β-arrestin to the receptors are believed to be the common mechanism to turn off activated GPCRs as an important mechanism for maintaining homeostasis (Wang et al., Trends Pharmacol Sci. 2018 Apr;39(4):367-386).

In addition to their canonical role in GPCR desensitization, recent studies have demonstrated a much broader, non-canonical function for this kinase family including phosphorylation of cytosolic substrates involved in cell signaling pathways stimulated by both GPCRs and non-GPCRs. Moreover, GRKs modulate signaling via phosphorylationindependent functions (Lieu and Koch, Expert Opin Ther Targets. 2019 Mar;23(3):201-214.). Since GRKs possess multiple biologic functions and have been shown to affect critical physiological and pathophysiological processes, they are considered as drug targets in diseases such as as cardiac hypertrophy and heart failure, obesity and diabetes, atherosclerosis, inflammation and fibrosis, neurodegeneration, opioid tolerance and addiction, and cancer (Hullmann et al., Pharmacol Res. 2016 Aug;110:52-64; Gold et al., Circ Res. 2012 Sep 28:111(8):1048-53; Li et al., Diabetes. 2013 Jan;62(1):291-8.; Lutz et al., Sci Rep. 2018 May 17;8(1):7745.; Xia et al., PLoS One. 2014 Mar 3;9(3):e90597; Packiriswamy and Parameswaran, Genes Immun. 2015 Sep;16(6):367-77; Murga et al., Front Pharmacol. 2019 Feb 19;10:112; Pham et al., Oncotarget. 2020 Apr 21;11(16):1448-1461; Jiang et al, Cell Death Dis. 2018 Feb 20;9(3):295; Sommer et al., Sci Rep. 2019 Oct 29;9(1):15548; Hendrickx et al., Front Pharmacol. 2018 Dec 17;9:1484; Marzano et al., Int J Mol Sci. 2021 Feb 15;22(4):1920; Patial et al., J Cell Physiol. 2011 May;226(5):1323-33; De Lucia et al., Cardiovasc Res. 2021 Feb 9:cvab044; Eguchi et al., Proc Natl Acad Sci U S A. 2021 Feb 2;118(5):e2012854118, Kliewer et al., Nat Commun. 2019 Jan 21;10(1):367).

Among the cardiovascular diseases that involve the heart is a pathologic condition termed concentric ventricular hypertrophy (VH). VH can affect both the right ventricular (RVH) as well as the left ventricular (LVH) with the latter being the more common form. In contrast to eccentric VH which is regarded as a physiologic, adaptive process resulting from intense aerobic exercise training or in pregnancy in response to increased blood volume, concentric VH is considered as a maladaptive cardiac remodeling of the heart that is commonly observed in conditions of increased hemodynamic or metabolic stress from various stressors including hypertension, congenital heart defects, valvular defects (aortic coarction or stenosis) and primary defects of the myocardium which directly cause VH (hypertrophic cardiomyopathy). The underlying commonality in these disease states is an increase in pressures that the ventricles try to compensate by undergoing thickening. VH as a result of increased ventricular pressure or volume load is problematic and actually poses a serious health risk which can even lead to heart failure (Nakamura and Sadoshima, Nat Rev Cardiol. 2018 Jul;15(7):387-407; Shimizu and Minamino, J Mol Cell Cardiol. 2016 Aug;97:245-62).

In the treatment of LVH, sarcolemmal receptors of the renin-angiotensin-aldosterone (RAAS) system remain the most important targets for the therapeutic treatment of cardiac hypertrophy. However, several trials have shown that a combination of RAAS inhibitors, despite synergistic, may increase the risk of unwanted side effects in some patients (Weir et al., Eur J Heart Fail. 2008 Feb;10(2):157-63). A variety of intracellular targets have been identified in maladaptive signaling cascades, but the lack of targetand organ-specific small molecules delays clinical translation (Bisping et al., J Cardiovasc Pharmacol. 2014 Oct;64(4):293-305).

GRK2 and GRK5 are the predominant GRKs in the heart and have been intensively studied for their potential role in cardiac hypertrophy (Gold et al., Circ Res. 2012 Sep 28;111(8):1048-53). It has been observed that GRK5 expression is increased after cardiac injury resulting in worsen disease prognosis and mortality which is clinically associated with maladaptive cardiac remodelling (Dzimiri et al., Eur J Pharmacol. 2004 Apr 12;489(3):167-77). In contrast, depletion GRK5 in knock-out (KO) mice delays maladaptive cardiac hypertrophy after ventricular pressure-overload (TAC) surgery (Traynham et al., Circ Res. 2015 Dec 4;117(12):1001-12). The development of TAC-induced hypertrophy seems to be dependent on the presence of GRK5 in the nucleus as deletion of its nuclear location sequence (NLS) significantly ameliorates maladaptive cardiac remodeling after TAC (Johnson et al., Mol Cell Biol. 2004 Dec;24(23):10169-79). This points to a non-canonical function of GRK5 in cardiac hypertrophy which is believed to be triggered by the nuclear translocation of GRK5 upon hypertrophic stress. Subsequent phosphorylation of HDAC5 by GRK5 in the nucleus leads to derepression of MEF2 resulting in activation of MEF2-dependent hypertrophic genes. In addition, nuclear GRK5 interacts and enhances the activity of pro-hypertrophic transcription factors NFkB and NFAT in a kinase-independent manner.

Little has been reported regarding GRK5 inhibitors in clinical development. The antiinflammatory, antiallergic immunomodulator Amlexanox is the only marketed drug that is known to moderately inhibit GRK5 (with an IC50 value of ca. 10 µM) which could contribute to its mechanism of action which, on the other hand, is not well-determined (Homan et al., Molecules. 2014 Oct 22;19(10):16937-49, Lee et al., Biomol Ther (Seoul). 2020 Sep 1;28(5):482-489).

Compared to GRK5, the role of GRK2 in cardiac hypertrophy is less clear. Although in vitro overexpression of GRK2 is enough to elict hypertrophic response via NFkB and NFAT activation, cardiac-specific transgenic GRK2 mice stressed with TAC undergo maladaptive hypertrophy not significantly different from non-transgenic controls but have increased mortality and decreased cardiac function. Thus, as GRK2 obviously has a role in other maladaptive pathways, dual inhibition of GRK2 and GRK5 could be a therapeutic strategy for pathological hypertrophy and overall cardiovascular health (Huang et al., Antioxid Redox Signal. 2014 Nov 10;21(14):2032-43; Pfleger et al., Nat Rev Cardiol. 2019 Oct;16(10):612-622).

EP207928A1 discloses 7-azaindol-3-yl-acrylamide derivatives as AKTs and CDC7 inhibitors and their use in the treatment of diseases mediated by AKT and CDC7, in particular cancer.

US2015/0079108A discloses pyrrolo[3,2-c]pyridine derivatives as GRK5 inhibitors and their use in the treatment of diabetes mellitus type 2.

It is object to the present invention to provide novel compounds as mono-specific GRK5 or dual-specific GRK2/5 inhibitors and/or pharmaceutically acceptable salts thereof, which can be used as pharmaceutically active agents, and use thereof especially for prophylaxis and/or treatment of selected from heart disease, inflammatory and immunological disease, metabolic disease and cancer as well as compositions comprising at least one of those compounds and/or pharmaceutically acceptable salts thereof as pharmaceutically active ingredients. The objective of the present invention is solved by the teaching of the independent claims. Further advantageous features, aspects and details of the invention are evident from the dependent claims, the description, and the examples of the present application.

The following compound is known as such from Chemical Abstracts without the disclosure of the chemical synthesis or any use thereof. This compound is excluded from the scope of the present application: *Chemical Abstracts,* April 28. 2004, "(E)-N-(1,3-benzodioxol-5-yl-methyl)-2-cyano-3-(1H-pyrrolo[2,3-b]pyridin-3-yl)acrylamide "

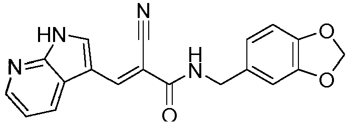

### Description of the invention

The present invention relates to a compound of the general formula (I) 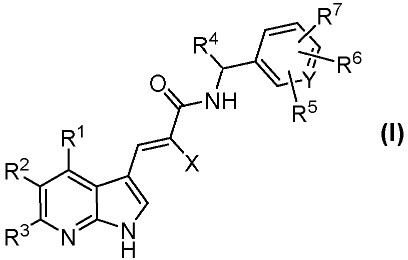 wherein
**X** represents -H or -CN,
**Y** represents CH or N,
**R¹** - R² represent independently of each other -H, -Ph, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, cyclo-C₃H₅, cyclo-C₄H₇, cyclo-C₅H₉, cyclo-C₆H₁₁, -F, -Cl, -Br, -CN, -OH, -NH-Ph, -**R⁹**, -O-**R¹⁰**, 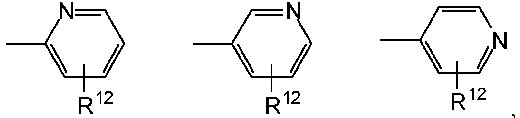 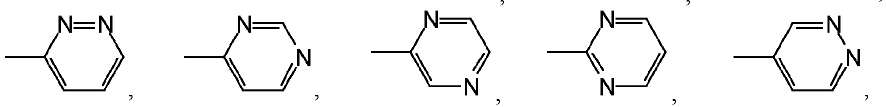 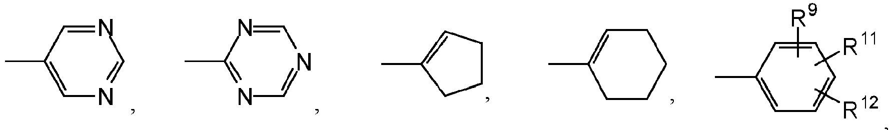 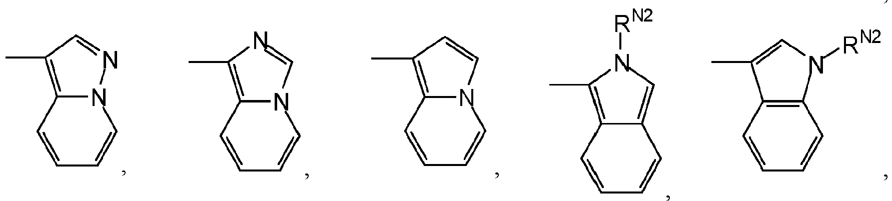 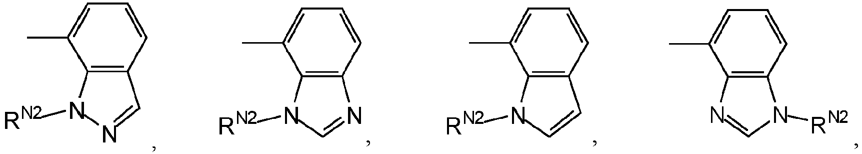 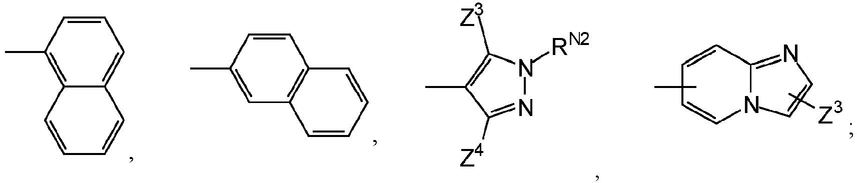
**R³** represents -H, -F, -Cl, or -Br;
**R⁴** represents -H, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH₂-OH, -C₂H₄-OH, -C₃H₆-OH, -CN, -CH₂-NH₂, -CH₂-NHCH₃, -CH₂-NHC₂H₅, -CH₂-NHC₃H₇, -CH₂-N(CH₃)₂, -CH₂-N(C₂H₅)₂, -CH₂-N(C₃H₇)₂, -C₂H₄-NH₂, -C₂H₄-NHCH₃, -C₂H₄-NHC₂H₅, -C₂H₄-NHC₃H₇, -C₂H₄-N(CH₃)₂, -C₂H₄-N(C₂H₅)₂, -C₂H₄-N(C₃H₇)₂, -C₃H₆-NH₂, -C₃H₆-NHCH₃, -C₃H₆₇-NHC₂H₅, -C₃H₆-NHC₃H₇, -C₃H₆-N(CH₃)₂, -C₃H₆-N(C₂H₅)₂, -C₃H₆-N(C₃H₇)₂;
**R⁵** - **R⁷** represent independently of each other **-R⁸,** -CH₂-**R⁸**, -SO₂-**R⁸**, 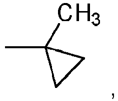 cyclo-C₃H₅, cyclo-C₄H₇, cyclo-C₅H₉, cyclo-C₆H₁₁, cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃**,** -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃**,** -COOH, -COOCH₃, -COOC₂H₅**,** -COOC₃H₇**,** -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇**,** -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₃, -SO₂NHCH(CH₃)₂, -SO₂NHC(CH₃)₃, -SO₂N(CH₃)₂, -SO₂N(C₂H₃)₂, -SO₂N(C₃H₇)₂, -SO₂N(cyclo-C₃H₅)₂, -SO₂N[CH(CH₃)₂]₂, -SO₂N[C(CH₃)₃]₂, -CH₂-SO₂NH₂, -CH₂-SO₂NHCH₃, -CH₂-SO₂NHC₂H₅, -CH₂-SO₂NHC₃H₇, -CH₂-SO₂NH-cyclo-C₃H₅, -CH₂-SO₂NHCH(CH₃)₂, -CH₂-SO₂NHC(CH₃)₃, -CH₂-SO₂N(CH₃)₂, -CH₂-SO₂N(C₂H₃)₂, -CH₂-SO₂N(C₃H₇)₂, -CH₂-SO₂N(cyclo-C₃H₅)₂, -CH₂-SO₂N[CH(CH₃)₂]₂, -CH₂-SO₂N[C(CH₃)₃]₂, -O-S(=O)CH₃, -O-S(=O)C₂H₅, -O-S(=O)C₃H₇, -O-S(=O)-cyclo-C₃H₅**,** -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅**,** -O-SO₂-C₃H₇**,** -O-SO₂-cyclo-C₃H₅, -O-SO₂-CH(CH₃)₂**,** -O-SO₂-C(CH₃)₃, -OCH₂F, -OCHF₂ -OCF₃, -CH₂-OCF₃, -C₂H₄-OCF₃, -C₃H₆-OCF₃**,** -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂**,** -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇**,** -CH₂-CH(CH₃)-C₂H₅**,** -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, or -C≡C-C≡C-C₂H₅, or
**R⁵** and **R⁶,** or **R⁶** and **R⁷** form together 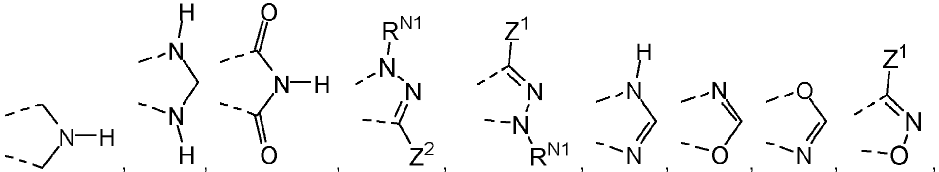 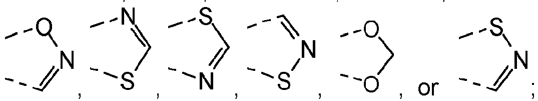
**R⁸** represents 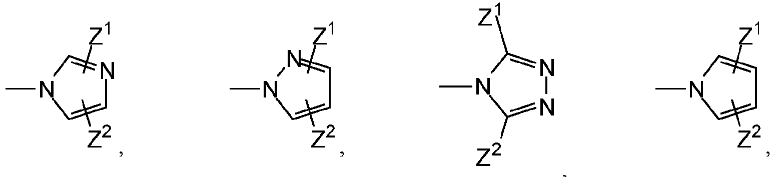 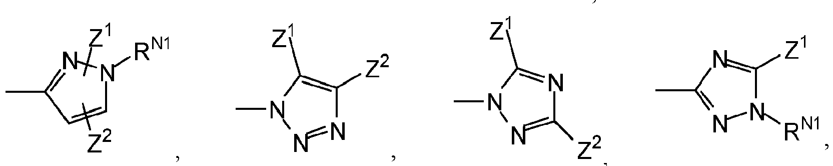 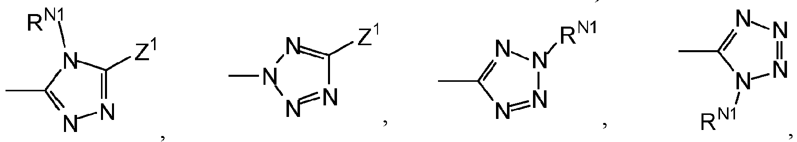 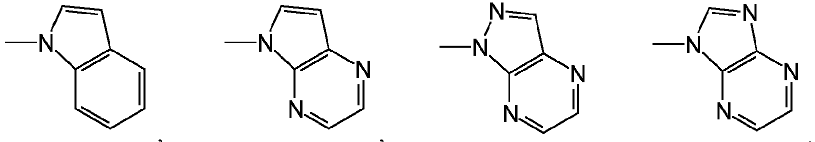 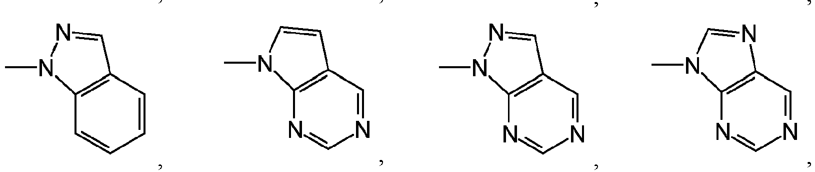 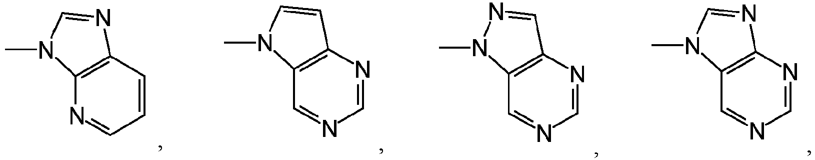 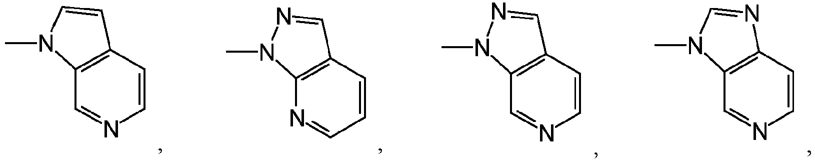 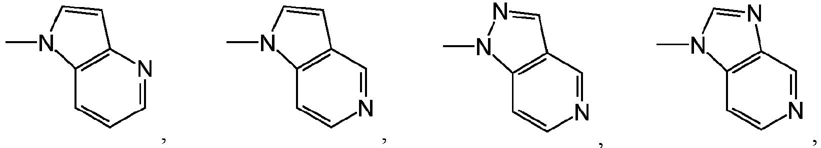 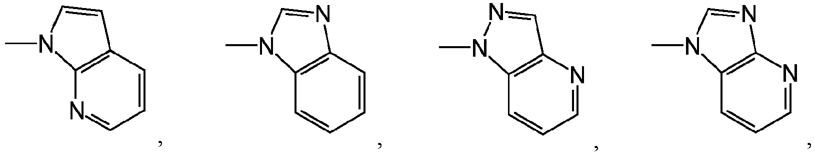 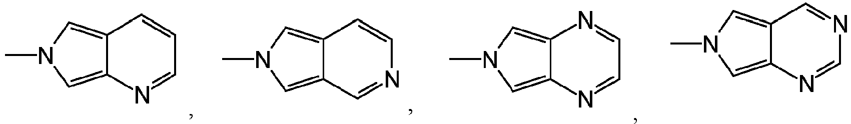 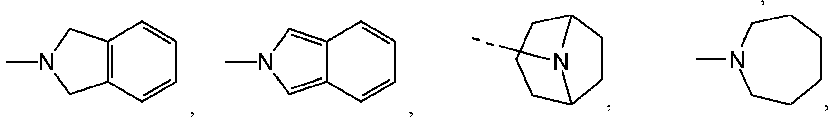 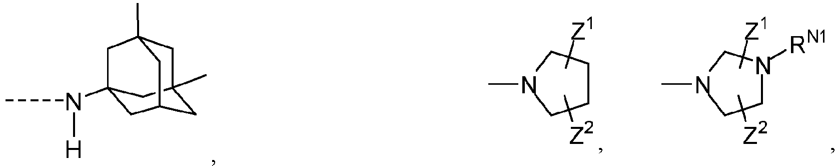 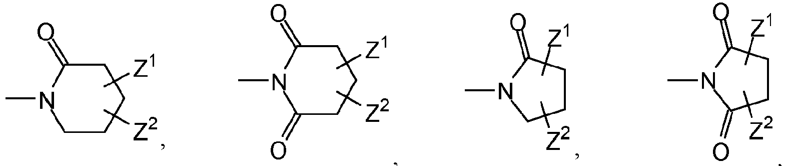 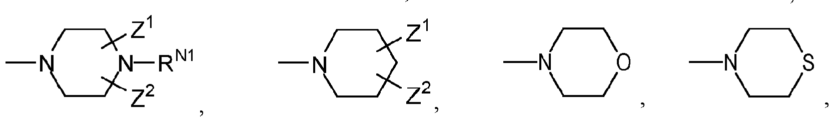 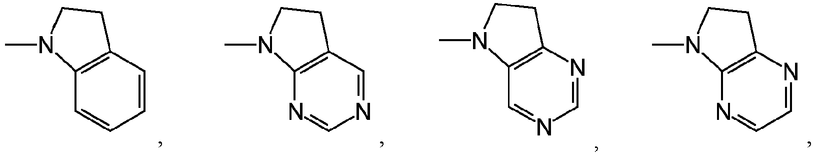 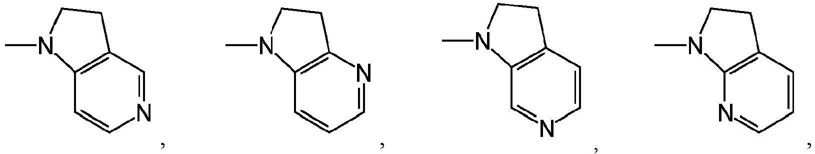 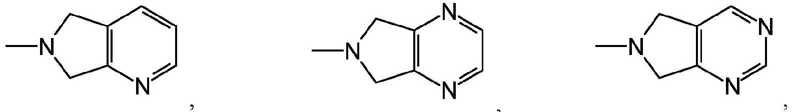 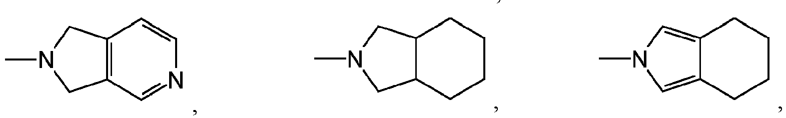 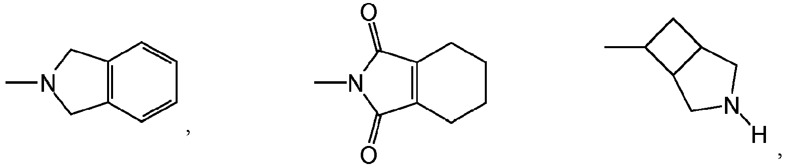 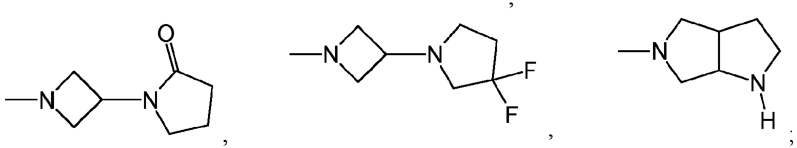
**R⁹** represents -H, 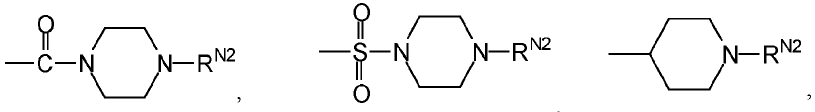 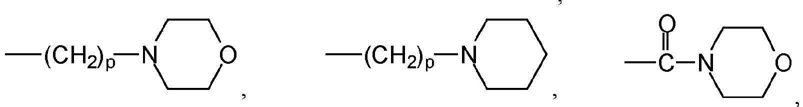 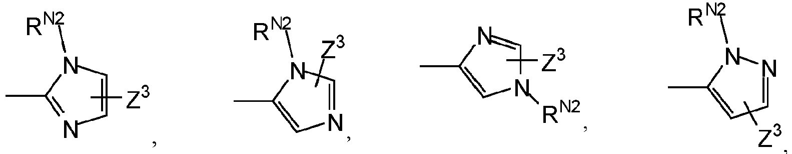 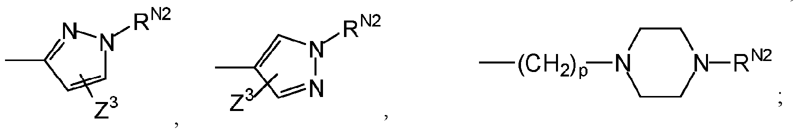
**R¹⁰** represents -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -(CH₂)ₙ-NH₂, -(CH₂)ₙ-NHCH₃, -(CH₂)ₙ-NHC₂H₅, -(CH₂)ₙ-NHC₃H₇, -(CH₂)ₙ-N(CH₃)₂, -(CH₂)ₙ-N(C₂H₅)₂, -(CH₂)ₙ-N(C₃H₇)₂, 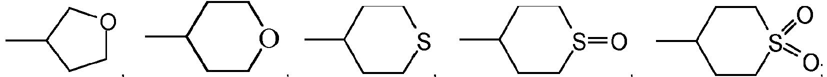 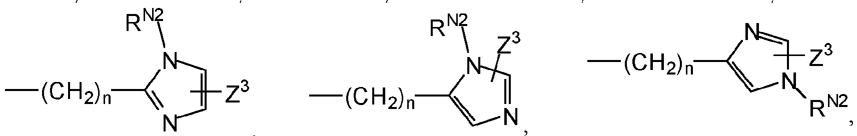 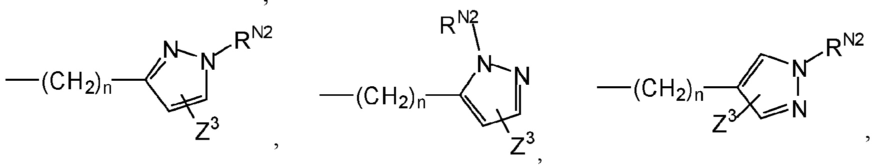
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₃, -(CH₂)ₘ-SO₂C₃H₇, -(CH₂)ₘ₋SO₂-cyclo-C₃H₅, -(CH₂)ₘ-SO₂CH(CH₃)₃, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ NHCO-cyclo-C₃H₅, -(CH₂)ₘ-NHCOC₃Hz, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₃)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₃, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N1}** and **R^{N2}** represent independently of each other -H, -CH₃, -C₂H₅, -C₃H₇, -CH(CH₃)₂, cyclo-C₃H₅, cyclo-C₄H₇, cyclo-C₅H₉, cyclo-C₆H₁₁, -C₄H₉, -C(CH₃)₃, -CH₂-CH(CH₃)₂, -CH(CH₃)-C₂H₅, -C₅H₁₁, -Ph, -CH₂-Ph, -CH=CH₂, -CH₂-CH=CH₂, -CH=CH-CH₃, -C≡CH, -CH₂-C≡CH, -C≡C-CH₃, -COCH₃, -COC₂H₅, -COC₃H₇, -CO-cyclo-C₃H₃, -COCH(CH₃)₂, -COOCH₃, -COOC₂H₅**,** -COOC₃H**₇,** -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 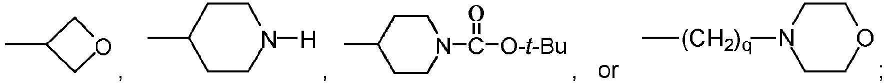
**Z¹** - **Z⁴** represent independently of each other -H, -CH₃, -C₂H₅, -C₃H₇, -CH(CH₃)₂, -Ph, -COCH₃, -COC₂H₅, -COOH, -COOCH₃, -COOC₂H₅**,** -NH₂, -NHCH₃, -NHC₂H₅, -N(CH₃)₂, -N(C₂H₅)₂, -CH₂-NH₂, -SO₃H, -F, -CI, -Br, -I, -CN, -CF₃, -OCF₃, -OH, -OCH₃, or -OC₂H₅;
m is an integer selected from 0, 1, 2, 3, or 4;
n is an integer selected from 1, 2, 3, or 4;
p is an integer selected from 0, 1, 2, 3, or 4;
q is an integer selected from 1, 2, 3, or 4;
with the proviso that when X is -H at least one of R¹ - R³ is not -H; and when **R⁴** is -H, at least one of **R¹** - **R³** and **R⁵** - **R⁷** is not -H; and the following compound is excluded from the scope of the present application: 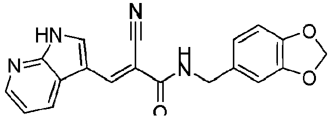 (E)-N-(1,3-benzodioxol-5-yl-methyl)-2-cyano-3-(1H-pyrrolo[2,3-b]pyridin-3-yl)acrylamide, or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof .

Preferred are compounds wherein R⁴ is different from hydrogen (-H). In case R⁴ is hydrogen one or at least one of the substituents **R¹** - **R**³ and **R⁵** - **R⁷** is not hydrogen and preferably two or three or more than three substituents **R¹** - **R³** and **R⁵** - **R⁷** are not hydrogen. Even more preferred are the compounds wherein one or at least one of the substituents **R⁴** - **R⁷** is not hydrogen. Still more preferred are the compounds wherein one or at least one of the substituents **R¹** - **R⁴** is not hydrogen. Most preferred are the compounds wherein one or at least one of the substituents **R¹** - **R³** is not hydrogen and wherein one or at least one of the substituents **R⁵** - **R⁷** is not hydrogen.

Preferably, the compounds of general formula (I) as defined herein are under the proviso that when X is -H at least one of **R¹** - **R³** is not -H and under the second proviso that **R⁵, R⁶** and R⁷ are not -H at the same time or preferably under the further proviso that at least one of **R¹** - **R³** is not -H, when **R⁵** and **R⁶** or **R⁶** and **R⁷** form together 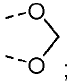 more preferably under the further proviso that when **X** is -CN or when **R⁴** is -H or -C₂H₅ or -CH₂OH or -C₂H₄OH, at least one of **R⁵, R⁶** and **R⁷** is different from -H or still more preferably under the second proviso that in case **X** is -CN and **R⁴** is -H or -C₂H₅ or -CH₂OH or -C₂H₄OH, at least one of **R⁵, R⁶** and **R⁷** is different from -H.

Preferably, a compound of the general formula **(Ia)** 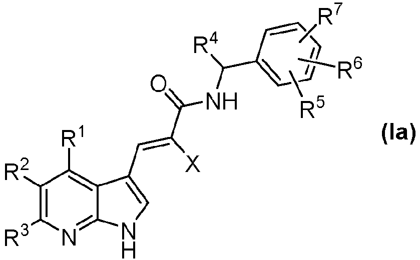 wherein
**X** represents -H or -CN,
**R¹** - **R²** represent independently of each other -H, -Ph, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, cyclo-C₃H₅, cyclo-C₄H₇, cyclo-C₅H₉, cyclo-C₆H₁₁, -F, -CI, -Br, -CN, -OH, -NH-Ph, **-R⁹,** -O-**R¹⁰**, 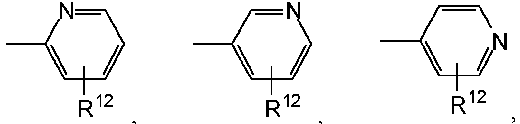 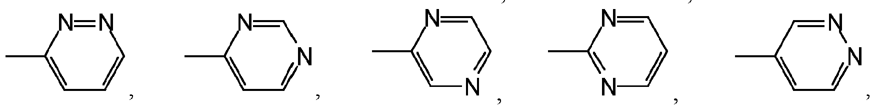 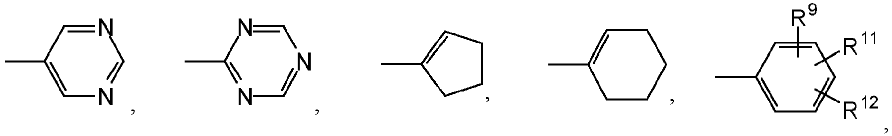 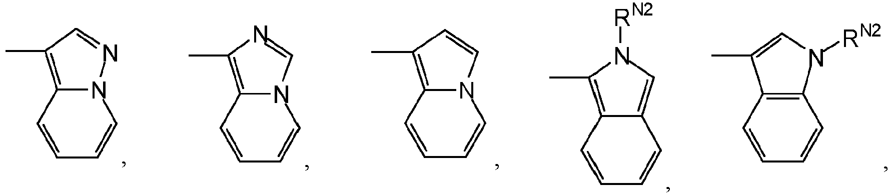 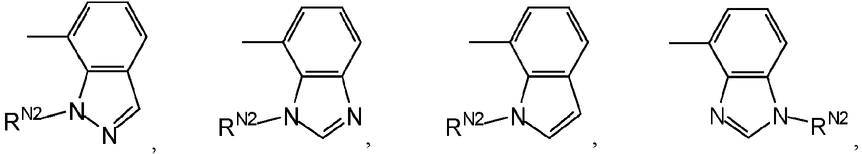 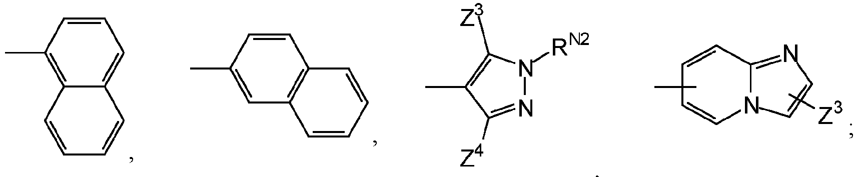
**R³** represents -H, -F, -CI, or -Br;
**R⁴** represents -H, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH₂-OH, -C₂H₄-OH, -C₃H₆-OH, -CN, -CH₂-NH₂, -CH₂-NHCH₃, -CH₂-NHC₂H₅, -CH₂-NHC₃H₇, -CH₂-N(CH₃)₂, -CH₂-N(C₂H₅)₂, -CH₂-N(C₃H₇)₂, -C₂H₄-NH₂, -C₂H₄-NHCH₃, -C₂H₄-NHC₂H₅, -C₂H₄-NHC₃H₇, -C₂H₄-N(CH₃)₂, -C₂H₄-N(C₂H₅)₂, -C₂H₄-N(C₃H₇)₂, -C₃H₆-NHz, -C₃H₈-NHCH₃, -C₃H₈-NHC₂H₅, -C₃H₆-NHC₃H₇, -C₃H₆-N(CH₃)₂, -C₃H₆-N(C₂H₅)₂, -C₃H₃-N(C₃H₇)₂;
**R⁵** - **R⁷** represent independently of each other **-R⁸,** -CH₂-**R⁸**, -SO₂-**R⁸**, 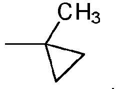 cyclo-C₃H₅, cyclo-C₄H₇, cyclo-C₅H₉, cyclo-C₆H₁₁, cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₈-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃**,** -COOH, -COOCH₃, -COOC₂H₅**,** -COOC₃H**₇,** -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₃, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅**,** -SO₃C₃H₇, -SO₃-cyclo-C₃H₅**,** -SO₃CH(CH₃)₂, -SO₃C(CH₃)₃**,** -SO₂NH₂, -SO₂NHCH₃, -SO₂NHC₂H₃, -SO₂NHC₃H₇, -SO₂NH-cyclo-C₃H₃, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -CH₂-SO₂NH₂, -CH₂-SO₂NHCH₃, -CH₂-SO₂NHC₂H₅, -CH₂-SO₂NHC₃H₇, CH₂-SO₂NH-cyclo-C₃H₃, -CH₂-SO₂NHCH(CH₃)₂, -CH₂-SO₂NHC(CH₃)₃, -CH₂-SO₂N(CH₃)₂, -CH₂-SO₂N(C₂H₃)₂, -CH₂-SO₂N(C₃H₇)₂, -CH₂-SO₂N(cyclo-C₃H₅)₂, -CH₂-SO₂N[CH(CH₃)₂]₂, -CH₂-SO₂N[C(CH₃)₃]₂, -O-S(=O)CH₃**,** -O-S(=O)C₂H₅**,** -O-S(=O)C₃H₇**,** -O-S(=O)-cyclo-C₃H₅**,** -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₃-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, or -C≡C-C≡C-C₂H₅, or
**R⁵** and **R⁶**, or **R⁶** and **R⁷** form together 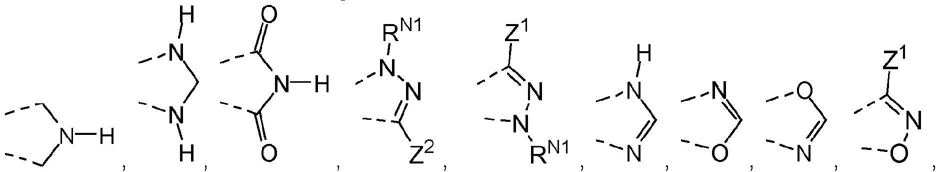 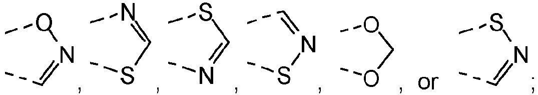
**R⁸** represents 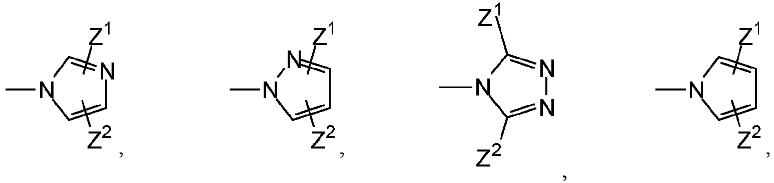 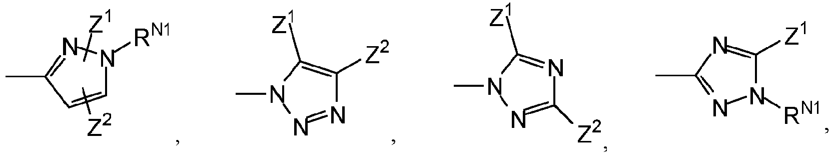 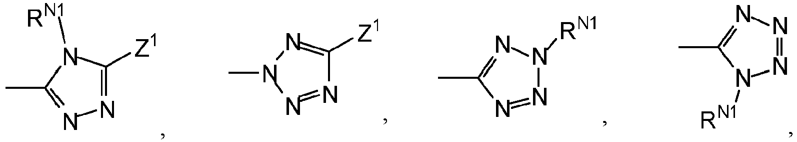 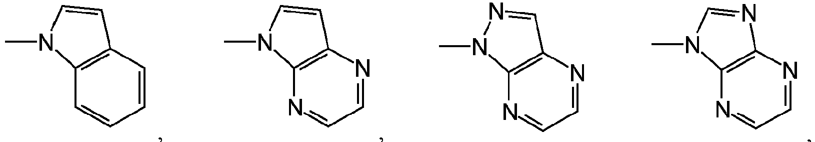 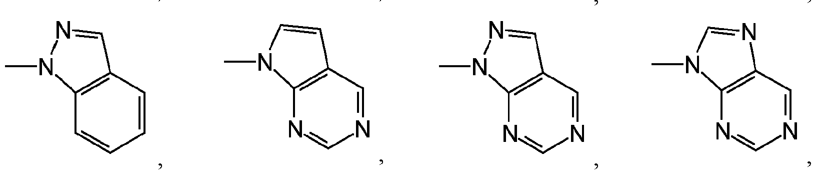 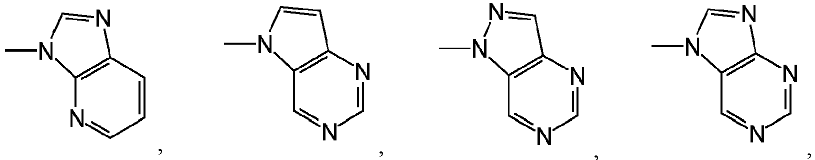 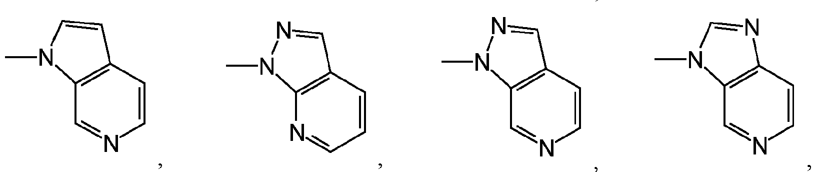 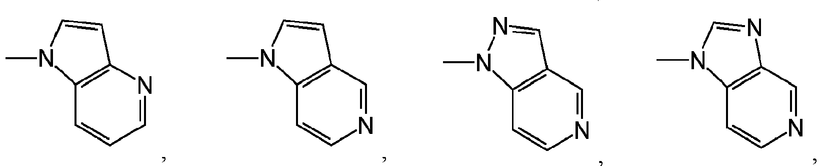 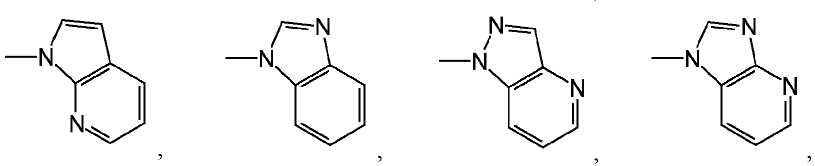 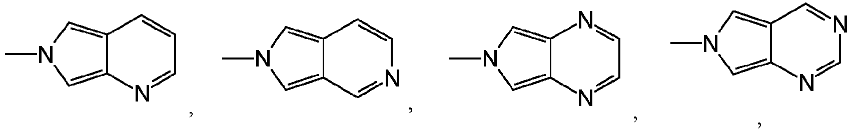 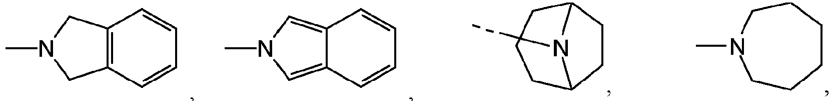 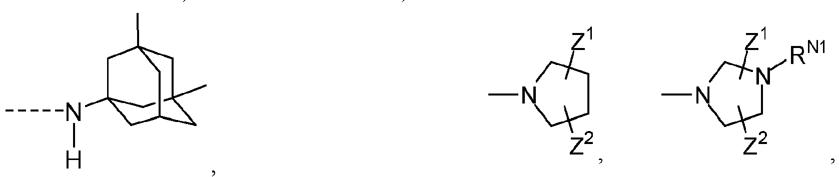 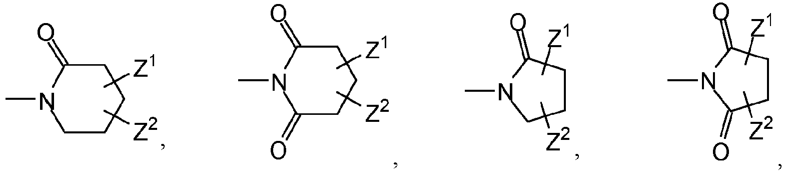 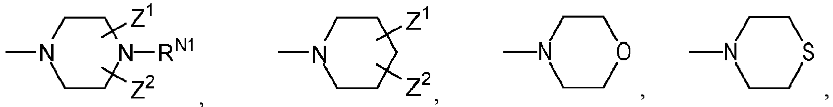 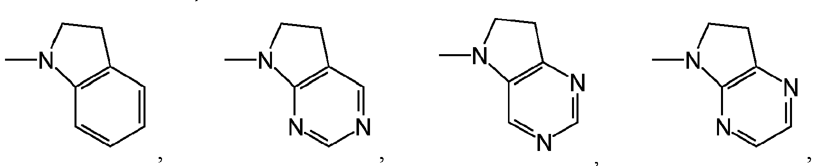 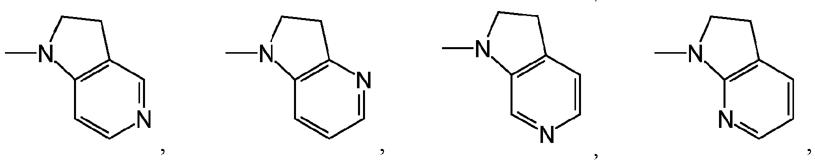 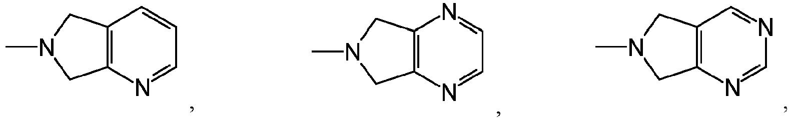 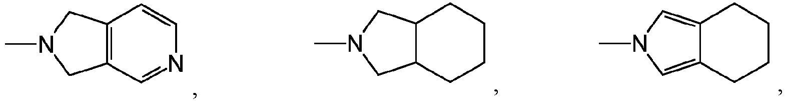 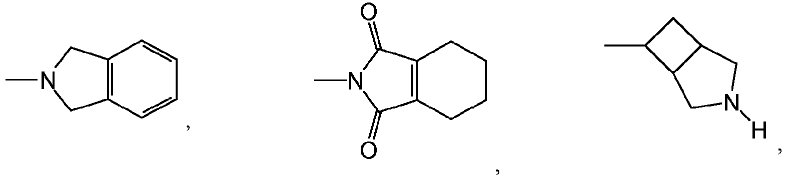 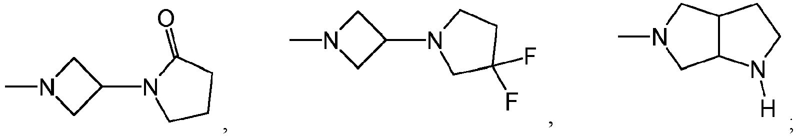
**R⁹** represents -H, 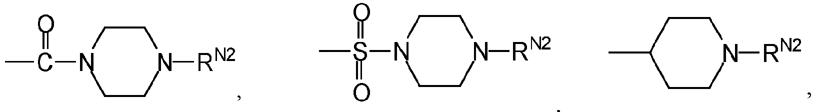 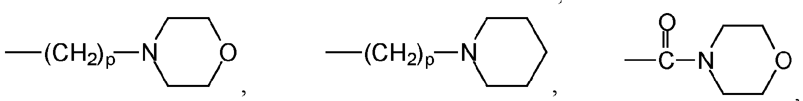 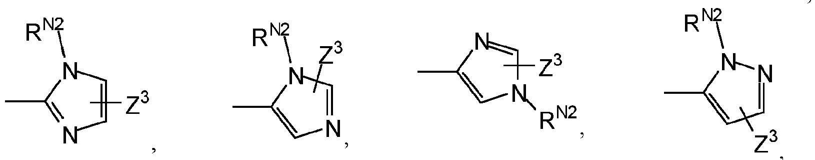 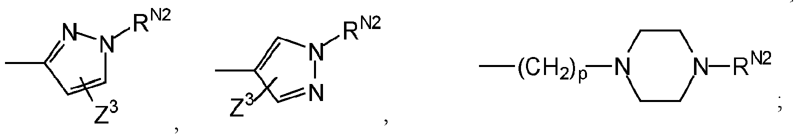
**R¹⁰** represents -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -(CH₂)ₙ-NH₂, -(CH₂)ₙ-NHCH₃, -(CH₂)ₙ-NHC₂H₅, -(CH₂)ₙ-NHC₃H₇, -(CH₂)ₙ-N(CH₃)₂, -(CH₂)ₙ-N(C₂H₅)₂, -(CH₂)ₙ-N(C₃H₇)₂, 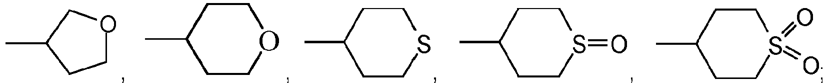 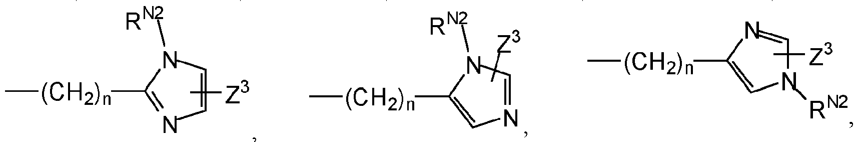 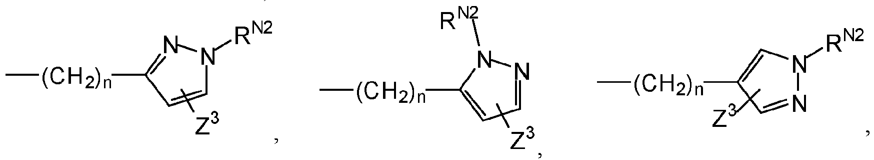
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N1}** and **R^{N2}** represent independently of each other -H, -CH₃, -C₂H₅, -C₃H₇, -CH(CH₃)₂, cyclo-C₃H₅, cyclo-C₄H₇, cyclo-C₅H₉, cyclo-C₆H₁₁, -C₄H₉, -C(CH₃)₃, -CH₂-CH(CH₃)₂, -CH(CH₃)-C₂H₅, -C₅H₁₁, -Ph, -CH₂-Ph, -CH=CH₂, -CH₂-CH=CH₂, -CH=CH-CH₃, -C≡CH, -CH₂-C≡CH, -C≡C-CH₃, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 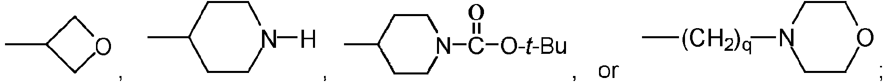
**Z¹** - **Z⁴** represent independently of each other -H, -CH₃, -C₂H₅, -C₃H₇, -CH(CH₃)₂, -Ph, -COCH₃, -COC₂H₅, -COOH, -COOCH₃, -COOC₂H₅, -NH₂, -NHCH₃, -NHC₂H₅, -N(CH₃)₂, -N(C₂H₅)₂, -CH₂-NH₂, -SO₃H, -F, -Cl, -Br, -I, -CN, -CF₃, -OCF₃, -OH, -OCH₃, or -OC₂H₅;
m is an integer selected from 0, 1, 2, 3, or 4;
n is an integer selected from 1, 2, 3, or 4;
p is an integer selected from 0, 1, 2, 3, or 4;
q is an integer selected from 1, 2, 3, or 4;
with the proviso that when X is -H at least one of R¹ - R³ is not -H; and when **R⁴** is -H, at least one of **R¹** - **R³** and **R⁵** - **R⁷** is not -H; and the following compound is excluded from the scope of the present application: 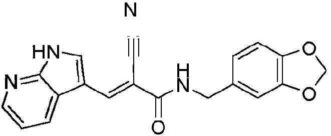
or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof .

Preferred are compounds wherein R⁴ is different from hydrogen (-H). In case R⁴ is hydrogen one or at least one of the substituents **R¹** - **R³** and **R⁵** - **R⁷** is not hydrogen and preferably two or three or more than three substituents **R¹** - **R³** and **R⁵** - **R⁷** are not hydrogen. Even more preferred are the compounds wherein one or at least one of the substituents **R⁴** - **R⁷** is not hydrogen. Still more preferred are the compounds wherein one or at least one of the substituents **R¹** - **R⁴** is not hydrogen. Most preferred are the compounds wherein one or at least one of the substituents **R¹** - **R³** is not hydrogen and wherein one or at least one of the substituents **R⁵** - **R⁷** is not hydrogen.

Preferably, the present invention relates to a compound of the general formula (**Ia**) 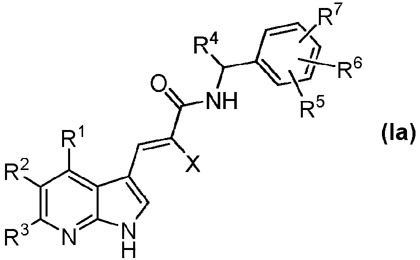 wherein
**X** represents -H or -CN,
**R¹** - **R²** represent independently of each other -H, -Ph, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, cyclo-C₃H₅, cyclo-C₄H₇, cyclo-C₅H₉, cyclo-C₆H₁₁, -F, -Cl, -Br, -CN, -OH, -NH-Ph, -**R⁹**, -O-**R¹⁰**, 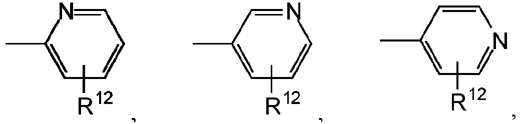 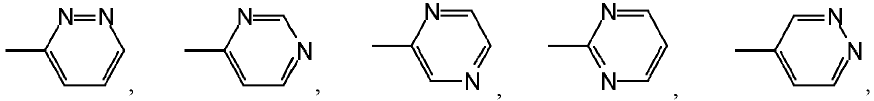 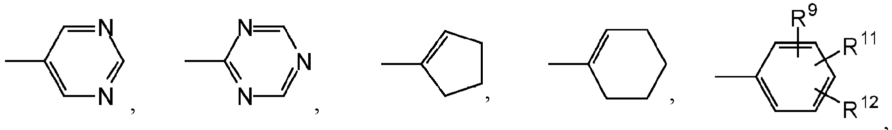 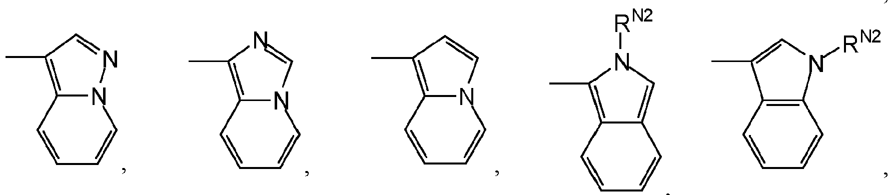 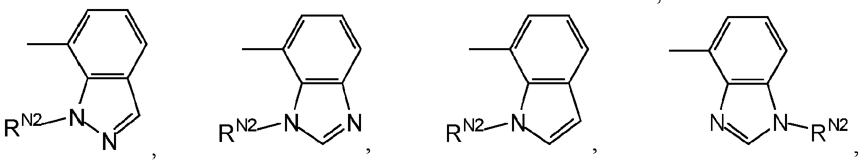 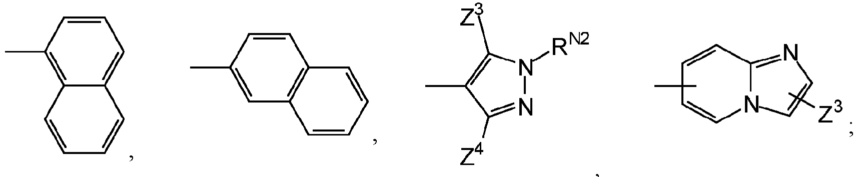
**R³** represents -H, -F, -CI, or -Br;
**R⁴** represents -H, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH₂-OH, -C₂H₄-OH, -C₃H₆-OH, -CN, -CH₂-NH₂, -CH₂-NHCH₃, -CH₂-NHC₂H₅, -CH₂-NHC₃H₇, -CH₂-N(CH₃)₂, -CH₂-N(C₂H₅)₂, -CH₂-N(C₃H₇)₂, -C₂H₄-NH₂, -C₂H₄-NHCH₃, -C₂H₄-NHC₂H₅, -C₂H₄-NHC₃H₇, -C₂H₄-N(CH₃)₂, -C₂H₄-N(C₂H₅)₂, -C₂H₄-N(C₃H₇)₂, -C₃H₆-NH₂, -C₃H₆-NHCH₃, -C₃H₆-NHC₂H₅, -C₃H₆-NHC₃H₇, -C₃H₆-N(CH₃)₂, -C₃H₆-N(C₂H₅)₂, -C₃H₆-N(C₃H₇)₂;
**R⁵** - **R⁷** represent independently of each other -**R⁸**, -CH₂-**R⁸**, -SO₂-**R⁸**, 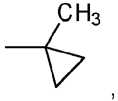 cyclo-C₃H₅, cyclo-C₄H₇, cyclo-C₅H₉, cyclo-C₆H₁₁, cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -CH₂-SO₂NH₂, -CH₂-SO₂NHCH₃, -CH₂-SO₂NHC₂H₅, -CH₂-SO₂NHC₃H₇, -CH₂-SO₂NH-cyclo-C₃H₅, -CH₂-SO₂NHCH(CH₃)₂, -CH₂-SO₂NHC(CH₃)₃, -CH₂-SO₂N(CH₃)₂, -CH₂-SO₂N(C₂H₅)₂, -CH₂-SO₂N(C₃H₇)₂, -CH₂-SO₂N(cyclo-C₃H₅)₂, -CH₂-SO₂N[CH(CH₃)₂]₂, -CH₂-SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, or -C≡C-C≡C-C₂H₅, or
**R⁵** and **R⁶**, or **R⁶** and **R⁷** form together 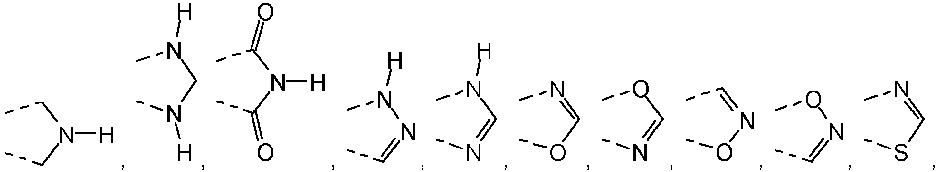 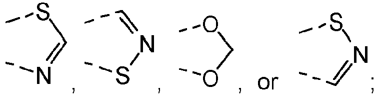
**R⁸** represents 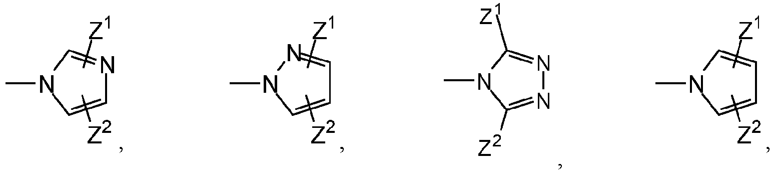 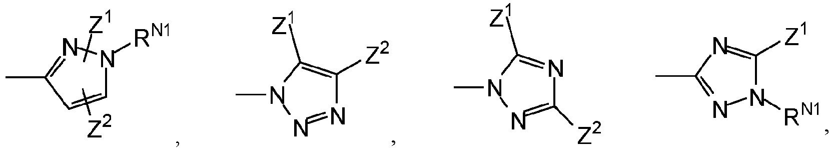 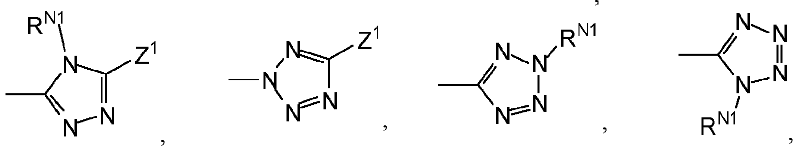 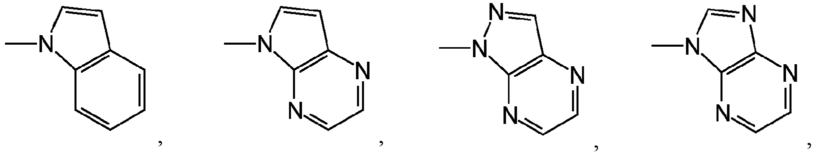 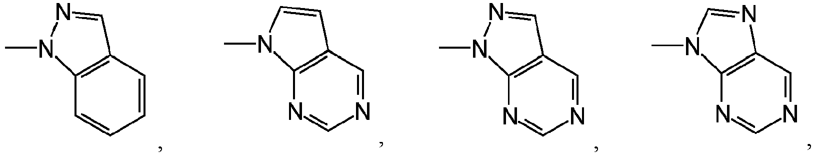 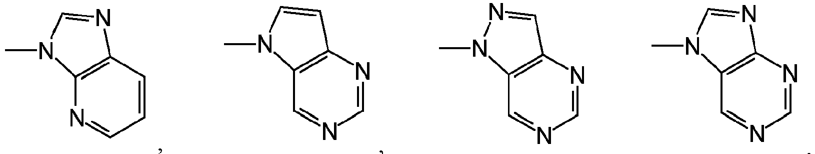 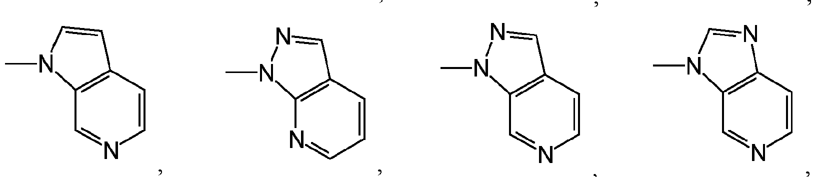 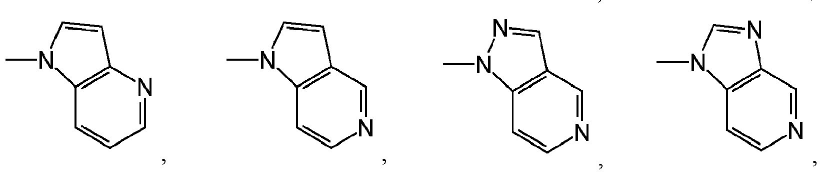 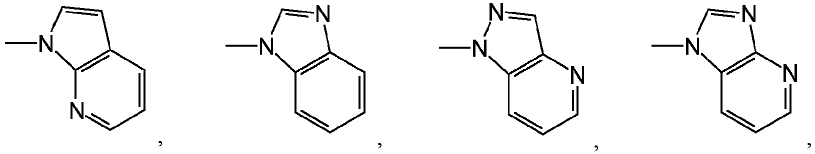 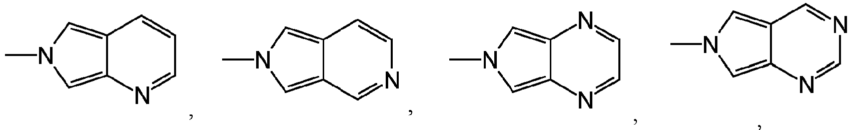 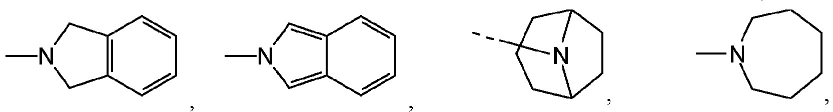 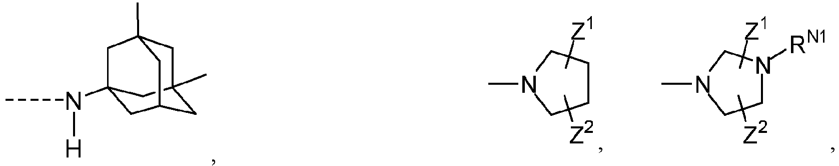 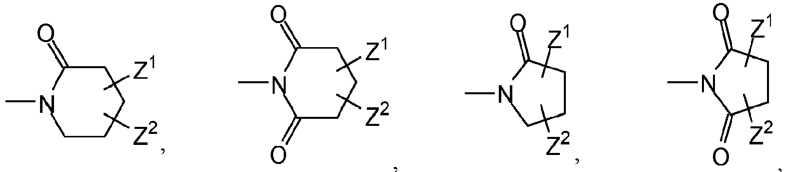 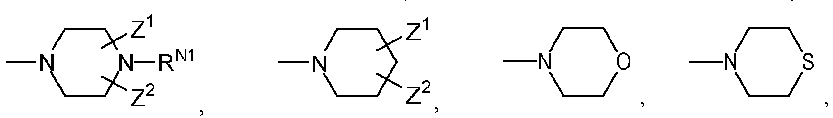 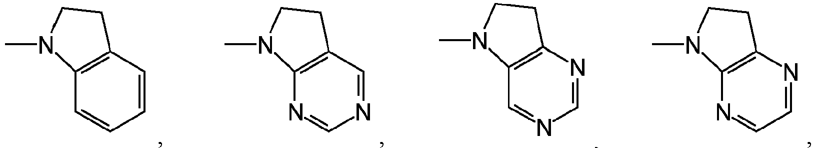 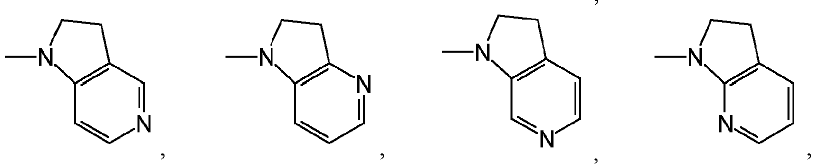 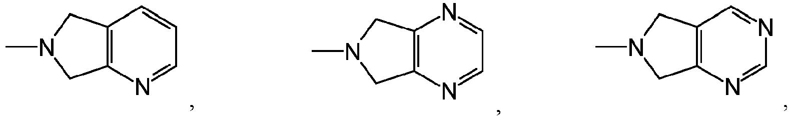 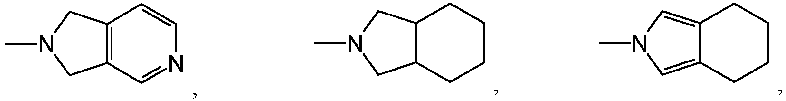 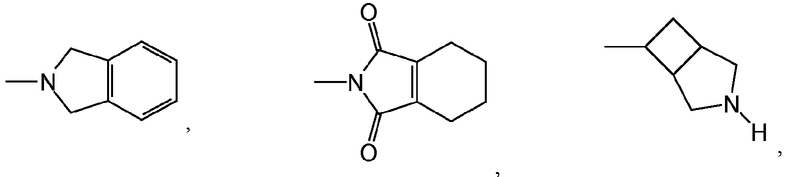 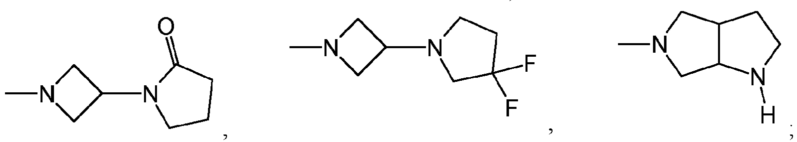
**R⁹** represents -H, 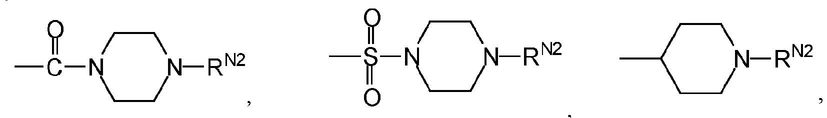 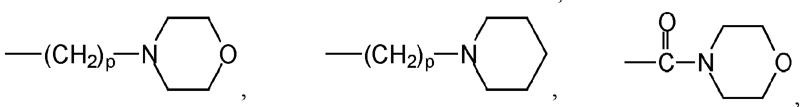 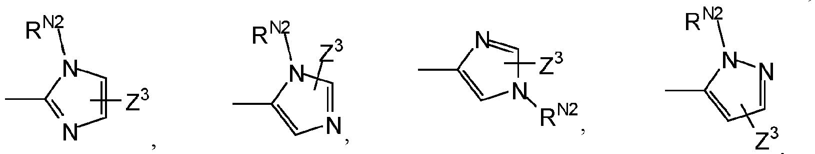 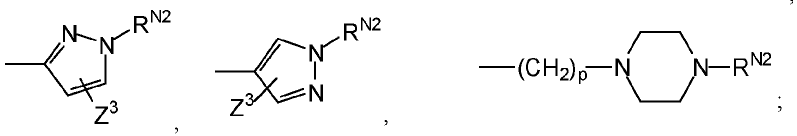
**R¹⁰** represents -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -(CH₂)ₙ-NH₂, -(CH₂)ₙ-NHCH₃, -(CH₂)ₙ-NHC₂H₅, -(CH₂)ₙ-NHC₃H₇, -(CH₂)ₙ-N(CH₃)₂, -(CH₂)ₙ-N(C₂H₅)₂, -(CH₂)ₙ-N(C₃H₇)₂, 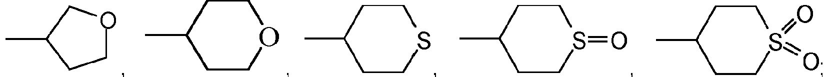 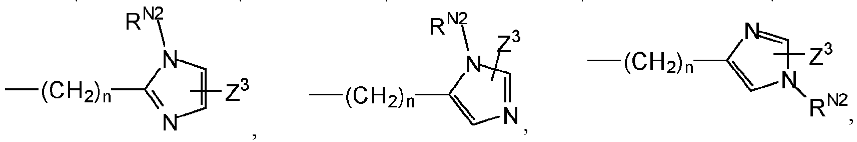 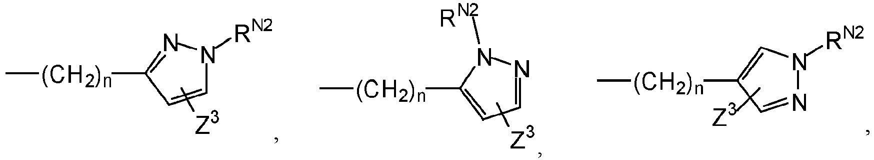
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N1}** and **R^{N2}** represent independently of each other -H, -CH₃, -C₂H₅, -C₃H₇, -CH(CH₃)₂, cyclo-C₃H₅, cyclo-C₄H₇, cyclo-C₅H₉, cyclo-C₆H₁₁, -C₄H₉, -C(CH₃)₃, -CH₂-CH(CH₃)₂, -CH(CH₃)-C₂H₅, -C₅H₁₁, -Ph, -CH₂-Ph, -CH=CH₂, -CH₂-CH=CH₂, -CH=CH-CH₃, -C≡CH, -CH₂-C≡CH, -C≡C-CH₃, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 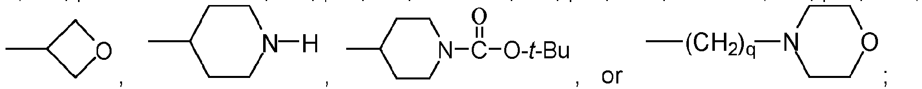
**Z¹** - **Z⁴** represent independently of each other -H, -CH₃, -C₂H₅, -C₃H₇, -CH(CH₃)₂, -Ph, -COCH₃, -COC₂H₅, -COOH, -COOCH₃, -COOC₂H₅, -NH₂, -NHCH₃, -NHC₂H₅, -N(CH₃)₂, -N(C₂H₅)₂, -CH₂-NH₂, -SO₃H, -F, -Cl, -Br, -I, -CN, -CF₃, -OCF₃, -OH, -OCH₃, or -OC₂H₅;
m is an integer selected from 0, 1, 2, 3, or 4;
n is an integer selected from 1, 2, 3, or 4;
p is an integer selected from 0, 1, 2, 3, or 4;
q is an integer selected from 1, 2, 3, or 4;
with the proviso that when X is -H at least one of R¹ - R³ is not -H; and when **R⁴** is -H, at least one of **R¹** - **R³** and **R⁵** - **R⁷** is not -H; and the following compound is excluded from the scope of the present application: 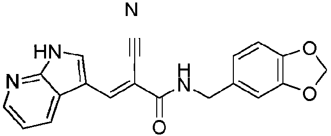 or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof.

Preferred are compounds wherein R⁴ is different from hydrogen (-H). In case R⁴ is hydrogen one or at least one of the substituents **R¹** - **R³** and **R⁵** - **R⁷** is not hydrogen and preferably two or three or more than three substituents **R¹** - **R³** and **R⁵** - **R⁷** are not hydrogen. Even more preferred are the compounds wherein one or at least one of the substituents **R⁴** - **R⁷** is not hydrogen. Still more preferred are the compounds wherein one or at least one of the substituents **R¹** - **R⁴** is not hydrogen. Most preferred are the compounds wherein one or at least one of the substituents **R¹** - **R³** is not hydrogen and wherein one or at least one of the substituents **R⁵** - **R⁷** is not hydrogen.

Preferably, the compounds of general formula (Ia) as defined herein are under the proviso that when X is -H at least one of R¹ - R³ is not -H and under the second proviso that **R⁵**, **R⁶** and **R⁷** are not -H at the same time or preferably under the further proviso that at least one of R¹ - R³ is not -H, when **R⁵** and **R⁶** or **R⁶** and **R⁷** form together 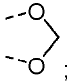 more preferably under the further proviso that when **X** is -CN or when **R⁴** is -H or -C₂H₅ or - CH₂OH or -C₂H₄OH, at least one of **R⁵**, **R⁶** and **R⁷** is different from -H or still more preferably under the second proviso that in case **X** is -CN and **R⁴** is -H or -C₂H₅ or - CH₂OH or -C₂H₄OH, at least one of **R⁵**, **R⁶** and **R⁷** is different from -H.

Also preferred are the following general formulae (**Ia-1**), (**Ia-2**), (**Ia-3**), and (I**a-4**) 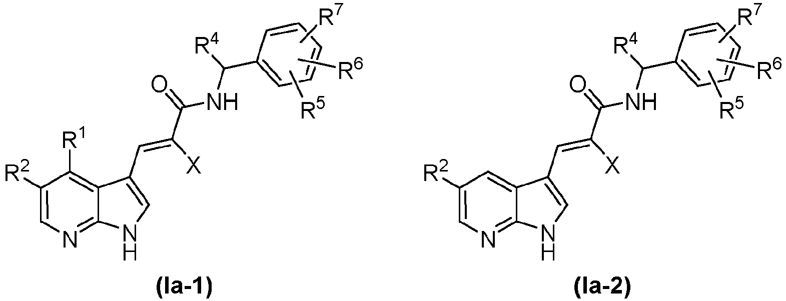 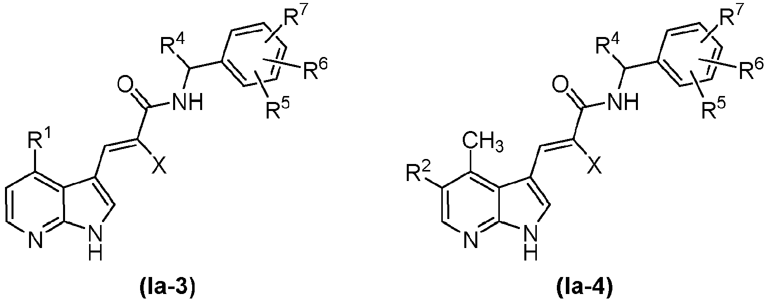 wherein the substituents **R¹** - **R⁷** and **X** have the meanings or the preferred meanings as disclosed herein, but the substituents **R¹** and **R²** are different from hydrogen (-H).

In general formula (I) and all other general formulae disclosed herein, the substituents **R⁵** and **R⁶** or **R⁶** and **R⁷** form together preferably one of the following five-membered ring systems 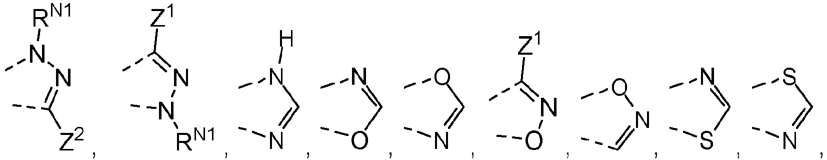 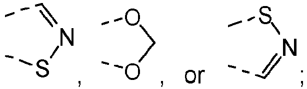 more preferably one of the following five-membered ring systems 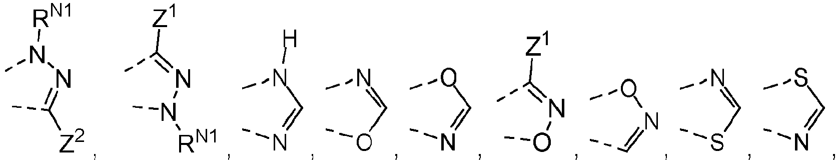 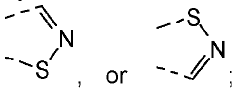 still more preferably one of the following five-membered ring systems

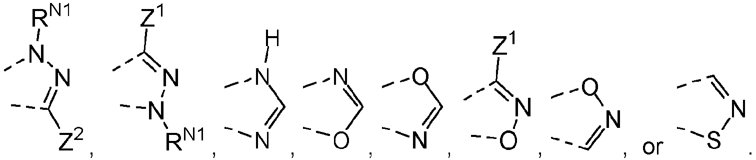

In some embodiments, the present invention refers to the compound of the formula (I), or (**Ia**), wherein
**R¹** and **R²** represent independently of each other -H, -Ph, -CH₃, -CF₃, -Cl, -Br, -CN, -OH, -NH-Ph, -**R⁹**, -O-**R¹⁰**, 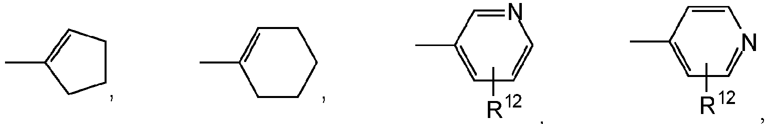 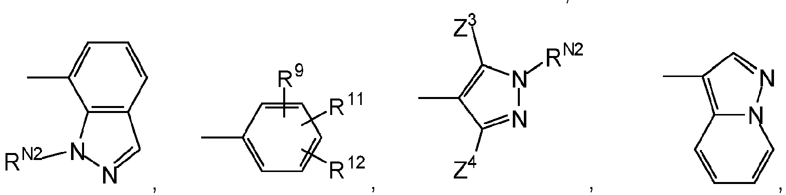 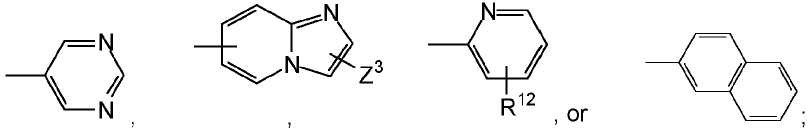
wherein **R⁹, R¹⁰**, **R¹¹**, **R¹²**, **R^{N2}, Z³**, **Z⁴** have the meanings as defined in the formula (I).

In some embodiments, the present invention refers to the compound of the formula (I), wherein
**R¹** represents -H, -CH₃, -Cl, 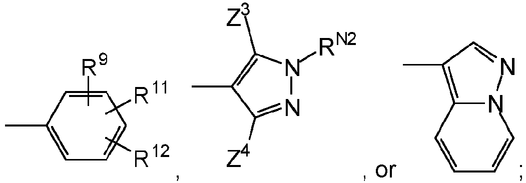
**R²** represents -H, -Ph, -CH₃, -CF₃, -CI, -Br, -CN, -OH, -NH-Ph, -**R⁹**, -O-**R¹⁰**, 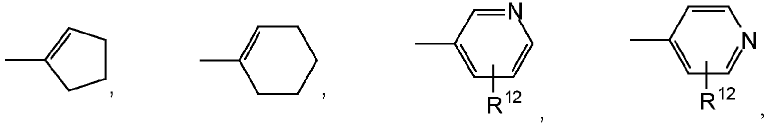 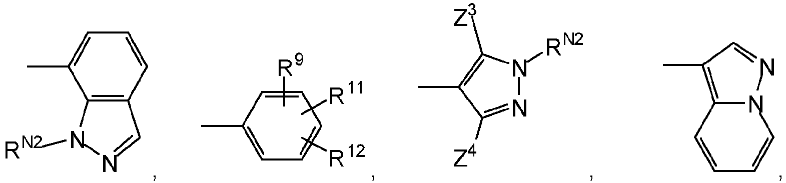 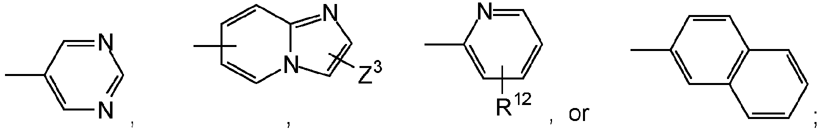
**R⁹** represents -H, 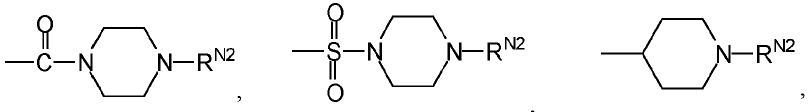 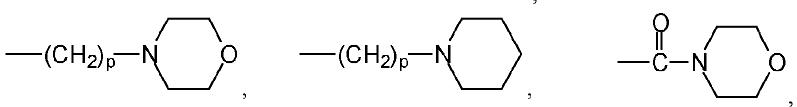 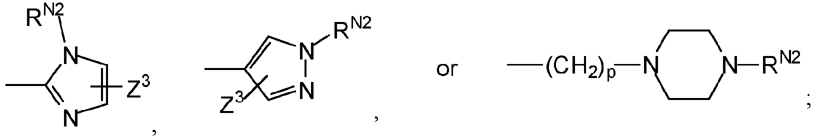
**R¹⁰** is -CH(CH₃)₂, -CH₂-CH(CH₃)₂, -CH(CH₃)-C₂H₅, -(CH₂)ₙ-N(CH₃)₂, 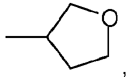 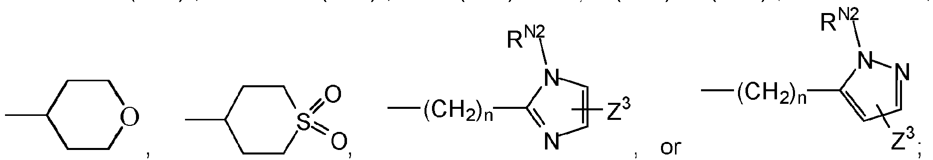
wherein **n, p, R¹¹**, **R¹²**, **R^{N2}**, **Z³**, **Z⁴** have the meanings as defined in the formula (I).

Preferred, is the compound of the formula (**I**): 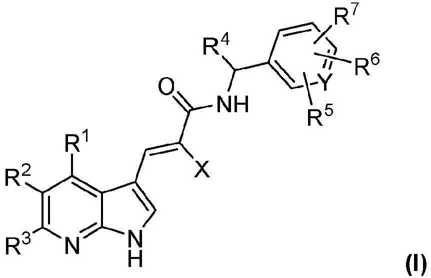 wherein
**X** represents -H or -CN,
**Y** represents CH or N,
**R¹** represents -H, -CH₃, -Cl, -Ph, 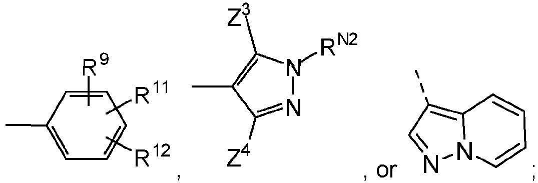
**R²** represents -H, -CH₃, -Ph, -Cl, -Br, -CF₃, -CN, -OH, -NH-Ph, -O-CH₂CH₂-N(CH₃)₂, -**R⁹**, -O-**R¹⁰**, 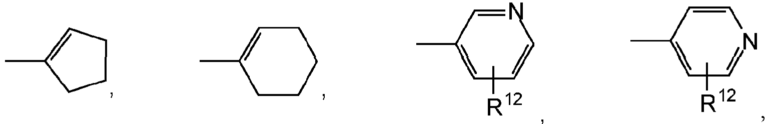 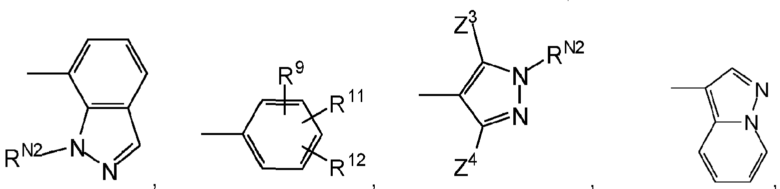 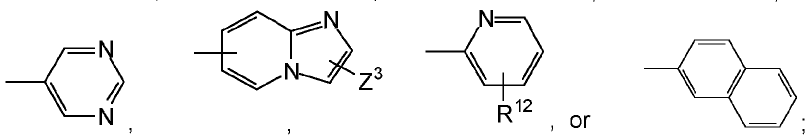
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵** , **R⁶** and **R⁷** represents independently of each other -**R⁸**, -CH₂-**R⁸**, -SO₂-**R⁸**, -H, -F, -Cl, -Br, -CN, -CH₃, -CF₃, -OCH₃, -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃,
or **R⁵** and **R⁶**, or **R⁶** and **R⁷** form 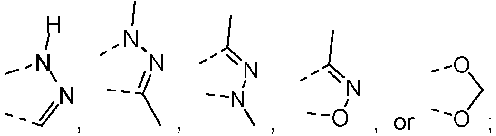
**R⁸** represents 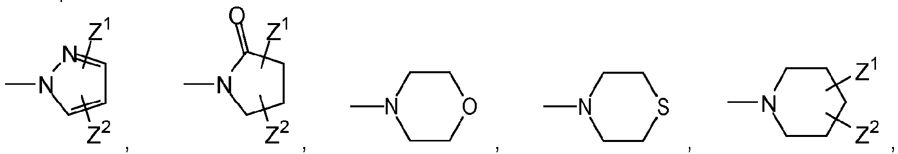 or 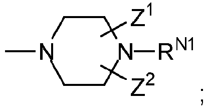 preferably 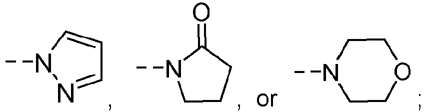
**R⁹** represents -H, 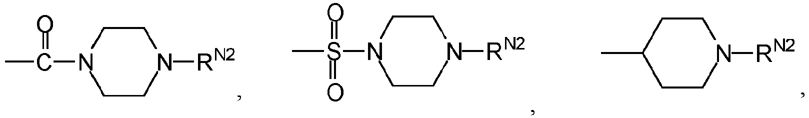 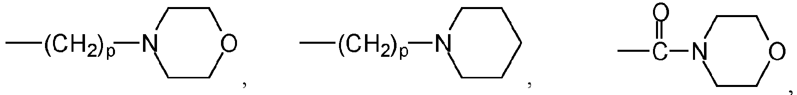 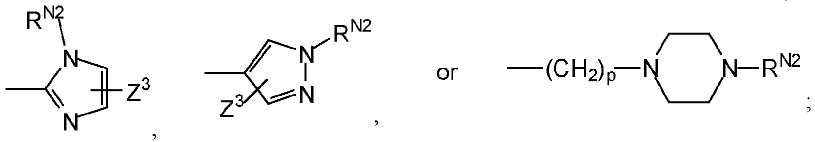
**R¹⁰** represents -CH(CH₃)₂, -CH₂-CH(CH₃)₂, -CH(CH₃)-C₂H_{5,} -(CH₂)ₙ-N(CH₃)₂, 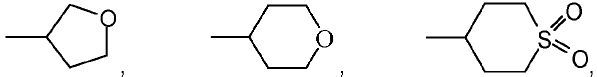 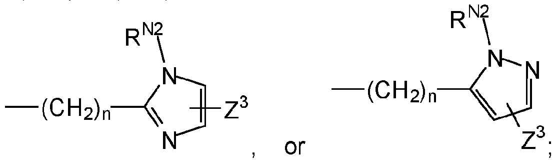
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₅, -(CH₂)ₘ-SO₂C₃H₇, -(CH₂)ₘ₋SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
Preferably, **R¹¹** and **R¹²** represent independently of each other -H, -CH₃, -C₂H₅, -C₃H₇, -CH(CH₃)₂, -CF₃, -(CH₂)ₘ-N(CH₃)₂, -(CH₂)ₘ-N(C₂H₅)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -O-(CH₂)ₘ-N(C₂H₅)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
More preferably, **R¹¹** and **R¹²** represent independently of each other -H, -CH₃, -C₂H₅, -CF₃, -(CH₂)ₘ-N(CH₃)₂, -OCH₃, -OC₂H₅, -F, -Cl, -Br, -CN, -(CH₂)ₘ-SO₂CH₃, -(CH₂)ₘ-SO₂-cyclo-C₃H₅, -O-(CH₂)ₘ-N(CH₃)₂, -NHCOCH₃, -(CH₂)ₘ-NHCOCH₃, -(CH₂)ₘ-NHCO-CH=CH₂, -SO₂NH₂, -SO₂NHCH₃, -SO₂N(CH₃)₂, -SO₂N(C₂H₅)₂, -SO₂N(C₃H₇)₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N1}** and **R^{N2}** represents independently of each other -H, -CH₃, -C₂H₅, -C₃H₇, -CH(CH₃)₂, cyclo-C₃H₅, cyclo-C₄H₇, -CH₂-CH(CH₃)₂, -Ph, -CH₂-Ph, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 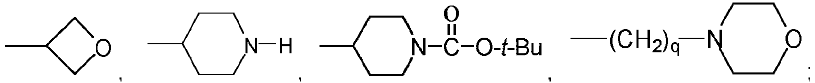
**Z¹**, **Z²**, **Z³** and **Z⁴** represent independently of each other -H, -CH₃, -C₂H₅, -C₃H₇, -CH(CH₃)₂, -F, -CI, -Br, -I, -CN, -CF₃;
m is an integer selected from 0, 1, 2, or 3;
n is an integer selected from 1, or 2;
p is an integer selected from 0, or 1;
q is an integer selected from 1, or 2;
with the proviso that when X is -H at least one of R¹ - R³ is not -H; when **R⁴** is -H, at least one of **R¹ - R³** and **R⁵ - R⁷** is not -H; and
   (E)-N-(1,3-benzodioxol-5-yl-methyl)-2-cyano-3-(1H-pyrrolo[2,3-b]pyridin-3-yl)acrylamide excluded;
or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof.

Also preferred, is the compound of the formula (la): 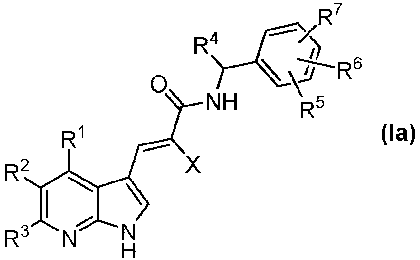 wherein
**X** represents -H or -CN,
**R¹** is -H, -CH₃, -Cl, -Ph, 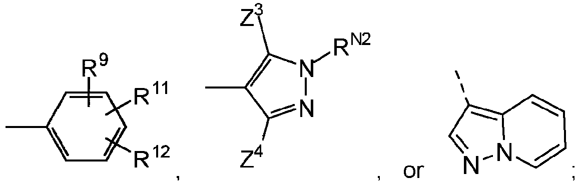
**R²** represents -H, -CH₃, -Ph, -Cl, -Br, -CF₃, -CN, -OH, -NH-Ph, -O-CH₂CH₂-N(CH₃)₂, **-R⁹,** -O-**R¹⁰**, 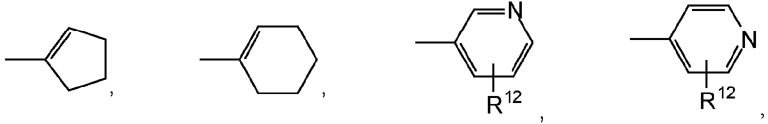 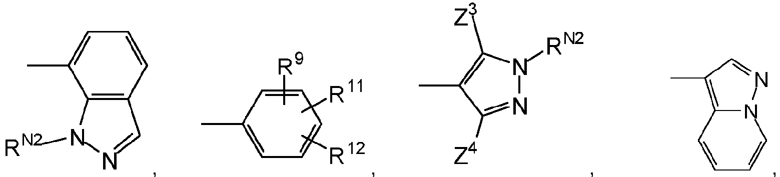 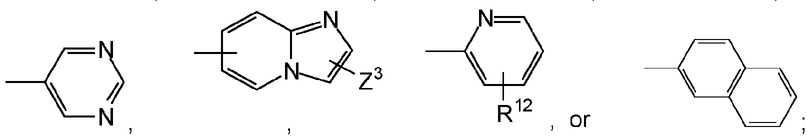
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵ , R⁶** and **R⁷** represents independently of each other **-R⁸**, -CH₂**-R⁸**, -SO₂**-R⁸**, -H, -F, -Cl, -Br, -CN, -CH₃, -CF₃, -OCH₃, -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, or **R⁵** and **R⁶** , or **R⁶** and **R⁷** form 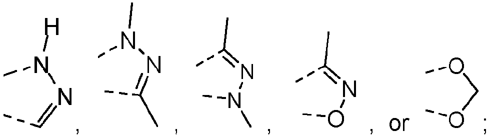
more preferred, **R⁵** and **R⁶,** or **R⁶** and **R⁷** form 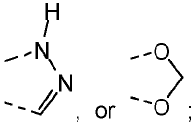
**R⁸** represents 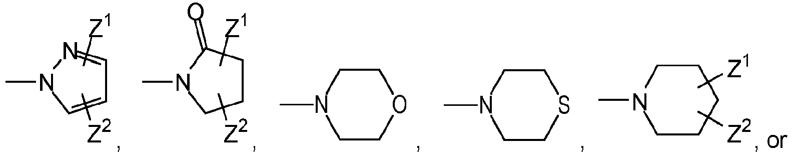 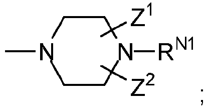 preferably 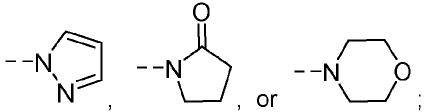
**R⁹** represents -H, 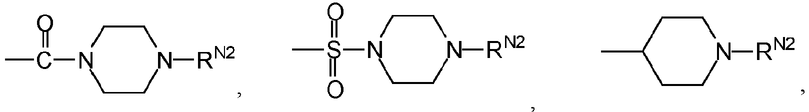 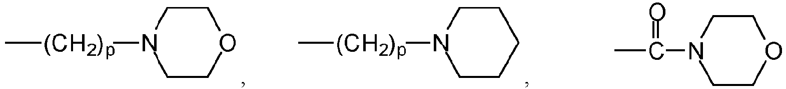 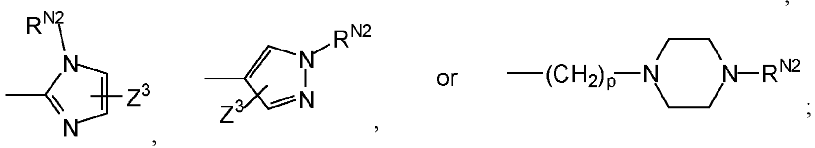
**R¹⁰** represents -CH(CH₃)₂, -CH₂-CH(CH₃)₂, -CHCH₃-C₂H_{5,} -(CH₂)ₙ-N(CH₃)₂, 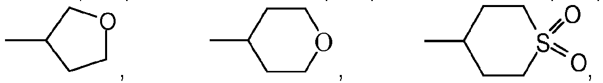 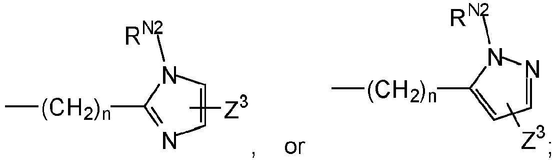
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
Preferably, **R¹¹** and **R¹²** represent independently of each other -H, -CH₃, -C₂H₅, -C₃H₇, -CH(CH₃)₂, -CF₃, -(CH₂)ₘ-N(CH₃)₂, -(CH₂)ₘ-N(C₂H₅)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -O-(CH₂)ₘ-N(C₂H₅)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
More preferably, **R¹¹** and **R¹²** represent independently of each other -H, -CH₃, -C₂H₅, -CF₃, -(CH₂)ₘ-N(CH₃)₂, -OCH₃, -OC₂H₅, -F, -Cl, -Br, -CN, -(CH₂)ₘ-SO₂CH₃, -(CH₂)ₘ-SO₂-cyclo-C₃H₅, -O-(CH₂)ₘ-N(CH₃)₂, -NHCOCH₃, -(CH₂)ₘ-NHCOCH₃, -(CH₂)ₘ-NHCO-CH=CH₂, -SO₂NH₂, -SO₂NHCH₃, -SO₂N(CH₃)₂, -SO₂N(C₂H₅)₂, -SO₂N(C₃H₇)₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N1}** and **R^{N2}** represents independently of each other -H, -CH₃, -C₂H₅, -C₃H₇, -CH(CH₃)₂, cyclo-C₃H₅, cyclo-C₄H₇, -CH₂-CH(CH₃)₂, -Ph, -CH₂-Ph, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 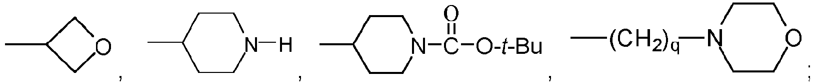
**Z¹**, **Z²**, **Z³** and **Z⁴** represent independently of each other -H, -CH₃, -C₂H₅, -C₃H₇, -CH(CH₃)₂, -F, -Cl, -Br, -I, -CN, -CF₃;
m is an integer selected from 0, 1, 2, or 3;
n is an integer selected from 1, or 2;
p is an integer selected from 0, or 1;
q is an integer selected from 1, or 2;
with the proviso that when **X** is -H, at least one of **R¹** - **R³** is not -H, when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded;
or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof .

More preferred, is the compound of the formula (I) 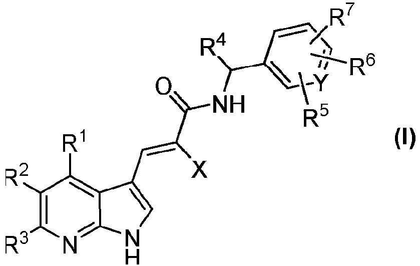 wherein
**X** represents -H or -CN,
**Y** represents CH or N,
**R¹** and **R²** represents independently of each other -H, -CH₃, -Ph, -Cl, -Br, -CF₃, -CN, -OH, -NH-Ph, -O-CH(CH₃)₂, -O-CH₂CH(CH₃)₂, -O-CH(CH₃)(CH₂ CH₃), -O-CH₂CH₂-N(CH₃)₂, 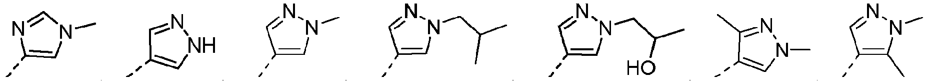 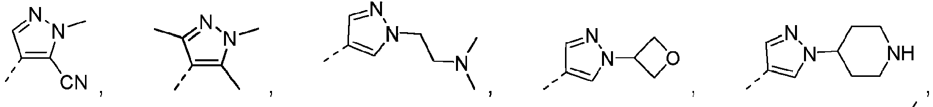 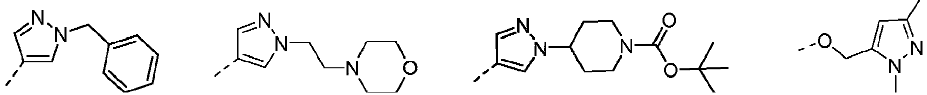 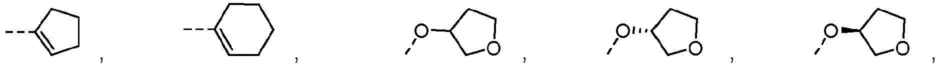 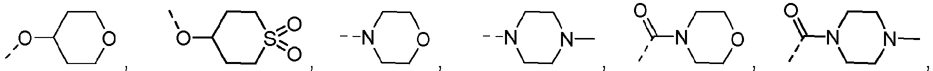 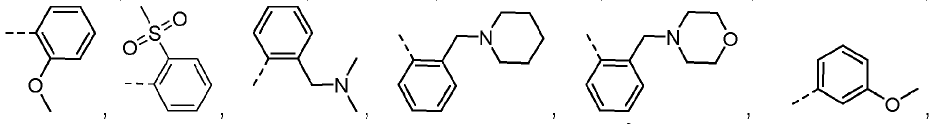 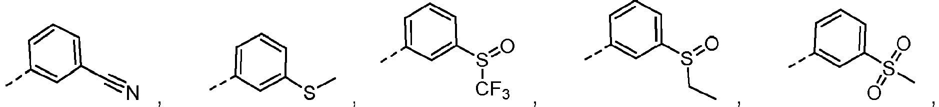 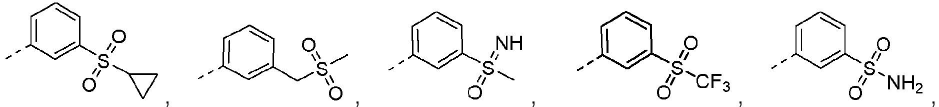 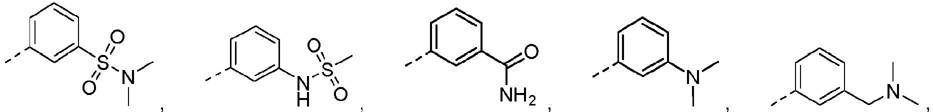 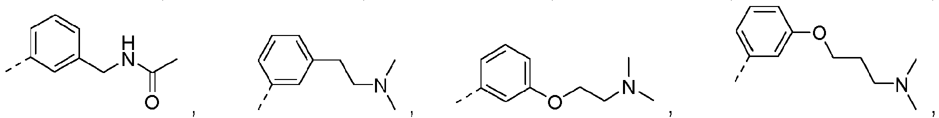 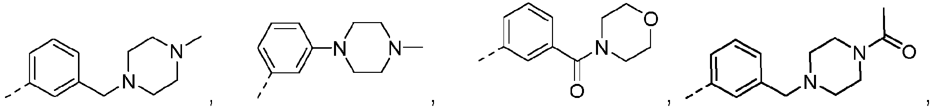 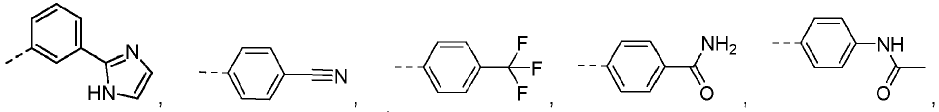 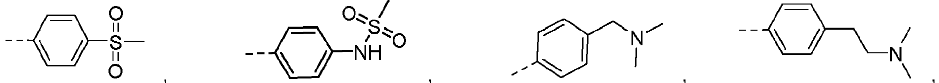 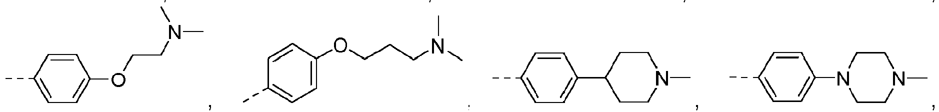 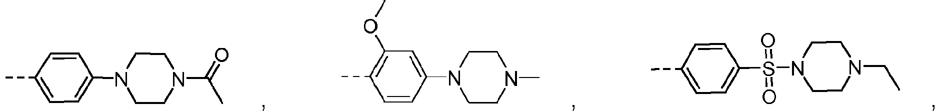 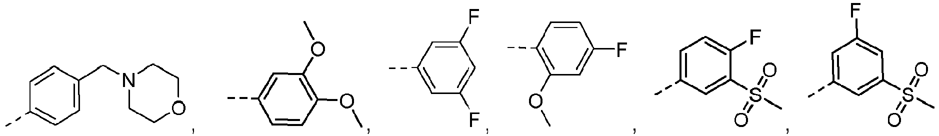 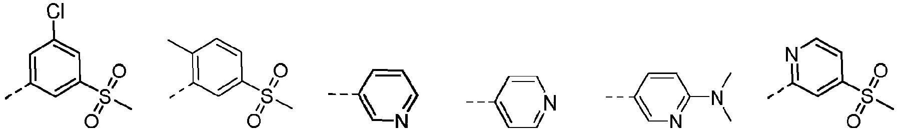 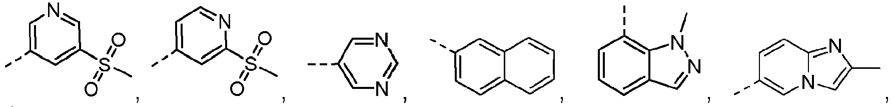 or 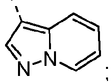
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵** , **R⁶** and **R⁷** represents independently of each other -H, -F, -Cl, -Br, -CN, , -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, 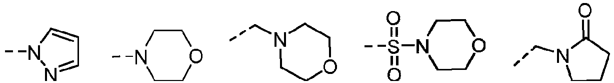
or **R⁵** and **R⁶,** or **R⁶** and **R⁷** form 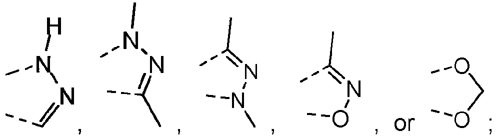 with the proviso that when X is -H at least one of R¹ - R³ is not -H; when **R⁴** is -H, at least one of **R¹** - **R³** and **R⁵** - **R⁷** is not -H; and
   (E)-N-(1,3-benzodioxol-5-yl-methyl)-2-cyano-3-(1H-pyrrolo[2,3-b]pyridin-3-yl)acrylamide is excluded;
or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof.

More preferred, is the compound of the formula **(Ia)** 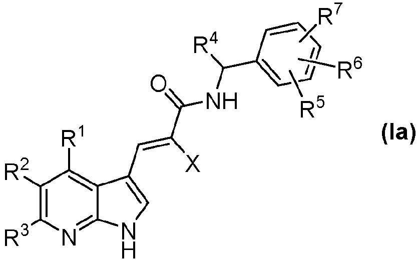 wherein
**X** represents -H or -CN,
**R¹** and **R²** represents independently of each other -H, -CH₃, -Ph, -Cl, -Br, -CF₃, -CN, -OH, -NH-Ph, -O-CH(CH₃)₂, -O-CH₂CH(CH₃)₂, -O-CH(CH₃)(CH₂ CH₃), -O-CH₂CH₂-N(CH₃)₂, 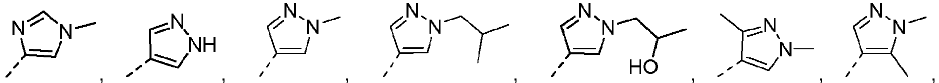 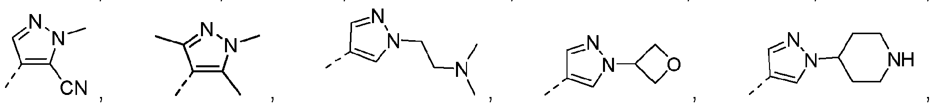 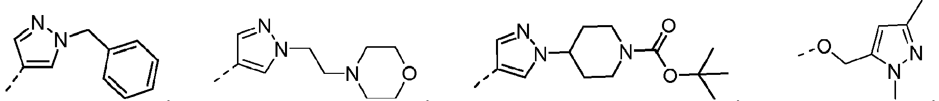 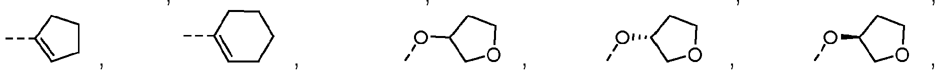 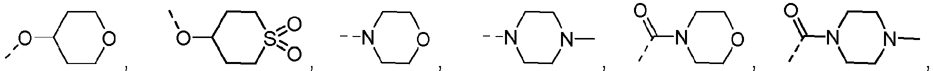 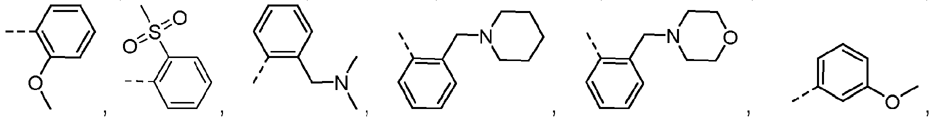 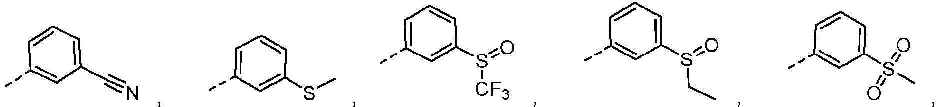 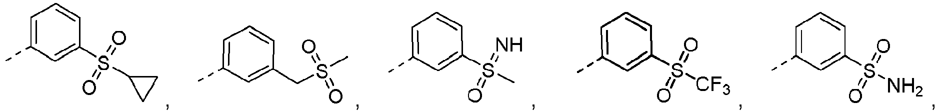 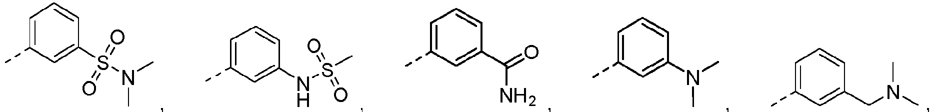 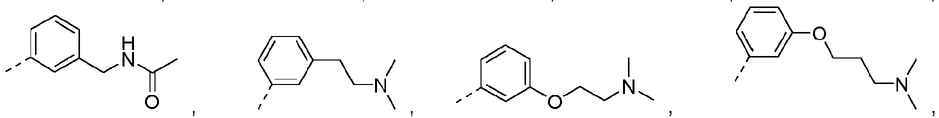 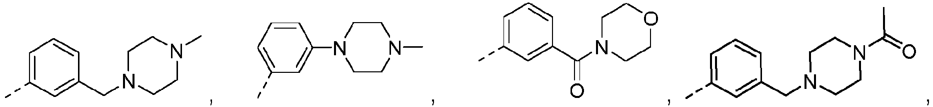 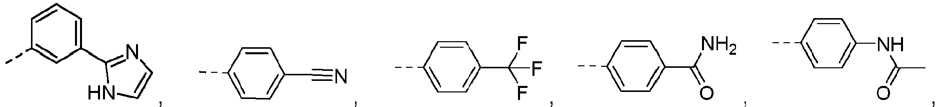 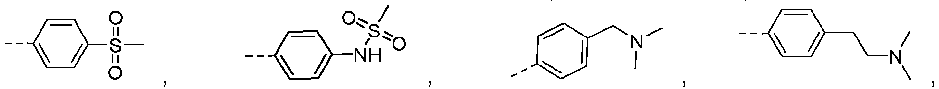 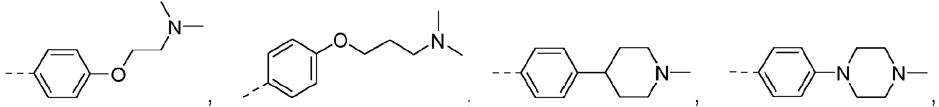 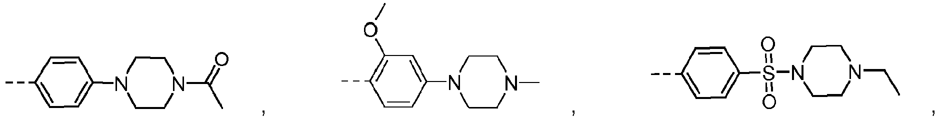 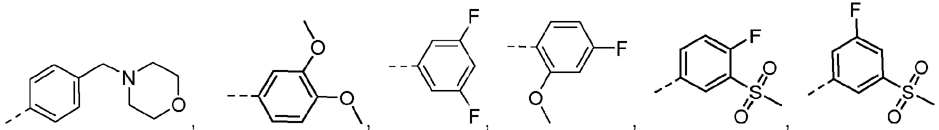 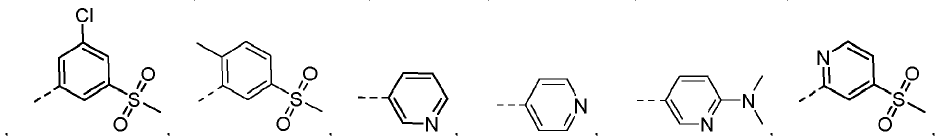 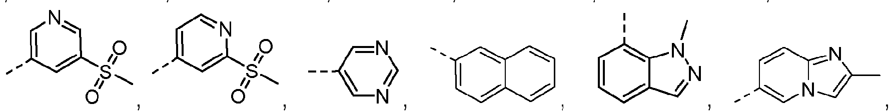 or 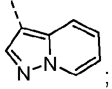
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵** , **R⁶** and **R⁷** represents independently of each other -H, -F, -Cl, -Br, -CN, , -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, 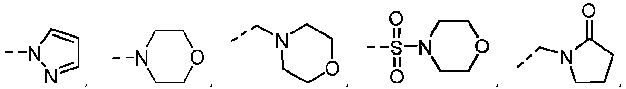
or **R⁵** and **R⁶,** or **R⁶** and **R⁷** form 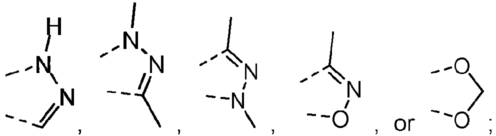
preferably, **R⁵** and **R⁶,** or **R⁶** and **R⁷** form 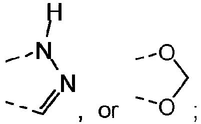 with the proviso that when X is -H, at least one of **R¹** - **R³** is not -H, when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded;
or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof .

Still more preferred, is the compound of the formula **(Ia)** wherein 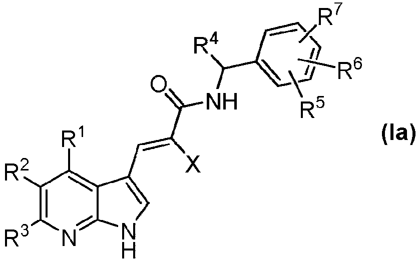
**X** represents -H or -CN,
**R¹** represents -H, -CH₃, -Cl, -Ph, 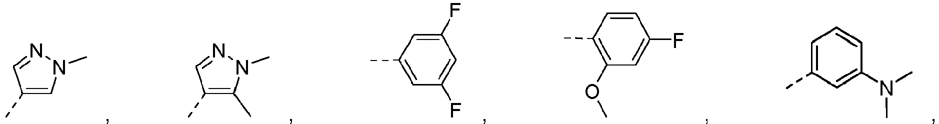 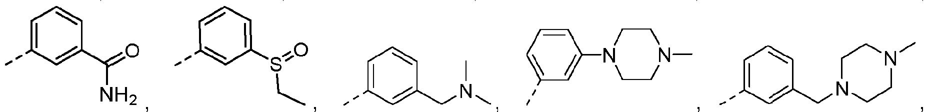 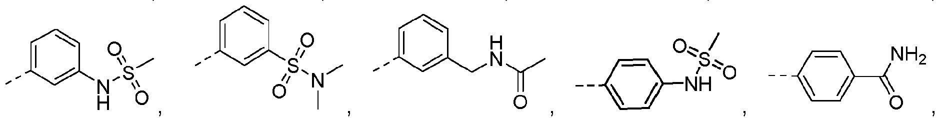 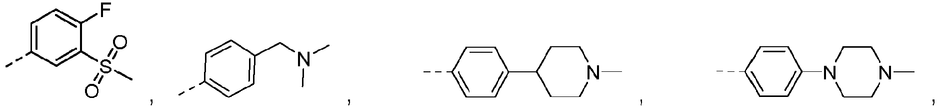 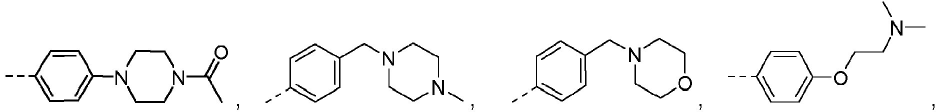 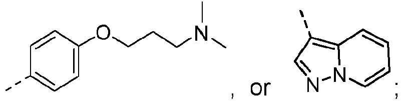
**R²** represents -H, -CH₃, -Cl, -Br, -CF₃, -CN, -OH, -Ph, -NH-Ph, -O-CH(CH₃)₂, -O-CH₂CH(CH₃)₂, -O-CH(CH₃)(CH₂ CH₃), -O-CH₂CH₂-N(CH₃)₂, 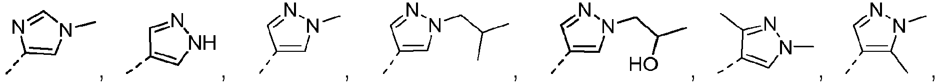 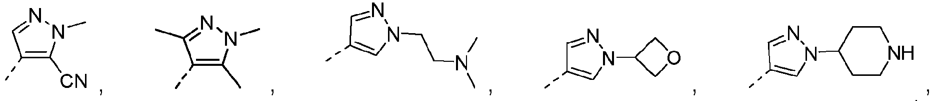 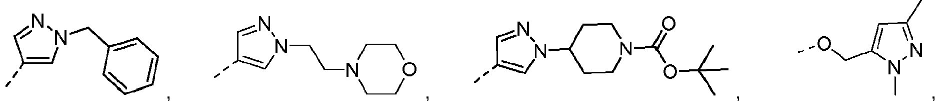 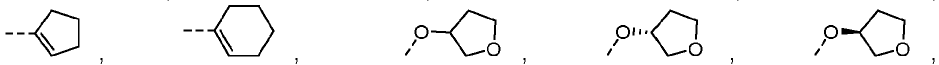 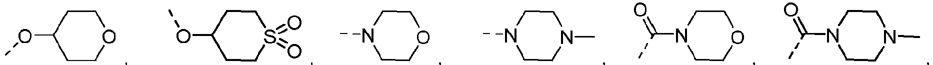 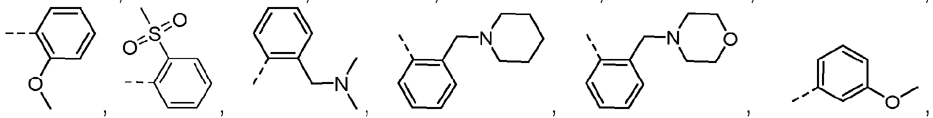 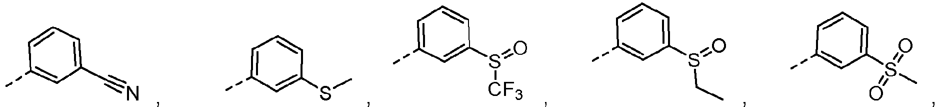 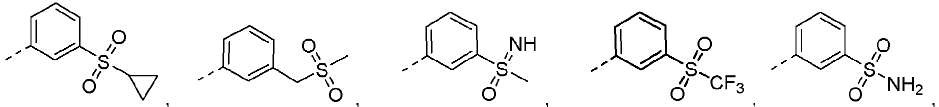 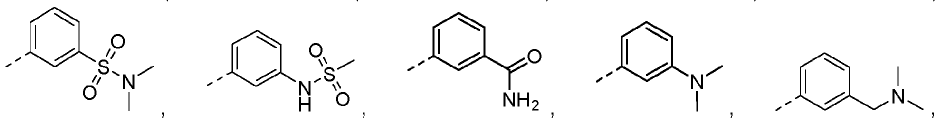 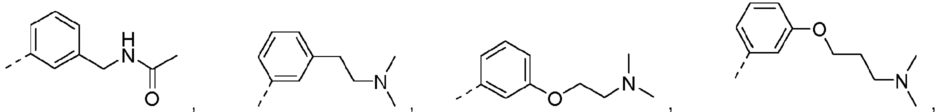 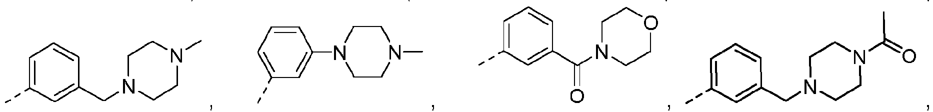 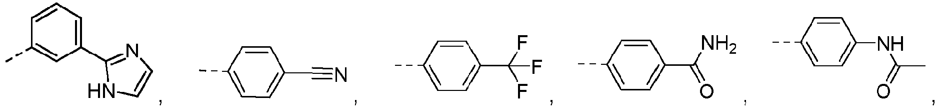 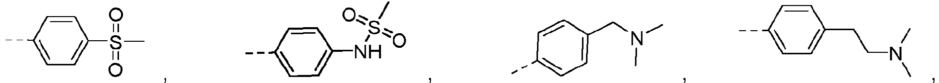 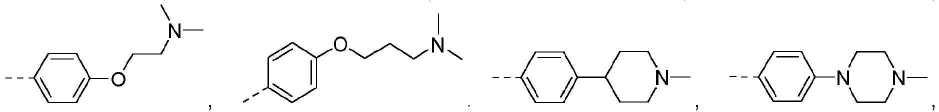 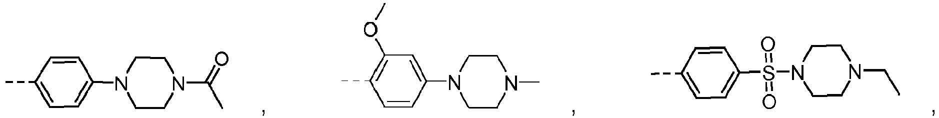 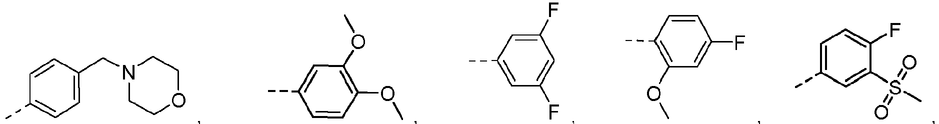 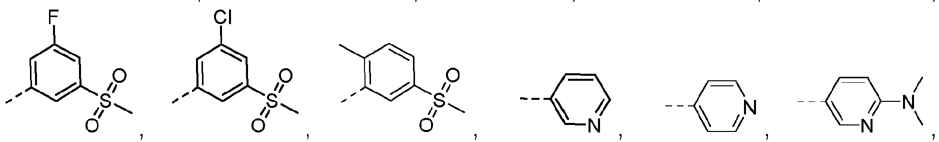 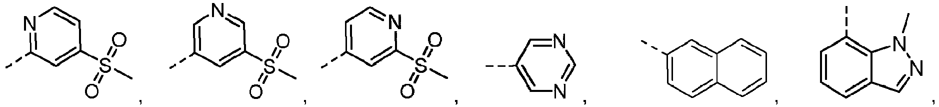 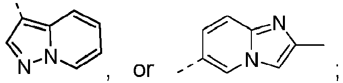
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵** , **R⁶** and **R⁷** represents independently of each other -H, -F, -Cl, -Br, -CN, , -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, 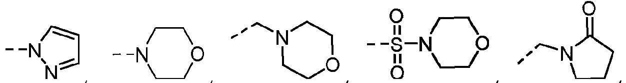
or **R⁵** and **R⁶,** or **R⁶** and **R⁷** form 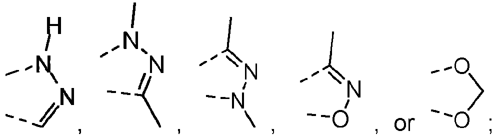
preferably, **R⁵** and **R⁶,** or **R⁶** and **R⁷** form 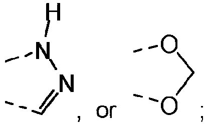 with the proviso that when **X** is -H, at least one of **R¹** - **R³** is not -H, when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded;
or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof .

In some embodiments, the present invention relates to the compound having any one of the formulae **(Ia)**, **(Ib)**, **(II-1)** - **(II-5)**: 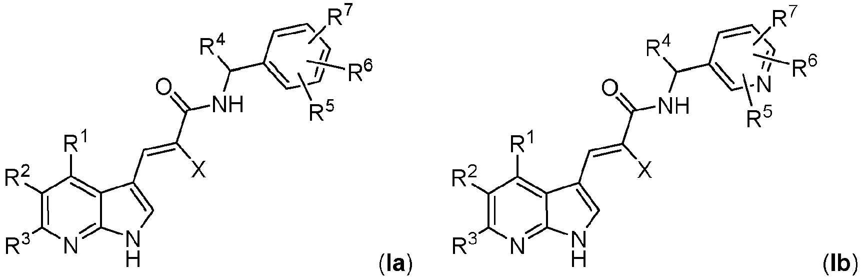 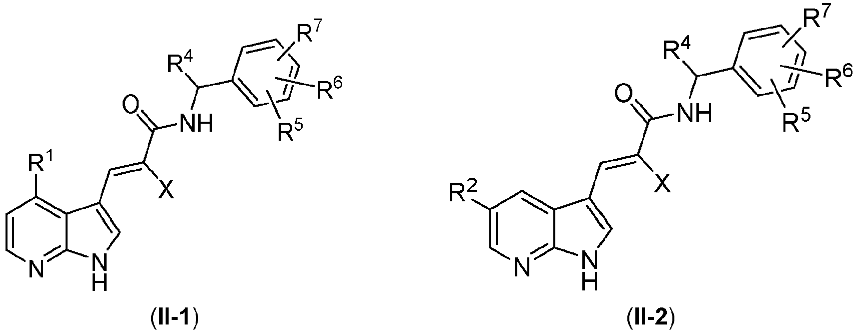 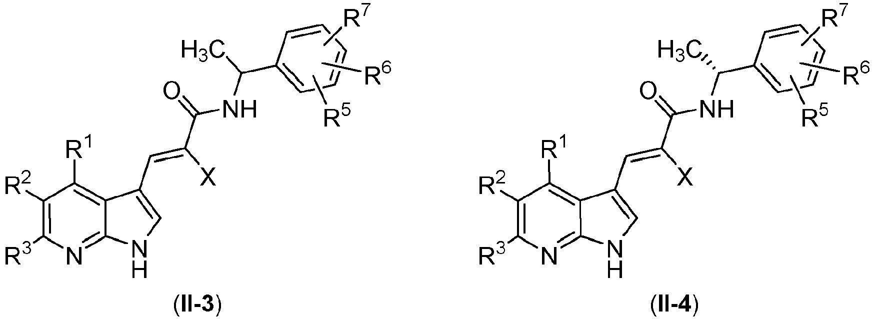 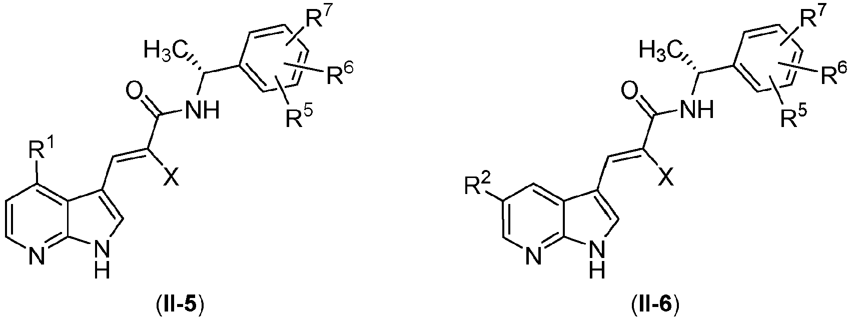 wherein **R¹, R², R³, R⁴, R⁵, R⁶, R⁷** and **X** have the meanings as defined in the formula (I).

In formulae (II-1) and (II-5) R¹ is preferably not -H.

In formulae (II-2) and (II-6) R² is preferably not -H.

In formulae (II-3) and (II-4) when X is -H at least one of R¹ - R³ is not -H and preferably at least one of **R⁵, R⁶** and **R⁷** is not -H; or more preferably in case **X** is -CN at least one of **R⁵, R⁶** and **R⁷** is not -H.

Preferably, is the compound of any one of the formulae **(II-1)** to **(II-6)**, wherein
**X** represents -H or -CN,
**R¹** represents -H, -CH₃, -CI, -Ph, 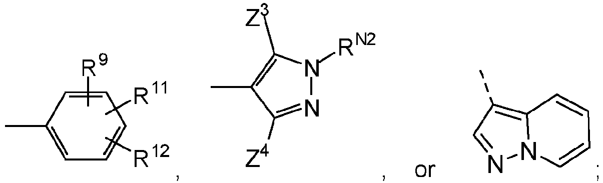
**R²** represents -H, -CH₃, -Ph, -Cl, -Br, -CF₃, -CN, -OH, -NH-Ph, -O-CH₂CH₂-N(CH₃)₂, -**R⁹**, -O-**R¹⁰**, 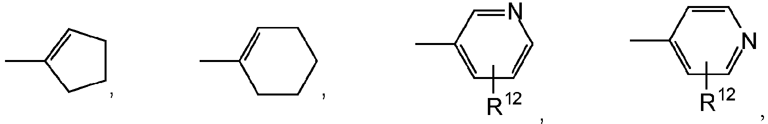 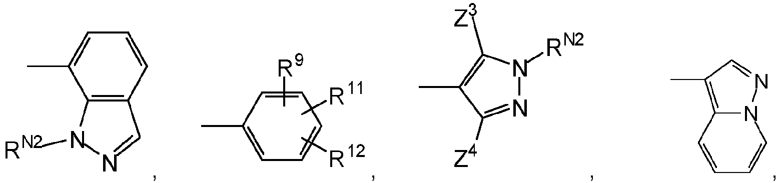 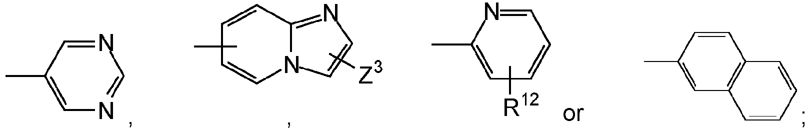
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵, R⁶** and **R⁷** represents independently of each other **-R⁸**, -CH₂**-R⁸**, -SO₂-**R⁸**, -H, -F, -Cl, -Br, -CN, -CH₃, -CF₃, -OCH₃, -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, or **R⁵** and **R⁶,** or **R⁶** and **R⁷** form 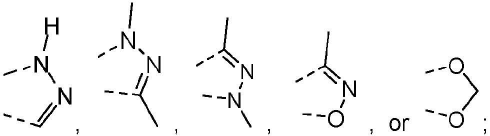
preferably, **R⁵** and **R⁶,** or **R⁶** and **R⁷** form 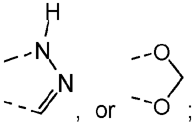
**R⁸** represents 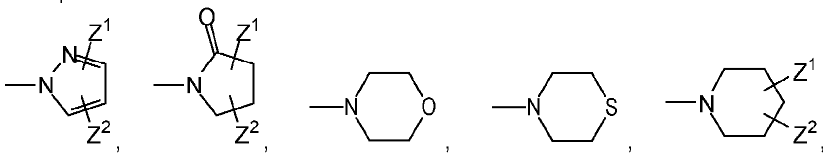 or 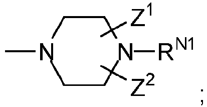 preferably 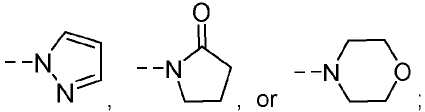
**R⁹** represents -H, 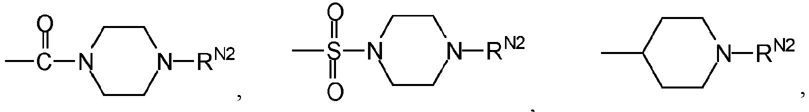 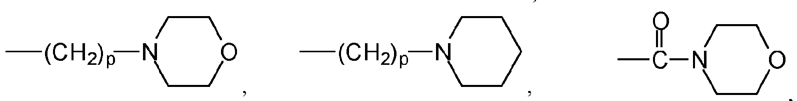 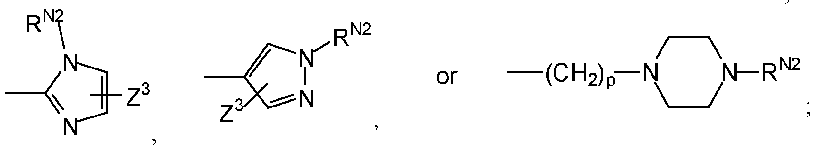
**R¹⁰** represents -CH(CH₃)₂, -CH₂-CH(CH₃)₂, -CH(CH₃)-C₂H₅, -(CH₂)ₙ-N(CH₃)₂, 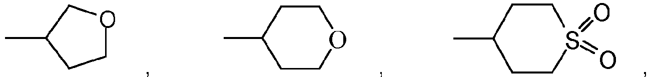 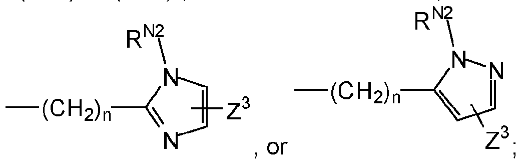
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘN(C3H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
Preferably, **R¹¹** and **R¹²** represent independently of each other -H, -CH₃, -C₂H₅, -C₃H₇, -CH(CH₃)₂, -CF₃, -(CH₂)ₘ-N(CH₃)₂, -(CH₂)ₘ-N(C₂H₅)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -O-(CH₂)ₘN(C₂H₅)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
More preferably, **R¹¹** and **R¹²** represent independently of each other -H, -CH₃, -C₂H₅, -CF₃, -(CH₂)ₘ-N(CH₃)₂, -OCH₃, -OC₂H₅, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -NHCOCH₃, -(CH₂)ₘ-NHCOCH₃, -(CH₂)ₘ-NHCO-CH=CH₂, -SO₂NH₂, -SO₂NHCH₃, -SO₂N(CH₃)₂, -SO₂N(C₂H₅)₂, -SO₂N(C₃H₇)₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N1}** and **R^{N2}** represents independently of each other -H, -CH₃, -C₂H₅, -C₃H₇, -CH(CH₃)₂, cyclo-C₃H₅, cyclo-C₄H₇, -CH₂-CH(CH₃)₂, -Ph, -CH₂-Ph, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 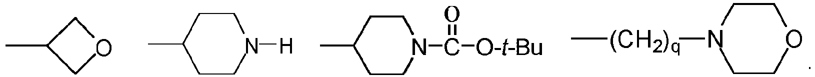
**Z¹**, **Z²**, **Z³** and **Z⁴** represent independently of each other -H, -CH₃, -C₂H₅, -C₃H₇, -CH(CH₃)₂, -F, -Cl, -Br, -I, -CN, -CF₃;
m is an integer selected from 0, 1, 2, or 3;
n is an integer selected from 1, or 2;
p is an integer selected from 0, or 1;
q is an integer selected from 1, or 2;
with the proviso that when **X** is -H, at least one of **R¹** - **R³** is not -H; when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded;

More preferably, is the compound of any one of the formulae **(II-1)** to **(II-6)**, wherein
**X** represents -H or -CN;
**R¹** and **R²** represents independently of each other -H, -CH₃, -Ph, -Cl, -Br, -CF₃, -CN, -OH, -NH-Ph, -O-CH(CH₃)₂, -O-CH₂CH(CH₃)₂, -O-CH(CH₃)(CH₂ CH₃), -O-CH₂CH₂-N(CH₃)₂, 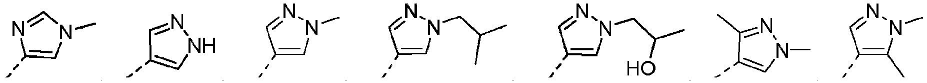 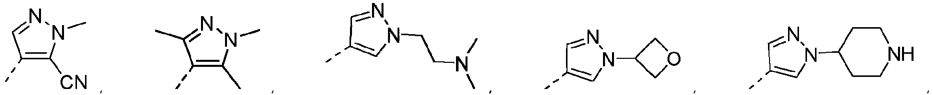 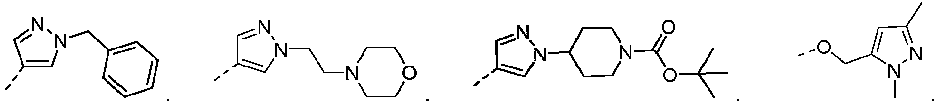 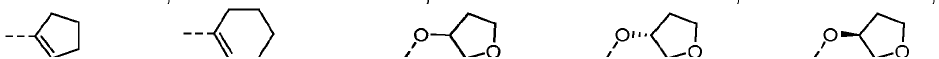 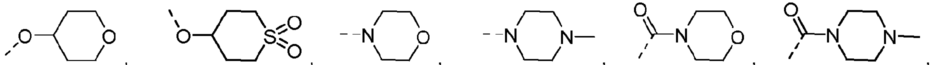 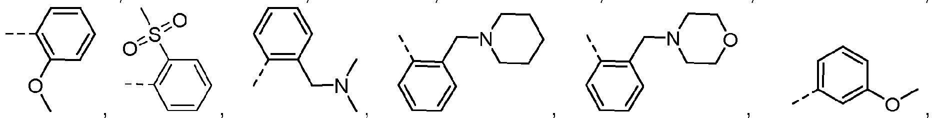 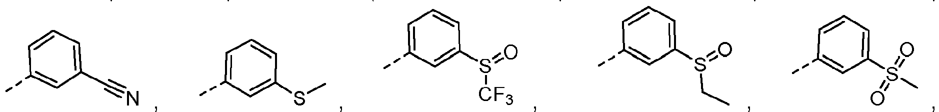 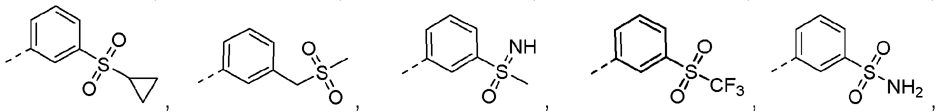 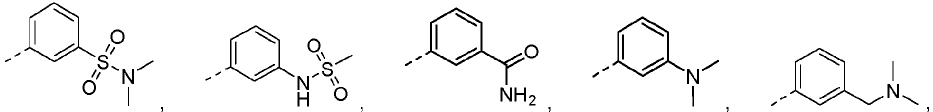 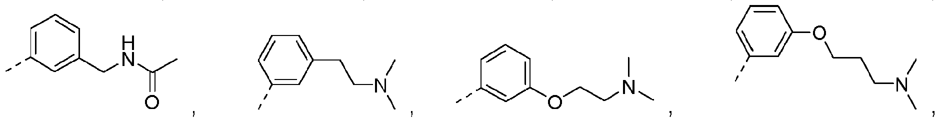 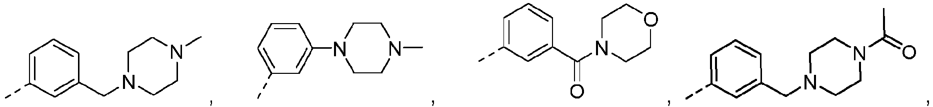 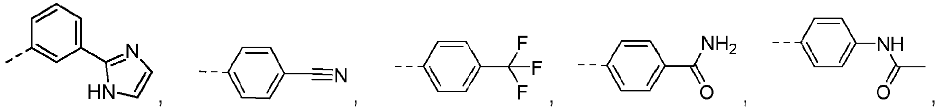 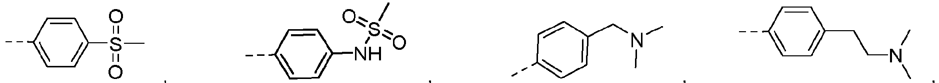 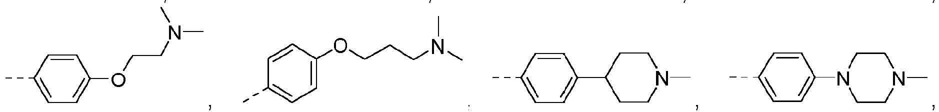 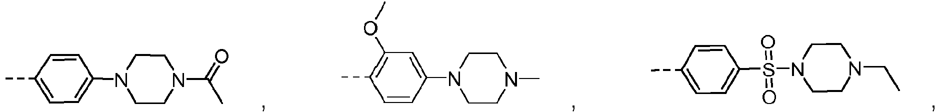 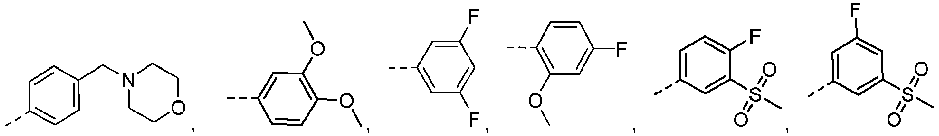 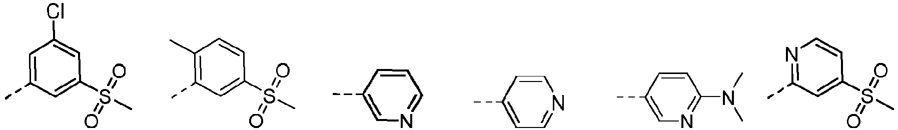 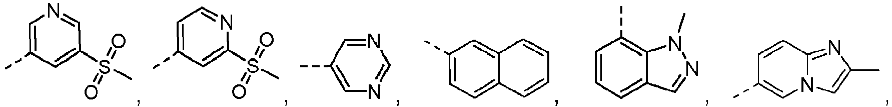 or 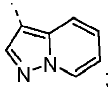
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵** , **R⁶** and **R⁷** represents independently of each other -H, -F, -Cl, -Br, -CN, , -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, 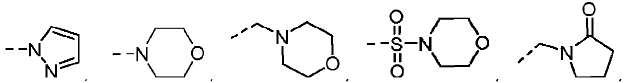
or **R⁵** and **R⁶**, or **R⁶** and **R⁷** form 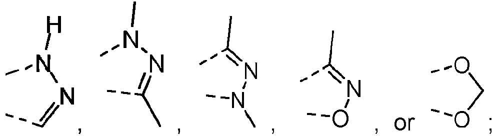
preferably,**R⁵** and **R⁶,** or **R⁶** and **R⁷** form 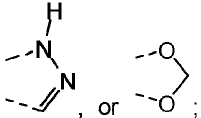 with the proviso that when **X** is -H, at least one of **R¹** - **R³** is not -H; when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded;

Still more preferably, is the compound of any one of the formulae **(II-1)** to **(II-6)**, wherein
**X** represents -H or -CN,
**R¹** represents -H, -CH₃, -Cl, -Ph, 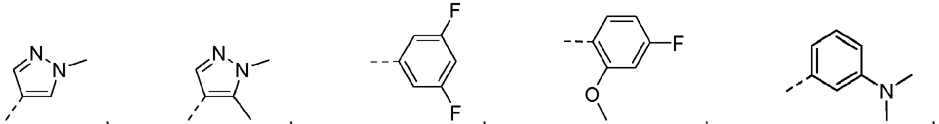 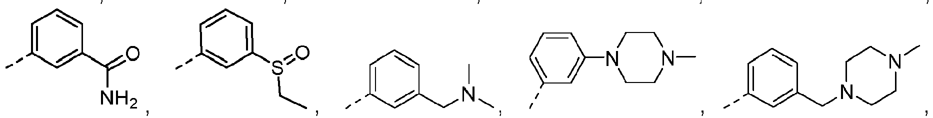 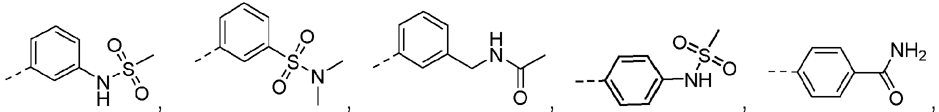 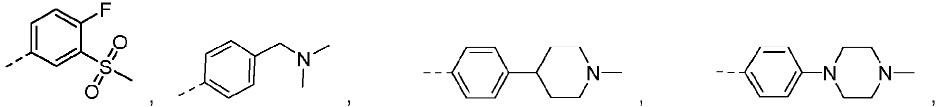 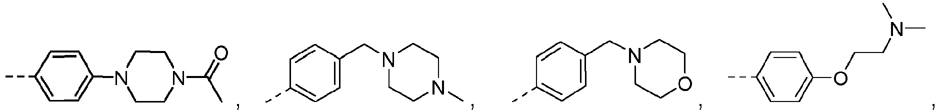 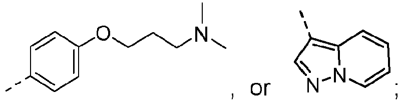
**R²** represents -H, -CH₃, -CI, -Br, -CF₃, -CN, -OH, -Ph, -NH-Ph, -O-CH(CH₃)₂, -O-CH₂CH(CH₃)₂, -O-CH(CH₃)(CH₂ CH₃), -O-CH₂CH₂-N(CH₃)₂, 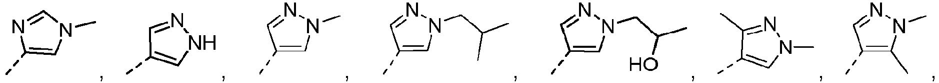 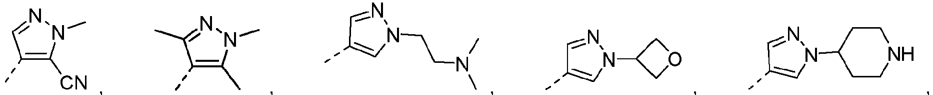 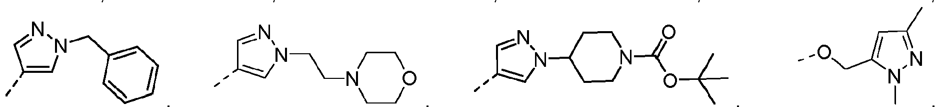 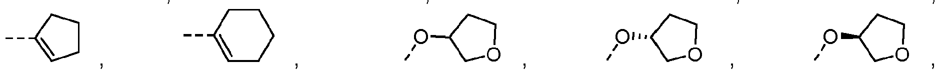 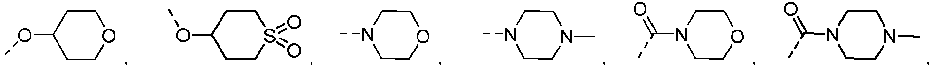 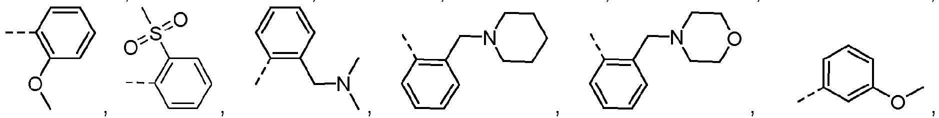 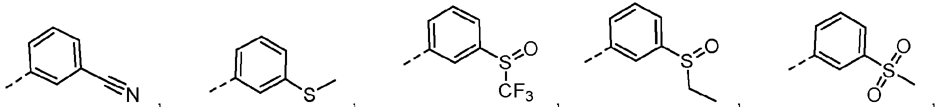 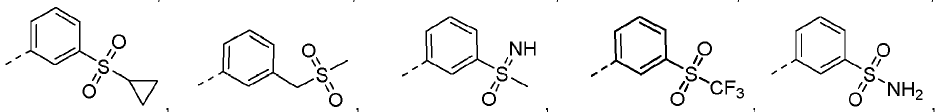 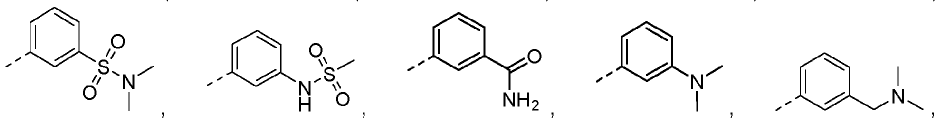 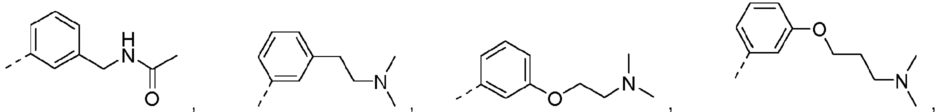 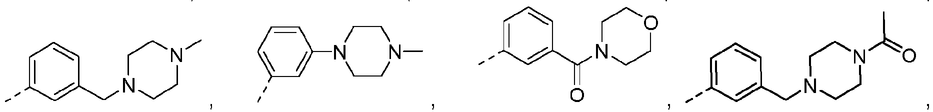 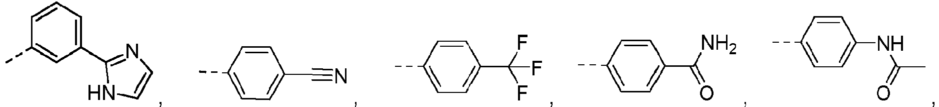 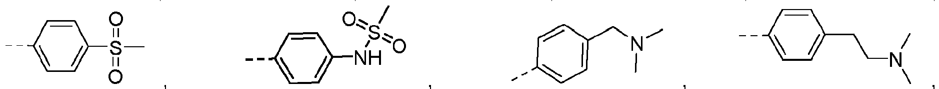 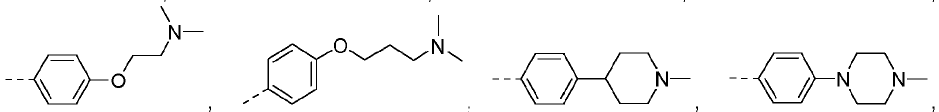 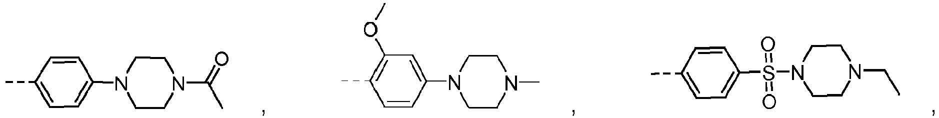 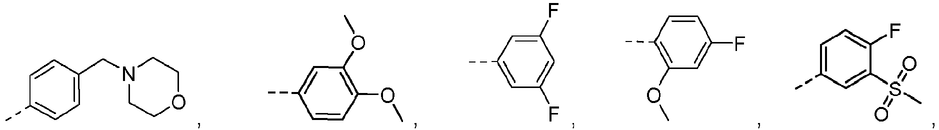 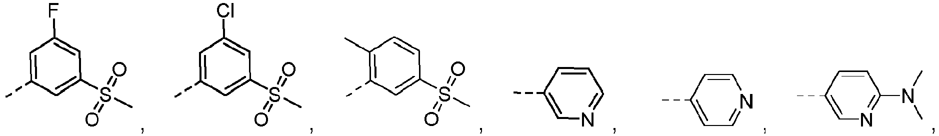 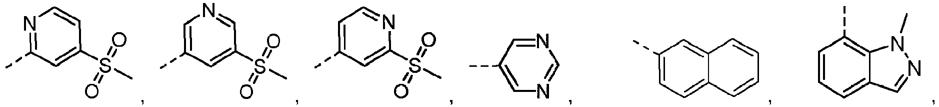 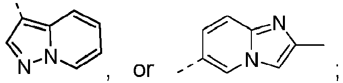
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁵** , **R⁶** and **R⁷** represents independently of each other -H, -F, -Cl, -Br, -CN, , -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, 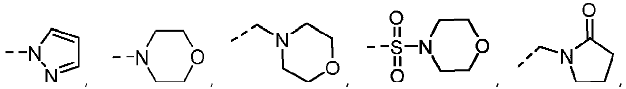
or **R⁵** and **R⁶,** or **R⁶** and **R⁷** form 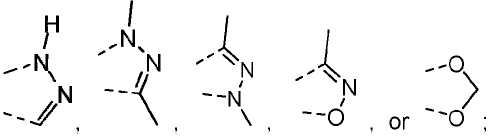
preferably, **R⁵** and **R⁶,** or **R⁶** and **R⁷** form 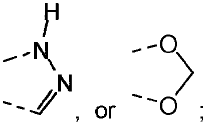 with the proviso that when X is -H, at least one of **R¹** - **R³** is not -H; when **R⁴** is -H, at least one of **R¹** - **R³** and **R⁵** - **R⁷** is not -H, and (E)-N-(1,3-benzodioxol-5-yl-methyl)-2-cyano-3-(1H-pyrrolo[2,3-b]pyridin-3-yl)acrylamide is excluded;

In some embodiments, the present invention relates to the compound having any one of the formulae (**III-1**) - (**III-6**): 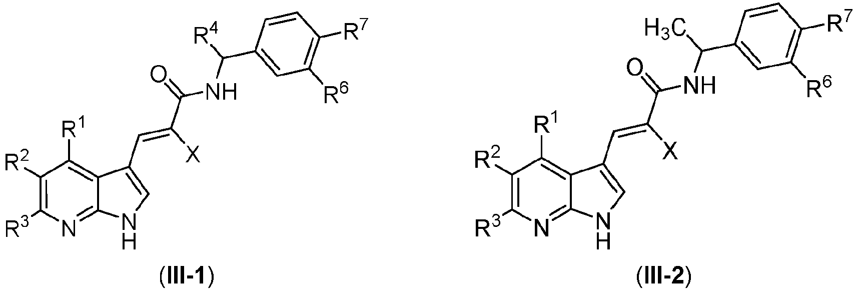 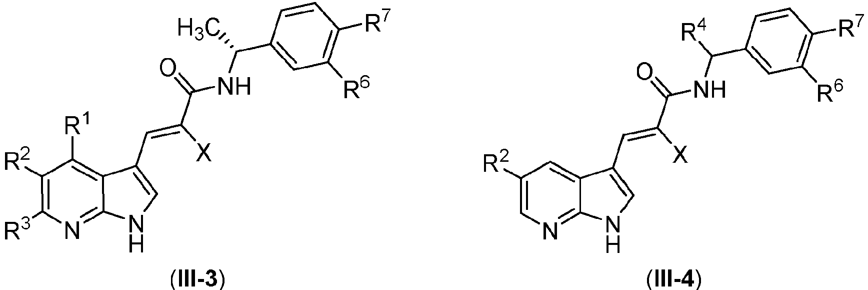 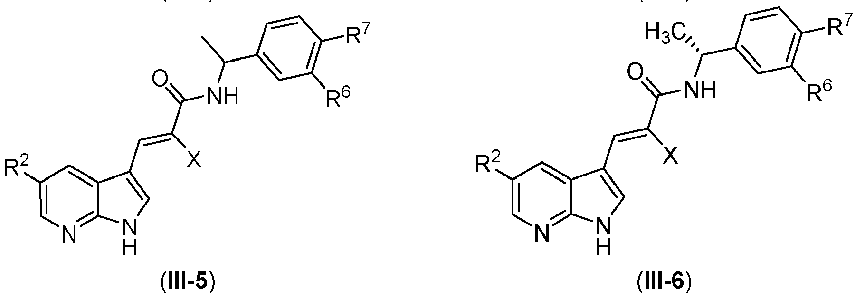 wherein **R¹, R², R³, R⁴, R⁶, R⁷** and **X** have the meanings as defined in the formula (I).

In formulae (III-4), (III-5) and (III-6) R² is preferably not -H.

In formulae (III-2) and (III-3) when X is -H at least one of R¹ - R³ is not -H and preferably at least one of **R⁶** and **R⁷** is not -H; or more preferably in case **X** is -CN at least one of **R⁶** and **R⁷** is not -H.

In formula (III-1) when X is -H at least one of R¹ - R³ is not -H and preferably at least one of **R⁶** and **R⁷** is not -H; or more preferably in case **X** is -CN or **R⁴** is -H or -C₂H₅ or -CH₂OH or -C₂H₄OH, at least one of **R⁶** and **R⁷** is not -H; or still more preferably in case **X** is -CN and **R⁴** is -H or -C₂H₅ or -CH₂OH or -C₂H₄OH, at least one of **R⁶** and **R⁷** is not -H.

Preferably, is the compound of any one of the formulae (**III-1**) to (**III-6**), wherein
**X** represents -H or -CN,
**R¹** represents -H, -CH₃, -CI, -Ph, 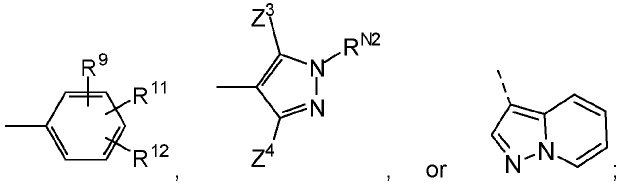
**R²** represents -H, -CH₃, -Ph, -CI, -Br, -CF₃, -CN, -OH, -NH-Ph, -O-CH₂CH₂-N(CH₃)₂, **-R⁹, -**O-**R¹⁰,** 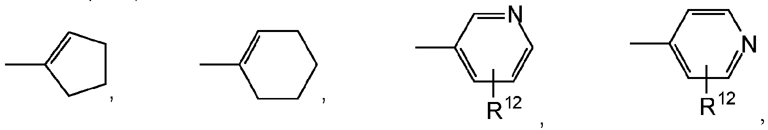 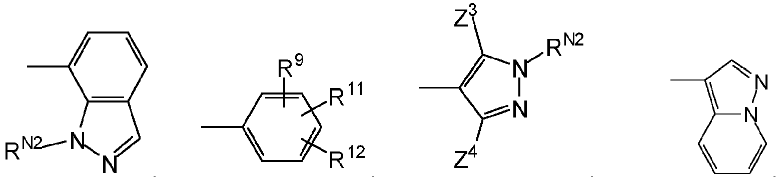 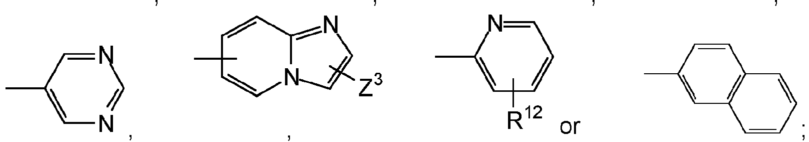
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁶** and **R⁷** represents independently of each other **-R⁸,** -CH₂-**R⁸**, -SO₂-**R⁸**, -H, -F, -Cl, -Br, -CN, -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃,
or **R⁶** and **R⁷** form 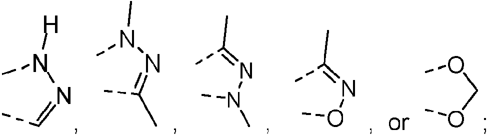
preferably, **R⁶** and **R⁷** form 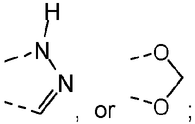
**R⁸** represents 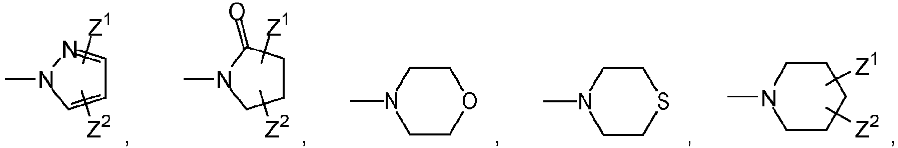 or 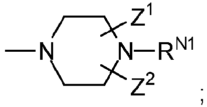 preferably 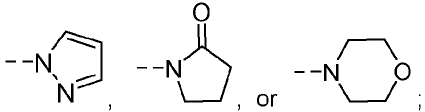
**R⁹** represents -H, 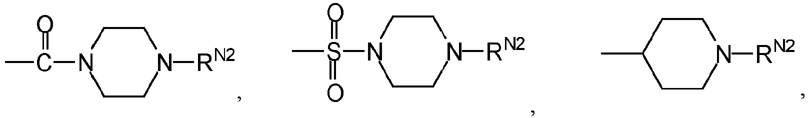 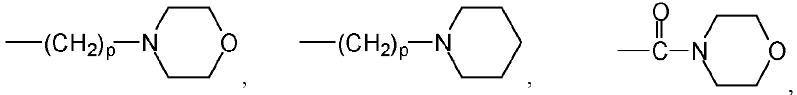 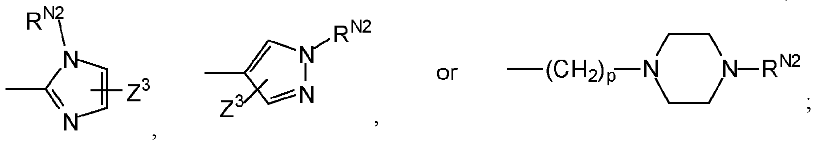
**R¹⁰** represents -CH(CH₃)₂, -CH₂-CH(CH₃)₂, -CH(CH₃)-C₂H₅, -(CH₂)ₙ-N(CH₃)₂, 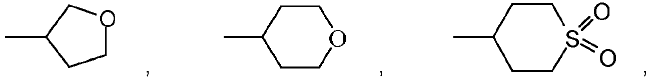 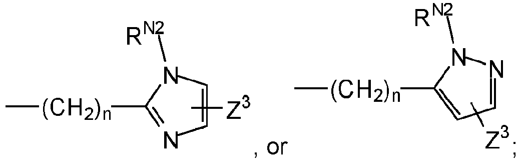
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
Preferably, **R¹¹** and **R¹²** represent independently of each other -H, -CH₃, -C₂H₅, -C₃H₇, -CH(CH₃)₂, -CF₃, -(CH₂)ₘ-N(CH₃)₂, -(CH₂)ₘ-N(C₂H₅)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -O-(CH₂)ₘ-N(C₂H₅)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
More preferably, **R¹¹** and **R¹²** represent independently of each other -H, -CH₃, -C₂H₅, -CF₃, -(CH₂)ₘ-N(CH₃)₂, -OCH₃, -OC₂H₅, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -NHCOCH₃, -(CH₂)ₘ-NHCOCH₃, -(CH₂)ₘ-NHCO-CH=CH₂, -SO₂NH₂, -SO₂NHCH₃, -SO₂N(CH₃)₂, -SO₂N(C₂H₅)₂, -SO₂N(C₃H₇)₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N1}** and **R^{N2}** represents independently of each other -H, -CH₃, -C₂H₅, -C₃H₇, -CH(CH₃)₂, cyclo-C₃H₅, cyclo-C₄H₇, -CH₂-CH(CH₃)₂, -Ph, -CH₂-Ph, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 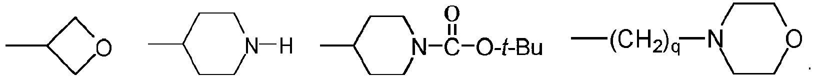
**Z¹, Z², Z³** and **Z⁴** represent independently of each other -H, -CH₃, -C₂H₅, -C₃H₇, -CH(CH₃)₂, -F, -CI, -Br, -I, -CN, -CF₃;
m is an integer selected from 0, 1, 2, or 3;
n is an integer selected from 1, or 2;
p is an integer selected from 0, or 1;
q is an integer selected from 1, or 2;
with the proviso that when **X** is -H, at least one of **R¹** - **R³** is not -H; when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded.

More preferably, is the compound of any one of the formulae (**III-1**) to (**III-6**), wherein
**X** represents -H or -CN;
**R¹** and **R²** represents independently of each other -H, -CH₃, -Ph, -CI, -Br, -CF₃, -CN, -OH, -NH-Ph, -O-CH(CH₃)₂, -O-CH₂CH(CH₃)₂, -O-CH(CH₃)(CH₂ CH₃), -O-CH₂CH₂-N(CH₃)₂, 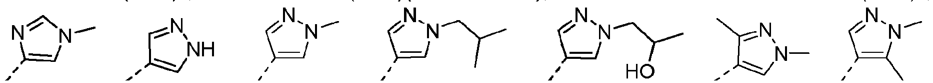 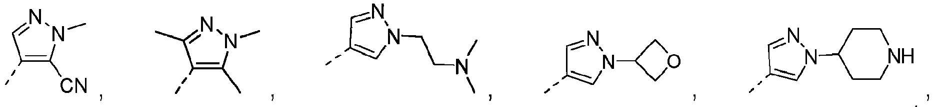 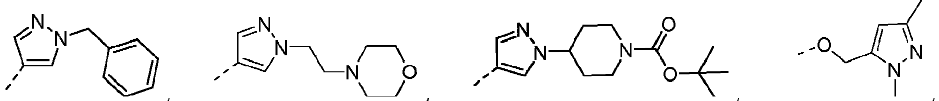 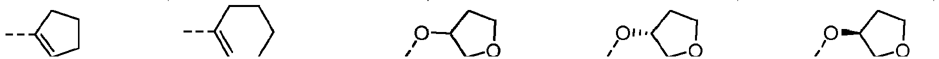 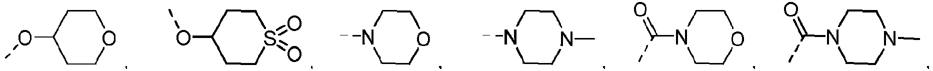 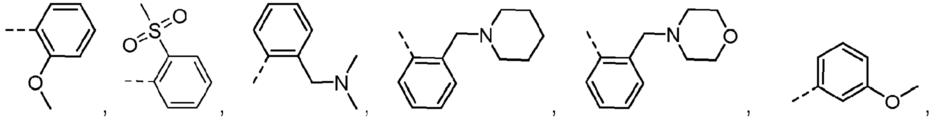 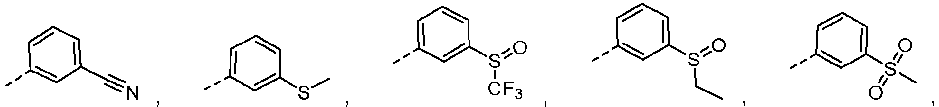 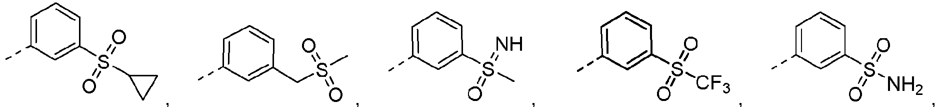 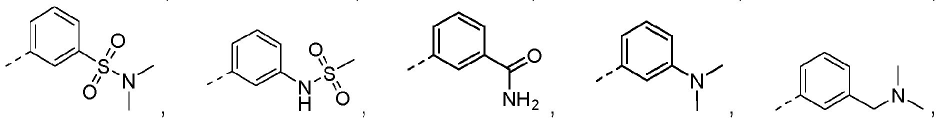 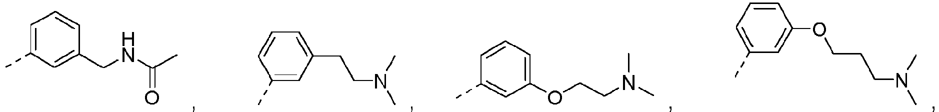 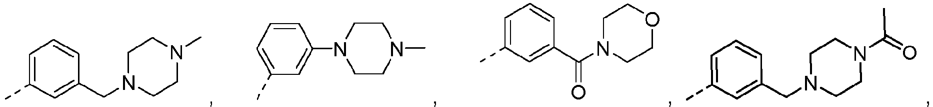 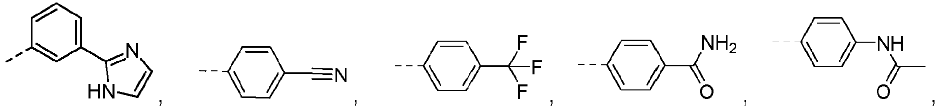 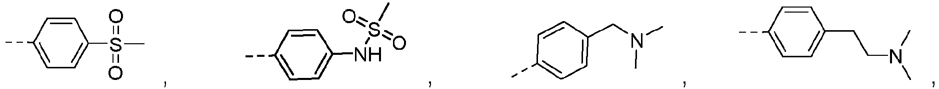 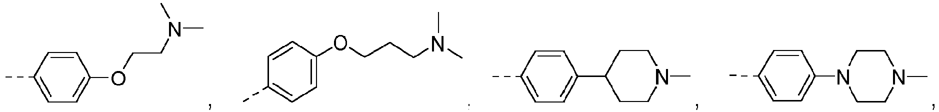 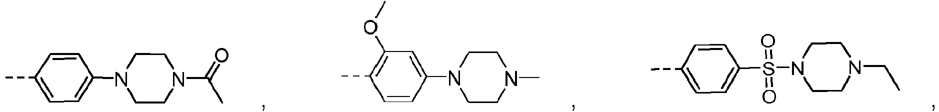 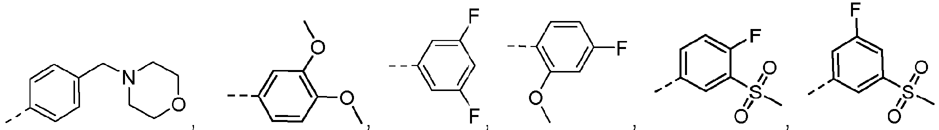 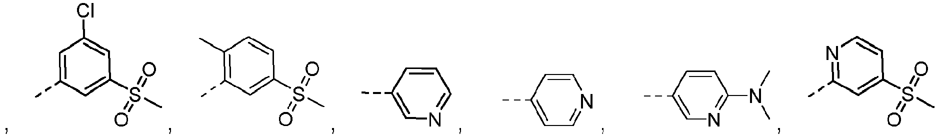 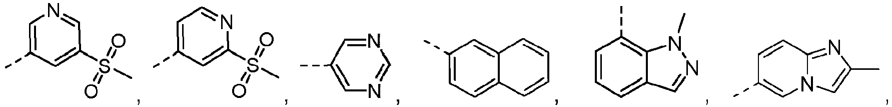 or 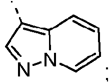
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁶** and **R⁷** represents independently of each other -H, -F, -Cl, -Br, -CN, , -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, 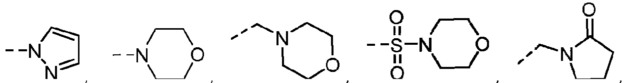
or **R⁶** and **R**⁷ form 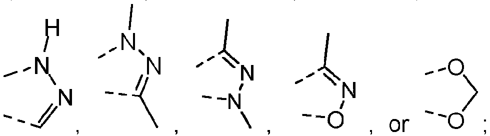
preferably, **R⁶** and **R⁷** form 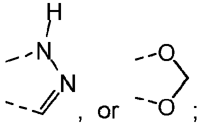 with the proviso that when **X** is -H, at least one of **R¹** - **R³** is not -H; when **R⁴** is -H, at least one of **R¹ - R**³ and **R⁵ - R⁷** is not -H; and (E)-N-(1,3-benzodioxol-5-yl-methyl)-2-cyano-3-(1H-pyrrolo[2,3-b]pyridin-3-yl)acrylamide is excluded.

Still more preferably, is the compound of any one of the formulae (**III**-**1**) to (**III-6**), wherein
**X** represents -H or -CN,
**R¹** represents -H, -CH₃, -CI, -Ph, 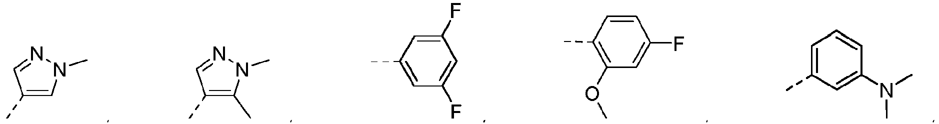 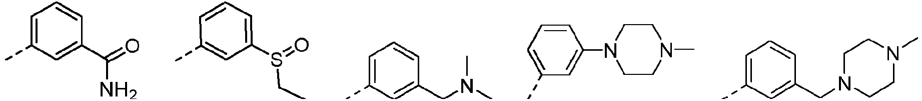 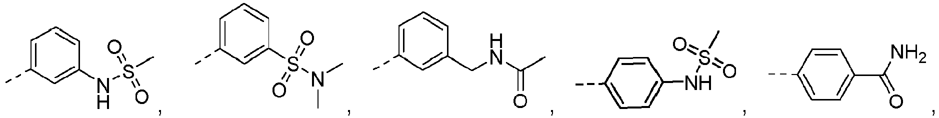 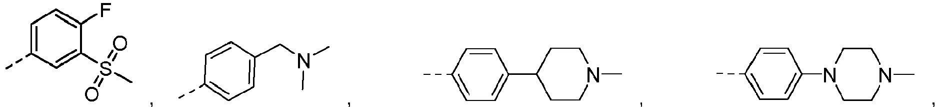 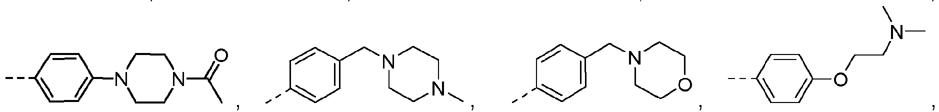 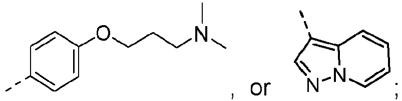
**R²** represents -H, -CH₃, -Cl, -Br, -CF₃, -CN, -OH, -Ph, -NH-Ph, -O-CH(CH₃)₂, -O-CH₂CH(CH₃)₂, -O-CH(CH₃)(CH₂ CH₃), -O-CH₂CH₂-N(CH₃)₂, 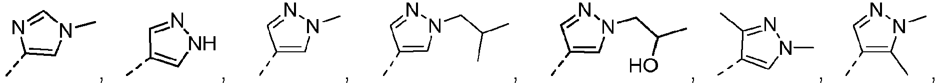 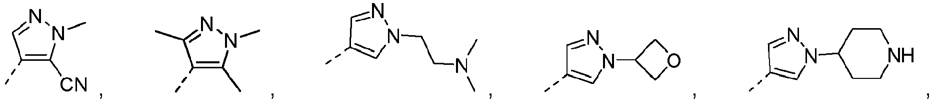 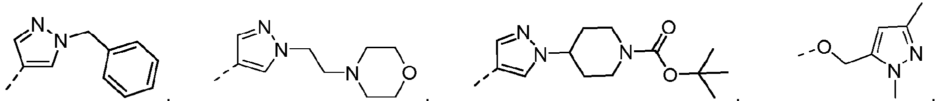 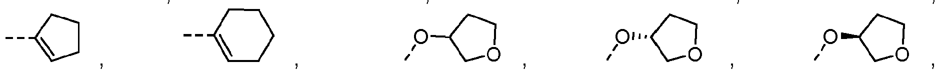 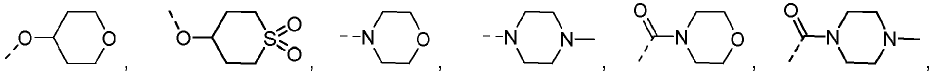 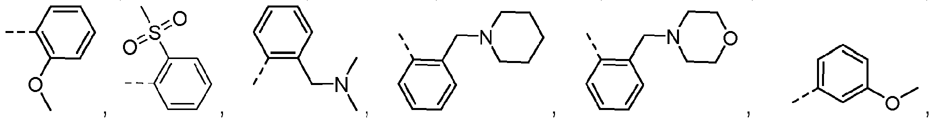 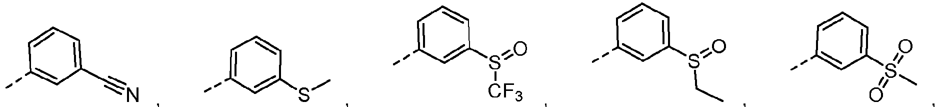 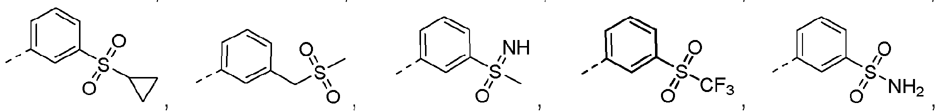 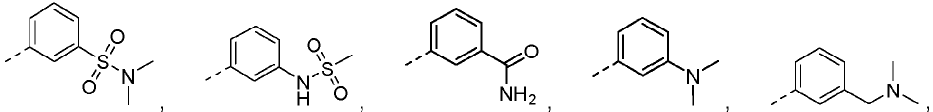 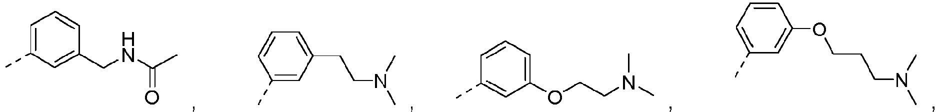 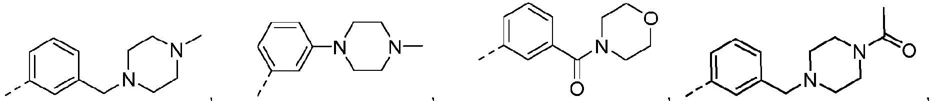 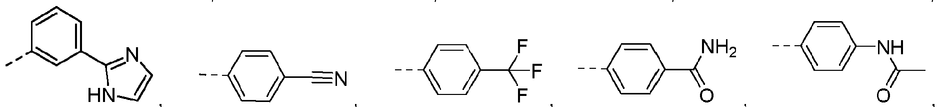 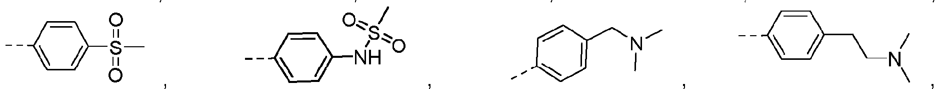 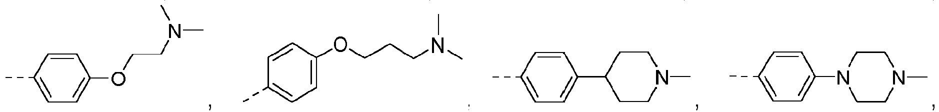 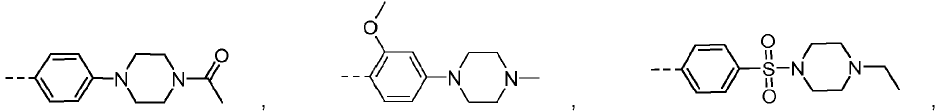 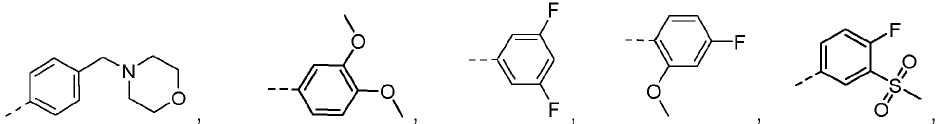 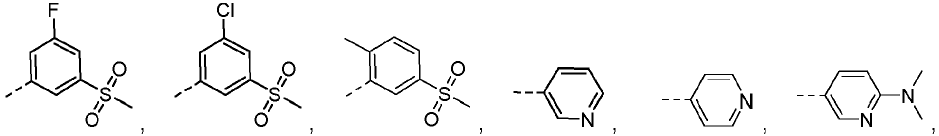 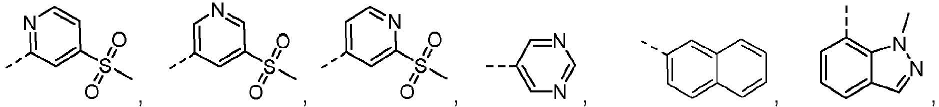 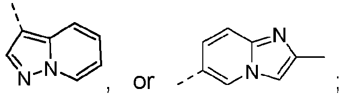
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁶** and **R⁷** represents independently of each other -H, -F, -Cl, -Br, -CN, , -CH₃, -CF₃, -OCH₃ , -COCH₃, -CONH₂, -NHCOCH₃, , -N(CH₃)₂, -SO₂CH₃, -SO₂NH₂, -SO₂NHCH₃, -CH₂-SO₂NH₂, , -NH-SO₂-CH₃, 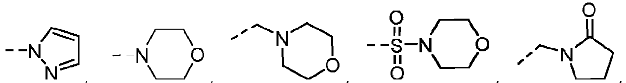
or **R⁶** and **R**⁷ form 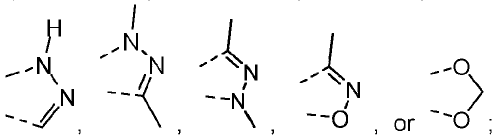
preferably, **R⁶** and **R⁷** form 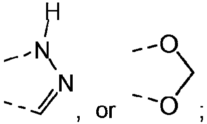 with the proviso that when **X** is -H, at least one of **R¹** - **R³** is not -H; when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded.

In some embodiments, the present invention relates to the compound having any one of the formulae (**IV-1**) - (**IV-6**) and (**V-1**) - (**V-6**): 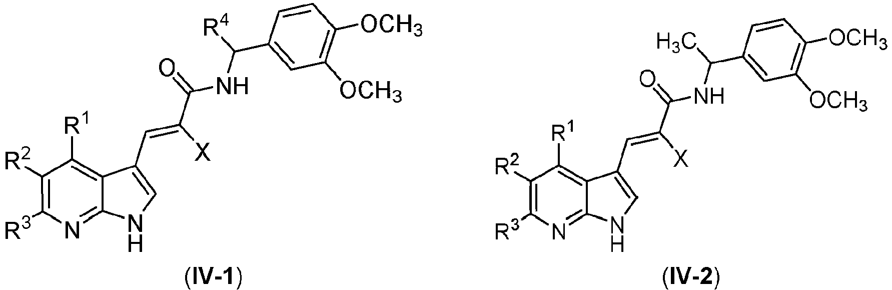 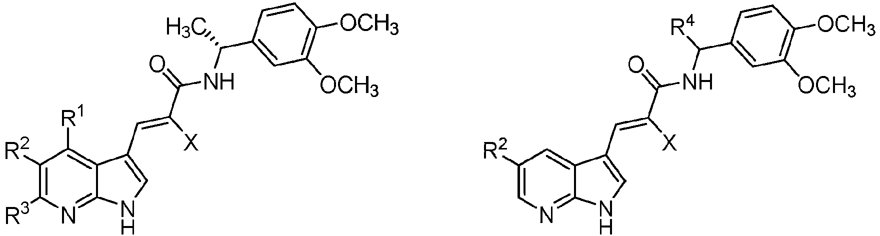 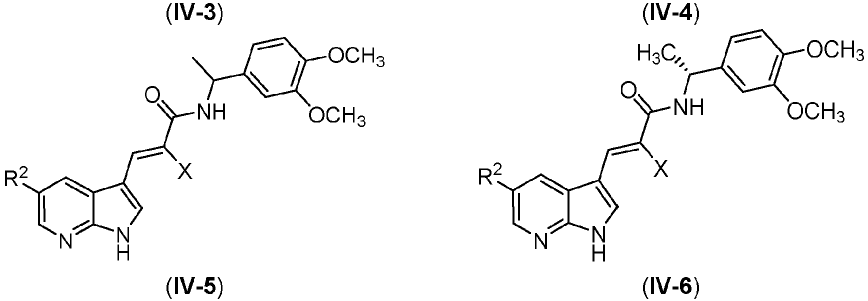 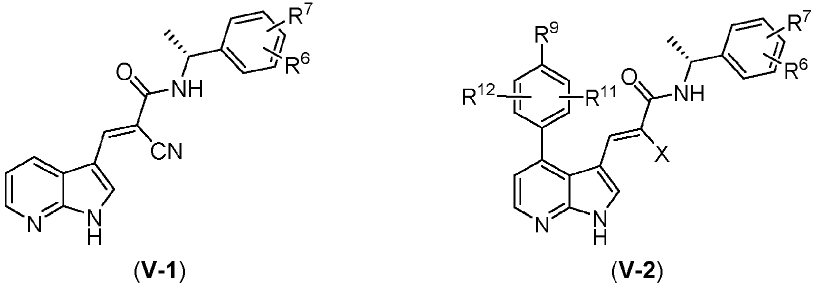 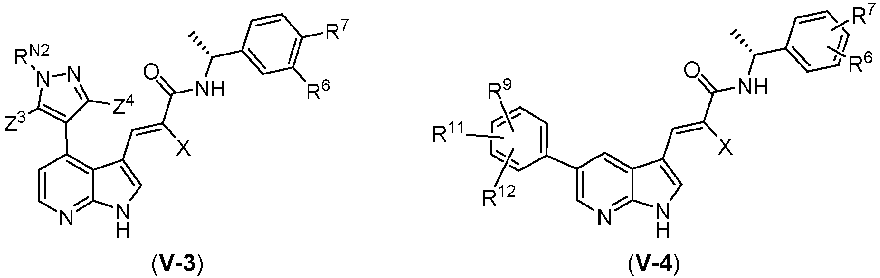 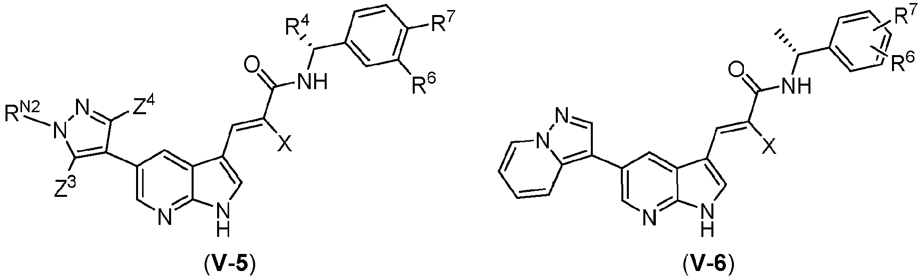 wherein **R¹, R², R³, R⁴, R⁶, R⁷, R⁹, R¹¹, R¹², R^{N2}, X, Z³,** and **Z⁴** have the meanings as defined in the formula (I).

In formulae (IV-4), (IV-5) and (IV-6) R² is preferably not -H.

In formula (V-1) one of R⁶ and R⁷ is preferably not -H.

Preferably, is the compound of any one of the formulae (**IV-1**) to (**IV-6**), 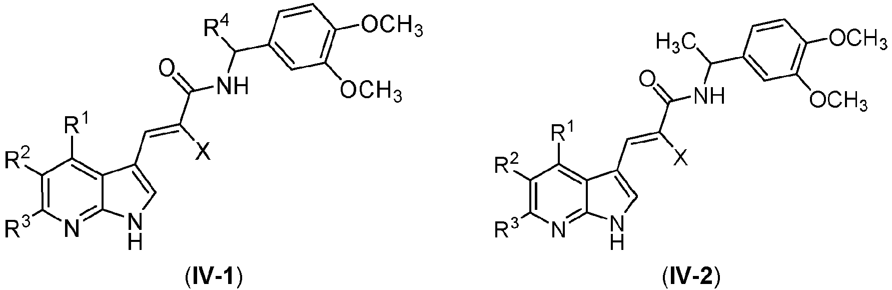 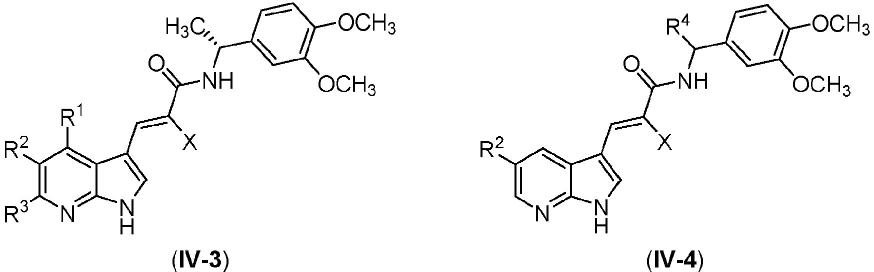 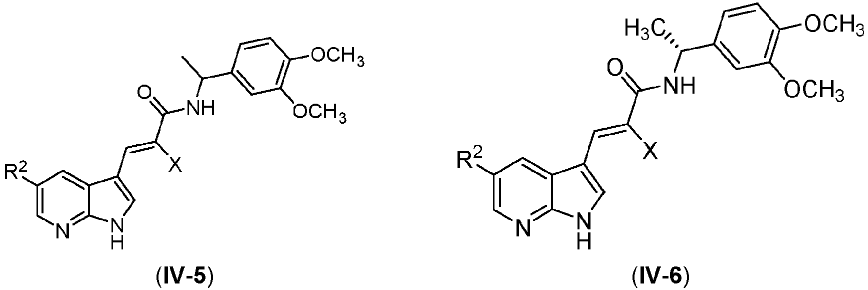 wherein
**X** represents -H or -CN,
**R¹** represents -H, -CH₃, -Cl, -Ph, 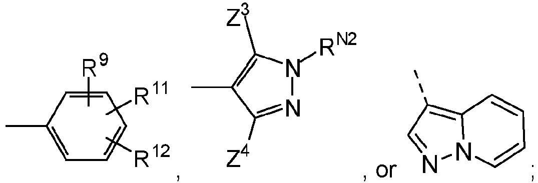
**R²** represents -H, -CH₃, -Ph, -CI, -Br, -CF₃, -CN, -OH, -NH-Ph, -O-CH₂CH₂-N(CH₃)₂, **-R⁹,** -O-**R¹⁰**, 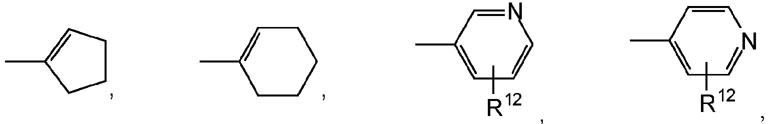 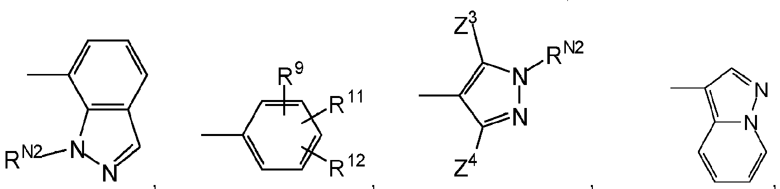 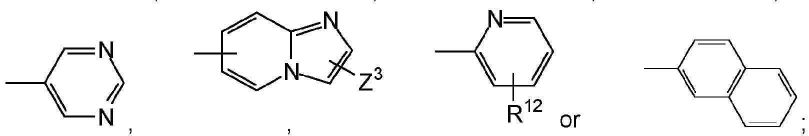
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁹** represents -H, 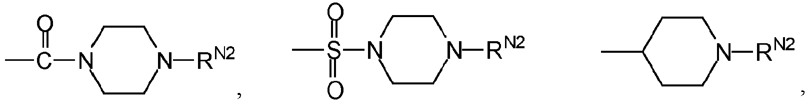 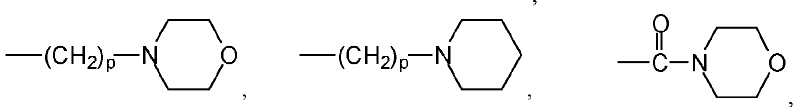 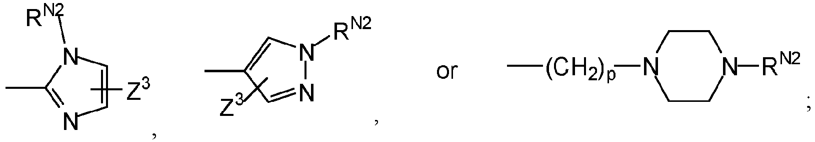
**R¹⁰** represents -CH(CH₃)₂, -CH₂-CH(CH₃)₂, -CH(CH₃)-C₂H₅, -(CH₂)ₙ-N(CH₃)₂, 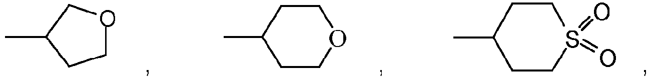 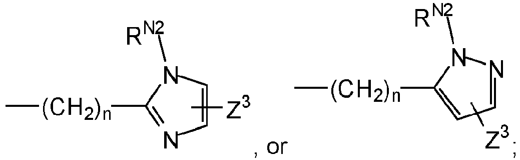
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂),-N(C₃H₇)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂ , -SCH₃, -SOCF₃, -SO₂CF₃, or -SO(NH)CH₃;
Preferably, **R¹¹** and **R¹²** represent independently of each other -H, -CH₃, -C₂H₅, -C₃H₇, -CH(CH₃)₂, -CF₃, -(CH₂)ₘ-N(CH₃)₂, -(CH₂)ₘ-N(C₂H₅)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -O-(CH₂)ₘ N(C₂H₅)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, - SCH₃, -SOCF₃, -SO₂CF₃, or -SO(NH)CH₃;
More preferably, **R¹¹** and **R¹²** represent independently of each other -H, -CH₃, -C₂H₅, -CF₃, -(CH₂)ₘ-N(CH₃)₂, -OCH₃, -OC₂H₅, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -NHCOCH₃, -(CH₂)ₘ-NHCOCH₃, -(CH₂)ₘ-NHCO-CH=CH₂, -SO₂NH₂, -SO₂NHCH₃, -SO₂N(CH₃)₂, -SO₂N(C₂H₅)₂, -SO₂N(C₃H₇)₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂ , - SCH₃, -SOCF₃, -SO₂CF₃, or -SO(NH)CH₃;
**R^{N2}** represents -H, -CH₃, -C₂H₅, -C₃H₇, -CH(CH₃)₂, cyclo-C₃H₅, cyclo-C₄H₇, -CH₂-CH(CH₃)₂, -Ph, -CH₂-Ph, -COCH₃, -COC₂H₅, -COC₃H₇, -CO-cyclo-C₃H₃, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 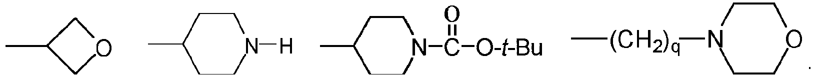
**Z³** and **Z⁴** represent independently of each other -H, -CH₃, -C₂H₅, -C₃H₇, -CH(CH₃)₂, -F, -Cl, -Br, -I, -CN, -CF₃;
m is an integer selected from 0, 1, 2, or 3;
n is an integer selected from 1, or 2;
p is an integer selected from 0, or 1;
q is an integer selected from 1, or 2;
with the proviso that when **X** is -H, at least one of **R¹** - **R³** is not -H; when **R⁴** is -H, at least one of **R¹** - **R³** and **R⁵ - R⁷** is not -H; and (E)-N-(1,3-benzodioxol-5-yl-methyl)-2-cyano-3-(1H-pyrrolo[2,3-b]pyridin-3-yl)acrylamide is excluded.

More preferably, is the compound of any one of the formulae (**IV-1**) to (**IV-6**), wherein
**X** represents -H or -CN;
**R¹** and **R²** represents independently of each other -H, -CH₃, -Ph, -CI, -Br, -CF₃, -CN, -OH, -NH-Ph, -O-CH(CH₃)₂, -O-CH₂CH(CH₃)₂, -O-CH(CH₃)(CH₂ CH₃), -O-CH₂CH₂-N(CH₃)₂, 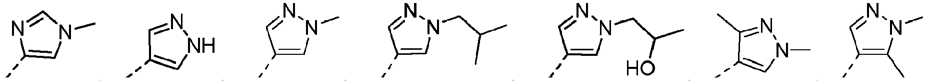 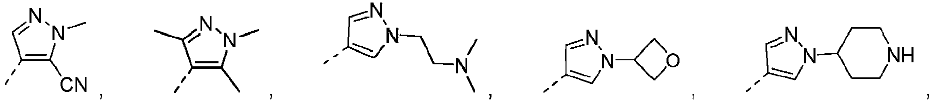 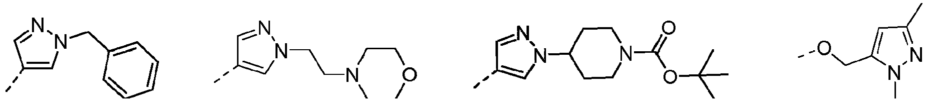 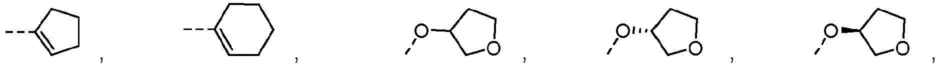 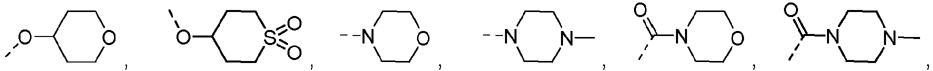 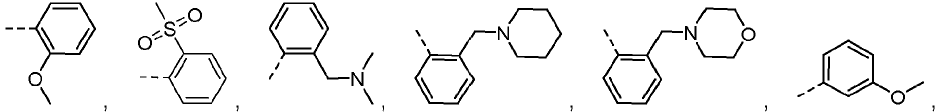 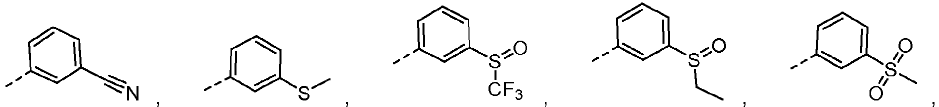 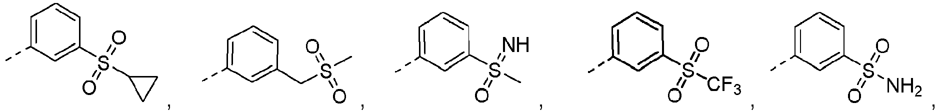 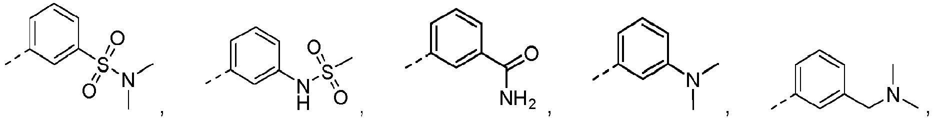 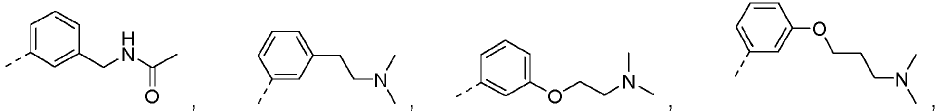 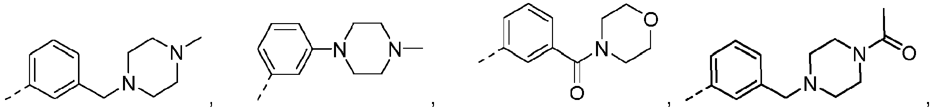 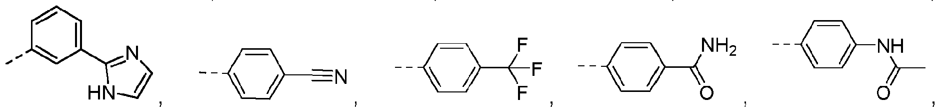 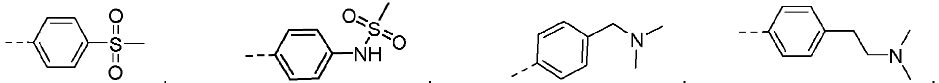 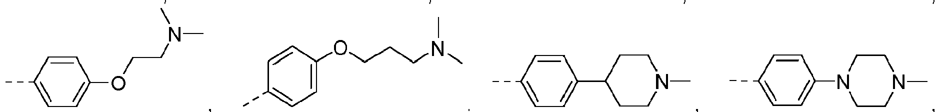 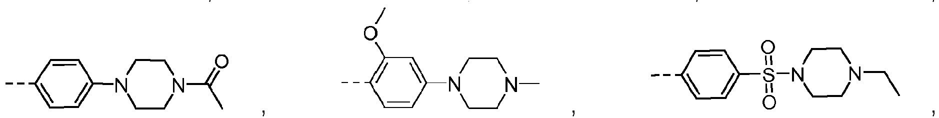 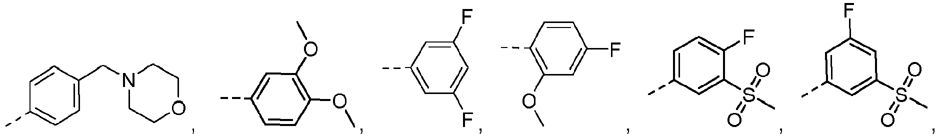 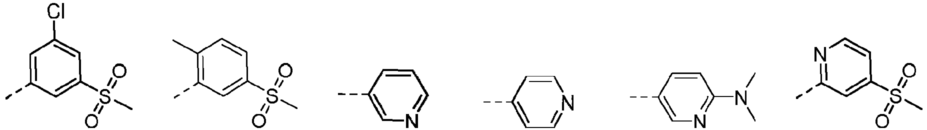 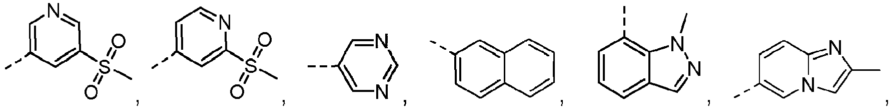 or 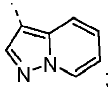
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
with the proviso that when **X** is -H, at least one of **R¹** - **R³** is not -H; when **R⁴** is -H, at least one of **R¹** - **R³** and **R⁵** - **R⁷** is not -H; and (E)-N-(1,3-benzodioxol-5-yl-methyl)-2-cyano-3-(1H-pyrrolo[2,3-b]pyridin-3-yl)acrylamide is excluded.

Still more preferably, is the compound of any one of the formulae (**IV-1**) to (**IV-6**), wherein
**X** represents -H or -CN,
**R¹** represents -H, -CH₃, -CI, -Ph, 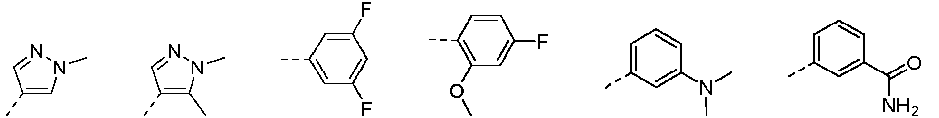 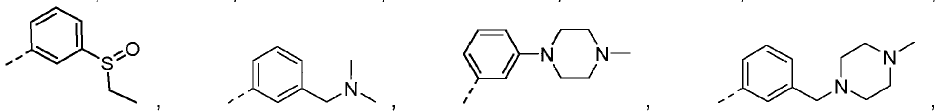 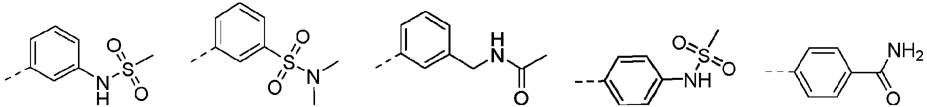 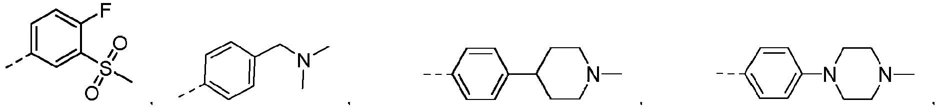 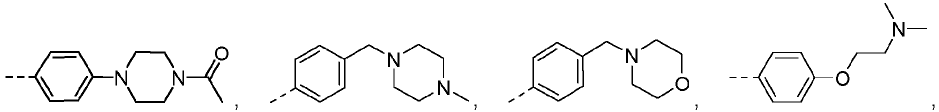 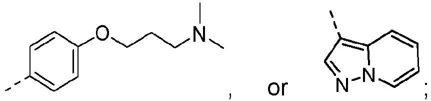
**R²** represents -H, -CH₃, -Cl, -Br, -CF₃, -CN, -OH, -Ph, -NH-Ph, -O-CH(CH₃)₂, -O-CH₂CH(CH₃)₂, -O-CH(CH₃)(CH₂ CH₃), -O-CH₂CH₂-N(CH₃)₂, 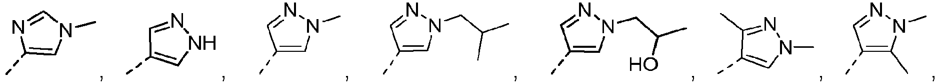 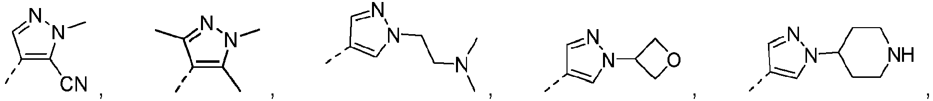 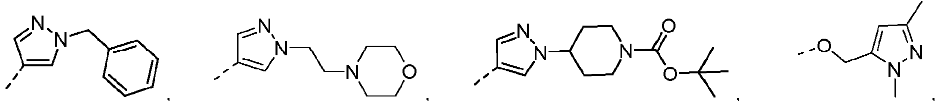 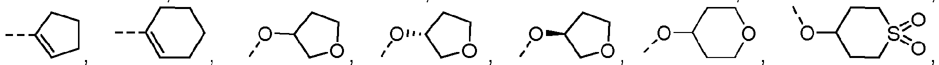 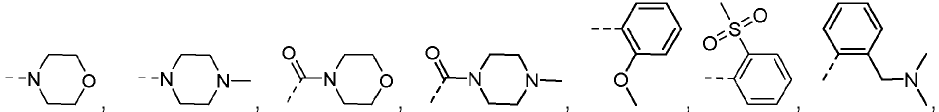 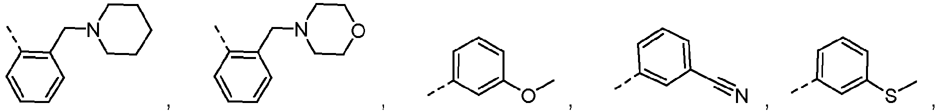 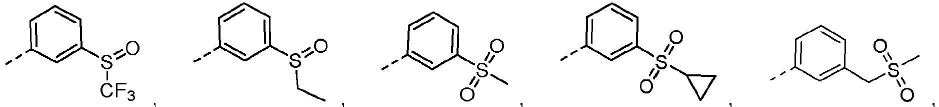 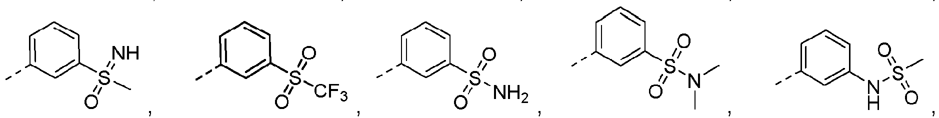 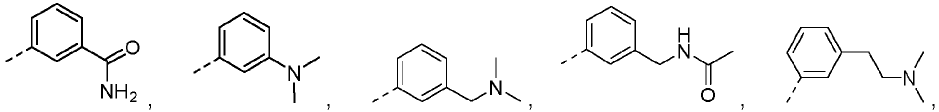 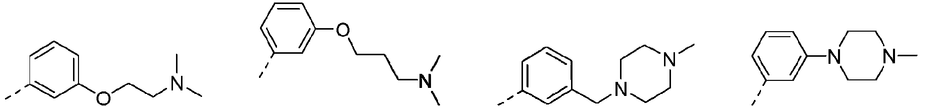 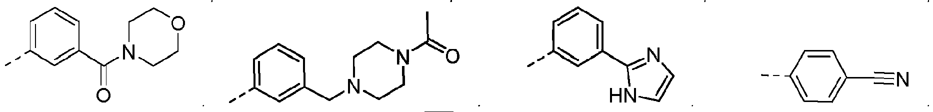 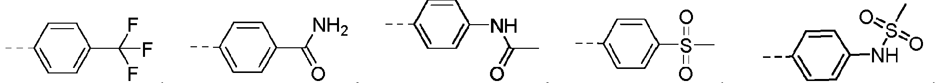 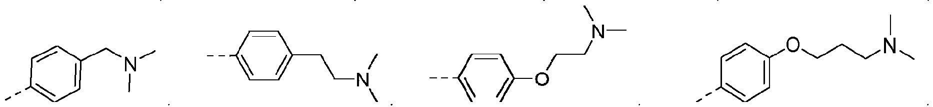 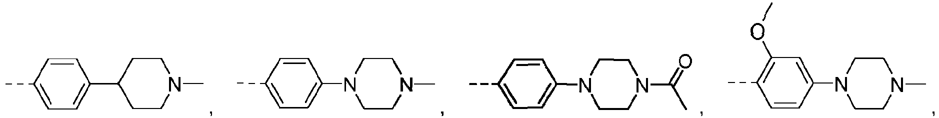 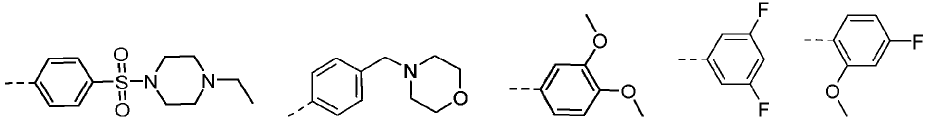 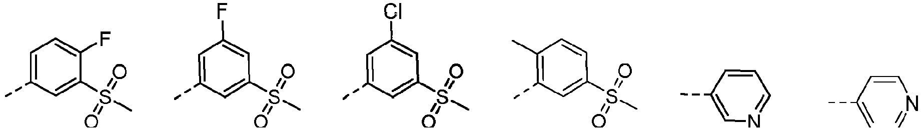 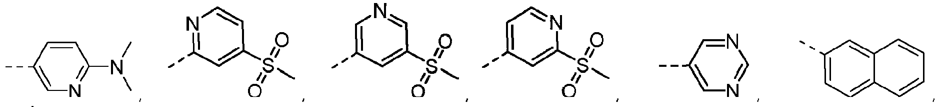 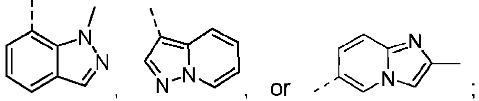
**R³** represents -H, -CH₃, or -Br;
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
with the proviso that when **X** is -H, at least one of **R¹ - R³** is not -H; when **R⁴** is -H, at least one of **R¹ - R³** and **R⁵ - R⁷** is not -H; and (E)-N-(1,3-benzodioxol-5-yl-methyl)-2-cyano-3-(1H-pyrrolo[2,3-b]pyridin-3-yl)acrylamide is excluded.

Preferred, is the compound of any one of the formulae (**V-1**) to (**V-6**): 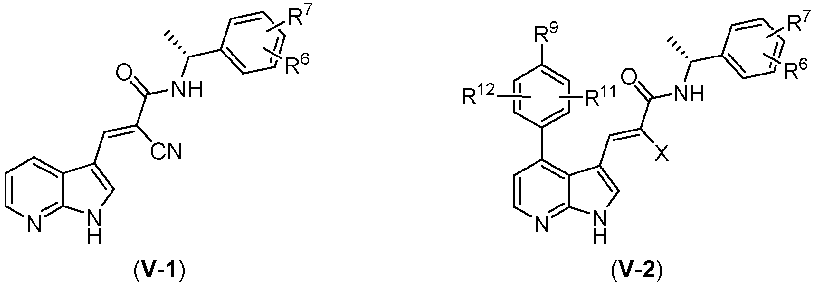 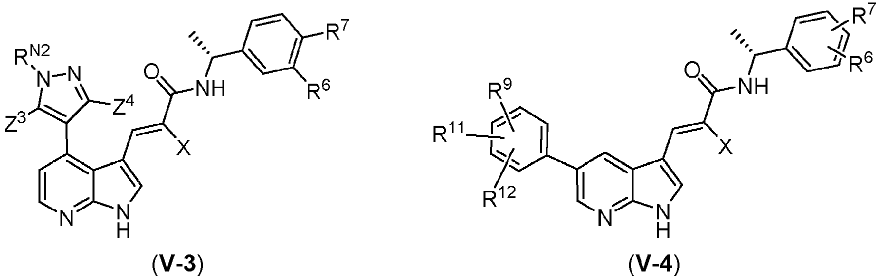 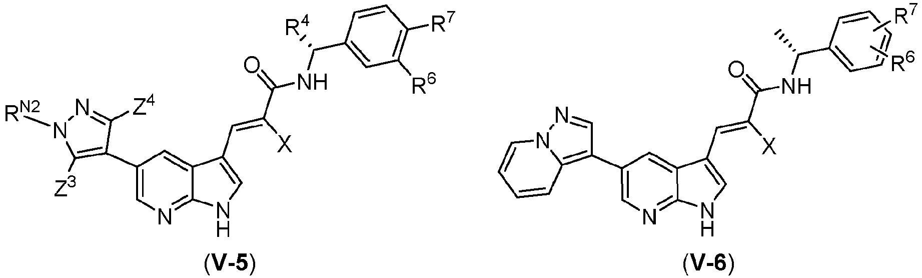 wherein
**X** represents -H or -CN,
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂;
**R⁶** and **R⁷** represents independently of each other -H, -F, -CI, -Br, -CN, -CH₃, -CF₃, -OCH₃, -COCH₃, -CONH₂, -NHCOCH₃, -N(CH₃)₂, -SO₂CH₃, -SO₂NH₂, -SO₂NHCH₃, -CH₂-SO₂NH₂, -NH-SO₂-CH₃, 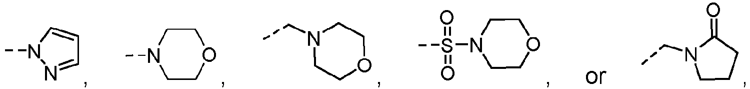
or **R⁶** and **R⁷** form 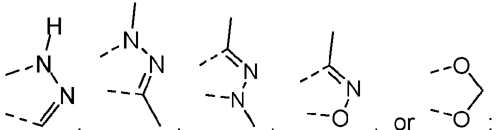
preferably, **R⁶** and **R⁷** form 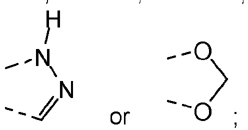
**R⁹** represents -H, 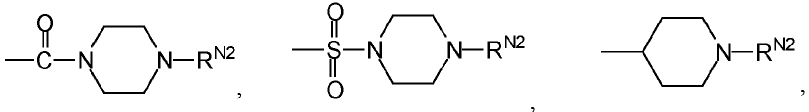 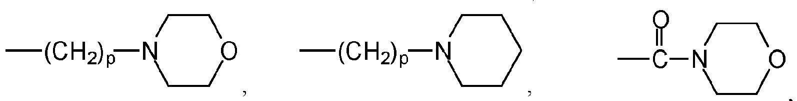 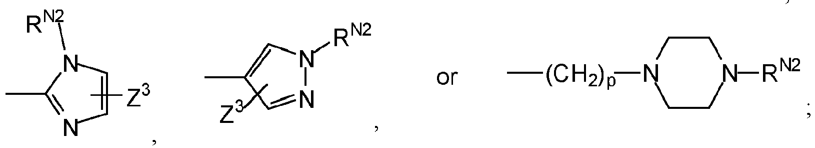
**R¹⁰** represents -CH(CH₃)₂, -CH₂-CH(CH₃)₂, -CH(CH₃)-C₂H₅, -(CH₂)ₙ-N(CH₃)_{2,} 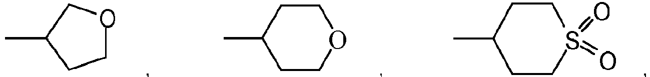 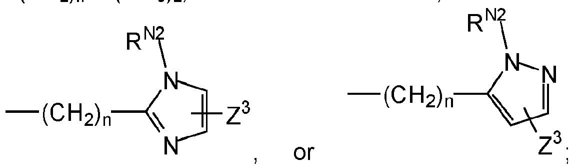
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C₂H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂ , -SCH₃, -SOCF₃, -SO₂CF₃, or -SO(NH)CH₃;
Preferably, **R¹¹** and **R¹²** represent independently of each other -H, -CH₃, -C₂H₅, -C₃H₇, -CH(CH₃)₂, -CF₃, -(CH₂)ₘ-N(CH₃)₂, -(CH₂)ₘ-N(C₂H₅)₂, -OCH₃, -OC₂H₅, -OC₃H₇, -O-cyclo-C₃H₅, -OCH(CH₃)₂, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -O-(CH₂)ₘ-N(C₂H₅)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N2}** represents -H, -CH₃, -C₂H₅, -C₃H₇, -CH(CH₃)₂, cyclo-C₃H₅, cyclo-C₄H₇, -CH₂-CH(CH₃)₂, -Ph, -CH₂-Ph, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 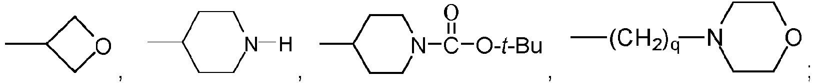
**Z³** and **Z⁴** represent independently of each other -H, -CH₃, -C₂H₅, -C₃H₇, -CH(CH₃)₂, -F, -Cl, -Br, -I, -CN, -CF₃;
m is an integer selected from 0, 1, 2, or 3;
n is an integer selected from 1, or 2;
p is an integer selected from 0, or 1;
q is an integer selected from 1, or 2.

More preferred, is the compound of any one of the formulae (**V-1**) to (**V-6**):
wherein
**X** represents -H or -CN,
**R⁴** represents -H, -CH₃, -CF₃, -CN, -CH₂OH, -CH₂CH₂OH, -CH₂N(CH₃)₂, -CH₂CH₂N(CH₃)₂, or -CH₂CH₂N(CH₂CH₃)₂,
**R⁶** and R⁷ represents independently of each other -H, -F, -Cl, -Br, -CN, , -CH₃, -CF₃, -OCH₃, -COCH₃, -CONH₂, -NHCOCH₃, -N(CH₃)₂, -SO₂CH₃, -SO₂NH₂, -SO₂NHCH₃, -CH₂-SO₂NH₂, , -NH-SO₂-CH₃, 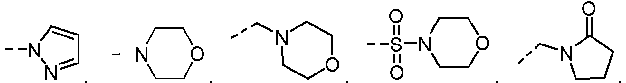
or **R⁶** and **R⁷** form 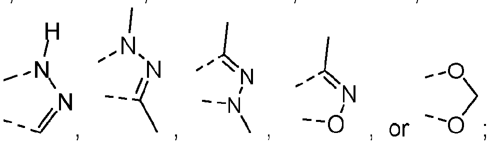
preferably, **R⁶** and **R⁷** form 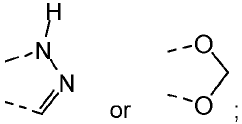
**R⁹** represents -H, 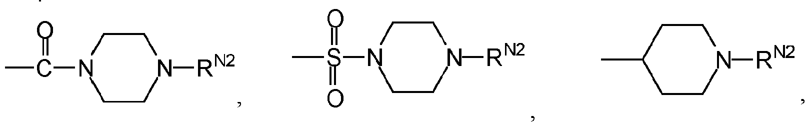 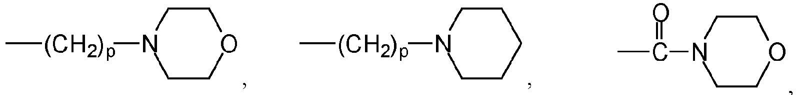 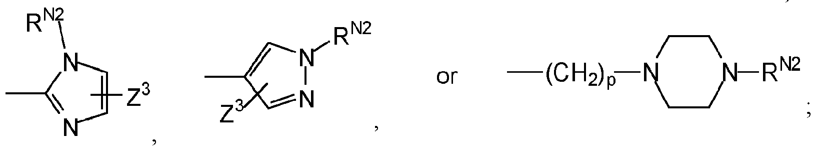
**R¹⁰** represents -CH(CH₃)₂, -CH₂-CH(CH₃)₂, -CH(CH₃)-C₂H₅, -(CH₂)ₙ-N(CH₃)₂, 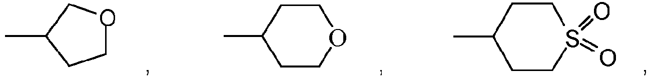 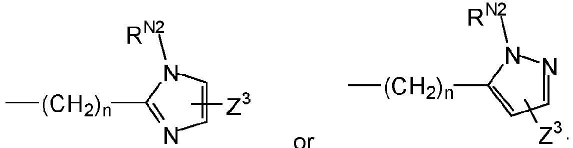
**R¹¹** and **R¹²** represent independently of each other -H, -CH₃, -C₂H₅, -CF₃, -(CH₂)ₘ-N(CH₃)₂, -OCH₃, -OC₂H₅, -F, -CI, -Br, -CN, -(CH₂)ₘ-SO₂CH₃, -(CH₂)ₘ-SO₂-cyclo-C₃H₅, -O-(CH₂)ₘ-N(CH₃)₂, -NHCOCH₃, -(CH₂)ₘ-NHCOCH₃, -(CH₂)ₘ-NHCO-CH=CH₂, -SO₂NH₂, -SO₂NHCH₃, -SO₂N(CH₃)₂, -SO₂N(C₂H₅)₂, -SO₂N(C₃H₇)₂, -NH-SO₂-CH₃, -NH-SO₂-C₂H₅, -NH-SO₂-C₃H₇, -NH-SO₂-cyclo-C₃Hs, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃;
**R^{N2}** represents -H, -CH₃, -C₂H₅, -CH₂-CH(CH₃)₂, -CH₂-Ph, -COCH₃, -COOCH₃, -COOC(CH₃)₃, -CH₂-CH(OH)-CH₃, -(CH₂)-N(CH₃)₂, -CH₂CH₂-N(CH₃)₂, 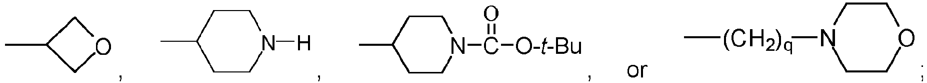
m is an integer selected from 0, 1, 2, or 3;
n is an integer selected from 1, or 2;
p is an integer selected from 0, or 1;
q is an integer selected from 1, or 2.

Preferably, the present invention relates to the compound having any one of the formulae (**VI-1**) - (**VI-6**): 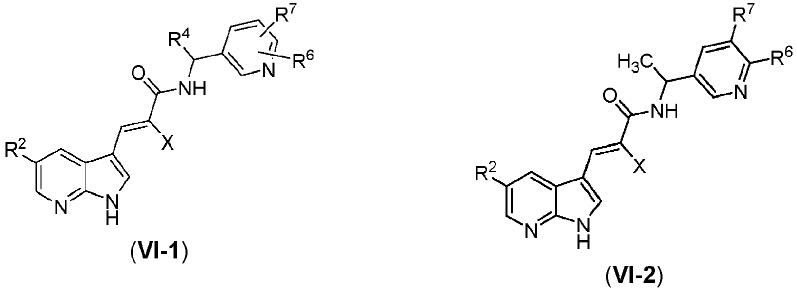 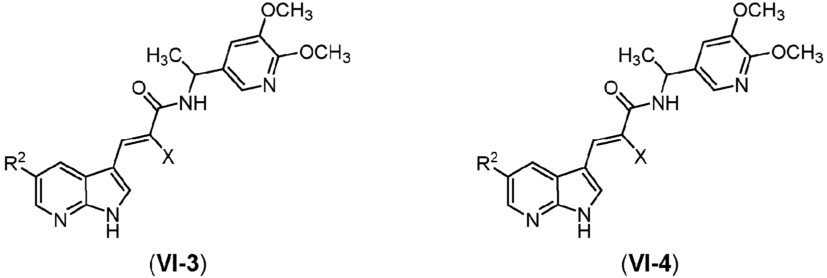 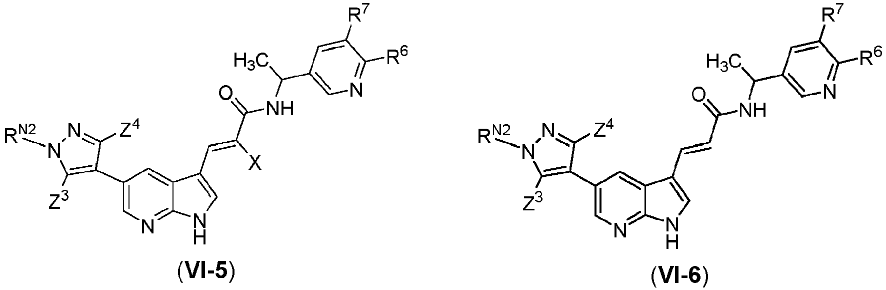 wherein **R², R⁴, R⁶, R⁷, R^{N2}**, **X, Z³,** and **Z⁴** have the meanings as defined in the formula (I).

Especially preferred compounds according to the present invention include compounds presented by Table 1.

**Table 1:**

| **Exam ple** | **Structure** | **Nomenclature** |
|---|---|---|
| **1** | 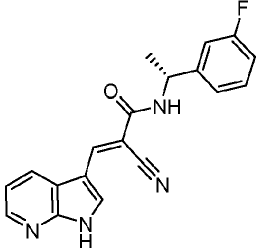 | (*R,E*)-2-cyano-*N*-(1-(3-fluorophenyl)ethyl)-3-(1*H*-pyrrolo[2,3-b]pyridin-3-yl)acrylamide |
| **6** | 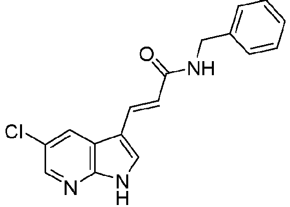 | (E)-N-benzyl-3-(5-chloro-1H-pyrrolo[2,3-b]pyridin-3-yl)acrylamide |
| **7** | 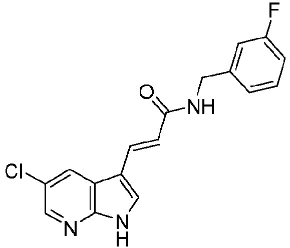 | (E)-3-(5-chloro-1H-pyrrolo[2,3-b]pyridin-3-yl)-N-(3-fluorobenzyl)acrylamide |
| **8** | 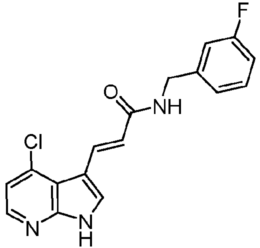 | (E)-3-(4-chloro-1H-pyrrolo[2,3-b]pyridin-3-yl)-N-(3-fluorobenzyl)acrylamide |
| **10** | 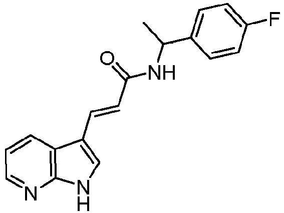 | (E)-N-(1-(4-fluorophenyl)ethyl)-3-(1H-pyrrolo[2,3-b]pyridin-3-yl)acrylamide |
| **11** | 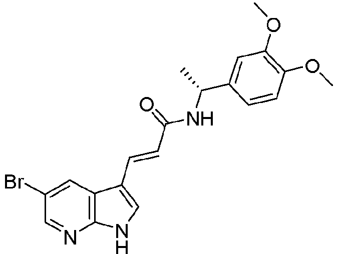 | (R,E)-3-(5-bromo-1H-pyrrolo[2,3-b]pyridin-3-yl)-N-(1-(3,4-dimethoxyphenyl)ethyl)acrylamide |
| **12** | 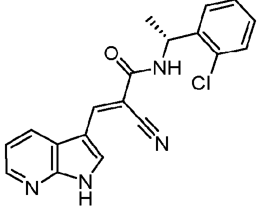 | (R,E)-N-(1-(2-chlorophenyl)ethyl)-2-cyano-3-(1H-pyrrolo[2,3-b]pyridin-3-yl)acrylamide |
| **13** | 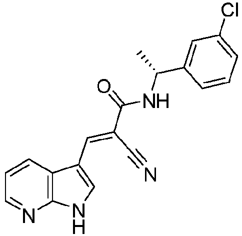 | (R,E)-N-(1-(3-chlorophenyl)ethyl)-2-cyano-3-(1H-pyrrolo[2,3-b]pyridin-3-yl)acrylamide |
| **14** | 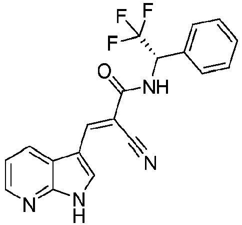 | (S,E)-2-cyano-3-(1H-pyrrolo[2,3-b]pyridin-3-yl)-N-(2,2,2-trifluoro-1-phenylethyl)acrylamide |
| **15** | 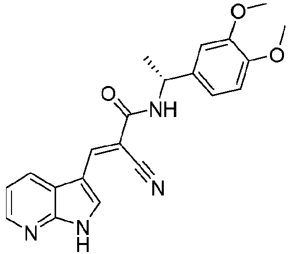 | (R,E)-2-cyano-N-(1-(3,4-dimethoxyphenyl) ethyl)-3-(1H-pyrrolo[2,3-b]pyridin-3-yl)acrylamide |
| **16** | 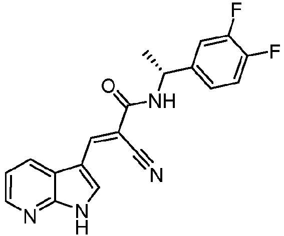 | (R,E)-2-cyano-N-(1-(3,4-difluorophenyl) ethyl)-3-(1H-pyrrolo[2,3-b]pyridin-3-yl)acrylamide |
| **17** | 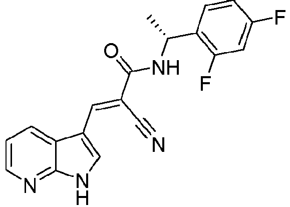 | (R,E)-2-cyano-N-(1-(2,4-difluorophenyl) ethyl)-3-(1H-pyrrolo[2,3-b]pyridin-3-yl)acrylamide |
| **18** | 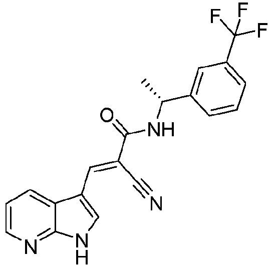 | (R,E)-2-cyano-3-(1H-pyrrolo[2,3-b]pyridin-3-yl)-N-(1-(3-(trifluoromethyl) phenyl)ethyl)acrylamide |
| **19** | 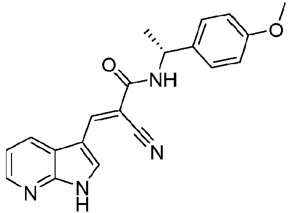 | (R,E)-2-cyano-N-(1-(4-methoxyphenyl) ethyl)-3-(1H-pyrrolo[2,3-b]pyridin-3-yl)acrylamide |
| **20** | 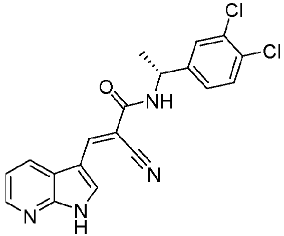 | (R,E)-2-cyano-N-(1-(3,4-dichlorophenyl) ethyl)-3-(1H-pyrrolo[2,3-b]pyridin-3-yl)acrylamide |
| **21** | 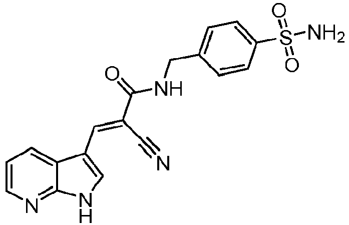 | (E)-2-cyano-3-(1H-pyrrolo[2,3-b]pyridin-3-yl)-N-(4-sulfamoylbenzyl)acrylamide |
| **22** | 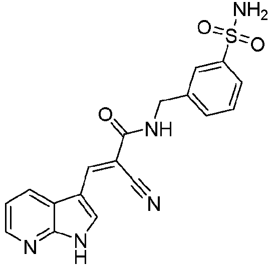 | (E)-2-cyano-3-(1H-pyrrolo[2,3-b]pyridin-3-yl)-N-(4-sulfamoylbenzyl)acrylamide |
| **23** | 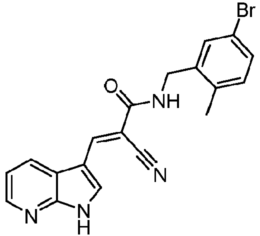 | (E)-N-(5-bromo-2-methylbenzyl)-2-cyano-3-(1 H-pyrrolo[2,3-b]pyridin-3-yl)acrylamide |
| **24** | 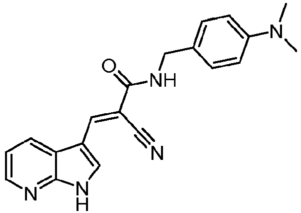 | (E)-2-cyano-N-(4-(dimethylamino)benzyl)-3-(1H-pyrrolo[2,3-b]pyridin-3-yl)acrylamide |
| **25** | 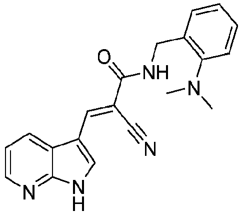 | (E)-2-cyano-N-(2-(dimethylamino)benzyl)-3-(1H-pyrrolo[2,3-b]pyridin-3-yl)acrylamide |
| **26** | 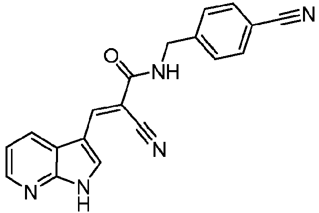 | (E)-2-cyano-N-(4-cyanobenzyl)-3-(1H-pyrrolo[2,3-b]pyridin-3-yl)acrylamide |
| **27** | 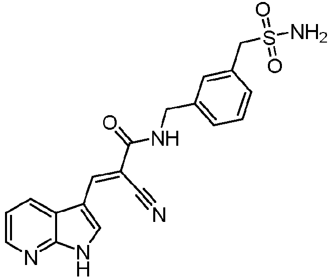 | (E)-2-cyano-3-(1H-pyrrolo[2,3-b]pyridin-3-yl)-N-(3-(sulfamoylmethyl)benzyl)-acrylamide |
| **28** | 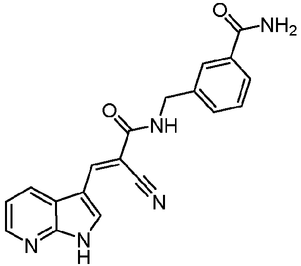 | (E)-3-((2-cyano-3-(1H-pyrrolo[2,3-b]pyridin-3-yl)acrylamido)methyl) benzamide |
| **29** | 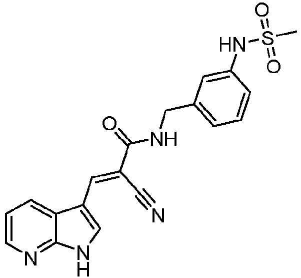 | (E)-2-cyano-N-(3-(methylsulfonamido) benzyl)-3-(1H-pyrrolo[2,3-b]pyridin-3-yl)acrylamide |
| **30** | 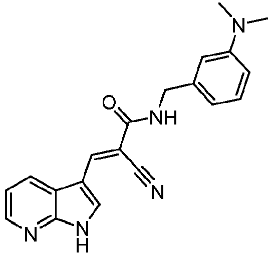 | (E)-2-cyano-N-(3-(dimethylamino)benzyl)-3-(1H-pyrrolo[2,3-b]pyridin-3-yl)acrylamide |
| **31** | 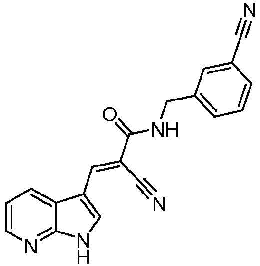 | (E)-2-cyano-N-(3-cyanobenzyl)-3-(1H-pyrrolo[2,3-b]pyridin-3-yl)acrylamide |
| **32** | 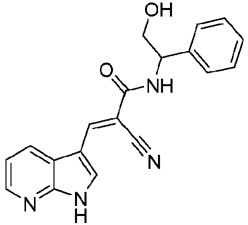 | (E)-2-cyano-N-(2-hydroxy-1-phenylethyl)-3-(1H-pyrrolo[2,3-b]pyridin-3-yl)acrylamide |
| **33** | 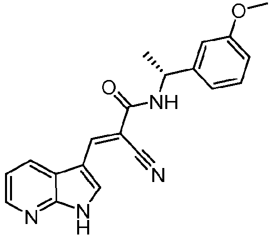 | (R,E)-2-cyano-N-(1-(3-methoxyphenyl) ethyl)-3-(1H-pyrrolo[2,3-b]pyridin-3-yl)acrylamide |
| **34** | 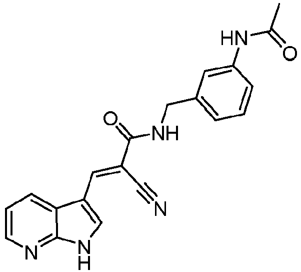 | (E)-N-(3-acetamidobenzyl)-2-cyano-3-(1H-pyrrolo[2,3-b]pyridin-3-yl)acrylamide |
| **35** | 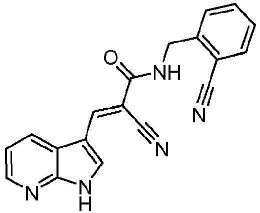 | (E)-2-cyano-N-(2-cyanobenzyl)-3-(1H-pyrrolo[2,3-b]pyridin-3-yl)acrylamide |
| **36** | 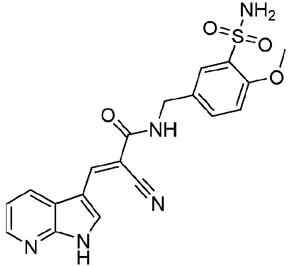 | (E)-2-cyano-N-(4-methoxy-3-sulfamoylbenzyl)-3-(1H-pyrrolo[2,3-b]pyridin-3-yl)acrylamide |
| **37** | 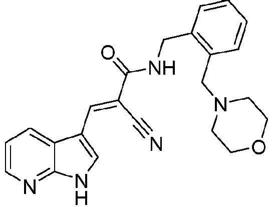 | (E)-2-cyano-N-(2-(morpholinomethyl) benzyl)-3-(1H-pyrrolo[2,3-b]pyridin-3-yl)acrylamide |
| **38** | 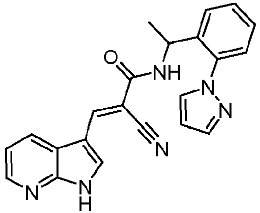 | (E)-N-(1-(2-(1H-pyrazol-1-yl)phenyl)ethyl)-2-cyano-3-(1H-pyrrolo[2,3-b]pyridin-3-yl)acrylamide |
| **39** | 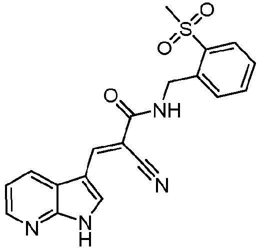 | (E)-2-cyano-N-(2-(methylsulfonyl)benzyl)-3-(1H-pyrrolo[2,3-b]pyridin-3-yl)acrylamide |
| **40** | 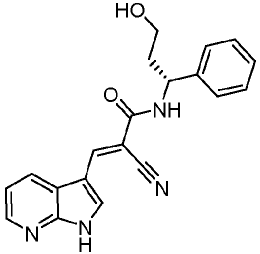 | (R, E)-2-cyano-N-(3-hydroxy-1-phenylpropyl)-3-(1H-pyrrolo[2,3-b]pyridin-3-yl)acrylamide |
| **41** | 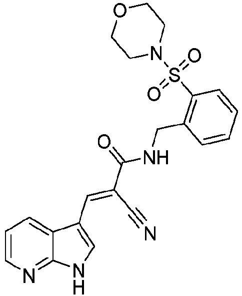 | (E)-2-cyano-N-(2-(morpholinosulfonyl) benzyl)-3-(1H-pyrrolo[2,3-b]pyridin-3-yl)acrylamide |
| **42** | 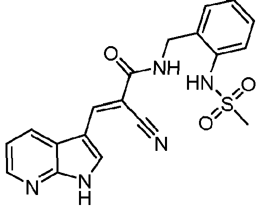 | (E)-2-cyano-N-(2-(methylsulfonamido) benzyl)-3-(1H-pyrrolo[2,3-b]pyridin-3-yl)acrylamide |
| **43** | 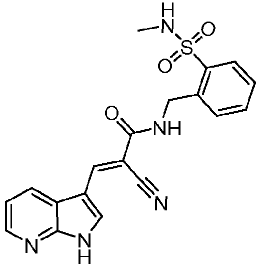 | (E)-2-cyano-N-(2-(N-methylsulfamoyl) benzyl)-3-(1H-pyrrolo[2,3-b]pyridin-3-yl)acrylamide |
| **44** | 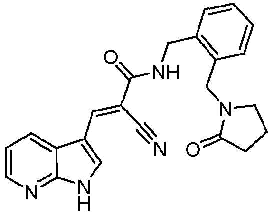 | (E)-2-cyano-N-(2-((2-oxopyrrolidin-1-yl)methyl)benzyl)-3-(1H-pyrrolo[2,3-b]pyridin-3-yl)acrylamide |
| **45** | 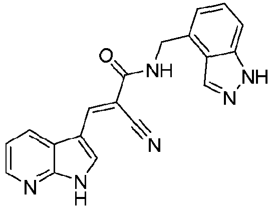 | (E)-N-((1H-indazol-4-yl)methyl)-2-cyano-3-(1H-pyrrolo[2,3-b]pyridin-3-yl)acrylamide |
| **46** | 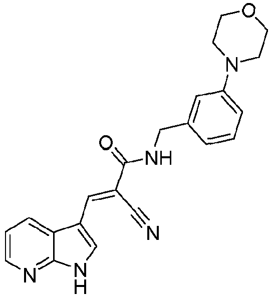 | (E)-2-cyano-N-(3-morpholinobenzyl)-3-(1H-pyrrolo[2,3-b]pyridin-3-yl)acrylamide |
| **47** | 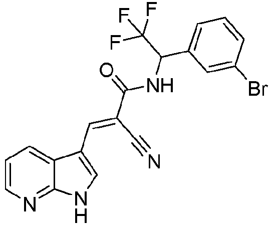 | (E)-N-(1-(3-bromophenyl)-2,2,2-trifluoroethyl)-2-cyano-3-(1H-pyrrolo[2,3-b]pyridin-3-yl)acrylamide |
| **48** | 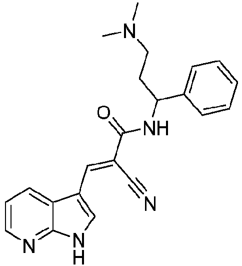 | (E)-2-cyano-N-(3-(dimethylamino)-1-phenylpropyl)-3-(1H-pyrrolo[2,3-b]pyridin-3-yl)acrylamide |
| **49** | 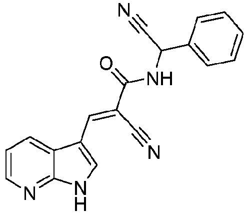 | (E)-2-cyano-N-(cyano(phenyl)methyl)-3-(1H-pyrrolo[2,3-b]pyridin-3-yl)acrylamide |
| **50** | 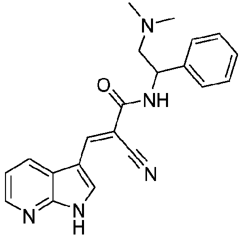 | (E)-2-cyano-N-(2-(dimethylamino)-1-phenylethyl)-3-(1H-pyrrolo[2,3-b]pyridin-3-yl)acrylamide |
| **51** | 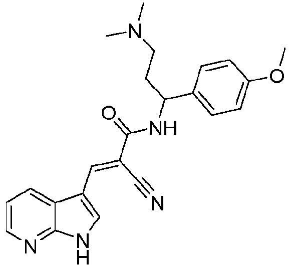 | (E)-2-cyano-N-(3-(dimethylamino)-1-(4-methoxyphenyl)propyl)-3-(1H-pyrrolo[2,3-b]pyridin-3-yl)acrylamide |
| **52** | 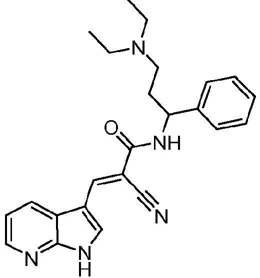 | (E)-2-cyano-N-(3-(diethylamino)-1-phenylpropyl)-3-(1H-pyrrolo[2,3-b]pyridin-3-yl)acrylamide |
| **53** | 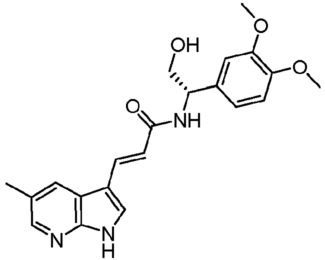 | (S, E)-N-(1-(3,4-dimethoxyphenyl)-2-hydroxyethyl)-3-(5-methyl-1H-pyrrolo[2,3-b]pyridin-3-yl)acrylamide |
| **54** | 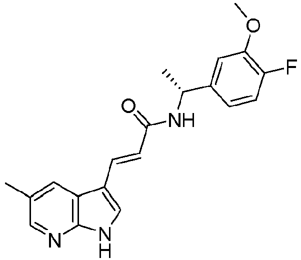 | (R,E)-N-(1-(4-fluoro-3-methoxyphenyl) ethyl)-3-(5-methyl-1H-pyrrolo[2,3-b]pyridin-3-yl)acrylamide |
| **55** | 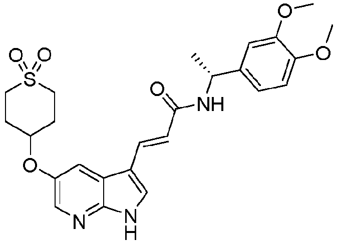 | (R,E)-N-(1-(3,4-dimethoxyphenyl)ethyl)-3-(5-((1,1-dioxidotetrahydro-2H-thiopyran-4-yl)oxy)-1H-pyrrolo[2,3-b]pyridin-3-yl)acrylamide |
| **56** | 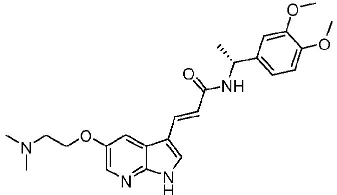 | (R,E)-N-(1-(3,4-dimethoxyphenyl)ethyl)-3-(5-(2-(dimethylamino)ethoxy)-1H-pyrrolo[2,3-b]pyridin-3-yl)acrylamide |
| **57** | 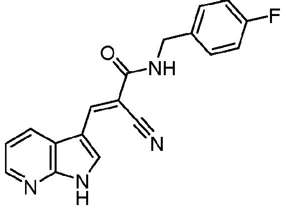 | (E)-2-cyano-N-(4-fluorobenzyl)-3-(1H-pyrrolo[2,3-b]pyridin-3-yl)acrylamide |
| **58** | 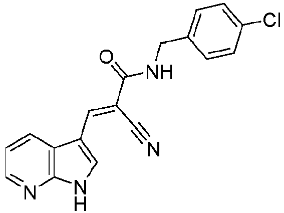 | (E)-N-(4-chlorobenzyl)-2-cyano-3-(1H-pyrrolo[2,3-b]pyridin-3-yl)acrylamide |
| **59** | 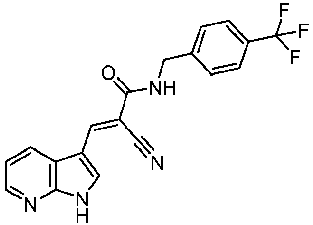 | (E)-2-cyano-3-(1H-pyrrolo[2,3-b]pyridin-3-yl)-N-(4-(trifluoromethyl)benzyl)acrylamide |
| **60** | 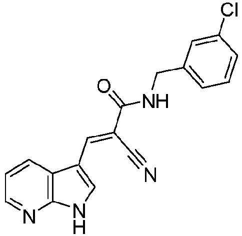 | (E)-N-(3-chlorobenzyl)-2-cyano-3-(1H-pyrrolo[2,3-b]pyridin-3-yl)acrylamide |
| **61** | 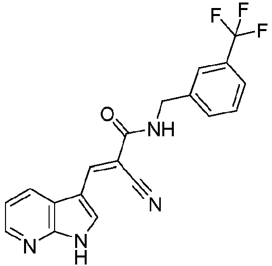 | (E)-2-cyano-3-(1H-pyrrolo[2,3-b]pyridin-3-yl)-N-(3-(trifluoromethyl)benzyl)acrylamide |
| **62** | 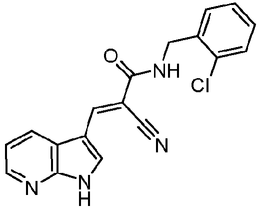 | (E)-N-(2-chlorobenzyl)-2-cyano-3-(1H-pyrrolo[2,3-b]pyridin-3-yl)acrylamide |
| **63** | 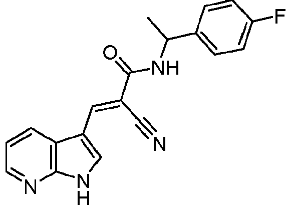 | (E)-2-cyano-N-(1-(4-fluorophenyl)ethyl)-3-(1H-pyrrolo[2,3-b]pyridin-3-yl)acrylamide |
| **64** | 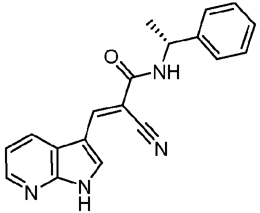 | (R,E)-2-cyano-N-(1-phenylethyl)-3-(1H-pyrrolo[2,3-b]pyridin-3-yl)acrylamide |
| **65** | 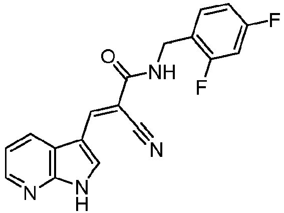 | (E)-2-cyano-N-(2,4-difluorobenzyl)-3-(1H-pyrrolo[2,3-b]pyridin-3-yl)acrylamide |
| **66** | 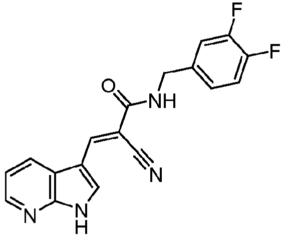 | (E)-2-cyano-N-(3,4-difluorobenzyl)-3-(1H-pyrrolo[2,3-b]pyridin-3-yl)acrylamide |
| **67** | 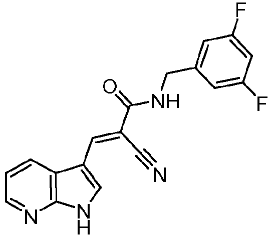 | (E)-2-cyano-N-(3,5-difluorobenzyl)-3-(1H-pyrrolo[2,3-b]pyridin-3-yl)acrylamide |
| **68** | 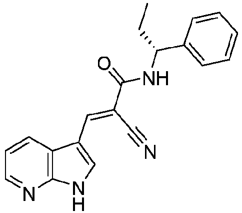 | (R,E)-2-cyano-N-(1-phenylpropyl)-3-(1H-pyrrolo[2,3-b]pyridin-3-yl)acrylamide |
| **69** | 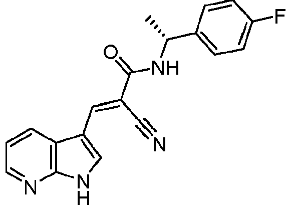 | (R, E)-2-cyano-N-(1-(4-fluorophenyl)ethyl)-3-(1H-pyrrolo[2,3-b]pyridin-3-yl)acrylamide |
| **70** | 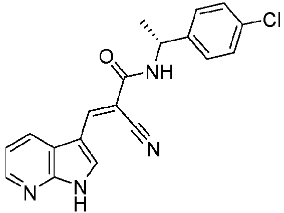 | (R,E)-N-(1-(4-chlorophenyl)ethyl)-2-cyano-3-(1H-pyrrolo[2,3-b]pyridin-3-yl)acrylamide |
| **71** | 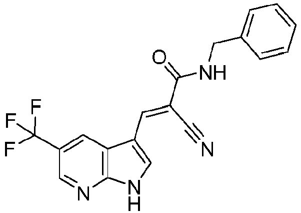 | (E)-N-benzyl-2-cyano-3-(5-(trifluoromethyl)-1H-pyrrolo[2,3-b]pyridin-3-yl)acrylamide |
| **72** | 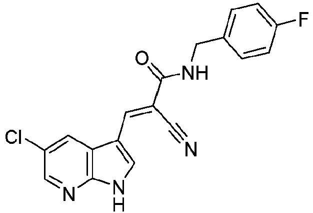 | (E)-3-(5-chloro-1H-pyrrolo[2,3-b]pyridin-3-yl)-2-cyano-N-(4-fluorobenzyl)acrylamide |
| **73** | 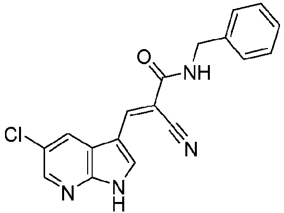 | (E)-N-benzyl-3-(5-chloro-1H-pyrrolo[2,3-b]pyridin-3-yl)-2-cyanoacrylamide |
| **74** | 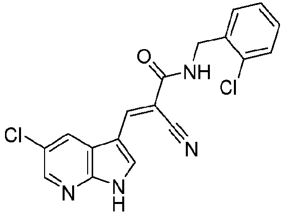 | (E)-3-(5-chloro-1H-pyrrolo[2,3-b]pyridin-3-yl)-N-(2-chlorobenzyl)-2-cyanoacrylamide |
| **75** | 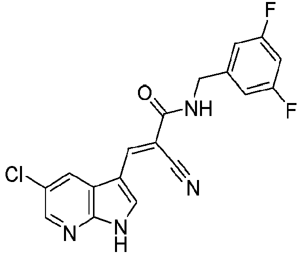 | (E)-3-(5-chloro-1H-pyrrolo[2,3-b]pyridin-3-yl)-2-cyano-N-(3,5-difluorobenzyl)acrylamide |
| **76** | 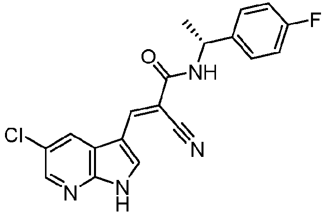 | (R,E)-3-(5-chloro-1H-pyrrolo[2,3-b]pyridin-3-yl)-2-cyano-N-(1-(4-fluorophenyl) ethylacrylamide |
| **77** | 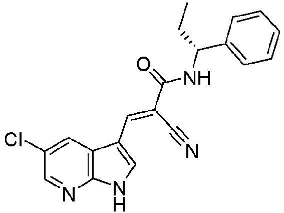 | (R,E)-3-(5-chloro-1H-pyrrolo[2,3-b]pyridin-3-yl)-2-cyano-N-(1-phenylpropyl) acrylamide |
| **78** | 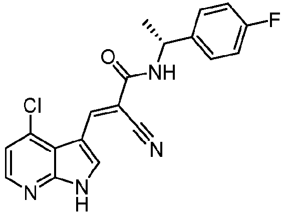 | (R,E)-3-(4-chloro-1H-pyrrolo[2,3-b]pyridin-3-yl)-2-cyano-N-(1-(4-fluorophenyl) ethyl)acrylamide |
| **79** | 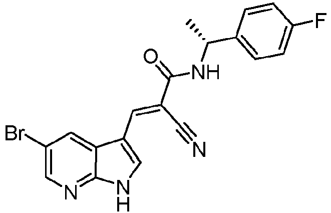 | (R,E)-3-(5-bromo-1H-pyrrolo[2,3-b]pyridin-3-yl)-2-cyano-N-(1-(4-fluorophenyl) ethyl)acrylamide |
| **80** | 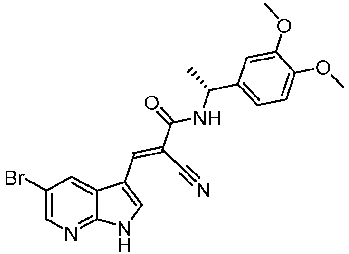 | (R,E)-3-(5-bromo-1H-pyrrolo[2,3-b]pyridin-3-yl)-2-cyano-N-(1-(3,4-dimethoxyphenyl) ethyl)acrylamide |
| **81** | 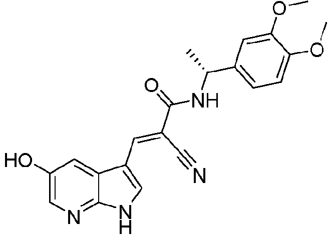 | (R, E)-2-cyano-N-(1-(3,4-dimethoxyphenyl)ethyl)-3-(5-hydroxy-1H-pyrrolo[2,3-b]pyridin-3-yl)acrylamide |
| **82** | 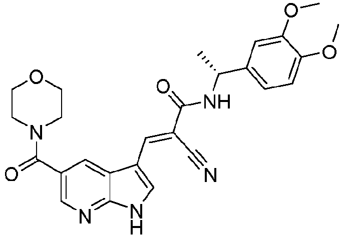 | (R,E)-2-cyano-N-(1-(3,4-dimethoxyphenyl)ethyl)-3-(5-((tetrahydro-2H-pyran-4-yl)oxy)-1H-pyrrolo[2,3-b]pyridin-3-yl)acrylamide |
| **83** | 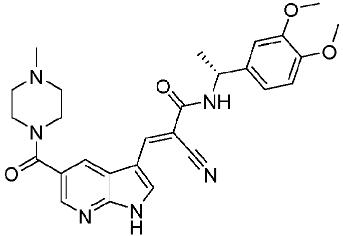 | (R,E)-2-cyano-N-(1-(3,4-dimethoxyphenyl)ethyl)-3-(5-(4-methylpiperazine-1-carbonyl)-1H-pyrrolo[2,3-b]pyridin-3-yl)acrylamide |
| **84** | 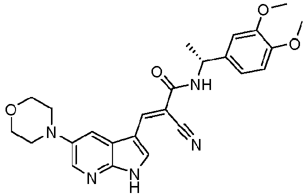 | (R,E)-2-cyano-N-(1-(3,4-dimethoxyphenyl) ethyl)-3-(5-morpholino-1H-pyrrolo[2,3-b]pyridin-3-yl)acrylamide |
| **85** | 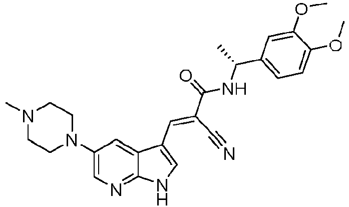 | (R,E)-2-cyano-N-(1-(3,4-dimethoxyphenyl) ethyl)-3-(5-morpholino-1H-pyrrolo[2,3-b]pyridin-3-yl)acrylamide |
| **86** | 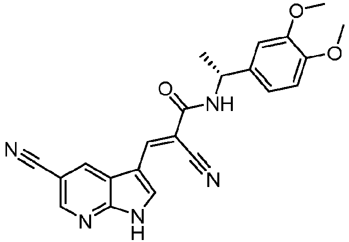 | (R,E)-2-cyano-3-(5-cyano-1H-pyrrolo[2,3-b]pyridin-3-yl)-N-(1-(3,4-dimethoxyphenyl)ethyl)acrylamide |
| **87** | 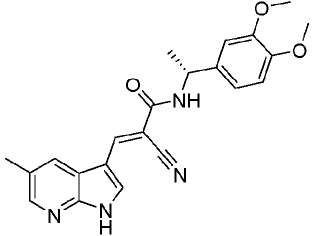 | (R,E)-2-cyano-N-(1-(3,4-dimethoxyphenyl) ethyl)-3-(5-methyl-1H-pyrrolo[2,3-b]pyridin-3-yl)acrylamide |
| **88** | 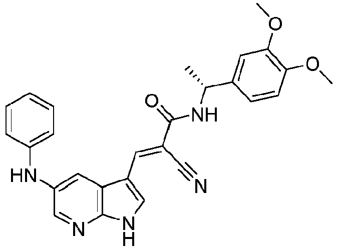 | (R,E)-2-cyano-N-(1-(3,4-dimethoxyphenyl) ethyl)-3-(5-(phenylamino)-1H-pyrrolo[2,3-b]pyridin-3-yl)acrylamide |
| **89** | 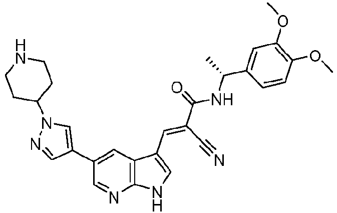 | (R,E)-2-cyano-N-(1-(3,4-dimethoxyphenyl) ethyl)-3-(5-(1-(piperidin-4-yl)-1H-pyrazol-4-yl)-1H-pyrrolo[2,3-b]pyridin-3-yl)acrylamide |
| **90** | 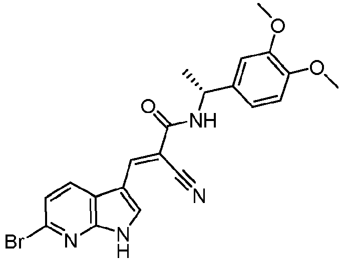 | (R,E)-3-(6-bromo-1H-pyrrolo[2,3-b]pyridin-3-yl)-2-cyano-N-(1-(3,4-dimethoxyphenyl) ethyl)acrylamide |
| **91** | 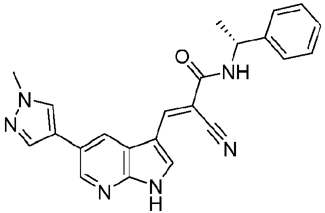 | (R,E)-2-cyano-3-(5-(1-methyl-1H-pyrazol-4-yl)-1H-pyrrolo[2,3-b]pyridin-3-yl)-N-(1-phenylethyl)acrylamide |
| **92** | 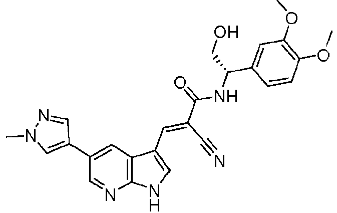 | (S,E)-2-cyano-N-(1-(3,4-dimethoxyphenyl) -2-hydroxyethyl)-3-(5-(1-methyl-1H-pyrazol-4-yl)-1H-pyrrolo[2,3-b]pyridin-3-yl)acrylamide |
| **93** | 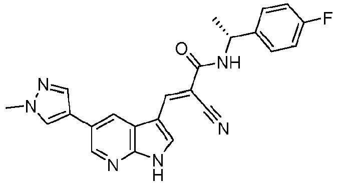 | (R,E)-2-cyano-N-(1-(4-fluorophenyl)ethyl)-3-(5-(1-methyl-1H-pyrazol-4-yl)-1H-pyrrolo[2,3-b]pyridin-3-yl)acrylamide |
| **94** | 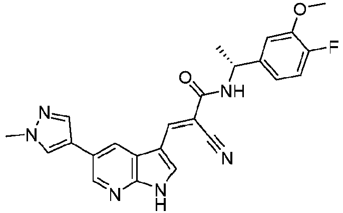 | (R,E)-2-cyano-N-(1-(4-fluoro-3-methoxyphenyl)ethyl)-3-(5-(1-methyl-1H-pyrazol-4-yl)-1H-pyrrolo[2,3-b]pyridin-3-yl)acrylamide |
| **95** | 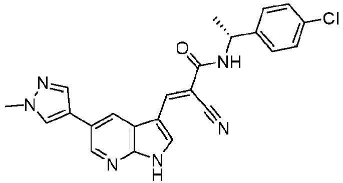 | (R,E)-N-(1-(4-chlorophenyl)ethyl)-2-cyano-3-(5-(1-methyl-1H-pyrazol-4-yl)-1H-pyrrolo[2,3-b]pyridin-3-yl)acrylamide |
| **96** | 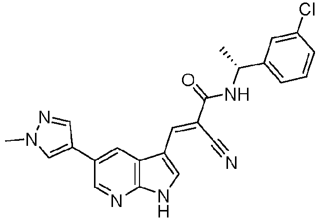 | (R,E)-N-(1-(3-chlorophenyl)ethyl)-2-cyano-3-(5-(1-methyl-1H-pyrazol-4-yl)-1H-pyrrolo[2,3-b]pyridin-3-yl)acrylamide |
| **97** | 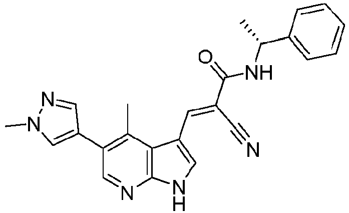 | (R,E)-2-cyano-3-(4-methyl-5-(1-methyl-1H-pyrazol-4-yl)-1H-pyrrolo[2,3-b]pyridin-3-yl)-N-(1-phenylethyl)acrylamide |
| **98** | 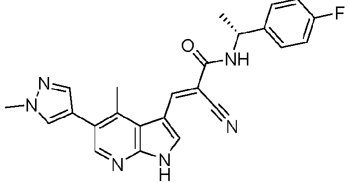 | (R,E)-2-cyano-N-(1-(4-fluorophenyl)ethyl)-3-(4-methyl-5-(1-methyl-1H-pyrazol-4-yl)-1H-pyrrolo[2,3-b]pyridin-3-yl)acrylamide |
| **99** | 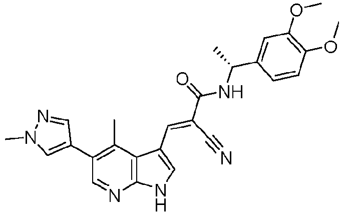 | (R,E)-2-cyano-N-(1-(3,4-dimethoxyphenyl) ethyl)-3-(4-methyl-5-(1-methyl-1H-pyrazol-4-yl)-1H-pyrrolo[2,3-b]pyridin-3-yl)acrylamide |
| **100** | 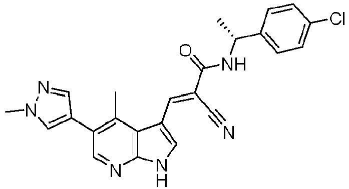 | (R,E)-N-(1-(4-chlorophenyl)ethyl)-2-cyano-3-(4-methyl-5-(1-methyl-1H-pyrazol-4-yl)-1H-pyrrolo[2,3-b]pyridin-3-yl)acrylamide |
| **101** | 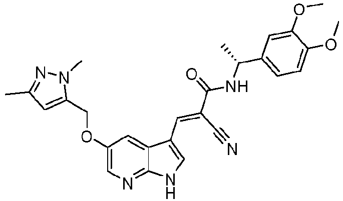 | (R,E)-2-cyano-N-(1-(3,4-dimethoxyphenyl) ethyl)-3-(5-((1,3-dimethyl-1H-pyrazol-5-yl)methoxy)-1H-pyrrolo[2,3-b]pyridin-3-yl)acrylamide |
| **102** | 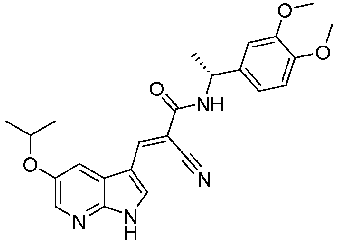 | (R,E)-2-cyano-N-(1-(3,4-dimethoxyphenyl) ethyl)-3-(5-isopropoxy-1H-pyrrolo[2,3-b]pyridin-3-yl)acrylamide |
| **103** | 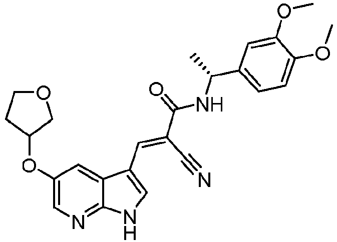 | (E)-2-cyano-N-((R)-1-(3,4-dimethoxyphenyl)ethyl)-3-(5-((tetrahydrofuran-3-yl)oxy)-1H-pyrrolo[2,3-b]pyridin-3-yl)acrylamide |
| **104** | 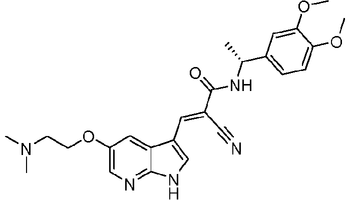 | (R,E)-2-cyano-N-(1-(3,4-dimethoxyphenyl) ethyl)-3-(5-(2-(dimethylamino)ethoxy)-1H-pyrrolo[2,3-b]pyridin-3-yl)acrylamide |
| **105** | 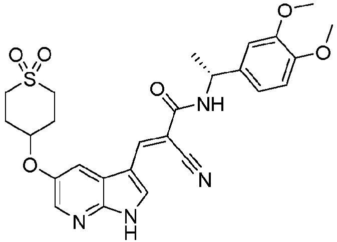 | (R,E)-2-cyano-N-(1-(3,4-dimethoxyphenyl) ethyl)-3-(5-((1,1-dioxidotetrahydro-2H-thiopyran-4-yl)oxy)-1H-pyrrolo[2,3-b]pyridin-3-yl)acrylamide |
| **106** | 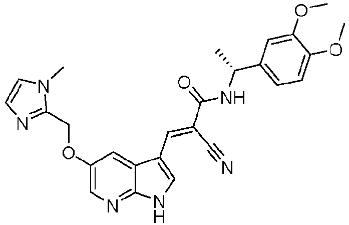 | (R,E)-2-cyano-N-(1-(3,4-dimethoxyphenyl) ethyl)-3-(5-((1-methyl-1H-imidazol-2-yl)methoxy)-1H-pyrrolo[2,3-b]pyridin-3-yl)acrylamide |
| **107** | 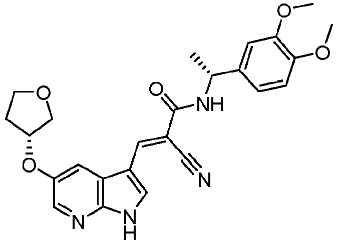 | (E)-2-cyano-N-((R)-1-(3,4-dimethoxyphenyl)ethyl)-3-(5-(((R)-tetrahydrofuran-3-yl)oxy)-1H-pyrrolo[2,3-b]pyridin-3-yl)acrylamide |
| **108** | 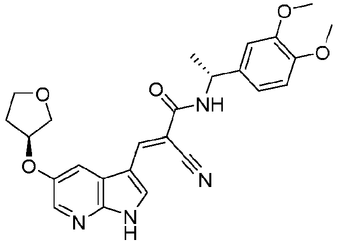 | (E)-2-cyano-N-((R)-1-(3,4-dimethoxyphenyl)ethyl)-3-(5-(((S)-tetrahydrofuran-3-yl)oxy)-1H-pyrrolo[2,3-b]pyridin-3-yl)acrylamide |
| **109** | 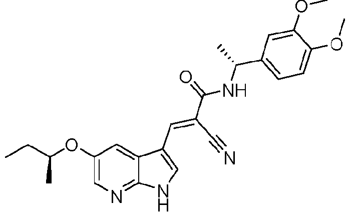 | (E)-3-(5-((S)-sec-butoxy)-1H-pyrrolo[2,3-b]pyridin-3-yl)-2-cyano-N-((R)-1-(3,4-dimethoxyphenyl)ethyl)acrylamide |
| **110** | 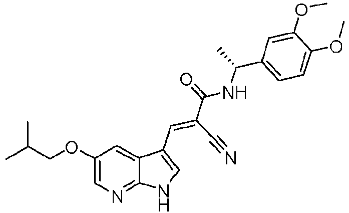 | (E)-3-(5-((S)-sec-butoxy)-1H-pyrrolo[2,3-b]pyridin-3-yl)-2-cyano-N-((R)-1-(3,4-dimethoxyphenyl)ethyl)acrylamide |
| **111** | 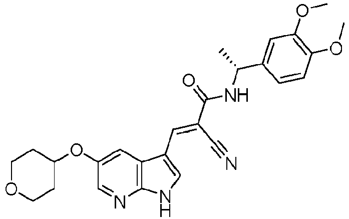 | (R,E)-2-cyano-N-(1-(3,4-dimethoxyphenyl) ethyl)-3-(5-((tetrahydro-2H-pyran-4-yl)oxy)-1H-pyrrolo[2,3-b]pyridin-3-yl)acrylamide |
| **112** | 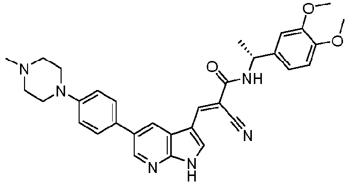 | (R,E)-2-cyano-N-(1-(3,4-dimethoxyphenyl) ethyl)-3-(5-(4-(4-methylpiperazin-1-yl)phenyl)-1H-pyrrolo[2,3-b]pyridin-3-yl)acrylamide |
| **113** | 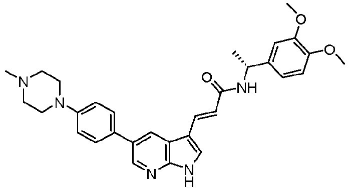 | (R,E)-N-(1-(3,4-dimethoxyphenyl)ethyl)-3-(5-(4-(4-methylpiperazin-1-yl)phenyl)-1H-pyrrolo[2,3-b]pyridin-3-yl)acrylamide |
| **114** | 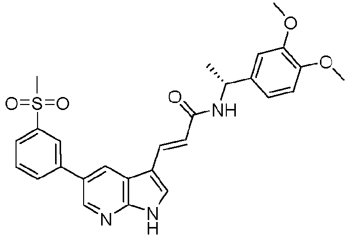 | (R,E)-N-(1-(3,4-dimethoxyphenyl)ethyl)-3-(5-(3-(methylsulfonyl)phenyl)-1H-pyrrolo[2,3-b]pyridin-3-yl)acrylamide |
| **115** | 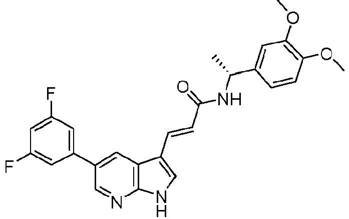 | (R,E)-3-(5-(3,5-difluorophenyl)-1H-pyrrolo[2,3-b]pyridin-3-yl)-N-(1-(3,4-dimethoxyphenyl)ethyl)acrylamide |
| **116** | 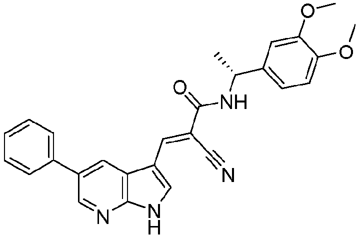 | (R,E)-2-cyano-N-(1-(3,4-dimethoxyphenyl) ethyl)-3-(5-(4-(4-methylpiperazin-1-yl)phenyl)-1H-pyrrolo[2,3-b]pyridin-3-yl)acrylamide |
| **117** | 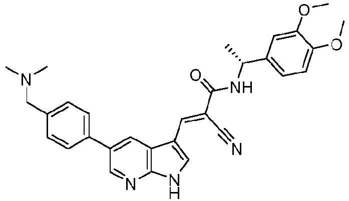 | (R,E)-2-cyano-N-(1-(3,4-dimethoxyphenyl) ethyl)-3-(5-(4-((dimethylamino)methyl)phenyl)-1H-pyrrolo[2,3-b]pyridin-3-yl)acrylamide |
| **118** | 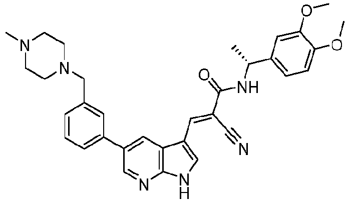 | (R,E)-2-cyano-N-(1-(3,4-dimethoxyphenyl) ethyl)-3-(5-(3-((4-methylpiperazin-1-yl)methyl)phenyl)-1H-pyrrolo[2,3-b]pyridin-3-yl)acrylamide |
| **119** | 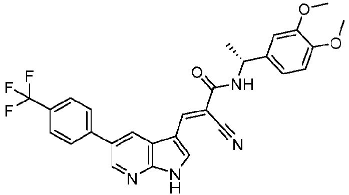 | (R,E)-2-cyano-N-(1-(3,4-dimethoxyphenyl) ethyl)-3-(5-(3-((4-methylpiperazin-1-yl)methyl)phenyl)-1H-pyrrolo[2,3-b]pyridin-3-yl)acrylamide |
| **120** | 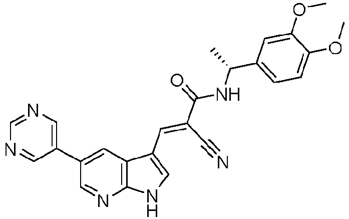 | (R,E)-2-cyano-N-(1-(3,4-dimethoxyphenyl) ethyl)-3-(5-(pyrimidin-5-yl)-1H-pyrrolo[2,3-b]pyridin-3-yl)acrylamide |
| **121** | 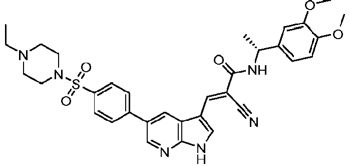 | (R,E)-2-cyano-N-(1-(3,4-dimethoxyphenyl) ethyl)-3-(5-(4-((4-ethylpiperazin-1-yl)sulfonyl)phenyl)-1H-pyrrolo[2,3-b]pyridin-3-yl)acrylamide |
| **122** | 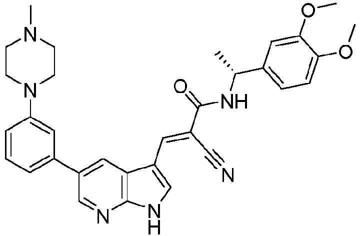 | (R,E)-2-cyano-N-(1-(3,4-dimethoxyphenyl) ethyl)-3-(5-(3-(4-methylpiperazin-1-yl)phenyl)-1H-pyrrolo[2,3-b]pyridin-3-yl)acrylamide |
| **123** | 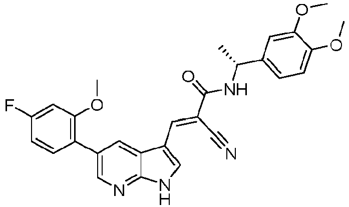 | (R,E)-2-cyano-N-(1-(3,4-dimethoxyphenyl) ethyl)-3-(5-(3-(4-methylpiperazin-1-yl)phenyl)-1H-pyrrolo[2,3-b]pyridin-3-yl)acrylamide |
| **124** | 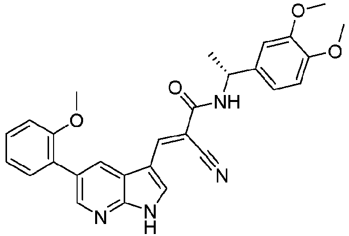 | (R,E)-2-cyano-N-(1-(3,4-dimethoxyphenyl) ethyl)-3-(5-(4-fluoro-2-methoxyphenyl)-1H-pyrrolo[2,3-b]pyridin-3-yl)acrylamide |
| **125** | 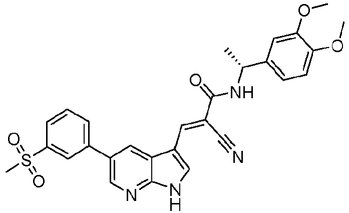 | (R,E)-2-cyano-N-(1-(3,4-dimethoxyphenyl) ethyl)-3-(5-(2-methoxyphenyl)-1H-pyrrolo[2,3-b]pyridin-3-yl)acrylamide |
| **126** | 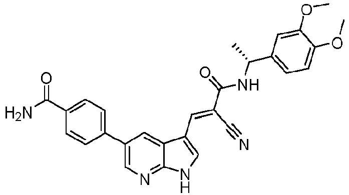 | (R,E)-4-(3-(2-cyano-3-((1-(3,4-dimethoxyphenyl)ethyl)amino)-3-oxoprop-1-en-1-yl)-1H-pyrrolo[2,3-b]pyridin-5-yl)benzamide |
| **127** | 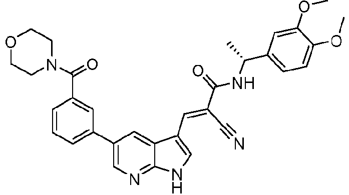 | (R,E)-2-cyano-N-(1-(3,4-dimethoxyphenyl)ethyl)-3-(5-(3-(morpholine-4-carbonyl)phenyl)-1H-pyrrolo[2,3-b]pyridin-3-yl)acrylamide |
| **128** | 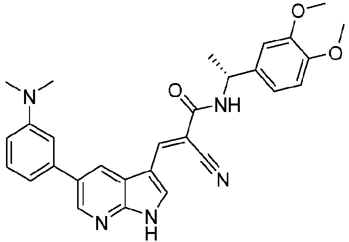 | (R,E)-2-cyano-N-(1-(3,4-dimethoxyphenyl) ethyl)-3-(5-(3-(dimethylamino)phenyl)-1H-pyrrolo[2,3-b]pyridin-3-yl)acrylamide |
| **129** | 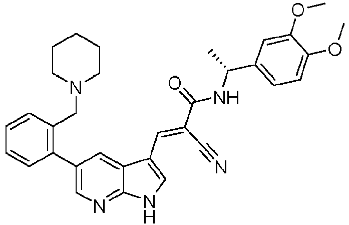 | (R,E)-2-cyano-N-(1-(3,4-dimethoxyphenyl) ethyl)-3-(5-(2-(piperidin-1-ylmethyl)phenyl)-1H-pyrrolo[2,3-b]pyridin-3-yl)acrylamide |
| **130** | 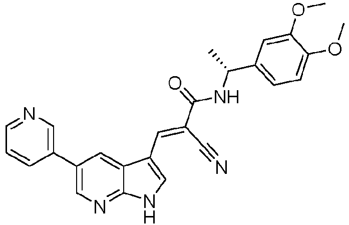 | (R,E)-2-cyano-N-(1-(3,4-dimethoxyphenyl) ethyl)-3-(5-(2-(piperidin-1-ylmethyl)phenyl)-1H-pyrrolo[2,3-b]pyridin-3-yl)acrylamide |
| **131** | 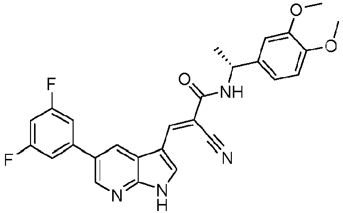 | (R,E)-2-cyano-N-(1-(3,4-dimethoxyphenyl) ethyl)-3-(5-(pyridin-3-yl)-1H-pyrrolo[2,3-b]pyridin-3-yl)acrylamide |
| **132** | 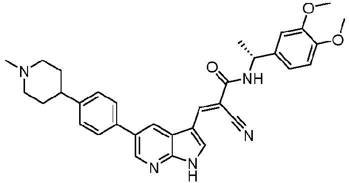 | (R,E)-2-cyano-N-(1-(3,4-dimethoxyphenyl) ethyl)-3-(5-(4-(1-methylpiperidin-4-yl)phenyl)-1H-pyrrolo[2,3-b]pyridin-3-yl)acrylamide |
| **133** | 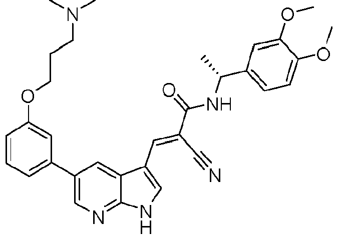 | (R,E)-2-cyano-N-(1-(3,4-dimethoxyphenyl) ethyl)-3-(5-(4-(1-methylpiperidin-4-yl)phenyl)-1H-pyrrolo[2,3-b]pyridin-3-yl)acrylamide |
| **134** | 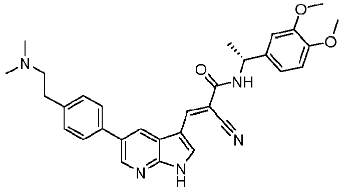 | (R,E)-2-cyano-N-(1-(3,4-dimethoxyphenyl) ethyl)-3-(5-(3-(3-(dimethylamino)propoxy)phenyl)-1H-pyrrolo[2,3-b]pyridin-3-yl)acrylamide |
| **135** | 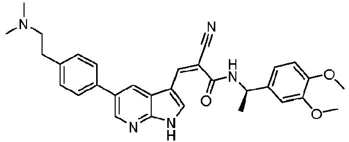 | (R,E)-2-cyano-N-(1-(3,4-dimethoxyphenyl) ethyl)-3-(5-(4-(2-(dimethylamino)ethyl)phenyl)-1H-pyrrolo[2,3-b]pyridin-3-yl)acrylamide |
| **136** | 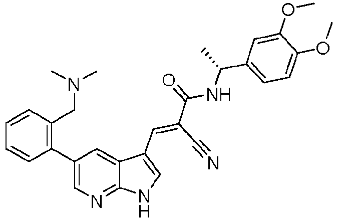 | (R,Z)-2-cyano-N-(1-(3,4-dimethoxyphenyl) ethyl)-3-(5-(4-(2-(dimethylamino)ethyl)phenyl)-1H-pyrrolo[2,3-b]pyridin-3-yl)acrylamide |
| **137** | 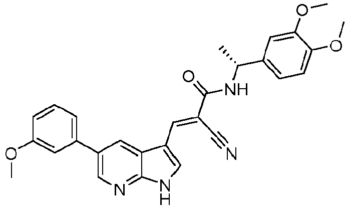 | (R,E)-2-cyano-N-(1-(3,4-dimethoxyphenyl) ethyl)-3-(5-(3-methoxyphenyl)-1H-pyrrolo[2,3-b]pyridin-3-yl)acrylamide |
| **138** | 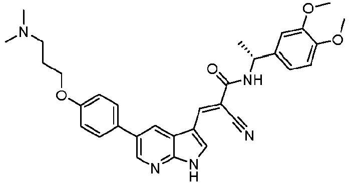 | (R,E)-2-cyano-N-(1-(3,4-dimethoxyphenyl) ethyl)-3-(5-(3-methoxyphenyl)-1H-pyrrolo[2,3-b]pyridin-3-yl)acrylamide |
| **139** | 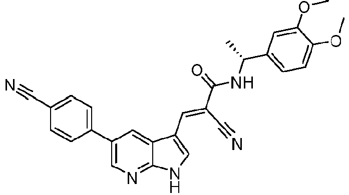 | (R,E)-2-cyano-N-(1-(3,4-dimethoxyphenyl) ethyl)-3-(5-(2-(morpholinomethyl)phenyl)-1H-pyrrolo[2,3-b]pyridin-3-yl)acrylamide |
| **140** | 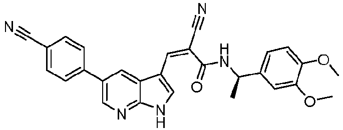 | (R,Z)-2-cyano-3-(5-(4-cyanophenyl)-1H-pyrrolo[2,3-b]pyridin-3-yl)-N-(1-(3,4-dimethoxyphenyl)ethyl)acrylamide |
| **141** | 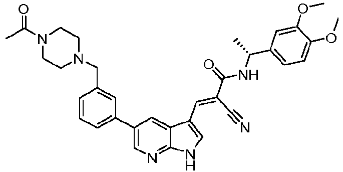 | (R,E)-3-(5-(3-((4-acetylpiperazin-1-yl)methyl)phenyl)-1H-pyrrolo[2,3-b]pyridin-3-yl)-2-cyano-N-(1-(3,4-dimethoxyphenyl)ethyl)acrylamide |
| **142** | 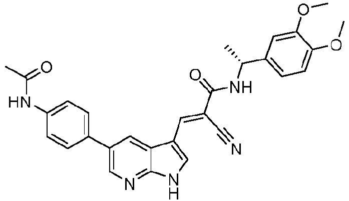 | (R,E)-3-(5-(3-((4-acetylpiperazin-1-yl)methyl)phenyl)-1H-pyrrolo[2,3-b]pyridin-3-yl)-2-cyano-N-(1-(3,4-dimethoxyphenyl)ethyl)acrylamide |
| **143** | 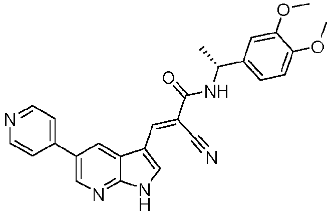 | (R,E)-3-(5-(4-acetamidophenyl)-1H-pyrrolo[2,3-b]pyridin-3-yl)-2-cyano-N-(1-(3,4-dimethoxyphenyl)ethyl)acrylamide |
| **144** | 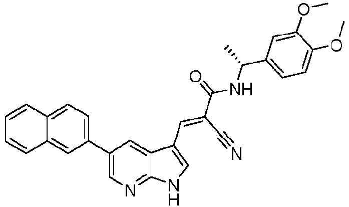 | (R,E)-2-cyano-N-(1-(3,4-dimethoxyphenyl) ethyl)-3-(5-(pyridin-4-yl)-1H-pyrrolo[2,3-b]pyridin-3-yl)acrylamide |
| **145** | 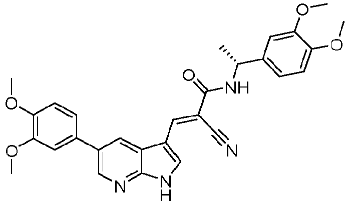 | (R,E)-2-cyano-N-(1-(3,4-dimethoxyphenyl) ethyl)-3-(5-(naphthalen-2-yl)-1H-pyrrolo[2,3-b]pyridin-3-yl)acrylamide |
| **146** | 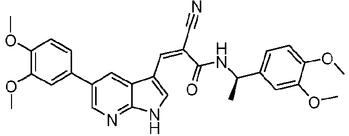 | (R,Z)-2-cyano-3-(5-(3,4-dimethoxyphenyl)-1H-pyrrolo[2,3-b]pyridin-3-yl)-N-(1-(3,4-dimethoxyphenyl)ethyl)acrylamide |
| **147** | 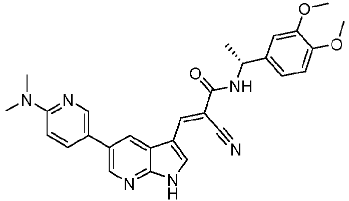 | (R,E)-2-cyano-N-(1-(3,4-dimethoxyphenyl) ethyl)-3-(5-(6-(dimethylamino)pyridin-3-yl)-1H-pyrrolo[2,3-b]pyridin-3-yl)acrylamide |
| **148** | 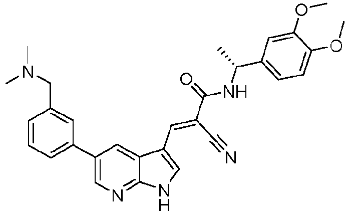 | (R,E)-2-cyano-N-(1-(3,4-dimethoxyphenyl) ethyl)-3-(5-(6-(dimethylamino)pyridin-3-yl)-1H-pyrrolo[2,3-b]pyridin-3-yl)acrylamide |
| **149** | 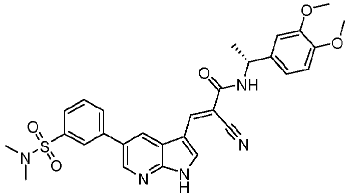 | (R,E)-2-cyano-N-(1-(3,4-dimethoxyphenyl) ethyl)-3-(5-(3-((dimethylamino)methyl)phenyl)-1H-pyrrolo[2,3-b]pyridin-3-yl)acrylamide |
| **150** | 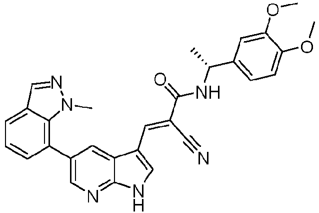 | (R,E)-2-cyano-N-(1-(3,4-dimethoxyphenyl) ethyl)-3-(5-(3-(N, N-dimethylsulfamoyl)phenyl)-1H-pyrrolo[2,3-b]pyridin-3-yl)acrylamide |
| **151** | 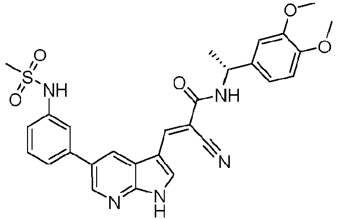 | (R,E)-2-cyano-N-(1-(3,4-dimethoxyphenyl)ethyl)-3-(5-(3-(methylsulfonamido)phenyl)-1H-pyrrolo[2,3-b]pyridin-3-yl)acrylamide |
| **152** | 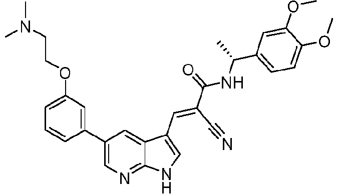 | (R, E)-2-cyano-N-(1-(3,4-dimethoxyphenyl)ethyl)-3-(5-(3-(methylsulfonamido)phenyl)-1H-pyrrolo[2,3-b]pyridin-3-yl)acrylamide |
| **153** | 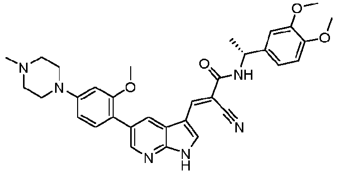 | (R, E)-2-cyano-N-(1-(3,4-dimethoxyphenyl)ethyl)-3-(5-(3-(2-(dimethylamino)ethoxy)phenyl)-1H-pyrrolo[2,3-b]pyridin-3-yl)acrylamide |
| **154** | 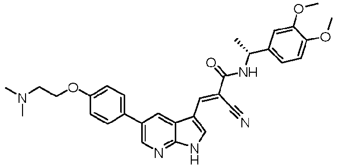 | (R, E)-2-cyano-N-(1-(3,4-dimethoxyphenyl)ethyl)-3-(5-(2-methoxy-4-(4-methylpiperazin-1-yl)phenyl)-1H-pyrrolo[2,3-b]pyridin-3-yl)acrylamide |
| **155** | 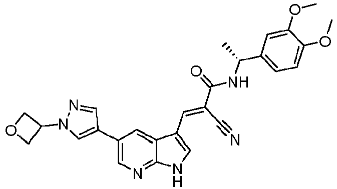 | (R, E)-2-cyano-N-(1-(3,4-dimethoxyphenyl)ethyl)-3-(5-(4-(2-(dimethylamino)ethoxy)phenyl)-1H-pyrrolo[2,3-b]pyridin-3-yl)acrylamide |
| **156** | 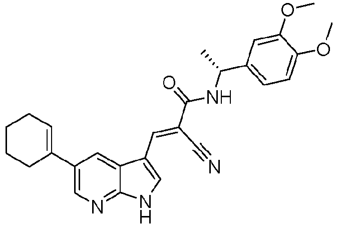 | (R,E)-2-cyano-3-(5-(cyclohex-1-en-1-yl)-1H-pyrrolo[2,3-b]pyridin-3-yl)-N-(1-(3,4-dimethoxyphenyl)ethyl)acrylamide |
| **157** | 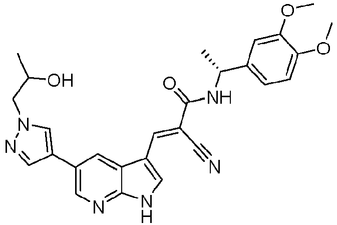 | (R,E)-2-cyano-3-(5-(cyclohex-1-en-1-yl)-1H-pyrrolo[2,3-b]pyridin-3-yl)-N-(1-(3,4-dimethoxyphenyl)ethyl)acrylamide |
| **158** | 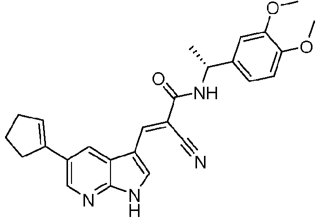 | (R,E)-2-cyano-3-(5-(cyclopent-1-en-1-yl)-1H-pyrrolo[2,3-b]pyridin-3-yl)-N-(1-(3,4-dimethoxyphenyl)ethyl)acrylamide |
| **159** | 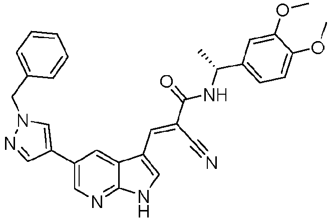 | (R,E)-2-cyano-3-(5-(cyclopent-1-en-1-yl)-1H-pyrrolo[2,3-b]pyridin-3-yl)-N-(1-(3,4-dimethoxyphenyl)ethyl)acrylamide |
| **160** | 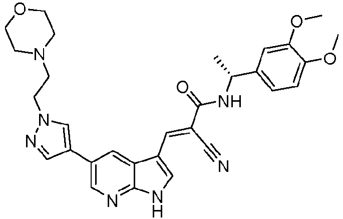 | (R,E)-3-(5-(1-benzyl-1H-pyrazol-4-yl)-1H-pyrrolo[2,3-b]pyridin-3-yl)-2-cyano-N-(1-(3,4-dimethoxyphenyl)ethyl)acrylamide |
| **161** | 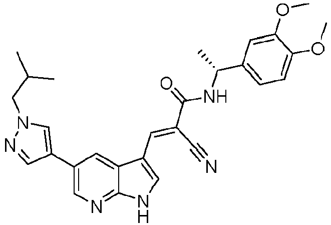 | (R,E)-2-cyano-N-(1-(3,4-dimethoxyphenyl)ethyl)-3-(5-(1-isobutyl-1H-pyrazol-4-yl)-1H-pyrrolo[2,3-b]pyridin-3-yl)acrylamide |
| **162** | 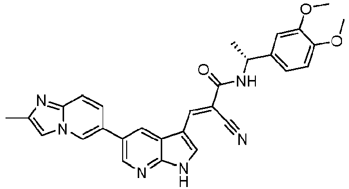 | (R,E)-2-cyano-N-(1-(3,4-dimethoxyphenyl)ethyl)-3-(5-(1-isobutyl-1H-pyrazol-4-yl)-1H-pyrrolo[2,3-b]pyridin-3-yl)acrylamide |
| **163** | 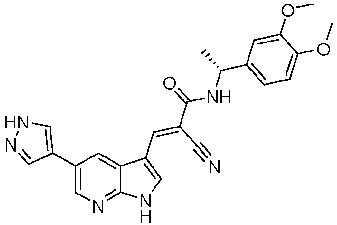 | (R,E)-2-cyano-N-(1-(3,4-dimethoxyphenyl)ethyl)-3-(5-(2-methylimidazo[1,2-a]pyridin-6-yl)-1H-pyrrolo[2,3-b]pyridin-3-yl)acrylamide |
| **164** | 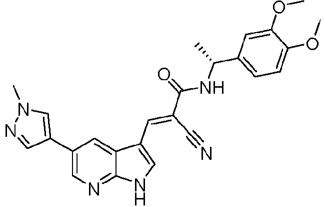 | (R,E)-3-(5-(1H-pyrazol-4-yl)-1H-pyrrolo[2,3-b]pyridin-3-yl)-2-cyano-N-(1-(3,4-dimethoxyphenyl)ethyl)acrylamide |
| **165** | 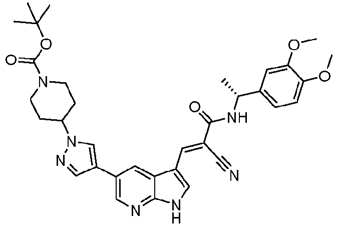 | (R, E)-2-cyano-N-(1-(3,4-dimethoxyphenyl)ethyl)-3-(5-(1-methyl-1H-pyrazol-4-yl)-1H-pyrrolo[2,3-b]pyridin-3-yl)acrylamide |
| **166** | 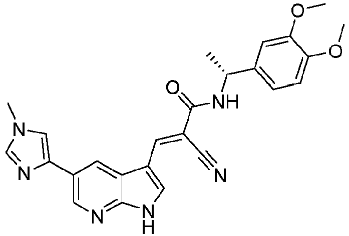 | (R,E)-2-cyano-N-(1-(3,4-dimethoxyphenyl)ethyl)-3-(5-(1-methyl-1H-imidazol-4-yl)-1H-pyrrolo[2,3-b]pyridin-3-yl)acrylamide |
| **167** | 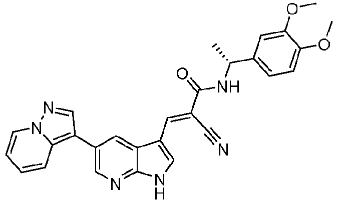 | (R,E)-2-cyano-N-(1-(3,4-dimethoxyphenyl)ethyl)-3-(5-(1-methyl-1H-imidazol-4-yl)-1H-pyrrolo[2,3-b]pyridin-3-yl)acrylamide |
| **168** | 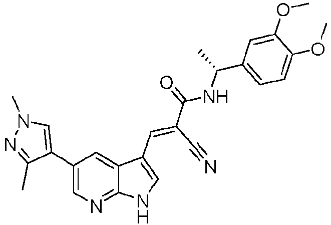 | (R,E)-2-cyano-N-(1-(3,4-dimethoxyphenyl)ethyl)-3-(5-(1,3-dimethyl-1H-pyrazol-4-yl)-1H-pyrrolo[2,3-b]pyridin-3-yl)acrylamide |
| **169** | 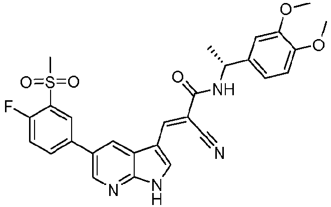 | (R,E)-2-cyano-N-(1-(3,4-dimethoxyphenyl)ethyl)-3-(5-(1,3-dimethyl-1H-pyrazol-4-yl)-1H-pyrrolo[2,3-b]pyridin-3-yl)acrylamide |
| **170** | 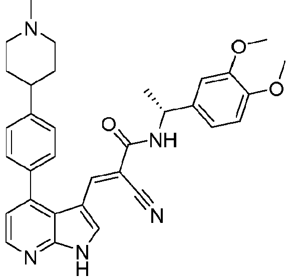 | (R,E)-2-cyano-N-(1-(3,4-dimethoxyphenyl)ethyl)-3-(5-(4-fluoro-3-(methylsulfonyl)phenyl)-1H-pyrrolo[2,3-b]pyridin-3-yl)acrylamide |
| **171** | 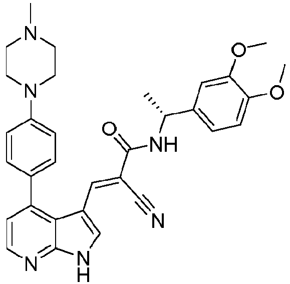 | (R, E)-2-cyano-N-(1-(3,4-dimethoxyphenyl)ethyl)-3-(4-(4-(4-methylpiperazin-1-yl)phenyl)-1H-pyrrolo[2,3-b]pyridin-3-yl)acrylamide |
| **172** | 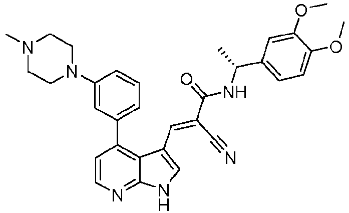 | (R, E)-2-cyano-N-(1-(3,4-dimethoxyphenyl)ethyl)-3-(4-(3-(4-methylpiperazin-1-yl)phenyl)-1H-pyrrolo[2,3-b]pyridin-3-yl)acrylamide |
| **173** | 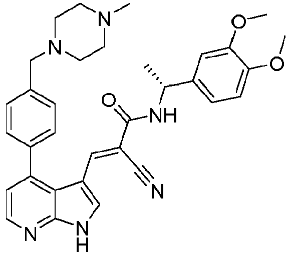 | (R, E)-2-cyano-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamide |
| **174** | 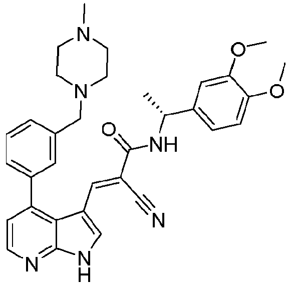 | (R, E)-2-cyano-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamide |
| **175** | 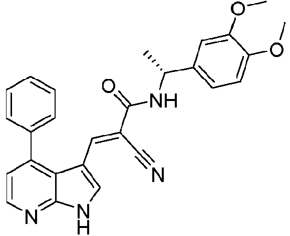 | (R, E)-2-cyano-N-(1-(3,4-dimethoxyphenyl)ethyl)-3-(4-(3-((4-methylpiperazin-1-yl)methyl)phenyl)-1H-pyrrolo[2,3-b]pyridin-3-yl)acrylamide |
| **176** | 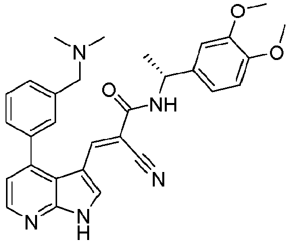 | (R,E)-2-cyano-N-(1-(3,4-dimethoxyphenyl)ethyl)-3-(4-(3-((dimethylamino)methyl)phenyl)-1H-pyrrolo[2,3-b]pyridin-3-yl)acrylamide |
| **177** | 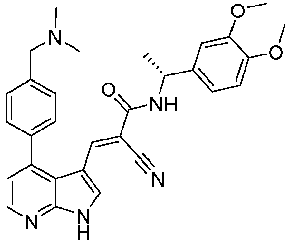 | (R,E)-2-cyano-N-(1-(3,4-dimethoxyphenyl)ethyl)-3-(4-(3-((dimethylamino)methyl)phenyl)-1H-pyrrolo[2,3-b]pyridin-3-yl)acrylamide |
| **178** | 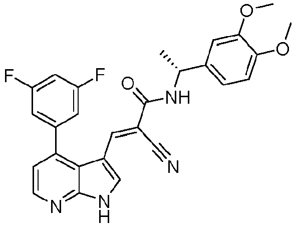 | (R,E)-2-cyano-N-(1-(3,4-dimethoxyphenyl)ethyl)-3-(4-(4-((dimethylamino)methyl)phenyl)-1H-pyrrolo[2,3-b]pyridin-3-yl)acrylamide |
| **179** | 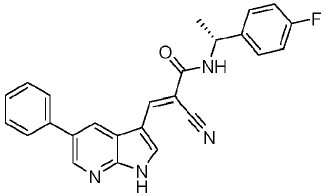 | (R, E)-2-cyano-3-(4-(3,5-difluorophenyl)-1H-pyrrolo[2,3-b]pyridin-3-yl)-N-(1-(3,4-dimethoxyphenyl)ethyl)acrylamide |
| **180** | 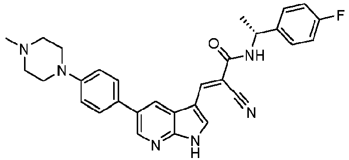 | (R,E)-2-cyano-N-(1-(4-fluorophenyl)ethyl)-3-(5-phenyl-1H-pyrrolo[2,3-b]pyridin-3-yl)acrylamide |
| **181** | 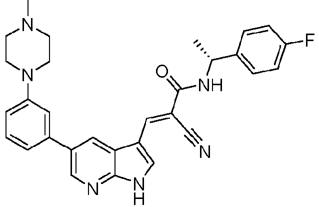 | (R,E)-2-cyano-N-(1-(4-fluorophenyl)ethyl)-3-(5-(3-(4-methylpiperazin-1-yl)phenyl)-1H-pyrrolo[2,3-b]pyridin-3-yl)acrylamide |
| **182** | 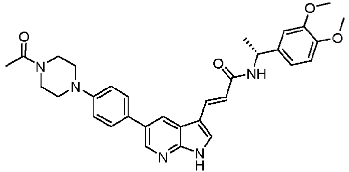 | (R,E)-2-cyano-N-(1-(4-fluorophenyl)ethyl)-3-(5-(3-(4-methylpiperazin-1-yl)phenyl)-1H-pyrrolo[2,3-b]pyridin-3-yl)acrylamide |
| **183** | 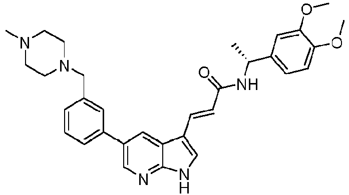 | (R,E)-3-(5-(4-(4-acetylpiperazin-1-yl)phenyl)-1H-pyrrolo[2,3-b]pyridin-3-yl)-N-(1-(3,4-dimethoxyphenyl)ethyl)acrylamide |
| **184** | 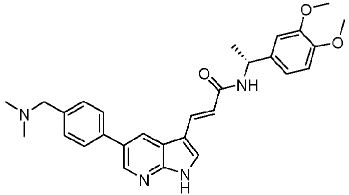 | (R,E)-N-(1-(3,4-dimethoxyphenyl)ethyl)-3-(5-(3-((4-methylpiperazin-1-yl)methyl)phenyl)-1H-pyrrolo[2,3-b]pyridin-3-yl)acrylamide |
| **185** | 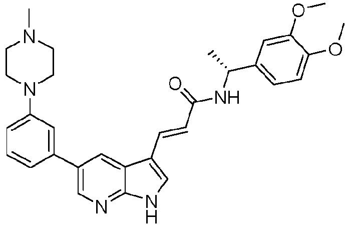 | (R,E)-N-(1-(3,4-dimethoxyphenyl)ethyl)-3-(5-(4-((dimethylamino)methyl)phenyl)-1H-pyrrolo[2,3-b]pyridin-3-yl)acrylamide |
| **186** | 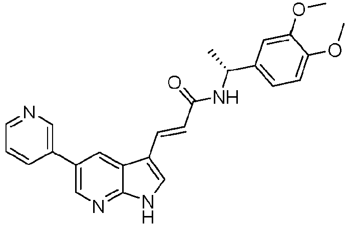 | (R,E)-N-(1-(3,4-dimethoxyphenyl)ethyl)-3-(5-(pyridin-3-yl)-1H-pyrrolo[2,3-b]pyridin-3-yl)acrylamide |
| **187** | 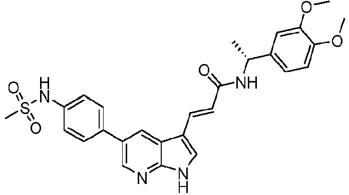 | (R,E)-N-(1-(3,4-dimethoxyphenyl)ethyl)-3-(5-(pyridin-3-yl)-1H-pyrrolo[2,3-b]pyridin-3-yl)acrylamide |
| **188** | 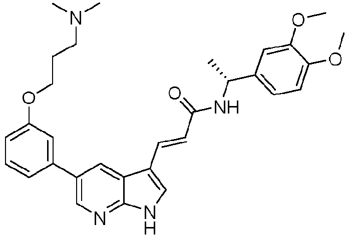 | (R,E)-N-(1-(3,4-dimethoxyphenyl)ethyl)-3-(5-(4-(methylsulfonamido)phenyl)-1H-pyrrolo[2,3-b]pyridin-3-yl)acrylamide |
| **189** | 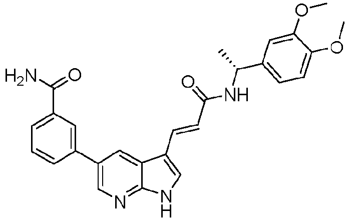 | (R,E)-N-(1-(3,4-dimethoxyphenyl)ethyl)-3-(5-(3-(3-(dimethylamino)propoxy)phenyl)-1H-pyrrolo[2,3-b]pyridin-3-yl)acrylamide |
| **190** | 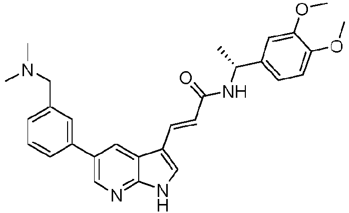 | (R,E)-3-(3-(3-((1-(3,4-dimethoxyphenyl)ethyl)amino)-3-oxoprop-1-en-1-yl)-1H-pyrrolo[2,3-b]pyridin-5-yl)benzamide |
| **191** | 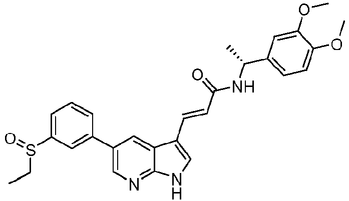 | (E)-N-((R)-1-(3,4-dimethoxyphenyl)ethyl)-3-(5-(3-(ethylsulfinyl)phenyl)-1H-pyrrolo[2,3-b]pyridin-3-yl)acrylamide |
| **192** | 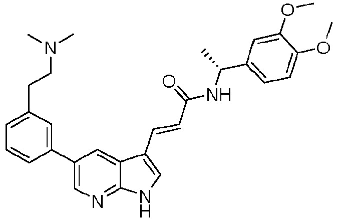 | (E)-N-((R)-1-(3,4-dimethoxyphenyl)ethyl)-3-(5-(3-(ethylsulfinyl)phenyl)-1H-pyrrolo[2,3-b]pyridin-3-yl)acrylamide |
| **193** | 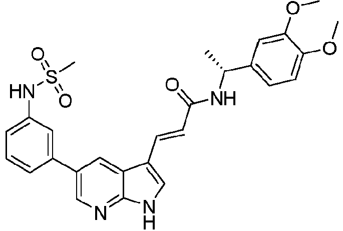 | (R,E)-N-(1-(3,4-dimethoxyphenyl)ethyl)-3-(5-(3-(2-(dimethylamino)ethyl)phenyl)-1H-pyrrolo[2,3-b]pyridin-3-yl)acrylamide |
| **194** | 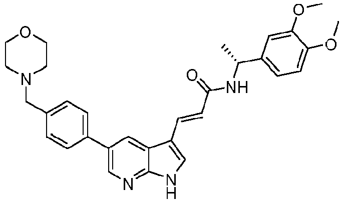 | (R,E)-N-(1-(3,4-dimethoxyphenyl)ethyl)-3-(5-(3-(methylsulfonamido)phenyl)-1H-pyrrolo[2,3-b]pyridin-3-yl)acrylamide |
| **195** | 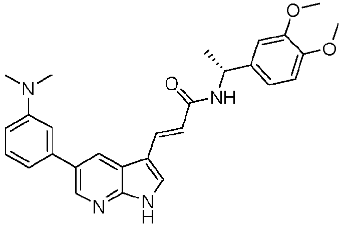 | (R,E)-N-(1-(3,4-dimethoxyphenyl)ethyl)-3-(5-(3-(dimethylamino)phenyl)-1H-pyrrolo[2,3-b]pyridin-3-yl)acrylamide |
| **196** | 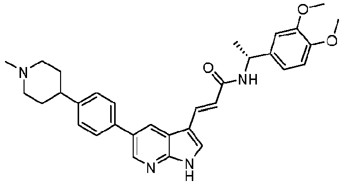 | (R,E)-N-(1-(3,4-dimethoxyphenyl)ethyl)-3-(5-(4-(1-methylpiperidin-4-yl)phenyl)-1H-pyrrolo[2,3-b]pyridin-3-yl)acrylamide |
| **197** | 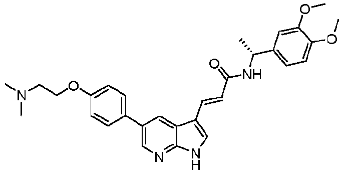 | (R,E)-N-(1-(3,4-dimethoxyphenyl)ethyl)-3-(5-(4-(2-(dimethylamino)ethoxy)phenyl)-1H-pyrrolo[2,3-b]pyridin-3-yl)acrylamide |
| **198** | 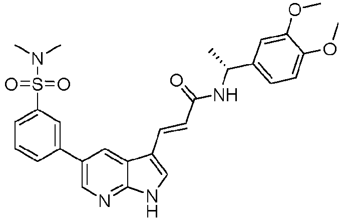 | (R,E)-N-(1-(3,4-dimethoxyphenyl)ethyl)-3-(5-(3-(N,N-dimethylsulfamoyl)phenyl)-1H-pyrrolo[2,3-b]pyridin-3-yl)acrylamide |
| **199** | 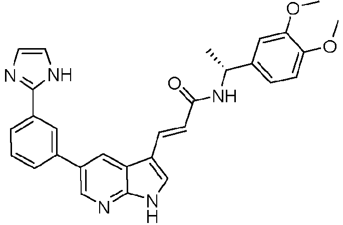 | (R,E)-3-(5-(3-(1H-imidazol-2-yl)phenyl)-1 H-pyrrolo[2,3-b]pyridin-3-yl)-N-(1-(3,4-dimethoxyphenyl)ethyl)acrylamide |
| **200** | 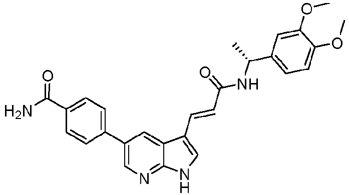 | (R,E)-4-(3-(3-((1-(3,4-dimethoxyphenyl)ethyl)amino)-3-oxoprop-1-en-1-yl)-1H-pyrrolo[2,3-b]pyridin-5-yl)benzamide |
| **201** | 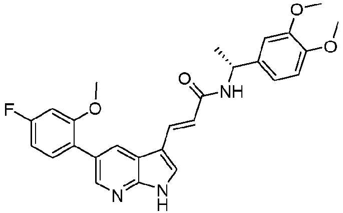 | (R,E)-N-(1-(3,4-dimethoxyphenyl)ethyl)-3-(5-(4-fluoro-2-methoxyphenyl)-1H-pyrrolo[2,3-b]pyridin-3-yl)acrylamide |
| **202** | 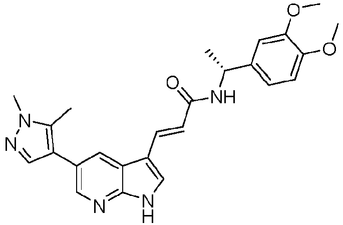 | (R,E)-N-(1-(3,4-dimethoxyphenyl)ethyl)-3-(5-(1,5-dimethyl-1H-pyrazol-4-yl)-1H-pyrrolo[2,3-b]pyridin-3-yl)acrylamide |
| **203** | 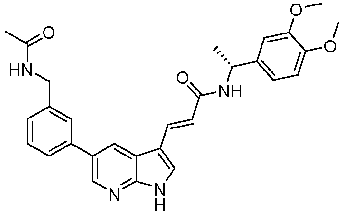 | (R,E)-3-(5-(3-(acetamidomethyl)phenyl)-1H-pyrrolo[2,3-b]pyridin-3-yl)-N-(1-(3,4-dimethoxyphenyl)ethyl)acrylamide |
| **204** | 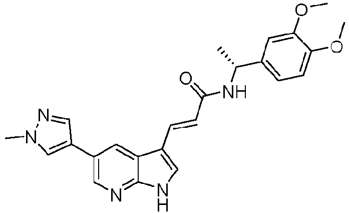 | (R,E)-N-(1-(3,4-dimethoxyphenyl)ethyl)-3-(5-(1-methyl-1H-pyrazol-4-yl)-1H-pyrrolo[2,3-b]pyridin-3-yl)acrylamide |
| **205** | 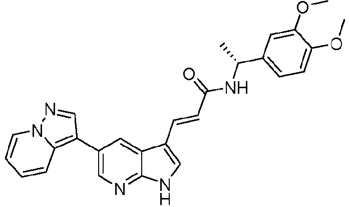 | (R,E)-N-(1-(3,4-dimethoxyphenyl)ethyl)-3-(5-(pyrazolo[1,5-a]pyridin-3-yl)-1H-pyrrolo[2,3-b]pyridin-3-yl)acrylamide |
| **206** | 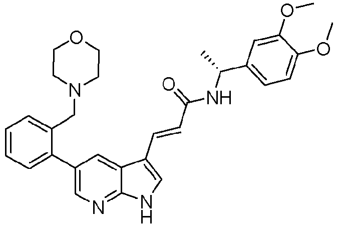 | (R,E)-N-(1-(3,4-dimethoxyphenyl)ethyl)-3-(5-(2-(morpholinomethyl)phenyl)-1H-pyrrolo[2,3-b]pyridin-3-yl)acrylamide |
| **207** | 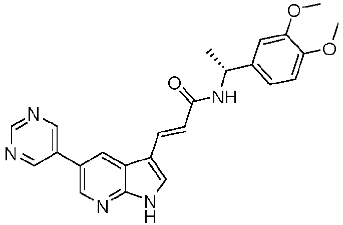 | (R,E)-N-(1-(3,4-dimethoxyphenyl)ethyl)-3-(5-(pyrimidin-5-yl)-1H-pyrrolo[2,3-b]pyridin-3-yl)acrylamide |
| **208** | 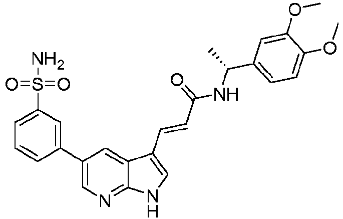 | (R,E)-N-(1-(3,4-dimethoxyphenyl)ethyl)-3-(5-(3-sulfamoylphenyl)-1H-pyrrolo[2,3-b]pyridin-3-yl)acrylamide |
| **209** | 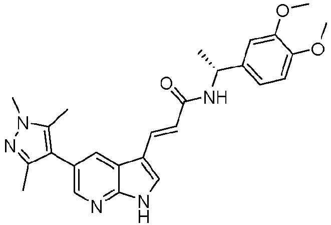 | (R,E)-N-(1-(3,4-dimethoxyphenyl)ethyl)-3-(5-(1,3,5-trimethyl-1H-pyrazol-4-yl)-1H-pyrrolo[2,3-b]pyridin-3-yl)acrylamide |
| **210** | 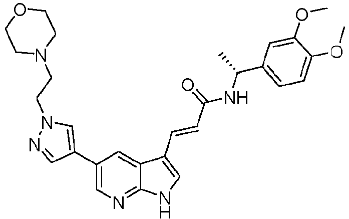 | (R,E)-N-(1-(3,4-dimethoxyphenyl)ethyl)-3-(5-(1-(2-morpholinoethyl)-1H-pyrazol-4-yl)-1H-pyrrolo[2,3-b]pyridin-3-yl)acrylamide |
| **211** | 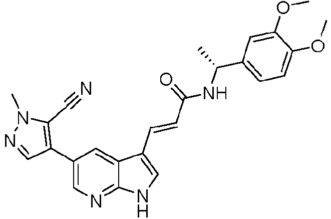 | (R,E)-3-(5-(5-cyano-1-methyl-1H-pyrazol-4-yl)-1H-pyrrolo[2,3-b]pyridin-3-yl)-N-(1-(3,4-dimethoxyphenyl)ethyl)acrylamide |
| **212** | 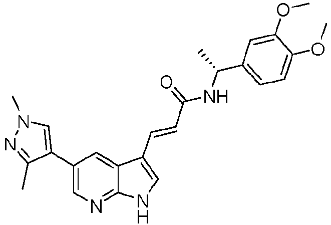 | (R,E)-N-(1-(3,4-dimethoxyphenyl)ethyl)-3-(5-(1,3-dimethyl-1H-pyrazol-4-yl)-1H-pyrrolo[2,3-b]pyridin-3-yl)acrylamide |
| **213** | 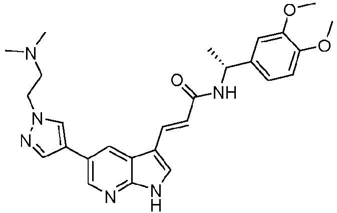 | (R,E)-N-(1-(3,4-dimethoxyphenyl)ethyl)-3-(5-(1-(2-(dimethylamino)ethyl)-1H-pyrazol-4-yl)-1H-pyrrolo[2,3-b]pyridin-3-yl)acrylamide |
| **214** | 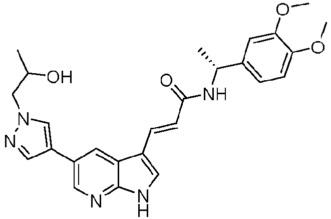 | (E)-N-((R)-1-(3,4-dimethoxyphenyl)ethyl)-3-(5-(1-(2-hydroxypropyl)-1H-pyrazol-4-yl)-1H-pyrrolo[2,3-b]pyridin-3-yl)acrylamide |
| **215** | 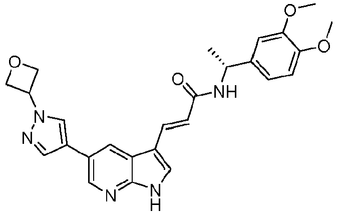 | (R,E)-N-(1-(3,4-dimethoxyphenyl)ethyl)-3-(5-(1-(oxetan-3-yl)-1H-pyrazol-4-yl)-1H-pyrrolo[2,3-b]pyridin-3-yl)acrylamide |
| **216** | 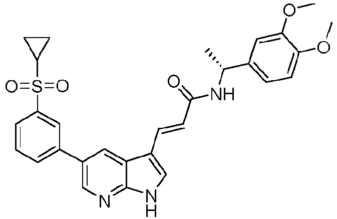 | (R,E)-3-(5-(3-(cyclopropylsulfonyl)phenyl)-1H-pyrrolo[2,3-b]pyridin-3-yl)-N-(1-(3,4-dimethoxyphenyl)ethyl)acrylamide |
| **217** | 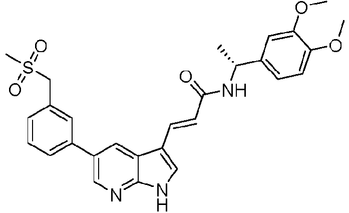 | (R,E)-N-(1-(3,4-dimethoxyphenyl)ethyl)-3-(5-(3-((methylsulfonyl)methyl)phenyl)-1H-pyrrolo[2,3-b]pyridin-3-yl)acrylamide |
| **218** | 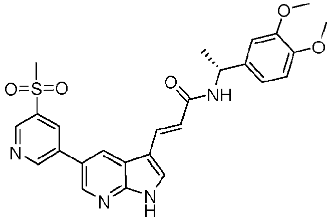 | (R,E)-N-(1-(3,4-dimethoxyphenyl)ethyl)-3-(5-(5-(methylsulfonyl)pyridin-3-yl)-1H-pyrrolo[2,3-b]pyridin-3-yl)acrylamide |
| **219** | 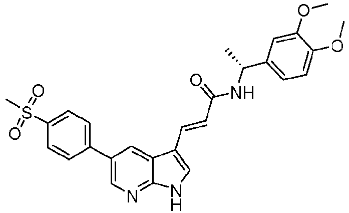 | (R,E)-N-(1-(3,4-dimethoxyphenyl)ethyl)-3-(5-(4-(methylsulfonyl)phenyl)-1H-pyrrolo[2,3-b]pyridin-3-yl)acrylamide |
| **220** | 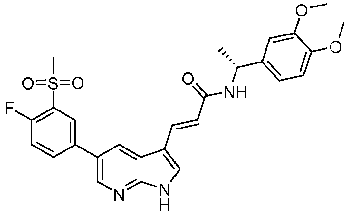 | (R,E)-N-(1-(3,4-dimethoxyphenyl)ethyl)-3-(5-(4-fluoro-3-(methylsulfonyl)phenyl)-1H-pyrrolo[2,3-b]pyridin-3-yl)acrylamide |
| **221** | 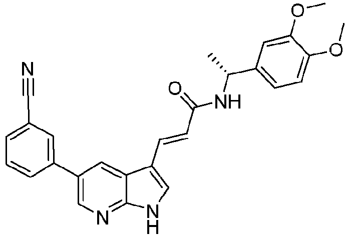 | (R,E)-3-(5-(3-cyanophenyl)-1H-pyrrolo[2,3-b]pyridin-3-yl)-N-(1-(3,4-dimethoxyphenyl)ethyl)acrylamide |
| **222** | 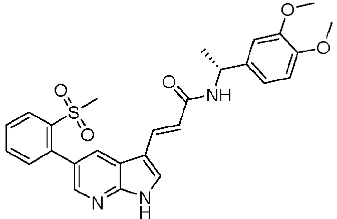 | (R,E)-N-(1-(3,4-dimethoxyphenyl)ethyl)-3-(5-(2-(methylsulfonyl)phenyl)-1H-pyrrolo[2,3-b]pyridin-3-yl)acrylamide |
| **223** | 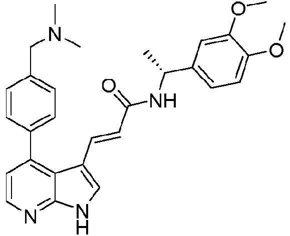 | (R,E)-N-(1-(3,4-dimethoxyphenyl)ethyl)-3-(4-(4-((dimethylamino)methyl)phenyl)-1H-pyrrolo[2,3-b]pyridin-3-yl)acrylamide |
| **224** | 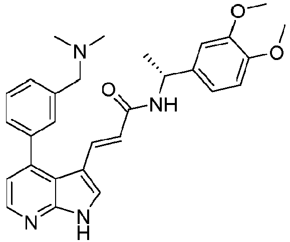 | (R,E)-N-(1-(3,4-dimethoxyphenyl)ethyl)-3-(4-(3-((dimethylamino)methyl)phenyl)-1H-pyrrolo[2,3-b]pyridin-3-yl)acrylamide |
| **225** | 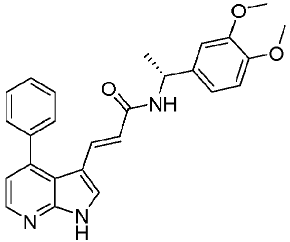 | (R, E)-N-(1-(3,4-dimethoxyphenyl)ethyl)-3-(4-phenyl-1H-pyrrolo[2,3-b]pyridin-3-yl)acrylamide |
| **226** | 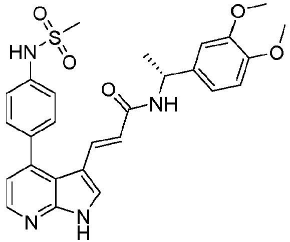 | (R,E)-N-(1-(3,4-dimethoxyphenyl)ethyl)-3-(4-(4-(methylsulfonamido)phenyl)-1H-pyrrolo[2,3-b]pyridin-3-yl)acrylamide |
| **227** | 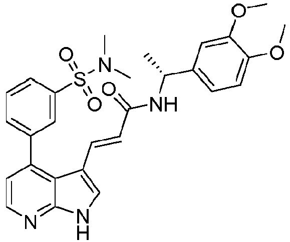 | (R,E)-N-(1-(3,4-dimethoxyphenyl)ethyl)-3-(4-(3-(N,N-dimethylsulfamoyl)phenyl)-1H-pyrrolo[2,3-b]pyridin-3-yl)acrylamide |
| **228** | 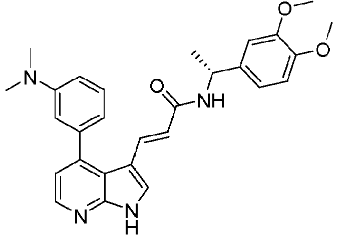 | (R,E)-N-(1-(3,4-dimethoxyphenyl)ethyl)-3-(4-(3-(dimethylamino)phenyl)-1H-pyrrolo[2,3-b]pyridin-3-yl)acrylamide |
| **229** | 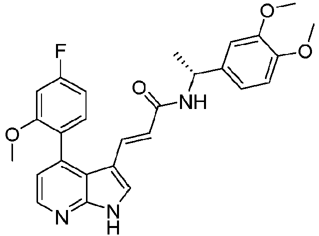 | (R,E)-N-(1-(3,4-dimethoxyphenyl)ethyl)-3-(4-(4-fluoro-2-methoxyphenyl)-1H-pyrrolo[2,3-b]pyridin-3-yl)acrylamide |
| **230** | 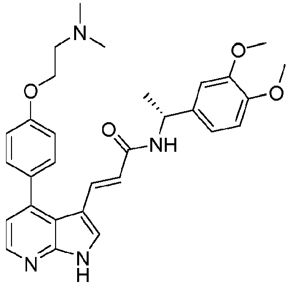 | (R,E)-N-(1-(3,4-dimethoxyphenyl)ethyl)-3-(4-(4-(2-(dimethylamino)ethoxy)phenyl)-1H-pyrrolo[2,3-b]pyridin-3-yl)acrylamide |
| **231** | 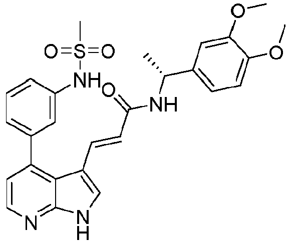 | (R,E)-N-(1-(3,4-dimethoxyphenyl)ethyl)-3-(4-(3-(methylsulfonamido)phenyl)-1H-pyrrolo[2,3-b]pyridin-3-yl)acrylamide |
| **232** | 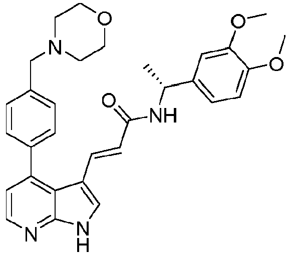 | (R,E)-N-(1-(3,4-dimethoxyphenyl)ethyl)-3-(4-(4-(morpholinomethyl)phenyl)-1H-pyrrolo[2,3-b]pyridin-3-yl)acrylamide |
| **233** | 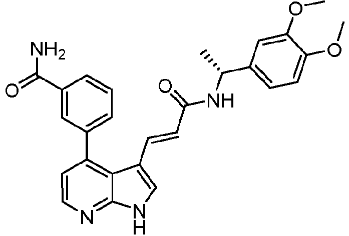 | (R, E)-3-(3-(3-((1-(3,4-dimethoxyphenyl)ethyl)amino)-3-oxoprop-1-en-1-yl)-1H-pyrrolo[2,3-b]pyridin-4-yl)benzamide |
| **234** | 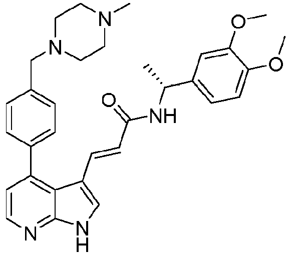 | (R,E)-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamide |
| **235** | 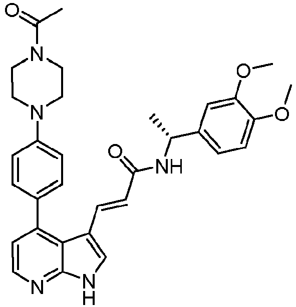 | (R,E)-3-(4-(4-(4-acetylpiperazin-1-yl)phenyl)-1H-pyrrolo[2,3-b]pyridin-3-yl)-N-(1-(3,4-dimethoxyphenyl)ethyl)acrylamide |
| **236** | 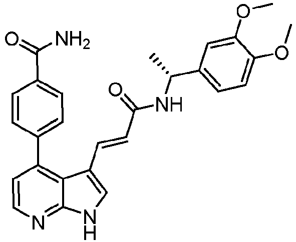 | (R,E)-4-(3-(3-((1-(3,4-dimethoxyphenyl)ethyl)amino)-3-oxoprop-1-en-1-yl)-1H-pyrrolo[2,3-b]pyridin-4-yl)benzamide |
| **237** | 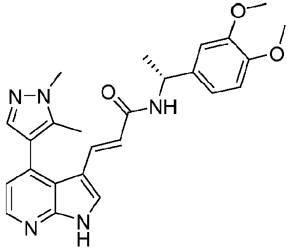 | (R,E)-N-(1-(3,4-dimethoxyphenyl)ethyl)-3-(4-(1,5-dimethyl-1H-pyrazol-4-yl)-1H-pyrrolo[2,3-b]pyridin-3-yl)acrylamide |
| **238** | 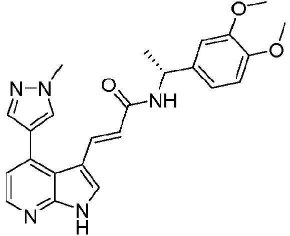 | (R,E)-N-(1-(3,4-dimethoxyphenyl)ethyl)-3-(4-(1-methyl-1H-pyrazol-4-yl)-1H-pyrrolo[2,3-b]pyridin-3-yl)acrylamide |
| **239** | 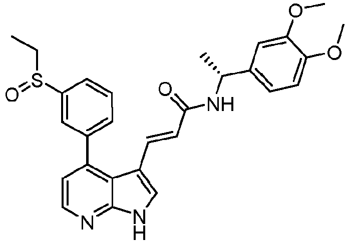 | (E)-N-((R)-1-(3,4-dimethoxyphenyl)ethyl)-3-(4-(3-(ethylsulfinyl)phenyl)-1H-pyrrolo[2,3-b]pyridin-3-yl)acrylamide |
| **240** | 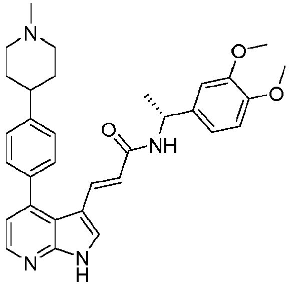 | (R,E)-N-(1-(3,4-dimethoxyphenyl)ethyl)-3-(4-(4-(1-methylpiperidin-4-yl)phenyl)-1H-pyrrolo[2,3-b]pyridin-3-yl)acrylamide |
| **241** | 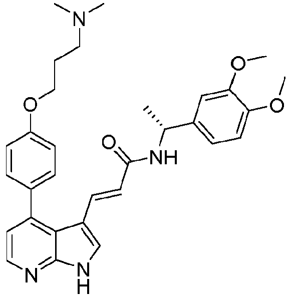 | (R,E)-N-(1-(3,4-dimethoxyphenyl)ethyl)-3-(4-(4-(3-(dimethylamino)propoxy)phenyl)-1H-pyrrolo[2,3-b]pyridin-3-yl)acrylamide |
| **242** | 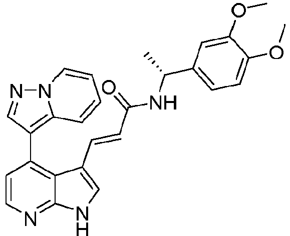 | (R,E)-N-(1-(3,4-dimethoxyphenyl)ethyl)-3-(4-(pyrazolo[1,5-a]pyridin-3-yl)-1H-pyrrolo[2,3-b]pyridin-3-yl)acrylamide |
| **243** | 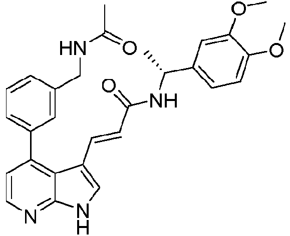 | (R,E)-3-(4-(3-(acetamidomethyl)phenyl)-1H-pyrrolo[2,3-b]pyridin-3-yl)-N-(1-(3,4-dimethoxyphenyl)ethyl)acrylamide |
| **244** | 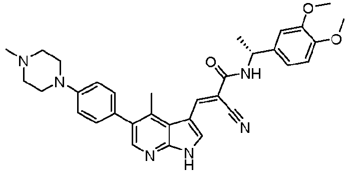 | (R,E)-2-cyano-N-(1-(3,4-dimethoxyphenyl)ethyl)-3-(4-methyl-5-(4-(4-methylpiperazin-1-yl)phenyl)-1H-pyrrolo[2,3-b]pyridin-3-yl)acrylamide |
| **245** | 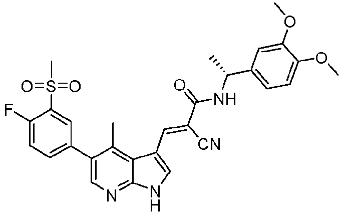 | (R,E)-2-cyano-N-(1-(3,4-dimethoxyphenyl)ethyl)-3-(5-(4-fluoro-3-(methylsulfonyl)phenyl)-4-methyl-1H-pyrrolo[2,3-b]pyridin-3-yl)acrylamide |
| **246** | 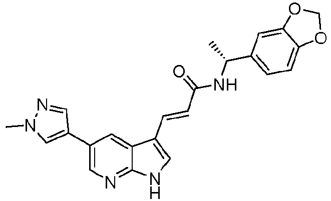 | (R,E)-N-(1-(benzo[d][1,3]dioxol-5-yl)ethyl)-3-(5-(1-methyl-1H-pyrazol-4-yl)-1H-pyrrolo[2,3-b]pyridin-3-yl)acrylamide |
| **247** | 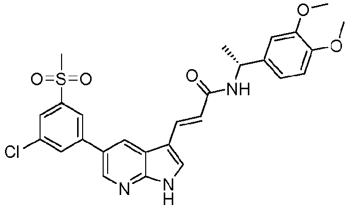 | (R, E)-3-(5-(3-chloro-5-(methylsulfonyl)phenyl)-1H-pyrrolo[2,3-b]pyridin-3-yl)-N-(1-(3,4-dimethoxyphenyl)ethyl)acrylamide |
| **248** | 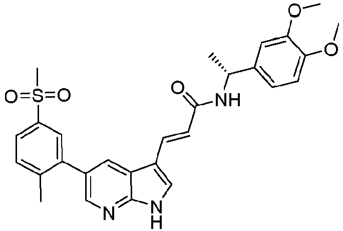 | (R, E)-N-(1-(3,4-dimethoxyphenyl)ethyl)-3-(5-(2-methyl-5-(methylsulfonyl)phenyl)-1H-pyrrolo[2,3-b]pyridin-3-yl)acrylamide |
| **249** | 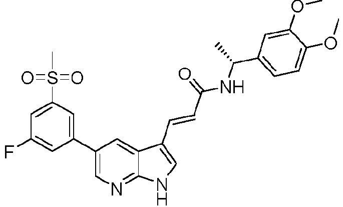 | (R,E)-N-(1-(3,4-dimethoxyphenyl)ethyl)-3-(5-(3-fluoro-5-(methylsulfonyl)phenyl)-1H-pyrrolo[2,3-b]pyridin-3-yl)acrylamide |
| **250** | 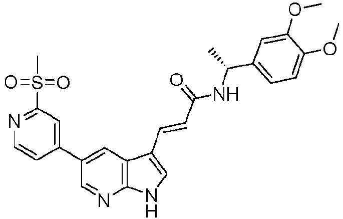 | (R,E)-N-(1-(3,4-dimethoxyphenyl)ethyl)-3-(5-(2-(methylsulfonyl)pyridin-4-yl)-1H-pyrrolo[2,3-b]pyridin-3-yl)acrylamide |
| **251** | 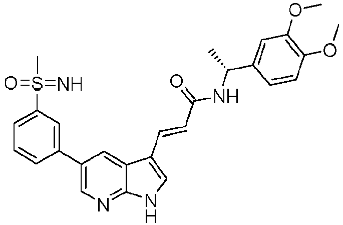 | (E)-N-((R)-1-(3,4-dimethoxyphenyl)ethyl)-3-(5-(3-(S-methylsulfonimidoyl)phenyl)-1H-pyrrolo[2,3-b]pyridin-3-yl)acrylamide |
| **252** | 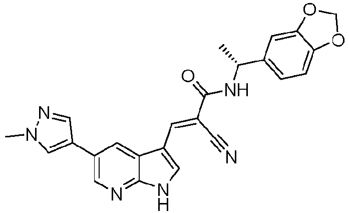 | (R,E)-N-(1-(benzo[d][1,3]dioxol-5-yl)ethyl)-2-cyano-3-(5-(1-methyl-1H-pyrazol-4-yl)-1H-pyrrolo[2,3-b]pyridin-3-yl)acrylamide |
| **253** | 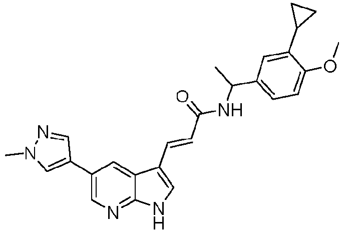 | (E)-N-(1-(3-cyclopropyl-4-methoxyphenyl)ethyl)-3-(5-(1-methyl-1H-pyrazol-4-yl)-1H-pyrrolo[2,3-b]pyridin-3-yl)acrylamide |
| **254** | 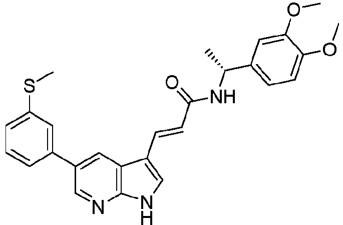 | (R,E)-N-(1-(3,4-dimethoxyphenyl)ethyl)-3-(5-(3-(methylthio)phenyl)-1H-pyrrolo[2,3-b]pyridin-3-yl)acrylamide |
| **255** | 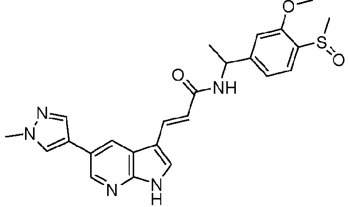 | (E)-N-(1-(3-methoxy-4-(methylsulfinyl)phenyl)ethyl)-3-(5-(1-methyl-1H-pyrazol-4-yl)-1H-pyrrolo[2,3-b]pyridin-3-yl)acrylamide |
| **256** | 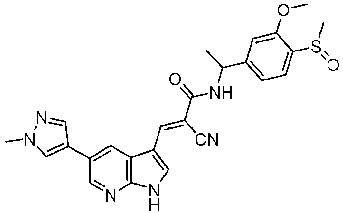 | (E)-2-cyano-N-(1-(3-methoxy-4-(methylsulfinyl)phenyl)ethyl)-3-(5-(1-methyl-1H-pyrazol-4-yl)-1H-pyrrolo[2,3-b]pyridin-3-yl)acrylamide |
| **257** | 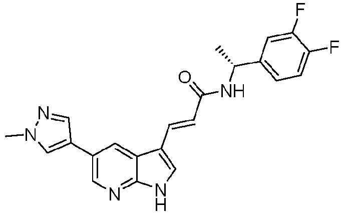 | (R,E)-N-(1-(3,4-difluorophenyl)ethyl)-3-(5-(1-methyl-1H-pyrazol-4-yl)-1H-pyrrolo[2,3-b]pyridin-3-yl)acrylamide |
| **258** | 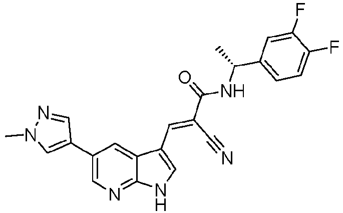 | (R,E)-2-cyano-N-(1-(3,4-difluorophenyl)ethyl)-3-(5-(1-methyl-1H-pyrazol-4-yl)-1H-pyrrolo[2,3-b]pyridin-3-yl)acrylamide |
| **259** | 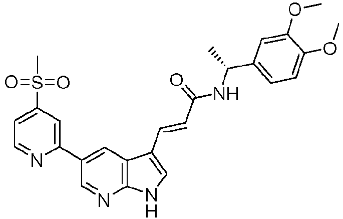 | (R,E)-N-(1-(3,4-dimethoxyphenyl)ethyl)-3-(5-(4-(methylsulfonyl)pyridin-2-yl)-1H-pyrrolo[2,3-b]pyridin-3-yl)acrylamide |
| **260** | 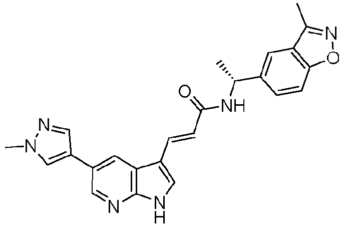 | (R,E)-3-(5-(1-methyl-1H-pyrazol-4-yl)-1H-pyrrolo[2,3-b]pyridin-3-yl)-N-(1-(3-methylbenzo[d]isoxazol-5-yl)ethyl)acrylamide |
| **261** | 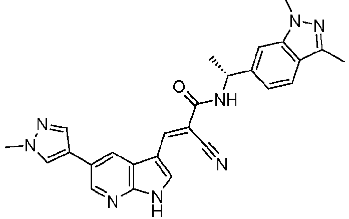 | (R,E)-2-cyano-N-(1-(1,3-dimethyl-1H-indazol-6-yl)ethyl)-3-(5-(1-methyl-1H-pyrazol-4-yl)-1H-pyrrolo[2,3-b]pyridin-3-yl)acrylamide |
| **262** | 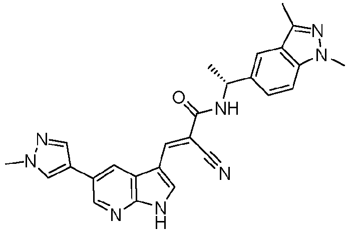 | (R,E)-2-cyano-N-(1-(1,3-dimethyl-1H-indazol-5-yl)ethyl)-3-(5-(1-methyl-1H-pyrazol-4-yl)-1H-pyrrolo[2,3-b]pyridin-3-yl)acrylamide |
| **263** | 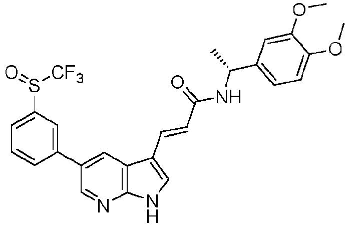 | (E)-N-((R)-1-(3,4-dimethoxyphenyl)ethyl)-3-(5-(3-((trifluoromethyl)sulfinyl)phenyl)-1H-pyrrolo[2,3-b]pyridin-3-yl)acrylamide |
| **264** | 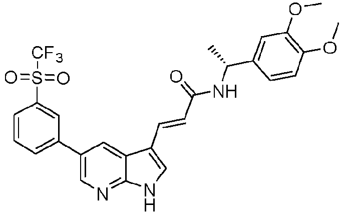 | (R,E)-N-(1-(3,4-dimethoxyphenyl)ethyl)-3-(5-(3-((trifluoromethyl)sulfonyl)phenyl)-1H-pyrrolo[2,3-b]pyridin-3-yl)acrylamide |
| **265** | 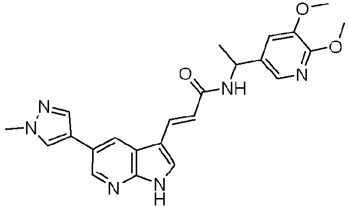 | (E)-N-(1-(5,6-dimethoxypyridin-3-yl)ethyl)-3-(5-(1-methyl-1H-pyrazol-4-yl)-1H-pyrrolo[2,3-b]pyridin-3-yl)acrylamide |

### Syntheses of compounds

The compound of formula (I) is prepared by reference to the methods illustrated in the following schemes 1-4. The compound of formula (I) is produced by Schemes 1-4 by the suitable selection of reagents with appropriate substitutions. Solvents, temperatures, pressures, and other reaction conditions may readily be selected by one of ordinary skill in the art. Starting materials are commercially available or readily prepared by one of ordinary skill in the art.

As shown in Scheme 1, in some embodiments, the present invention is directed to a method for producing the compound of the formula (I) comprising:
**Step 1:** providing 1H-pyrrolo[2,3-b]pyridine-3-carbaldehyde compound (**2***) 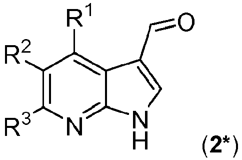
**Step 2:** performing a condensation reaction of compound (**2***) with a compound (**3***) 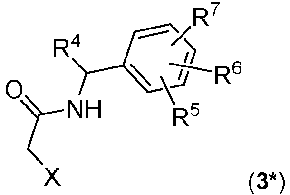 to obtain the compound of the formula (la) 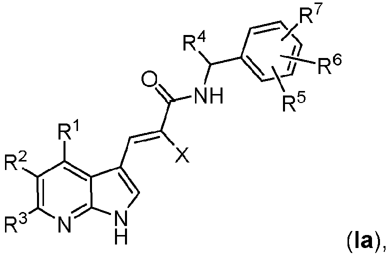 wherein **R¹, R²**, **R³, R⁴, R⁵**, **R⁶, R⁷** and **X** have the same meanings as defined in the formula (I);
   or
**Step1:** providing 1H-pyrrolo[2,3-b]pyridine-3-carbaldehyde compound (**2***) 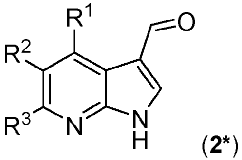
**Step2a:** performing a condensation reaction of compound (**2***) with maleic acid or cyano acetic acid to obtain acrylic acid compound (**4***) 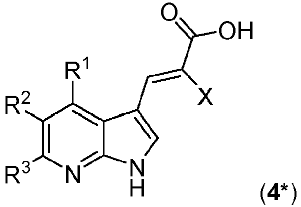
**Step 3**: performing a coupling reaction of compound (**4***) with an amine compound (**5***) 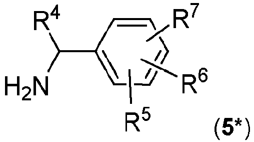 to obtain the compound of the formula (**Ia**) 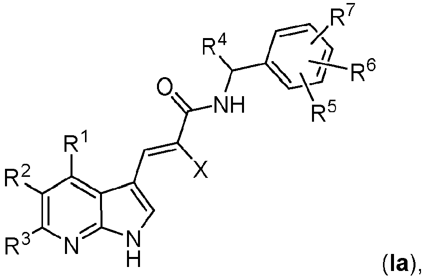 wherein **R¹**, **R²**, **R³, R⁴, R⁵**, **R⁶, R⁷** and **X** have the same meanings as defined in the formula (**I**).

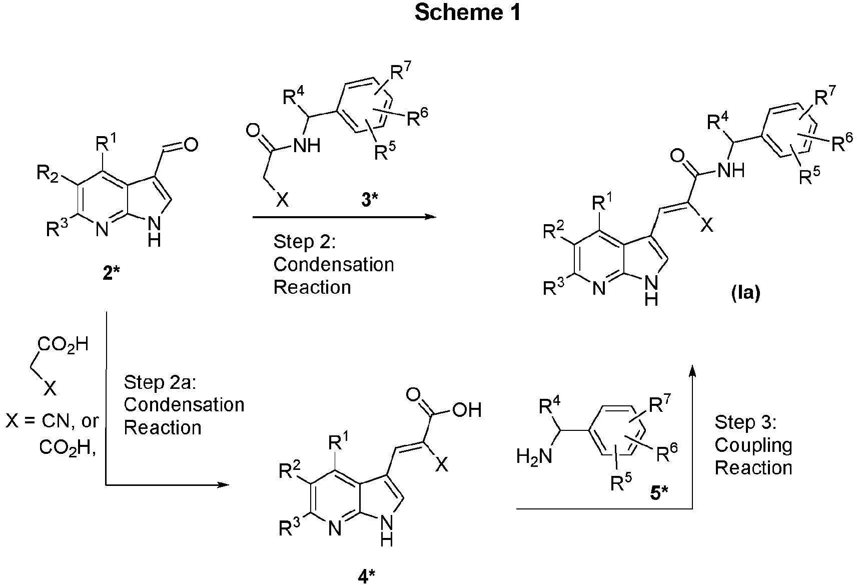

In each of the steps **2**, and **2a** condensation reaction is performed in the present of an amine such as pyridine, piperidine and a mixture thereof. The molar ratio of compound **2*** and compound **3*** is 1:1 to 1:3, preferred 1:1 to 1:2, more preferred, 1:1 to 1:1.5, most preferred 1:1.2 to 1:1.5. The molar ratio of compound **2*** and maleic acid or cyanoacetic acid is 1:1 to 1:5, preferred 1:1 to 1:4, more preferred, 1:2 to 1:4, most preferred 1:2 to 1:3. The reaction temperature is in a range of 70°C to 150°C, preferred 80°C to 130°C, more preferred 90°C to 120°C, and most preferred 100°C to 120°C.

In each of the steps **3**, the amide coupling reaction is performed in the presence of a coupling reagent and a base in an aprotic solvent. The coupling reagent activates a carboxyl acid of compound (**4***) and promote the coupling reaction with an amino group of the compound (**5***). Any of the following coupling reagent can be used to activate the carboxylic group: BOP, PyBOP, AOP, PyAOP, TBTU, EEDQ, Polyphosphoric Acid (PPA), DPPA, HATU, HOBt, HOAt, DCC, EDCI, BOP-Cl, TFFH, Brop, PyBrop, and CIP, preferred HATU and HOBt. The base may be a tertiary amine such as Et₃N, and DIPEA, DABCO, DBU, pyrrolidine or piperidine. The molar ratio of the compound (**4***) and compound **5*** is in a range of 1:1 to 1:2, preferred 1:1 to 1.5, and the molar ratio of the comopund (**4***) and the coupling reagent is in a range of 1:1 to 1:2, preferred 1:1 to 1:1.5. The molar ratio of the comopund (**4***) and the base is in a range of 1:1 to 1:5, preferred 1:2 to 1:4. The aprotic solvent is DMF, acetonirile, dichloromethane, chlorform, THF, dioxane, or a mixture thereof. The reaction temperature is in a range of - 20°C to 30°C, preferred -10°C to 30°C, more preffered -10°C to 20°C, and most preferred -10°C to 10°C.

An alternative synthetic route to obtain the product compound I**a** is shown in Scheme 2.

**Step4A:** Performing a Mitsunobu reaction of compound **6*** with alcohol compound **6** to obtain the product compound (**Ia**) using standard Mitsunobu-conditions.

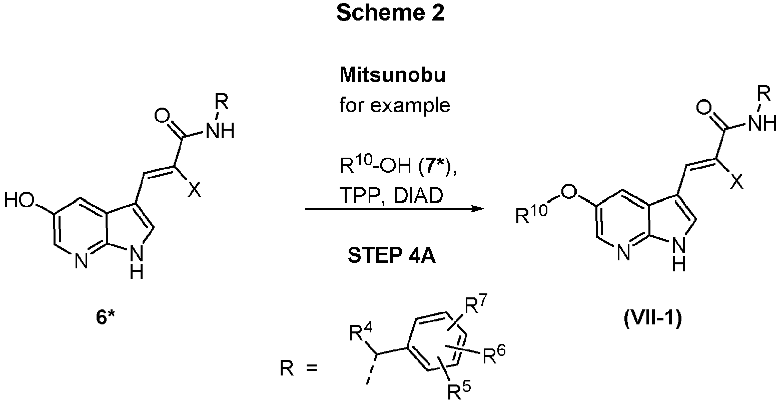

In the Step **4A**, the molar ratio of compound **6*** and compound **7*** is in a range of 1:1 to 1:2, preferred 1:1 to 1:1.5, more preferred 1:1 to 1:1.3. The molar ratio of compound 6* and tripehnylphosphine (TTP) is in a range of 1:1 to 1:5, preferred 1:1 to 1:3, more preferred 1:1 to 1:2. The molar ratio of compound 6* and diisopropyl azodicarboxylate (DIAD) is in a range of 1:1 to 1:3, preferred, 1:1 to 1:2, more preferred 1:1 to 1:1.5. The reaction is pefomred in an aprotic solvent such as dichloromethane, and chlorform,. The reaction temperature is in a range of -20°C to 30°C, preferred -10°C to 30°C, more preffered -10°C to 30°C, and most preferred 0°C to 25°C.

Another method for producing a compound of the formula (**Ia**) is shown in Scheme 3. **Step 4B:** Performing a cross-coupling reaction of the compound **8*** with compound **9*** in the presence of a palladium catalyst and base to obtain the final compound (la).

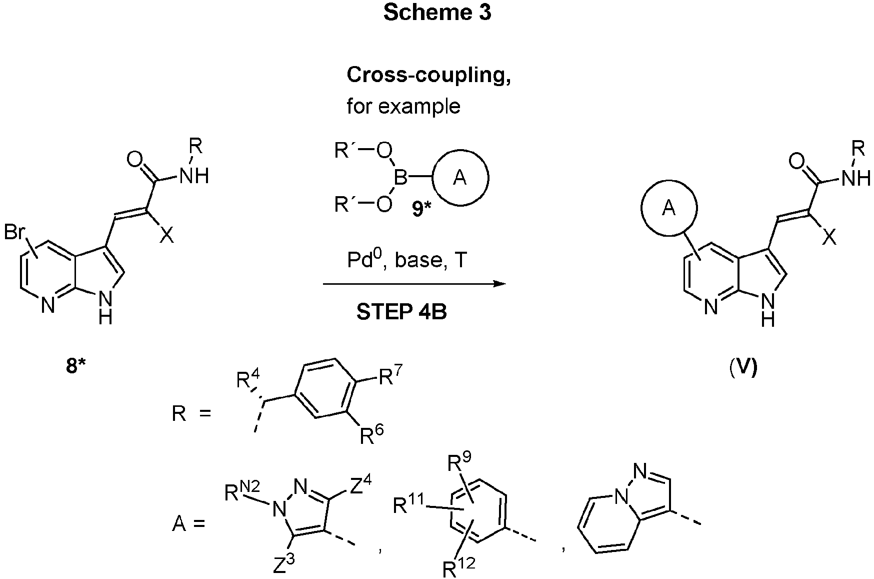

In the step **4B**, coupling compound is performed in the present of the palladium catalyst, and the base .The palladium catalyst for C-C coupling reaction (Suzuki reaction) is Pd(0) or Pd(II) catalyst, preferred PdCl₂, Pd(PPh₃)₄, Pd(acac)₂, PdCl₂(CH₃CN)₂, PdCl₂(PCy₃)₂, PdCl₂(PPh₃)₂, Pd(dppf)Cl₂, [(π-ally)PdCl]₂, (SIPr)PdCl₂(TEA). Optionally, further phosphine ligand may be used together with the palladium catalyst.

A ratio of the palladium catalyst to the starting meterial is in the range of 0.01 to 20 mol-%, preferred 0.01-10 mol-%, more preferred 0.01-5 mol-%, most preferred 0.01-1 mol-%.

The Pd(0) catalyst may be tetrakis(triphenylphosphine)palladium(0) [Pd(PPh₃)₄], tris(dibenzylideneacetone)di-palladium(0) [Pd₂(dba)₃]. Pd(II) catalyst may be dichlorobis(triphenylphosphine)-palladium(II) [Pd(PPh₃)₂Cl₂], palladium(II) acetate and triphenylphosphine or [1,1'-bis(diphenylphosphino)ferrocene]palladium dichloride. The reaction is preferably carried out in a mixture of a solvent like dioxane, DMF, DME, THF, or isopropanol with water and in the presence of a base.

The base may be an organic base or inorganic bases. The organic base may be tertiary amine such as Et₃N, and DIPEA, DABCO, DBU, pyrrolidine or piperidine. The inorganic base may be Na₂CO₃, K₂CO₃, Cs₂CO₃ or K₃PO₄. A ratio of the first base to the starting meterial is in the range of 1.0 to 5.0 equivalents, preferred 1.0 to 3.0 equivalents, more preferred 1.0 to 3.0 equivalents, most preferred 1.0 to 2.0 equivalents.

Preferred, the boronic acid derivative **9*** may be a boronic acid (R' = -H) or an ester of the boronic acid, e.g. its isopropyl ester (R' = -CH(CH₃)₂), an ester derived from pinacol (R'-R' = -C(CH₃)₂-C(CH₃)₂-).

Preferred, this reaction is performed in a polar aprotic solvent such as DMF or DMSO under N₂ atmosphere at a temperature in a range of 80°C to 200°C, preferred 100°C to 180°C, more preferred 100°C to 150°C, and most preferred 120°C to 150°C. Optionally, this reaction is carried out in the microwave.

### Syntheses of intermediates

The intermediates for compound of formula (I) are prepared by reference to the methods illustrated in the following schemes4-5. The intermediates are produced by Schemes 5-7 by the suitable selection of reagents with appropriate substitution. Solvents, temperatures, pressures, and other reaction conditions may readily be selected by one of ordinary skill in the art. Starting materials are commercially available or readily prepared by one of ordinary skill in the art.

As shown in Scheme 4, optionally the method for producing the compound of the formula (I) further comprises the following steps before the **Step 1:**
**Step1a:** providing 1H-pyrrolo[2,3-b]pyridine compound (**1***) 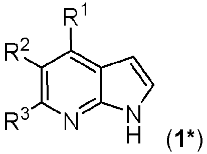
**Step1b:** performing a formylation reaction of 1H-pyrrolo[2,3-b]pyridine compound (**1***) to obtain 1H-pyrrolo[2,3-b]pyridine-3-aldehyde compound (**2***); 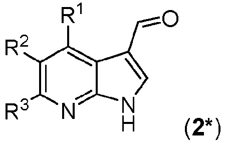 wherein **R¹, R²**, and **R³** have the same meanings as defined in the formula (I).

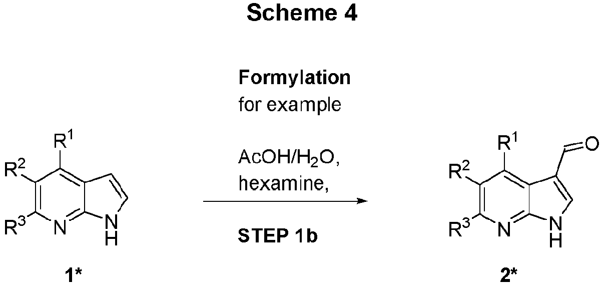

The synthetic route for the intermediate (**6***) used for a late stage Mitsunobu reaction (Scheme 2) is described in Scheme 5.

| | |
|---|---|
| **Step F7:** | Performing a Mitsunobu reaction with compound **11*** using standard Mitsunobu-condition to obtain the allyl-protected product compound (**12***). |
| **Step E7:** | Performing formylation reaction to obtain the product compound (**13***). |
| **Step B7:** | Performing a condensation reaction with the aldehyde compound (**13***) and the 2-cyano amide compound (**3***) to obtain the product compound (**14***). |
| **Step G7:** | Performing a deprotection of the allyl PG using a palladium catalyst and barbituric acid in order to obtain product compound (**6***). |

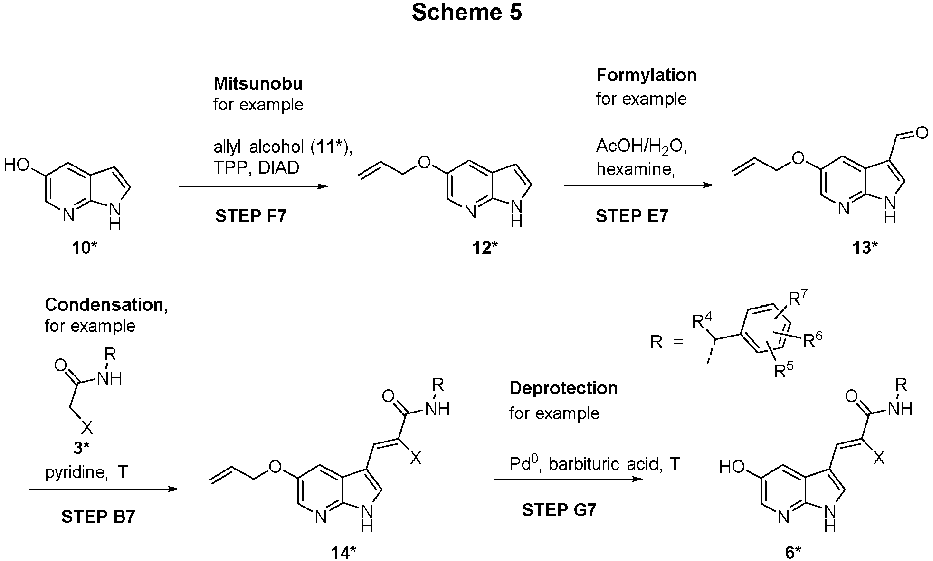

### Indication

In a further aspect of the present invention, the novel compounds according to the general formula (I) are for use as pharmaceutically active agent.

Surprisingly it was found that the above-mentioned compounds of general formula (I), as well as the pharmaceutical compositions thereof are acting as G protein-coupled receptor kinases (GRKs) inhibitors and more specifically as inhibitor of GRK2/5 kinases.

In the present application, the Lance Kinase Activity Assay was performed to determine IC₅₀ values of compounds of general formula (I) against GRK5 and GRK2. Table 4 shows activity data (indicated as IC₅₀ values) in the biochemical Lance assay. It is proven that the inventive compounds inhibits GRK5 and GRK2 very effectively The pharmaceutical compositions according to the present invention comprise at least one compound according to the present invention as an active ingredient together with at least one pharmaceutically acceptable (i.e. non-toxic) carrier, excipient and/or diluent.

As used herein, G protein-coupled receptor kinases (GRKs) "inhibitor" refers to any compound capable of downregulating, decreasing, suppressing or otherwise regulating the amount and/or activity of a kinase. Inhibition of these GRKs can be achieved by any of a variety of mechanisms known in the art, including, but not limited to binding directly to said kinase polypeptide, denaturing or otherwise inactivating said kinase, or inhibiting the expression of the gene (e.g., transcription to mRNA, translation to a nascent polypeptide, and/or final polypeptide modifications to a mature protein), which encodes said kinase.

As used herein the term "inhibiting" or "inhibition" refers to the ability of a compound to downregulate, decrease, reduce, suppress, inactivate, or inhibit at least partially the activity of an enzyme, or the expression of an enzyme or protein.

Further aspects of the present invention relate to the compound of the general formula (I) , or the above-mentioned pharmaceutical composition, for use in prophylaxis and/or treatment of diseases caused by or associated with G protein-coupled receptor kinases 5 (GRK5) selected from heart disease, inflammatory and immunological disease, metabolic disease and cancer.

Preferred, the diseases caused by or associated with G protein-coupled receptor kinases 5 (GRK5) are heart diseases, in particular cardiovascular diseases.

Further aspects of the present invention relate to the use of the compounds of general formula (I) for the preparation of a pharmaceutical composition useful for prophylaxis and/or treatment of diseases caused by or associated with G protein-coupled receptor kinases 5 (GRK5) selected from heart disease, inflammatory and immunological disease, metabolic disease and cancer, preferred cardiovascular diseases.

The term "effective amount" means an amount of compound that, when administered to a patient in need of such treatment, is sufficient to
(i) treat or prevent a particular disease, condition, or disorder which can be treated with a GRK5 inhibitor;
(ii) attenuate, ameliorate, or eliminate one or more symptoms of the particular disease, condition, or disorder; or
(iii) prevent or delay the onset of one or more symptoms of the particular disease, condition, or disorder described herein.

The amount of a compound of general formula (I) that will correspond to such an amount will vary depending upon factors such as the particular compound, disease condition and its severity, the identity (e.g., weight) of the patient in need of treatment, but can nevertheless be routinely determined by one skilled in the art.

### Cardiovascular diseases

The inventive compounds, or the pharmaceutical composition thereof are useful for prophylaxis and/or treatment of cardiovascular diseases such as cardiac hypertrophy, adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome.

Preferred are cardiac hypertrophy, adult congenital heart disease, aneurysms, angina, angina pectoris, arrhythmias, cardiovascular disease prevention, cardiomyopathies, congestive heart failure, myocardial infarction, pulmonary hypertension, hypertrophic growth, restenosis, stenosis, thrombosis and arteriosclerosis.

### Inflammatory and immunological disease

Nuclear factor κ-B (NFκB) signaling pathway is intricately tied with many inflammatory processes and thus, regulatory molecules in NFκB signaling pathway are potential therapeutic targets in a number of inflammatory diseases. GRKs, in particular GRK2 and GRK5 have been reported to modulate NFκB signaling pathway in immune and non-immune cells (Packiriswamy and Parameswaran, Genes Immun. 2015 Sep;16(6):367-77).

Therefore, the inventive compounds, or the pharmaceutical composition thereof are useful for prophylaxis and/or treatment of an inflammatory and/or an immunological disease, wherein the inflammatory and/or the immunological disease includes inflammatory disease associated with an autoimmune disease, a central nervous system (CNS) inflammatory disease, a joint inflammation disease, an inflammatory digestive tract disease, and inflammatory skin. Among them, Inflammatory Bowel Disease, Rheumatoid Arthritis and Multiple Sclerosis are of particular interest.

### Metabolic diseases

As GRK5 modulates the insulin release in a significant manner (WO 2012/028335 A1), it became a preferred target for the therapy of metabolic diseases, preferably for the therapy of diabetes, obesity and impaired adipogenesis. Thus in a preferred embodiment of this invention, the compounds of general formula (I), pharmaceutical acceptable salts thereof, or the pharmaceutical composition thereof are for use for the treatment and/or prophylaxis of the metabolic disease selected from diabetes, obesity and impaired adipogenesis.

Furthermore, it was found that the inhibition of GRK5 is correlated to a significant increase in insulin release. Consequently, GRK5 represents a preferable target for the therapy of diabetes mellitus type 2. Thus, another aspect of the present invention is directed to the compound of general formula (I) and/or pharmaceutically acceptable salts thereof for use in the treatment or prophylaxis of diabetes mellitus type 2.

Moreover the compounds of the present application influence the glucose dependent regulation of insulin production and/or release and/or the increase of glucose uptake. Therefore, another aspect of the present invention is directed to the compounds of general formula (I) for use in glucose dependent regulation of insulin production and/or release of insulin, as well as for increasing glucose uptake.

### Cancer

Furthermore, the compounds of the present application or the pharmaceutical composition thereof are useful for the treatment and/or prophylaxis of cancer, wherein the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma, tongue cancer, astrocytomas, bronchial cancer, laryngeal cancer, malignant melanoma, oesophageal cancer, and renal cell cancer.

Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer.

The compounds enlisted explicitly in Table 1 are preferred to be used within the methods or indications disclosed herein.

The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the 3-substituted 1H-pyrrolo[2,3-b]pyridine compounds of the formula (I) or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to ca. 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein as described in the following claims.

### Preparation of compounds:

### General Information:

All reactions involving air- or moisture-sensitive reagents or intermediates were carried out in flame-dried glassware under an argon atmosphere. Dry solvents (THF, toluene, MeOH, DMF, DCM) were used as commercially available. ¹H-NMR and ¹³C-NMR were recorded on a Bruker DRX400 (400 MHz). Multiplicities are indicated as: br s (broadened singlet), s (singlet), d (doublet), t (triplet), q (quartet), quin (quintet), m (multiplet); and coupling constants (J) are given in Hertz (Hz). HPLC - electrospray mass spectra (HPLC ES-MS) were obtained using Waters Acquity Performance Liquid Chromatography (UPLC) equipped SQ 3100 Mass detector spectrometer. Column: Acquity UPLC BEH C18 1.7um, 2.1x50mm. Flow: 0.5ml/min. Eluents: A: H₂O with 0.05% formic acid and B: ACN with 0.05% TFA. All chemicals and solvents were purchased from commercial sources like Sigma-Aldrich, Fluka, TCI, Acros Organics, ABCR, Alfa Aesar, Enamine, VWR, Combi-Blocks, Apollo Scientific, Aquilla Pharmatech, Ark Pharm, D-L Chiral Chemicals, ChemBridge, Renno Tech, Accela, KeyOrganics, Pharmablock and Chem Impex. Unless otherwise noted, all commercially available compounds were used as received without further purifications.

### Abbreviations used in the description of the chemistry and in the Examples that follow are:

ACN or MeCN (acetonitrile); br (broad); BOC (tet-butyloxycarbonyl), CDCl₃ (deuterated chloroform); cHex (cyclohexane); DCM (dichloromethane); DIAD (diisopropyl azodicarboxylate); DIPEA (di-iso-propylethylamine); DMF (dimethylformamide); DMSO (dimethyl sulfoxide); eq. (equivalent); ES (electrospray); EDTA (ethylenediaminetetraacetic acid), EtOAc (ethyl acetate); EtOH (ethanol); HATU (*O*-(7-azabenzotriazol-1-yl)-*N,N,N',N'*-tetramethyluronium hexafluorophosphate); HBTU (3-[Bis(dimethylamino)methyliumyl]-3*H*-benzotriazol-1-oxide hexafluorophosphate); HCl (hydrochloric acid); AcOH (acetic acid); H₂O (water); K₂CO₃ (potassium carbonate); KOH (potassium hydroxide); MeOH (methanol); MS (mass spectrometry); Mwt (molecular weight); NaHCO₃ (sodium hydrogencarbonate); NH₄Cl (ammonium chloride); NIS (N-lodosuccinimide); NMP (N-methyl-2-pyrrolidon); NMR (nuclear magnetic resonance); iPrOH (iso-propanol);; RP (reversed-phase); RT (room temperature); sat. aq. (saturated aqueous); SiO₂ (silica gel); TFA (trifluoroacetic acid); THF (tetrahydrofurane).

### Preparative Examples

### Example 1:

### (R,E)-2-cyano-N-(1-(3-fluorophenyl)ethyl)-3-(1H-pyrrolo[2,3-b]pyridin-3-yl)acrylamide (A1)

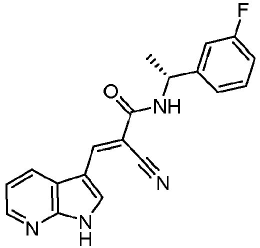

To a solution of HBTU (201 mg, 0.53 mmol, 1.5 eq.) in DMF (4 mL) at 0 °C was added (E)-2-cyano-3-(1H-pyrrolo[2,3-b]pyridin-3-yl)acrylic acid (75 mg, 0.35 mmol, 1.0 eq.) and DIPEA (135 mg, 1.1 mmol, 3.0 eq.). The mixture was stirred for 15min. Then (R)-2-cyano-N-(1-(3-fluorophenyl)ethyl)acetamide (73 mg, 0.53 mmol, 1.5 eq.) was added and the mixture was stirred for 4h at 0 °C. Afterwards, the solvents were evaporated in vacuo. The crude was purified by reverse phase RP-HPLC (column: C18), using H₂O (0.1 %TFA) and ACN (0.1 %TFA) as eluents. The desired fractions were lyophilized to yield the title compound **A1** (14 mg, 0.04 mmol, 12%) as a yellow solid.

¹H NMR (400MHz, d₆-DMSO, 300K) δ 12.87 (s, 1H), 8.68 (d, *J* = 7.8 Hz, 1H). 8.52-8.48 (m, 1H), 8.45 (s, 1H), 8.43-8.36 (m, 2H), 7.43-7.35 (m, 1H), 7.34-7.29 (m, 1H), 7.27-7-21 (m, 2H), 5.14-5.04 (m, 1H), 1.49 (d, *J =* 7.1 Hz, 3H). MS (ES) C₁₉H₁₅FN₄O requires: 334, found: 335 (M+H)⁺.

The examples in the following table **1A** were prepared according to the procedure described for **A1** (Example **1**):

**Table 1A**

| **Example** | **Structure** | **M** | **[M+H]⁺** |
|---|---|---|---|
| **6** | 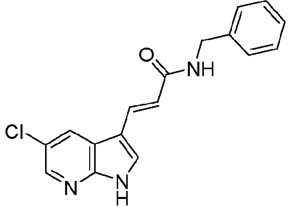 | 311 | 312 |
| **7** | 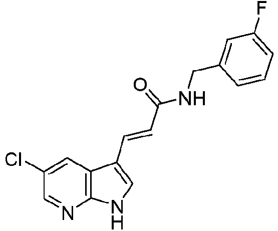 | 329 | 330 |
| **8** | 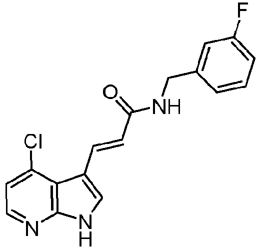 | 329 | 330 |
| **10** | 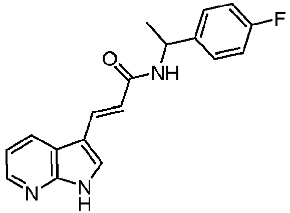 | 309 | 310 |
| **11** | 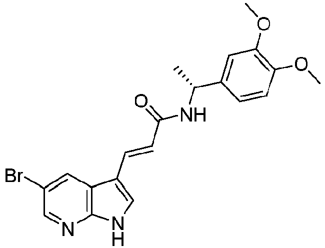 | 429 | 430 |
| **12** | 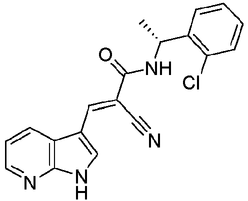 | 350 | 351 |
| **13** | 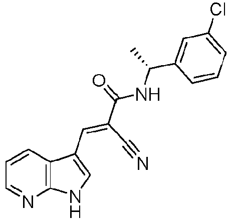 | 350 | 351 |
| **14** | 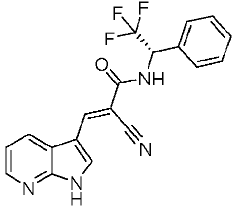 | 370 | 371 |
| **15** | 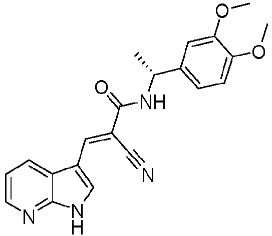 | 376 | 377 |
| **16** | 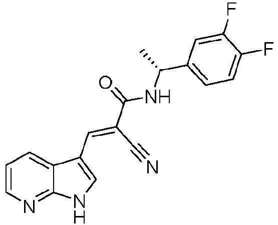 | 352 | 353 |
| **17** | 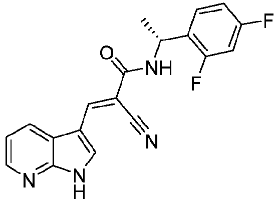 | 352 | 353 |
| **18** | 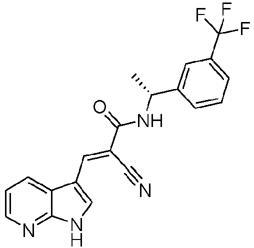 | 384 | 385 |
| **19** | 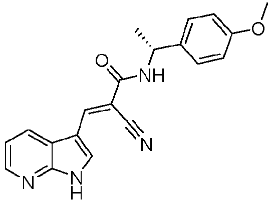 | 346 | 347 |
| **20** | 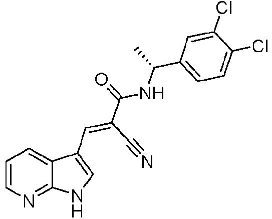 | 385 | 385 |
| **21** | 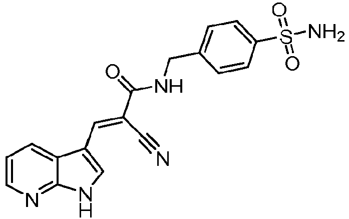 | 381 | 382 |
| **22** | 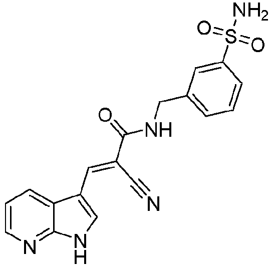 | 381 | 382 |
| **23** | 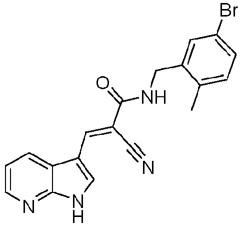 | 395 | 395 |
| **24** | 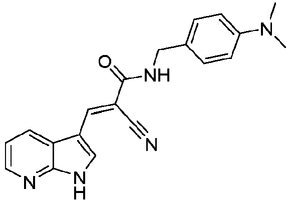 | 345 | 346 |
| **25** | 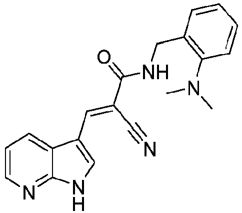 | 345 | 346 |
| **26** | 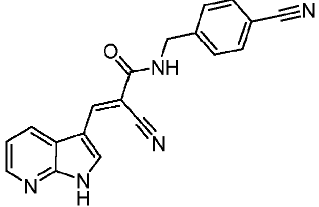 | 327 | 328 |
| **27** | 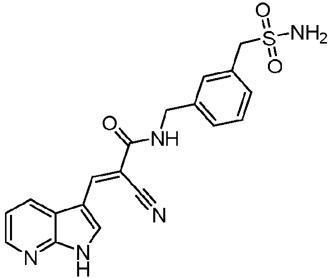 | 395 | 396 |
| **28** | 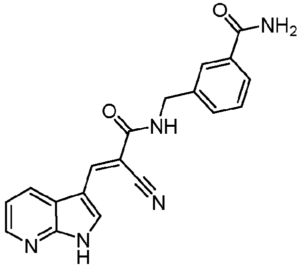 | 345 | 346 |
| **29** | 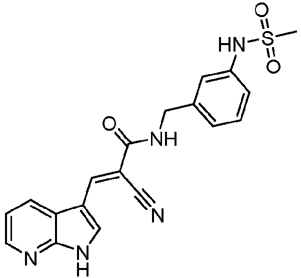 | 395 | 396 |
| **30** | 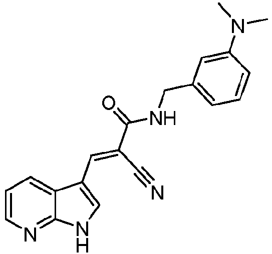 | 345 | 346 |
| **31** | 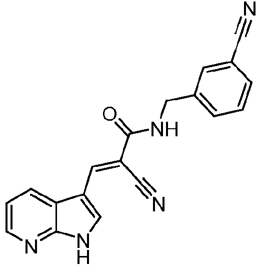 | 327 | 328 |
| **32** | 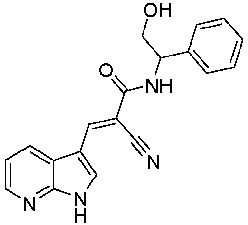 | 332 | 333 |
| **33** | 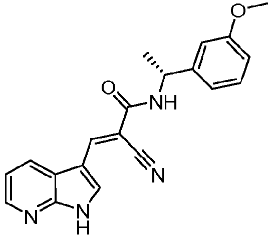 | 346 | 347 |
| **34** | 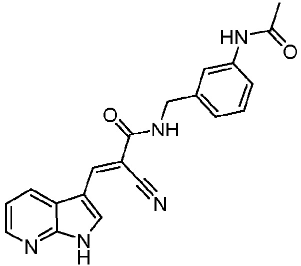 | 359 | 360 |
| **35** | 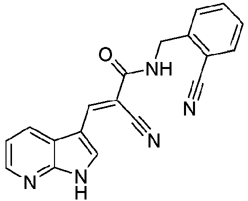 | 327 | 328 |
| **36** | 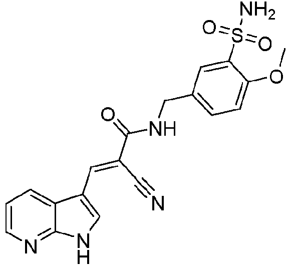 | 411 | 412 |
| **37** | 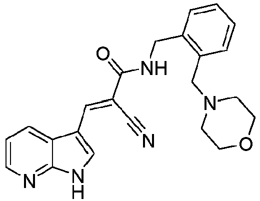 | 401 | 402 |
| **38** | 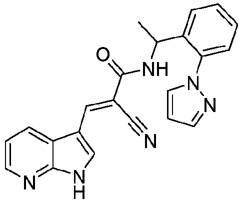 | 382 | 383 |
| **39** | 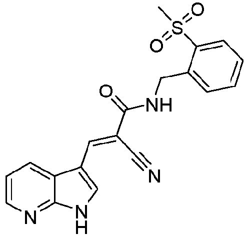 | 380 | 381 |
| **40** | 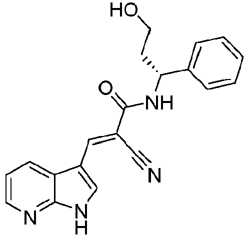 | 346 | 347 |
| **41** | 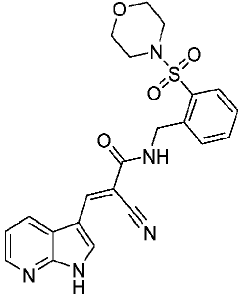 | 451 | 452 |
| **42** | 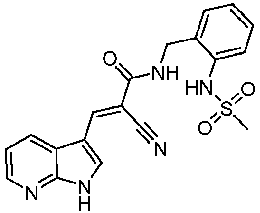 | 395 | 396 |
| **43** | 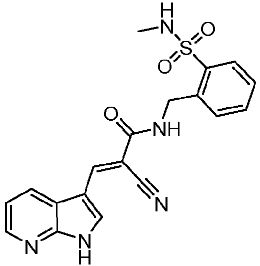 | 395 | 396 |
| **44** | 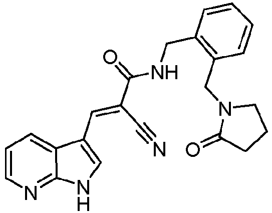 | 399 | 400 |
| **45** | 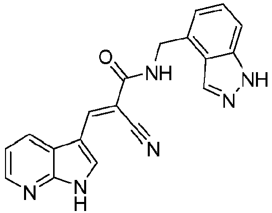 | 342 | 343 |
| **46** | 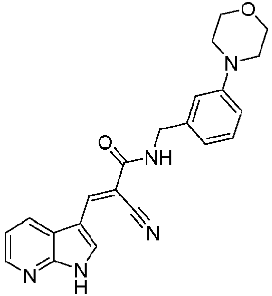 | 387 | 388 |
| **47** | 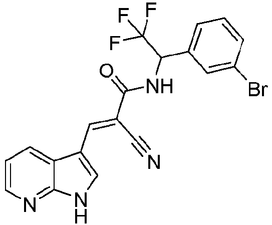 | 448 | 449 |
| **48** | 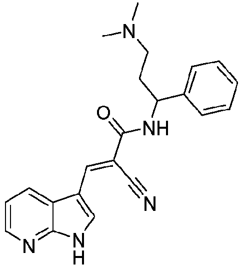 | 373 | 374 |
| **49** | 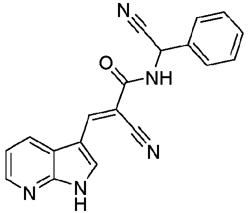 | 327 | 328 |
| **50** | 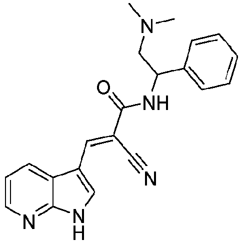 | 359 | 360 |
| **51** | 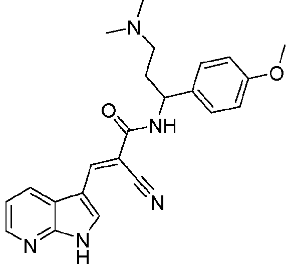 | 403 | 404 |
| **52** | 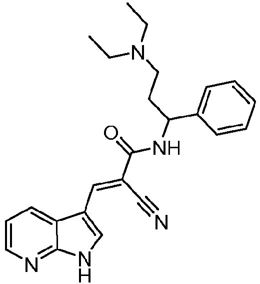 | 401 | 402 |
| **53** | 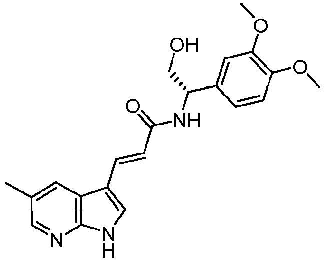 | 381 | 382 |
| **54** | 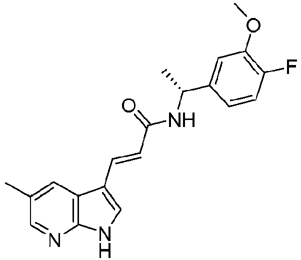 | 353 | 354 |
| **55** | 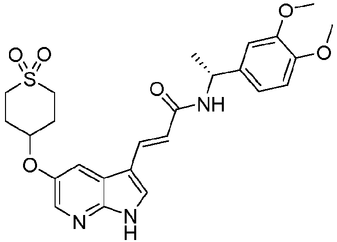 | 500 | 501 |
| **56** | 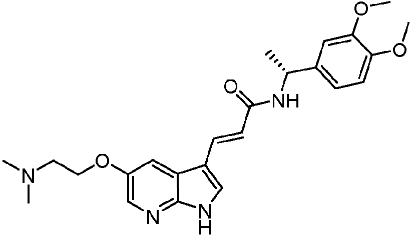 | 438 | 439 |
| **246** | 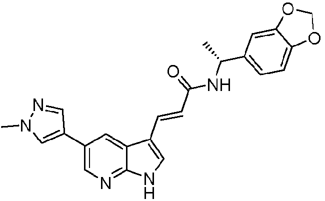 | 415 | 416 |
| **253** | 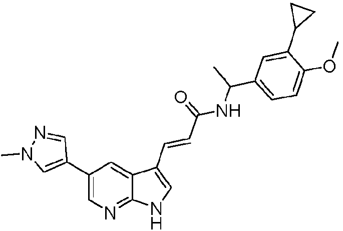 | 441 | 442 |
| **255** | 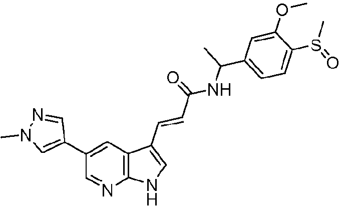 | 463 | 464 |
| **257** | 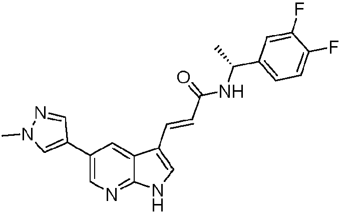 | 407 | 408 |
| **260** | 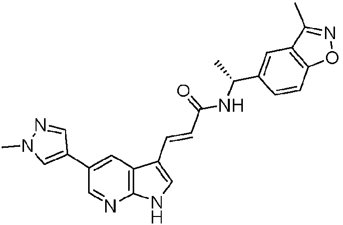 | 426 | 427 |
| **265** | 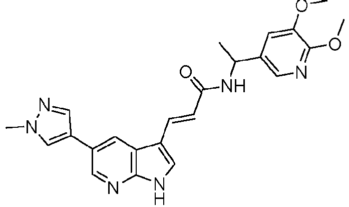 | 432 | 433 |

### Example 57:

### (E)-2-cyano-N-(4-fluorobenzyl)-3-(1H-pyrrolo[2,3-b]pyridin-3-yl)acrylamide (B1)

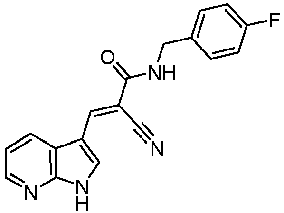

7-Azaindole-3-carboxaldehyde (73 mg, 0.5 mmol, 1.0 eq.) was dissolved in dry pyridine (3 mL). 2-Cyano-N-(4-fluorobenzyl)acetamide (125 mg, 0.65 mmol, 1.3 eq.) was added to the solution. The reaction mixture was stirred for 48 h at 110 °C. The solvents were removed in vacuo. EtOH was added and the resulting precipitate was filtered and led to the desired product **B1** (141 mg, 0.44 mmol, 88 %) as a beige solid.

¹H NMR (400MHz, d₆-DMSO, 300K) δ 12.87 (s, 1H), 8.84 (t, *J* = 6.0 Hz, 1H), 8.49 (d, *J* = 10.2 Hz, 2H), 8.43-8.36 (m, 2H), 7.41-7.33 (m, 2H), 7.32-7.27 (m, 1H), 7.20-7.12 (m, 2H), 4.42 (d, *J* = 5.9 Hz, 2H). MS (ES) C₁₈H₁₃FN₄O requires: 320, found: 321 (M+H)⁺.

The examples in the following table were prepared according to the procedure described for **B1** (Example 57).

In order to obtain example 89, a subsequent standard acidic BOC-deprotection was performed.

**Table 1B**

| **Example** | **Structure** | **M** | **[M+H]⁺** |
|---|---|---|---|
| **58** | 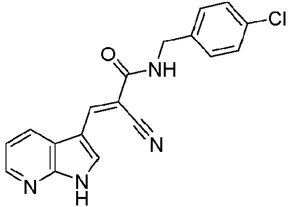 | 336 | 337 |
| **59** | 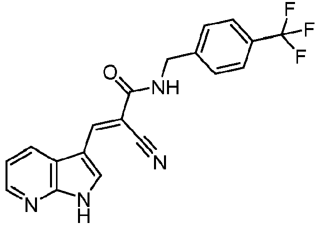 | 370 | 371 |
| **60** | 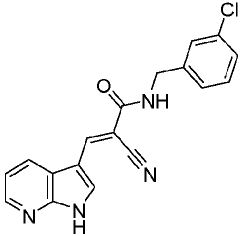 | 336 | 337 |
| **61** | 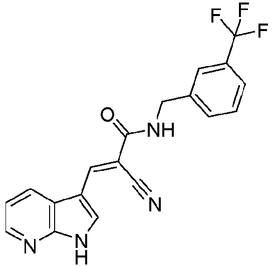 | 370 | 371 |
| **62** | 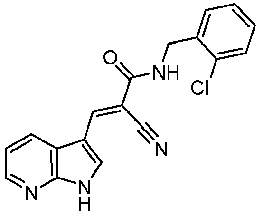 | 336 | 337 |
| **63** | 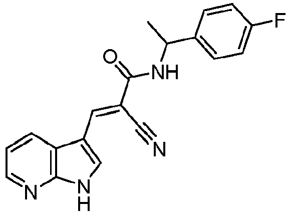 | 334 | 335 |
| **64** | 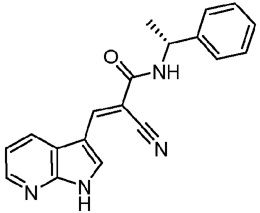 | 316 | 317 |
| **65** | 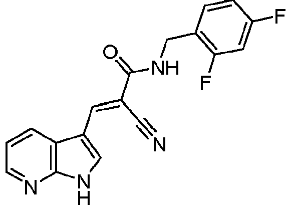 | 338 | 339 |
| **66** | 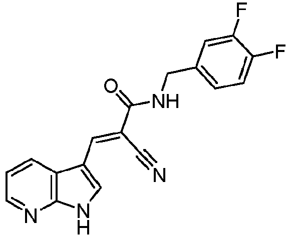 | 338 | 339 |
| **67** | 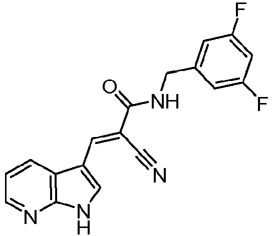 | 338 | 339 |
| **68** | 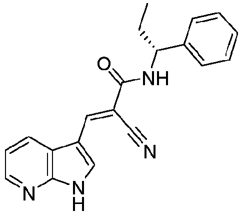 | 330 | 331 |
| **69** | 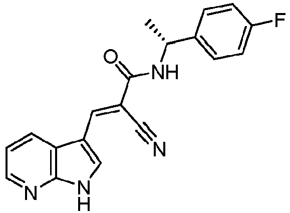 | 334 | 335 |
| **70** | 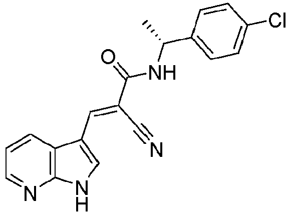 | 350 | 351 |
| **71** | 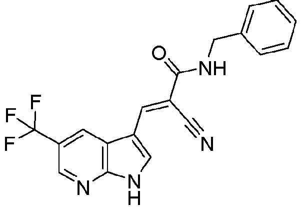 | 370 | 371 |
| **72** | 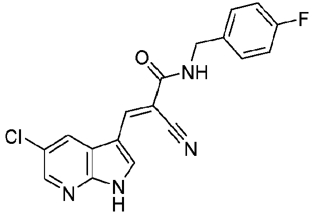 | 354 | 355 |
| **73** | 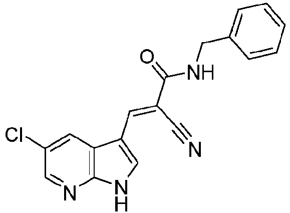 | 336 | 337 |
| **74** | 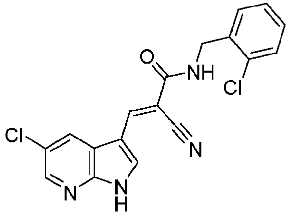 | 371 | 371 |
| **75** | 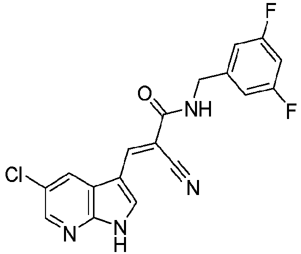 | 372 | 373 |
| **76** | 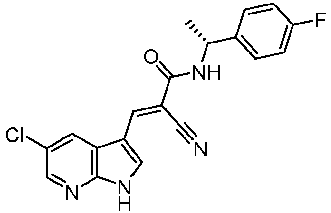 | 368 | 369 |
| **77** | 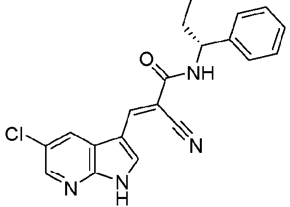 | 364 | 365 |
| **78** | 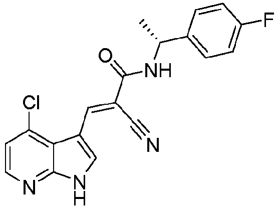 | 368 | 369 |
| **79** | 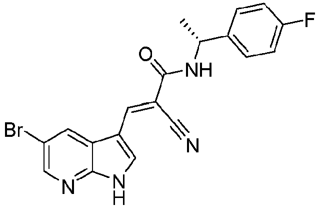 | 413 | 413 |
| **80** | 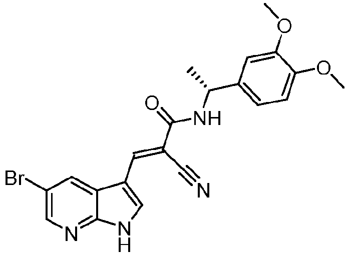 | 455 | 455 |
| **81** | 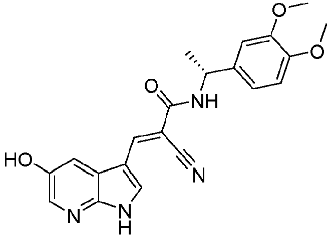 | 392 | 393 |
| **82** | 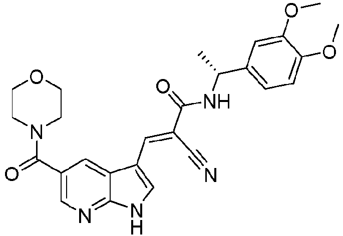 | 489 | 490 |
| **83** | 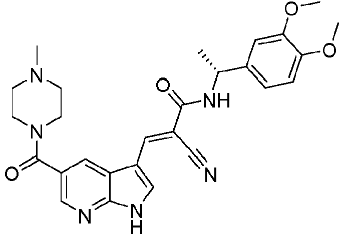 | 502 | 503 |
| **84** | 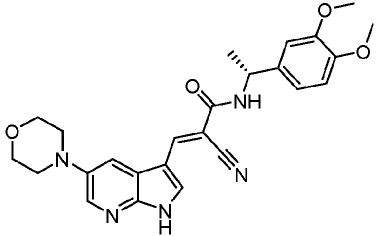 | 461 | 462 |
| **85** | 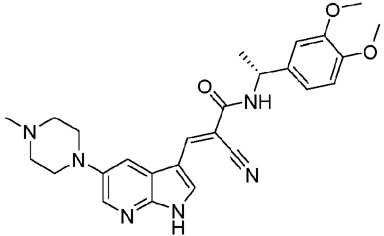 | 474 | 475 |
| **86** | 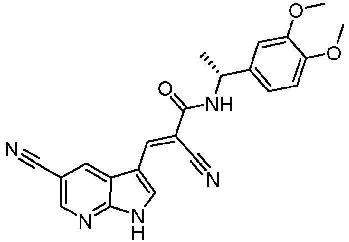 | 401 | 402 |
| **87** | 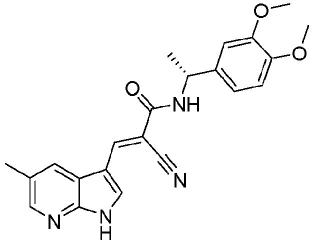 | 390 | 391 |
| **88** | 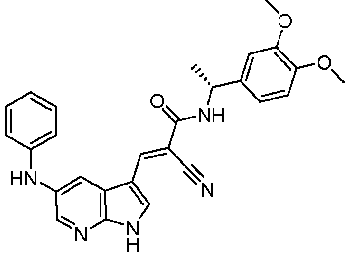 | 467 | 468 |
| **89** | 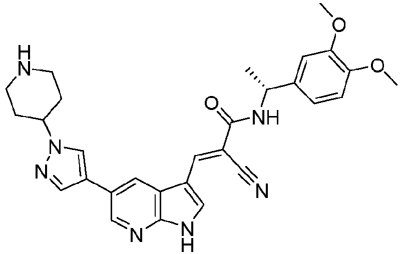 | 525 | 526 |
| **90** | 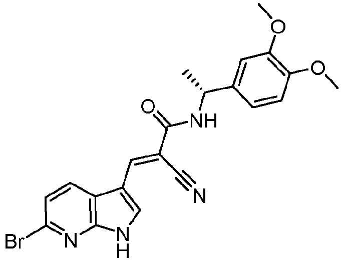 | 454 | 455 |
| **91** | 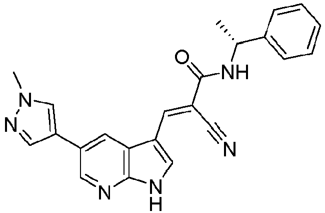 | 396 | 397 |
| **92** | 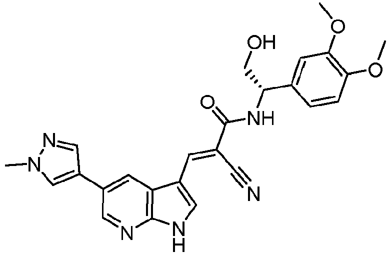 | 472 | 473 |
| **93** | 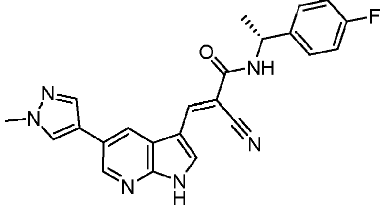 | 414 | 415 |
| **94** | 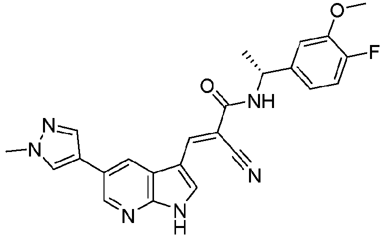 | 444 | 445 |
| **95** | 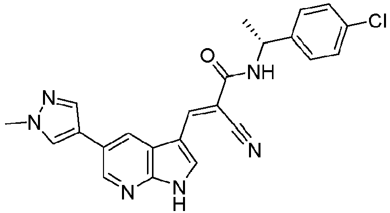 | 430 | 431 |
| **96** | 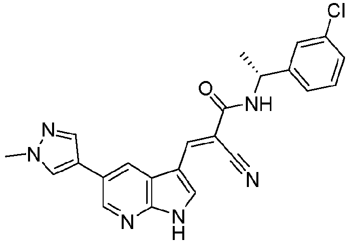 | 430 | 431 |
| **97** | 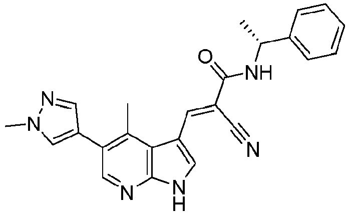 | 410 | 411 |
| **98** | 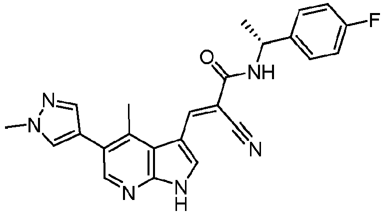 | 428 | 429 |
| **99** | 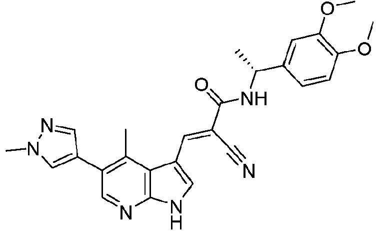 | 470 | 471 |
| **100** | 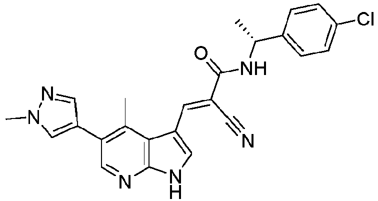 | 444 | 445 |
| **101** | 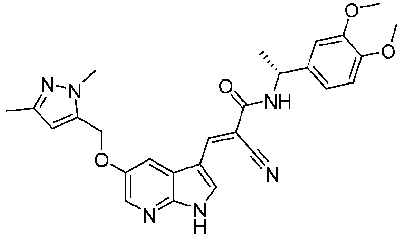 | 500 | 501 |
| **102** | 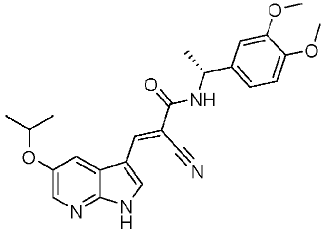 | 434 | 435 |
| **103** | 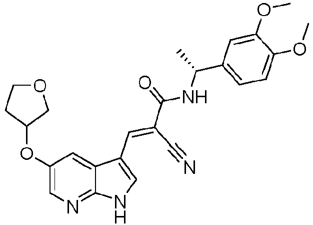 | 462 | 463 |
| **104** | 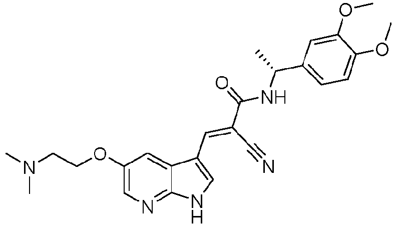 | 463 | 464 |
| **105** | 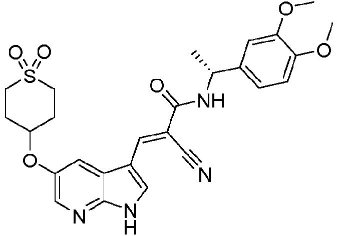 | 524 | 525 |
| **106** | 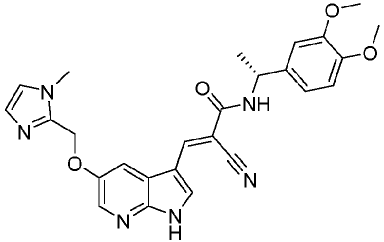 | 486 | 487 |
| **107** | 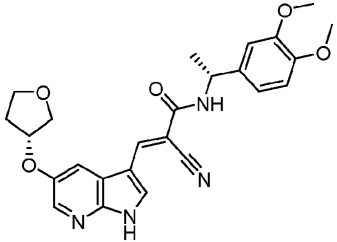 | 462 | 463 |
| **108** | 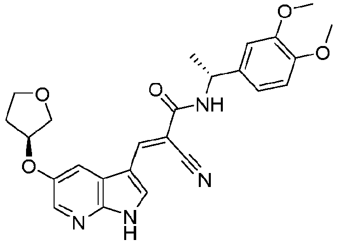 | 462 | 463 |
| **252** | 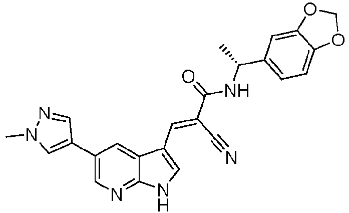 | 440 | 441 |
| **256** | 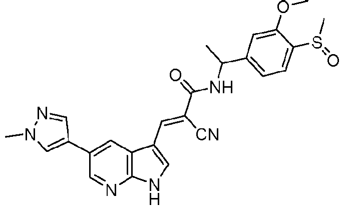 | 488 | 489 |
| **258** | 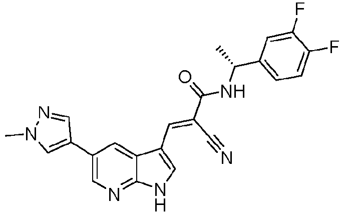 | 432 | 433 |
| **261** | 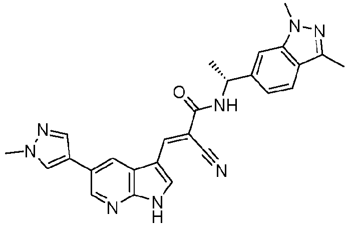 | 464 | 465 |
| **262** | 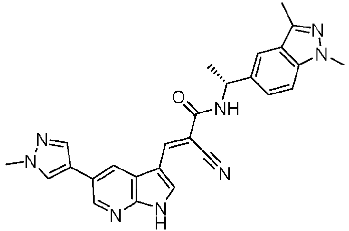 | 464 | 465 |

### Example 109:

### (E)-3-(5-((S)-sec-butoxy)-1H-pyrrolo[2,3-b]pyridin-3-yl)-2-cyano-N-((R)-1-(3,4-dimethoxyphenyl)ethyl)acrylamide (C1)

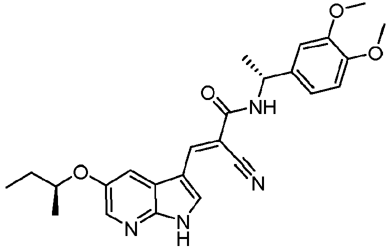

To a suspension of (R,E)-2-cyano-N-(1-(3,4-dimethoxyphenyl)ethyl)-3-(5-hydroxy-1H-pyrrolo[2,3-b]pyridin-3-yl)acrylamide (17 mg, 0.043 mmol, 1.0eq.) (R)-(-)-2-Butanol (4 mg, 0.054 mmol, 1.25 eq.) and triphenylphosphine (22.7 mg, 0.087 mmol, 2 eq.) in dry DCM (5 mL), a solution of diisopropyl azodicarboxylate (13 mg, 0.065 mmol 1.5 eq.) in DCM (1mL) was added dropwise at 0 °C. After 15 min, the reaction mixture was allowed to warm to room temperature and stirred for another 1 h. The reaction was quenched by the addition of H₂O and the resulting mixture was purified by reverse phase RP-HPLC (column: C18), using H₂O (0.1 %TFA) and ACN (0.1 %TFA) as eluents. The desired fractions were lyophilized to yield the title compound **C1** (2.1 mg, 0.0047 mmol, 11%) as a white solid.

¹H NMR (400MHz, d₆-DMSO, 300K) δ 12.79-12.34 (m, 1H), 9.06-7.91 (m, 5H), 7.10-6-81 (m, 3H), 5.10-4.97 (m, 1H), 4.55-4.36 (m, 1H), 3.77-3-73 (m, 3H), 3.73-3-69 (m, 3H), 1.77-1.56 (m, 2H), 1.50.1.38 (m, 3H), 1.29-1.24 (m, 3H), 0.96 (t, J = 7.4 Hz, 3H). MS (ES) C₂₅H₂₈N₄O₄ requires: 448, found: 449 (M+H)⁺.

The examples in the following table were prepared according to the procedure described for **C1** (Example 109).

**Table 1C**

| **Example** | **Structure** | M | **[M+H]⁺** |
|---|---|---|---|
| **110** | 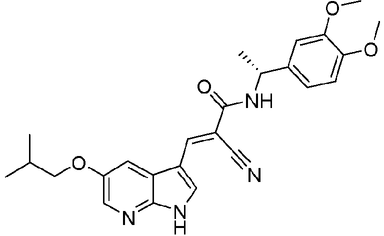 | 448 | 449 |
| **111** | 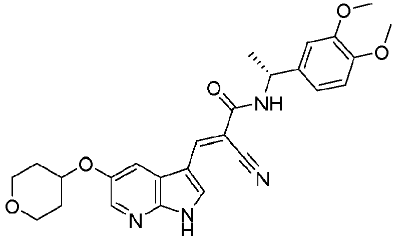 | 476 | 477 |

### Example 112:

### (R,E)-2-cyano-N-(1-(3,4-dimethoxyphenyl)ethyl)-3-(5-(4-((dimethylamino)methyl)phenyl)-1H-pyrrolo[2,3-b]pyridin-3-yl)acrylamide (D1)

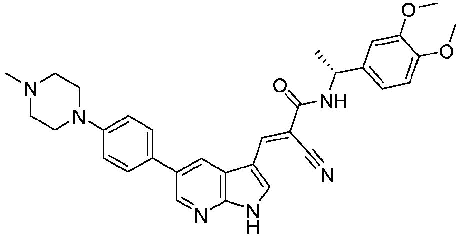

(*R,E*)-3-(5-bromo-1*H*-pyrrolo[2,3-*b*]pyridin-3-yl)-2-cyano-*N*-(1-(3,4-dimethoxyphenyl)ethyl)acrylamide (60 mg, 0.13 mmol, 1.0 eq.), (4-(4-methylpiperazin-1-yl)phenyl)boronic acid (57 mg, 0.26 mmol, 2.0 eq.), potassium carbonate (36 mg, 0.26 mmol, 2.0 eq.) was suspended in DMF/H₂O (10:1) (4 mL). The reaction mixture was degassed with a stream of N₂ for 5 min, tetrakis(triphenylphosphine)palladium(0) (15 mg, 0.01, 0.1 eq.) was added and the reaction mixture was heated to 130 °C for 2 h in the microwave. The crude solution was directly purified by reverse phase RP-HPLC (column: C18), using H₂O (0.1%TFA) and ACN (0.1%TFA) as eluents. The desired fractions were lyophilized to yield the title compound **D1** (8.9 mg, 0.016 mmol, 12 %) as a yellow solid (TFA salt). ¹H NMR (400MHz, d₆-DMSO, 300K) δ 12.88 (s, 1H), 9.63 (s, 1H), 8.66 (d, *J* = 2.1 Hz, 1H), 8.62 (d, *J* = 2.0 Hz, 1H), 8.54-8.48 (m, 3H), 7.71 (d, *J* = 8.6 Hz, 2H), 7.15 (d, *J* = 8.7 Hz, 2H), 7.06 (s, 1H), 6.95-6.88 (m, 2H), 3.99-3.90 (m, 2H), 3.77 (s, 3H), 3.73 (s, 3H), 3.58-3-50 (m, 2H), 3.27-3.12 (m, 2H), 3.07-2.95 (m, 2H), 2.89 (s, 3H), 1.48 (d, *J* = 6.9 Hz, 3H).

MS (ES) C₃₂H₃₄N₆O₃ requires: 550, found: 551 (M+H)⁺.

The examples in the following table 1D were prepared according to the procedure described for **D1** (Example 112).

**Table 1D**

| **Example** | **Structure** | **M** | **[M+H]⁺** |
|---|---|---|---|
| **113** | 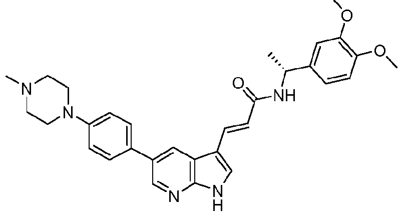 | 525 | 526 |
| **114** | 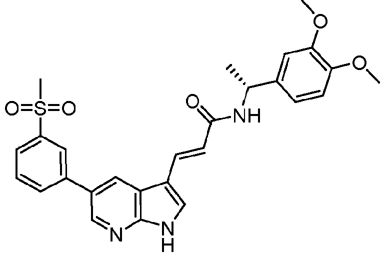 | 505 | 506 |
| **115** | 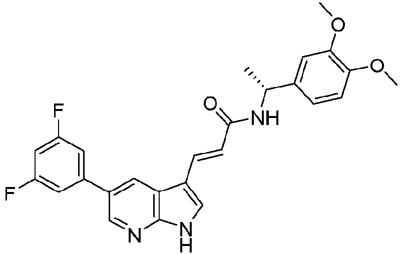 | 463 | 464 |
| **116** | 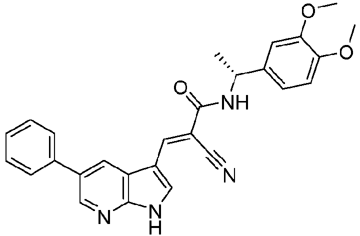 | 452 | 453 |
| **117** | 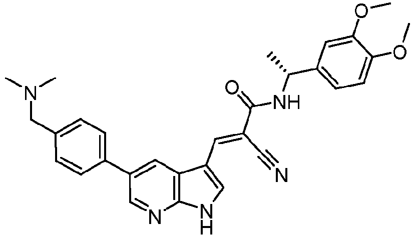 | 509 | 510 |
| **118** | 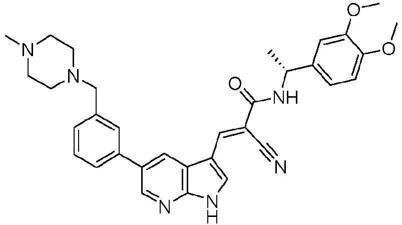 | 564 | 565 |
| **119** | 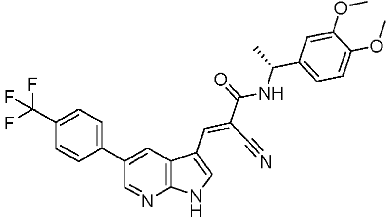 | 520 | 521 |
| **120** | 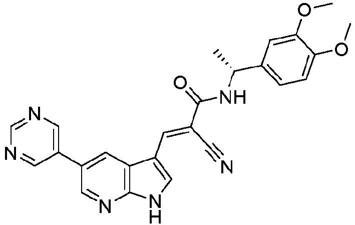 | 454 | 455 |
| **121** | 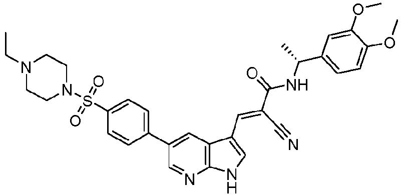 | 628 | 629 |
| **122** | 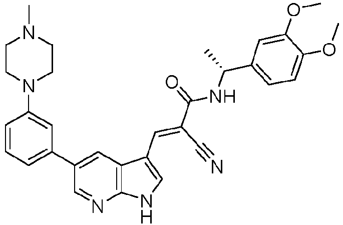 | 550 | 551 |
| **123** | 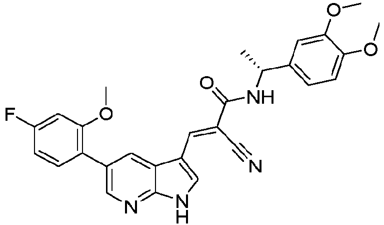 | 500 | 501 |
| **124** | 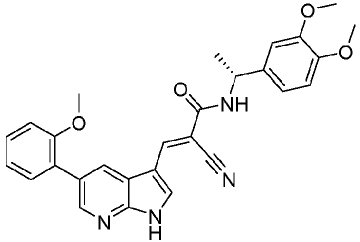 | 482 | 483 |
| **125** | 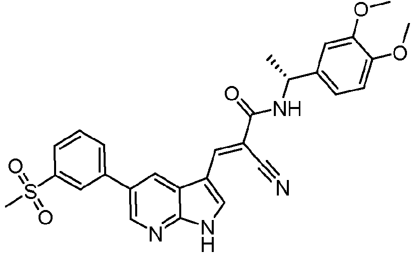 | 530 | 531 |
| **126** | 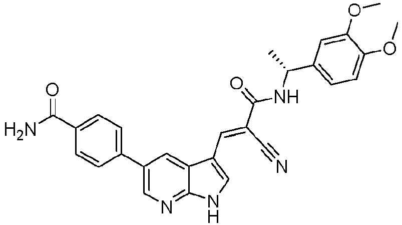 | 495 | 496 |
| **127** | 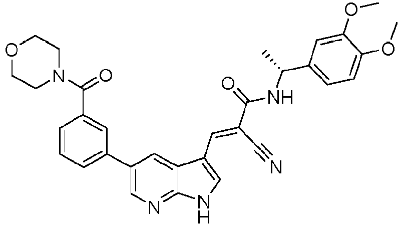 | 565 | 566 |
| **128** | 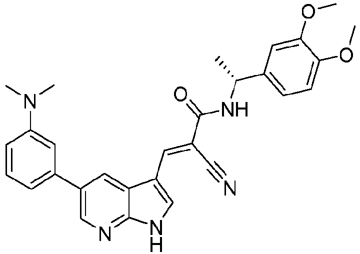 | 495 | 496 |
| **129** | 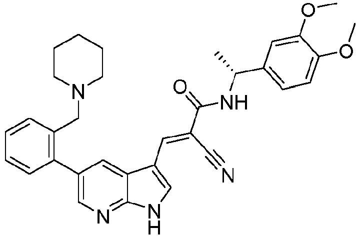 | 549 | 550 |
| **130** | 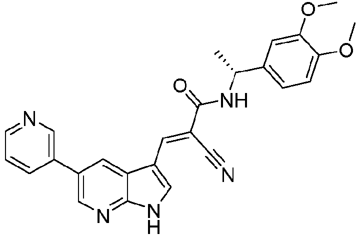 | 453 | 454 |
| **131** | 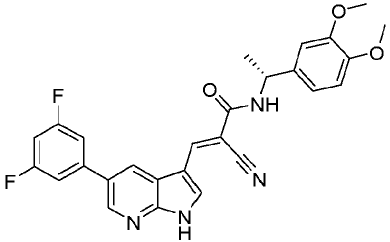 | 488 | 489 |
| **132** | 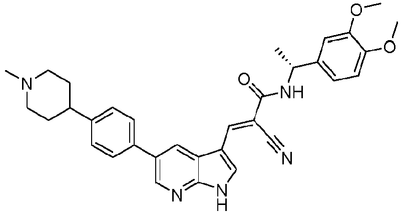 | 549 | 550 |
| **133** | 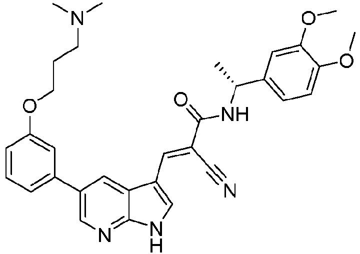 | 553 | 554 |
| **134** | 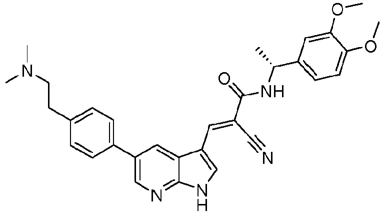 | 523 | 524 |
| **135** | 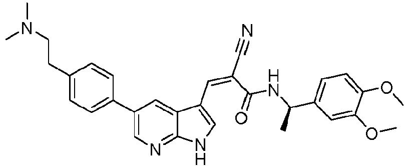 | 523 | 524 |
| **136** | 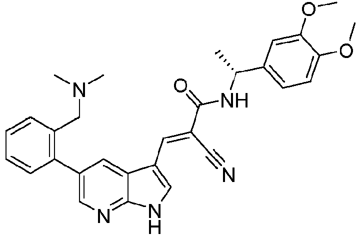 | 509 | 510 |
| **137** | 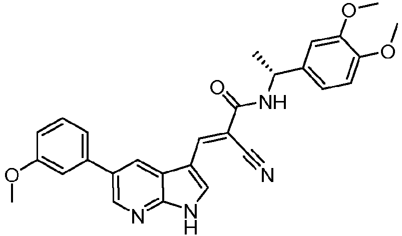 | 482 | 483 |
| **138** | 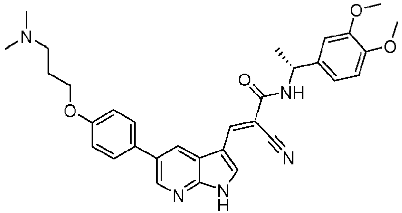 | 553 | 554 |
| **139** | 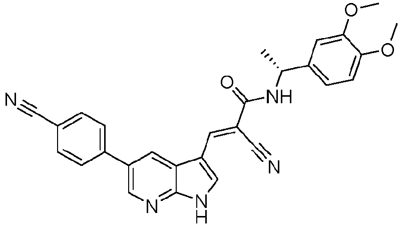 | 477 | 478 |
| **140** | 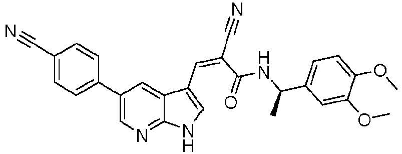 | 477 | 478 |
| **141** | 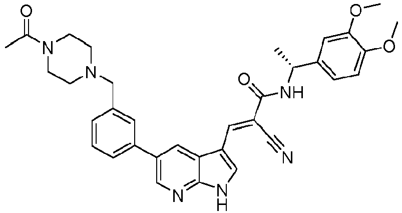 | 592 | 593 |
| **142** | 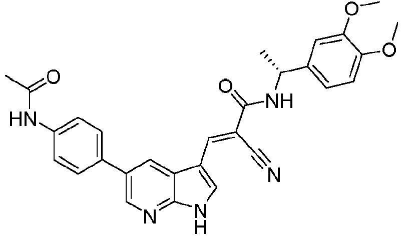 | 509 | 510 |
| **143** | 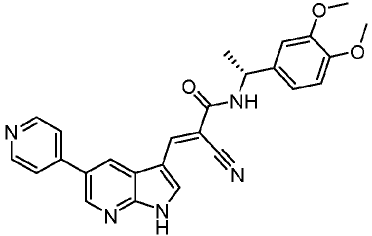 | 453 | 454 |
| **144** | 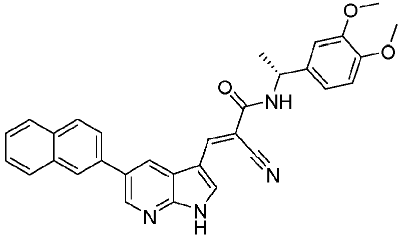 | 502 | 503 |
| **145** | 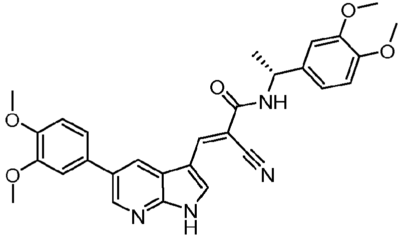 | 512 | 513 |
| **146** | 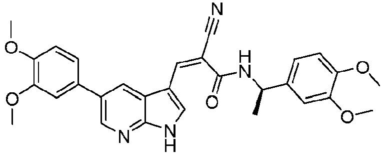 | 512 | 513 |
| **147** | 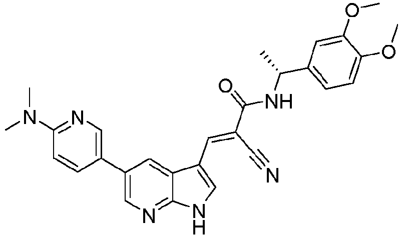 | 496 | 497 |
| **148** | 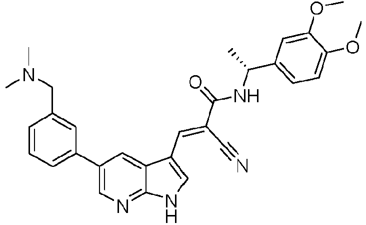 | 509 | 510 |
| **149** | 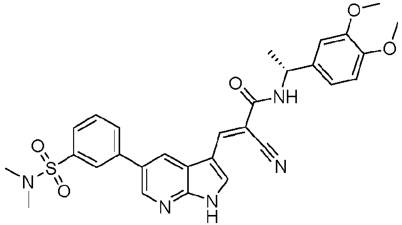 | 559 | 560 |
| **150** | 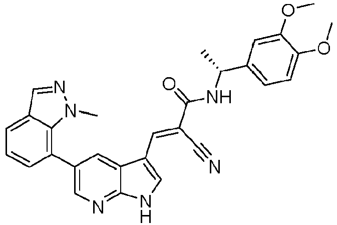 | 506 | 507 |
| **151** | 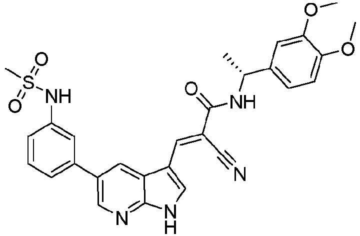 | 545 | 546 |
| **152** | 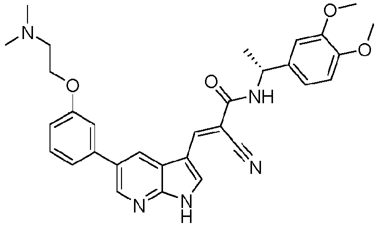 | 539 | 540 |
| **153** | 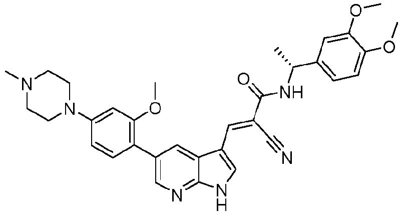 | 580 | 581 |
| **154** | 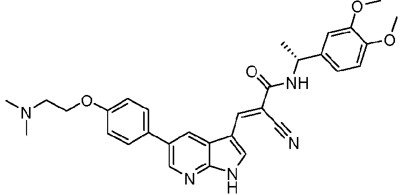 | 539 | 540 |
| **155** | 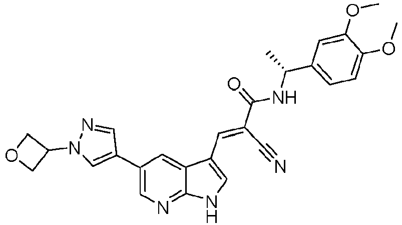 | 498 | 499 |
| **156** | 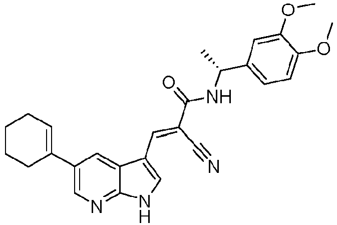 | 456 | 457 |
| **157** | 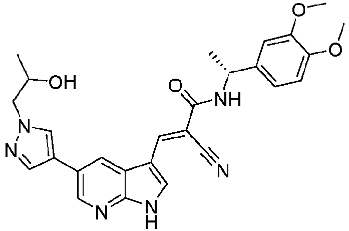 | 500 | 501 |
| **158** | 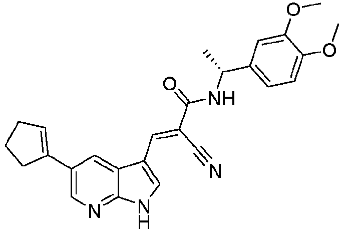 | 442 | 443 |
| **159** | 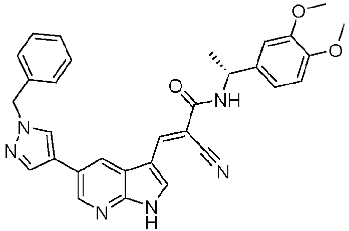 | 532 | 533 |
| **160** | 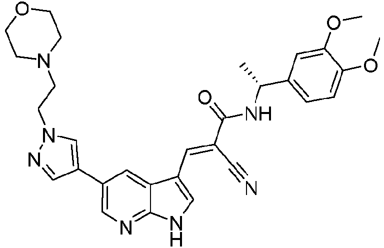 | 555 | 556 |
| **161** | 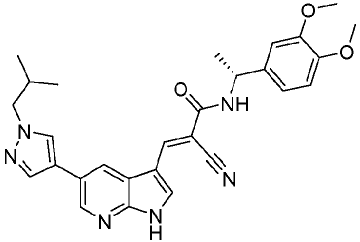 | 498 | 499 |
| **162** | 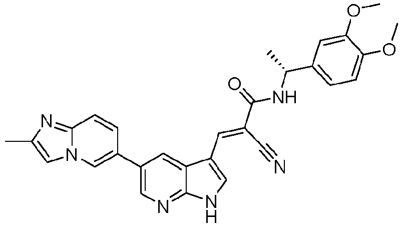 | 506 | 507 |
| **163** | 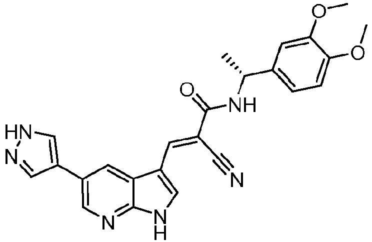 | 442 | 443 |
| **164** | 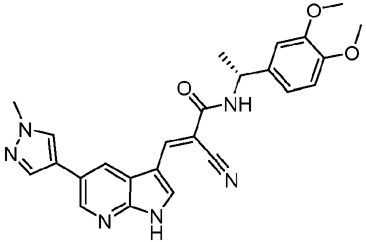 | 456 | 457 |
| **165** | 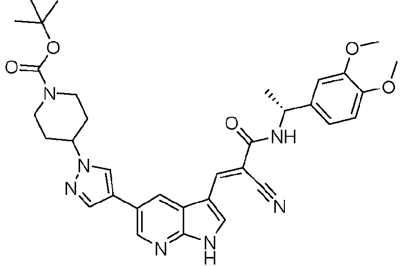 | 625 | 626 |
| **166** | 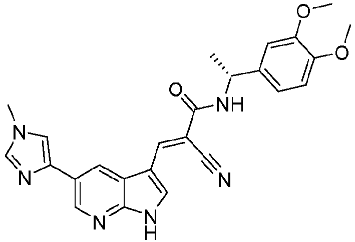 | 456 | 457 |
| **167** | 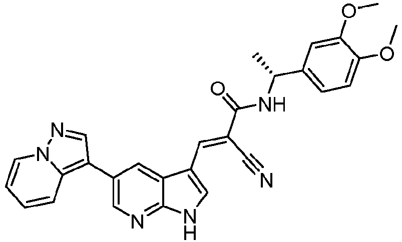 | 492 | 493 |
| **168** | 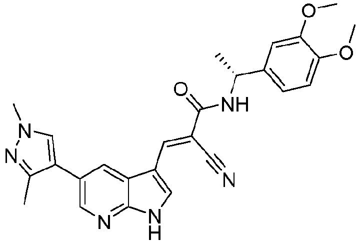 | 470 | 471 |
| **169** | 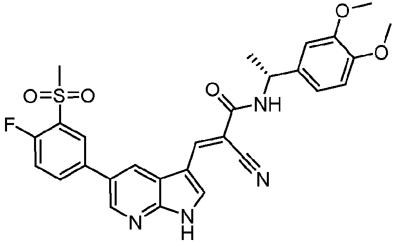 | 548 | 549 |
| **170** | 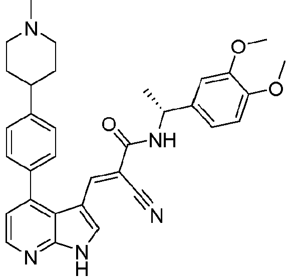 | 549 | 550 |
| **171** | 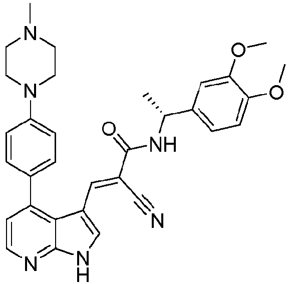 | 550 | 551 |
| **172** | 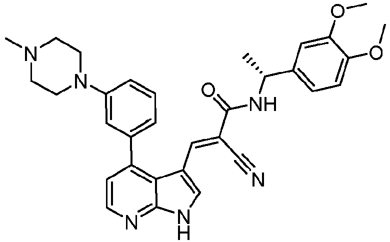 | 550 | 551 |
| **173** | 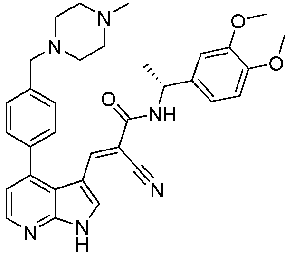 | 564 | 565 |
| **174** | 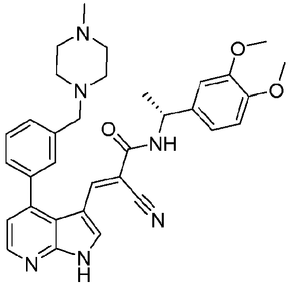 | 564 | 565 |
| **175** | 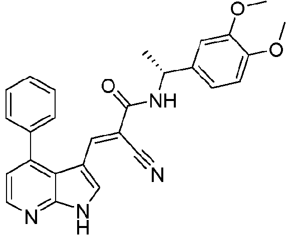 | 452 | 453 |
| **176** | 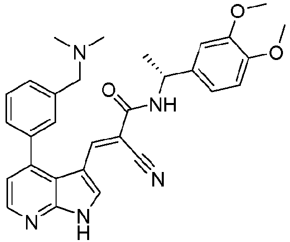 | 509 | 510 |
| **177** | 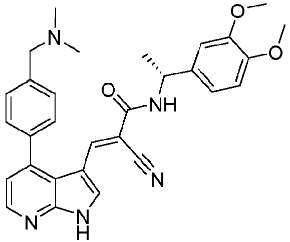 | 509 | 510 |
| **178** | 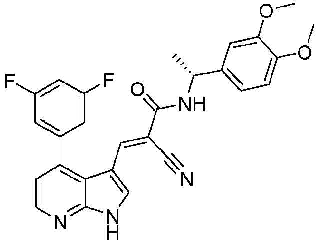 | 488 | 489 |
| **179** | 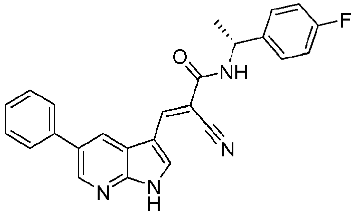 | 410 | 411 |
| **180** | 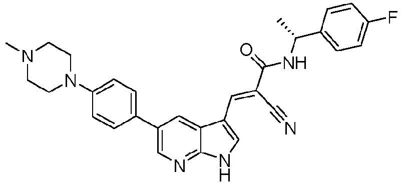 | 508 | 509 |
| **181** | 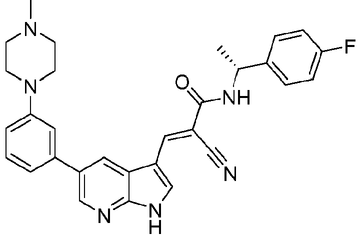 | 508 | 509 |
| **182** | 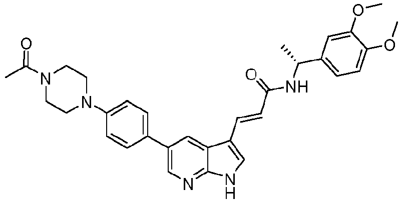 | 553 | 554 |
| **183** | 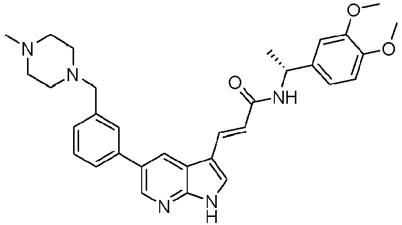 | 539 | 540 |
| **184** | 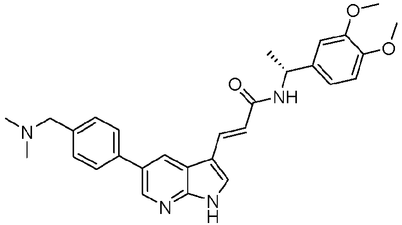 | 484 | 485 |
| **185** | 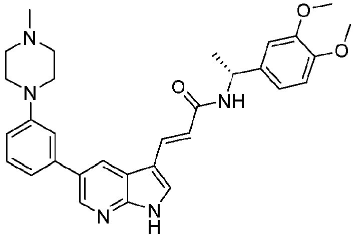 | 525 | 526 |
| **186** | 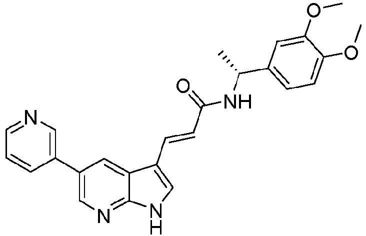 | 428 | 429 |
| **187** | 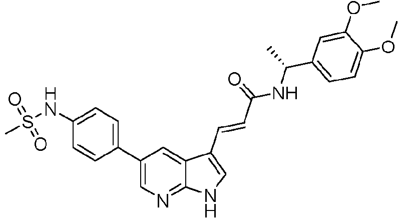 | 520 | 521 |
| **188** | 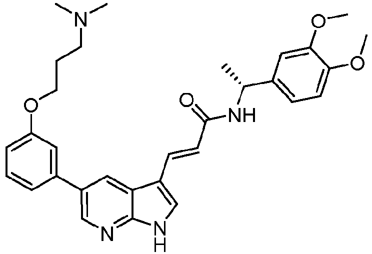 | 528 | 529 |
| **189** | 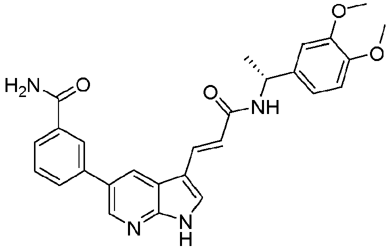 | 470 | 471 |
| **190** | 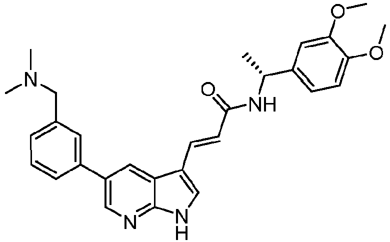 | 484 | 485 |
| **191** | 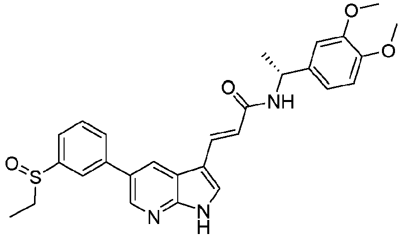 | 503 | 504 |
| **192** | 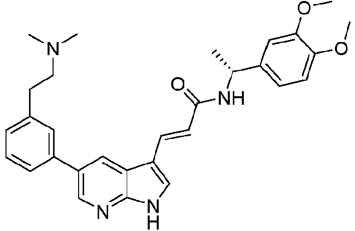 | 498 | 499 |
| **193** | 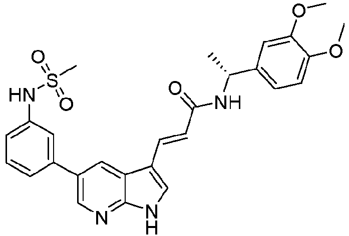 | 520 | 521 |
| **194** | 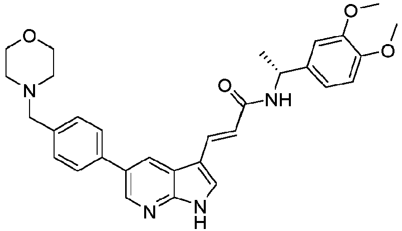 | 526 | 527 |
| **195** | 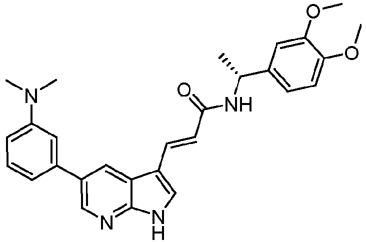 | 470 | 471 |
| **196** | 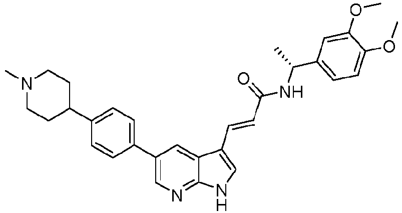 | 524 | 525 |
| **197** | 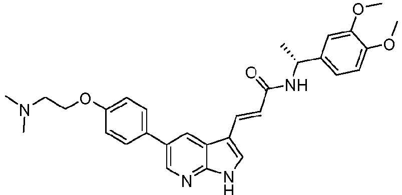 | 514 | 515 |
| **198** | 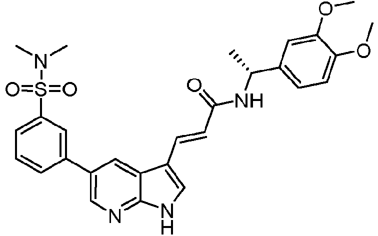 | 534 | 535 |
| **199** | 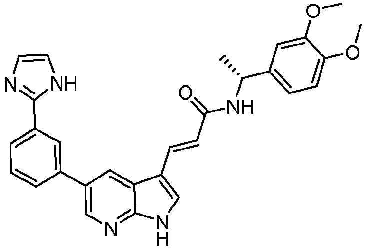 | 493 | 494 |
| **200** | 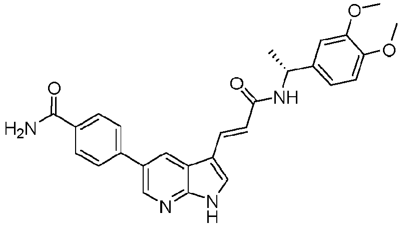 | 470 | 471 |
| **201** | 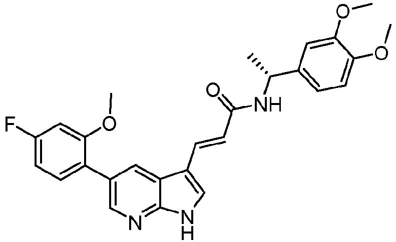 | 475 | 476 |
| **202** | 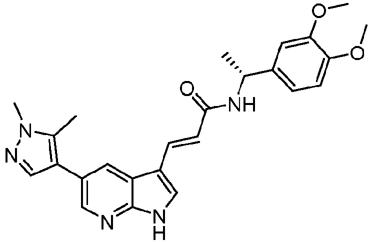 | 445 | 446 |
| **203** | 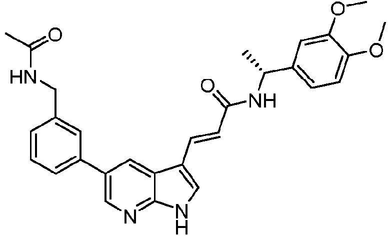 | 498 | 499 |
| **204** | 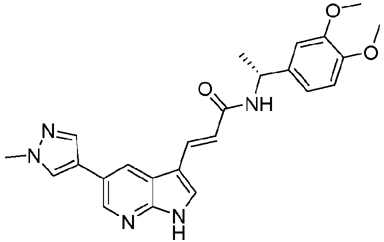 | 431 | 432 |
| **205** | 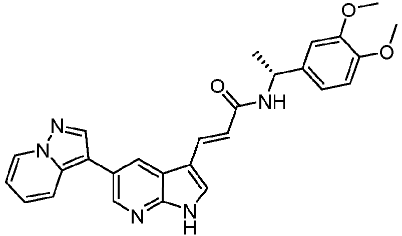 | 467 | 468 |
| **206** | 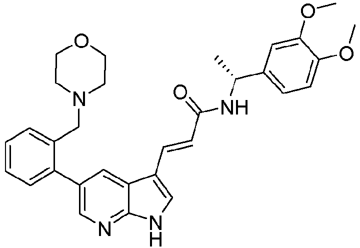 | 526 | 527 |
| **207** | 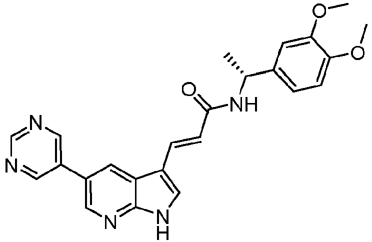 | 429 | 430 |
| **208** | 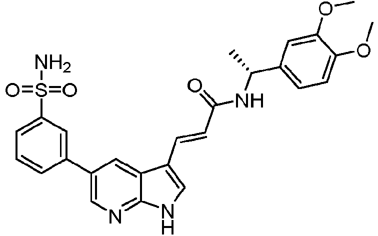 | 506 | 507 |
| **209** | 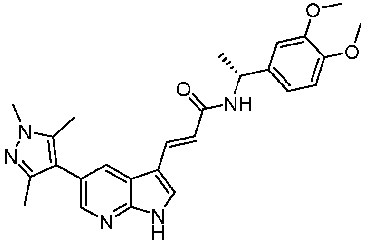 | 459 | 460 |
| **210** | 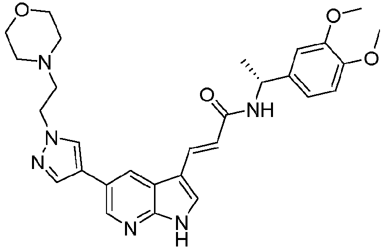 | 530 | 531 |
| **211** | 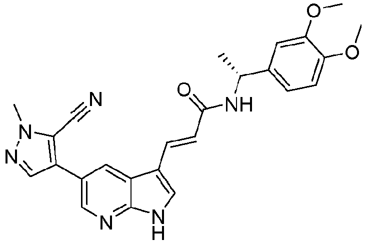 | 456 | 457 |
| **212** | 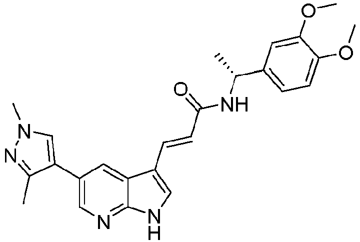 | 445 | 446 |
| **213** | 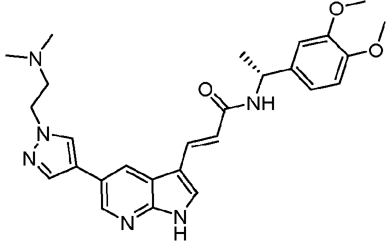 | 488 | 489 |
| **214** | 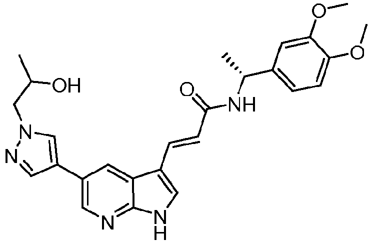 | 475 | 476 |
| **215** | 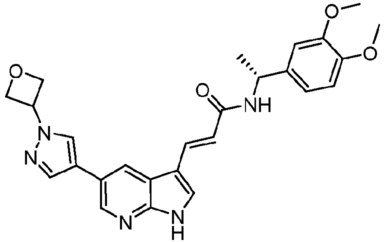 | 473 | 474 |
| **216** | 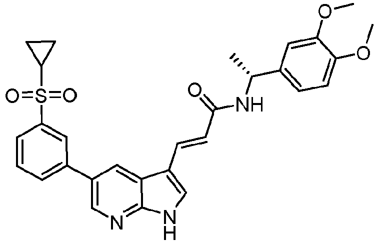 | 531 | 532 |
| **217** | 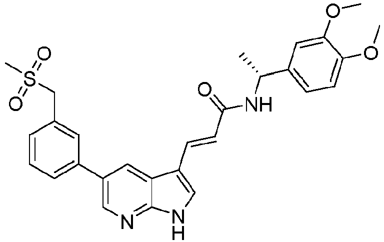 | 519 | 520 |
| **218** | 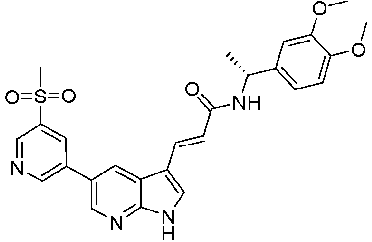 | 506 | 507 |
| **219** | 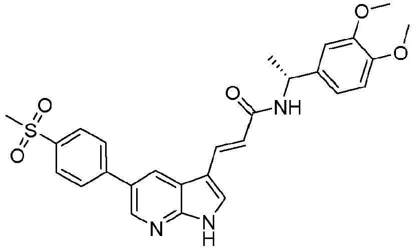 | 505 | 506 |
| **220** | 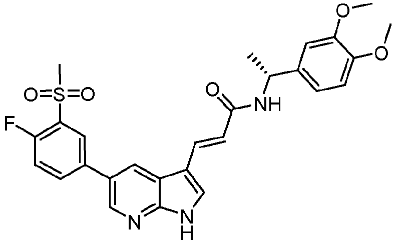 | 523 | 524 |
| **221** | 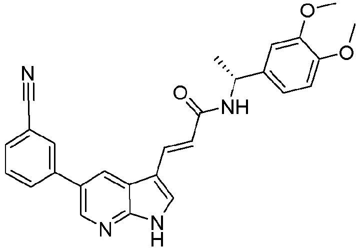 | 452 | 453 |
| **222** | 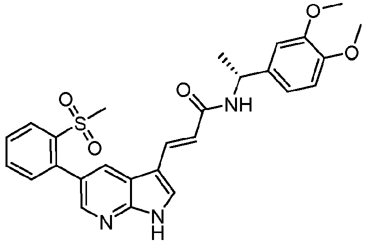 | 505 | 506 |
| **223** | 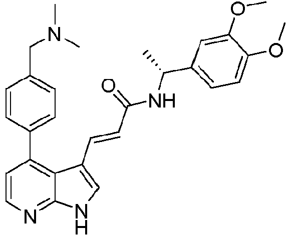 | 484 | 485 |
| **224** | 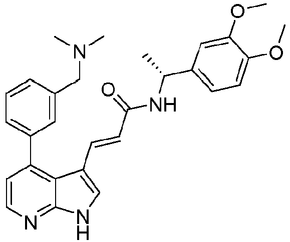 | 484 | 485 |
| **225** | 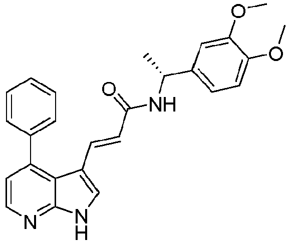 | 427 | 428 |
| **226** | 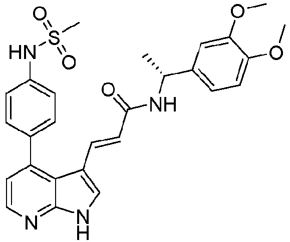 | 520 | 521 |
| **227** | 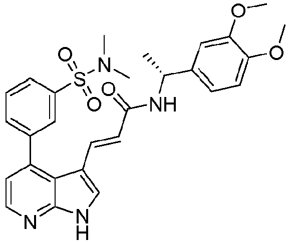 | 534 | 535 |
| **228** | 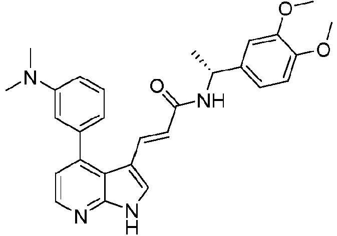 | 470 | 471 |
| **229** | 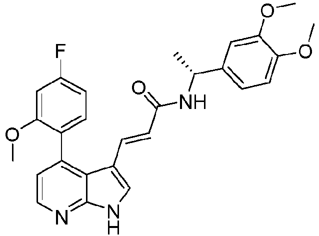 | 475 | 476 |
| **230** | 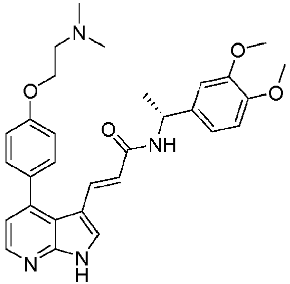 | 514 | 515 |
| **231** | 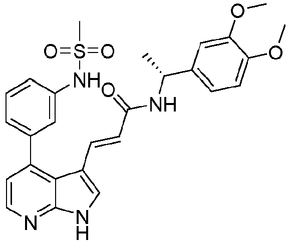 | 520 | 521 |
| **232** | 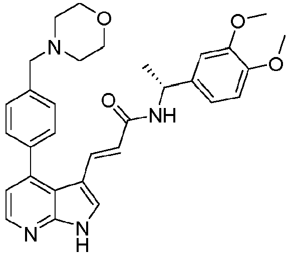 | 526 | 527 |
| **233** | 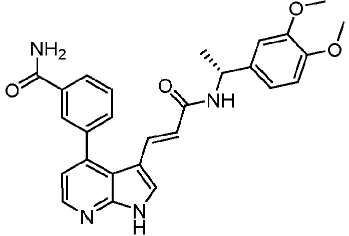 | 470 | 471 |
| **234** | 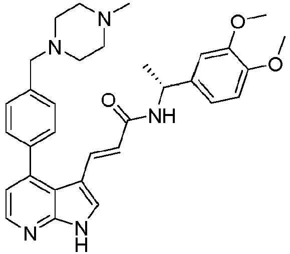 | 539 | 540 |
| **235** | 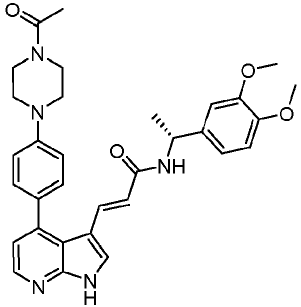 | 553 | 554 |
| **236** | 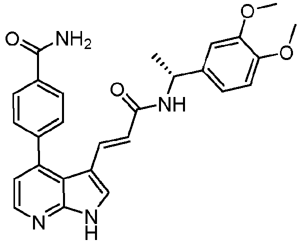 | 470 | 471 |
| **237** | 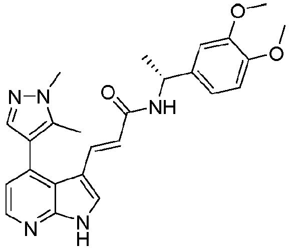 | 445 | 446 |
| **238** | 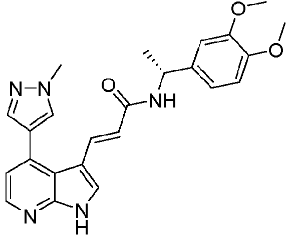 | 431 | 432 |
| **239** | 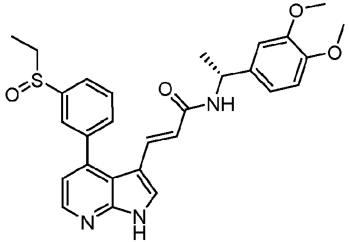 | 503 | 504 |
| **240** | 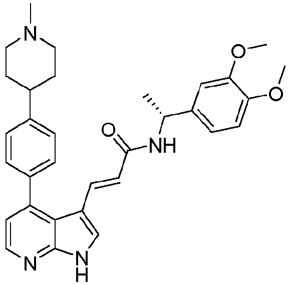 | 524 | 525 |
| **241** | 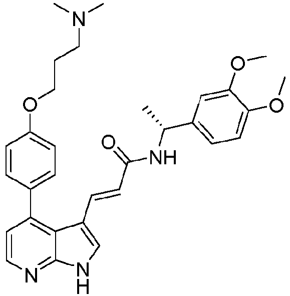 | 528 | 529 |
| **242** | 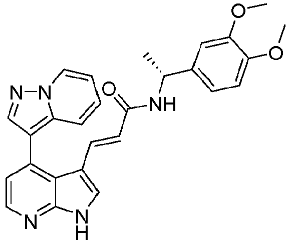 | 467 | 468 |
| **243** | 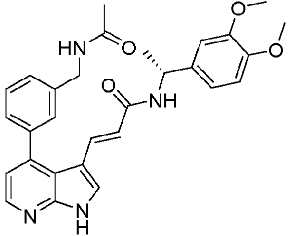 | 498 | 499 |
| **244** | 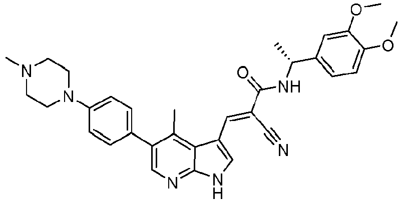 | 564 | 565 |
| **245** | 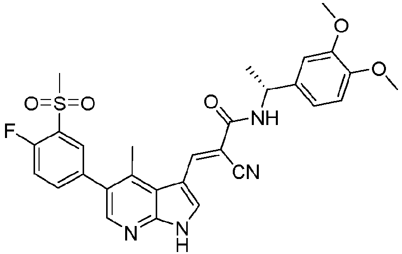 | 562 | 563 |
| **247** | 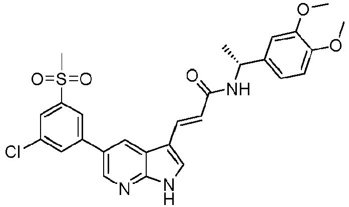 | 539 | 540 |
| **248** | 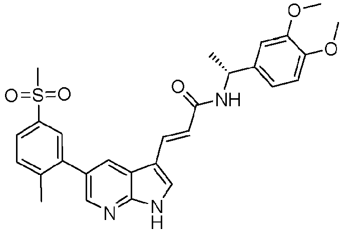 | 519 | 520 |
| **254** | 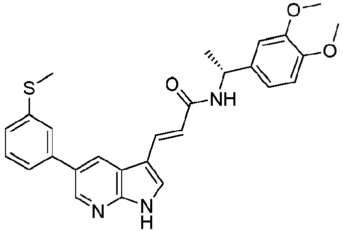 | 473 | 474 |
| **263** | 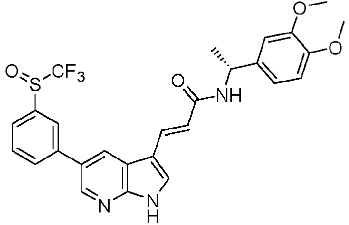 | 543 | 544 |
| **264** | 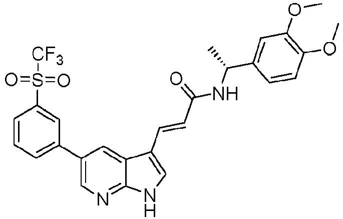 | 559 | 560 |

### EXAMPLE 249: (R,E)-N-(1-(3,4-dimethoxyphenyl)ethyl)-3-(5-(3-fluoro-5-(methylsulfonyl)phenyl)-1H-pyrrolo[2,3-b]pyridin-3-yl)acrylamide

### (Procedure H1)

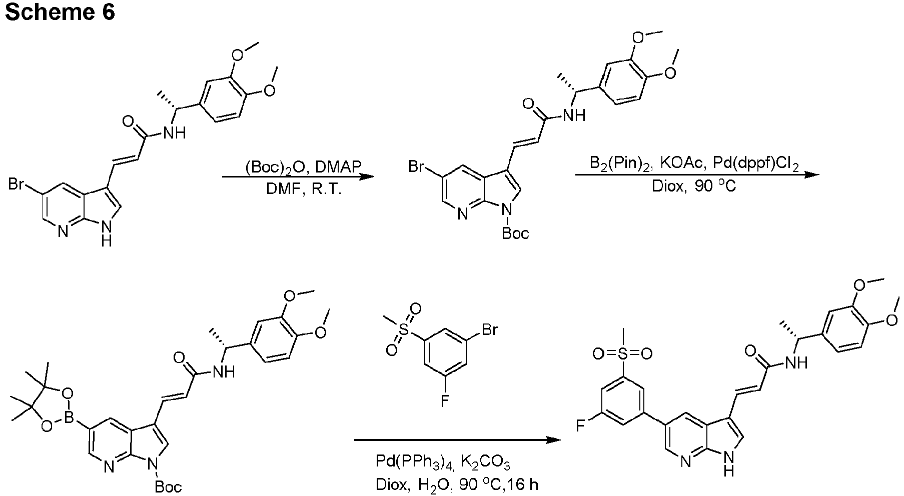

### Step 1: tert-butyl (R,E)-5-bromo-3-(3-((1-(3,4-dimethoxyphenyl)ethyl)amino)-3-oxoprop-1-en-1-yl)-1H-pyrrolo[2,3-b]pyridine-1-carboxylate

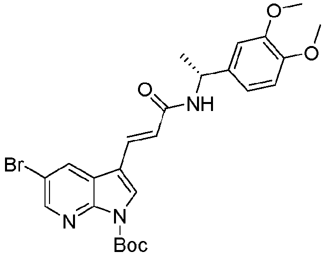

To a solution of (R,E)-3-(5-bromo-1H-pyrrolo[2,3-b]pyridin-3-yl)-N-(1-(3,4-dimethoxyphenyl)ethyl)acrylamide (500 mg, 1.16 mmol, 1.0 eq.) in dry THF (60 mL) at 0 °C was added DMAP (156 mg, 1.27 mmol, 1.1 eq.), followed by Boc₂O (301 mg, 1.40 mmol, 1.2 eq.) in three portions. After 2 min the reaction mixture was warmed to RT and stirred for 1 h. The mixture was extracted with EtOAc. The organic layer was washed with sat. brine, dried over MgSO₄ and the solvent was removed in vacuo. The crude product was purified by flash chromatography on silica gel (0-100% PE/EA) to yield the desired product **249a*** (478 mg, 77.6%). LC-MS: 431.6 [MS-99]⁺.

### Step 2: tert-Butyl (R,E)-3-(3-((1-(3,4-dimethoxyphenyl)ethyl)amino)-3-oxoprop-1-en-1-yl)-5-(4,4,5,5-tetramethyl-1,3,2-dioxaborolan-2-yl)-1H-pyrrolo[2,3-b]pyridine-1-carboxylate

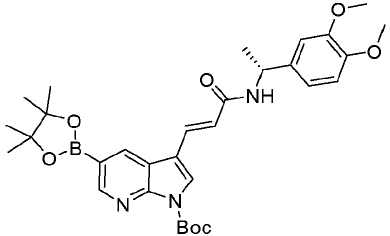

To a solution of tert-butyl (R,E)-5-bromo-3-(3-((1-(3,4-dimethoxyphenyl)ethyl)amino)-3-oxoprop-1-en-1-yl)-1H-pyrrolo[2,3-b]pyridine-1-carboxylate (94 mg, 0.17 mmol, 1.0 eq.) in dry dioxane (1 mL) was added B₂(Pin)₂ (62.77 mg, 0.25 mmol, 1.5 eq.), AcOK (33.08 mg, 0.34 mmol, 2.0 eq.) and Pd(dppf)Cl₂ (12.5 mg, 0.017 mmol, 0.1 eq.). The reaction mixture was recharged with Ar for three times and heat to 90 °C overnight. The mixture was filtered through celite and wash the cake was rinsed with EA. The filtrate was concentrated in vacuo and the crude residue was purified by flash chromatography on silical gel (PE/EA:0%~100%) to yield the product **249b*** (37 mg, 36.2 %). LC-MS: 578.3 [M+H]⁺.

### Step 3: (R,E)-N-(1-(3,4-dimethoxyphenyl)ethyl)-3-(5-(3-fluoro-5-(methylsulfonyl) phenyl)-1H-pyrrolo[2,3-b]pyridin-3-yl)acrylamide (249)

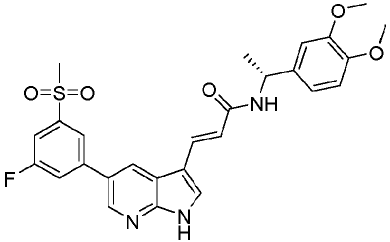

To a solution of 1-bromo-3-fluoro-5-(methylsulfonyl)benzene (148 mg, 0.26 mmol, 1.5 eq.) in dioxane/H₂O (3/1, 0.4 mL) were added tert-butyl (R,E)-3-(3-((1-(3,4-dimethoxyphenyl)ethyl)amino)-3-oxoprop-1-en-1-yl)-5-(4,4,5,5-tetramethyl-1,3,2-dioxaborolan-2-yl)-1H-pyrrolo[2,3-b]pyridine-1-carboxylate (43 mg, 0.17 mmol, 1.0 eq.), K₂CO₃ (46.92 mg, 0.34 mmol, 2.0 eq.), and Pd(PPh₃)₄(19.64 mg, 0.017 mmol, 0.1 eq.). The reaction mixture was recharged with Ar for 5 min and heated to 90 °C overnight. The mixture was purified by reversed-phase Pre-HPLC (column:C18), using aq. HCOOH and ACN as eluents. The desired fractions were collected and lyophilized to yield the final product **249** (10 mg, 7.5%). HPLC purity: 98.51%; ¹H NMR (DMSO-D₆, 400 MHz) *δ* 12.23 (s, 1H), 8.64 (d, J = 2.1 Hz, 1H), 8.50 (d, J = 2.2 Hz, 1H), 8.12 (d, *J* = 8.2 Hz, 1H), 8.06 (t, J = 1.6 Hz, 1H), 7.99 (dt, *J₁* = 10.0 Hz, *J₂* = 2.2 Hz, 1H), 7.93 (d, J = 1.7 Hz, 1H), 7.74 - 7.70 (m, 1H), 7.56 (d, J = 15.9 Hz, 1H), 6.90 (d, J = 2.0 Hz, 1H), 6.84 (d, J = 8.3 Hz, 1H), 6.81 (dd, *J₁* = 8.3 Hz, *J₂* = 1.9 Hz, 1H), 6.70 (d, J = 15.9 Hz, 1H), 4.99 (s, 1H), 3.69 (s, 3H), 3.65 (s, 3H), 3.32 (s, 3H), 1.35 (d, J = 7.0 Hz, 3H); LC-MS: 524.1 [M+H]⁺.

**The examples in the following table 1E were prepared according to the procedure H1 described for Example 249:**

**Table 1E**

| **Example** | **Structure** | **M** | **[M+H]⁺** |
|---|---|---|---|
| **250** | 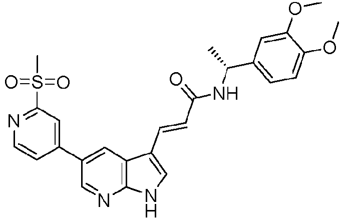 | 506 | 507 |
| **251** | 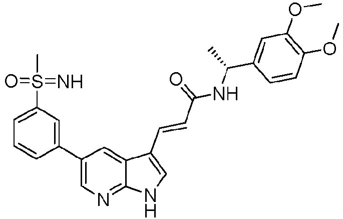 | 504 | 505 |
| **259** | 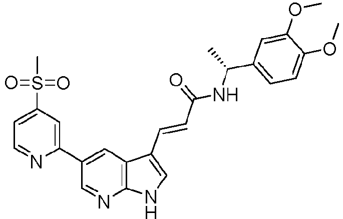 | 506 | 507 |

### Preparative examples of intermediates

### Example intermediate I-A-1:

### 5-((1,1-dioxidotetrahydro-2H-thiopyran-4-yl)oxy)-1H-pyrrolo[2,3-b]pyridine-3-carbaldehyde (E1)

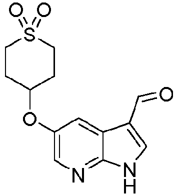

4-((1H-pyrrolo[2,3-b]pyridin-5-yl)oxy)tetrahydro-2H-thiopyran 1,1-dioxide (190 mg, 0.71 mmol, 1 eq.) was suspended in acetic acid/H₂O (1:3, 12 mL). Hexamethylentetramine (200 mg, 1.4 mmol, 2 eq.) was added and the reaction mixture was stirred for 12 h at 120 °C. The solution was diluted with EtOAc and washed tree times with aq. sat. NaHCO₃-solution. The organic layer was dried over MgSO₄ and solvent was removed in vacuo. The crude product was purified by flash chromatography on silica gel (0-100 % EtOAc/cHex, 0-100 % MeOH/DCM) to yield the desired product **E1** (90 mg, 0.31 mmol, 44 %).

¹H NMR (300MHz, d₆-DMSO, 300K) δ 12.65 (s, 1H), 9.89 (s, 1H), 8.44 (s, 1H), 8.21 (d, *J* = 2.7 Hz, 1H), 8.06 (d, *J* = 2.8 Hz, 1H), 4.86-4.66 (m, 1H), 3.29-3.08 (m, 4H), 2.33-2.14 (m, 4H). MS (ES) C₁₃H₁₄N₂O₄S requires: 294, found: 295 (M+H)⁺.

The examples in the following table were prepared according to the procedure described for **E1** (**I-A-1**).

| **Example** | **Structure** | **M** | **[M+H]⁺** |
|---|---|---|---|
| **I-A-2** | 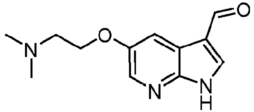 | 233 | 234 |
| **I-A-3** | 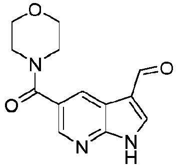 | 259 | 260 |
| **I-A-4** | 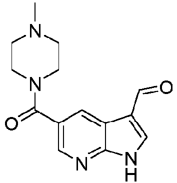 | 272 | 273 |
| **I-A-5** | 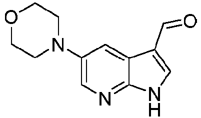 | 231 | 232 |
| **I-A-6** | 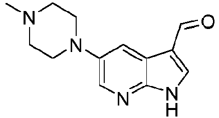 | 244 | 245 |
| **I-A-7** | 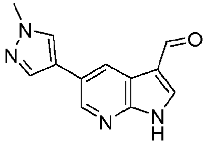 | 226 | 227 |
| **I-A-8** | 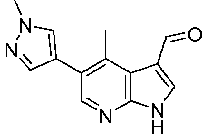 | 240 | 241 |
| **I-A-9** | 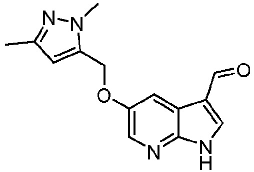 | 270 | 271 |
| **I-A-10** | 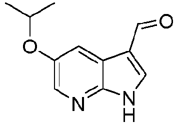 | 204 | 205 |
| **I-A-11** | 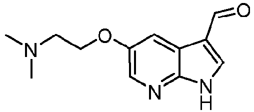 | 233 | 234 |
| **I-A-12** | 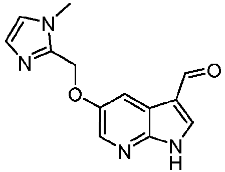 | 256 | 257 |

### Example intermediate I-B-1:

### (E)-3-(5-((1,1-dioxidotetrahydro-2H-thiopyran-4-yl)oxy)-1H-pyrrolo[2,3-b]pyridin-3-yl)acrylic acid (F1)

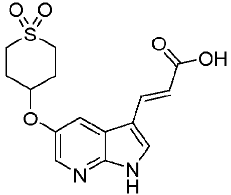

To a solution of 5-((1,1-dioxidotetrahydro-2H-thiopyran-4-yl)oxy)-1H-pyrrolo[2,3-b]pyridine-3-carbaldehyde (37 mg, 0.13 mmol, 1 eq.) in pyridine (4 mL), piperidine (0.2 mL) and malonic acid (37 mg, 0.35 mmol, 2.8 eq.) were added and the reaction mixture was stirred for 12 h at 120 °C. The solution was diluted with EtOAc and washed tree times with aq. sat. NaHCO₃-solution. The organic layer was dried over MgSO₄ and solvent was removed in vacuo. The crude product was purified by flash chromatography on silica gel (0-100 % EtOAc/cHex, 0-100 % MeOH/DCM) to yield the desired product **F1** (34 mg, 0.10 mmol, 77 %). MS (ES) C₁₅H₁₆N₂O₅S requires: 336, found: 337 (M+H)⁺.

The examples in the following table were prepared according to the procedure described for **F1** (**I-B-1**)**.**

| **Example** | **Structure** | **M** | **[M+H]⁺** |
|---|---|---|---|
| **I-B-2** | 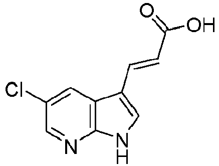 | 222 | 223 |
| **I-B-3** | 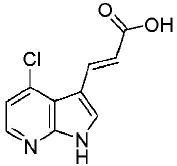 | 222 | 223 |
| **I-B-4** | 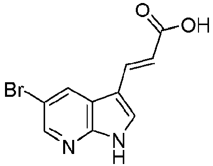 | 266 | 267 |
| **I-B-5** | 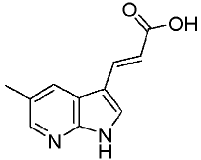 | 202 | 203 |
| **I-B-6** | 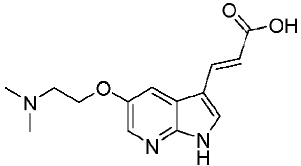 | 275 | 276 |

### Example intermediate I-C-1:

### (R,E)-2-cyano-N-(1-(3,4-dimethoxyphenyl)ethyl)-3-(5-hydroxy-1H-pyrrolo[2,3-b]pyridin-3-yl)acrylamide (G4 = Example 81)

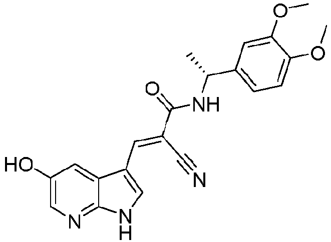

(R,E)-3-(5-(Allyloxy)-1H-pyrrolo[2,3-b]pyridin-3-yl)-2-cyano-N-(1-(3,4-dimethoxyphenyl)ethyl)acrylamide (57 mg, 0.132 mmol, 1.0 eq.) was dissolved in MeOH (3 mL). Barbituric acid (34 mg, 0.264 mmol, 2.0 eq.) and tetrakis(triphenylphosphine) palladium(0) (15.2 mg, 0.013 mmol, 0.1 eq.) were added to the solution. The reaction mixture was stirred for 30 min at 45 °C. After a complete reaction, the solvent was removed in vacuo and the crude was purified by flash chromatography on silica gel (0-100 % EtOAc/cHex) to yield the desired product **G4** (27 mg, 0.069 mmol, 52 %) as a yellowish solid.

¹H NMR (400MHz, d₆-DMSO, 300K) δ 12.62 (s, 1H), 9.61 (s, 1H), 8.50 (d, J = 8.0 Hz, 1H), 8.39 (s, 1H), 8.31 (s, 1H), 7.99 (d, J = 2.6 Hz, 1H), 7.68 (d, J = 2.6 Hz, 1H), 7.02 (s, 1H), 6.91-6.90 (m, 2H), 5.08-4.98 (m, 1H), 3.77 (2, 3H), 3.73 (s, 3H), 1.47 (d, J = 7.0 Hz, 3H). MS (ES) C₂₁H₂₀N₄O₄ requires: 392, found: 393 (M+H)⁺.

### Step 1: 5-(allyloxy)-1H-pyrrolo[2,3-b]pyridine (G1)

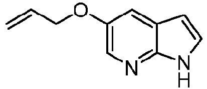

1*H*-Pyrrolo[2,3-*b*]pyridin-5-ol (5.8 g, 43 mmol, 1.0 eq.) was dissolved in dry THF (20 mL). Allyl alcohol (3 g, 52 mmol, 1.2 eq.) and triphenylphosphine (13.7 g, 52 mmol, 1.2 eq.) were added to the solution. DIAD (10.5 g, 52 mmol, 1.2 eq.) was added dropwise via syringe at 0 °C over 5 min. After 10 min the reaction mixture was allowed to warm to RT. The reaction was stirred for 1 h. The reaction was quenched with drops of H₂O and the solvents were removed in vacuo. The crude product was purified by flash chromatography on silica gel (0-100 % EtOAc/ cHex) to yield the desired product **G1.** MS (ES) C₁₀H₁₀N₂O requires: 174, found: 175 (M+H)⁺.

### Step 2: 5-(allyloxy)-1H-pyrrolo[2,3-b]pyridine-3-carbaldehyde (G2)

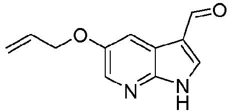

**G1** (50 mg, 0.29 mmol, 1 eq.) was suspended in acetic acid/H₂O (1:3, 5mL). Hexamethylentetramine (80 mg, 0.57 mmol, 2 eq.) was added and the reaction mixture was stirred for 12 h at 110 °C. After a complete reaction, the solution was diluted with EtOAc and washed tree times with aq. sat. NaHCO₃-solution. The organic layer was dried over MgSO4 and solvent was removed in vacuo.

The crude product was purified by flash chromatography on silica gel (0-100 % EtOAc/ cHex) to yield the desired product **G2** (35 mg, 0.17 mmol, 60 %) as a white solid. MS (ES) C₁₁H₁₀N₂O₂ requires: 202, found: 203 (M+H)⁺.

### Step 3: (R,E)-3-(5-(allyloxy)-1H-pyrrolo[2,3-b]pyridin-3-yl)-2-cyano-N-(1-(3,4-dimethoxyphenyl)ethyl)acrylamide (G3)

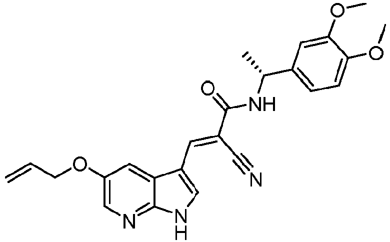

**G2** (30 mg, 0.19 mmol, 1 eq.) and (*R*)-2-cyano-N-(1-(3,4-dimethoxyphenyl)ethyl)acetamide (41 mg, 0.2 mmol, 1.1 eq.) was dissolved in pyridine (2 mL). The reaction mixture was stirred for 48 h at 120 °C. The solvent was removed in vacuo and the crude was purified by flash chromatography on silica gel (0-100 % EtOAc/cHex) to yield the desired product **G3** (57 mg, 0.13 mmol, 89 %) as a yellowish solid. ¹H NMR (400MHz, d₆-DMSO, 300K) δ 12.74 (s, 1H), 9.00-8.33 (m1, 3H), 8.16-7-86 (m, 2H), 7.02 (s, 1H), 6.97-6.79 (m, 2H), 6.15-5.99 (m, 1H), 5.42 (d, *J* = 17.3 Hz, 1H), 5.27 (d, *J* = 10.5 Hz, 1H), 5.08-4.96 (m, 1H), 4.69-4.59 (m, 2H), 3.75 (s, 1H), 3.71 (s, 1H), 1.49-1.34 (m, 3H). MS (ES) C₂₄H₂₄N₄O₄ requires: 432, found: 433 (M+H)⁺.

### Example intermediate I-H-1:

### 4,4,5,5-tetramethyl-2-(3-((trifluoromethyl)sulfinyl)phenyl)-1,3,2-dioxaborolane

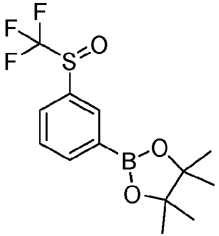

To a solution of 1-bromo-3-((trifluoromethyl)sulfinyl)benzene **H1)** (150 mg, 0.35 mmol, 1.0 eq.) in dioxane were added diboron pinacol ester (104.76 mg, 0.42 mmol, 1.2 eq.), potassium acetate (68.70 mg, 0.70 mmol, 2.0 eq.), and Pd(dppf)Cl₂ (21.95 mg, 0.03 mmol, 0.1 eq.). The reaction mixture was recharged with Ar for three times, and heated to 100 °C with vigorous stirring for 12 h. The mixture was then cooled to room temperature, diluted with water (5 mL), and extracted with EtOAc (5 mL x 3). The combined organic layers were washed with brine (10 mL), and dried over Na₂SO₄. The solvent was removed in vacuo and the crude residue was purified by flash chromatography (0-100% EtOAc/PE) to afford the product **H2** (135.1 mg, 76.1%). LC-MS: 321.1 [M+H]⁺.

### Step 1: 1-Bromo-3-((trifluoromethyl)sulfinyl)benzene (H1)

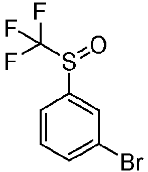

To a solution of (3-bromophenyl)(trifluoromethyl)sulfane (200 mg, 0.78 mmol, 1.0 eq.) in dry CH₃CN (1 mL) was added m-CPBA (214.8 mg, 0.93 mmol, 1.2 eq.). The reaction mixture was heated to 40 °C for 12 h, then cooled to room temperature, diluted with aq. Na₂SO₃ (5 mL) and extracted with EtOAc (5 mL x 3). The combined organic layers were washed with brine (10 mL) and dried over Na₂SO₄. The solvent was removed in vacuo and the residue was purified by flash chromatography (0-100% EtOAc/PE) to provide the product **H1** (65.5 mg, 30.7%). LC-MS: 272.9 [M+H]+.

### Example intermediate I-J-1:

### 1-(5,6-dimethoxypyridin-3-yl)ethan-1-amine

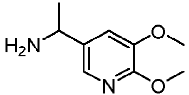

To a solution of (S)-N-(1-(5,6-dimethoxypyridin-3-yl)ethyl)-2-methylpropane-2-sulfinamide **J2** (150 mg, 0.51 mmol, 1.0 eq.) in DCM (1 mL) was added 4 N HCl in 1,4-dioxane (0.7 mL, 2.61 mmol, 4 M, 5.0 eq.). The reaction was stirred at room temperature and monitored by LC-MS. The solvent was removed and the crude product **J3** (HCl salt) was used directly in the next step. LC-MS: 183.0 [M+H]+.

### Step 1: (S,E)-N-((5,6-dimethoxypyridin-3-yl)methylene)-2-methylpropane-2-sulfinamide (J1)

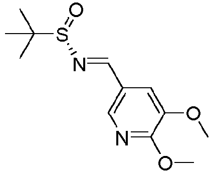

A solution of 5,6-dimethoxynicotinaldehyde (500 mg, 3.0 mmol, 1.0 eq.), (S)-2-methylpropane-2-sulfinamide (440 mg, 3.6 mmol, 1.2 eq.) and Ti(OEt)4 (2.7 g, 9.0 mmol, 2.0 eq., containing 34%wt TiO₂) in dry THF (10 mL) was stirred at room temperature overnight. Water and EtOAc were added, and the insoluble solid was filtered off. The filtrate was transferred to separatory funnel and the organic layer was separated, washed with sat. brine, and dried over Na₂SO₄. The solvent was removed in vacuo and the crude residue was purified by flash chromatography on silica gel (PE/EA: 0% ~ 9%) to provide the product **J1** (642 mg, 79.4%). 1H NMR (DMSO-D6, 400 MHz) δ 8.60 (s, 1H), 8.34 (d, J = 1.8 Hz, 1H), 7.71 (d, J = 1.8 Hz, 1H), 4.01 (s, 3H), 3.92 (s, 3H), 1.24 (s, 9H).

### Step 2: (S)-N-(1-(5,6-Dimethoxypyridin-3-yl)ethyl)-2-methylpropane-2-sulfinamide (J2)

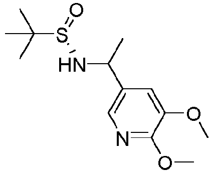

To a solution of (S,E)-N-((5,6-dimethoxypyridin-3-yl)methylene)-2-methylpropane-2-sulfinamide (642 mg, 2.4 mmol, 1.0 eq.) in dry THF (12 mL) at -50 °C under Ar atmosphere was added lithium methide (1.3 mL, 3.2 mmol, 2.7 M in DEM, 1.5 eq.) dropwise. The reaction mixture was stirred at -50 °C and monitored by LC-MS. After completion, saturated NH₄Cl (aq.) was added slowly to quench the reaction. The mixture was extracted with EtOAc, and the organic layer was washed with sat. brine, dried over Na₂SO₄. The solvent was removed in vacuo to give the crude product **J2** (648.3 mg, two diastereoisomers 1:1) which was used in the next step without further purification. 1H NMR (DMSO-D6, 400 MHz) δ 7.71 (dd, J1 = 14.5 Hz, J2 = 1.8 Hz, 1H), 7.36 (dd, J1 = 38.2 Hz, J2 = 1.9 Hz, 1H), 5.55 (dd, J1 = 91.4 Hz, J2 = 6.1 Hz, 1H), 4.31-4.48 (m, 1H), 3.90 (d, J = 1.3 Hz, 3H), 3.83 (d, J = 7.6 Hz, 3H), 1.50 (dd, J1 = 17.8 Hz, J2 = 6.8 Hz, 3H), 1.17 (d, J = 4.7 Hz, 9H).

### Example intermediate I-K-1:

### 1-(3-Methylbenzo[d]isoxazol-5-yl)ethan-1-one

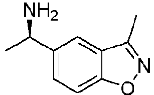

To a solution of **K6** (1.50 g, 7.05 mmol, 1.0 eq.) in 1,4-dioxane (10 mL) at 0 °C was added 4 N HCl in 1,4-dioxane (10 mL). The mixture was stirred at 0 °C for 4 h. The solvent was removed in vacuo and the crude product **K7** (HCl salt) was used in the next step without further purification. LC-MS: 160.0 [M-NH2]⁺

### Step: 1: 1-(5-Bromo-2-hydroxyphenyl)ethan-1-one oxime (K1)

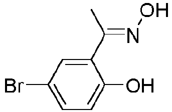

To a solution of 1-(5-bromo-2-hydroxyphenyl)ethan-1-one (10.0 g, 46.73 mmol, 1.0 eq.) in ethanol (60 mL) was added hydroxylamine hydrochloride (4.87 g, 70.10 mmol, 1.5 eq.). The reaction mixture was stirred at 80 °C for 16 h, then cooled to RT. The solvent was removed in vacuo and the crude product was purified by flash chromatography on silica gel (0-100% EtOAc/PE, 0-10 % MeOH/DCM) to yield the product **K1** (8.7 g, 82%). LC-MS: 229.6 [M+H]⁺.

### Step 2: 5-Bromo-3-methylbenzo[d]isoxazole (K2)

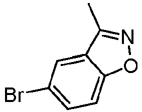

To a solution of 1-(5-bromo-2-hydroxyphenyl)ethan-1-one oxime (8.7 g, 38.00 mmol, 1.0 eq.) in DMA (60 mL) at 0 °C were added acetic anhydride (4.26 g, 41.8 mmol, 1.1 eq.) and K₂CO₃ (2.89 g, 20.9 mmol, 0.5 eq.). The reaction mixture was stirred at 100°C for 5 h, cooled to RT, then water (60 mL) and DCM (60 mL) were added. The organic layer was separated and washed with sat. brine, dried over Na₂SO₄. The solvent was removed in vacuo and the crude product was purified by flash chromatography on silica gel (0-60% EtOAc/PE) to yield the product **K2** (5.8 g, 72.4%). LC-MS: 211.6 [M+H]⁺.

### Step 3: 5-(1-Ethoxyvinyl)-3-methylbenzo[d]isoxazole (K3)

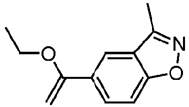

A solution of 5-bromo-3-methylbenzo[d]isoxazole (5.8 g, 27.49 mmol, 1.0 eq.), tributyl(1-ethoxyvinyl)tin (5.46 g, 41.24 mmol, 1.5 eq.), and Pd(PPh₃)₄ (1.59 g, 1.37 mmol, 0.05 eq) in 1,4-dioxane (80 mL) was recharged with Ar for three times and stirred at 100 °C overnight. The reaction mixture was cooled to room temperature and aq. sat. potassium fluoride (200 mL) was added. The mixture was filtered through celite, and sat. NH₄Cl (aq.) and EtOAc were added. The organic layer was separated and washed with sat. brine, dried over Na₂SO₄. The solvent was removed in vacuo and the crude residue was purified by flash chromatography on silica gel (0-55% PE/EtOAc) to yield the product **K3** (2.83 g, 50.7%). LC-MS: 203.6 [M+H]⁺.

### Step 4: 1-(3-Methylbenzo[d]isoxazol-5-yl)ethan-1-one (K4)

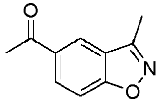

To a solution of 5-(1-ethoxyvinyl)-3-methylbenzo[d]isoxazole (2.83 g, 13.93 mmol, 1.0 eq.) in 1,4-dioxane/water (10 mL /1 mL) was added HCl in 1,4-dioxane (2 mL) at 0 °C. The mixture was stirred at 0 °C for 4 h. The solvent was removed in vacuo and the crude product **K4** was used in the next step without further purification. LC-MS: 175.6 [M+H]⁺.

### Step 5: (R,E)-2-Methyl-N-(1-(3-methylbenzo[d]isoxazol-5-yl)ethylidene)propane-2-sulfinamide (K5)

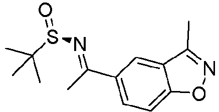

To a solution of 1-(3-methylbenzo[d]isoxazol-5-yl)ethan-1-one (1.80 g, 10.19 mmol, 1.0 eq.) and (*R*)-2-methylpropane-2-sulfinamide (1.85 g, 15.29 mmol, 1.5 eq.) in THF (60 mL) was added Ti(OEt)₄ (8.8 g, 25.48 mmol, 2.5 eq., containing 34%wt TiO₂). The mixture was recharged with N₂ balloon for three times and stirred at 70 °C overnight. The mixture containing **K5** was used directly in the next step. LC-MS: 279.1 [M+H]⁺.

### Step 6: (R)-2-Methyl-N-((R)-1-(3-methylbenzo[d]isoxazol-5-yl)ethyl)propane-2-sulfinamide (K6)

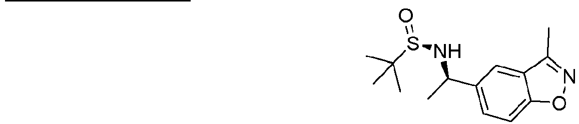

The above solution containing **K5** was cooled to -50 °C, then NaBH₄ (3.90 g, 102.5 mmol, 10.0 eq.) was added. The mixture was recharged with N₂ balloon for three times and stirred at -50 °C for 12 h. Upon completion, sat. brine (100 mL) was added and the mixture was filtered through celite. Saturated NH₄Cl (aq.) and EtOAc were added to the filtrate. The organic layer was separated and washed with sat. brine, dried over Na₂SO₄. The solvent was removed in vacuo and the crude residue was purified by flash chromatography on silica gel (0-55% EtOAc/PE) to yield the product **K6** (1.30 g, 46.6%). LC-MS: 281.1 [M+H]⁺.

### Example intermediate I-L-1:

### 1-(3-Cyclopropyl-4-methoxyphenyl)ethan-1-amine (L4)

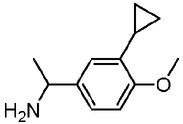

To a solution of (S)-N-(1-(3-cyclopropyl-4-methoxyphenyl)ethyl)-2-methylpropane-2-sulfinamide (170 mg, 0.58 mmol, 1.0 eq.) in DCM (1.5 mL) was added 4 N HCl in 1,4-dioxane (0.72 mL, 2.88 mmol, 4 M, 5.0 eq.). The reaction was stirred at room temperature and monitored by LC-MS. After completion, the solvent was removed in vacuo and the crude product **L4** (HCl salt) was used directly in the next step. LC-MS: 175.0 [M-NH₂]⁺.

### Step 1: (S)-N-(3-Bromo-4-methoxybenzylidene)-2-methylpropane-2-sulfinamide (L1)

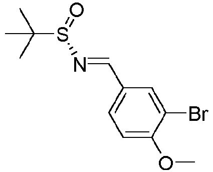

A solution of 3-bromo-4-methoxybenzaldehyde (1 g, 4.65 mmol, 1.0 eq.), (S)-2-methylpropane-2-sulfinamide (676.3 mg, 5.58 mmol, 1.2 eq.) and Ti(OEt)₄ (4.82 g, 13.95 mmol, 3.0 eq., containing 34%wt TiO₂) in dry THF (15 mL). was stirred at room temperature for 2.5 h. Water and EtOAc were added, and insoluble solid was filtered off through celite. The two layers of the obtained clear liquid was separated. The organic layer was washed with sat. brine, dried over Na₂SO₄. The solvent was removed in vacuo and the residue was purified by flash chromatography on silica gel (0-30% EtOAc/PE) to give the product **I-L1** (1.19 g, 80.2%). LC-MS: 318.2 [M+H]⁺.

### Step 2: (S)-N-(1-(3-Bromo-4-methoxyphenyl)ethyl)-2-methylpropane-2-sulfinamide (L2)

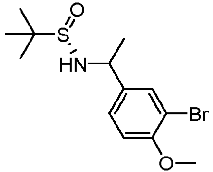

To a solution of (S,E)-N-(3-bromo-4-methoxybenzylidene)-2-methylpropane-2-sulfinamide (963.3 mg, 3.03 mmol, 1.0 eq.) in dry THF (15 mL) at -50 °C was added lithium methide (2.2 mL, 6.06 mmol, 2.7 M in DEM, 2.0 eq.) dropwise. The reaction mixture was kept at -50 °C and monitored by LC-MS. After completion of the reaction, sat. NH₄Cl (aq.) was added slowly to quench the reaction. The mixture was extracted with EtOAc, and the organic layer was washed with sat. brine, dried over Na₂SO₄. The solvent was removed in vacuo to give the crude product **L2** (1.09 g, two diastereoisomers 1:1) which was used in the next step without purification. LC-MS: 334.0 [M+H]⁺.

### Step 3: (S)-N-(1-(3-Cyclopropyl-4-methoxyphenyl)ethyl)-2-methylpropane-2-sulfinamide (L3)

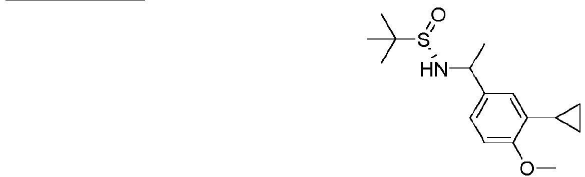

A suspension of (S)-N-(1-(3-bromo-4-methoxyphenyl)ethyl)-2-methylpropane-2-sulfinamide (360 mg, 1.08 mmol, 1.0 eq.), 2-cyclopropyl-4,4,5,5-tetramethyl-1,3,2-dioxaborolane (272 mg, 1.62 mmol, 1.5 eq.), anhydrous Na₂CO₃ (228.3 mg, 2.15 mmol, 2.0 eq.) and Pd(dppf)Cl₂ (79 mg, 0.11 mmol, 0.1 eq.) in 1,4-dioxane/water (4.2/1.4 mL) was heated at 90 °C overnight. The mixture was cooled to room temperature, and water and EtOAc were added. The organic phase was separated, washed with sat. brine, dried over Na₂SO₄. The solvent was removed in vacuo and the crude residue was purified by flash chromatography on silica gel (0-50% EtOAc/PE) to afford the product **L3** (170 mg, 53.4%). LC-MS: 296.0 [M+H]⁺.

### Example intermediate I-M-1:

### 1-(3-Methoxy-4-(methylsulfinyl)phenyl)ethan-1-amine (M6)

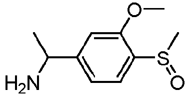

To a solution of tert-butyl (1-(3-methoxy-4-(methylsulfinyl)phenyl)ethyl)carbamate **(M5)** (310 mg, 0.99 mmol, 1.0 eq.) in DCM (4.5 mL) was added 4 N HCl in 1,4-dioxane (1.24 mL, 4.95 mmol, 4 M, 5.0 eq.). The reaction mixture was stirred at room temperature and monitored by LC-MS. The solvent was removed in vacuo to yield the crude product **M6** (HCl salt), which was used directly in the next step. LC-MS: 214.0 [M+H]⁺.

### Step 1: (S)-N-(3-Methoxy-4-(methylthio)benzylidene)-2-methylpropane-2-sulfinamide (M1)

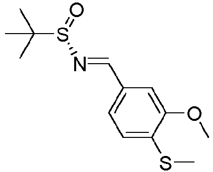

A solution of 3-methoxy-4-(methylthio)benzaldehyde (0.5 g, 2.74 mmol, 1.0 eq.), (S)-2-methylpropane-2-sulfinamide ( 399 mg, 3.29 mmol, 1.2 eq.) and Ti(OEt)₄ (1.9 g, 5.48 mmol, 2.0 eq., containing 34%wt TiO₂) was stirred at room temperature for 2.5 h. Water and EtOAc were added, and the insoluble solid was filtered off through celite. The two layers of the obtained clear liquid was separated. The organic layer was washed with sat. brine, dried over Na₂SO₄. The solvent was removed in vacuo and the residue was purified by flash chromatography on silica gel (0-40% EtOAc/PE) to afford the product **M1** (0.65 g, 83.1%). LC-MS: 286.1 [M+H]⁺.

### Step 2: (S)-N-(1-(3-Methoxy-4-(methylthio)phenyl)ethyl)-2-methylpropane-2-sulfinamide (M2)

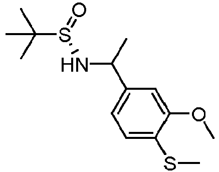

To a solution of (S)-N-(3-methoxy-4-(methylthio)benzylidene)-2-methylpropane-2-sulfinamide (650 mg, 2.28 mmol, 1.0 eq.) in dry THF (12 mL) at -50 °C was added lithium methide (1.3 mL, 3.41 mmol, 2.7 M in DEM, 1.5 eq.) dropwise. The reaction was kept at -50 °C and monitored by LC-MS. Upon completion, saturated NH₄Cl (aq.) was added slowly to quench the reaction. The mixture was extracted with EtOAc, and the organic layer was washed with sat. brine, dried over Na₂SO₄. The solvent was removed in vacuo to give the crude product **M2** (0.67 g, two diastereoisomers 1:1) which was used in the next step without purification. LC-MS: 301.6 [M+H]⁺.

### Step 3: 1-(3-Methoxy-4-(methylthio)phenyl)ethan-1-amine (M3)

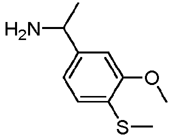

To a solution of (S)-N-(1-(3-methoxy-4-(methylthio)phenyl)ethyl)-2-methylpropane-2-sulfinamide (670 mg, 2.22 mmol, 1.0 eq.) in DCM (3 mL) was added 4 N HCl in 1,4-dioxane (2.8 mL, 11.11 mmol, 4 M, 5.0 eq.). The reaction was stirred at room temperature and monitored by LC-MS. The solvent was removed in vacuo to yield the crude product **(M3)** (HCl salt), which was used directly in the next step. LC-MS: 181.0 [M-NH₂]⁺.

### Step 4: tert-Butyl (1-(3-methoxy-4-(methylthio)phenyl)ethyl)carbamate (M4)

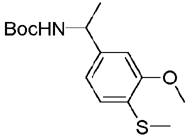

A suspension of 1-(3-methoxy-4-(methylthio)phenyl)ethan-1-amine (519 mg, 2.22 mmol, HCl salt, 1.0 eq.), (Boc)₂O (533 mg, 2.44 mmol, 1.1 eq.), and triethylamine (0.87 mL, 6.22 mmol, 2.8 eq.) in DCM (14 mL) was stirred at 0 °C. The reaction mixture was warmed up to room temperature and monitored by LC-MS. Upon completion, sat. NaHCO₃ (aq.) and DCM were added, and the organic phase was separated, washed with sat. brine, dried over Na₂SO₄. The solvent was removed in vacuo to give the crude product **M4** (600 mg, 91%). LC-MS: 298.0 [M+H]⁺.

### Step 5: tert-Butyl (1-(3-methoxy-4-(methylsulfinyl)phenyl)ethyl)carbamate (M5)

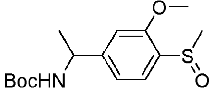

To a solution of tert-butyl (1-(3-methoxy-4-(methylthio)phenyl)ethyl)carbamate (300 mg, 1.02 mmol, 1.0 eq.) in DCM (9 mL) at 0 °C was added *m*-CPBA (225 mg, 1.11 mmol, 1.1 eq.). The reaction was slowly warmed up to room temperature and monitored by LC-MS. Upon completion, sat. NaHCO₃ (aq.) was added to adjust the aq. solution pH ~ 8-9. The reaction mixture was extracted with EtOAc and the organic layer was separated, washed with sat. brine, dried over Na₂SO₄. The solvent was removed in vacuo to give the crude product **M5** (310 mg). LC-MS: 313.8 [M+H]⁺.

### Example intermediate I-N-1:

### (R)-1-(1,3-Dimethyl-1H-indazol-5-yl)ethan-1-amine (N7)

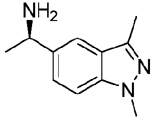

To a solution of (R)-N-((R)-1-(1,3-dimethyl-1H-indazol-5-yl)ethyl)-2-methylpropane-2-sulfinamide **N6** (1.30 g, 4.43 mmol, 1.0 eq.) in 1,4-dioxane (10 mL) was added 4 N HCl in 1,4-dioxane (10 mL, 40 mmol, 4 M, 9.0 eq.). The reaction mixture was stirred at room temperature and monitored by LC-MS. The solvent was removed in vacuo and the crude product **N7** (HCl salt) was used directly in the next step. LC-MS: 189.8 [M+H]⁺.

### Step 1: 1-(2-Amino-5-bromophenyl)ethan-1-one oxime (N1)

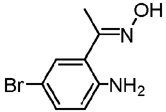

To a solution of 1-(2-amino-5-bromophenyl)ethan-1-one (10.0 g, 46.53 mmol, 1.0 eq.) in ethanol (60 mL) was added hydroxylamine hydrochloride (4.89 g, 70.43 mmol, 1.5 eq.).The reaction mixture was stirred at 80 °C for 16 h, and then cooled to rt. The solvent was removed and the resulting residue was purified by flash chromatography on silica gel (0-100% EtOAc/PE, 0-10% MeOH/DCM) to yield the product **N1** (8.10 g, 76.4%). LC-MS: 228.6 [M+H]⁺.

### Step 2: 5-Bromo-3-methyl-1H-indazole (N2)

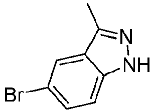

To a solution of 1-(2-amino-5-bromophenyl)ethan-1-one oxime **N1** (8.10 g, 34.53 mmol, 1.0 eq.) in DCM (60 mL) at 0 °C were added trifluoroacetic anhydride (7.51 g, 37.30 mmol, 1.08 eq.) and DIPEA (15.45 g, 119.7 mmol, 3.0 eq.) dropwise. The reaction mixture was stirred at 0 °C for 8 h, then water (60 mL) and DCM (60 mL) were added. The organic layer was separated and washed with sat. brine, dried over Na₂SO₄. The solvent was removed in vacuo and the residue was purified by flash chromatography on silica gel (0-60% PE/EtOAc) to yield the product **N2** (6.36 g, 87.8%). LC-MS: 210.6 [M+H]⁺.

### Step 3: 5-Bromo-1,3-dimethyl-1H-indazole (N3)

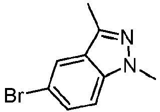

To a solution of 5-bromo-3-methyl-1H-indazole **N2** (6.36 g, 30.30 mmol, 1.0 eq.) in DMF (40 mL) was added NaH (1.45 g, 36.36 mmol, 60%wt in paraffin, 1.2 eq,) at 0 °C, then methyl iodide (6.45 g, 45.45 mmol, 1.5 eq.) was added dropwise. The reaction mixture was stirred at 0 °C for 2 h, then sat. NH₄Cl (aq.) (60 mL) and DCM (60 mL) were added. The organic layer was separated, washed with sat. brine, and dried over Na₂SO₄. The solvent was removed in vacuo and the crude residue was purified by flash chromatography on silica gel (0-60% EtOAc/PE) to yield the product **N3** (3.10 g, 45.6%). ¹H NMR (CDCl₃, 400 MHz) *δ* 7.78 (d, *J* = 2.0 Hz, 1H), 7.43 (dd, *J₁* = 8.9 Hz, *J₂* = 1.8 Hz, 1H), 7.20 (d, *J* = 8.9 Hz, 1H), 3.98 (s, 3H), 2.53 (s, 3H). LC-MS: 224.6 [M+H]⁺.

### Step 4: 5-(1-Ethoxyvinyl)-1,3-dimethyl-1H-indazole (N4)

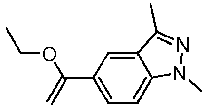

To a solution of 5-bromo-1,3-dimethyl-1H-indazole **N3** (3.10 g, 13.84 mmol, 1.0 eq.) and tributyl(1-ethoxyvinyl)tin (7.52 g, 20.76 mmol, 1.5 eq.) in 1,4-dioxane (50 mL) was added Pd(PPh₃)₄ (1.59 g, 1.38 mmol, 0.1 eq.). The mixture was recharged with N₂ balloon for three times and stirred at 100 °C overnight. Then the reaction mixture was cooled to room temperature and sat. potassium fluoride (aq.) (200 mL) was added. The resulting mixture was filtered through celite, and sat. NH₄Cl (aq.) and EtOAc were added. The organic layer was separated and washed with sat. brine, dried over Na₂SO₄. The solvent was removed in vacuo and the crude residue was purified by flash chromatography on silica gel (0-70% PE/EtOAc) to yield the product **N4** (2.16 g, 72.3%). LC-MS: 216.6 [M+H]⁺.

### Step 5: 1-(1,3-Dimethyl-1H-indazol-5-yl)ethan-1-one (N5)

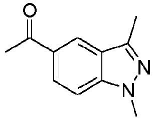

To a solution of 5-(1-ethoxyvinyl)-1,3-dimethyl-1H-indazole **N4** (2.16 g, 10.0 mmol, 1.0 eq.) in 1,4-dioxane (10 mL) was added 2 M HCl (aq.) (2 mL) at 0 °C. The mixture was stirred at 0 °C for 4 h. The solvent was removed in vacuo and the crude product **N5** (1.80 g, 95.8%) was used in the next step without further purification. LC-MS: 188.6 [M+H]⁺.

### Step 6: (R)-N-((R)-1-(1,3-Dimethyl-1H-indazol-5-yl)ethyl)-2-methylpropane-2-sulfinamide (N6)

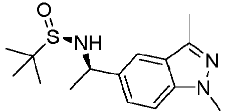

To a solution of 1-(1,3-dimethyl-1H-indazol-5-yl)ethan-1-one **N5** (1.80 g, 9.58 mmol, 1.0 eq.) and (R)-2-methylpropane-2-sulfinamide (1.73 g, 14.25 mmol, 1.5 eq.) in THF (60 mL) was added Ti(OEt)₄ (15.93 g, 23.75 mmol, 2.5 eq, containing 34%wt TiO₂). The mixture was recharged with N₂ balloon for three times and stirred at 70 °C overnight. The mixture was then cooled to -48 °C and NaBH₄ (3.64 g, 95.80 mmol, 10.0 eq.) was added. Upon completion, sat. brine (100 mL) was added and the mixture was filtered through celite. Sat. NH₄Cl (aq.) and EtOAc were added to the filtrate. The organic layer was separated and washed with sat. brine, dried over Na₂SO₄. The solvent was removed in vacuo and the crude residue was purified by flash chromatography on silica gel (0-60% EtOAc/PE) to yield the product **N6** (1.30 g, 46.2%). LC-MS: 293.8 [M+H]⁺.

### Example intermediate I-O-1:

### (R)-1-(1,3-Dimethyl-1H-indazol-6-yl)ethan-1-amine (08)

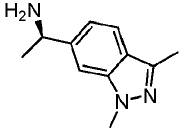

To a solution of (R)-N-((R)-1-(1,3-dimethyl-1H-indazol-6-yl)ethyl)-2-methylpropane-2-sulfinamide **O7** (1.35 g, 4.61 mmol, 1.0 eq.) in 1,4-dioxane (10 mL) was added 4 N HCl in 1,4-dioxane (10 mL, 40 mmol, 4 M, 8.7 eq.). The reaction mixture was stirred at room temperature and monitored by LC-MS. The solvent was removed in vacuo and the crude product **O8** (HCl salt) was used directly in the next step. LC-MS: 189.6 [M+H]⁺.

### Step 1: 1-(2-Amino-4-bromophenyl)ethan-1-one oxime (O1)

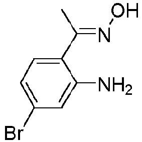

To a solution of 1-(2-amino-4-bromophenyl)ethan-1-one (10.0 g, 46.53 mmol, 1.0 eq.) in ethanol (60 mL) was added hydroxylamine hydrochloride (4.89 g, 70.43 mmol, 1.5 eq.). The reaction mixture was stirred at 80 °C for 16 h, and then cooled to rt. The solvent was removed in vacuo, and the resulting residue was purified by flash chromatography on silica gel (0-100% EtOAc/PE, 0-10% MeOH/DCM) to yield the product **O1** (9.10 g, 85.8%). LC-MS: 228.6 [M+H]⁺.

### Step 2: 6-Bromo-3-methyl-1H-indazole (O2)

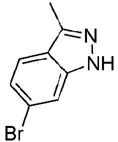

To a solution of 1-(2-amino-4-bromophenyl)ethan-1-one oxime **O1** (9.10 g, 39.91 mmol, 1.0 eq.) in DCM (60 mL) were added dropwise trifluoroacetic anhydride (8.61 g, 43.05 mmol, 1.08 eq.) and DIPEA (15.45 g, 119.70 mmol, 3.0 eq.) at 0 °C. The reaction mixture was stirred at 0 °C for 8 h. Water (60 mL) and DCM (60 mL) were added, and the organic layer was separated, washed with sat. brine, dried over Na₂SO₄. The solvent was removed in vacuo and the residue was purified by flash chromatography on silica gel (0-60% EtOAc/PE) to yield the product **O2** (7.80 g, 93.0%). ¹H NMR (DMSO-D₆, 400 MHz) *δ* 12.76 (s, 1H), 7.65-7.70 (m, 2H), 7.20 (dd, *J₁* = 8.6 Hz, *J₂* = 1.5 Hz, 1H), 2.48 (s, 3H). LC-MS: 210.6 [M+H]⁺.

### Step 3: 6-Bromo-1,3-dimethyl-1H-indazole (O3)

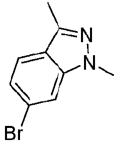

To a solution of 6-bromo-3-methyl-1H-indazole **O2** (7.80 g, 37.15 mmol, 1.0 eq.) in DMF (40 mL) at 0°C was added NaH (1.78 g, 44.58 mmol, 60%wt in paraffin, 1.2 eq.), followed by methyl iodide (7.91 g, 55.73 mmol, 1.5 eq.) dropwise. The reaction mixture was stirred for 2 h at 0°C. Upon completion, sat. NH₄Cl (aq.) (60 mL) and DCM (60 mL) were added. The organic layer was separated, washed with sat. brine, dried over Na₂SO₄. The solvent was removed in vacuo and the crude residue was purified by flash chromatography on silica gel (0-60% EtOAc/PE) to yield the product **O3** (3.10 g, 37.3%). ¹H NMR (DMSO-D₆, 400 MHz) *δ* 7.88 (d, *J* = 1.5 Hz, 1H), 7.65 (d, *J* = 8.5 Hz, 1H), 7.20 (dd, *J₁* = 8.6 Hz, *J₂* = 1.5 Hz, 1H), 3.93 (s, 3H), 2.45 (s, 3H). LC-MS: 224.6 [M+H]⁺.

### Step 4: 6-(1-Ethoxyvinyl)-1,3-dimethyl-1H-indazole (O4)

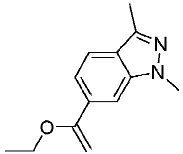

To a solution of 6-bromo-1,3-dimethyl-1H-indazole **O3** (3.10 g, 13.84 mmol, 1.0 eq.) and tributyl(1-ethoxyvinyl)tin (7.52 g, 20.76 mmol, 1.5 eq.) in 1,4-dioxane (50 mL) was added Pd(PPh₃)₄ (1.59 g, 1.38 mmol, 0.1 eq.). The mixture was recharged with N₂ balloon for three times and stirred at 100 °C overnight. The reaction mixture was cooled to room temperature and sat. potassium fluoride (aq.) (200 mL) was added. The resulting mixture was filtered through celite and sat. NH₄Cl (aq.) and EtOAc were added. The organic layer was separated, washed with sat. brine, and dried over Na₂SO₄. The solvent was removed in vacuo and the crude residue was purified by flash chromatography on silica gel (0-55% EtOAc/PE) to yield the product **O4** (2.03 g, 68.0%). LC-MS: 216.6 [M+H]⁺.

### Step 5: 1-(1,3-Dimethyl-1H-indazol-6-yl)ethan-1-one (05)

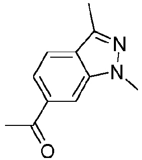

To a solution of 6-(1-ethoxyvinyl)-1,3-dimethyl-1H-indazole **O4** (2.03 g, 9.40 mmol, 1.0 eq.) in 1,4-dioxane/water (10 mL/1 mL) at 0 °C was added 2 M HCl (aq.) (4 mL). The mixture was stirred at 0 °C for 4 h. The solvent was removed in vacuo and the crude product **O5** (1.68 g, 95%) was used in the next step without further purification. LC-MS: 188.6 [M+H]⁺.

### Step 6: (R)-N-(1-(1,3-Dimethyl-1H-indazol-6-yl)ethylidene)-2-methylpropane-2-sulfinamide (O6)

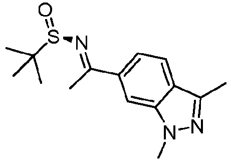

To a solution of 1-(1,3-dimethyl-1H-indazol-6-yl)ethan-1-one **O5** (1.68 g, 8.93 mmol, 1.0 eq.) and (R)-2-methylpropane-2-sulfinamide (1.62 g, 13.40 mmol, 1.5 eq.) in THF (60 mL) was added Ti(OEt)₄ (14.97 g, 22.33 mmol, containing 34%wt TiO₂, 2.5 eq.). The reaction mixture was recharged with N₂ balloon for three times and then refluxed overnight. The mixture containing **O6** was cooled to rt and used directly in the next step. LC-MS: 291.6 [M+H]⁺.

### Step 7: (R)-N-((R)-1-(1,3-Dimethyl-1H-indazol-6-yl)ethyl)-2-methylpropane-2-sulfinamide (O7)

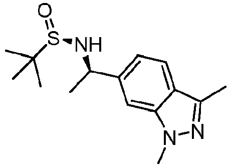

The above solution containing (R)-N-(1-(1,3-dimethyl-1H-indazol-6-yl)ethylidene)-2-methylpropane-2-sulfinamide **O6**_was cooled to -50 °C, then NaBH₄ (4.41 g, 116.09 mmol, 13.0 eq.) was added. The mixture was recharged with N₂ balloon for three times and stirred at -50 °C for 12 h. After complete consumption by LC-MS detection, sat. brine (100 mL) was added. The mixture was filtered through celite, then sat. NH₄Cl (aq.) and EtOAc were added. The organic layer was separated and washed with sat. brine, dried over Na₂SO₄. The solvent was removed in vacuo and the crude residue was purified by flash chromatography on silica gel (0-55% PE/EtOAc) to yield the product **O7** (1.35 g, 51.6%). LC-MS: 293.8 [M+H]⁺.

### Biological Assays

The exemplified compounds described herein were tested for activity and were found to have an IC₅₀ value less than 10 µM in the following assay:

### 1. Measurement of GRK5 + GRK2 kinase activity

This protocol describes how the Lance Kinase Activity Assay was performed to determine IC₅₀ values of compounds of general formula (I) against GRK5 and GRK2.

The principle behind this enzymatic assay is based upon the phosphorylation of the Ulight-peptide substrate. It is detected by using a specific EU-labeled anti-phospho peptide antibody. The binding of the Eu labeled anti-phospho peptide antibody to the phosphorylated ULight labeled peptide gives rise to a FRET-signal.

Binding of an inhibitor to the kinase prevents phosphorylation of the Ulight-substrate, resulting in a loss of FRET. In tables 2 and 3 the relevant information for these two LANCE assays is summarized.

**Table 2: Reagents, stock concentrations and final assay concentrations for GRK5.**

| **Reagents** | **Stock concentration** | **Working concentration** | **Final assay concentration** | **Supplier** |
|---|---|---|---|---|
| ULight-Topo IIα substrate | 5 µM | 166,67 nM | 50 nM | Perkin Elmer |
| Eu-Anti-Phospho-DNA-Topoisomerase 2α Antibody (AB) | 625 nM | 4nM | 2 nM | Perkin Elmer |
| GRK5 | 2,731µM | 33,33 nM | 10 nM | Thermo Scientific |
| ATP | 100mM | 32,5 µM | 6,5 µM | Sigma |

**Table 3: Reagents, stock concentrations and final assay concentrations for GRK2.**

| **Reagents** | **Stock concentration** | **Working concentration** | **Final assay concentration** | **Supplier** |
|---|---|---|---|---|
| ULight-Topo IIα substrate | 5 µM | 166,67 nM | 50 nM | Perkin Elmer |
| Eu-Anti-Phospho-DNA-Topoisomerase 2α Antibody (AB) | 625 nM | 4nM | 2 nM | Perkin Elmer |
| GRK2 | 5,954 µM | 66,67 nM | 20 nM | Thermo Scientific |
| ATP | 100mM | 75 µM | 15 µM | Sigma |

For every sample, 2 µl of assay buffer (50mM HEPES pH 7,5, 10mM MgCL₂, 1mM EGTA 0,01% Tween 20, 1% DMSO, 2mM DTT) were transferred into a suitable assay plate (e.g. Corning #3673). Compounds were added in a concentration range from 10µM to 0.0025µM using an acoustic dispenser (Echo520 from Labcycte, San Jose, USA) equipped with Echo Dose Response software. After that 6µl of kinase-substrate mix was added. Reaction was started by addition of 2µl ATP working solution and mixed using a Bioshake 5000 microplate shaker (Q Instruments, Jena, Germany).

After 1 h incubation at room temperature the reaction was stopped with 10µl detection mix containing the Eu-labeled phosphospecific antibody and 10mM EDTA. After a second incubation period of 1 h at room temperature the FRET signal was measured at 340 nm excitation, 665 nm and 615 nm emission (for the ULight-substrate and Eu-AB, respectively) with an Envision microplate reader (Perkin Elmer, Waltham, MA, USA) with 50 µs delay and 300 µs integration time. IC₅₀ values were determined from the sigmoidal dose response curves with the software Quattro Workflow (Quattro GmbH, Munich, Germany).

**Table 4** shows activity data in the biochemical Lance assay. Inhibition is indicated as IC₅₀ [nM] ("-" = not measured). Compounds having an activity designated as "A" provided an IC₅₀ ≤ 50 nM; compounds having an activity designated as "B" provided an 50 nM < IC₅₀ ≤ 100 nM; compounds having an activity designated as "C" provided an 100 nM < IC₅₀ ≤ 500 nM; compounds having an activity designated as "D" provided an 500nM < IC₅₀ ≤ 1000 nM; compounds having an activity designated as "E" provided an 1000nM < IC₅₀ ≤ 10000nM; and compounds having an activity designated as "F" provided an IC₅₀ > 10000nM.

**Table 4:**

| **Example** | **GRK5 LANCE Assay (IC-50) [nM]** | **GRK2 LANCE Assay (IC-50) [nM]** |
|---|---|---|
| **1** | **C** | **C** |
| **6** | **C** | **E** |
| **7** | **C** | **E** |
| **8** | **E** | **D** |
| **10** | **E** | **E** |
| **11** | **A** | **E** |
| **12** | **C** | **D** |
| **13** | **B** | **C** |
| **14** | **D** | **E** |
| **15** | **B** | **E** |
| **16** | **B** | **B** |
| **17** | **C** | **C** |
| **18** | **C** | **D** |
| **19** | **C** | **E** |
| **20** | **C** | **C** |
| **21** | **E** | **F** |
| **22** | **B** | **A** |
| **23** | **E** | **F** |
| **24** | **E** | **F** |
| **25** | **E** | **F** |
| **26** | **E** | **E** |
| **27** | **E** | **E** |
| **28** | **D** | **C** |
| **29** | **D** | **C** |
| **30** | **E** | **F** |
| **31** | **D** | **E** |
| **32** | **B** | **B** |
| **33** | **A** | **A** |
| **34** | **C** | **A** |
| **35** | **C** | **E** |
| **36** | **C** | **E** |
| **37** | **E** | **F** |
| **38** | **E** | **E** |
| **39** | **C** | **E** |
| **40** | **C** | **C** |
| **41** | **C** | **E** |
| **42** | **D** | **E** |
| **43** | **C** | **E** |
| **44** | **E** | **E** |
| **45** | **D** | **E** |
| **46** | **E** | **F** |
| **47** | **E** | **F** |
| **48** | **A** | **B** |
| **49** | **E** | **F** |
| **50** | **C** | **C** |
| **51** | **A** | **D** |
| **52** | **C** | **C** |
| **53** | **A** | **E** |
| **54** | **A** | **A** |
| **55** | **A** | **F** |
| **56** | **B** | **F** |
| **57** | **C** | **E** |
| **58** | **C** | **E** |
| **59** | **E** | **E** |
| **60** | **C** | **C** |
| **61** | **D** | **E** |
| **62** | **C** | **E** |
| **63** | **C** | **C** |
| **64** | **B** | **C** |
| **65** | **E** | **E** |
| **66** | **C** | **C** |
| **67** | **D** | **D** |
| **68** | **C** | **C** |
| **69** | **A** | **B** |
| **70** | **C** | **D** |
| **71** | **E** | **F** |
| **72** | **E** | **E** |
| **73** | **E** | **E** |
| **74** | **E** | **F** |
| **75** | **E** | **F** |
| **76** | **C** | **D** |
| **77** | **C** | **E** |
| **78** | **C** | **D** |
| **79** | **C** | **E** |
| **80** | **B** | **F** |
| **81** | **A** | **E** |
| **82** | **C** | **F** |
| **83** | **C** | **F** |
| **84** | **A** | **E** |
| **85** | **A** | **F** |
| **86** | **C** | **F** |
| **87** | **A** | **D** |
| **88** | **A** | **E** |
| **89** | **A** | **E** |
| **90** | **C** | **F** |
| **91** | **A** | **C** |
| **92** | **A** | **E** |
| **93** | **A** | **C** |
| **94** | **A** | **B** |
| **95** | **B** | **E** |
| **96** | **A** | **C** |
| **97** | **D** | **D** |
| **98** | **D** | **D** |
| **99** | **C** | **F** |
| **100** | **D** | **E** |
| **101** | **B** | **F** |
| **102** | **A** | **F** |
| **103** | **A** | **E** |
| **104** | **A** | **E** |
| **105** | **A** | **E** |
| **106** | **A** | **E** |
| **107** | **A** | **D** |
| **108** | **A** | **E** |
| **109** | **A** | **F** |
| **110** | **A** | **E** |
| **111** | **A** | **E** |
| **112** | **A** | **D** |
| **113** | **A** | **D** |
| **114** | **A** | **E** |
| **115** | **C** | **F** |
| **116** | **B** | **F** |
| **117** | **A** | **E** |
| **118** | **A** | **E** |
| **119** | **E** | **F** |
| **120** | **B** | **E** |
| **121** | **C** | **F** |
| **122** | **A** | **F** |
| **123** | **C** | **F** |
| **124** | **C** | **F** |
| **125** | **A** | **F** |
| **126** | **B** | **F** |
| **127** | **A** | **F** |
| **128** | **C** | **F** |
| **129** | **E** | **F** |
| **130** | **A** | **F** |
| **131** | **B** | **E** |
| **132** | **A** | **D** |
| **133** | **E** | **E** |
| **134** | **A** | **E** |
| **135** | **A** | **E** |
| **136** | **C** | **F** |
| **137** | **A** | **F** |
| **138** | **A** | **D** |
| **139** | **B** | **F** |
| **140** | **A** | **F** |
| **141** | **A** | **E** |
| **142** | **A** | **E** |
| **143** | **A** | **E** |
| **144** | **C** | **F** |
| **145** | **A** | **F** |
| **146** | **B** | **F** |
| **147** | **A** | **E** |
| **148** | **A** | **E** |
| **149** | **A** | **E** |
| **150** | **D** | **F** |
| **151** | **A** | **F** |
| **152** | **A** | **E** |
| **153** | **A** | **D** |
| **154** | **A** | **D** |
| **155** | **A** | **E** |
| **156** | **C** | **F** |
| **157** | **A** | **E** |
| **158** | **A** | **F** |
| **159** | **C** | **F** |
| **160** | **A** | **E** |
| **161** | **A** | **F** |
| **162** | **A** | **E** |
| **163** | **A** | **E** |
| **164** | **A** | **E** |
| **165** | **B** | **E** |
| **166** | **A** | **E** |
| **167** | **A** | **E** |
| **168** | **A** | **E** |
| **169** | **A** | **E** |
| **170** | **A** | **E** |
| **171** | **A** | **E** |
| **172** | **A** | **E** |
| **173** | **A** | **E** |
| **174** | **A** | **E** |
| **175** | **A** | **E** |
| **176** | **A** | **E** |
| **177** | **A** | **E** |
| **178** | **C** | **F** |
| **179** | **C** | **E** |
| **180** | **B** | **C** |
| **181** | **A** | **C** |
| **182** | **A** | **C** |
| **183** | **A** | **F** |
| **184** | **B** | **F** |
| **185** | **A** | **E** |
| **186** | **A** | **E** |
| **187** | **A** | **E** |
| **188** | **A** | **D** |
| **189** | **A** | **E** |
| **190** | **A** | **E** |
| **191** | **A** | **E** |
| **192** | **A** | **E** |
| **193** | **A** | **E** |
| **194** | **A** | **E** |
| **195** | **A** | **F** |
| **196** | **A** | **C** |
| **197** | **A** | **D** |
| **198** | **A** | **E** |
| **199** | **A** | **F** |
| **200** | **A** | **E** |
| **201** | **C** | **F** |
| **202** | **A** | **E** |
| **203** | **A** | **E** |
| **204** | **A** | **E** |
| **205** | **A** | **E** |
| **206** | **E** | **F** |
| **207** | **D** | **F** |
| **208** | **A** | **E** |
| **209** | **B** | **F** |
| **210** | **A** | **E** |
| **211** | **A** | **D** |
| **212** | **A** | **E** |
| **213** | **A** | **E** |
| **214** | **A** | **E** |
| **215** | **A** | **E** |
| **216** | **A** | **E** |
| **217** | **A** | **E** |
| **218** | **A** | **F** |
| **219** | **A** | **E** |
| **220** | **A** | **E** |
| **221** | **A** | **F** |
| **222** | **E** | **F** |
| **223** | **C** | **F** |
| **224** | **C** | **F** |
| **225** | **B** | **E** |
| **226** | **B** | **E** |
| **227** | **C** | **E** |
| **228** | **B** | **E** |
| **229** | **E** | **F** |
| **230** | **A** | **E** |
| **231** | **C** | **F** |
| **232** | **B** | **E** |
| **233** | **B** | **E** |
| **234** | **C** | **E** |
| **235** | **B** | **F** |
| **236** | **C** | **F** |
| **237** | **C** | **F** |
| **238** | **C** | **F** |
| **239** | **D** | **F** |
| **240** | **B** | **E** |
| **241** | **B** | **E** |
| **242** | **C** | **F** |
| **243** | **C** | **F** |
| **244** | **D** | **E** |
| **245** | **D** | **F** |
| **246** | **A** | **E** |
| **247** | **A** | **F** |
| **248** | **A** | **F** |
| **249** | **A** | **E** |
| **250** | **A** | **E** |
| **251** | **A** | **D** |
| **252** | **A** | **D** |
| **253** | **B** | **F** |
| **254** | **A** | **F** |
| **255** | **C** | **F** |
| **256** | **B** | **E** |
| **257** | **A** | **C** |
| **258** | **A** | **C** |
| **259** | **A** | **E** |
| **260** | **A** | **B** |
| **261** | **A** | **F** |
| **262** | **A** | **F** |
| **263** | **A** | **F** |
| **264** | **A** | **F** |
| **265** | **B** | **F** |

### 2. In vitro cardiomyocyte hypertrophy assay

5-6 days old Wistar rats were sacrificed by decapitation and neonatal rat ventricular cardiac myocytes (NRVCMs) were prepared using the Neonatal heart dissociation kit (Miltenyi Biotec) according to the manufacturer's instructions. The cardiac single cell suspension was resuspended in DMEM low glucose medium supplemented with 2% fetal bovine serum (FBS) and 50 µg/ml penicillin/streptomycin. 1500 cells/well were seeded on 0.1% gelatin-coated 384-well plates and incubated in medium at 37 °C and 5% CO2 for 24 h prior to the addition of test compounds to the cells at titrated concentrations. Cellular hypertrophy was then stimulated by adding phenylephrine and leukemia inhibitory factor at final concentrations of 20 µM and 50 ng/ml, respectively, and the cells were incubated for another 96 h. For immunocytochemistry, cells were fixed with 4% formaldehyde on day 5 of compound treatment and cardiomyocytes were stained with antibodies against a-actinin while cell nuclei were stained with DAPI. Image acquisition and phenotypic analysis was performed using an Image-Xpress Micro XL automated microscope (Molecular Devices).

**Table 5** shows activity data measured in the *in vitro* cardiomyocyte hypertrophy assay. Inhibition of cellular hypertrophy is indicated as IC₅₀ [nM]. Compounds having an activity designated as "A" provided an IC₅₀ ≤ 500 nM; compounds having an activity designated as "B" provided an 500 nM < IC₅₀ ≤ 1000 nM; compounds having an activity designated as "C" provided an IC₅₀ > 1000nM.

| **Example** | **Hypertrophy Assay (IC-50) [nM]** |
|---|---|
| **205** | **B** |
| **94** | **A** |
| **92** | **C** |
| **169** | **B** |
| **117** | **B** |
| **89** | **C** |
| **213** | **C** |
| **104** | **C** |
| **113** | **A** |
| **252** | **A** |
| **254** | **C** |
| **219** | **C** |

## Claims

1. Compound of the general formula (I) 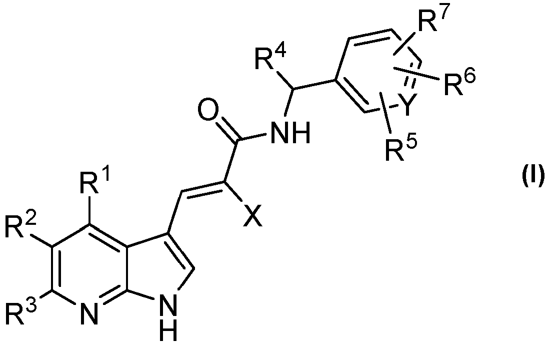 wherein
**X** represents -H or -CN,
**Y** represents CH or N,
**R¹** - **R²** represent independently of each other -H, -Ph, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, cyclo-C₃H₅, cyclo-C₄H₇, cyclo-C₅H₉, cyclo-C₆H₁₁, -F, -Cl, -Br, -CN, -OH, -NH-Ph, **-R⁹**, **-O-R¹⁰**, 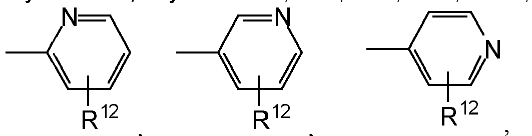 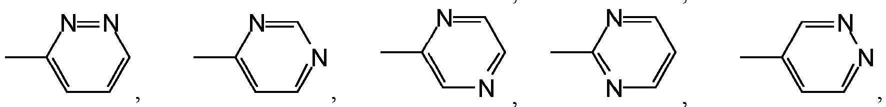 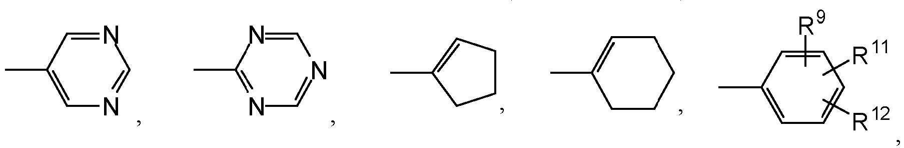 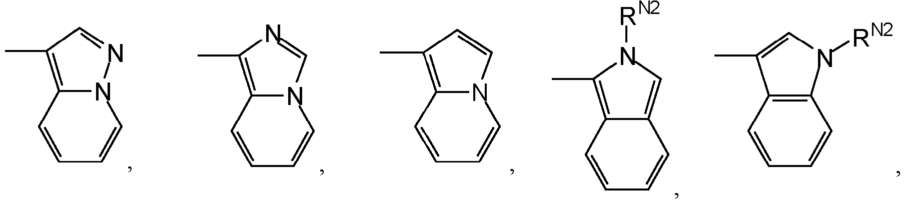 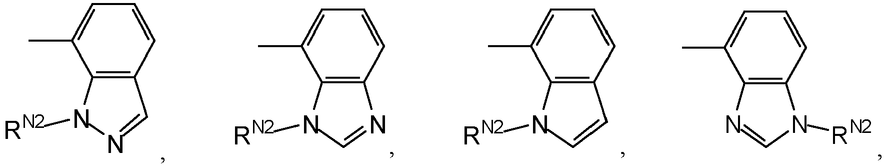 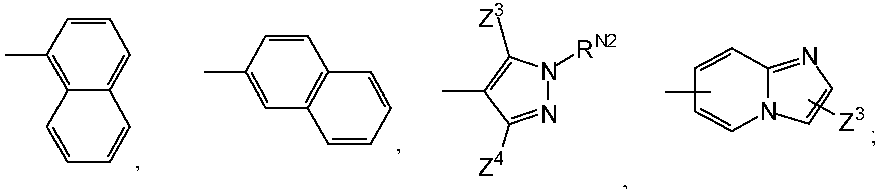
**R³** represents -H, -F, -Cl, or -Br;
**R⁴** represents -H, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH₂-OH, -C₂H₄-OH, -C₃H₆-OH, -CN, -CH₂-NH₂, -CH₂-NHCH₃, -CH₂-NHC₂H₅, -CH₂-NHC₃H₇, -CH₂-N(CH₃)₂, -CH₂-N(C₂H₅)₂, -CH₂-N(C₃H₇)₂, -C₂H₄-NH₂, -C₂H₄-NHCH₃, -C₂H₄-NHC₂H₅, -C₂H₄-NHC₃H₇, -C₂H₄-N(CH₃)₂, -C₂H₄-N(C₂H₅)₂, -C₂H₄-N(C₃H₇)₂, -C₃H₆-NH₂, -C₃H₆-NHCH₃, -C₃H₆-NHC₂H₅, -C₃H₆-NHC₃H₇, -C₃H₆-N(CH₃)₂, -C₃H₆-N(C₂H₅)₂, -C₃H₆-N(C₃H₇)₂;
**R⁵ - R⁷** represent independently of each other **-R⁸,** -CH₂-**R⁸**, -SO₂**-R⁸,** 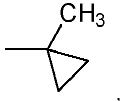 cyclo-C₃H₅, cyclo-C₄H₇, cyclo-C₅H₉, cyclo-C₆H₁₁, cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃He-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -CH₂-SO₂NH₂, -CH₂-SO₂NHCH₃, -CH₂-SO₂NHC₂H₅, -CH₂-SO₂NHC₃H₇, -CH₂-SO₂NH-cyclo-C₃H₅, -CH₂-SO₂NHCH(CH₃)₂, -CH₂-SO₂NHC(CH₃)₃, -CH₂-SO₂N(CH₃)₂, -CH₂-SO₂N(C₂H₅)₂, -CH₂-SO₂N(C₃H₇)₂, -CH₂-SO₂N(cyclo-C₃H₅)₂, -CH₂-SO₂N[CH(CH₃)₂]₂, -CH₂-SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂Hs, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H_{5,} -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂Hs, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C=CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, or -C≡C-C≡C-C₂H₅,
or
**R⁵** and **R⁶,** or **R⁶ and R**⁷ form together 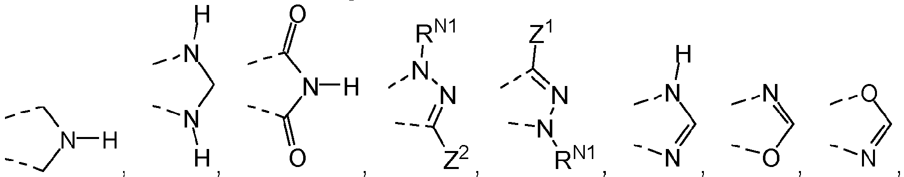 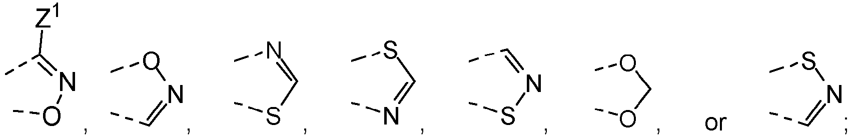
**R⁸** represents 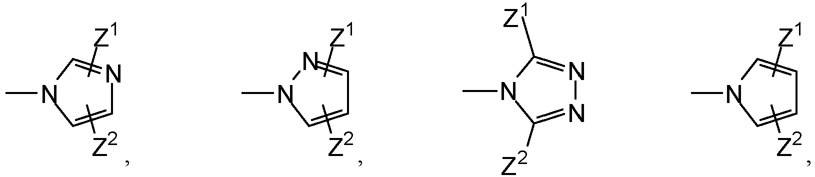 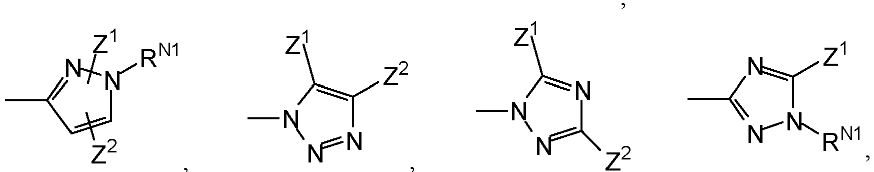 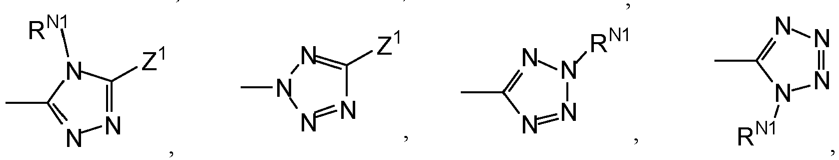 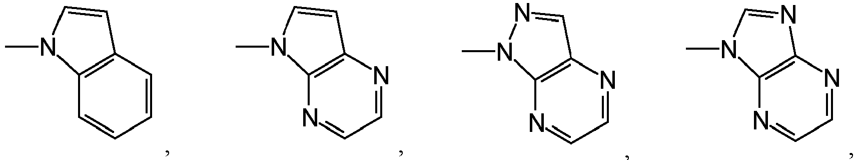 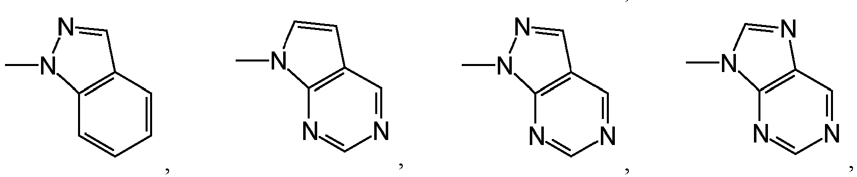 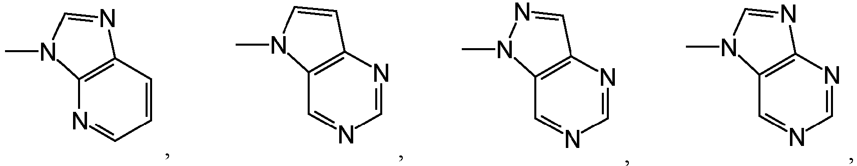 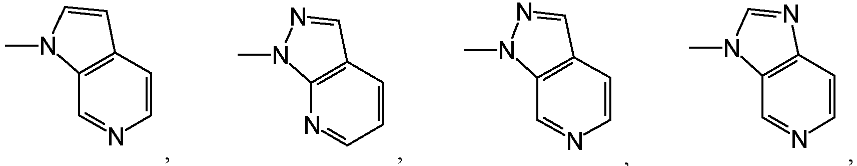 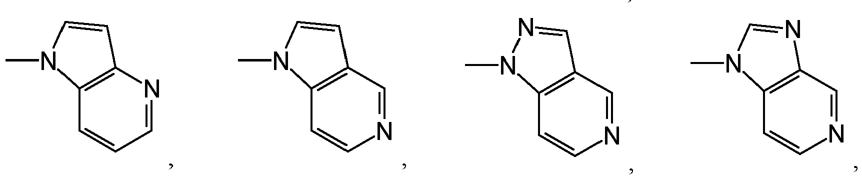 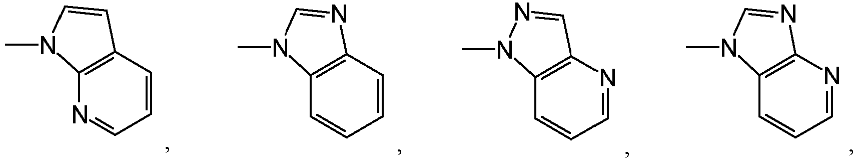 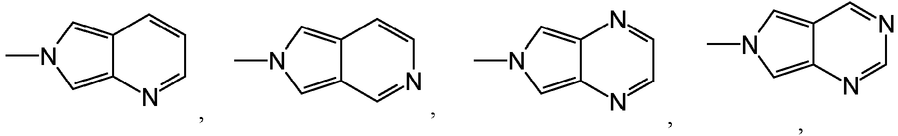 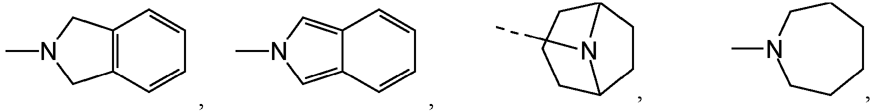 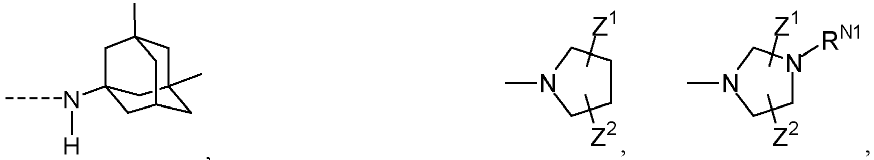 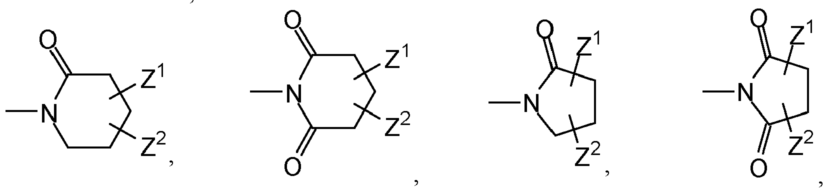 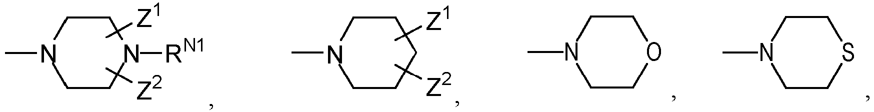 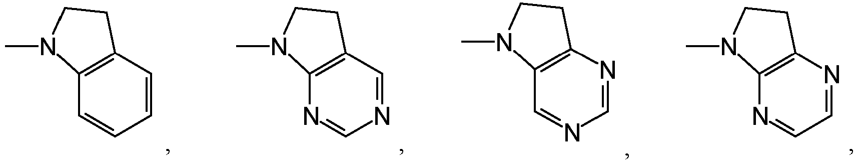 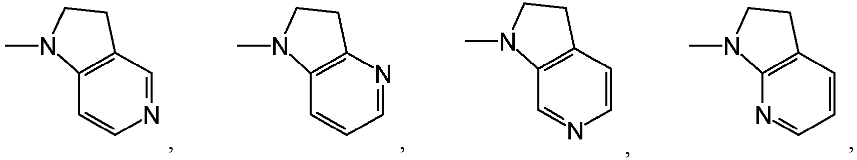 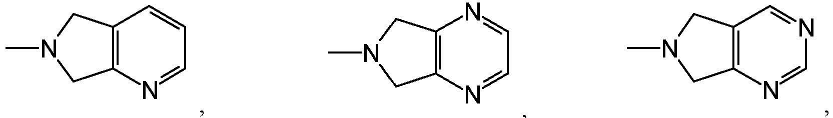 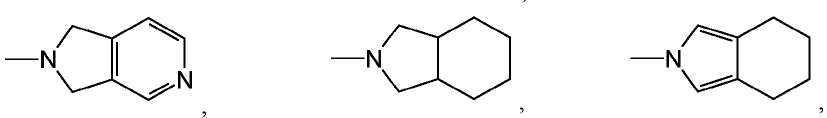 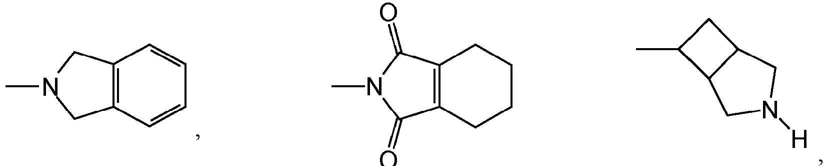 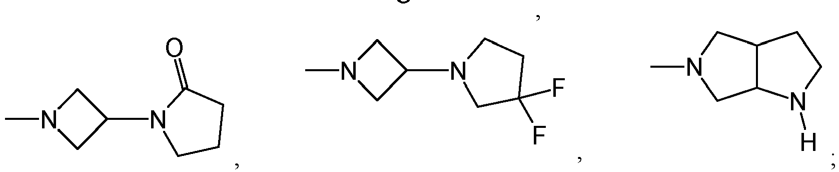
**R⁹** represents -H, 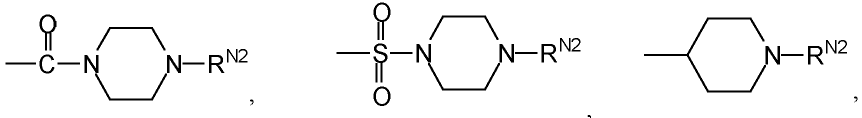 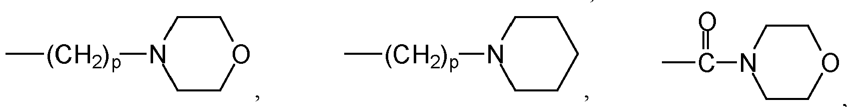 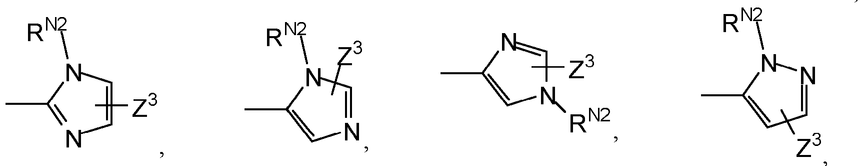 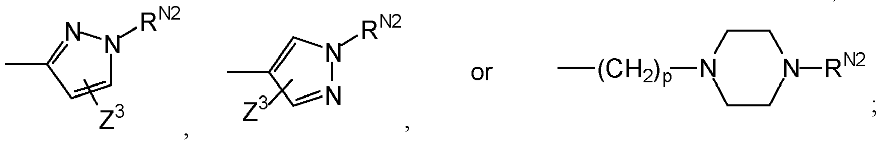
R¹⁰ represents -CH₃, -CF₃, -C₂H₅, -C₃H₇, -CH(CH₃)₂, -C₄H₉, -CH₂-CH(CH₃)₂, -CH(CH₃)-C₂Hs, -C(CH₃)₃, -C₅H₁₁, -(CH₂)ₙ-NH₂, -(CH₂)ₙ-NHCH₃, -(CH₂)ₙ-NHC₂H₅, -(CH₂)ₙ-NHC₃H₇, -(CH₂)ₙ-N(CH₃)₂, -(CH₂)ₙ-N(C₂H₅)₂, -(CH₂)ₙ-N(C₃H₇)₂, 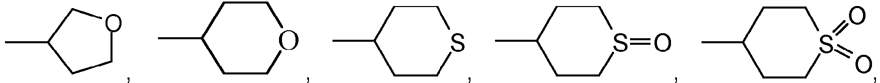 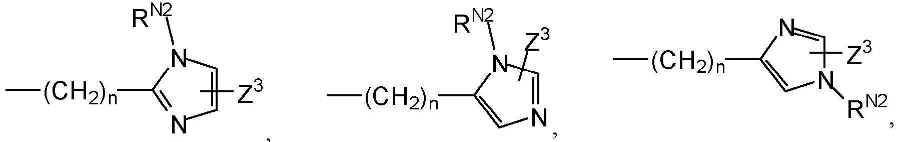 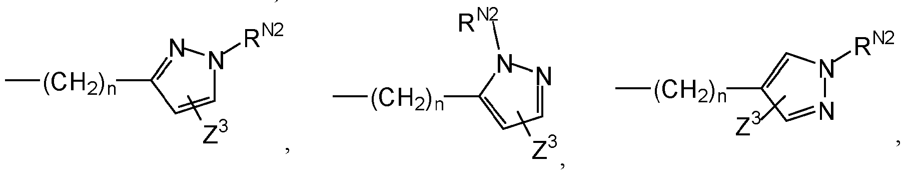
**R¹¹** and **R¹²** represent independently of each other -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)m-NHCH₃, -O-(CH₂)ₘ-NHC₂H₅, -O-(CH₂)m-NHC₃H₇^{,} -O-(CH₂)ₘ-N(CH₃)₂, -O-(CH₂)m-_{N}(C₂H₅)², -O-(CH₂)m-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)m-NHCOC₃H₇, -(CH₂)ₘ-NHCO-CH(CH₃)2, -(CH₂)m-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, or -SO(NH)CH₃,
**R^{N1}** and **R^{N2}** represent independently of each other -H, -CH₃, -C₂H₅, -C₃H₇, -CH(CH₃)₂, cyclo-C₃H₅, cyclo-C₄H₇, cyclo-C₅H₉, cyclo-C₆H₁₁, -C₄H₉, -C(CH₃)₃, -CH₂-CH(CH₃)₂, -CH(CH₃)-C₂Hs, -C₅H₁₁, -Ph, -CH₂-Ph, -CH=CH₂, -CH₂-CH=CH₂, -CH=CH-CH₃, -C=CH, -CH₂-C≡CH, -C≡C-CH₃, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 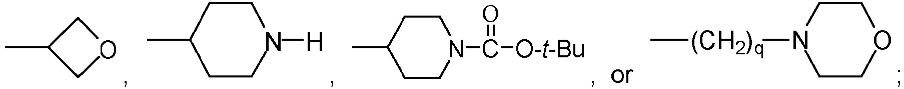
**Z¹** - **Z⁴** represent independently of each other -H, -CH₃, -C₂H₅, -C₃H₇, -CH(CH₃)₂, -Ph, -COCH₃, -COC₂H₅, -COOH, -COOCH₃, -COOC₂H₅, -NH₂, -NHCH₃, -NHC₂H₅, -N(CH₃)₂, -N(C₂H₅)₂, -CH₂-NH₂, -SO₃H, -F, -Cl, -Br, -1, -CN, -CF₃, -OCF₃, -OH, -OCH₃, or -OC₂H₅;
m is an integer selected from 0, 1, 2, 3, or 4;
n is an integer selected from 1, 2, 3, or 4;
p is an integer selected from 0, 1, 2, 3, or 4;
q is an integer selected from 1, 2, 3, or 4;
with the proviso that when **X** is -H at least one of **R¹** - **R³ is** not -H; and when **R⁴** is -H, at least one of **R¹** = **R³** and **R⁵ - R⁷** is not -H; and the following compound is excluded from the scope of the present application: 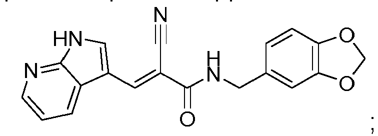
or an enantiomer, a stereoisomeric form, a mixture of enantiomers, a diastereomer, a mixture of diastereomers, a hydrate, a solvate, an acid salt form, a tautomer, or a racemate of the above mentioned compound, or a pharmaceutically acceptable salt thereof.

2. The compound according to claim 1, wherein
**R¹** and **R²** represent independently of each other -H, -Ph, -CH₃, -CF₃, -CI, -Br, -CN, -OH, -NH-Ph, -R⁹, -O-R¹⁰, 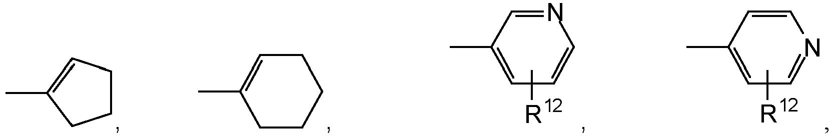 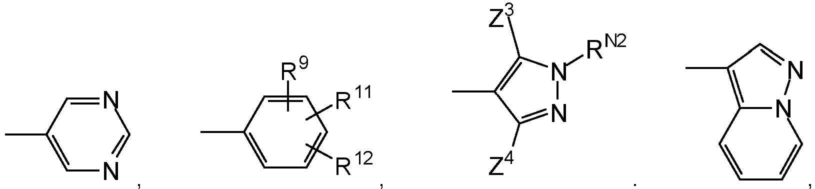 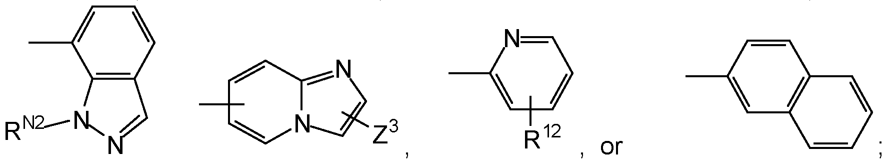
wherein **R⁹,** R¹⁰, **R¹¹, R¹², R^{N2},** Z³, **Z⁴** have **the** meanings as defined in claim 1.

3. The compound according to claim 1 or 2, wherein
R¹ represents -H, -CH₃, -Cl, 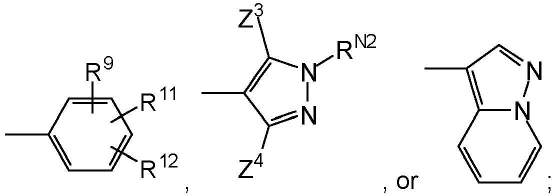
**R²** represents -H, -Ph, -CH₃, -CF₃, -Cl, -Br, -CN, -OH, -NH-Ph, **-R⁹,** -O-R**¹⁰,** 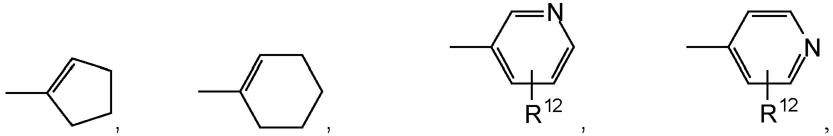 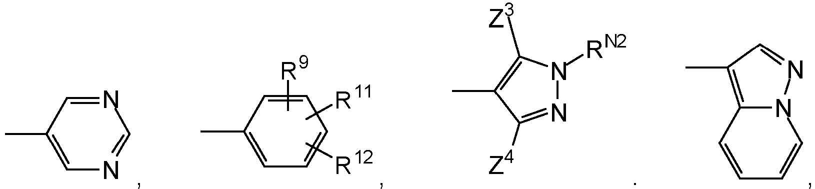 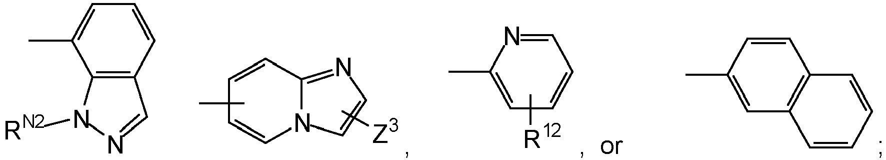
**R⁹** represents -H, 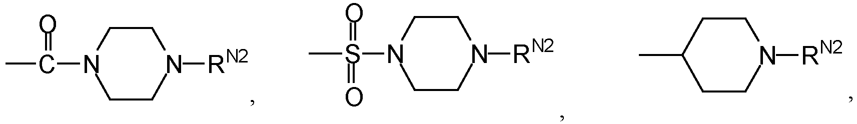 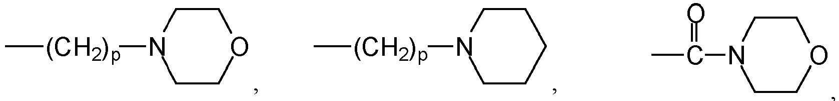 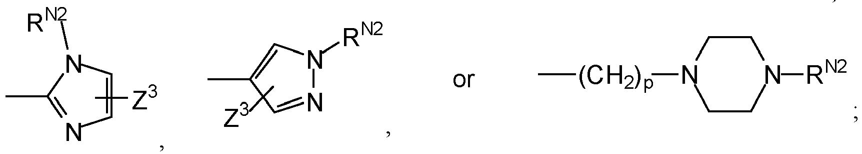
**R¹⁰** represents -CH(CH₃)₂, -CH₂-CH(CH₃)₂, -CH(CH₃)-C₂Hs, -(CH₂)ₙ-N(CH₃)₂, 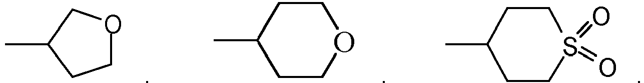 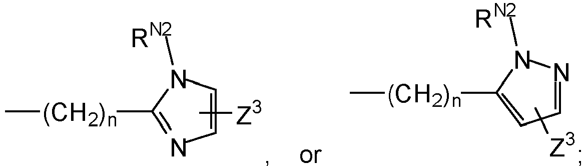
wherein **n, p, R¹¹, R¹², R^{N2}, Z³, Z⁴** have the meanings as defined in claim 1.

4. The compound according to any one of claims 1 - 3, wherein the compound has any one of the formulae **(Ia), (Ib),** and **(II-1)** - **(II-6)** 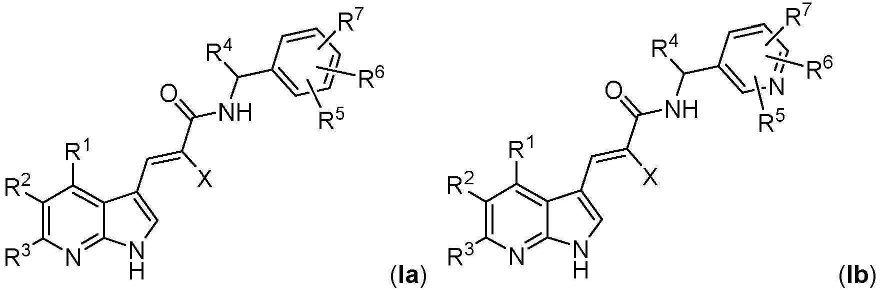 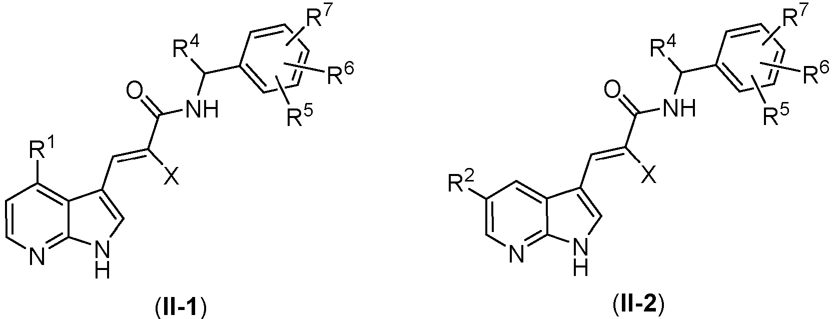 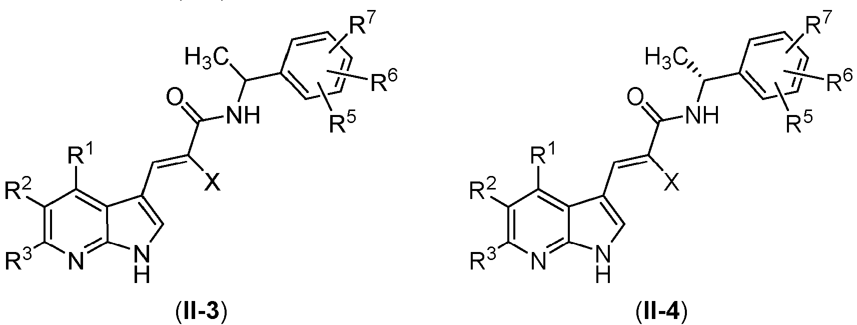 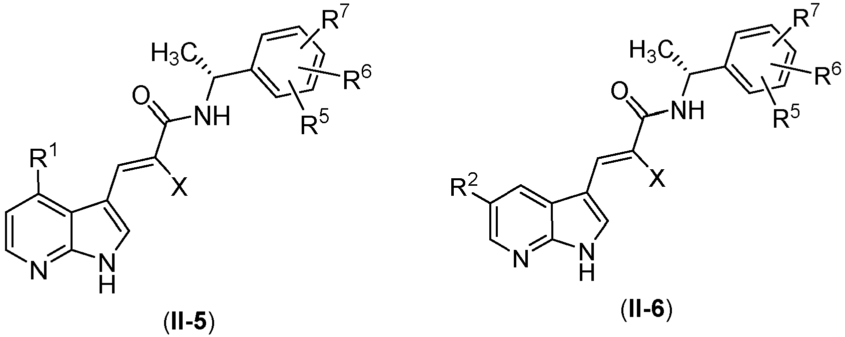 wherein **R¹, R², R³, R⁴, R⁵, R⁶, R⁷** and **X** have the meanings as defined in claim 1.

5. The compound according to any one of claims 1 - 4, wherein the compound has any one of the formulae **(III-1) - (III-6)** 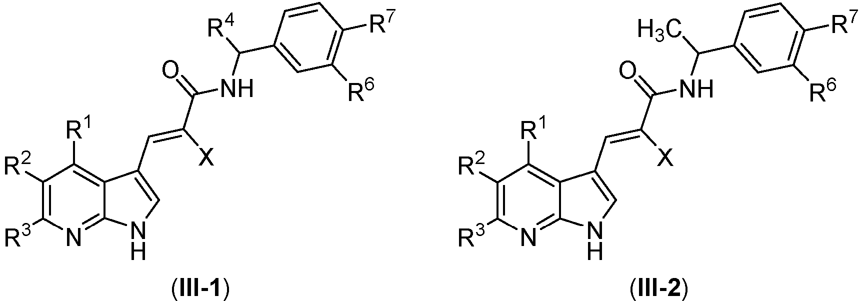 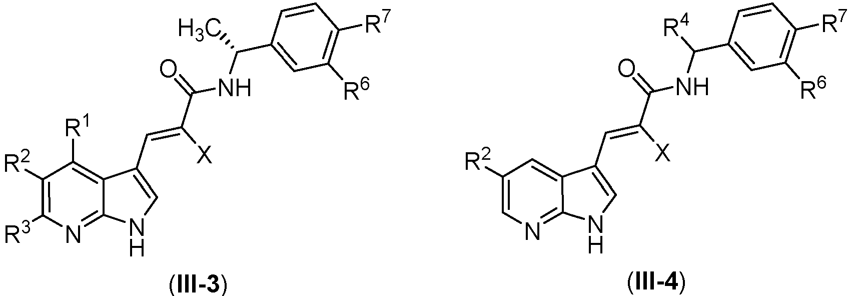 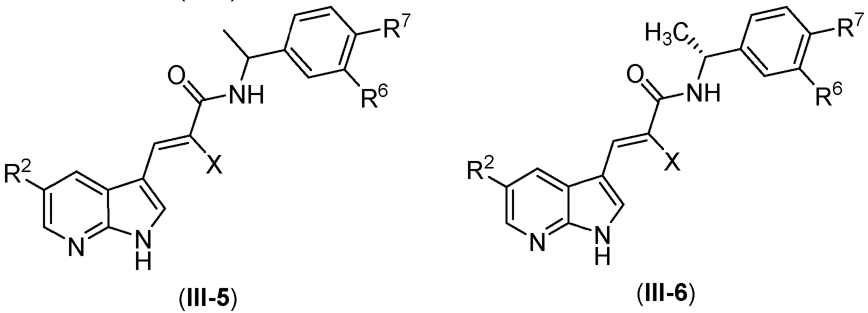 wherein **R¹, R², R³, R⁴, R⁶, R⁷** and X have the meanings as defined in claim 1.

6. The compound according to any one of claims 1 - 5, wherein the compound has any one of the formulae **(IV-1) - (IV-6)** or **(V-1) - (V-6)** 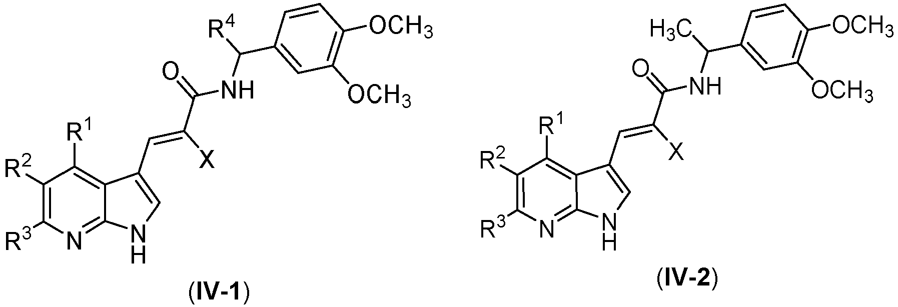 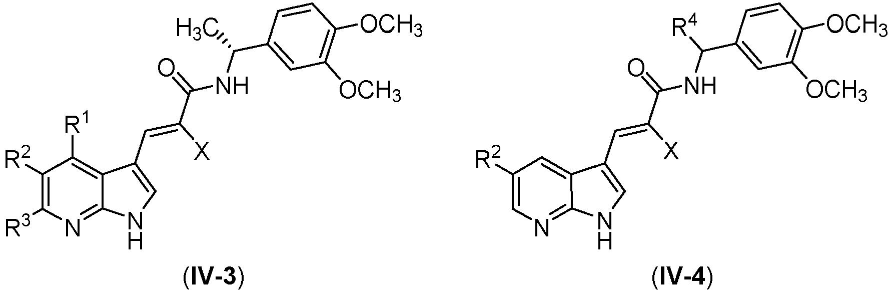 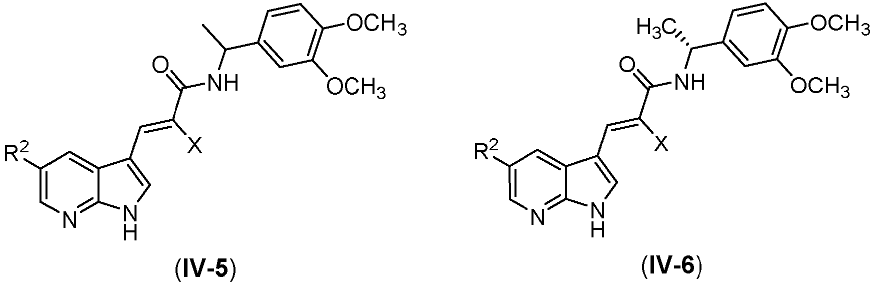 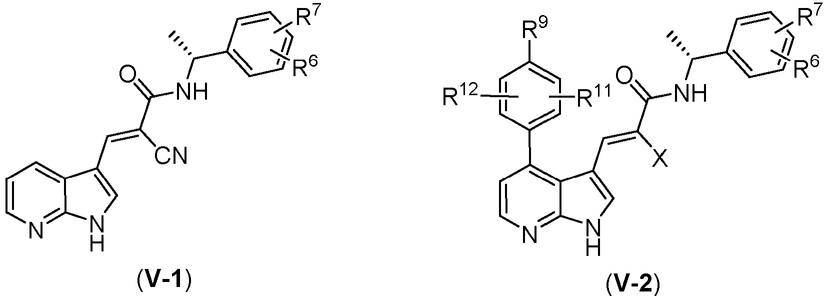 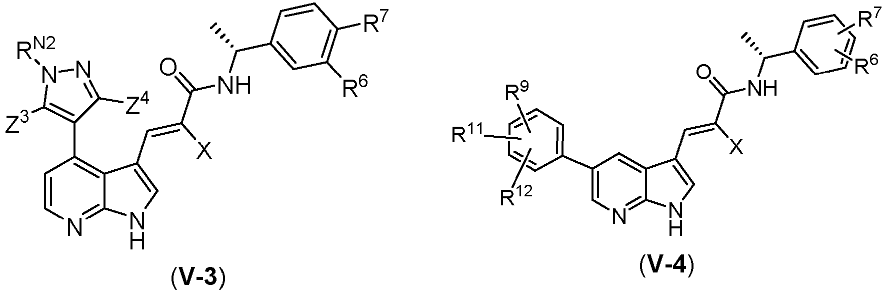 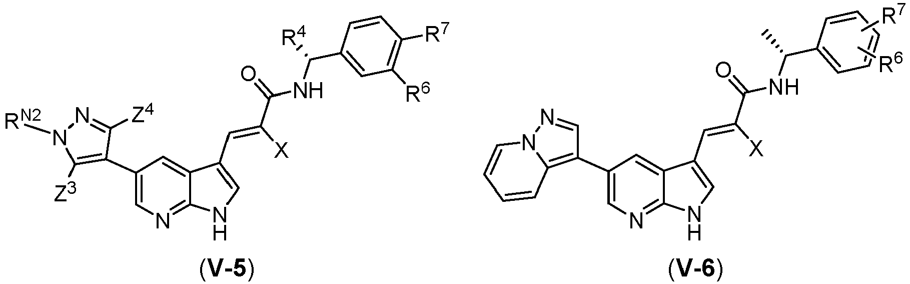 wherein **R¹, R², R³, R⁴, R⁶, R⁷, R⁹, R¹¹, R¹², R^{N2}, X, Z³,** and **Z⁴** have the meanings as defined in claim 1.

7. A compound selected from the group consisting of:
| **Compound** | **Nomenclature** |
|---|---|
| **1** | (*R,E*)-2-cyano-*N*-(1-(3-fluorophenyl)ethyl)-3-(1*H*-pyrrolo[2,3-b]pyridin-3-yl)acrylamide |
| **6** | (E)-N-benzyl-3-(5-chloro-1H-pyrrolo[2,3-b]pyridin-3-yl)acrylamide |
| **7** | (E)-3-(5-chloro-1H-pyrrolo[2,3-b]pyridin-3-yl)-N-(3-fluorobenzyl)acrylamide |
| **8** | (E)-3-(4-chloro-1H-pyrrolo[2,3-b]pyridin-3-yl)-N-(3-fluorobenzyl)acrylamide |
| **10** | (E)-N-(1-(4-fluorophenyl)ethyl)-3-(1H-pyrrolo[2,3-b]pyridin-3-yl)acrylamide |
| **11** | (R,E)-3-(5-bromo-1H-pyrrolo[2,3-b]pyridin-3-yl)-N-(1-(3,4-dimethoxyphenyl)ethyl)acrylamide |
| **12** | (R,E)-N-(1-(2-ch orophenyl)ethyl)-2-cyano-3-(1H-pyrrolo[2,3-b]pyridin-3-yl)acrylamide |
| **13** | (R,E)-N-(1-(3-chlorophenyl)ethyl)-2-cyano-3-(1H-pyrrolo[2,3-b]pyridin-3-yl)acrylamide |
| **14** | (S,E)-2-cyano-3-(1H-pyrrolo[2,3-b]pyridin-3-yl)-N-(2,2,2-trifluoro-1-phenylethyl)acrylamide |
| **15** | (R,E)-2-cyano-N-(1-(3,4-dimethoxyphenyl)ethyl)-3-(1H-pyrrolo[2,3-b]pyridin-3-yl)acrylamide |
| **16** | (R,E)-2-cyano-N-(1-(3,4-difluorophenyl)ethyl)-3-(1H-pyrrolo[2,3-b]pyridin-3-yl)acrylamide |
| **17** | (R,E)-2-cyano-N-(1-(2,4-difluorophenyl)ethyl)-3-(1H-pyrrolo[2,3-b]pyridin-3-yl)acrylamide |
| **18** | (R,E)-2-cyano-3-(1H-pyrrolo[2,3-b]pyridin-3-yl)-N-(1-(3-(trifluoromethyl)phenyl)ethyl)acrylamide |
| **19** | (R,E)-2-cyano-N-(1-(4-methoxyphenyl)ethyl)-3-(1H-pyrrolo[2,3-b]pyridin-3-yl)acrylamide |
| **20** | (R,E)-2-cyano-N-(1-(3,4-dichlorophenyl)ethyl)-3-(1H-pyrrolo[2,3-b]pyridin-3-yl)acrylamide |
| **21** | (E)-2-cyano-3-(1H-pyrrolo[2,3-b]pyridin-3-yl)-N-(4-sulfamoylbenzyl)acrylamide |
| **22** | (E)-2-cyano-3-(1H-pyrrolo[2,3-b]pyridin-3-yl)-N-(4-sulfamoylbenzyl)acrylamide |
| **23** | (E)-N-(5-bromo-2-methylbenzyl)-2-cyano-3-(1H-pyrrolo[2,3-b]pyridin-3-yl)acrylamide |
| **24** | (E)-2-cyano-N-(4-(dimethylamino)benzyl)-3-(1H-pyrrolo[2,3-b]pyridin-3-yl)acrylamide |
| **25** | (E)-2-cyano-N-(2-(dimethylamino)benzyl)-3-(1H-pyrrolo[2,3-b]pyridin-3-yl)acrylamide |
| **26** | (E)-2-cyano-N-(4-cyanobenzyl)-3-(1H-pyrrolo[2,3-b]pyridin-3-yl)acrylamide |
| **27** | (E)-2-cyano-3-(1H-pyrrolo[2,3-b]pyridin-3-yl)-N-(3-(sulfamoylmethyl)benzyl)-acrylamide |
| **28** | (E)-3-((2-cyano-3-(1H-pyrrolo[2,3-b]pyridin-3-yl)acrylamido)methyl)benzamide |
| **29** | (E)-2-cyano-N-(3-(methylsulfonamido)benzyl)-3-(1H-pyrrolo[2,3-b]pyridin-3-yl)acrylamide |
| **30** | (E)-2-cyano-N-(3-(dimethylamino)benzyl)-3-(1H-pyrrolo[2,3-b]pyridin-3-yl)acrylamide |
| **31** | (E)-2-cyano-N-(3-cyanobenzyl)-3-(1H-pyrrolo[2,3-b]pyridin-3-yl)acrylamide |
| **32** | (E)-2-cyano-N-(2-hydroxy-1-phenylethyl)-3-(1H-pyrrolo[2,3-b]pyridin-3-yl)acrylamide |
| **33** | (R,E)-2-cyano-N-(1-(3-methoxyphenyl)ethyl)-3-(1H-pyrrolo[2,3-b]pyridin-3-yl)acrylamide |
| **34** | (E)-N-(3-acetamidobenzyl)-2-cyano-3-(1H-pyrrolo[2,3-b]pyridin-3-yl)acrylamide |
| **35** | (E)-2-cyano-N-(2-cyanobenzyl)-3-(1H-pyrrolo[2,3-b]pyridin-3-yl)acrylamide |
| **36** | (E)-2-cyano-N-(4-methoxy-3-sulfamoylbenzyl)-3-(1H-pyrrolo[2,3-b]pyridin-3-yl)acrylamide |
| **37** | (E)-2-cyano-N-(2-(morpholinomethyl)benzyl)-3-(1H-pyrrolo[2,3-b]pyridin-3-yl)acrylamide |
| **38** | (E)-N-(1-(2-(1H-pyrazol-1-yl)phenyl)ethyl)-2-cyano-3-(1H-pyrrolo[2,3-b]pyridin-3-yl)acrylamide |
| **39** | (E)-2-cyano-N-(2-(methylsulfonyl)benzyl)-3-(1H-pyrrolo[2,3-b]pyridin-3-yl)acrylamide |
| **40** | (R,E)-2-cyano-N-(3-hydroxy-1-phenylpropyl)-3-(1H-pyrrolo[2,3-b]pyridin-3-yl)acrylamide |
| **41** | (E)-2-cyano-N-(2-(morpholinosulfonyl)benzyl)-3-(1H-pyrrolo[2,3-b]pyridin-3-yl)acrylamide |
| **42** | (E)-2-cyano-N-(2-(methylsulfonamido)benzyl)-3-(1H-pyrrolo[2,3-b]pyridin-3-yl)acrylamide |
| **43** | (E)-2-cyano-N-(2-(N-methylsulfamoyl)benzyl)-3-(1H-pyrrolo[2,3-b]pyridin-3-yl)acrylamide |
| **44** | (E)-2-cyano-N-(2-((2-oxopyrrolidin-1-yl)methyl)benzyl)-3-(1H-pyrrolo[2,3-b]pyridin-3-yl)acrylamide |
| **45** | (E)-N-((1H-indazol-4-yl)methyl)-2-cyano-3-(1H-pyrrolo[2,3-b]pyridin-3-yl)acrylamide |
| **46** | (E)-2-cyano-N-(3-morpholinobenzyl)-3-(1H-pyrrolo[2,3-b]pyridin-3-yl)acrylamide |
| **47** | (E)-N-(1-(3-bromophenyl)-2,2,2-trifluoroethyl)-2-cyano-3-(1H-pyrrolo[2,3-b]pyridin-3-yl)acrylamide |
| **48** | (E)-2-cyano-N-(3-(dimethylamino)-1-phenylpropyl)-3-(1H-pyrrolo[2,3-b]pyridin-3-yl)acrylamide |
| **49** | (E)-2-cyano-N-(cyano(phenyl)methyl)-3-(1H-pyrrolo[2,3-b]pyridin-3-yl)acrylamide |
| **50** | (E)-2-cyano-N-(2-(dimethylamino)-1-phenylethyl)-3-(1H-pyrrolo[2,3-b]pyridin-3-yl)acrylamide |
| **51** | (E)-2-cyano-N-(3-(dimethylamino)-1-(4-methoxyphenyl)propyl)-3-(1H-pyrrolo[2,3-b]pyridin-3-yl)acrylamide |
| **52** | (E)-2-cyano-N-(3-(diethylamino)-1-phenylpropyl)-3-(1H-pyrrolo[2,3-b]pyridin-3-yl)acrylamide |
| **53** | (S,E)-N-(1-(3,4-dimethoxyphenyl)-2-hydroxyethyl)-3-(5-methyl-1H-pyrrolo[2,3-b]pyridin-3-yl)acrylamide |
| **54** | (R,E)-N-(1-(4-fluoro-3-methoxyphenyl)ethyl)-3-(5-methyl-1H-pyrrolo[2,3-b]pyridin-3-yl)acrylamide |
| **55** | (R,E)-N-(1-(3,4-dimethoxyphenyl)ethyl)-3-(5-((1,1-dioxidotetrahydro-2H-thiopyran-4-yl)oxy)-1H-pyrrolo[2,3-b]pyridin-3-yl)acrylamide |
| **56** | (R, E)-N-(1-(3,4-dimethoxyphenyl)ethyl)-3-(5-(2-(dimethylamino)ethoxy)-1H-pyrrolo[2,3-b]pyridin-3-yl)acrylamide |
| **57** | (E)-2-cyano-N-(4-fluorobenzyl)-3-(1H-pyrrolo[2,3-b]pyridin-3-yl)acrylamide |
| **58** | (E)-N-(4-chlorobenzyl)-2-cyano-3-(1H-pyrrolo[2,3-b]pyridin-3-yl)acrylamide |
| **59** | (E)-2-cyano-3-(1H-pyrrolo[2,3-b]pyridin-3-yl)-N-(4-(trifluoromethyl)benzyl)acrylamide |
| **60** | (E)-N-(3-chlorobenzyl)-2-cyano-3-(1H-pyrrolo[2,3-b]pyridin-3-yl)acrylamide |
| **61** | (E)-2-cyano-3-(1H-pyrrolo[2,3-b]pyridin-3-yl)-N-(3-(trifluoromethyl)benzyl)acrylamide |
| **62** | (E)-N-(2-chlorobenzyl)-2-cyano-3-(1H-pyrrolo[2,3-b]pyridin-3-yl)acrylamide |
| **63** | (E)-2-cyano-N-(1-(4-fluorophenyl)ethyl)-3-(1H-pyrrolo[2,3-b]pyridin-3-yl)acrylamide |
| **64** | (R,E)-2-cyano-N-(1-phenylethyl)-3-(1H-pyrrolo[2,3-b]pyridin-3-yl)acrylamide |
| **65** | (E)-2-cyano-N-(2,4-difluorobenzyl)-3-(1H-pyrrolo[2,3-b]pyridin-3-yl)acrylamide |
| **66** | (E)-2-cyano-N-(3,4-difluorobenzyl)-3-(1H-pyrrolo[2,3-b]pyridin-3-yl)acrylamide |
| **67** | (E)-2-cyano-N-(3,5-difluorobenzyl)-3-(1H-pyrrolo[2,3-b]pyridin-3-yl)acrylamide |
| **68** | (R,E)-2-cyano-N-(1-phenylpropyl)-3-(1H-pyrrolo[2,3-b]pyridin-3-yl)acrylamide |
| **69** | (R,E)-2-cyano-N-(1-(4-fluorophenyl)ethyl)-3-(1H-pyrrolo[2,3-b]pyridin-3-yl)acrylamide |
| **70** | (R,E)-N-(1-(4-chlorophenyl)ethyl)-2-cyano-3-(1H-pyrrolo[2,3-b]pyridin-3-yl)acrylamide |
| **71** | (E)-N-benzyl-2-cyano-3-(5-(trifluoromethyl)-1H-pyrrolo[2,3-b]pyridin-3-yl)acrylamide |
| **72** | (E)-3-(5-chloro-1H-pyrrolo[2,3-b]pyridin-3-yl)-2-cyano-N-(4-fluorobenzyl)acrylamide |
| **73** | (E)-N-benzyl-3-(5-chloro-1H-pyrrolo[2,3-b]pyridin-3-yl)-2-cyanoacrylamide |
| **74** | (E)-3-(5-chloro-1H-pyrrolo[2,3-b]pyridin-3-yl)-N-(2-chlorobenzyl)-2-cyanoacrylamide |
| **75** | (E)-3-(5-chloro-1H-pyrrolo[2,3-b]pyridin-3-yl)-2-cyano-N-(3,5-difluorobenzyl)acrylamide |
| **76** | (R,E)-3-(5-chloro-1H-pyrrolo[2,3-b]pyridin-3-yl)-2-cyano-N-(1-(4-fluorophenyl)ethyl)acrylam ide |
| **77** | (R,E)-3-(5-chloro-1H-pyrrolo[2,3-b]pyridin-3-yl)-2-cyano-N-(1-phenylpropyl)acrylamide |
| **78** | (R,E)-3-(4-chloro-1H-pyrrolo[2,3-b]pyridin-3-yl)-2-cyano-N-(1-(4-fluorophenyl)ethyl)acrylam ide |
| **79** | (R,E)-3-(5-bromo-1H-pyrrolo[2,3-b]pyridin-3-yl)-2-cyano-N-(1-(4-fluorophenyl)ethyl)acrylam ide |
| **80** | (R,E)-3-(5-bromo-1H-pyrrolo[2,3-b]pyridin-3-yl)-2-cyano-N-(1-(3,4-dimethoxyphenyl)ethyl)acrylamide |
| **81** | (R,E)-2-cyano-N-(1-(3,4-dimethoxyphenyl)ethyl)-3-(5-hydroxy-1H-pyrrolo[2,3-b]pyrid in-3-yl)acrylamide |
| **82** | (R,E)-2-cyano-N-(1-(3,4-dimethoxyphenyl)ethyl)-3-(5-((tetrahydro-2H-pyran-4-yl)oxy)-1H-pyrrolo[2,3-b]pyridin-3-yl)acrylamide |
| **83** | (R,E)-2-cyano-N-(1-(3,4-dimethoxyphenyl)ethyl)-3-(5-(4-methylpiperazine-1-carbonyl)-1H-pyrrolo[2,3-b]pyridin-3-yl)acrylamide |
| **84** | (R,E)-2-cyano-N-(1-(3,4-dimethoxyphenyl)ethyl)-3-(5-morpholino-1H-pyrrolo[2,3-b]pyridin-3-yl)acrylamide |
| **85** | (R,E)-2-cyano-N-(1-(3,4-dimethoxyphenyl)ethyl)-3-(5-morpholino-1H-pyrrolo[2,3-b]pyridin-3-yl)acrylamide |
| **86** | (R,E)-2-cyano-3-(5-cyano-1H-pyrrolo[2,3-b]pyridin-3-yl)-N-(1-(3,4-dimethoxyphenyl)ethyl)acrylamide |
| **87** | (R,E)-2-cyano-N-(1-(3,4-dimethoxyphenyl)ethyl)-3-(5-methyl-1H-pyrrolo[2,3-b]pyrid in-3-yl)acrylamide |
| **88** | (R,E)-2-cyano-N-(1-(3,4-dimethoxyphenyl)ethyl)-3-(5-(phenylamino)-1H-pyrrolo[2,3-b]pyridin-3-yl)acrylamide |
| **89** | (R,E)-2-cyano-N-(1-(3,4-dimethoxyphenyl)ethyl)-3-(5-(1-(piperidin-4-yl)-1H-pyrazol-4-yl)-1H-pyrrolo[2,3-b]pyridin-3-yl)acrylamide |
| **90** | (R,E)-3-(6-bromo-1H-pyrrolo[2,3-b]pyridin-3-yl)-2-cyano-N-(1-(3,4-dimethoxyphenyl)ethyl)acrylamide |
| **91** | (R,E)-2-cyano-3-(5-(1-methyl-1H-pyrazol-4-yl)-1H-pyrrolo[2,3-b]pyridin-3-yl)-N-(1-phenylethyl)acrylamide |
| **92** | (S,E)-2-cyano-N-(1-(3,4-dimethoxyphenyl)-2-hydroxyethyl)-3-(5-(1-methyl-1H-pyrazol-4-yl)-1H-pyrrolo[2,3-b]pyridin-3-yl)acrylamide |
| **93** | (R,E)-2-cyano-N-(1-(4-fluorophenyl)ethyl)-3-(5-(1-methyl-1H-pyrazol-4-yl)-1H-pyrrolo[2,3-b]pyridin-3-yl)acrylamide |
| **94** | (R,E)-2-cyano-N-(1-(4-fluoro-3-methoxyphenyl)ethyl)-3-(5-(1-methyl-1H-pyrazol-4-yl)-1H-pyrrolo[2,3-b]pyridin-3-yl)acrylamide |
| **95** | (R,E)-N-(1-(4-chlorophenyl)ethyl)-2-cyano-3-(5-(1-methyl-1H-pyrazol-4-yl)-1H-pyrrolo[2,3-b]pyridin-3-yl)acrylamide |
| **96** | (R,E)-N-(1-(3-chlorophenyl)ethyl)-2-cyano-3-(5-(1-methyl-1H-pyrazol-4-yl)-1H-pyrrolo[2,3-b]pyridin-3-yl)acrylamide |
| **97** | (R,E)-2-cyano-3-(4-methyl-5-(1-methyl-1H-pyrazol-4-yl)-1H-pyrrolo[2,3-b]pyridin-3-yl)-N-(1-phenylethyl)acrylamide |
| **98** | (R,E)-2-cyano-N-(1-(4-fluorophenyl)ethyl)-3-(4-methyl-5-(1-methyl-1H-pyrazol-4-yl)-1H-pyrrolo[2,3-b]pyridin-3-yl)acrylamide |
| **99** | (R,E)-2-cyano-N-(1-(3,4-dimethoxyphenyl)ethyl)-3-(4-methyl-5-(1-methyl-1H-pyrazol-4-yl)-1H-pyrrolo[2,3-b]pyridin-3-yl)acrylamide |
| **100** | (R,E)-N-(1-(4-chlorophenyl)ethyl)-2-cyano-3-(4-methyl-5-(1-methyl-1H-pyrazol-4-yl)-1H-pyrrolo[2,3-b]pyridin-3-yl)acrylamide |
| **101** | (R,E)-2-cyano-N-(1-(3,4-dimethoxyphenyl)ethyl)-3-(5-((1,3-dimethyl-1H-pyrazol-5-yl)methoxy)-1H-pyrrolo[2,3-b]pyridin-3-yl)acrylamide |
| **102** | (R,E)-2-cyano-N-(1-(3,4-dimethoxyphenyl)ethyl)-3-(5-isopropoxy-1H-pyrrolo[2,3-b]pyridin-3-yl)acrylamide |
| **103** | (E)-2-cyano-N-((R)-1-(3,4-dimethoxyphenyl)ethyl)-3-(5-((tetrahydrofuran-3-yl)oxy)-1H-pyrrolo[2,3-b]pyridin-3-yl)acrylamide |
| **104** | (R,E)-2-cyano-N-(1-(3,4-dimethoxyphenyl)ethyl)-3-(5-(2-(dimethylamino)ethoxy)-1H-pyrrolo[2,3-b]pyridin-3-yl)acrylamide |
| **105** | (R,E)-2-cyano-N-(1-(3,4-dimethoxyphenyl)ethyl)-3-(5-((1,1-dioxidotetrahydro-2H-thiopyran-4-yl)oxy)-1H-pyrrolo[2,3-b]pyridin-3-yl)acrylamide |
| **106** | (R,E)-2-cyano-N-(1-(3,4-dimethoxyphenyl)ethyl)-3-(5-((1-methyl-1H-imidazol-2-yl)methoxy)-1H-pyrrolo[2,3-b]pyridin-3-yl)acrylamide |
| **107** | (E)-2-cyano-N-((R)-1-(3,4-dimethoxyphenyl)ethyl)-3-(5-(((R)-tetrahydrofuran-3-yl)oxy)-1H-pyrrolo[2,3-b]pyridin-3-yl)acrylamide |
| **108** | (E)-2-cyano-N-((R)-1-(3,4-dimethoxyphenyl)ethyl)-3-(5-(((S)-tetrahydrofuran-3-yl)oxy)-1H-pyrrolo[2,3-b]pyridin-3-yl)acrylamide |
| **109** | (E)-3-(5-((S)-sec-butoxy)-1H-pyrrolo[2,3-b]pyridin-3-yl)-2-cyano-N-((R)-1-(3,4-dimethoxyphenyl)ethyl)acrylamide |
| **110** | (E)-3-(5-((S)-sec-butoxy)-1H-pyrrolo[2,3-b]pyridin-3-yl)-2-cyano-N-((R)-1-(3,4-dimethoxyphenyl)ethyl)acrylamide |
| **111** | (R,E)-2-cyano-N-(1-(3,4-dimethoxyphenyl)ethyl)-3-(5-((tetrahydro-2H-pyran-4-yl)oxy)-1H-pyrrolo[2,3-b]pyridin-3-yl)acrylamide |
| **112** | (R,E)-2-cyano-N-(1-(3,4-dimethoxyphenyl)ethyl)-3-(5-(4-(4-methylpiperazin-1-yl)phenyl)-1H-pyrrolo[2,3-b]pyridin-3-yl)acrylamide |
| **113** | (R,E)-N-(1-(3,4-dimethoxyphenyl)ethyl)-3-(5-(4-(4-methylpiperazin-1-yl)phenyl)-1H-pyrrolo[2,3-b]pyridin-3-yl)acrylamide |
| **114** | (R, E)-N-(1-(3,4-dimethoxyphenyl)ethyl)-3-(5-(3-(methylsulfonyl)phenyl)-1H-pyrrolo[2,3-b]pyridin-3-yl)acrylamide |
| **115** | (R,E)-3-(5-(3,5-difluorophenyl)-1H-pyrrolo[2,3-b]pyridin-3-yl)-N-(1-(3,4-dimethoxyphenyl)ethyl)acrylam ide |
| **116** | (R,E)-2-cyano-N-(1-(3,4-dimethoxyphenyl)ethyl)-3-(5-(4-(4-methylpiperazin-1-yl)phenyl)-1H-pyrrolo[2,3-b]pyridin-3-yl)acrylamide |
| **117** | (R,E)-2-cyano-N-(1-(3,4-dimethoxyphenyl)ethyl)-3-(5-(4-((dimethylamino)methyl)phenyl)-1H-pyrrolo[2,3-b]pyridin-3-yl)acrylamide |
| **118** | (R,E)-2-cyano-N-(1-(3,4-dimethoxyphenyl)ethyl)-3-(5-(3-((4-methylpiperazin-1-yl)methyl)phenyl)-1H-pyrrolo[2,3-b]pyridin-3-yl)acrylamide |
| **119** | (R,E)-2-cyano-N-(1-(3,4-dimethoxyphenyl)ethyl)-3-(5-(3-((4-methylpiperazin-1-yl)methyl)phenyl)-1H-pyrrolo[2,3-b]pyridin-3-yl)acrylamide |
| **120** | (R,E)-2-cyano-N-(1-(3,4-dimethoxyphenyl)ethyl)-3-(5-(pyrimidin-5-yl)-1H-pyrrolo[2,3-b]pyridin-3-yl)acrylamide |
| **121** | (R,E)-2-cyano-N-(1-(3,4-dimethoxyphenyl)ethyl)-3-(5-(4-((4-ethylpiperazin-1-yl)sulfonyl)phenyl)-1H-pyrrolo[2,3-b]pyridin-3-yl)acrylamide |
| **122** | (R,E)-2-cyano-N-(1-(3,4-dimethoxyphenyl)ethyl)-3-(5-(3-(4-methylpiperazin-1-yl)phenyl)-1H-pyrrolo[2,3-b]pyridin-3-yl)acrylamide |
| **123** | (R,E)-2-cyano-N-(1-(3,4-dimethoxyphenyl)ethyl)-3-(5-(3-(4-methylpiperazin-1-yl)phenyl)-1H-pyrrolo[2,3-b]pyridin-3-yl)acrylamide |
| **124** | (R,E)-2-cyano-N-(1-(3,4-dimethoxyphenyl)ethyl)-3-(5-(4-fluoro-2-methoxyphenyl)-1H-pyrrolo[2,3-b]pyridin-3-yl)acrylamide |
| **125** | (R, E)-2-cyano-N-(1-(3,4-dimethoxyphenyl)ethyl)-3-(5-(2-methoxyphenyl)-1H-pyrrolo[2,3-b]pyridin-3-yl)acrylamide |
| **126** | (R,E)-4-(3-(2-cyano-3-((1-(3,4-dimethoxyphenyl)ethyl)amino)-3-oxoprop-1-en-1-yl)-1H-pyrrolo[2,3-b]pyridin-5-yl)benzamide |
| **127** | (R,E)-2-cyano-N-(1-(3,4-dimethoxyphenyl)ethyl)-3-(5-(3-(morpholine-4-carbonyl)phenyl)-1H-pyrrolo[2,3-b]pyridin-3-yl)acrylamide |
| **128** | (R,E)-2-cyano-N-(1-(3,4-dimethoxyphenyl)ethyl)-3-(5-(3-(dimethylamino)phenyl)-1H-pyrrolo[2,3-b]pyridin-3-yl)acrylamide |
| **129** | (R,E)-2-cyano-N-(1-(3,4-dimethoxyphenyl)ethyl)-3-(5-(2-(piperidin-1-ylmethyl)phenyl)-1H-pyrrolo[2,3-b]pyridin-3-yl)acrylamide |
| **130** | (R,E)-2-cyano-N-(1-(3,4-dimethoxyphenyl)ethyl)-3-(5-(2-(piperidin-1-ylmethyl)phenyl)-1H-pyrrolo[2,3-b]pyridin-3-yl)acrylamide |
| **131** | (R,E)-2-cyano-N-(1-(3,4-dimethoxyphenyl)ethyl)-3-(5-(pyridin-3-yl)-1H-pyrrolo[2,3-b]pyridin-3-yl)acrylamide |
| **132** | (R,E)-2-cyano-N-(1-(3,4-dimethoxyphenyl)ethyl)-3-(5-(4-(1-methylpiperidin-4-yl)phenyl)-1H-pyrrolo[2,3-b]pyridin-3-yl)acrylamide |
| **133** | (R,E)-2-cyano-N-(1-(3,4-dimethoxyphenyl)ethyl)-3-(5-(4-(1-methylpiperidin-4-yl)phenyl)-1H-pyrrolo[2,3-b]pyridin-3-yl)acrylamide |
| **134** | (R,E)-2-cyano-N-(1-(3,4-dimethoxyphenyl)ethyl)-3-(5-(3-(3-(dimethylamino)propoxy)phenyl)-1H-pyrrolo[2,3-b]pyridin-3-yl)acrylamide |
| **135** | (R,E)-2-cyano-N-(1-(3,4-dimethoxyphenyl)ethyl)-3-(5-(4-(2-(dimethylamino)ethyl)phenyl)-1H-pyrrolo[2,3-b]pyridin-3-yl)acrylamide |
| **136** | (R,Z)-2-cyano-N-(1-(3,4-dimethoxyphenyl)ethyl)-3-(5-(4-(2-(dimethylamino)ethyl)phenyl)-1H-pyrrolo[2,3-b]pyridin-3-yl)acrylamide |
| **137** | (R,E)-2-cyano-N-(1-(3,4-dimethoxyphenyl)ethyl)-3-(5-(3-methoxyphenyl)-1H-pyrrolo[2,3-b]pyridin-3-yl)acrylamide |
| **138** | (R,E)-2-cyano-N-(1-(3,4-dimethoxyphenyl)ethyl)-3-(5-(3-methoxyphenyl)-1H-pyrrolo[2,3-b]pyridin-3-yl)acrylamide |
| **139** | (R,E)-2-cyano-N-(1-(3,4-dimethoxyphenyl)ethyl)-3-(5-(2-(morpholinomethyl)phenyl)-1H-pyrrolo[2,3-b]pyridin-3-yl)acrylamide |
| **140** | (R,Z)-2-cyano-3-(5-(4-cyanophenyl)-1H-pyrrolo[2,3-b]pyridin-3-yl)-N-(1-(3,4-dimethoxyphenyl)ethyl)acrylamide |
| **141** | (R,E)-3-(5-(3-((4-acetylpiperazin-1-yl)methyl)phenyl)-1H-pyrrolo[2,3-b]pyridin-3-yl)-2-cyano-N-(1-(3,4-dimethoxyphenyl)ethyl)acrylamide |
| **142** | (R,E)-3-(5-(3-((4-acetylpiperazin-1-yl)methyl)phenyl)-1H-pyrrolo[2,3-b]pyridin-3-yl)-2-cyano-N-(1-(3,4-dimethoxyphenyl)ethyl)acrylamide |
| **143** | (R,E)-3-(5-(4-acetamidophenyl)-1H-pyrrolo[2,3-b]pyridin-3-yl)-2-cyano-N-(1-(3,4-dimethoxyphenyl)ethyl)acrylamide |
| **144** | (R,E)-2-cyano-N-(1-(3,4-dimethoxyphenyl)ethyl)-3-(5-(pyridin-4-yl)-1H-pyrrolo[2,3-b]pyridin-3-yl)acrylamide |
| **145** | (R,E)-2-cyano-N-(1-(3,4-dimethoxyphenyl)ethyl)-3-(5-(naphthalen-2-yl)-1H-pyrrolo[2,3-b]pyridin-3-yl)acrylamide |
| **146** | (R,Z)-2-cyano-3-(5-(3,4-dimethoxyphenyl)-1H-pyrrolo[2,3-b]pyridin-3-yl)-N-(1-(3,4-dimethoxyphenyl)ethyl)acrylamide |
| **147** | (R,E)-2-cyano-N-(1-(3,4-dimethoxyphenyl)ethyl)-3-(5-(6-(dimethylamino)pyridin-3-yl)-1H-pyrrolo[2,3-b]pyridin-3-yl)acrylamide |
| **148** | (R,E)-2-cyano-N-(1-(3,4-dimethoxyphenyl)ethyl)-3-(5-(6-(dimethylamino)pyridin-3-yl)-1H-pyrrolo[2,3-b]pyridin-3-yl)acrylamide |
| **149** | (R,E)-2-cyano-N-(1-(3,4-dimethoxyphenyl)ethyl)-3-(5-(3-((dimethylamino)methyl)phenyl)-1H-pyrrolo[2,3-b]pyridin-3-yl)acrylamide |
| **150** | (R,E)-2-cyano-N-(1-(3,4-dimethoxyphenyl)ethyl)-3-(5-(3-(N,N-dimethylsulfamoyl)phenyl)-1H-pyrrolo[2,3-b]pyridin-3-yl)acrylamide |
| **151** | (R,E)-2-cyano-N-(1-(3,4-dimethoxyphenyl)ethyl)-3-(5-(3-(methylsulfonamido)phenyl)-1H-pyrrolo[2,3-b]pyridin-3-yl)acrylamide |
| **152** | (R,E)-2-cyano-N-(1-(3,4-dimethoxyphenyl)ethyl)-3-(5-(3-(methylsulfonamido)phenyl)-1H-pyrrolo[2,3-b]pyridin-3-yl)acrylamide |
| **153** | (R,E)-2-cyano-N-(1-(3,4-dimethoxyphenyl)ethyl)-3-(5-(3-(2-(dimethylamino)ethoxy)phenyl)-1H-pyrrolo[2,3-b]pyridin-3-yl)acrylamide |
| **154** | (R,E)-2-cyano-N-(1-(3,4-dimethoxyphenyl)ethyl)-3-(5-(2-methoxy-4-(4-methylpiperazin-1-yl)phenyl)-1H-pyrrolo[2,3-b]pyridin-3-yl)acrylamide |
| **155** | (R,E)-2-cyano-N-(1-(3,4-dimethoxyphenyl)ethyl)-3-(5-(4-(2-(dimethylamino)ethoxy)phenyl)-1H-pyrrolo[2,3-b]pyridin-3-yl)acrylamide |
| **156** | (R,E)-2-cyano-3-(5-(cyclohex-1-en-1-yl)-1H-pyrrolo[2,3-b]pyridin-3-yl)-N-(1-(3,4-dimethoxyphenyl)ethyl)acrylamide |
| **157** | (R,E)-2-cyano-3-(5-(cyclohex-1-en-1-yl)-1H-pyrrolo[2,3-b]pyridin-3-yl)-N-(1-(3,4-dimethoxyphenyl)ethyl)acrylam ide |
| **158** | (R,E)-2-cyano-3-(5-(cyclopent-1-en-1-yl)-1H-pyrrolo[2,3-b]pyridin-3-yl)-N-(1-(3,4-dimethoxyphenyl)ethyl)acrylamide |
| **159** | (R,E)-2-cyano-3-(5-(cyclopent-1-en-1-yl)-1H-pyrrolo[2,3-b]pyridin-3-yl)-N-(1-(3,4-dimethoxyphenyl)ethyl)acrylamide |
| **160** | (R,E)-3-(5-(1-benzyl-1H-pyrazol-4-yl)-1H-pyrrolo[2,3-b]pyridin-3-yl)-2-cyano-N-(1-(3,4-dimethoxyphenyl)ethyl)acrylamide |
| **161** | (R,E)-2-cyano-N-(1-(3,4-dimethoxyphenyl)ethyl)-3-(5-(1-isobutyl-1H-pyrazol-4-yl)-1H-pyrrolo[2,3-b]pyridin-3-yl)acrylamide |
| **162** | (R,E)-2-cyano-N-(1-(3,4-dimethoxyphenyl)ethyl)-3-(5-(1-isobutyl-1H-pyrazol-4-yl)-1H-pyrrolo[2,3-b]pyridin-3-yl)acrylamide |
| **163** | (R,E)-2-cyano-N-(1-(3,4-dimethoxyphenyl)ethyl)-3-(5-(2-methylimidazo[1,2-a]pyridin-6-yl)-1H-pyrrolo[2,3-b]pyridin-3-yl)acrylamide |
| **164** | (R,E)-3-(5-(1H-pyrazol-4-yl)-1H-pyrrolo[2,3-b]pyridin-3-yl)-2-cyano-N-(1-(3,4-dimethoxyphenyl)ethyl)acrylamide |
| **165** | (R,E)-2-cyano-N-(1-(3,4-dimethoxyphenyl)ethyl)-3-(5-(1-methyl-1H-pyrazol-4-yl)-1H-pyrrolo[2,3-b]pyridin-3-yl)acrylamide |
| **166** | (R,E)-2-cyano-N-(1-(3,4-dimethoxyphenyl)ethyl)-3-(5-(1-methyl-1H-imidazol-4-yl)-1H-pyrrolo[2,3-b]pyridin-3-yl)acrylamide |
| **167** | (R,E)-2-cyano-N-(1-(3,4-dimethoxyphenyl)ethyl)-3-(5-(1-methyl-1H-imidazol-4-yl)-1H-pyrrolo[2,3-b]pyridin-3-yl)acrylamide |
| **168** | (R,E)-2-cyano-N-(1-(3,4-dimethoxyphenyl)ethyl)-3-(5-(1,3-dimethyl-1H-pyrazol-4-yl)-1H-pyrrolo[2,3-b]pyridin-3-yl)acrylamide |
| **169** | (R,E)-2-cyano-N-(1-(3,4-dimethoxyphenyl)ethyl)-3-(5-(1,3-dimethyl-1H-pyrazol-4-yl)-1H-pyrrolo[2,3-b]pyridin-3-yl)acrylamide |
| **170** | (R,E)-2-cyano-N-(1-(3,4-dimethoxyphenyl)ethyl)-3-(5-(4-fluoro-3-(methylsulfonyl)phenyl)-1H-pyrrolo[2,3-b]pyridin-3-yl)acrylamide |
| **171** | (R,E)-2-cyano-N-(1-(3,4-dimethoxyphenyl)ethyl)-3-(4-(4-(4-methylpiperazin-1-yl)phenyl)-1H-pyrrolo[2,3-b]pyridin-3-yl)acrylamide |
| **172** | (R,E)-2-cyano-N-(1-(3,4-dimethoxyphenyl)ethyl)-3-(4-(3-(4-methylpiperazin-1-yl)phenyl)-1H-pyrrolo[2,3-b]pyridin-3-yl)acrylamide |
| **173** | (R,E)-2-cyano-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamide |
| **174** | (R,E)-2-cyano-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamide |
| **175** | (R,E)-2-cyano-N-(1-(3,4-dimethoxyphenyl)ethyl)-3-(4-(3-((4-methylpiperazin-1-yl)methyl)phenyl)-1H-pyrrolo[2,3-b]pyridin-3-yl)acrylamide |
| **176** | (R,E)-2-cyano-N-(1-(3,4-dimethoxyphenyl)ethyl)-3-(4-(3-((dimethylamino)methyl)phenyl)-1H-pyrrolo[2,3-b]pyridin-3-yl)acrylamide |
| **177** | (R,E)-2-cyano-N-(1-(3,4-dimethoxyphenyl)ethyl)-3-(4-(3-((dimethylamino)methyl)phenyl)-1H-pyrrolo[2,3-b]pyridin-3-yl)acrylamide |
| **178** | (R,E)-2-cyano-N-(1-(3,4-dimethoxyphenyl)ethyl)-3-(4-(4-((dimethylamino)methyl)phenyl)-1H-pyrrolo[2,3-b]pyridin-3-yl)acrylamide |
| **179** | (R,E)-2-cyano-3-(4-(3,5-difluorophenyl)-1H-pyrrolo[2,3-b]pyridin-3-yl)-N-(1-(3,4-dimethoxyphenyl)ethyl)acrylam ide |
| **180** | (R,E)-2-cyano-N-(1-(4-fluorophenyl)ethyl)-3-(5-phenyl-1H-pyrrolo[2,3-b]pyrid in-3-yl)acrylamide |
| **181** | (R,E)-2-cyano-N-(1-(4-fluorophenyl)ethyl)-3-(5-(3-(4-methylpiperazin-1-yl)phenyl)-1H-pyrrolo[2,3-b]pyridin-3-yl)acrylamide |
| **182** | (R,E)-2-cyano-N-(1-(4-fluorophenyl)ethyl)-3-(5-(3-(4-methylpiperazin-1-yl)phenyl)-1H-pyrrolo[2,3-b]pyridin-3-yl)acrylamide |
| **183** | (R,E)-3-(5-(4-(4-acetylpiperazin-1-yl)phenyl)-1H-pyrrolo[2,3-b]pyridin-3-yl)-N-(1-(3,4-dimethoxyphenyl)ethyl)acrylamide |
| **184** | (R,E)-N-(1-(3,4-dimethoxyphenyl)ethyl)-3-(5-(3-((4-methylpiperazin-1-yl)methyl)phenyl)-1H-pyrrolo[2,3-b]pyridin-3-yl)acrylamide |
| **185** | (R, E)-N-(1-(3,4-dimethoxyphenyl)ethyl)-3-(5-(4-((dimethylamino)methyl)phenyl)-1H-pyrrolo[2,3-b]pyridin-3-yl)acrylamide |
| **186** | (R,E)-N-(1-(3,4-dimethoxyphenyl)ethyl)-3-(5-(pyridin-3-yl)-1H-pyrrolo[2,3-b]pyridin-3-yl)acrylamide |
| **187** | (R,E)-N-(1-(3,4-dimethoxyphenyl)ethyl)-3-(5-(pyridin-3-yl)-1H-pyrrolo[2,3-b]pyridin-3-yl)acrylamide |
| **188** | (R, E)-N-(1-(3,4-dimethoxyphenyl)ethyl)-3-(5-(4-(methylsulfonamido)phenyl)-1H-pyrrolo[2,3-b]pyridin-3-yl)acrylamide |
| **189** | (R, E)-N-(1-(3,4-dimethoxyphenyl)ethyl)-3-(5-(3-(3-(dimethylamino)propoxy)phenyl)-1H-pyrrolo[2,3-b]pyridin-3-yl)acrylamide |
| **190** | (R,E)-3-(3-(3-((1-(3,4-dimethoxyphenyl)ethyl)amino)-3-oxoprop-1-en-1-yl)-1H-pyrrolo[2,3-b]pyridin-5-yl)benzamide |
| **191** | (E)-N-((R)-1-(3,4-dimethoxyphenyl)ethyl)-3-(5-(3-(ethylsulfinyl)phenyl)-1H-pyrrolo[2,3-b]pyridin-3-yl)acrylamide |
| **192** | (E)-N-((R)-1-(3,4-dimethoxyphenyl)ethyl)-3-(5-(3-(ethylsulfinyl)phenyl)-1H-pyrrolo[2,3-b]pyridin-3-yl)acrylamide |
| **193** | (R, E)-N-(1-(3,4-dimethoxyphenyl)ethyl)-3-(5-(3-(2-(dimethylamino)ethyl)phenyl)-1H-pyrrolo[2,3-b]pyridin-3-yl)acrylamide |
| **194** | (R, E)-N-(1-(3,4-dimethoxyphenyl)ethyl)-3-(5-(3-(methylsulfonamido)phenyl)-1H-pyrrolo[2,3-b]pyridin-3-yl)acrylamide |
| **195** | (R, E)-N-(1-(3,4-dimethoxyphenyl)ethyl)-3-(5-(3-(dimethylamino)phenyl)-1H-pyrrolo[2,3-b]pyridin-3-yl)acrylamide |
| **196** | (R,E)-N-(1-(3,4-dimethoxyphenyl)ethyl)-3-(5-(4-(1-methylpiperidin-4-yl)phenyl)-1H-pyrrolo[2,3-b]pyridin-3-yl)acrylamide |
| **197** | (R, E)-N-(1-(3,4-dimethoxyphenyl)ethyl)-3-(5-(4-(2-(dimethylamino)ethoxy)phenyl)-1H-pyrrolo[2,3-b]pyridin-3-yl)acrylamide |
| **198** | (R, E)-N-(1-(3,4-dimethoxyphenyl)ethyl)-3-(5-(3-(N, N-dimethylsulfamoyl)phenyl)-1H-pyrrolo[2,3-b]pyridin-3-yl)acrylamide |
| **199** | (R,E)-3-(5-(3-(1H-imidazol-2-yl)phenyl)-1H-pyrrolo[2,3-b]pyridin-3-yl)-N-(1-(3,4-dimethoxyphenyl)ethyl)acrylamide |
| **200** | (R,E)-4-(3-(3-((1-(3,4-dimethoxyphenyl)ethyl)amino)-3-oxoprop-1-en-1-yl)-1H-pyrrolo[2,3-b]pyridin-5-yl)benzamide |
| **201** | (R,E)-N-(1-(3,4-dimethoxyphenyl)ethyl)-3-(5-(4-fluoro-2-methoxyphenyl)-1H-pyrrolo[2,3-b]pyridin-3-yl)acrylamide |
| **202** | (R,E)-N-(1-(3,4-dimethoxyphenyl)ethyl)-3-(5-(1,5-dimethyl-1H-pyrazol-4-yl)-1H-pyrrolo[2,3-b]pyridin-3-yl)acrylamide |
| **203** | (R,E)-3-(5-(3-(acetamidomethyl)phenyl)-1H-pyrrolo[2,3-b]pyridin-3-yl)-N-(1-(3,4-dimethoxyphenyl)ethyl)acrylam ide |
| **204** | (R,E)-N-(1-(3,4-dimethoxyphenyl)ethyl)-3-(5-(1-methyl-1H-pyrazol-4-yl)-1H-pyrrolo[2,3-b]pyridin-3-yl)acrylamide |
| **205** | (R,E)-N-(1-(3,4-dimethoxyphenyl)ethyl)-3-(5-(pyrazolo[1,5-a]pyridin-3-yl)-1H-pyrrolo[2,3-b]pyridin-3-yl)acrylamide |
| **206** | (R, E)-N-(1-(3,4-dimethoxyphenyl)ethyl)-3-(5-(2-(morpholinomethyl)phenyl)-1H-pyrrolo[2,3-b]pyridin-3-yl)acrylamide |
| **207** | (R, E)-N-(1-(3,4-dimethoxyphenyl)ethyl)-3-(5-(pyrim idin-5-yl)-1H-pyrrolo[2,3-b]pyrid in-3-yl)acrylamide |
| **208** | (R,E)-N-(1-(3,4-dimethoxyphenyl)ethyl)-3-(5-(3-sulfamoylphenyl)-1H-pyrrolo[2,3-b]pyridin-3-yl)acrylamide |
| **209** | (R,E)-N-(1-(3,4-dimethoxyphenyl)ethyl)-3-(5-(1,3,5-trimethyl-1H-pyrazol-4-yl)-1H-pyrrolo[2,3-b]pyridin-3-yl)acrylamide |
| **210** | (R, E)-N-(1-(3,4-dimethoxyphenyl)ethyl)-3-(5-(1-(2-morpholinoethyl)-1H-pyrazol-4-yl)-1H-pyrrolo[2,3-b]pyridin-3-yl)acrylamide |
| **211** | (R,E)-3-(5-(5-cyano-1-methyl-1H-pyrazol-4-yl)-1H-pyrrolo[2,3-b]pyridin-3-yl)-N-(1-(3,4-dimethoxyphenyl)ethyl)acrylamide |
| **212** | (R,E)-N-(1-(3,4-dimethoxyphenyl)ethyl)-3-(5-(1,3-dimethyl-1H-pyrazol-4-yl)-1H-pyrrolo[2,3-b]pyridin-3-yl)acrylamide |
| **213** | (R, E)-N-(1-(3,4-dimethoxyphenyl)ethyl)-3-(5-(1-(2-(dimethylamino)ethyl)-1H-pyrazol-4-yl)-1H-pyrrolo[2,3-b]pyridin-3-yl)acrylamide |
| **214** | (E)-N-((R)-1-(3,4-dimethoxyphenyl)ethyl)-3-(5-(1-(2-hydroxypropyl)-1H-pyrazol-4-yl)-1H-pyrrolo[2,3-b]pyridin-3-yl)acrylamide |
| **215** | (R,E)-N-(1-(3,4-dimethoxyphenyl)ethyl)-3-(5-(1-(oxetan-3-yl)-1H-pyrazol-4-yl)-1H-pyrrolo[2,3-b]pyridin-3-yl)acrylamide |
| **216** | (R,E)-3-(5-(3-(cyclopropylsulfonyl)phenyl)-1H-pyrrolo[2,3-b]pyridin-3-yl)-N-(1-(3,4-dimethoxyphenyl)ethyl)acrylamide |
| **217** | (R, E)-N-(1-(3,4-dimethoxyphenyl)ethyl)-3-(5-(3-((methylsulfonyl)methyl)phenyl)-1H-pyrrolo[2,3-b]pyridin-3-yl)acrylamide |
| **218** | (R, E)-N-(1-(3,4-dimethoxyphenyl)ethyl)-3-(5-(5-(methylsulfonyl)pyridin-3-yl)-1H-pyrrolo[2,3-b]pyridin-3-yl)acrylamide |
| **219** | (R, E)-N-(1-(3,4-dimethoxyphenyl)ethyl)-3-(5-(4-(methylsulfonyl)phenyl)-1H-pyrrolo[2,3-b]pyridin-3-yl)acrylamide |
| **220** | (R,E)-N-(1-(3,4-dimethoxyphenyl)ethyl)-3-(5-(4-fluoro-3-(methylsulfonyl)phenyl)-1H-pyrrolo[2,3-b]pyridin-3-yl)acrylamide |
| **221** | (R,E)-3-(5-(3-cyanophenyl)-1H-pyrrolo[2,3-b]pyridin-3-yl)-N-(1-(3,4-dimethoxyphenyl)ethyl)acrylamide |
| **222** | (R, E)-N-(1-(3,4-dimethoxyphenyl)ethyl)-3-(5-(2-(methylsulfonyl)phenyl)-1H-pyrrolo[2,3-b]pyridin-3-yl)acrylamide |
| **223** | (R,E)-N-(1-(3,4-dimethoxyphenyl)ethyl)-3-(4-(4-((dimethylamino)methyl)phenyl)-1H-pyrrolo[2,3-b]pyridin-3-yl)acrylamide |
| **224** | (R,E)-N-(1-(3,4-dimethoxyphenyl)ethyl)-3-(4-(3-((dimethylamino)methyl)phenyl)-1H-pyrrolo[2,3-b]pyridin-3-yl)acrylamide |
| **225** | (R,E)-N-(1-(3,4-dimethoxyphenyl)ethyl)-3-(4-phenyl-1H-pyrrolo[2,3-b]pyridin-3-yl)acrylamide |
| **226** | (R,E)-N-(1-(3,4-dimethoxyphenyl)ethyl)-3-(4-(4-(methylsulfonamido)phenyl)-1H-pyrrolo[2,3-b]pyridin-3-yl)acrylamide |
| **227** | (R,E)-N-(1-(3,4-dimethoxyphenyl)ethyl)-3-(4-(3-(N,N-dimethylsulfamoyl)phenyl)-1H-pyrrolo[2,3-b]pyridin-3-yl)acrylamide |
| **228** | (R,E)-N-(1-(3,4-dimethoxyphenyl)ethyl)-3-(4-(3-(dimethylamino)phenyl)-1H-pyrrolo[2,3-b]pyridin-3-yl)acrylamide |
| **229** | (R,E)-N-(1-(3,4-dimethoxyphenyl)ethyl)-3-(4-(4-fluoro-2-methoxyphenyl)-1H-pyrrolo[2,3-b]pyridin-3-yl)acrylamide |
| **230** | (R,E)-N-(1-(3,4-dimethoxyphenyl)ethyl)-3-(4-(4-(2-(dimethylamino)ethoxy)phenyl)-1H-pyrrolo[2,3-b]pyridin-3-yl)acrylamide |
| **231** | (R,E)-N-(1-(3,4-dimethoxyphenyl)ethyl)-3-(4-(3-(methylsulfonamido)phenyl)-1H-pyrrolo[2,3-b]pyridin-3-yl)acrylamide |
| **232** | (R,E)-N-(1-(3,4-dimethoxyphenyl)ethyl)-3-(4-(4-(morpholinomethyl)phenyl)-1H-pyrrolo[2,3-b]pyridin-3-yl)acrylamide |
| **233** | (R,E)-3-(3-(3-((1-(3,4-dimethoxyphenyl)ethyl)amino)-3-oxoprop-1-en-1-yl)-1H-pyrrolo[2,3-b]pyridin-4-yl)benzamide |
| **234** | (R, E)-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamide |
| **235** | (R,E)-3-(4-(4-(4-acetylpiperazin-1-yl)phenyl)-1H-pyrrolo[2,3-b]pyridin-3-yl)-N-(1-(3,4-dimethoxyphenyl)ethyl)acrylamide |
| **236** | (R,E)-4-(3-(3-((1-(3,4-dimethoxyphenyl)ethyl)amino)-3-oxoprop-1-en-1-yl)-1H-pyrrolo[2,3-b]pyridin-4-yl)benzamide |
| **237** | (R,E)-N-(1-(3,4-dimethoxyphenyl)ethyl)-3-(4-(1,5-dimethyl-1H-pyrazol-4-yl)-1H-pyrrolo[2,3-b]pyridin-3-yl)acrylamide |
| **238** | (R,E)-N-(1-(3,4-dimethoxyphenyl)ethyl)-3-(4-(1-methyl-1H-pyrazol-4-yl)-1H-pyrrolo[2,3-b]pyridin-3-yl)acrylamide |
| **239** | (E)-N-((R)-1-(3,4-dimethoxyphenyl)ethyl)-3-(4-(3-(ethylsulfinyl)phenyl)-1H-pyrrolo[2,3-b]pyridin-3-yl)acrylamide |
| **240** | (R, E)-N-(1-(3,4-dimethoxyphenyl)ethyl)-3-(4-(4-(1-methylpiperidin-4-yl)phenyl)-1H-pyrrolo[2,3-b]pyridin-3-yl)acrylamide |
| **241** | (R,E)-N-(1-(3,4-dimethoxyphenyl)ethyl)-3-(4-(4-(3-(dimethylamino)propoxy)phenyl)-1H-pyrrolo[2,3-b]pyridin-3-yl)acrylamide |
| **242** | (R,E)-N-(1-(3,4-dimethoxyphenyl)ethyl)-3-(4-(pyrazolo[1,5-a]pyridin-3-yl)-1H-pyrrolo[2,3-b]pyridin-3-yl)acrylamide |
| **243** | (R,E)-3-(4-(3-(acetamidomethyl)phenyl)-1H-pyrrolo[2,3-b]pyridin-3-yl)-N-(1-(3,4-dimethoxyphenyl)ethyl)acrylamide |
| **244** | (R,E)-2-cyano-N-(1-(3,4-dimethoxyphenyl)ethyl)-3-(4-methyl-5-(4-(4-methylpiperazin-1-yl)phenyl)-1H-pyrrolo[2,3-b]pyridin-3-yl)acrylamide |
| **245** | (R,E)-2-cyano-N-(1-(3,4-dimethoxyphenyl)ethyl)-3-(5-(4-fluoro-3-(methylsulfonyl)phenyl)-4-methyl-1H-pyrrolo[2,3-b]pyridin-3-yl)acrylamide |
| **246** | (R,E)-N-(1-(benzo[d][1,3]dioxol-5-yl)ethyl)-3-(5-(1-methyl-1H-pyrazol-4-yl)-1H-pyrrolo[2,3-b]pyridin-3-yl)acrylamide |
| **247** | (R,E)-3-(5-(3-chloro-5-(methylsulfonyl)phenyl)-1H-pyrrolo[2,3-b]pyridin-3-yl)-N-(1-(3,4-dimethoxyphenyl)ethyl)acrylamide |
| **248** | (R,E)-N-(1-(3,4-dimethoxyphenyl)ethyl)-3-(5-(2-methyl-5-(methylsulfonyl)phenyl)-1H-pyrrolo[2,3-b]pyridin-3-yl)acrylamide |
| **249** | (R,E)-N-(1-(3,4-dimethoxyphenyl)ethyl)-3-(5-(3-fluoro-5-(methylsulfonyl)phenyl)-1H-pyrrolo[2,3-b]pyridin-3-yl)acrylamide |
| **250** | (R,E)-N-(1-(3,4-dimethoxyphenyl)ethyl)-3-(5-(2-(methylsulfonyl)pyridin-4-yl)-1H-pyrrolo[2,3-b]pyridin-3-yl)acrylamide |
| **251** | (E)-N-((R)-1-(3,4-dimethoxyphenyl)ethyl)-3-(5-(3-(S-methylsulfonimidoyl)phenyl)-1H-pyrrolo[2,3-b]pyridin-3-yl)acrylamide |
| **252** | (R,E)-N-(1-(benzo[d][1,3]dioxol-5-yl)ethyl)-2-cyano-3-(5-(1-methyl-1H-pyrazol-4-yl)-1H-pyrrolo[2,3-b]pyridin-3-yl)acrylamide |
| **253** | (E)-N-(1-(3-cyclopropyl-4-methoxyphenyl)ethyl)-3-(5-(1-methyl-1H-pyrazol-4-yl)-1H-pyrrolo[2,3-b]pyridin-3-yl)acrylamide |
| **254** | (R,E)-N-(1-(3,4-dimethoxyphenyl)ethyl)-3-(5-(3-(methylthio)phenyl)-1H-pyrrolo[2,3-b]pyridin-3-yl)acrylamide |
| **255** | (E)-N-(1-(3-methoxy-4-(methylsulfinyl)phenyl)ethyl)-3-(5-(1-methyl-1H-pyrazol-4-yl)-1H-pyrrolo[2,3-b]pyridin-3-yl)acrylamide |
| **256** | (E)-2-cyano-N-(1-(3-methoxy-4-(methylsulfinyl)phenyl)ethyl)-3-(5-(1-methyl-1H-pyrazol-4-yl)-1H-pyrrolo[2,3-b]pyridin-3-yl)acrylamide |
| **257** | (R,E)-N-(1-(3,4-difluorophenyl)ethyl)-3-(5-(1-methyl-1H-pyrazol-4-yl)-1H-pyrrolo[2,3-b]pyridin-3-yl)acrylamide |
| **258** | (R,E)-2-cyano-N-(1-(3,4-difluorophenyl)ethyl)-3-(5-(1-methyl-1H-pyrazol-4-yl)-1H-pyrrolo[2,3-b]pyridin-3-yl)acrylamide |
| **259** | (R,E)-N-(1-(3,4-dimethoxyphenyl)ethyl)-3-(5-(4-(methylsulfonyl)pyridin-2-yl)-1H-pyrrolo[2,3-b]pyridin-3-yl)acrylamide |
| **260** | (R,E)-3-(5-(1-methyl-1H-pyrazol-4-yl)-1H-pyrrolo[2,3-b]pyridin-3-yl)-N-(1-(3-methylbenzo[d]isoxazol-5-yl)ethyl)acrylamide |
| **261** | (R,E)-2-cyano-N-(1-(1,3-dimethyl-1H-indazol-6-yl)ethyl)-3-(5-(1-methyl-1H-pyrazol-4-yl)-1H-pyrrolo[2,3-b]pyridin-3-yl)acrylamide |
| **262** | (R,E)-2-cyano-N-(1-(1,3-dimethyl-1H-indazol-5-yl)ethyl)-3-(5-(1-methyl-1H-pyrazol-4-yl)-1H-pyrrolo[2,3-b]pyridin-3-yl)acrylamide |
| **263** | (E)-N-((R)-1-(3,4-dimethoxyphenyl)ethyl)-3-(5-(3-((trifluoromethyl)sulfinyl)phenyl)-1H-pyrrolo[2,3-b]pyridin-3-yl)acrylamide |
| **264** | (R,E)-N-(1-(3,4-dimethoxyphenyl)ethyl)-3-(5-(3-((trifluoromethyl)sulfonyl)phenyl)-1H-pyrrolo[2,3-b]pyridin-3-yl)acrylamide |
| **265** | (E)-N-(1-(5,6-dimethoxypyridin-3-yl)ethyl)-3-(5-(1-methyl-1H-pyrazol-4-yl)-1H-pyrrolo[2,3-b]pyridin-3-yl)acrylamide |
or a pharmaceutically acceptable salt thereof.

8. A pharmaceutical composition comprising at least one compound according to claims 1 - 7 as an active ingredient, together with at least one pharmaceutically acceptable carrier, excipient and/or diluent.

9. A compound according to claims 1 - 7 for use as a medicament.

10. A compound according to claims 1 - 7, or a pharmaceutical composition according to claim 8, for use in prophylaxis and/or treatment of a disease caused by or associated with G protein-coupled receptor kinases 5 (GRK5) selected from heart disease, inflammatory and immunological disease, metabolic disease and cancer.

11. The compound for use or the pharmaceutical composition for use according to claim 10, wherein the heart disease is selected from: cardiac hypertrophy, adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome.

12. The compound for use or the pharmaceutical composition for use according to claim 10, wherein the inflammatory and immunological disease is selected from: inflammatory disease associated with an autoimmune disease, a central nervous system (CNS) inflammatory disease, a joint inflammation disease, an inflammatory digestive tract disease, and inflammatory skin

13. The compound for use or the pharmaceutical composition for use according to claim 10, wherein the metabolic disease is selected from diabetes, obesity and impaired adipogenesis.

14. The compound for use or the pharmaceutical composition for use according to claim 10, wherein the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, nonsmall cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma, tongue cancer, astrocytomas, bronchial cancer, laryngeal cancer, malignant melanoma, oesophageal cancer, and renal cell cancer.

15. A method for producing the compound of the formula **(la)** according to claim 1 comprising:
**Step 1:** providing 1H-pyrrolo[2,3-b]pyridine-3-carbaldehyde compound **(2*)** 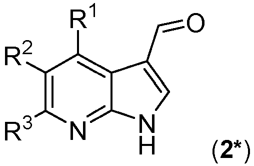
**Step 2:** performing a condensation reaction of compound **(2*)** with a compound **(3*)** 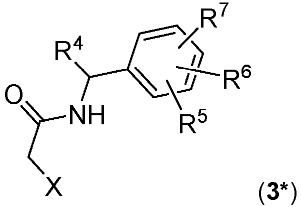
to obtain the compound of the formula **(la)** 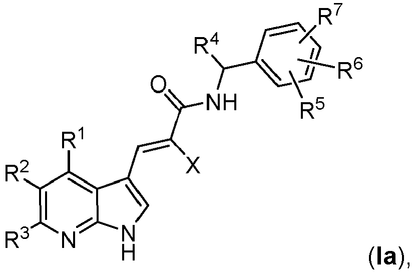
wherein **R¹, R², R³, R⁴, R⁵, R⁶, R⁷** and **X** have the same meanings as defined in claim 1;
**or**
**Step1:** providing 1H-pyrrolo[2,3-b]pyridine-3-carbaldehyde compound **(2*)** 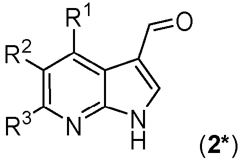
**Step2a:** performing a condensation reaction of compound **(2*)** with maleic acid or cyano acetic acid to obtain acrylic acid compound **(4*)** 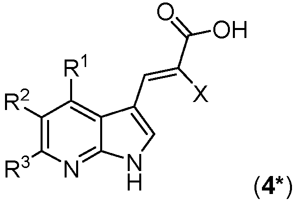
**Step 3:** performing a coupling reaction of compound **(4*)** with an amine compound **(5*)** 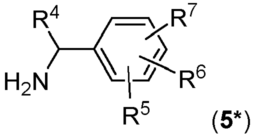
to obtain the compound of the formula **(la)** 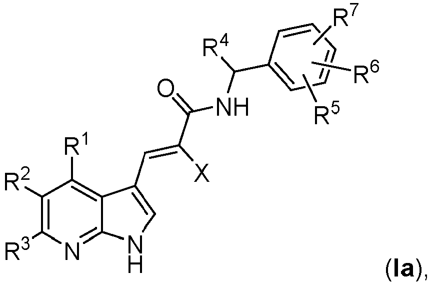
wherein **R¹, R², R³, R⁴, R⁵, R⁶, R⁷** and **X** have the same meanings as defined in claim 1.

## Patentansprüche

1. Verbindung der allgemeinen Formel (I) 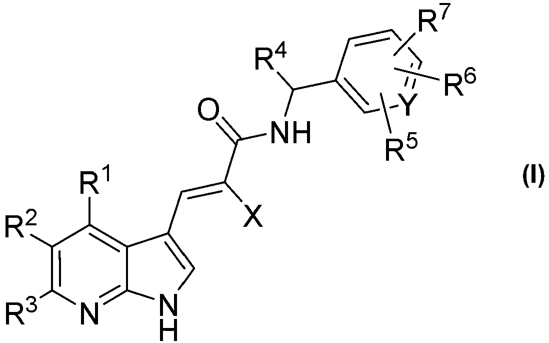 wobei
**X** -H oder -CN darstellt,
**Y** CH oder N darstellt,
**R¹** - **R²** unabhängig voneinander -H, -Ph, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, cyclo-C₃H₅, cyclo-C₄H₇, cyclo-C₅H₉, cyclo-C₆H₁₁, -F, -Cl, -Br, -CN, -OH, -NH-Ph, -**R⁹**, -O-**R¹⁰**, 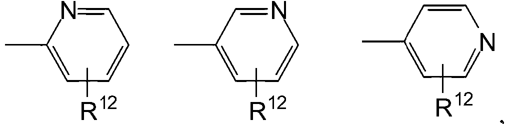 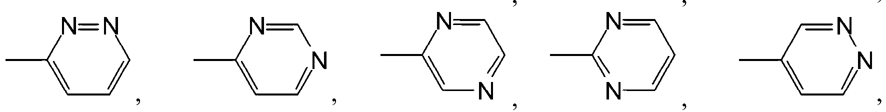 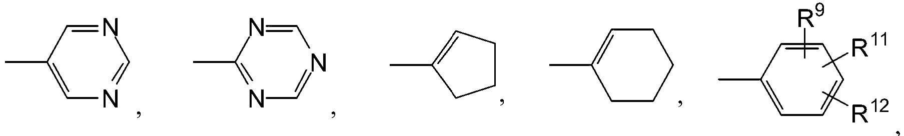 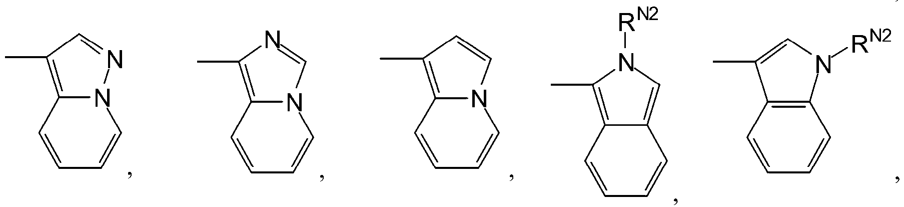 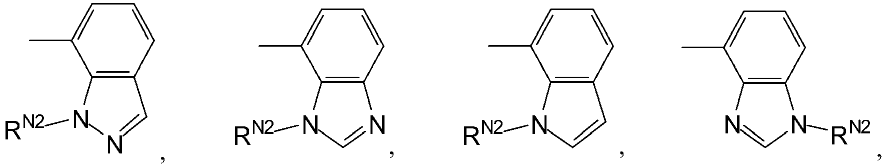 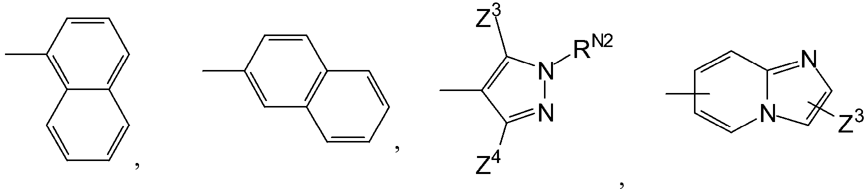 darstellen;
**R³** -H, -F, -CI oder -Br darstellt;
**R⁴** -H, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH₂-OH, -C₂H₄-OH, -C₃H₆-OH, -CN, -CH₂-NH₂, -CH₂-NHCH₃, -CH₂-NHC₂H₅, -CH₂-NHC₃H₇, -CH₂-N(CH₃)₂, -CH₂-N(C₂H₅)₂, -CH₂-N(C₃H₇)₂, -C₂H₄-NH₂, -C₂H₄-NHCH₃, -C₂H₄-NHC₂H₅, -C₂H₄-NHC₃H₇, -C₂H₄-N(CH₃)₂, -C₂H₄-N(C₂H₅)₂, -C₂H₄-N(C₃H₇)₂, -C₃H₆-NH₂, -C₃H₆-NHCH₃, -C₃H₆-NHC₂H₅, -C₃H₆-NHC₃H₇, -C₃H₆-N(CH₃)₂, -C₃H₆-N(C₂H₅)₂, -C₃H₆-N(C₃H₇)₂ darstellt;
**R⁵** - **R⁷** unabhängig voneinander -**R⁸**, -CH₂-**R⁸**, -SO₂-**R⁸**, 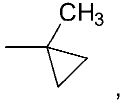 cyclo-C₃H₅, cyclo-C₄H₇, cyclo-C₅H₉, cyclo-C₆H₁₁, cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -CI, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -CH₂-SO₂NH₂, -CH₂-SO₂NHCH₃, -CH₂-SO₂NHC₂H₅, -CH₂-SO₂NHC₃H₇, -CH₂-SO₂NH-cyclo-C₃H₅, -CH₂-SO₂NHCH(CH₃)₂, -CH₂-SO₂NHC(CH₃)₃, -CH₂-SO₂N(CH₃)₂, -CH₂-SO₂N(C₂H₅)₂, -CH₂-SO₂N(C₃H₇)₂, -CH₂-SO₂N(cyclo-C₃H₅)₂, -CH₂-SO₂N[CH(CH₃)₂]₂, -CH₂-SO₂N[C(CH₃)₃]₂, -O-S(=O)CH₃, -O-S(=O)C₂H₅**,** -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃Hz, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C=CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃ oder -C≡C-C≡C-C₂H₅ darstellen,
oder
**R⁵** und **R⁶** oder **R⁶** und **R⁷** gemeinsam 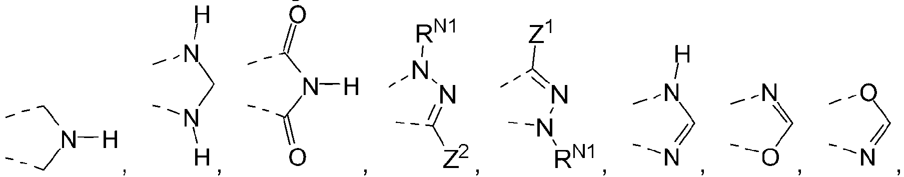 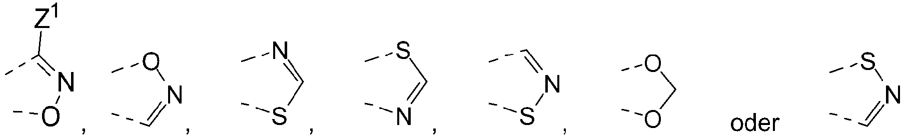
bilden; **R⁸** 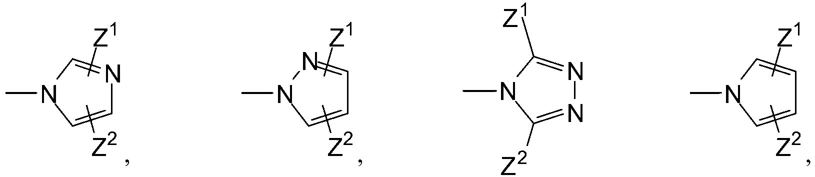 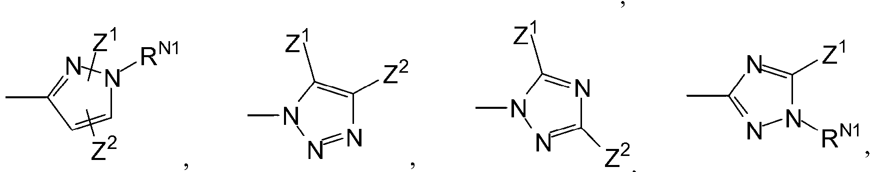 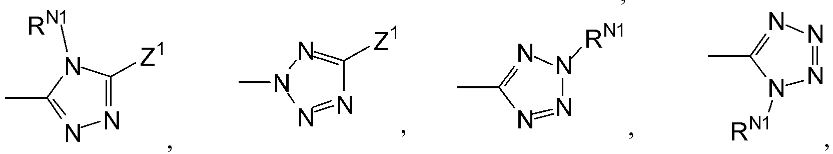 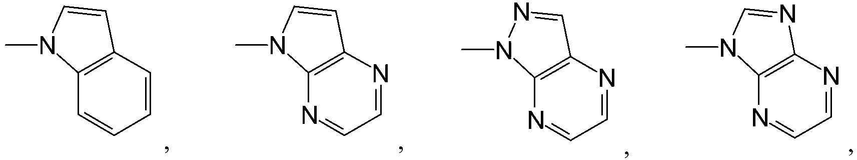 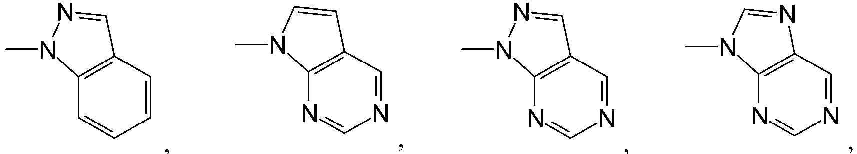 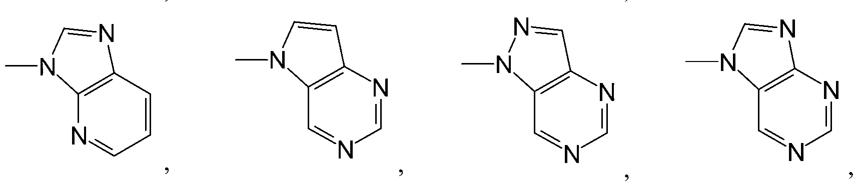 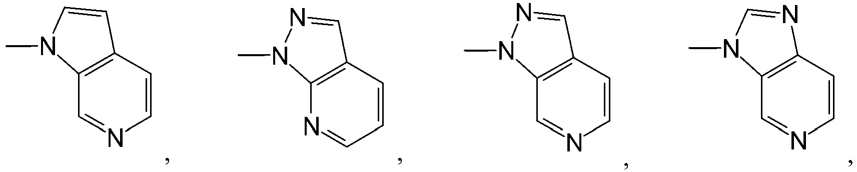 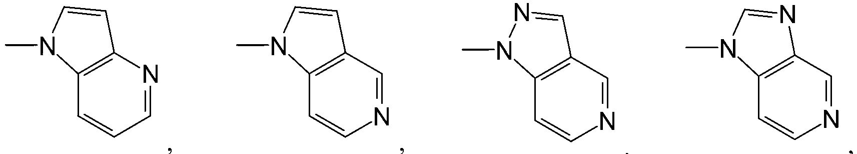 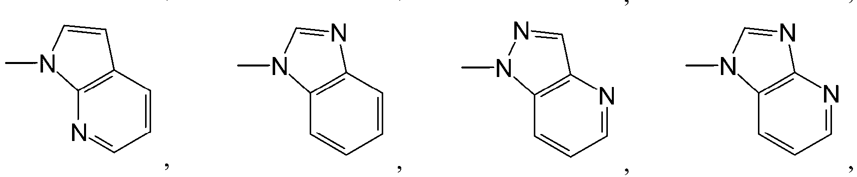 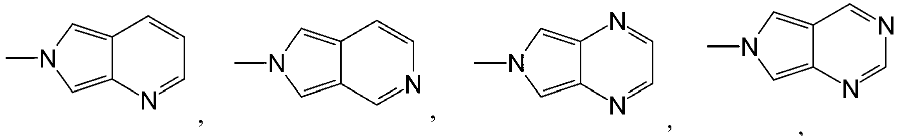 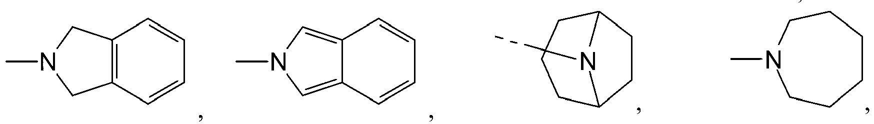 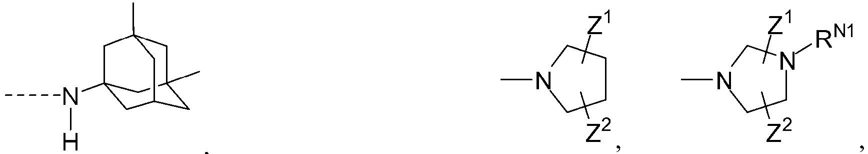 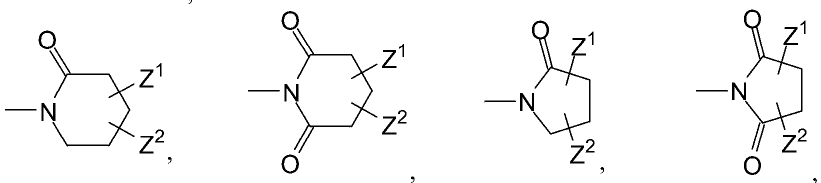 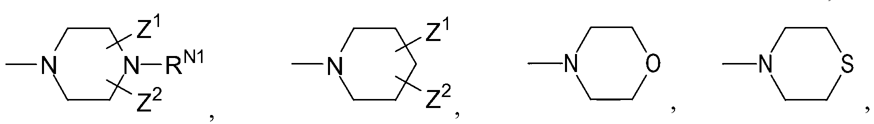 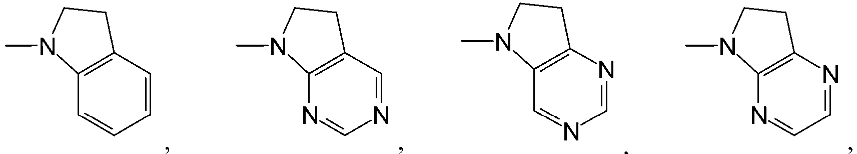 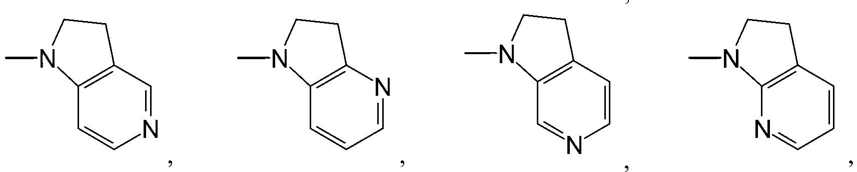 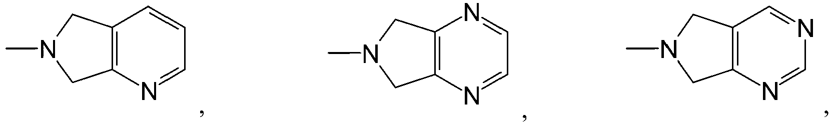 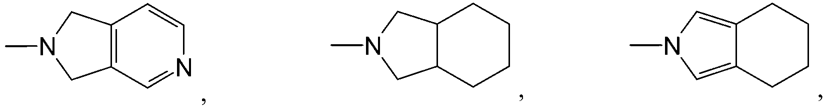 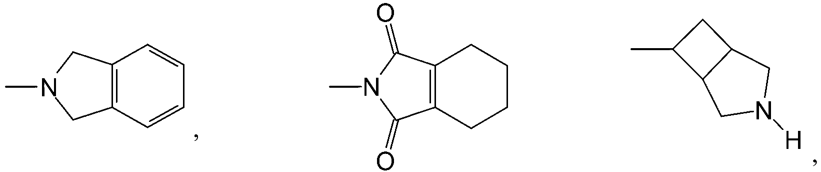 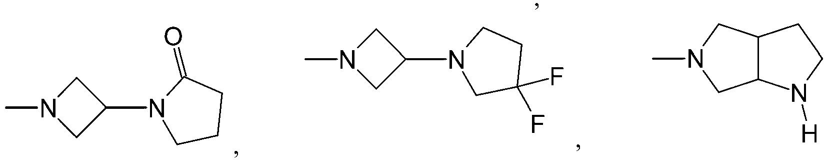 darstellt;
**R⁹** -H, 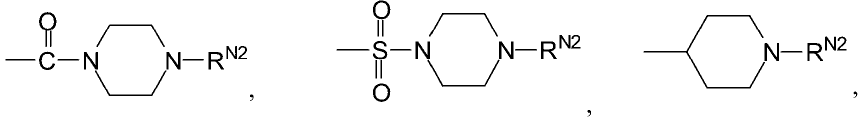 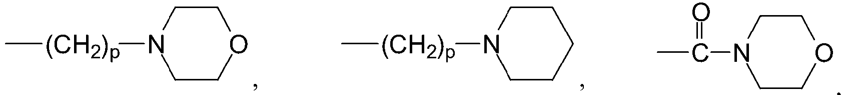 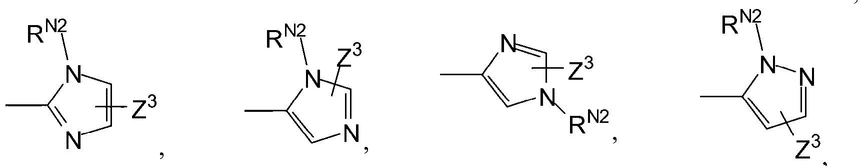 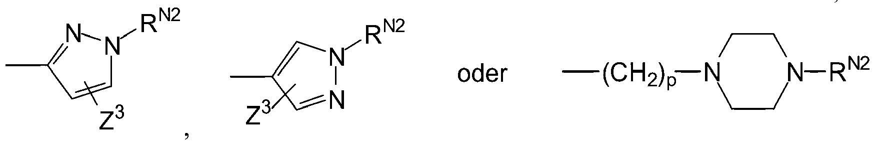 darstellt;
**R¹⁰** -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -(CH2)ₙ-NH2, -(CH₂)ₙ-NHCH₃, -(CH₂)ₙ-NHC₂H₅, -(CH₂)ₙ-NHC₃H₇, -(CH₂)ₙ-N(CH₃)₂, -(CH₂)ₙ-N(C₂H₅)₂, -(CH₂)ₙ-N(C₃H₇)₂, 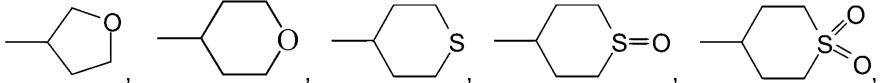 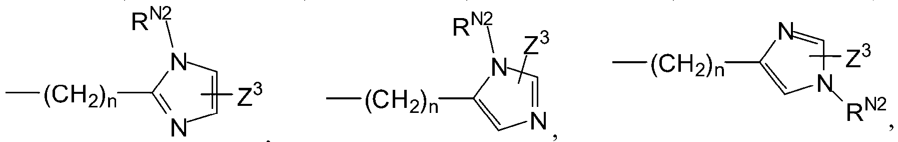 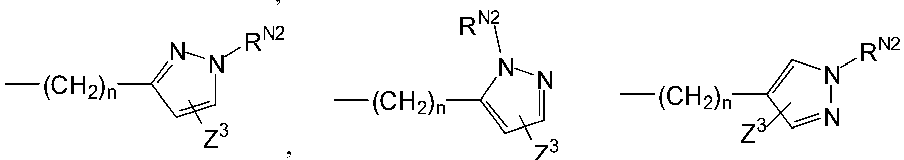 darstellt,
**R¹¹** und **R¹²** unabhängig voneinander -H, -CH₃, -C₂H₅, -C₃H₇, -CH(CH₃)₂, -CF₃, -(CH₂)ₘ-NH₂, -(CH₂)ₘ-NHCH₃, -(CH₂)ₘ-NHC₂H₅, -(CH₂)ₘ-NHC₃H₇, -(CH₂)ₘ-N(CH₃)₂, -(CH₂)ₘ-N(C₂H₅)₂, -(CH₂)ₘ-N(C₃H₇)₂, -OCH₃, -OC₂H₅, -OC₃H₇, -O-cyclo-C₃H₅, -OCH(CH₃)₂, -F, -Cl, -Br, -CN, -(CH₂)ₘ-SO₂CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)m-NH₂, -O-(CH₂)m-NHCH₃, -O-(CH₂)m-NHC₂H₅, -O-(CH₂)m-NHC₃H₇, -O-(CH₂)m-N(CH₃)₂, -O-(CH₂)m-N(C₂H₅)₂, -O-(CH₂)m-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃ oder -SO(NH)CH₃ darstellen;
**R^{N1}** und **R^{N2}** unabhängig voneinander -H, -CH₃, -C₂H₅, -C₃H₇, -CH(CH₃)₂, cyclo-C₃H₅, cyclo-C₄H₇, cyclo-C₅H₉, cyclo-C₆H₁₁, -C₄H₉, -C(CH₃)₃, -CH₂-CH(CH₃)₂, -CH(CH₃)-C₂H₅, -C₅H₁₁, -Ph, -CH₂-Ph, -CH=CH₂, -CH₂-CH=CH₂, -CH=CH-CH₃, -C=CH, -CH₂-C≡CH, -C≡C-CH₃, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 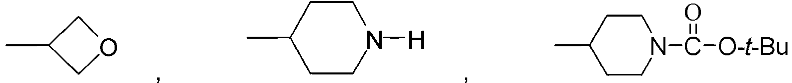 oder 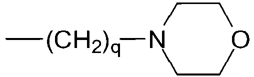 darstellen;
**Z¹** - **Z⁴** unabhängig voneinander -H, -CH₃, -C2H5, -C₃H₇, -CH(CH₃)₂, -Ph, -COCH₃, -COC₂H₅, -COOH, -COOCH₃, -COOC₂H₅, -NH₂, -NHCH₃, -NHC₂H₅, -N(CH₃)₂, -N(C₂H₅)₂, -CH₂-NH₂, -SO₃H, -F, -Cl, -Br, -I, -CN, -CF₃, -OCF₃, -OH, -OCH₃ oder -OC₂H₅ darstellen;
m eine ganze Zahl ausgewählt aus 0, 1, 2, 3 oder 4 ist;
n eine ganze Zahl ausgewählt aus 1, 2, 3 oder 4 ist;
p eine ganze Zahl ausgewählt aus 0, 1, 2, 3 oder 4 ist;
q eine ganze Zahl ausgewählt aus 1, 2, 3 oder 4 ist;
mit dem Vorbehalt, dass, wenn **X** -H ist, mindestens einer von **R¹** - **R³** nicht -H ist; und wenn **R⁴** -H ist, mindestens einer von **R¹** - **R³** und **R⁵** - **R⁷** nicht -H ist;
und die folgende Verbindung vom Umfang der vorliegenden Anmeldung ausgeschlossen ist: 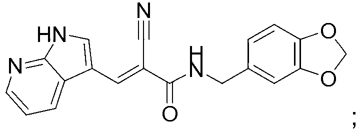
oder ein Enantiomer, eine stereoisomere Form, eine Mischung von Enantiomeren, ein Diastereomer, eine Mischung von Diastereomeren, ein Hydrat, ein Solvat, eine Säuresalzform, ein Tautomer oder ein Racemat der oben genannten Verbindung oder ein pharmazeutisch verträgliches Salz davon.

2. Die Verbindung gemäß Anspruch 1, wobei
**R¹** und **R²** unabhängig voneinander -H, -Ph, -CH₃, -CF₃, -Cl, -Br, -CN, -OH, -NH-Ph, -**R⁹**, -O-**R¹⁰**, 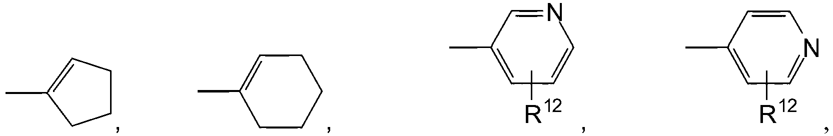 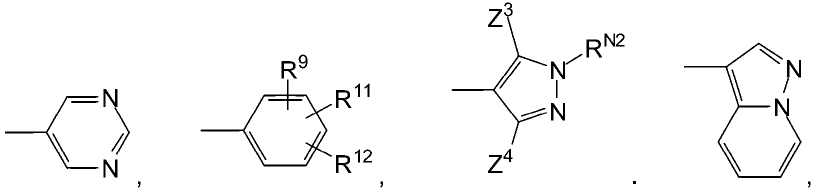 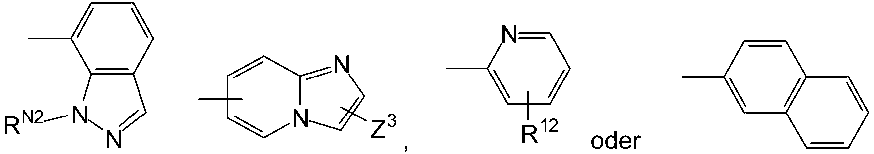 darstellen;
wobei **R⁹, R¹⁰**, **R¹¹**, **R¹², R^{N2}, Z³, Z⁴** die in Anspruch 1 definierten Bedeutungen haben.

3. Die Verbindung gemäß Anspruch 1 oder 2, wobei
**R¹** -H, -CH₃, -Cl, 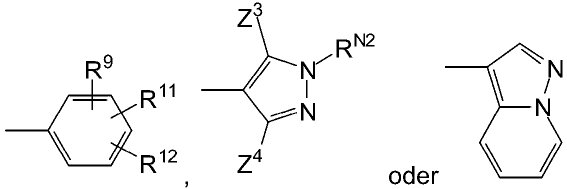 darstellt;
**R²** -H, -Ph, -CH₃, -CF₃, -CI, -Br, -CN, -OH, -NH-Ph, -**R⁹**, -O-**R¹⁰**, 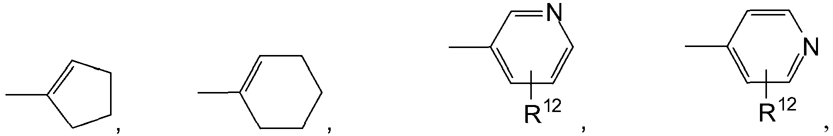 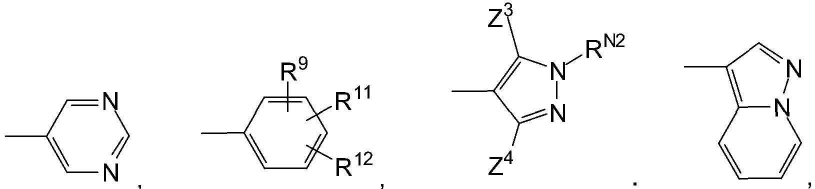 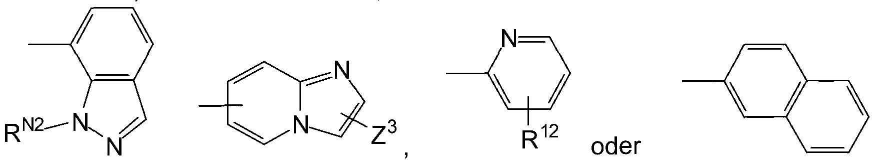 darstellt;
**R⁹** -H, 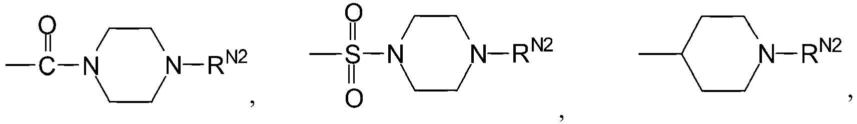 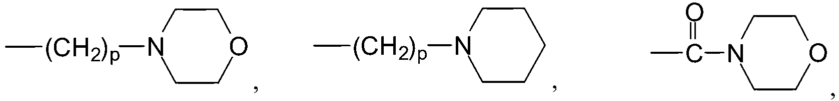 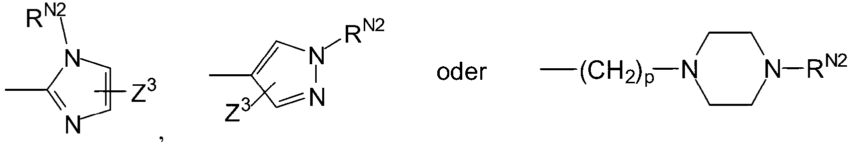 darstellt;
**R¹⁰** -CH(CH₃)₂, -CH₂-CH(CH₃)₂, -CH(CH₃)-C₂H₅, -(CH₂)ₙ-N(CH₃)₂, 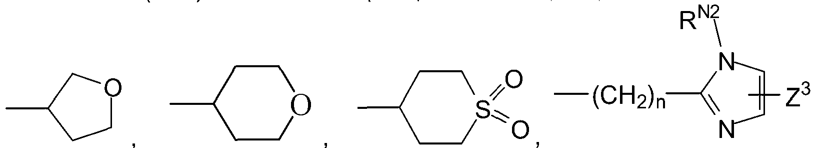 oder 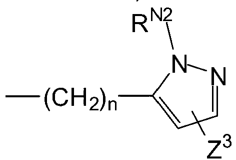 darstellt;
wobei **n, p, R¹¹, R¹², R^{N2}, Z³, Z⁴** die in Anspruch 1 definierten Bedeutungen haben.

4. Die Verbindung gemäß einem der Ansprüche 1 - 3, wobei die Verbindung eine der Formeln **(la), (Ib)** und (**II-1**) - (**II-6**) hat 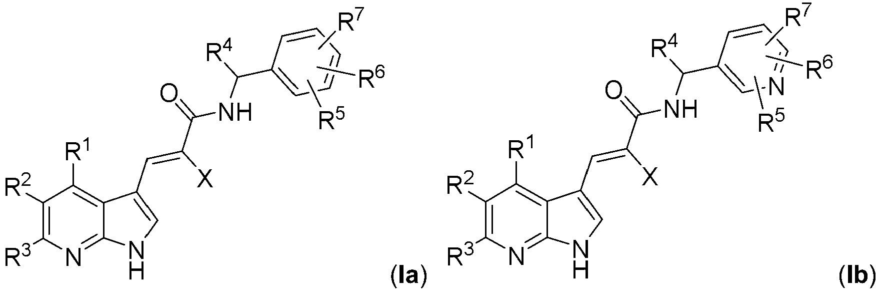 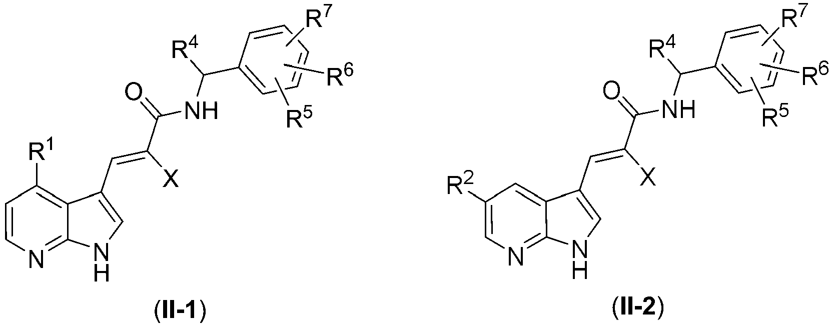 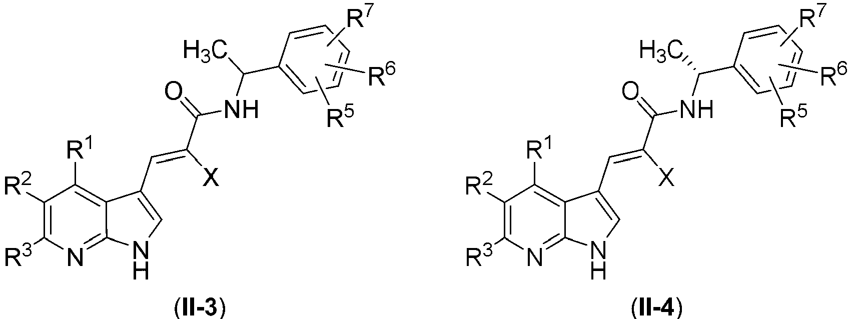 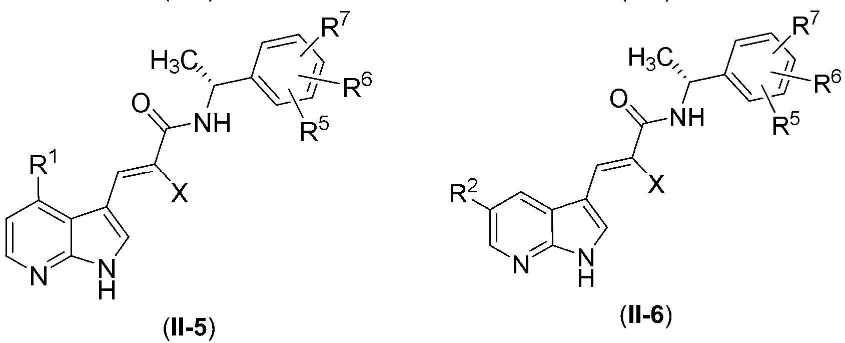 wobei **R¹, R², R³, R⁴, R⁵, R⁶, R⁷** und **X** die in Anspruch 1 definierten Bedeutungen haben.

5. Die Verbindung gemäß einem der Ansprüche 1 - 4, wobei die Verbindung eine der Formeln (**III-1**) - (**III-6**) hat 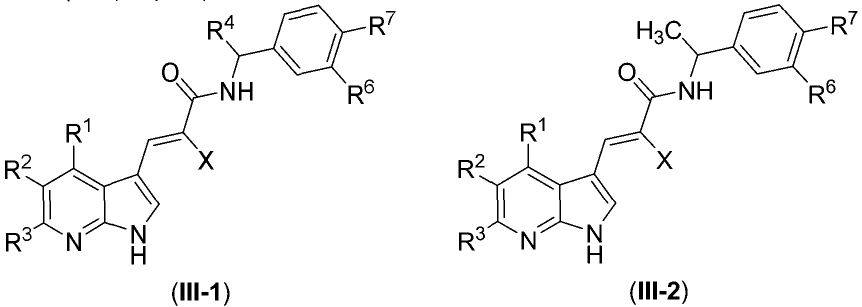 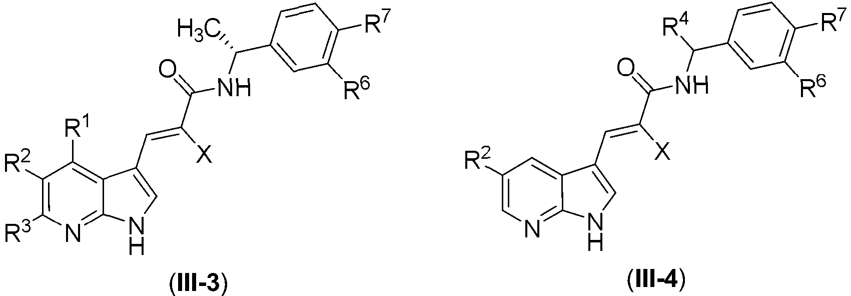 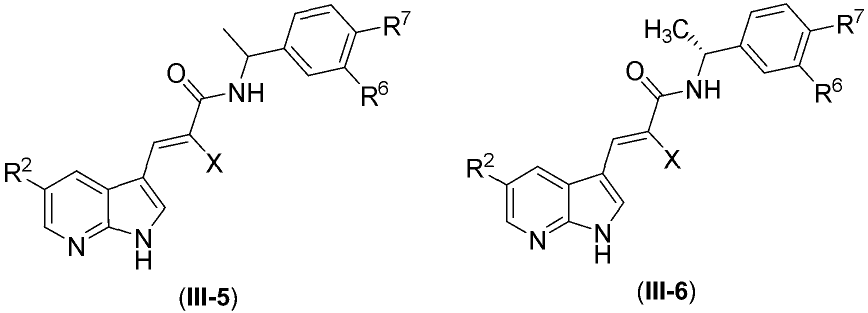 wobei **R¹, R², R³, R⁴, R⁶, R⁷** und **X** die in Anspruch 1 definierten Bedeutungen haben.

6. Die Verbindung gemäß einem der Ansprüche 1 - 5, wobei die Verbindung eine der Formeln (IV-1) - (IV-6) oder (V-1) - (V-6) hat 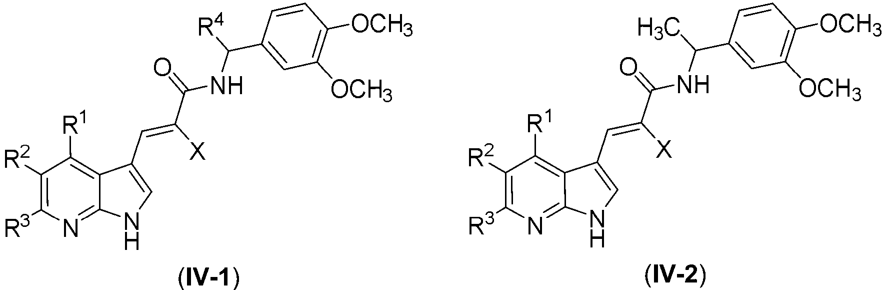 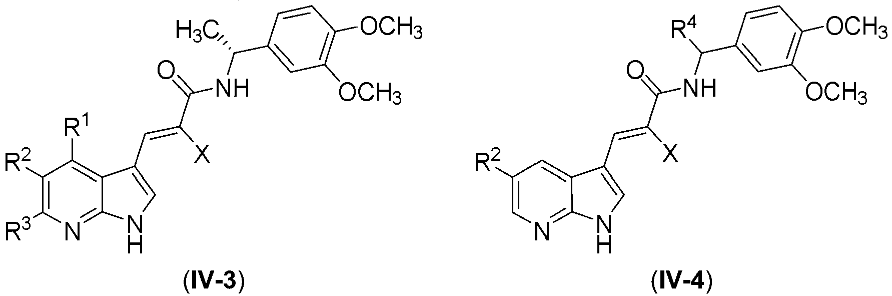 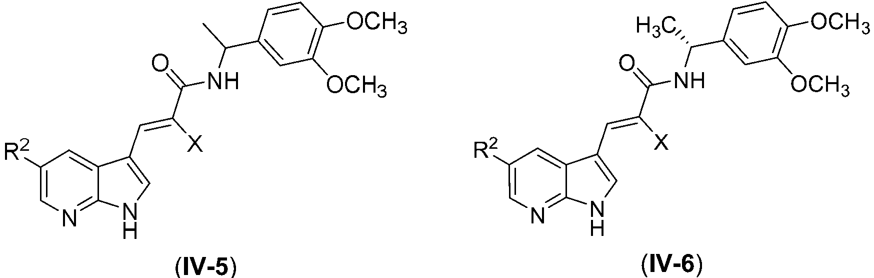 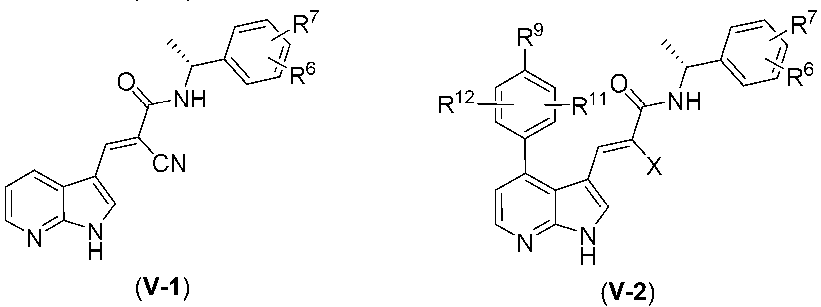 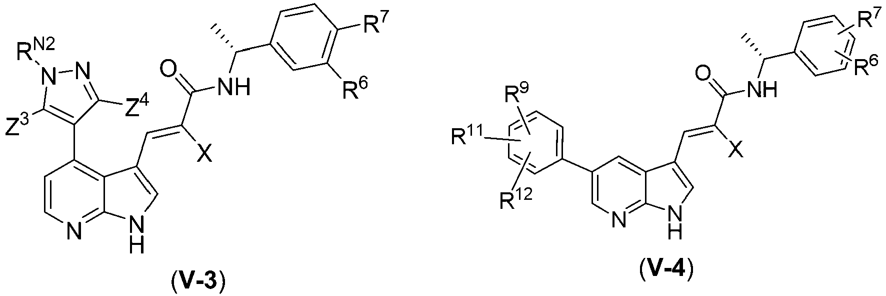 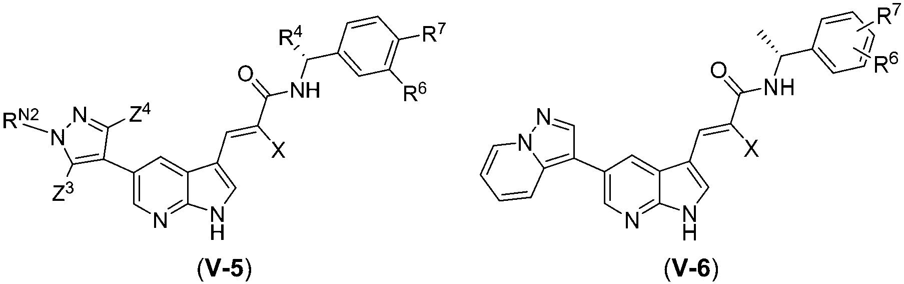 wobei **R¹**, **R², R³, R⁴, R⁶, R⁷**, **R⁹, R¹¹, R¹², R^{N2}, X**, **Z³** und **Z⁴** die in Anspruch 1 definierten Bedeutungen haben.

7. Eine Verbindung ausgewählt aus der Gruppe bestehend aus:
| **Verbindung** | **Nomenklatur** |
|---|---|
| **1** | (*R,E*)-2-Cyano-*N*-(1-(3-fluorphenyl)ethyl)-3-(1*H*-pyrrolo[2,3-b]pyridin-3-yl)acrylamid |
| **6** | (E)-N-Benzyl-3-(5-chlor-1H-pyrrolo[2,3-b]pyridin-3-yl)acrylamid |
| **7** | (E)-3-(5-Chlor-1H-pyrrolo[2,3-b]pyridin-3-yl)-N-(3-fluorbenzyl)acrylamid |
| **8** | (E)-3-(4-Chlor-1H-pyrrolo[2,3-b]pyridin-3-yl)-N-(3-fluorbenzyl)acrylamid |
| **10** | (E)-N-(1-(4-Fluorphenyl)ethyl)-3-(1H-pyrrolo[2,3-b]pyridin-3-yl)acrylamid |
| **11** | (R,E)-3-(5-Brom-1H-pyrrolo[2,3-b]pyridin-3-yl)-N-(1-(3,4-dimethoxyphenyl)ethyl)acrylamid |
| **12** | (R,E)-N-(1-(2-Chlorphenyl)ethyl)-2-cyano-3-(1H-pyrrolo[2,3-b]pyridin-3-yl)acrylamid |
| **13** | (R,E)-N-(1-(3-Chlorphenyl)ethyl)-2-cyano-3-(1H-pyrrolo[2,3-b]pyridin-3-yl)acrylamid |
| **14** | (S,E)-2-Cyano-3-(1H-pyrrolo[2,3-b]pyridin-3-yl)-N-(2,2,2-trifluor-1-phenylethyl)acrylamid |
| **15** | (R,E)-2-Cyano-N-(1-(3,4-dimethoxyphenyl)ethyl)-3-(1H-pyrrolo[2,3-b]pyridin-3-yl)acrylamid |
| **16** | (R,E)-2-Cyano-N-(1-(3,4-difluorphenyl)ethyl)-3-(1H-pyrrolo[2,3-b]pyridin-3-yl)acrylamid |
| **17** | (R,E)-2-Cyano-N-(1-(2,4-difluorphenyl)ethyl)-3-(1 H-pyrrolo[2,3-b]pyridin-3-yl)acrylamid |
| **18** | (R,E)-2-Cyano-3-(1H-pyrrolo[2,3-b]pyridin-3-yl)-N-(1-(3-(trifluormethyl)phenyl)ethyl)acrylamid |
| **19** | (R,E)-2-Cyano-N-(1-(4-methoxyphenyl)ethyl)-3-(1H-pyrrolo[2,3-b]pyridin-3-yl)acrylamid |
| **20** | (R,E)-2-Cyano-N-(1-(3,4-dichlorphenyl)ethyl)-3-(1H-pyrrolo[2,3-b]pyridin-3-yl)acrylamid |
| **21** | (E)-2-Cyano-3-(1H-pyrrolo[2,3-b]pyridin-3-yl)-N-(4-sulfamoylbenzyl)acrylamid |
| **22** | (E)-2-Cyano-3-(1H-pyrrolo[2,3-b]pyridin-3-yl)-N-(4-sulfamoylbenzyl)acrylamid |
| **23** | (E)-N-(5-Brom-2-methylbenzyl)-2-cyano-3-(1H-pyrrolo[2,3-b]pyridin-3-yl)acrylamid |
| **24** | (E)-2-Cyano-N-(4-(dimethylamino)benzyl)-3-(1H-pyrrolo[2,3-b]pyridin-3-yl)acrylamid |
| **25** | (E)-2-Cyano-N-(2-(dimethylamino)benzyl)-3-(1H-pyrrolo[2,3-b]pyridin-3-yl)acrylamid |
| **26** | (E)-2-Cyano-N-(4-cyanobenzyl)-3-(1H-pyrrolo[2,3-b]pyridin-3-yl)acrylamid |
| **27** | (E)-2-Cyano-3-(1H-pyrrolo[2,3-b]pyridin-3-yl)-N-(3-(sulfamoylmethyl)benzyl)-acrylamid |
| **28** | (E)-3-((2-Cyano-3-(1H-pyrrolo[2,3-b]pyridin-3-yl)acrylamido)methyl)benzamid |
| **29** | (E)-2-Cyano-N-(3-(methylsulfonamido)benzyl)-3-(1H-pyrrolo[2,3-b]pyridin-3-yl)acrylamid |
| **30** | (E)-2-Cyano-N-(3-(dimethylamino)benzyl)-3-(1H-pyrrolo[2,3-b]pyridin-3-yl)acrylamid |
| **31** | (E)-2-Cyano-N-(3-cyanobenzyl)-3-(1H-pyrrolo[2,3-b]pyridin-3-yl)acrylamid |
| **32** | (E)-2-Cyano-N-(2-hydroxy-1-phenylethyl)-3-(1H-pyrrolo[2,3-b]pyridin-3-yl)acrylamid |
| **33** | (R,E)-2-Cyano-N-(1-(3-methoxyphenyl)ethyl)-3-(1H-pyrrolo[2,3-b]pyridin-3-yl)acrylamid |
| **34** | (E)-N-(3-Acetamidobenzyl)-2-cyano-3-(1H-pyrrolo[2,3-b]pyridin-3-yl)acrylamid |
| **35** | (E)-2-Cyano-N-(2-cyanobenzyl)-3-(1H-pyrrolo[2,3-b]pyridin-3-yl)acrylamid |
| **36** | (E)-2-Cyano-N-(4-methoxy-3-sulfamoylbenzyl)-3-(1H-pyrrolo[2,3-b]pyridin-3-yl)acrylamid |
| **37** | (E)-2-Cyano-N-(2-(morpholinomethyl)benzyl)-3-(1H-pyrrolo[2,3-b]pyridin-3-yl)acrylamid |
| **38** | (E)-N-(1-(2-(1H-Pyrazol-1-yl)phenyl)ethyl)-2-cyano-3-(1H-pyrrolo[2,3-b]pyridin-3-yl)acrylamid |
| **39** | (E)-2-Cyano-N-(2-(methylsulfonyl)benzyl)-3-(1H-pyrrolo[2,3-b]pyridin-3-yl)acrylamid |
| **40** | (R,E)-2-Cyano-N-(3-hydroxy-1-phenylpropyl)-3-(1H-pyrrolo[2,3-b]pyridin-3-yl)acrylamid |
| **41** | (E)-2-Cyano-N-(2-(morpholinosulfonyl)benzyl)-3-(1H-pyrrolo[2,3-b]pyridin-3-yl)acrylamid |
| **42** | (E)-2-Cyano-N-(2-(methylsulfonamido)benzyl)-3-(1H-pyrrolo[2,3-b]pyridin-3-yl)acrylamid |
| **43** | (E)-2-Cyano-N-(2-(N-methylsulfamoyl)benzyl)-3-(1H-pyrrolo[2,3-b]pyridin-3-yl)acrylamid |
| **44** | (E)-2-Cyano-N-(2-((2-oxopyrrolidin-1-yl)methyl)benzyl)-3-(1H-pyrrolo[2,3-b]pyridin-3-yl)acrylamid |
| **45** | (E)-N-((1H-Indazol-4-yl)methyl)-2-cyano-3-(1H-pyrrolo[2,3-b]pyridin-3-yl)acrylamid |
| **46** | (E)-2-Cyano-N-(3-morpholinobenzyl)-3-(1H-pyrrolo[2,3-b]pyridin-3-yl)acrylamid |
| **47** | (E)-N-(1-(3-Bromphenyl)-2,2,2-trifluorethyl)-2-cyano-3-(1H-pyrrolo[2,3-b]pyridin-3-yl)acrylamid |
| **48** | (E)-2-Cyano-N-(3-(dimethylamino)-1-phenylpropyl)-3-(1H-pyrrolo[2,3-b]pyridin-3-yl)acrylamid |
| **49** | (E)-2-Cyano-N-(cyano(phenyl)methyl)-3-(1H-pyrrolo[2,3-b]pyridin-3-yl)acrylamid |
| **50** | (E)-2-cCyano-N-(2-(dimethylamino)-1-phenylethyl)-3-(1H-pyrrolo[2,3-b]pyridin-3-yl)acrylamid |
| **51** | (E)-2-Cyano-N-(3-(dimethylamino)-1-(4-methoxyphenyl)propyl)-3-(1H-pyrrolo[2,3-b]pyridin-3-yl)acrylamid |
| **52** | (E)-2-Cyano-N-(3-(diethylamino)-1-phenylpropyl)-3-(1H-pyrrolo[2,3-b]pyridin-3-yl)acrylamid |
| **53** | (S,E)-N-(1-(3,4-Dimethoxyphenyl)-2-hydroxyethyl)-3-(5-methyl-1H-pyrrolo[2,3-b]pyridin-3-yl)acrylamid |
| **54** | (R,E)-N-(1-(4-Fluor-3-methoxyphenyl)ethyl)-3-(5-methyl-1H-pyrrolo[2,3-b]pyridin-3-yl)acrylamid |
| **55** | (R,E)-N-(1-(3,4-Dimethoxyphenyl)ethyl)-3-(5-((1,1-dioxidotetrahydro-2H-thiopyran-4-yl)oxy)-1H-pyrrolo[2,3-b]pyridin-3-yl)acrylamid |
| **56** | (R,E)-N-(1-(3,4-Dimethoxyphenyl)ethyl)-3-(5-(2-(dimethylamino)ethoxy)-1H-pyrrolo[2,3-b]pyridin-3-yl)acrylamid |
| **57** | (E)-2-Cyano-N-(4-fluorbenzyl)-3-(1H-pyrrolo[2,3-b]pyridin-3-yl)acrylamid |
| **58** | (E)-N-(4-Chlorbenzyl)-2-cyano-3-(1H-pyrrolo[2,3-b]pyridin-3-yl)acrylamid |
| **59** | (E)-2-Cyano-3-(1H-pyrrolo[2,3-b]pyridin-3-yl)-N-(4-(trifluormethyl)benzyl)acrylamid |
| **60** | (E)-N-(3-Chlorbenzyl)-2-cyano-3-(1H-pyrrolo[2,3-b]pyridin-3-yl)acrylamid |
| **61** | (E)-2-Cyano-3-(1H-pyrrolo[2,3-b]pyridin-3-yl)-N-(3-(trifluormethyl)benzyl)acrylamid |
| **62** | (E)-N-(2-Chlorbenzyl)-2-cyano-3-(1H-pyrrolo[2,3-b]pyridin-3-yl)acrylamid |
| **63** | (E)-2-Cyano-N-(1-(4-fluorphenyl)ethyl)-3-(1H-pyrrolo[2,3-b]pyridin-3-yl)acrylamid |
| **64** | (R,E)-2-Cyano-N-(1-phenylethyl)-3-(1H-pyrrolo[2,3-b]pyridin-3-yl)acrylamid |
| **65** | (E)-2-Cyano-N-(2,4-difluorbenzyl)-3-(1H-pyrrolo[2,3-b]pyridin-3-yl)acrylamide |
| **66** | (E)-2-Cyano-N-(3,4-difluorbenzyl)-3-(1H-pyrrolo[2,3-b]pyridin-3-yl)acrylamid |
| **67** | (E)-2-Cyano-N-(3,5-difluorbenzyl)-3-(1H-pyrrolo[2,3-b]pyridin-3-yl)acrylamid |
| **68** | (R,E)-2-Cyano-N-(1-phenylpropyl)-3-(1H-pyrrolo[2,3-b]pyridin-3-yl)acrylamid |
| **69** | (R,E)-2-Cyano-N-(1-(4-fluorphenyl)ethyl)-3-(1H-pyrrolo[2,3-b]pyridin-3-yl)acrylamid |
| **70** | (R,E)-N-(1-(4-Chlorphenyl)ethyl)-2-cyano-3-(1H-pyrrolo[2,3-b]pyridin-3-yl)acrylamid |
| **71** | (E)-N-Benzyl-2-cyano-3-(5-(trifluormethyl)-1H-pyrrolo[2,3-b]pyridin-3-yl)acrylamid |
| **72** | (E)-3-(5-Chlor-1H-pyrrolo[2,3-b]pyridin-3-yl)-2-cyano-N-(4-fluorbenzyl)acrylamid |
| **73** | (E)-N-Benzyl-3-(5-chlor-1H-pyrrolo[2,3-b]pyridin-3-yl)-2-cyanoacrylamid |
| **74** | (E)-3-(5-Chlor-1H-pyrrolo[2,3-b]pyridin-3-yl)-N-(2-chlorbenzyl)-2-cyanoacrylamid |
| **75** | (E)-3-(5-Chlor-1H-pyrrolo[2,3-b]pyridin-3-yl)-2-cyano-N-(3,5-difluorbenzyl)acrylamid |
| **76** | (R,E)-3-(5-Chlor-1H-pyrrolo[2,3-b]pyridin-3-yl)-2-cyano-N-(1-(4-fluorphenyl)ethyl)acrylamid |
| **77** | (R,E)-3-(5-Chlor-1H-pyrrolo[2,3-b]pyridin-3-yl)-2-cyano-N-(1-phenylpropyl)acrylamid |
| **78** | (R,E)-3-(4-Chlor-1H-pyrrolo[2,3-b]pyridin-3-yl)-2-cyano-N-(1-(4-fluorphenyl)ethyl)acrylamid |
| **79** | (R,E)-3-(5-Brom-1H-pyrrolo[2,3-b]pyridin-3-yl)-2-cyano-N-(1-(4-fluorphenyl)ethyl)acrylamid |
| **80** | (R,E)-3-(5-Brom-1H-pyrrolo[2,3-b]pyridin-3-yl)-2-cyano-N-(1-(3,4-dimethoxyphenyl)ethyl)acrylamid |
| **81** | (R,E)-2-Cyano-N-(1-(3,4-dimethoxyphenyl)ethyl)-3-(5-hydroxy-1H-pyrrolo[2,3-b]pyridin-3-yl)acrylamid |
| **82** | (R,E)-2-Cyano-N-(1-(3,4-dimethoxyphenyl)ethyl)-3-(5-((tetrahydro-2H-pyran-4-yl)oxy)-1H-pyrrolo[2,3-b]pyridin-3-yl)acrylamid |
| **83** | (R,E)-2-Cyano-N-(1-(3,4-dimethoxyphenyl)ethyl)-3-(5-(4-methylpiperazin-1-carbonyl)-1H-pyrrolo[2,3-b]pyridin-3-yl)acrylamid |
| **84** | (R,E)-2-Cyano-N-(1-(3,4-dimethoxyphenyl)ethyl)-3-(5-morpholino-1H-pyrrolo[2,3-b]pyridin-3-yl)acrylamid |
| **85** | (R,E)-2-Cyano-N-(1-(3,4-dimethoxyphenyl)ethyl)-3-(5-morpholino-1H-pyrrolo[2,3-b]pyridin-3-yl)acrylamid |
| **86** | (R,E)-2-Cyano-3-(5-cyano-1H-pyrrolo[2,3-b]pyridin-3-yl)-N-(1-(3,4-dimethoxyphenyl)ethyl)acrylamid |
| **87** | (R,E)-2-Cyano-N-(1-(3,4-dimethoxyphenyl)ethyl)-3-(5-methyl-1H-pyrrolo[2,3-b]pyridin-3-yl)acrylamid |
| **88** | (R,E)-2-Cyano-N-(1-(3,4-dimethoxyphenyl)ethyl)-3-(5-(phenylamino)-1H-pyrrolo[2,3-b]pyridin-3-yl)acrylamid |
| **89** | (R,E)-2-Cyano-N-(1-(3,4-dimethoxyphenyl)ethyl)-3-(5-(1-(piperidin-4-yl)-1H-pyrazol-4-yl)-1H-pyrrolo[2,3-b]pyridin-3-yl)acrylamid |
| **90** | (R,E)-3-(6-Brom-1H-pyrrolo[2,3-b]pyridin-3-yl)-2-cyano-N-(1-(3,4-dimethoxyphenyl)ethyl)acrylamid |
| **91** | (R,E)-2-Cyano-3-(5-(1-methyl-1H-pyrazol-4-yl)-1H-pyrrolo[2,3-b]pyridin-3-yl)-N-(1-phenylethyl)acrylamid |
| **92** | (S,E)-2-Cyano-N-(1-(3,4-dimethoxyphenyl)-2-hydroxyethyl)-3-(5-(1-methyl-1H-pyrazol-4-yl)-1H-pyrrolo[2,3-b]pyridin-3-yl)acrylamid |
| **93** | (R,E)-2-Cyano-N-(1-(4-fluorphenyl)ethyl)-3-(5-(1-methyl-1H-pyrazol-4-yl)-1H-pyrrolo[2,3-b]pyridin-3-yl)acrylamid |
| **94** | (R,E)-2-Cyano-N-(1-(4-fluor-3-methoxyphenyl)ethyl)-3-(5-(1-methyl-1H-pyrazol-4-yl)-1H-pyrrolo[2,3-b]pyridin-3-yl)acrylamid |
| **95** | (R,E)-N-(1-(4-Chlorphenyl)ethyl)-2-cyano-3-(5-(1-methyl-1H-pyrazol-4-yl)-1H-pyrrolo[2,3-b]pyridin-3-yl)acrylamid |
| **96** | (R,E)-N-(1-(3-Chlorphenyl)ethyl)-2-cyano-3-(5-(1-methyl-1H-pyrazol-4-yl)-1H-pyrrolo[2,3-b]pyridin-3-yl)acrylamid |
| **97** | (R,E)-2-Cyano-3-(4-methyl-5-(1-methyl-1 H-pyrazol-4-yl)-1 H-pyrrolo[2,3-b]pyridin-3-yl)-N-(1-phenylethyl)acrylamid |
| **98** | (R,E)-2-Cyano-N-(1-(4-fluorphenyl)ethyl)-3-(4-methyl-5-(1-methyl-1H-pyrazol-4-yl)-1H-pyrrolo[2,3-b]pyridin-3-yl)acrylamid |
| **99** | (R,E)-2-Cyano-N-(1-(3,4-dimethoxyphenyl)ethyl)-3-(4-methyl-5-(1-methyl-1H-pyrazol-4-yl)-1H-pyrrolo[2,3-b]pyridin-3-yl)acrylamid |
| **100** | (R,E)-N-(1-(4-Chlorphenyl)ethyl)-2-cyano-3-(4-methyl-5-(1-methyl-1H-pyrazol-4-yl)-1H-pyrrolo[2,3-b]pyridin-3-yl)acrylamid |
| **101** | (R,E)-2-Cyano-N-(1-(3,4-dimethoxyphenyl)ethyl)-3-(5-((1,3-dimethyl-1H-pyrazol-5-yl)methoxy)-1H-pyrrolo[2,3-b]pyridin-3-yl)acrylamid |
| **102** | (R,E)-2-Cyano-N-(1-(3,4-dimethoxyphenyl)ethyl)-3-(5-isopropoxy-1H-pyrrolo[2,3-b]pyridin-3-yl)acrylamid |
| **103** | (E)-2-Cyano-N-((R)-1-(3,4-dimethoxyphenyl)ethyl)-3-(5-((tetrahydrofuran-3-yl)oxy)-1H-pyrrolo[2,3-b]pyridin-3-yl)acrylamid |
| **104** | (R,E)-2-Cyano-N-(1-(3,4-dimethoxyphenyl)ethyl)-3-(5-(2-(dimethylamino)ethoxy)-1H-pyrrolo[2,3-b]pyridin-3-yl)acrylamid |
| **105** | (R,E)-2-Cyano-N-(1-(3,4-dimethoxyphenyl)ethyl)-3-(5-((1,1-dioxidotetrahydro-2H-thiopyran-4-yl)oxy)-1H-pyrrolo[2,3-b]pyridin-3-yl)acrylamid |
| **106** | (R,E)-2-Cyano-N-(1-(3,4-dimethoxyphenyl)ethyl)-3-(5-((1-methyl-1H-imidazol-2-yl)methoxy)-1H-pyrrolo[2,3-b]pyridin-3-yl)acrylamid |
| **107** | (E)-2-Cyano-N-((R)-1-(3,4-dimethoxyphenyl)ethyl)-3-(5-(((R)-tetrahydrofuran-3-yl)oxy)-1H-pyrrolo[2,3-b]pyridin-3-yl)acrylamid |
| **108** | (E)-2-Cyano-N-((R)-1-(3,4-dimethoxyphenyl)ethyl)-3-(5-(((S)-tetrahydrofuran-3-yl)oxy)-1H-pyrrolo[2,3-b]pyridin-3-yl)acrylamide |
| **109** | (E)-3-(5-((S)-sec-Butoxy)-1H-pyrrolo[2,3-b]pyridin-3-yl)-2-cyano-N-((R)-1-(3,4-dimethoxyphenyl)ethyl)acrylamid |
| **110** | (E)-3-(5-((S)-sec-Butoxy)-1H-pyrrolo[2,3-b]pyridin-3-yl)-2-cyano-N-((R)-1-(3,4-dimethoxyphenyl)ethyl)acrylamid |
| **111** | (R,E)-2-Cyano-N-(1-(3,4-dimethoxyphenyl)ethyl)-3-(5-((tetrahydro-2H-pyran-4-yl)oxy)-1H-pyrrolo[2,3-b]pyridin-3-yl)acrylamid |
| **112** | (R,E)-2-Cyano-N-(1-(3,4-dimethoxyphenyl)ethyl)-3-(5-(4-(4-methylpiperazin-1-yl)phenyl)-1H-pyrrolo[2,3-b]pyridin-3-yl)acrylamid |
| **113** | (R,E)-N-(1-(3,4-Dimethoxyphenyl)ethyl)-3-(5-(4-(4-methylpiperazin-1-yl)phenyl)-1H-pyrrolo[2,3-b]pyridin-3-yl)acrylamid |
| **114** | (R,E)-N-(1-(3,4-Dimethoxyphenyl)ethyl)-3-(5-(3-(methylsulfonyl)phenyl)-1H-pyrrolo[2,3-b]pyridin-3-yl)acrylamid |
| **115** | (R,E)-3-(5-(3,5-Difluorphenyl)-1H-pyrrolo[2,3-b]pyridin-3-yl)-N-(1-(3,4-dimethoxyphenyl)ethyl)acrylamid |
| **116** | (R,E)-2-Cyano-N-(1-(3,4-dimethoxyphenyl)ethyl)-3-(5-(4-(4-methylpiperazin-1-yl)phenyl)-1H-pyrrolo[2,3-b]pyridin-3-yl)acrylamid |
| **117** | (R,E)-2-Cyano-N-(1-(3,4-dimethoxyphenyl)ethyl)-3-(5-(4-((dimethylamino)methyl)phenyl)-1H-pyrrolo[2,3-b]pyridin-3-yl)acrylamid |
| **118** | (R,E)-2-Cyano-N-(1-(3,4-dimethoxyphenyl)ethyl)-3-(5-(3-((4-methylpiperazin-1-yl)methyl)phenyl)-1H-pyrrolo[2,3-b]pyridin-3-yl)acrylamid |
| **119** | (R,E)-2-Cyano-N-(1-(3,4-dimethoxyphenyl)ethyl)-3-(5-(3-((4-methylpiperazin-1-yl)methyl)phenyl)-1H-pyrrolo[2,3-b]pyridin-3-yl)acrylamid |
| **120** | (R,E)-2-Cyano-N-(1-(3,4-dimethoxyphenyl)ethyl)-3-(5-(pyrimidin-5-yl)-1H-pyrrolo[2,3-b]pyridin-3-yl)acrylamid |
| **121** | (R,E)-2-Cyano-N-(1-(3,4-dimethoxyphenyl)ethyl)-3-(5-(4-((4-ethylpiperazin-1-yl)sulfonyl)phenyl)-1H-pyrrolo[2,3-b]pyridin-3-yl)acrylamid |
| **122** | (R,E)-2-Cyano-N-(1-(3,4-dimethoxyphenyl)ethyl)-3-(5-(3-(4-methylpiperazin-1-yl)phenyl)-1H-pyrrolo[2,3-b]pyridin-3-yl)acrylamid |
| **123** | (R,E)-2-Cyano-N-(1-(3,4-dimethoxyphenyl)ethyl)-3-(5-(3-(4-methylpiperazin-1-yl)phenyl)-1H-pyrrolo[2,3-b]pyridin-3-yl)acrylamid |
| **124** | (R,E)-2-Cyano-N-(1-(3,4-dimethoxyphenyl)ethyl)-3-(5-(4-fluor-2-methoxyphenyl)-1H-pyrrolo[2,3-b]pyridin-3-yl)acrylamid |
| **125** | (R,E)-2-Cyano-N-(1-(3,4-dimethoxyphenyl)ethyl)-3-(5-(2-methoxyphenyl)-1H-pyrrolo[2,3-b]pyridin-3-yl)acrylamid |
| **126** | (R,E)-4-(3-(2-Cyano-3-((1-(3,4-dimethoxyphenyl)ethyl)amino)-3-oxoprop-1-en-1-yl)-1H-pyrrolo[2,3-b]pyridin-5-yl)benzamid |
| **127** | (R,E)-2-Cyano-N-(1-(3,4-dimethoxyphenyl)ethyl)-3-(5-(3-(morpholine-4-carbonyl)phenyl)-1H-pyrrolo[2,3-b]pyridin-3-yl)acrylamid |
| **128** | (R,E)-2-Cyano-N-(1-(3,4-dimethoxyphenyl)ethyl)-3-(5-(3-(dimethylamino)phenyl)-1H-pyrrolo[2,3-b]pyridin-3-yl)acrylamid |
| **129** | (R,E)-2-Cyano-N-(1-(3,4-dimethoxyphenyl)ethyl)-3-(5-(2-(piperidin-1-ylmethyl)phenyl)-1H-pyrrolo[2,3-b]pyridin-3-yl)acrylamid |
| **130** | (R,E)-2-Cyano-N-(1-(3,4-dimethoxyphenyl)ethyl)-3-(5-(2-(piperidin-1-ylmethyl)phenyl)-1H-pyrrolo[2,3-b]pyridin-3-yl)acrylamid |
| **131** | (R,E)-2-Cyano-N-(1-(3,4-dimethoxyphenyl)ethyl)-3-(5-(pyridin-3-yl)-1H-pyrrolo[2,3-b]pyridin-3-yl)acrylamid |
| **132** | (R,E)-2-Cyano-N-(1-(3,4-dimethoxyphenyl)ethyl)-3-(5-(4-(1-methylpiperidin-4-yl)phenyl)-1H-pyrrolo[2,3-b]pyridin-3-yl)acrylamid |
| **133** | (R,E)-2-Cyano-N-(1-(3,4-dimethoxyphenyl)ethyl)-3-(5-(4-(1-methylpiperidin-4-yl)phenyl)-1H-pyrrolo[2,3-b]pyridin-3-yl)acrylamid |
| **134** | (R,E)-2-Cyano-N-(1-(3,4-dimethoxyphenyl)ethyl)-3-(5-(3-(3-(dimethylamino)propoxy)phenyl)-1H-pyrrolo[2,3-b]pyridin-3-yl)acrylamid |
| **135** | (R,E)-2-Cyano-N-(1-(3,4-dimethoxyphenyl)ethyl)-3-(5-(4-(2-(dimethylamino)ethyl)phenyl)-1H-pyrrolo[2,3-b]pyridin-3-yl)acrylamid |
| **136** | (R,Z)-2-Cyano-N-(1-(3,4-dimethoxyphenyl)ethyl)-3-(5-(4-(2-(dimethylamino)ethyl)phenyl)-1H-pyrrolo[2,3-b]pyridin-3-yl)acrylamid |
| **137** | (R,E)-2-Cyano-N-(1-(3,4-dimethoxyphenyl)ethyl)-3-(5-(3-methoxyphenyl)-1H-pyrrolo[2,3-b]pyridin-3-yl)acrylamid |
| **138** | (R,E)-2-Cyano-N-(1-(3,4-dimethoxyphenyl)ethyl)-3-(5-(3-methoxyphenyl)-1H-pyrrolo[2,3-b]pyridin-3-yl)acrylamid |
| **139** | (R,E)-2-Cyano-N-(1-(3,4-dimethoxyphenyl)ethyl)-3-(5-(2-(morpholinomethyl)phenyl)-1H-pyrrolo[2,3-b]pyridin-3-yl)acrylamid |
| **140** | (R,Z)-2-Cyano-3-(5-(4-cyanophenyl)-1 H-pyrrolo[2,3-b]pyridin-3-yl)-N-(1-(3,4-dimethoxyphenyl)ethyl)acrylamide |
| **141** | (R,E)-3-(5-(3-((4-Acetylpiperazin-1-yl)methyl)phenyl)-1H-pyrrolo[2,3-b]pyridin-3-yl)-2-cyano-N-(1-(3,4-dimethoxyphenyl)ethyl)acrylamid |
| **142** | (R,E)-3-(5-(3-((4-Acetylpiperazin-1-yl)methyl)phenyl)-1H-pyrrolo[2,3-b]pyridin-3-yl)-2-cyano-N-(1-(3,4-dimethoxyphenyl)ethyl)acrylamid |
| **143** | (R,E)-3-(5-(4-Acetamidophenyl)-1H-pyrrolo[2,3-b]pyridin-3-yl)-2-cyano-N-(1-(3,4-dimethoxyphenyl)ethyl)acrylamid |
| **144** | (R,E)-2-Cyano-N-(1-(3,4-dimethoxyphenyl)ethyl)-3-(5-(pyridin-4-yl)-1H-pyrrolo[2,3-b]pyridin-3-yl)acrylamid |
| **145** | (R,E)-2-Cyano-N-(1-(3,4-dimethoxyphenyl)ethyl)-3-(5-(naphthalen-2-yl)-1H-pyrrolo[2,3-b]pyridin-3-yl)acrylamid |
| **146** | (R,Z)-2-Cyano-3-(5-(3,4-dimethoxyphenyl)-1H-pyrrolo[2,3-b]pyridin-3-yl)-N-(1-(3,4-dimethoxyphenyl)ethyl)acrylamid |
| **147** | (R,E)-2-Cyano-N-(1-(3,4-dimethoxyphenyl)ethyl)-3-(5-(6-(dimethylamino)pyridin-3-yl)-1H-pyrrolo[2,3-b]pyridin-3-yl)acrylamid |
| **148** | (R,E)-2-Cyano-N-(1-(3,4-dimethoxyphenyl)ethyl)-3-(5-(6-(dimethylamino)pyridin-3-yl)-1H-pyrrolo[2,3-b]pyridin-3-yl)acrylamid |
| **149** | (R, E)-2-Cyano-N-(1 -(3,4-dimethoxyphenyl)ethyl)-3-(5-(3-((dimethylamino)methyl)phenyl)-1H-pyrrolo[2,3-b]pyridin-3-yl)acrylamid |
| **150** | (R,E)-2-Cyano-N-(1-(3,4-dimethoxyphenyl)ethyl)-3-(5-(3-(N,N-dimethylsulfamoyl)phenyl)-1H-pyrrolo[2,3-b]pyridin-3-yl)acrylamid |
| **151** | (R,E)-2-Cyano-N-(1-(3,4-dimethoxyphenyl)ethyl)-3-(5-(3-(methylsulfonamido)phenyl)-1H-pyrrolo[2,3-b]pyridin-3-yl)acrylamid |
| **152** | (R,E)-2-Cyano-N-(1-(3,4-dimethoxyphenyl)ethyl)-3-(5-(3-(methylsulfonamido)phenyl)-1H-pyrrolo[2,3-b]pyridin-3-yl)acrylamid |
| **153** | (R,E)-2-Cyano-N-(1-(3,4-dimethoxyphenyl)ethyl)-3-(5-(3-(2-(dimethylamino)ethoxy)phenyl)-1H-pyrrolo[2,3-b]pyridin-3-yl)acrylamid |
| **154** | (R,E)-2-Cyano-N-(1-(3,4-dimethoxyphenyl)ethyl)-3-(5-(2-methoxy-4-(4-methylpiperazin-1-yl)phenyl)-1H-pyrrolo[2,3-b]pyridin-3-yl)acrylamid |
| **155** | (R,E)-2-Cyano-N-(1-(3,4-dimethoxyphenyl)ethyl)-3-(5-(4-(2-(dimethylamino)ethoxy)phenyl)-1H-pyrrolo[2,3-b]pyridin-3-yl)acrylamid |
| **156** | (R,E)-2-Cyano-3-(5-(cyclohex-1-en-1-yl)-1H-pyrrolo[2,3-b]pyridin-3-yl)-N-(1-(3,4-dimethoxyphenyl)ethyl)acrylamid |
| **157** | (R,E)-2-Cyano-3-(5-(cyclohex-1-en-1-yl)-1H-pyrrolo[2,3-b]pyridin-3-yl)-N-(1-(3,4-dimethoxyphenyl)ethyl)acrylamid |
| **158** | (R,E)-2-Cyano-3-(5-(cyclopent-1-en-1-yl)-1H-pyrrolo[2,3-b]pyridin-3-yl)-N-(1-(3,4-dimethoxyphenyl)ethyl)acrylamid |
| **159** | (R,E)-2-Cyano-3-(5-(cyclopent-1-en-1-yl)-1H-pyrrolo[2,3-b]pyridin-3-yl)-N-(1-(3,4-dimethoxyphenyl)ethyl)acrylamid |
| **160** | (R,E)-3-(5-(1-Benzyl-1H-pyrazol-4-yl)-1H-pyrrolo[2,3-b]pyridin-3-yl)-2-cyano-N-(1-(3,4-dimethoxyphenyl)ethyl)acrylamid |
| **161** | (R,E)-2-Cyano-N-(1-(3,4-dimethoxyphenyl)ethyl)-3-(5-(1-isobutyl-1H-pyrazol-4-yl)-1H-pyrrolo[2,3-b]pyridin-3-yl)acrylamid |
| **162** | (R,E)-2-Cyano-N-(1-(3,4-dimethoxyphenyl)ethyl)-3-(5-(1-isobutyl-1H-pyrazol-4-yl)-1H-pyrrolo[2,3-b]pyridin-3-yl)acrylamid |
| **163** | (R,E)-2-Cyano-N-(1-(3,4-dimethoxyphenyl)ethyl)-3-(5-(2-methylimidazo[1,2-a]pyridin-6-yl)-1H-pyrrolo[2,3-b]pyridin-3-yl)acrylamid |
| **164** | (R,E)-3-(5-(1H-Pyrazol-4-yl)-1H-pyrrolo[2,3-b]pyridin-3-yl)-2-cyano-N-(1-(3,4-dimethoxyphenyl)ethyl)acrylamid |
| **165** | (R,E)-2-Cyano-N-(1-(3,4-dimethoxyphenyl)ethyl)-3-(5-(1-methyl-1H-pyrazol-4-yl)-1H-pyrrolo[2,3-b]pyridin-3-yl)acrylamid |
| **166** | (R,E)-2-Cyano-N-(1-(3,4-dimethoxyphenyl)ethyl)-3-(5-(1-methyl-1H-imidazol-4-yl)-1H-pyrrolo[2,3-b]pyridin-3-yl)acrylamid |
| **167** | (R,E)-2-Cyano-N-(1-(3,4-dimethoxyphenyl)ethyl)-3-(5-(1-methyl-1H-imidazol-4-yl)-1H-pyrrolo[2,3-b]pyridin-3-yl)acrylamid |
| **168** | (R,E)-2-Cyano-N-(1-(3,4-dimethoxyphenyl)ethyl)-3-(5-(1,3-dimethyl-1H-pyrazol-4-yl)-1H-pyrrolo[2,3-b]pyridin-3-yl)acrylamid |
| **169** | (R,E)-2-Cyano-N-(1-(3,4-dimethoxyphenyl)ethyl)-3-(5-(1,3-dimethyl-1H-pyrazol-4-yl)-1H-pyrrolo[2,3-b]pyridin-3-yl)acrylamid |
| **170** | (R,E)-2-Cyano-N-(1-(3,4-dimethoxyphenyl)ethyl)-3-(5-(4-fluor-3-(methylsulfonyl)phenyl)-1H-pyrrolo[2, 3-b]pyridin-3-yl)acrylamid |
| **171** | (R,E)-2-Cyano-N-(1-(3,4-dimethoxyphenyl)ethyl)-3-(4-(4-(4-methylpiperazin-1-yl)phenyl)-1H-pyrrolo[2,3-b]pyridin-3-yl)acrylamid |
| **172** | (R,E)-2-Cyano-N-(1-(3,4-dimethoxyphenyl)ethyl)-3-(4-(3-(4-methylpiperazin-1-yl)phenyl)-1H-pyrrolo[2,3-b]pyridin-3-yl)acrylamid |
| **173** | (R,E)-2-Cyano-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamid |
| **174** | (R,E)-2-Cyano-N-(1-(3,4-dimethoxyphenyl)ethyl)-3-(4-(4-((4-methylpiperazin-1-yl)methyl)phenyl)-1H-pyrrolo[2,3-b]pyridin-3-yl)acrylamid |
| **175** | (R,E)-2-Cyano-N-(1-(3,4-dimethoxyphenyl)ethyl)-3-(4-(3-((4-methylpiperazin-1-yl)methyl)phenyl)-1H-pyrrolo[2,3-b]pyridin-3-yl)acrylamid |
| **176** | (R,E)-2-Cyano-N-(1-(3,4-dimethoxyphenyl)ethyl)-3-(4-(3-((dimethylamino)methyl)phenyl)-1H-pyrrolo[2,3-b]pyridin-3-yl)acrylamid |
| **177** | (R,E)-2-Cyano-N-(1-(3,4-dimethoxyphenyl)ethyl)-3-(4-(3-((dimethylamino)methyl)phenyl)-1H-pyrrolo[2,3-b]pyridin-3-yl)acrylamid |
| **178** | (R,E)-2-Cyano-N-(1-(3,4-dimethoxyphenyl)ethyl)-3-(4-(4-((dimethylamino)methyl)phenyl)-1H-pyrrolo[2,3-b]pyridin-3-yl)acrylamid |
| **179** | (R,E)-2-Cyano-3-(4-(3,5-difluorphenyl)-1H-pyrrolo[2,3-b]pyridin-3-yl)-N-(1-(3,4-dimethoxyphenyl)ethyl)acrylamid |
| **180** | (R,E)-2-Cyano-N-(1-(4-fluorphenyl)ethyl)-3-(5-phenyl-1H-pyrrolo[2,3-b]pyridin-3-yl)acrylamid |
| **181** | (R,E)-2-Cyano-N-(1-(4-fluorphenyl)ethyl)-3-(5-(3-(4-methylpiperazin-1-yl)phenyl)-1H-pyrrolo[2,3-b]pyridin-3-yl)acrylamid |
| **182** | (R,E)-2-Cyano-N-(1-(4-fluorphenyl)ethyl)-3-(5-(3-(4-methylpiperazin-1-yl)phenyl)-1H-pyrrolo[2,3-b]pyridin-3-yl)acrylamid |
| **183** | (R,E)-3-(5-(4-(4-Acetylpiperazin-1-yl)phenyl)-1H-pyrrolo[2,3-b]pyridin-3-yl)-N-(1-(3,4-dimethoxyphenyl)ethyl)acrylamid |
| **184** | (R, E)-N-(1-(3,4-Dimethoxyphenyl)ethyl)-3-(5-(3-((4-methylpiperazin-1-yl)methyl)phenyl)-1H-pyrrolo[2,3-b]pyridin-3-yl)acrylamid |
| **185** | (R,E)-N-(1-(3,4-Dimethoxyphenyl)ethyl)-3-(5-(4-((dimethylamino)methyl)phenyl)-1H-pyrrolo[2,3-b]pyridin-3-yl)acrylamid |
| **186** | (R,E)-N-(1-(3,4-Dimethoxyphenyl)ethyl)-3-(5-(pyridin-3-yl)-1H-pyrrolo[2,3-b]pyridin-3-yl)acrylamid |
| **187** | (R,E)-N-(1-(3,4-Dimethoxyphenyl)ethyl)-3-(5-(pyridin-3-yl)-1H-pyrrolo[2,3-b]pyridin-3-yl)acrylamid |
| **188** | (R,E)-N-(1-(3,4-Dimethoxyphenyl)ethyl)-3-(5-(4-(methylsulfonamido)phenyl)-1H-pyrrolo[2,3-b]pyridin-3-yl)acrylamid |
| **189** | (R,E)-N-(1-(3,4-Dimethoxyphenyl)ethyl)-3-(5-(3-(3-(dimethylamino)propoxy)phenyl)-1H-pyrrolo[2,3-b]pyridin-3-yl)acrylamid |
| **190** | (R,E)-3-(3-(3-((1-(3,4-Dimethoxyphenyl)ethyl)amino)-3-oxoprop-1-en-1-yl)-1H-pyrrolo[2,3-b]pyridin-5-yl)benzamid |
| **191** | (E)-N-((R)-1-(3,4-Dimethoxyphenyl)ethyl)-3-(5-(3-(ethylsulfinyl)phenyl)-1H-pyrrolo[2,3-b]pyridin-3-yl)acrylamid |
| **192** | (E)-N-((R)-1-(3,4-Dimethoxyphenyl)ethyl)-3-(5-(3-(ethylsulfinyl)phenyl)-1H-pyrrolo[2,3-b]pyridin-3-yl)acrylamid |
| **193** | (R,E)-N-(1-(3,4-Dimethoxyphenyl)ethyl)-3-(5-(3-(2-(dimethylamino)ethyl)phenyl)-1H-pyrrolo[2,3-b]pyridin-3-yl)acrylamid |
| **194** | (R,E)-N-(1-(3,4-Dimethoxyphenyl)ethyl)-3-(5-(3-(methylsulfonamido)phenyl)-1H-pyrrolo[2,3-b]pyridin-3-yl)acrylamid |
| **195** | (R,E)-N-(1-(3,4-Dimethoxyphenyl)ethyl)-3-(5-(3-(dimethylamino)phenyl)-1H-pyrrolo[2,3-b]pyridin-3-yl)acrylamid |
| **196** | (R,E)-N-(1-(3,4-Dimethoxyphenyl)ethyl)-3-(5-(4-(1-methylpiperidin-4-yl)phenyl)-1H-pyrrolo[2,3-b]pyridin-3-yl)acrylamid |
| **197** | (R,E)-N-(1-(3,4-Dimethoxyphenyl)ethyl)-3-(5-(4-(2-(dimethylamino)ethoxy)phenyl)-1H-pyrrolo[2,3-b]pyridin-3-yl)acrylamid |
| **198** | (R,E)-N-(1-(3,4-Dimethoxyphenyl)ethyl)-3-(5-(3-(N,N-dimethylsulfamoyl)phenyl)-1H-pyrrolo[2,3-b]pyridin-3-yl)acrylamid |
| **199** | (R,E)-3-(5-(3-(1H-Imidazol-2-yl)phenyl)-1H-pyrrolo[2,3-b]pyridin-3-yl)-N-(1-(3,4-dimethoxyphenyl)ethyl)acrylamid |
| **200** | (R,E)-4-(3-(3-((1-(3,4-Dimethoxyphenyl)ethyl)amino)-3-oxoprop-1-en-1-yl)-1H-pyrrolo[2,3-b]pyridin-5-yl)benzamid |
| **201** | (R,E)-N-(1-(3,4-Dimethoxyphenyl)ethyl)-3-(5-(4-fluor-2-methoxyphenyl)-1H-pyrrolo[2,3-b]pyridin-3-yl)acrylamid |
| **202** | (R,E)-N-(1-(3,4-Dimethoxyphenyl)ethyl)-3-(5-(1,5-dimethyl-1H-pyrazol-4-yl)-1H-pyrrolo[2,3-b]pyridin-3-yl)acrylamid |
| **203** | (R,E)-3-(5-(3-(Acetamidomethyl)phenyl)-1H-pyrrolo[2,3-b]pyridin-3-yl)-N-(1-(3,4-dimethoxyphenyl)ethyl)acrylamid |
| **204** | (R,E)-N-(1-(3,4-Dimethoxyphenyl)ethyl)-3-(5-(1-methyl-1H-pyrazol-4-yl)-1H-pyrrolo[2,3-b]pyridin-3-yl)acrylamide |
| **205** | (R,E)-N-(1-(3,4-Dimethoxyphenyl)ethyl)-3-(5-(pyrazolo[1,5-a]pyridin-3-yl)-1H-pyrrolo[2,3-b]pyridin-3-yl)acrylamide |
| **206** | (R,E)-N-(1-(3,4-Dimethoxyphenyl)ethyl)-3-(5-(2-(morpholinomethyl)phenyl)-1H-pyrrolo[2,3-b]pyridin-3-yl)acrylamid |
| **207** | (R,E)-N-(1-(3,4-Dimethoxyphenyl)ethyl)-3-(5-(pyrimidin-5-yl)-1H-pyrrolo[2,3-b]pyridin-3-yl)acrylamid |
| **208** | (R,E)-N-(1-(3,4-Dimethoxyphenyl)ethyl)-3-(5-(3-sulfamoylphenyl)-1H-pyrrolo[2,3-b]pyridin-3-yl)acrylamid |
| **209** | (R,E)-N-(1-(3,4-Dimethoxyphenyl)ethyl)-3-(5-(1,3,5-trimethyl-1H-pyrazol-4-yl)-1H-pyrrolo[2,3-b]pyridin-3-yl)acrylamid |
| **210** | (R,E)-N-(1-(3,4-Dimethoxyphenyl)ethyl)-3-(5-(1-(2-morpholinoethyl)-1H-pyrazol-4-yl)-1H-pyrrolo[2,3-b]pyridin-3-yl)acrylamid |
| **211** | (R,E)-3-(5-(5-Cyano-1-methyl-1H-pyrazol-4-yl)-1H-pyrrolo[2,3-b]pyridin-3-yl)-N-(1-(3,4-dimethoxyphenyl)ethyl)acrylamid |
| **212** | (R,E)-N-(1-(3,4-Dimethoxyphenyl)ethyl)-3-(5-(1,3-dimethyl-1H-pyrazol-4-yl)-1H-pyrrolo[2,3-b]pyridin-3-yl)acrylamid |
| **213** | (R,E)-N-(1-(3,4-Dimethoxyphenyl)ethyl)-3-(5-(1-(2-(dimethylamino)ethyl)-1H-pyrazol-4-yl)-1H-pyrrolo[2,3-b]pyridin-3-yl)acrylamid |
| **214** | (E)-N-((R)-1-(3,4-Dimethoxyphenyl)ethyl)-3-(5-(1-(2-hydroxypropyl)-1H-pyrazol-4-yl)-1H-pyrrolo[2,3-b]pyridin-3-yl)acrylamid |
| **215** | (R,E)-N-(1-(3,4-Dimethoxyphenyl)ethyl)-3-(5-(1-(oxetan-3-yl)-1H-pyrazol-4-yl)-1H-pyrrolo[2,3-b]pyridin-3-yl)acrylamid |
| **216** | (R,E)-3-(5-(3-(Cyclopropylsulfonyl)phenyl)-1H-pyrrolo[2,3-b]pyridin-3-yl)-N-(1-(3,4-dimethoxyphenyl)ethyl)acrylamid |
| **217** | (R,E)-N-(1-(3,4-Dimethoxyphenyl)ethyl)-3-(5-(3-((methylsulfonyl)methyl)phenyl)-1H-pyrrolo[2,3-b]pyridin-3-yl)acrylamid |
| **218** | (R,E)-N-(1-(3,4-Dimethoxyphenyl)ethyl)-3-(5-(5-(methylsulfonyl)pyridin-3-yl)-1H-pyrrolo[2,3-b]pyridin-3-yl)acrylamid |
| **219** | (R,E)-N-(1-(3,4-Dimethoxyphenyl)ethyl)-3-(5-(4-(methylsulfonyl)phenyl)-1H-pyrrolo[2,3-b]pyridin-3-yl)acrylamid |
| **220** | (R,E)-N-(1-(3,4-Dimethoxyphenyl)ethyl)-3-(5-(4-fluor-3-(methylsulfonyl)phenyl)-1H-pyrrolo[2,3-b]pyridin-3-yl)acrylamid |
| **221** | (R,E)-3-(5-(3-Cyanophenyl)-1H-pyrrolo[2,3-b]pyridin-3-yl)-N-(1-(3,4-dimethoxyphenyl)ethyl)acrylamid |
| **222** | (R,E)-N-(1-(3,4-Dimethoxyphenyl)ethyl)-3-(5-(2-(methylsulfonyl)phenyl)-1H-pyrrolo[2,3-b]pyridin-3-yl)acrylamid |
| **223** | (R,E)-N-(1-(3,4-Dimethoxyphenyl)ethyl)-3-(4-(4-((dimethylamino)methyl)phenyl)-1H-pyrrolo[2,3-b]pyridin-3-yl)acrylamid |
| **224** | (R,E)-N-(1-(3,4-Dimethoxyphenyl)ethyl)-3-(4-(3-((dimethylamino)methyl)phenyl)-1H-pyrrolo[2,3-b]pyridin-3-yl)acrylamid |
| **225** | (R,E)-N-(1-(3,4-Dimethoxyphenyl)ethyl)-3-(4-phenyl-1H-pyrrolo[2,3-b]pyridin-3-yl)acrylamid |
| **226** | (R,E)-N-(1-(3,4-Dimethoxyphenyl)ethyl)-3-(4-(4-(methylsulfonamido)phenyl)-1H-pyrrolo[2,3-b]pyridin-3-yl)acrylamid |
| **227** | (R,E)-N-(1-(3,4-Dimethoxyphenyl)ethyl)-3-(4-(3-(N,N-dimethylsulfamoyl)phenyl)-1H-pyrrolo[2,3-b]pyridin-3-yl)acrylamid |
| **228** | (R,E)-N-(1-(3,4-Dimethoxyphenyl)ethyl)-3-(4-(3-(dimethylamino)phenyl)-1H-pyrrolo[2,3-b]pyridin-3-yl)acrylamid |
| **229** | (R,E)-N-(1-(3,4-Dimethoxyphenyl)ethyl)-3-(4-(4-fluor-2-methoxyphenyl)-1H-pyrrolo[2,3-b]pyridin-3-yl)acrylamid |
| **230** | (R,E)-N-(1-(3,4-Dimethoxyphenyl)ethyl)-3-(4-(4-(2-(dimethylamino)ethoxy)phenyl)-1H-pyrrolo[2,3-b]pyridin-3-yl)acrylamid |
| **231** | (R,E)-N-(1-(3,4-Dimethoxyphenyl)ethyl)-3-(4-(3-(methylsulfonamido)phenyl)-1H-pyrrolo[2,3-b]pyridin-3-yl)acrylamid |
| **232** | (R,E)-N-(1-(3,4-Dimethoxyphenyl)ethyl)-3-(4-(4-(morpholinomethyl)phenyl)-1H-pyrrolo[2,3-b]pyridin-3-yl)acrylamid |
| **233** | (R,E)-3-(3-(3-((1-(3,4-Dimethoxyphenyl)ethyl)amino)-3-oxoprop-1-en-1-yl)-1H-pyrrolo[2,3-b]pyridin-4-yl)benzamid |
| **234** | (R,E)-N-(1-(3,4-Dimethoxyphenyl)ethyl)-3-(4-(4-((4-methylpiperazin-1-yl)methyl)phenyl)-1H-pyrrolo[2,3-b]pyridin-3-yl)acrylamid |
| **235** | (R,E)-3-(4-(4-(4-Acetylpiperazin-1-yl)phenyl)-1H-pyrrolo[2,3-b]pyridin-3-yl)-N-(1-(3,4-dimethoxyphenyl)ethyl)acrylamid |
| **236** | (R,E)-4-(3-(3-((1-(3,4-Dimethoxyphenyl)ethyl)amino)-3-oxoprop-1-en-1-yl)-1H-pyrrolo[2,3-b]pyridin-4-yl)benzamid |
| **237** | (R,E)-N-(1-(3,4-Dimethoxyphenyl)ethyl)-3-(4-(1,5-dimethyl-1H-pyrazol-4-yl)-1H-pyrrolo[2,3-b]pyridin-3-yl)acrylamid |
| **238** | (R,E)-N-(1-(3,4-Dimethoxyphenyl)ethyl)-3-(4-(1-methyl-1H-pyrazol-4-yl)-1H-pyrrolo[2,3-b]pyridin-3-yl)acrylamid |
| **239** | (E)-N-((R)-1-(3,4-Dimethoxyphenyl)ethyl)-3-(4-(3-(ethylsulfinyl)phenyl)-1H-pyrrolo[2,3-b]pyridin-3-yl)acrylamid |
| **240** | (R,E)-N-(1-(3,4-Dimethoxyphenyl)ethyl)-3-(4-(4-(1-methylpiperidin-4-yl)phenyl)-1H-pyrrolo[2,3-b]pyridin-3-yl)acrylamid |
| **241** | (R,E)-N-(1-(3,4-Dimethoxyphenyl)ethyl)-3-(4-(4-(3-(dimethylamino)propoxy)phenyl)-1H-pyrrolo[2,3-b]pyridin-3-yl)acrylamid |
| **242** | (R,E)-N-(1-(3,4-Dimethoxyphenyl)ethyl)-3-(4-(pyrazolo[1,5-a]pyridin-3-yl)-1H-pyrrolo[2,3-b]pyridin-3-yl)acrylamid |
| **243** | (R,E)-3-(4-(3-(Acetamidomethyl)phenyl)-1H-pyrrolo[2,3-b]pyridin-3-yl)-N-(1-(3,4-dimethoxyphenyl)ethyl)acrylamid |
| **244** | (R,E)-2-Cyano-N-(1-(3,4-dimethoxyphenyl)ethyl)-3-(4-methyl-5-(4-(4-methylpiperazin-1-yl)phenyl)-1H-pyrrolo[2,3-b]pyridin-3-yl)acrylamid |
| **245** | (R,E)-2-Cyano-N-(1-(3,4-dimethoxyphenyl)ethyl)-3-(5-(4-fluor-3-(methylsulfonyl)phenyl)-4-methyl-1H-pyrrolo[2,3-b]pyridin-3-yl)acrylamid |
| **246** | (R,E)-N-(1-(Benzo[d][1,3]dioxol-5-yl)ethyl)-3-(5-(1-methyl-1H-pyrazol-4-yl)-1H-pyrrolo[2,3-b]pyridin-3-yl)acrylamide |
| **247** | (R,E)-3-(5-(3-Chlor-5-(methylsulfonyl)phenyl)-1H-pyrrolo[2,3-b]pyridin-3-yl)-N-(1-(3,4-dimethoxyphenyl)ethyl)acrylamid |
| **248** | (R,E)-N-(1-(3,4-Dimethoxyphenyl)ethyl)-3-(5-(2-methyl-5-(methylsulfonyl)phenyl)-1H-pyrrolo[2,3-b]pyridin-3-yl)acrylamid |
| **249** | (R,E)-N-(1-(3,4-Dimethoxyphenyl)ethyl)-3-(5-(3-fluor-5-(methylsulfonyl)phenyl)-1H-pyrrolo[2,3-b]pyridin-3-yl)acrylamid |
| **250** | (R,E)-N-(1-(3,4-Dimethoxyphenyl)ethyl)-3-(5-(2-(methylsulfonyl)pyridin-4-yl)-1H-pyrrolo[2,3-b]pyridin-3-yl)acrylamid |
| **251** | (E)-N-((R)-1-(3,4-Dimethoxyphenyl)ethyl)-3-(5-(3-(S-methylsulfonimidoyl)phenyl)-1H-pyrrolo[2,3-b]pyridin-3-yl)acrylamid |
| **252** | (R,E)-N-(1-(Benzo[d][1,3]dioxol-5-yl)ethyl)-2-cyano-3-(5-(1-methyl-1H-pyrazol-4-yl)-1H-pyrrolo[2,3-b]pyridin-3-yl)acrylamid |
| **253** | (E)-N-(1-(3-Cyclopropyl-4-methoxyphenyl)ethyl)-3-(5-(1-methyl-1H-pyrazol-4-yl)-1H-pyrrolo[2,3-b]pyridin-3-yl)acrylamid |
| **254** | (R,E)-N-(1-(3,4-Dimethoxyphenyl)ethyl)-3-(5-(3-(methylthio)phenyl)-1H-pyrrolo[2,3-b]pyridin-3-yl)acrylamid |
| **255** | (E)-N-(1-(3-Methoxy-4-(methylsulfinyl)phenyl)ethyl)-3-(5-(1-methyl-1H-pyrazol-4-yl)-1H-pyrrolo[2,3-b]pyridin-3-yl)acrylamid |
| **256** | (E)-2-Cyano-N-(1-(3-methoxy-4-(methylsulfinyl)phenyl)ethyl)-3-(5-(1-methyl-1H-pyrazol-4-yl)-1H-pyrrolo[2,3-b]pyridin-3-yl)acrylamid |
| **257** | (R,E)-N-(1-(3,4-Difluorphenyl)ethyl)-3-(5-(1-methyl-1H-pyrazol-4-yl)-1H-pyrrolo[2,3-b]pyridin-3-yl)acrylamid |
| **258** | (R,E)-2-Cyano-N-(1-(3,4-difluorphenyl)ethyl)-3-(5-(1-methyl-1H-pyrazol-4-yl)-1H-pyrrolo[2,3-b]pyridin-3-yl)acrylamid |
| **259** | (R,E)-N-(1-(3,4-Dimethoxyphenyl)ethyl)-3-(5-(4-(methylsulfonyl)pyridin-2-yl)-1H-pyrrolo[2,3-b]pyridin-3-yl)acrylamid |
| **260** | (R,E)-3-(5-(1-Methyl-1H-pyrazol-4-yl)-1H-pyrrolo[2,3-b]pyridin-3-yl)-N-(1-(3-methylbenzo[d]isoxazol-5-yl)ethyl)acrylamid |
| **261** | (R,E)-2-Cyano-N-(1-(1,3-dimethyl-1H-indazol-6-yl)ethyl)-3-(5-(1-methyl-1H-pyrazol-4-yl)-1H-pyrrolo[2,3-b]pyridin-3-yl)acrylamid |
| **262** | (R,E)-2-Cyano-N-(1-(1,3-dimethyl-1H-indazol-5-yl)ethyl)-3-(5-(1-methyl-1H-pyrazol-4-yl)-1H-pyrrolo[2,3-b]pyridin-3-yl)acrylamid |
| **263** | (E)-N-((R)-1-(3,4-Dimethoxyphenyl)ethyl)-3-(5-(3-((trifluormethyl)sulfinyl)phenyl)-1H-pyrrolo[2,3-b]pyridin-3-yl)acrylamid |
| **264** | (R,E)-N-(1-(3,4-Dimethoxyphenyl)ethyl)-3-(5-(3-((trifluormethyl)sulfonyl)phenyl)-1H-pyrrolo[2,3-b]pyridin-3-yl)acrylamid |
| **265** | (E)-N-(1-(5,6-Dimethoxypyridin-3-yl)ethyl)-3-(5-(1-methyl-1H-pyrazol-4-yl)-1H-pyrrolo[2,3-b]pyridin-3-yl)acrylamid |
oder ein pharmazeutisch verträgliches Salt davon.

8. Eine pharmazeutische Zusammensetzung umfassend mindestens eine Verbindung gemäß den Ansprüchen 1 bis 7 als Wirkstoff zusammen mit mindestens einem pharmazeutisch verträglichen Träger, Hilfsstoff und/oder Verdünnungsmittel.

9. Eine Verbindung gemäß den Ansprüchen 1 bis 7 zur Verwendung als Medikament.

10. Eine Verbindung gemäß den Ansprüchen 1 bis 7 oder eine pharmazeutische Zusammensetzung gemäß Anspruch 8 zur Verwendung bei der Prophylaxe und/oder Behandlung einer Erkrankung, die durch G-Protein-gekoppelte Rezeptorkinasen 5 (GRK5) verursacht wird oder damit assoziiert ist, ausgewählt aus Herzerkrankungen, entzündlichen und immunologischen Erkrankungen, Stoffwechselerkrankungen und Krebs.

11. Die Verbindung zur Verwendung oder die pharmazeutische Zusammensetzung zur Verwendung gemäß Anspruch 10, wobei die Herzerkrankung ausgewählt wird aus: Herzhypertrophie, angeborener Herzerkrankung bei Erwachsenen, Aneurysma, stabiler Angina pectoris, instabiler Angina pectoris, Angina pectoris, angioneurotischem Ödem, Aortenklappenstenose, Aortenaneurysma, Arrhythmie, arrhythmogene rechtsventrikuläre Dysplasie, Arteriosklerose, arteriovenöse Fehlbildungen, Vorhofflimmern, Behçet-Syndrom, Bradykardie, Herztamponade, Kardiomegalie, kongestive Kardiomyopathie, hypertrophe Kardiomyopathie, restriktive Kardiomyopathie, Prävention von Herz-Kreislauf-Erkrankungen, Karotisstenose, Hirnblutung, Churg-Strauss-Syndrom, Diabetes, Ebstein-Anomalie, Eisenmenger-Komplex, Cholesterinembolie, bakterielle Endokarditis, fibromuskuläre Dysplasie, angeborene Herzfehler, Herzerkrankungen, kongestive Herzinsuffizienz, Herzklappenerkrankungen, Herzinfarkt, Epiduralhämatom, Hämatom, subdural, Hippel-Lindau-Syndrom, Hyperämie, Hypertonie, pulmonale Hypertonie, hypertrophes Wachstum, linksventrikuläre Hypertrophie, rechtsventrikuläre Hypertrophie, hypoplastisches Linksherzsyndrom, Hypotonie, intermittierende Claudicatio, ischämische Herzerkrankung, Klippel-Trenaunay-Weber-Syndrom, laterales medulläres Syndrom, Long-QT-Syndrom, Mitralklappenprolaps, Moyamoya-Krankheit, mukokutanes Lymphknotensyndrom, Myokardinfarkt, Myokardischämie, Myokarditis, Perikarditis, periphere Gefäßerkrankungen, Venenentzündung, Polyarteritis nodosa, Pulmonalatresie, Raynaud-Syndrom, Restenose, Sneddon-Syndrom, Stenose, Superior-Vena-Cava-Syndrom, Syndrom X, Tachykardie, Takayasu-Arteriitis, hereditäre hämorrhagische Teleangiektasie, Teleangiektasie, Arteriitis temporalis, Fallot-Tetralogie, Thromboangiitis obliterans, Thrombose, Thromboembolie, Trikuspidalatresie, Krampfadern, Gefäßerkrankungen, Vaskulitis, Vasospasmus, Kammerflimmern, Williams-Syndrom, periphere Gefäßerkrankung, Krampfadern und Beingeschwüre, tiefe Venenthrombose, Wolff-Parkinson-White-Syndrom.

12. Die Verbindung zur Verwendung oder die pharmazeutische Zusammensetzung zur Verwendung gemäß Anspruch 10, wobei die entzündliche und immunologische Erkrankung ausgewählt wird aus: einer mit einer Autoimmunerkrankung assoziierten entzündlichen Erkrankung, einer entzündlichen Erkrankung des Zentralnervensystems (ZNS), einer Gelenkentzündungserkrankung, einer entzündlichen Erkrankung des Verdauungstrakts und einer entzündlichen Hauterkrankung.

13. Die Verbindung zur Verwendung oder die pharmazeutische Zusammensetzung zur Verwendung gemäß Anspruch 10, wobei die Stoffwechselerkrankung aus Diabetes, Adipositas und gestörter Adipogenese ausgewählt wird.

14. Die Verbindung zur Verwendung oder die pharmazeutische Zusammensetzung zur Verwendung gemäß Anspruch 10, wobei der Krebs ausgewählt wird aus der Gruppe bestehend aus: Adenokarzinom, Aderhautmelanom, akuter Leukämie, Akustikusneurinom, Ampullenkarzinom, Analkarzinom, Astrozytom, Basalzellkarzinom, Pankreaskrebs, Desmoidtumor, Blasenkrebs, Bronchialkarzinom, nicht-kleinzelligem Lungenkrebs (NSCLC), Brustkrebs, Burkitt-Lymphom, Korpuskarzinom, CUP-Syndrom (Karzinom unbekannter Primärursache), Kolorektalkarzinom, Dünndarmkrebs, Dünndarmtumoren, Eierstockkrebs, Endometriumkarzinom, Ependymom, epitheliale Krebsarten, Ewing-Tumoren, Magen-Darm-Tumoren, Magenkrebs, Gallenblasenkrebs, Gallenblasenkarzinome, Gebärmutterkrebs, Gebärmutterhalskrebs, Glioblastome, gynäkologische Tumoren, Tumoren im Hals-Nasen-Ohren-Bereich, hämatologische Neoplasien, Haarzell-Leukämie, Harnröhrenkrebs, Hautkrebs, Haut-Hodenkrebs, Hirntumoren (Gliome), Hirnmetastasen, Hodenkrebs, Hypophysentumoren, Karzinoide, Kaposi-Sarkom, Kehlkopfkrebs, Keimzelltumor, Knochenkrebs, kolorektales Karzinom, Kopf-Hals-Tumoren (Tumoren im Bereich von Hals, Nase und Ohren), Kolonkarzinom, Kraniopharyngeome, Mundhöhlenkrebs (Krebs im Mundbereich und an den Lippen), Krebs des Zentralnervensystems, Leberkrebs, Lebermetastasen, Leukämie, Augenlidtumor, Lungenkrebs, Lymphknotenkrebs (Hodgkin/Non-Hodgkin), Lymphome, Magenkrebs, malignes Melanom, maligne Neoplasie, maligne Tumoren des Magen-Darm-Traktes, Mammakarzinom, Rektumkarzinom, Medulloblastome, Melanom, Meningeome, Hodgkin-Krankheit, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome und osteoplastische Karzinome, Osteosarkome, Ovarialkarzinom, Pankreaskarzinom, Peniskrebs, Plasmozytom, Plattenepithelkarzinom des Kopfes und Halses (SCCHN), Prostatakrebs, Rachenkrebs, Rektumkarzinom, Retinoblastom, Vaginalkrebs, Schilddrüsenkarzinom, Schneeberger-Krankheit, Speiseröhrenkrebs, Spinaliome, T-Zell-Lymphom (Mycosis fungoides), Thymom, Tubenkarzinom, Augentumoren, Harnröhrenkrebs, urologische Tumoren, Urothelkarzinom, Vulvakrebs, Warzenbildung, Weichteiltumoren, Weichteilsarkom, Wilms-Tumor, Gebärmutterhalskrebs, Zungenkrebs, Astrozytome, Bronchialkrebs, Kehlkopfkrebs, malignes Melanom, Speiseröhrenkrebs und Nierenzellkrebs.

15. Ein Verfahren zur Herstellung der Verbindung der Formel (la) gemäß Anspruch 1 umfassend:
**Schritt 1:** Bereitstellen von 1H-Pyrrolo[2,3-b]pyridin-3-carbaldehyd-Verbindung (2*) 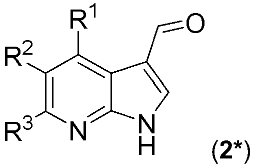
**Schritt** 2: Durchführen einer Kondensationsreaktion der Verbindung (2*) mit einer Verbindung (3*) 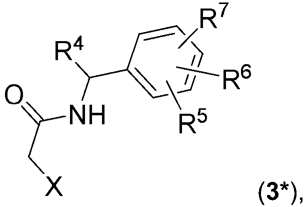
um die Verbindung der Formel (la) zu erhalten 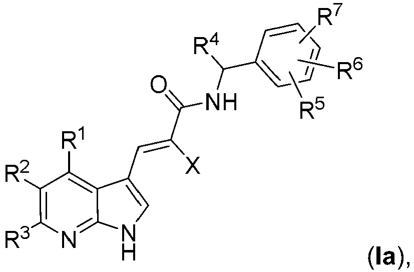
wobei **R¹, R², R³, R⁴, R⁵, R⁶, R⁷** und **X** die in Anspruch 1 definierten Bedeutungen haben;
**oder**
**Schritt 1:** Bereitstellen von 1H-Pyrrolo[2,3-b]pyridin-3-carbaldehyd-Verbindung **(2*)** 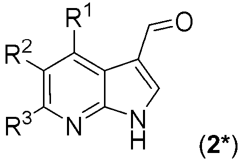
**Schritt** 2a: Durchführen einer Kondensationsreaktion von Verbindung (2*) mit Maleinsäure oder Cyanoessigsäure, um Acrylsäure-Verbindung (4*), zu erhalten 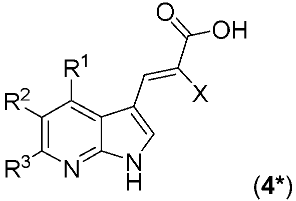
**Schritt** 3: Durchführen einer Kupplungsreaktion von (4*) mit einer Aminverbindung (5*) 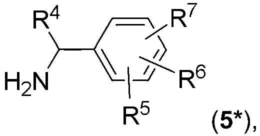
um die Verbindung der Formel (la) zu erhalten 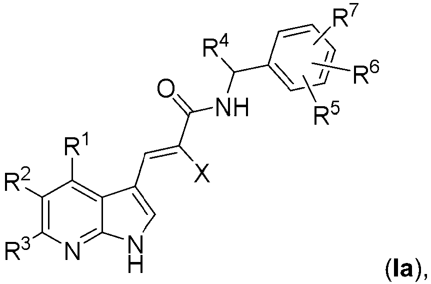
wobei **R¹**, **R², R³, R⁴, R⁵, R⁶, R⁷** und X die in Anspruch 1 definierten Bedeutungen haben.

## Revendications

1. Composé de la formule générale (I) 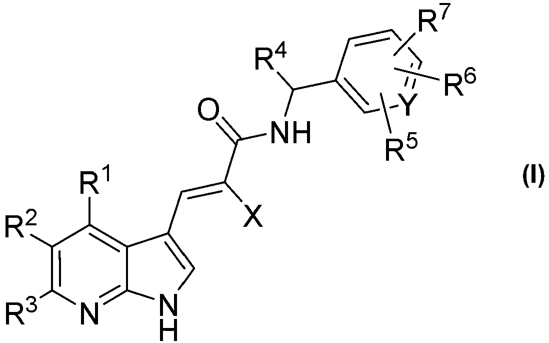 où
**X** représente -H ou -CN,
**Y** représente CH ou N,
**R¹** - **R²** R2 représentent indépendamment les uns des autres -H, -Ph, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, cyclo-C₃H₅, cyclo-C₄H₇, cyclo-C₅H₉, cyclo-C₆H₁₁, -F, -CI, -Br, -CN, -OH, -NH-Ph, -**R⁹**, -O-**R¹⁰**, 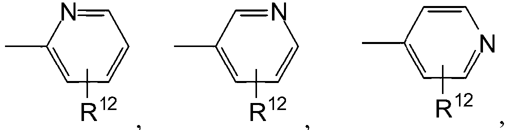 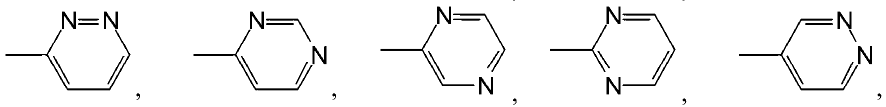 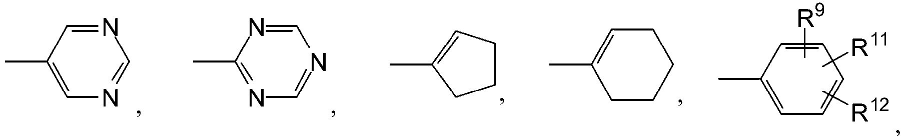 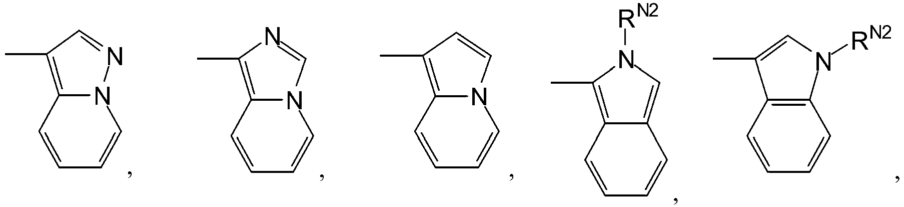 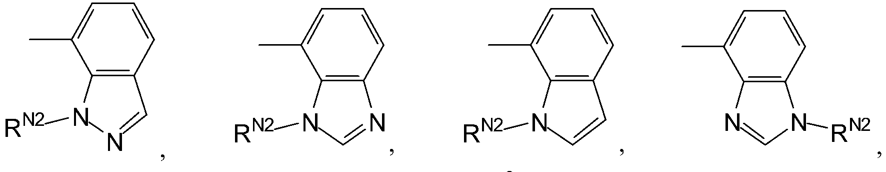 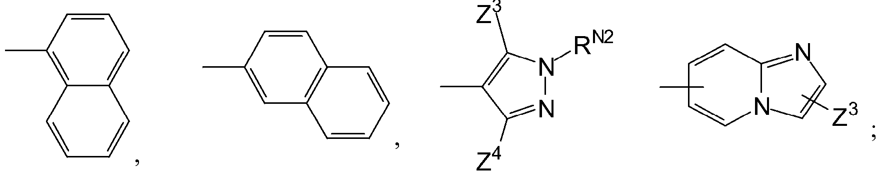
**R³** représente -H, -F, -CI, ou -Br;
**R⁴** représente -H, -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH₂-OH, -C₂H₄-OH, -C₃H₆-OH, -CN, -CH₂-NH₂, -CH₂-NHCH₃, -CH₂-NHC₂H₅, -CH₂-NHC₃H₇, -CH₂-N(CH₃)₂, -CH₂-N(C₂H₅)₂, -CH₂-N(C₃H₇)₂, -C₂H₄-NH₂, -C₂H₄-NHCH₃, -C₂H₄-NHC₂H₅, -C₂H₄-NHC₃H₇, -C₂H₄-N(CH₃)₂, -C₂H₄-N(C₂H₅)₂, -C₂H₄-N(C₃H₇)₂, -C₃H₆-NH₂, -C₃H₆-NHCH₃, -C₃H₆-NHC₂H₅, -C₃H₆-NHC₃H₇, -C₃H₆-N(CH₃)₂, -C₃H₆-N(C₂H₅)₂, -C₃H₆-N(C₃H₇)₂;
**R⁵** - **R⁷** représentent indépendamment les uns des autres -**R⁸**, -CH₂-**R⁸,** -SO₂-**R⁸**, 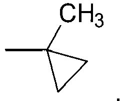 cyclo-C₃H₅, cyclo-C₄H₇, cyclo-C₅H₉, cyclo-C₆H₁₁, cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -CI, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -CH₂-SO₂NH₂, -CH₂-SO₂NHCH₃, -CH₂-SO₂NHC₂H₅, -CH₂-SO₂NHC₃H₇, -CH₂-SO₂NH-cyclo-C₃H₅, -CH₂-SO₂NHCH(CH₃)₂, -CH₂-SO₂NHC(CH₃)₃, -CH₂-SO₂N(CH₃)₂, -CH₂-SO₂N(C₂H₅)₂, -CH₂-SO₂N(C₃H₇)₂, -CH₂-SO₂N(cyclo-C₃H₅)₂, -CH₂-SO₂N[CH(CH₃)₂]₂, -CH₂-SO₂N[C(CH₃)₃]₂, -O-S(=O)CH₃, -O-S(=O)C₂H₅, -O-S(**=**O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂ -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O--CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cycio-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₁, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)_{2]}=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, ou -C≡C-C≡C-C₂H₅,
ou
**R⁵** et **R⁶,** ou **R⁶** et **R⁷** forment ensemble 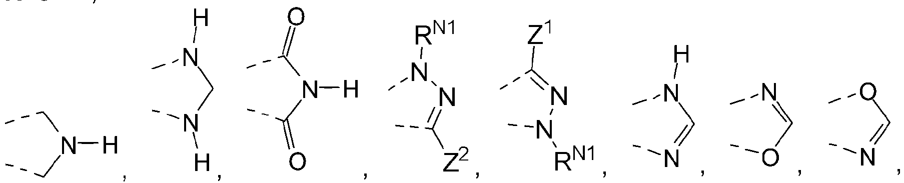 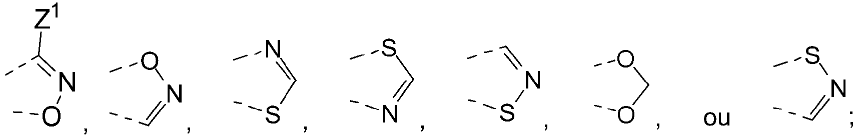
**R⁸** représente 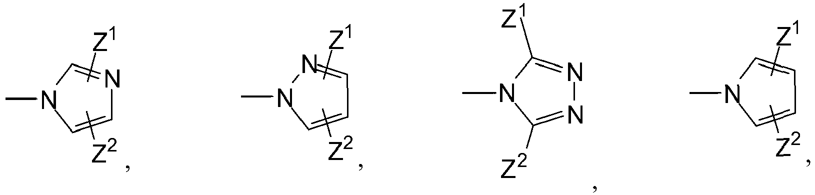 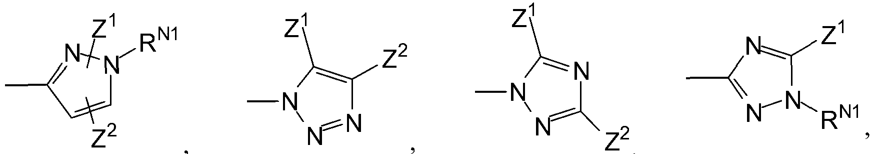 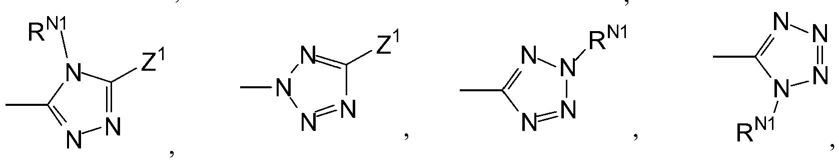 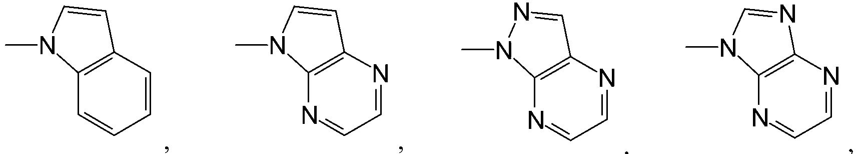 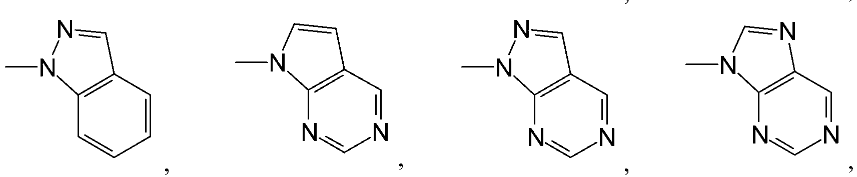 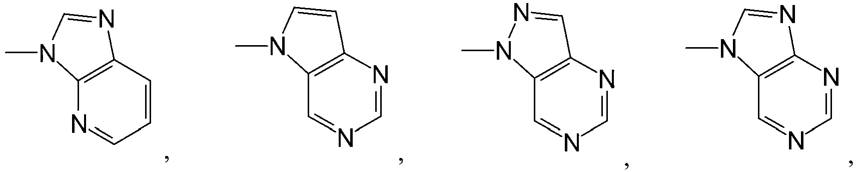 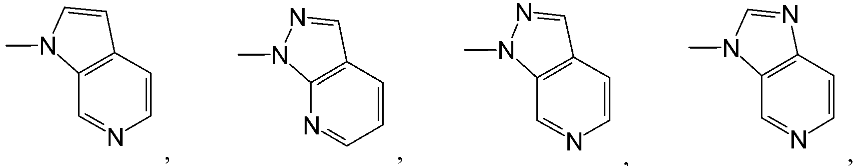 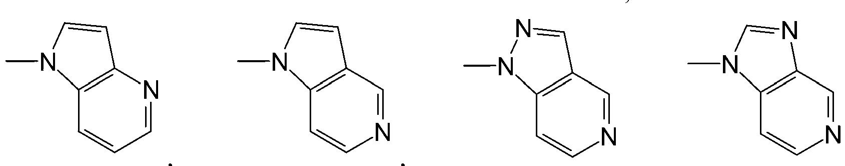 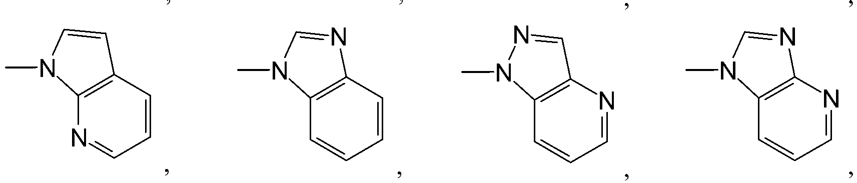 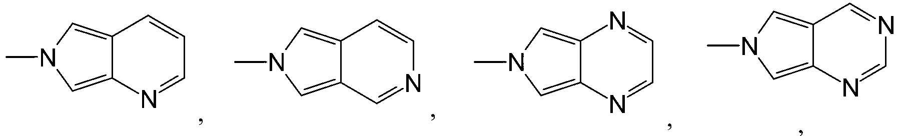 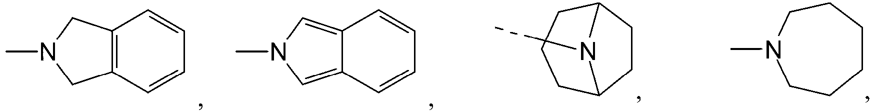 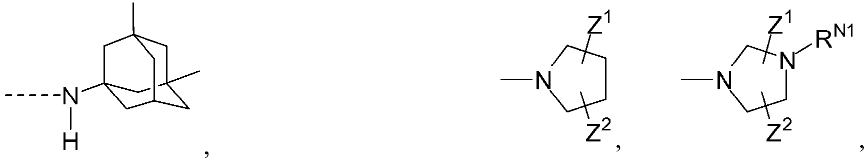 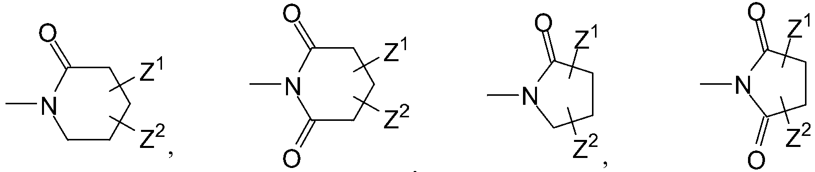 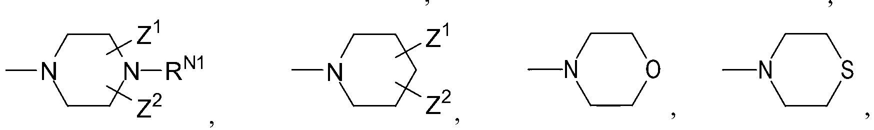 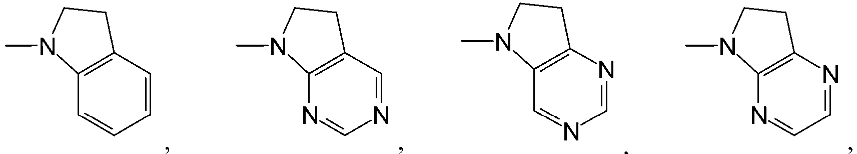 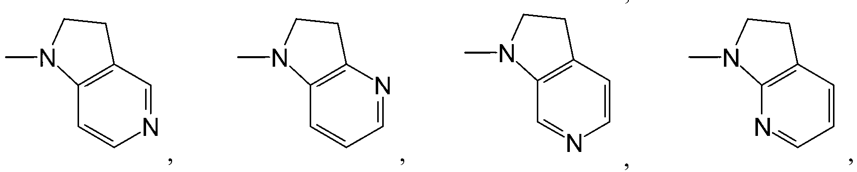 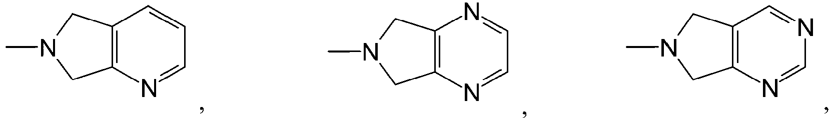 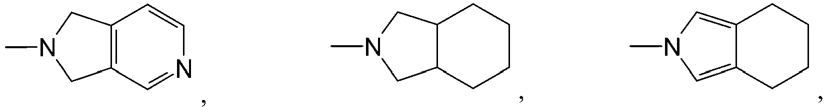 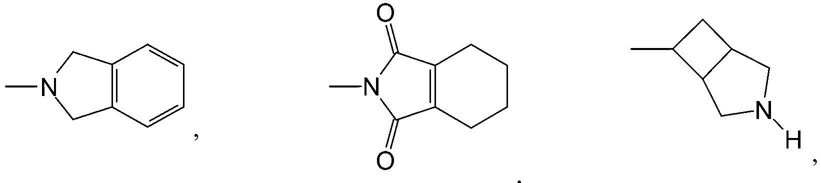 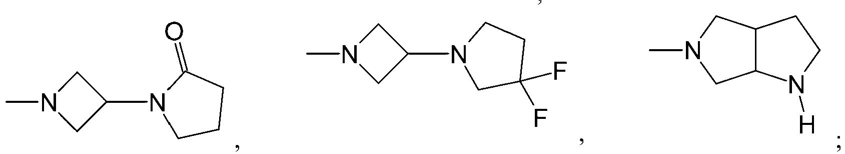
**R⁹** représente -H, 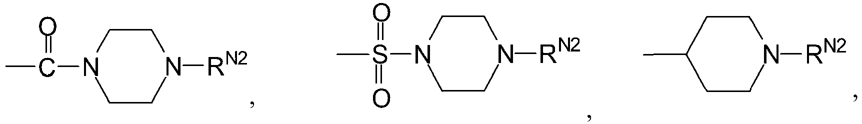 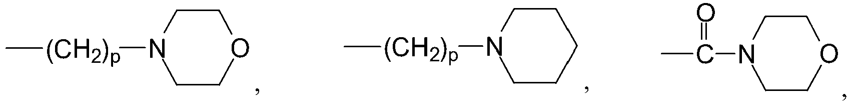 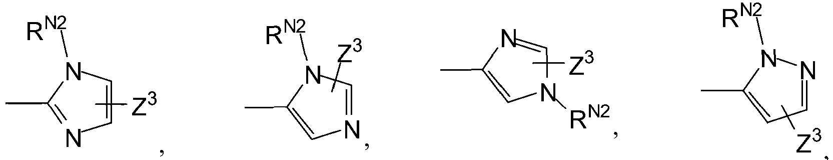 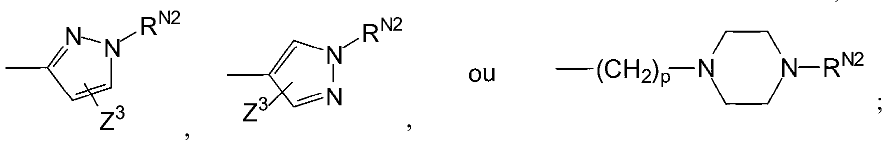
**R¹⁰** représente -CH₃, -CF₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -(CH₂)ₙ-NH₂, -(CH₂)ₙ-NHCH₃, -(CH₂)ₙ-NHC₂H₅, -(CH₂)ₙ-NHC₃H₇, -(CH₂)ₙ-N(CH₃)₂, -(CH₂)ₙ-N(C₂H₅)₂, -(CH₂)ₙ-N(C₃H₇)₂, 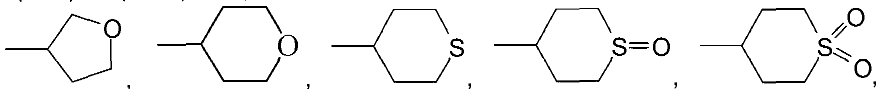 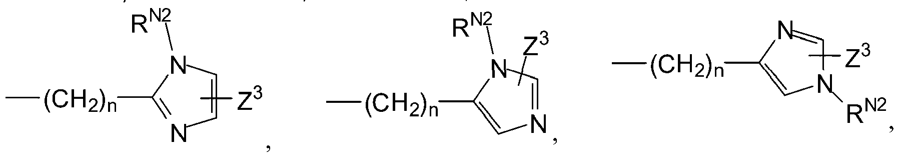 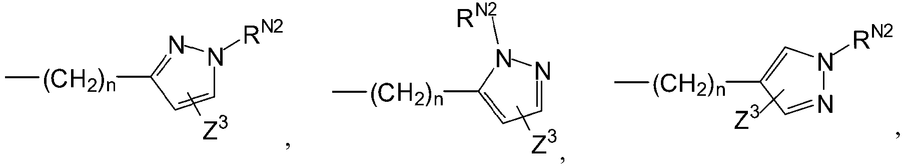
**R¹¹** et **R¹²** représentent indépendamment les uns des autres -H, -CH₃, -C₂H₅, -C₃H₇,
-CH(CH₃)₂, -CF₃, -(CH₂)ₘ-NH₂, -(CH₂)ₘ-NHCH₃, -(CH₂)ₘ-NHC₂H₅, -(CH₂)ₘ-NHC3H₇, -(CH2)ₘ-N(CH₃)₂, -(CH₂)ₘ-N(C2H₅)₂, -(CH₂)ₘ-N(C₃H₇)2, -OCH₃, -OC₂H₅, -OC₃H₇, -O-cyclo-C₃H₅, -OCH(CH₃)₂, -F, -Cl, -Br, -CN, -(CH2)ₘ-SO2CH₃, -(CH₂)ₘ-SO₂C₂H₅, -(CH₂)ₘ-SO₂C₃H₇, -(CH₂)ₘ-SO₂-cyclo-C₃H₅, -(CH₂)ₘ-SO₂CH(CH₃)₂, -(CH₂)ₘ-SO₂C(CH₃)₃, -O-(CH₂)ₘ-NH₂, -O-(CH₂)ₘ-NHCH₃, -O-(CH₂)ₘ-NHC₂H₅, -O-(CH₂)ₘ-NHC₃H₇, -O-(CH₂)ₘ-N(CH₃)₂, -O-(CH₂)ₘ-N(C2H₅)₂, -O-(CH₂)ₘ-N(C₃H₇)₂, -NHCOCH₃, -(CH₂)ₘ-NHCOCH₃, -(CH₂)ₘ-NHCOC₂H₅, -(CH₂)ₘ-NHCO-cyclo-C₃H₅, -(CH₂)ₘ-NHCOC₃H₇, -(CH₂)ₘ-NHCO-CH(CH₃)₂, -(CH₂)ₘ-NHCO-CH=CH₂, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -NH-SO₂-CH₃, -NH-SO₂-C₂H₅, -NH-SO₂-C₃H₇, -NH-SO₂-cyclo-C₃H₅, -NH-SO₂-CH(CH₃)₂, -CONH₂, -SOCH₃, -SOC₂H₅, -SOC₃H₇, -SO-cyclo-C₃H₅, -SOCH(CH₃)₂, -SCH₃, -SOCF₃, -SO₂CF₃, ou -SO(NH)CH₃ ;
**R^{N1}** et **R^{N2}** représentent indépendamment les uns des autres -H, -CH₃, -C₂H₅, -C₃H₇, -CH(CH₃)₂, cyclo-C₃H₅, cyclo-C₄H₇, cyclo-C₅H₉, cyclo-C₆H₁₁, -C₄H₉, -C(CH₃)₃, -CH₂-CH(CH₃)₂, -CH(CH₃)-C₂H₅, -C₅H₁₁, -Ph, -CH₂-Ph, -CH=CH₂, -CH₂-CH=CH₂, -CH=CH-CH₃, -C=CH, -CH₂-C≡CH, -C≡C-CH₃, -COCH₃, -COC₂H₅, -COC₃H₇, -CO-cyclo-C₃H₅, -COCH(CH₃)₂, -COOCH₃, -COOC₂H₅, -COOC₃H₇, -COO-cyclo-C₃H₅, -COOCH(CH₃)₂, -COOC(CH₃)₃, -CH₂-CH(OH)-CH₃, -(CH₂)_{q}-NH₂, -(CH₂)_{q}-NHCH₃, -(CH₂)_{q}-NHC₂H₅, -(CH₂)_{q}-NHC₃H₇, -(CH₂)_{q}-N(CH₃)₂, -(CH₂)_{q}-N(C₂H₅)₂, -(CH₂)_{q}-N(C₃H₇)₂, 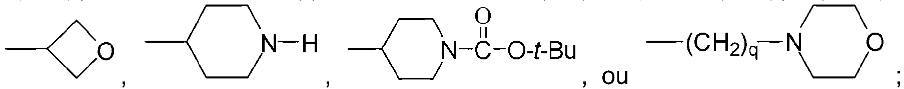
**Z¹** - **Z⁴** représentent indépendamment les uns des autres -H, -CH₃, -C₂H₅, -C₃H₇, -CH(CH₃)₂, -Ph, -COCH₃, -COC₂H₅, -COOH, -COOCH₃, -COOC₂H₅, -NH₂, -NHCH₃, -NHC₂H₅, -N(CH₃)₂, -N(C₂H₅)₂, -CH₂-NH₂, -SO₃H, -F, -CI, -Br, -I, -CN, -CF₃, -OCF₃, -OH, -OCH₃, ou -OC₂H₅ ;
m est un entier sélectionné parmi 0, 1, 2, 3, ou 4 ;
n est un entier sélectionné parmi 1, 2, 3, ou 4 ;
p est un entier sélectionné parmi 0, 1, 2, 3, ou 4 ;
q est un entier sélectionné parmi 1, 2, 3, ou 4 ;
sous réserve que lorsque X est -H, au moins l'un des **R¹** - **R³** n'est pas -H ; et lorsque **R⁴** est -H, au moins l'un des **R¹** - **R³** et **R⁵** - **R⁷** n'est pas -H ; et le composé suivant est exclu du champ d'application de la présente demande de brevet : 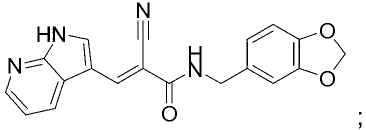
ou un énantiomère, une forme stéréoisomérique, un mélange d'énantiomères, un diastéréo-isomère, un mélange de diastéréo-isomères, un hydrate, un solvate, une forme de sel d'acide, un tautomère ou un racémate du composé susmentionné, ou un de ses sels de formule acceptable dans le domaine pharmaceutique.

2. Le composé conforme à la revendication 1, où
**R¹** et **R²** représentent indépendamment les uns des autres -H, -Ph, -CH₃, -CF₃, -CI, -Br, -CN, -OH, -NH-Ph, **-R⁹**, -O-**R¹⁰**, 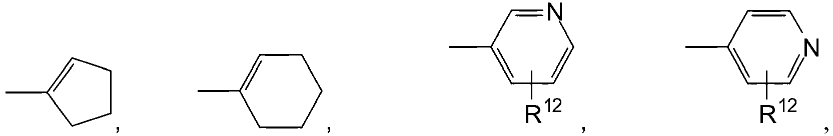 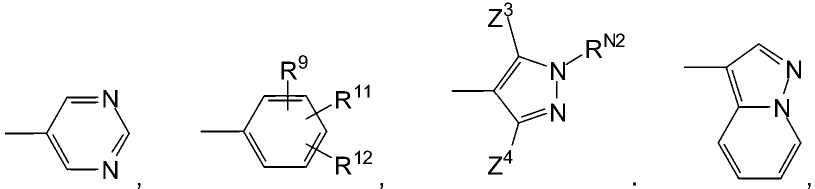 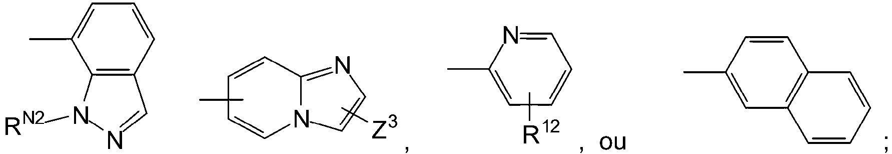
où **R⁹**, **R¹⁰**, **R¹¹**, **R¹²**, **R^{N2}**, **Z³**, **Z⁴** ont la signification définie dans la revendication 1.

3. Le composé conforme à la revendication 1 ou 2, où
**R¹** représente -H, -CH₃, -CI, 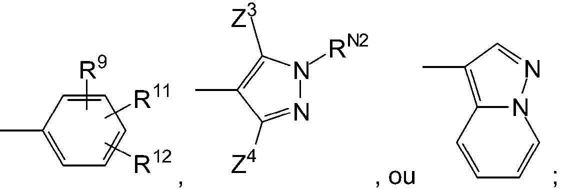
**R²** représente -H, -Ph, -CH₃, -CF₃, -CI, -Br, -CN, -OH, -NH-Ph, **-R⁹**, -O-**R¹⁰**, 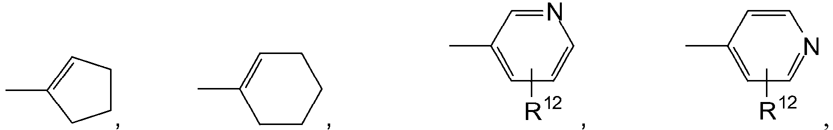 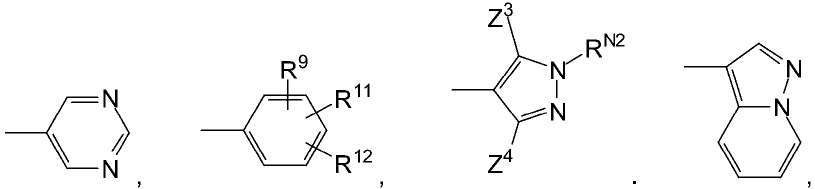 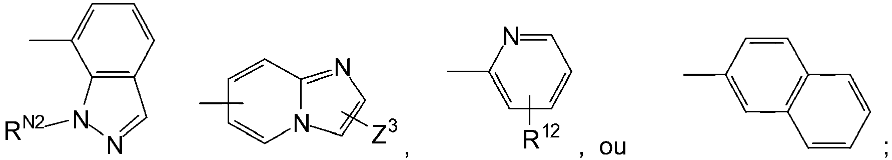
**R⁹** représente -H, 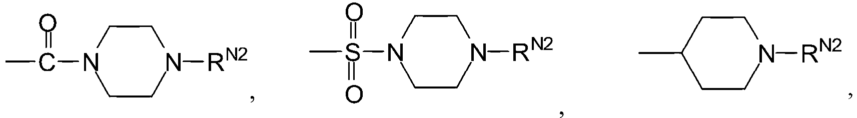 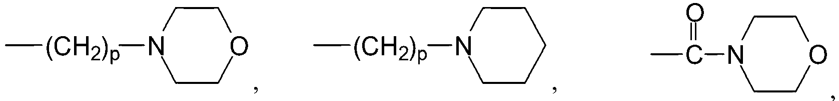 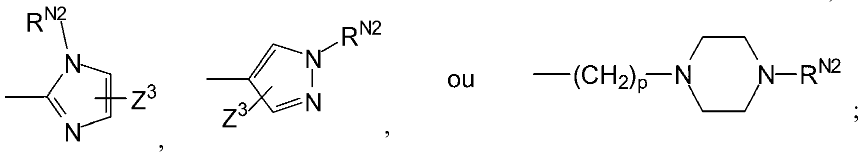
**R¹⁰** représente -CH(CH₃)₂, -CH₂-CH(CH₃)₂, -CH(CH₃)-C₂H₅, -(CH₂)ₙ-N(CH₃)₂, 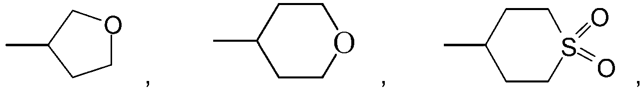 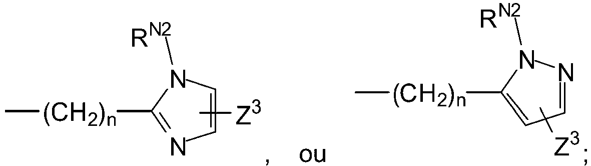
où **n**, p, **R¹¹**, **R¹²**, **R^{N2}**, **Z³**, **Z⁴** ont la signification définie dans la revendication 1.

4. Le composé conforme à l'une des revendications 1 à 3, où ledit composé correspond à l'une des formules (la), (Ib), et (**II-1**) - (**II-6**) 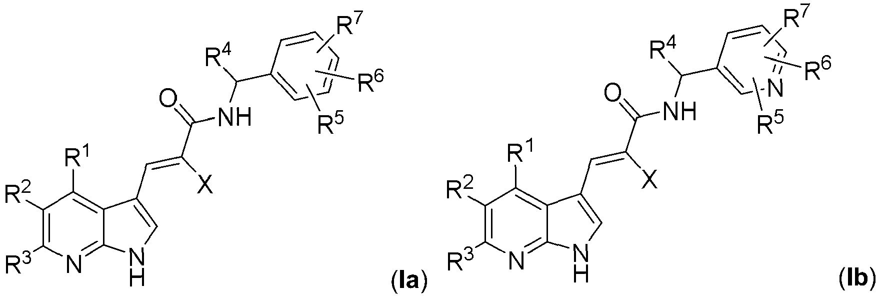 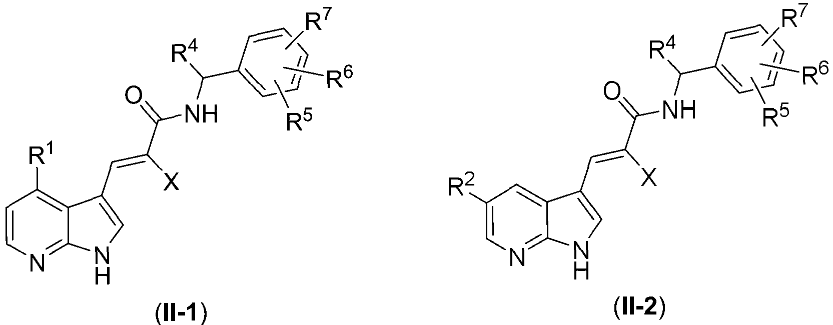 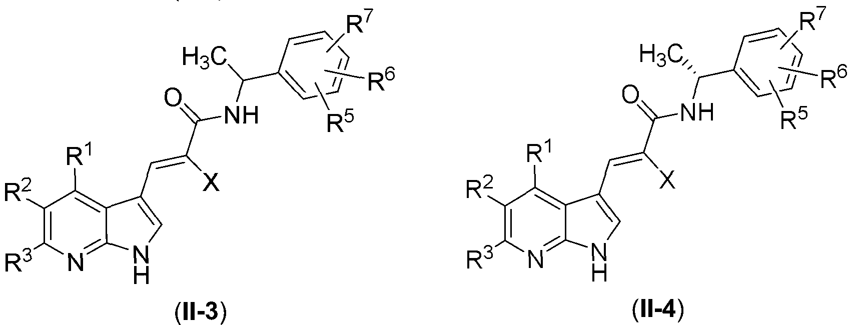 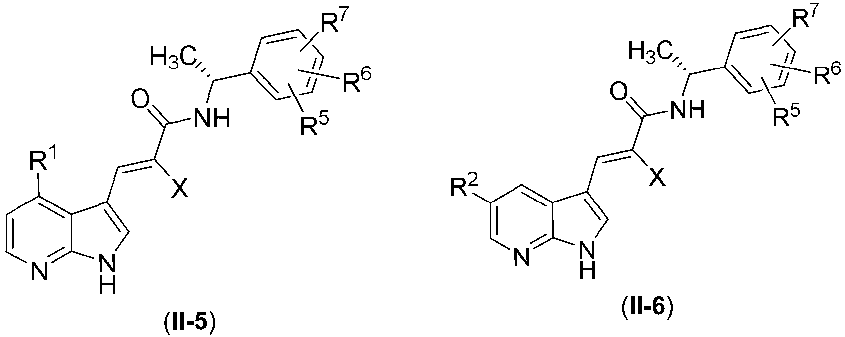 où **R¹, R², R³, R⁴, R⁵, R⁶, R⁷** et **X** ont les significations définies dans la revendication 1.

5. Le composé conforme à l'une des revendications 1 à 4, où ledit composé correspond à l'une des formules (**III-1**) - (**III-6**) 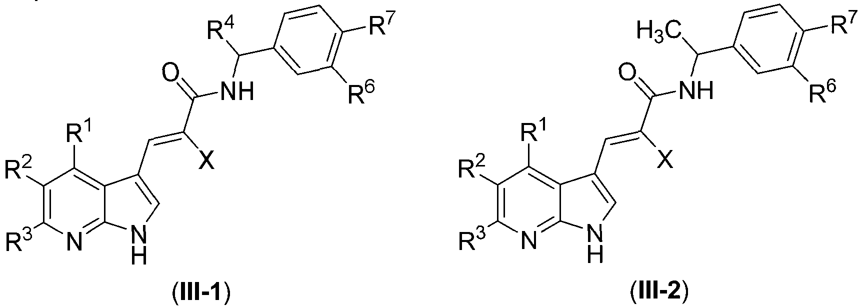 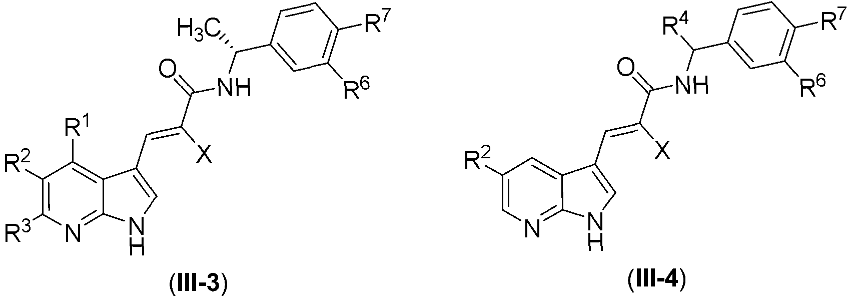 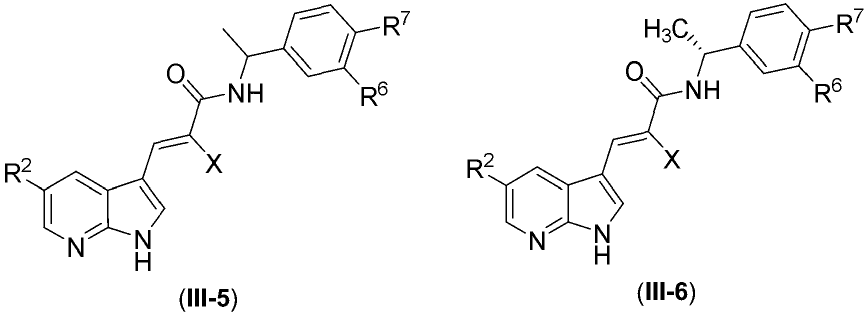 où **R¹, R², R³, R⁴, R⁶, R⁷** et X ont les significations définies dans la revendication 1.

6. Le composé conforme à l'une des revendications 1 à 5, où ledit composé correspond à l'une des formules (IV-1) - (IV-6) ou (V-1) - (V-6) 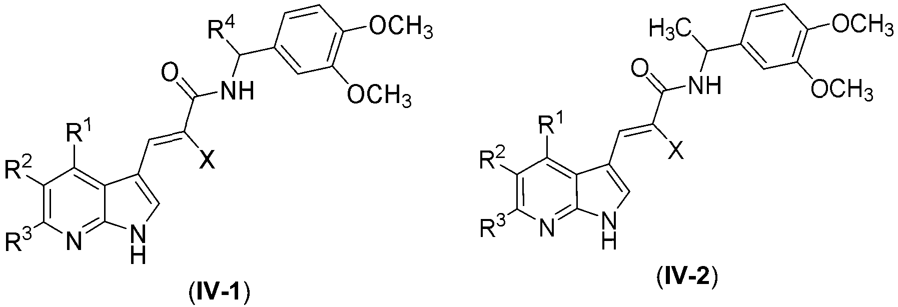 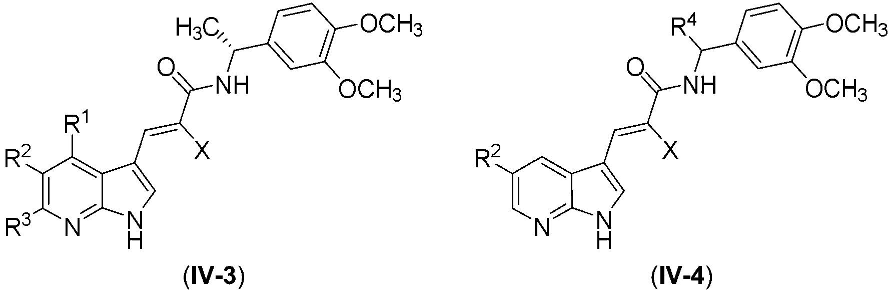 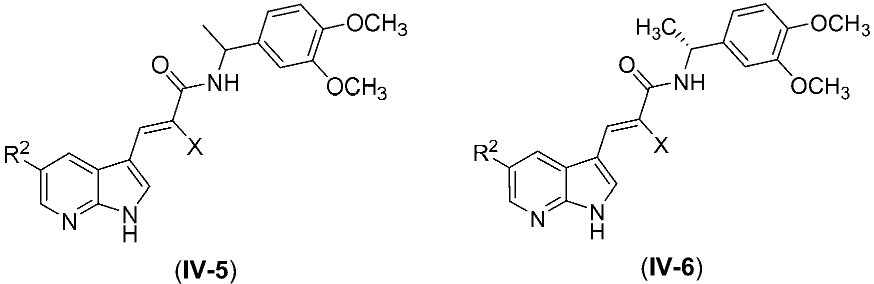 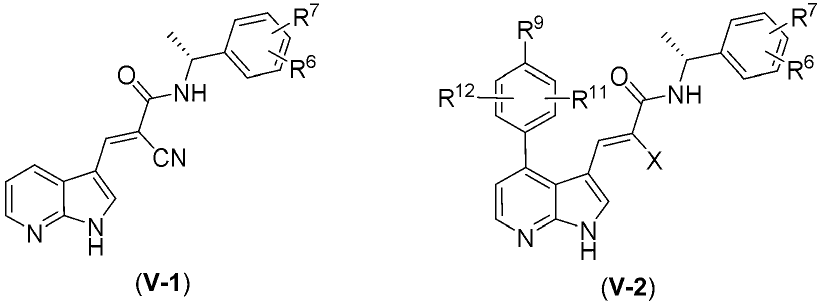 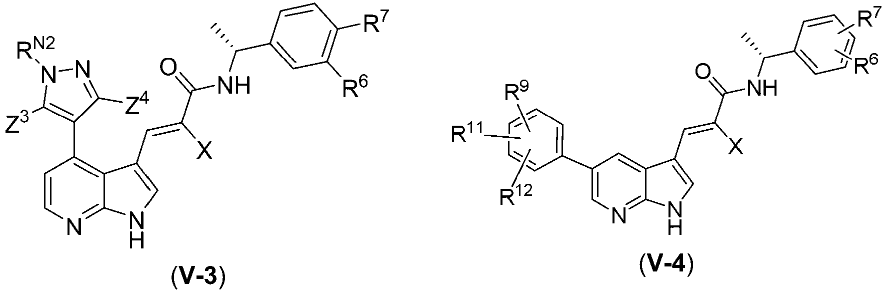 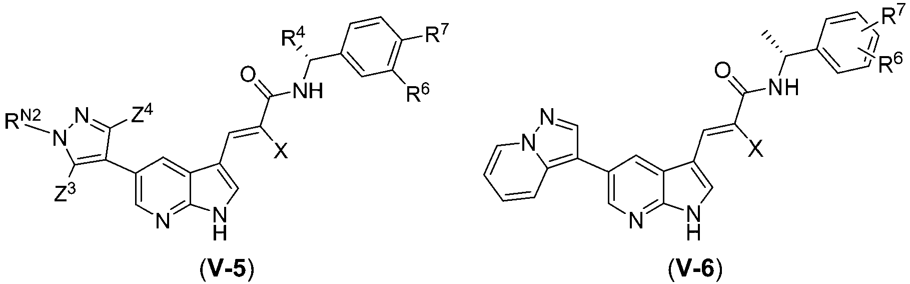 où **R¹, R², R³, R⁴, R⁶, R⁷, R⁹**, **R¹¹**, **R¹²**, **R^{N2}**, **X**, **Z³**, et **Z⁴** ont les significations définies dans la revendication 1.

7. Un composé sélectionné parmi les groupes comprenant :
| **Composé** | **Nomenclature** |
|---|---|
| **1** | (*R,E*)-2-cyano-*N*-(1-(3-fluorophényl)éthyl)-3-(1H-pyrrolo[2,3-b]pyridin-3-yl)acrylamide |
| **6** | (E)-N-benzyl-3-(5-chloro-1H-pyrrolo[2,3-b]pyridin-3-yl)acrylamide |
| **7** | (E)-3-(5-chloro-1H-pyrrolo[2,3-b]pyridin-3-yl)-N-(3-fluorobenzyl)acrylamide |
| **8** | (E)-3-(4-chloro-1H-pyrrolo[2,3-b]pyridin-3-yl)-N-(3-fluorobenzyl)acrylamide |
| **10** | (E)-N-(1-(4-fluorophényl)éthyl)-3-(1H-pyrrolo[2,3-b]pyridin-3-yl)acrylamide |
| **11** | (R,E)-3-(5-bromo-1H-pyrrolo[2,3-b]pyridin-3-yl)-N-(1-(3,4-diméthoxyphényl)éthyl)acrylamide |
| **12** | (R,E)-N-(1-(2-chlorophényl)éthyl)-2-cyano-3-(1H-pyrrolo[2,3-b]pyridin-3-yl)acrylamide |
| **13** | (R,E)-N-(1-(3-chlorophényl)éthyl)-2-cyano-3-(1H-pyrrolo[2,3-b]pyridin-3-yl)acrylamide |
| **14** | (S,E)-2-cyano-3-(1H-pyrrolo[2,3-b]pyridin-3-yl)-N-(2,2,2-trifluoro-1-phényléthyl)acrylamide |
| **15** | (R,E)-2-cyano-N-(1-(3,4-diméthoxyphényl)éthyl)-3-(1H-pyrrolo[2,3-b]pyridin-3-yl)acrylamide |
| **16** | (R,E)-2-cyano-N-(1-(3,4-difluorophényl)éthyl)-3-(1H-pyrrolo[2,3-b]pyridin-3-yl)acrylamide |
| **17** | (R,E)-2-cyano-N-(1-(2,4-difluorophényl)éthyl)-3-(1 H-pyrrolo[2,3-b]pyridin-3-yl)acrylamide |
| **18** | (R,E)-2-cyano-3-(1H-pyrrolo[2,3-b]pyridin-3-yl)-N-(1-(3-(trifluorométhyl)phényl)éthyl)acrylamide |
| **19** | (R,E)-2-cyano-N-(1-(4-méthoxyphényl)éthyl)-3-(1 H-pyrrolo[2,3-b]pyridin-3-yl)acrylamide |
| **20** | (R,E)-2-cyano-N-(1-(3,4-dichlorophényl)éthyl)-3-(1 H-pyrrolo[2,3-b]pyridin-3-yl)acrylamide |
| **21** | (E)-2-cyano-3-(1H-pyrrolo[2,3-b]pyridin-3-yl)-N-(4-sulfamoylbenzyl)acrylamide |
| **22** | (E)-2-cyano-3-(1H-pyrrolo[2,3-b]pyridin-3-yl)-N-(4-sulfamoylbenzyl)acrylamide |
| **23** | (E)-N-(5-bromo-2-méthylbenzyl)-2-cyano-3-(1H-pyrrolo[2,3-b]pyridin-3-yl)acrylamide |
| **24** | (E)-2-cyano-N-(4-(diméthylamino)benzyl)-3-(1H-pyrrolo[2,3-b]pyridin-3-yl)acrylamide |
| **25** | (E)-2-cyano-N-(2-(diméthylamino)benzyl)-3-(1H-pyrrolo[2,3-b]pyridin-3-yl)acrylamide |
| **26** | (E)-2-cyano-N-(4-cyanobenzyl)-3-(1H-pyrrolo[2,3-b]pyridin-3-yl)acrylamide |
| **27** | (E)-2-cyano-3-(1H-pyrrolo[2,3-b]pyridin-3-yl)-N-(3-(sulfamoylméthyl)benzyl)-acrylamide |
| **28** | (E)-3-((2-cyano-3-(1H-pyrrolo[2,3-b]pyridin-3-yl)acrylamido)méthyl)benzamide |
| **29** | (E)-2-cyano-N-(3-(méthylsulfonamido)benzyl)-3-(1H-pyrrolo[2,3-b]pyridin-3-yl)acrylamide |
| **30** | (E)-2-cyano-N-(3-(diméthylamino)benzyl)-3-(1H-pyrrolo[2,3-b]pyridin-3-yl)acrylamide |
| **31** | (E)-2-cyano-N-(3-cyanobenzyl)-3-(1H-pyrrolo[2,3-b]pyridin-3-yl)acrylamide |
| **32** | (E)-2-cyano-N-(2-hydroxy-1-phényléthyl)-3-(1H-pyrrolo[2,3-b]pyridin-3-yl)acrylamide |
| **33** | (R,E)-2-cyano-N-(1-(3-méthoxyphényl)éthyl)-3-(1H-pyrrolo[2,3-b]pyridin-3-yl)acrylamide |
| **34** | (E)-N-(3-acétamidobenzyl)-2-cyano-3-(1H-pyrrolo[2,3-b]pyridin-3-yl)acrylamide |
| **35** | (E)-2-cyano-N-(2-cyanobenzyl)-3-(1H-pyrrolo[2,3-b]pyridin-3-yl)acrylamide |
| **36** | (E)-2-cyano-N-(4-méthoxy-3-sulfamoylbenzyl)-3-(1H-pyrrolo[2,3-b]pyridin-3-yl)acrylamide |
| **37** | (E)-2-cyano-N-(2-(morpholinométhyl)benzyl)-3-(1H-pyrrolo[2,3-b]pyridin-3-yl)acrylamide |
| **38** | (E)-N-(1-(2-(1H-pyrazol-1-yl)phényl)éthyl)-2-cyano-3-(1H-pyrrolo[2,3-b]pyridin-3-yl)acrylamide |
| **39** | (E)-2-cyano-N-(2-(méthylsulfonyl)benzyl)-3-(1H-pyrrolo[2,3-b]pyridin-3-yl)acrylamide |
| **40** | (R,E)-2-cyano-N-(3-hydroxy-1-phénylpropyl)-3-(1H-pyrrolo[2,3-b]pyridin-3-yl)acrylamide |
| **41** | (E)-2-cyano-N-(2-(morpholinosulfonyl)benzyl)-3-(1H-pyrrolo[2,3-b]pyridin-3-yl)acrylamide |
| **42** | (E)-2-cyano-N-(2-(méthylsulfonamido)benzyl)-3-(1H-pyrrolo[2,3-b]pyridin-3-yl)acrylamide |
| **43** | (E)-2-cyano-N-(2-(N-méthylsulfamoyl)benzyl)-3-(1H-pyrrolo[2,3-b]pyridin-3-yl)acrylamide |
| **44** | (E)-2-cyano-N-(2-((2-oxopyrrolidin-1-yl)méthyl)benzyl)-3-(1H-pyrrolo[2,3-b]pyridin-3-yl)acrylamide |
| **45** | (E)-N-((1H-indazol-4-yl)méthyl)-2-cyano-3-(1H-pyrrolo[2,3-b]pyridin-3-yl)acrylamide |
| **46** | (E)-2-cyano-N-(3-morpholinobenzyl)-3-(1H-pyrrolo[2,3-b]pyridin-3-yl)acrylamide |
| **47** | (E)-N-(1-(3-bromophényl)-2,2,2-trifluoroéthyl)-2-cyano-3-(1H-pyrrolo[2,3-b]pyridin-3-yl)acrylamide |
| **48** | (E)-2-cyano-N-(3-(diméthylamino)-1-phénylpropyl)-3-(1H-pyrrolo[2,3-b]pyridin-3-yl)acrylamide |
| **49** | (E)-2-cyano-N-(cyano(phényl)méthyl)-3-(1H-pyrrolo[2,3-b]pyridin-3-yl)acrylamide |
| **50** | (E)-2-cyano-N-(2-(diméthylamino)-1-phényléthyl)-3-(1H-pyrrolo[2,3-b]pyridin-3-yl)acrylamide |
| **51** | (E)-2-cyano-N-(3-(diméthylamino)-1-(4-méthoxyphényl)propyl)-3-(1H-pyrrolo[2,3-b]pyridin-3-yl)acrylamide |
| **52** | (E)-2-cyano-N-(3-(diéthylamino)-1-phénylpropyl)-3-(1H-pyrrolo[2,3-b]pyridin-3-yl)acrylamide |
| **53** | (S,E)-N-(1-(3,4-diméthoxyphényl)-2-hydroxyéthyl)-3-(5-méthyl-1H-pyrrolo[2,3-b]pyridin-3-yl)acrylamide |
| **54** | (R,E)-N-(1-(4-fluoro-3-méthoxyphényl)éthyl)-3-(5-méthyl-1H-pyrrolo[2,3-b]pyridin-3-yl)acrylamide |
| **55** | (R,E)-N-(1-(3,4-diméthoxyphényl)éthyl)-3-(5-((1,1-dioxidotétrahydro-2H-thiopyran-4-yl)oxy)-1H-pyrrolo[2,3-b]pyridin-3-yl)acrylamide |
| **56** | (R,E)-N-(1-(3,4-diméthoxyphényl)éthyl)-3-(5-(2-(diméthylamino)éthoxy)-1H-pyrrolo[2,3-b]pyridin-3-yl)acrylamide |
| **57** | (E)-2-cyano-N-(4-fluorobenzyl)-3-(1H-pyrrolo[2,3-b]pyridin-3-yl)acrylamide |
| **58** | (E)-N-(4-chlorobenzyl)-2-cyano-3-(1H-pyrrolo[2,3-b]pyridin-3-yl)acrylamide |
| **59** | (E)-2-cyano-3-(1H-pyrrolo[2,3-b]pyridin-3-yl)-N-(4-(trifluorométhyl)benzyl)acrylamide |
| **60** | (E)-N-(3-chlorobenzyl)-2-cyano-3-(1H-pyrrolo[2,3-b]pyridin-3-yl)acrylamide |
| **61** | (E)-2-cyano-3-(1H-pyrrolo[2,3-b]pyridin-3-yl)-N-(3-(trifluorométhyl)benzyl)acrylamide |
| **62** | (E)-N-(2-chlorobenzyl)-2-cyano-3-(1H-pyrrolo[2,3-b]pyridin-3-yl)acrylamide |
| **63** | (E)-2-cyano-N-(1-(4-fluorophényl)éthyl)-3-(1H-pyrrolo[2,3-b]pyridin-3-yl)acrylamide |
| **64** | (R,E)-2-cyano-N-(1-phényléthyl)-3-(1H-pyrrolo[2,3-b]pyridin-3-yl)acrylamide |
| **65** | (E)-2-cyano-N-(2,4-difluorobenzyl)-3-(1H-pyrrolo[2,3-b]pyridin-3-yl)acrylamide |
| **66** | (E)-2-cyano-N-(3,4-difluorobenzyl)-3-(1H-pyrrolo[2,3-b]pyridin-3-yl)acrylamide |
| **67** | (E)-2-cyano-N-(3,5-difluorobenzyl)-3-(1H-pyrrolo[2,3-b]pyridin-3-yl)acrylamide |
| **68** | (R,E)-2-cyano-N-(1-phénylpropyl)-3-(1H-pyrrolo[2,3-b]pyridin-3-yl)acrylamide |
| **69** | (R,E)-2-cyano-N-(1-(4-fluorophényl)éthyl)-3-(1H-pyrrolo[2,3-b]pyridin-3-yl)acrylamide |
| **70** | (R,E)-N-(1-(4-chlorophényl)éthyl)-2-cyano-3-(1H-pyrrolo[2,3-b]pyridin-3-yl)acrylamide |
| **71** | (E)-N-benzyl-2-cyano-3-(5-(trifluorométhyl)-1H-pyrrolo[2,3-b]pyridin-3-yl)acrylamide |
| **72** | (E)-3-(5-chloro-1H-pyrrolo[2,3-b]pyridin-3-yl)-2-cyano-N-(4-fluorobenzyl)acrylamide |
| **73** | (E)-N-benzyl-3-(5-chloro-1H-pyrrolo[2,3-b]pyridin-3-yl)-2-cyanoacrylamide |
| **74** | (E)-3-(5-chloro-1H-pyrrolo[2,3-b]pyridin-3-yl)-N-(2-chlorobenzyl)-2-cyanoacrylamide |
| **75** | (E)-3-(5-chloro-1H-pyrrolo[2,3-b]pyridin-3-yl)-2-cyano-N-(3,5-difluorobenzyl)acrylamide |
| **76** | (R,E)-3-(5-chloro-1H-pyrrolo[2,3-b]pyridin-3-yl)-2-cyano-N-(1-(4-fluorophényl)éthyl)acrylamide |
| **77** | (R,E)-3-(5-chloro-1H-pyrrolo[2,3-b]pyridin-3-yl)-2-cyano-N-(1-phénylpropyl)acrylamide |
| **78** | (R,E)-3-(4-chloro-1H-pyrrolo[2,3-b]pyridin-3-yl)-2-cyano-N-(1-(4-fluorophényl)éthyl)acrylamide |
| **79** | (R,E)-3-(5-bromo-1H-pyrrolo[2,3-b]pyridin-3-yl)-2-cyano-N-(1-(4-fluorophényl)éthyl)acrylamide |
| **80** | (R,E)-3-(5-bromo-1H-pyrrolo[2,3-b]pyridin-3-yl)-2-cyano-N-(1-(3,4-diméthoxyphényl)éthyl)acrylamide |
| **81** | (R,E)-2-cyano-N-(1-(3,4-diméthoxyphényl)éthyl)-3-(5-hydroxy-1H-pyrrolo[2,3-b]pyridin-3-yl)acrylamide |
| **82** | (R,E)-2-cyano-N-(1-(3,4-diméthoxyphényl)éthyl)-3-(5-((tétrahydro-2H-pyran-4-yl)oxy)-1H-pyrrolo[2,3-b]pyridin-3-yl)acrylamide |
| **83** | (R,E)-2-cyano-N-(1-(3,4-diméthoxyphényl)éthyl)-3-(5-(4-méthylpipérazine-1-carbonyl)-1H-pyrrolo[2,3-b]pyridin-3-yl)acrylamide |
| **84** | (R,E)-2-cyano-N-(1-(3,4-diméthoxyphényl)éthyl)-3-(5-morpholino-1H-pyrrolo[2,3-b]pyridin-3-yl)acrylamide |
| **85** | (R,E)-2-cyano-N-(1-(3,4-diméthoxyphényl)éthyl)-3-(5-morpholino-1H-pyrrolo[2,3-b]pyridin-3-yl)acrylamide |
| **86** | (R,E)-2-cyano-3- 5-cyano-1H-pyrrolo[2,3-b]pyridin-3-yl)-N-(1-(3,4-diméthoxyphényl)éthyl)acrylamide |
| **87** | (R,E)-2-cyano-N-(1-(3,4-diméthoxyphényl)éthyl)-3-(5-méthyl-1H-pyrrolo[2,3-b]pyridin-3-yl)acrylamide |
| **88** | (R,E)-2-cyano-N-(1-(3,4-diméthoxyphényl)éthyl)-3-(5-(phénylamino)-1H-pyrrolo[2,3-b]pyridin-3-yl)acrylamide |
| **89** | (R,E)-2-cyano-N-(1-(3,4-diméthoxyphényl)éthyl)-3-(5-(1-(pipéridin-4-yl)-1H-pyrazol-4-yl)-1H-pyrrolo[2,3-b]pyridin-3-yl)acrylamide |
| **90** | (R,E)-3-(6-bromo-1H-pyrrolo[2,3-b]pyridin-3-yl)-2-cyano-N-(1-(3,4-diméthoxyphényl)éthyl)acrylamide |
| **91** | (R,E)-2-cyano-3-(5-(1-méthyl-1H-pyrazol-4-yl)-1H-pyrrolo[2,3-b]pyridin-3-yl)-N-(1-phényléthyl)acrylamide |
| **92** | (S,E)-2-cyano-N-(1-(3,4-diméthoxyphényl)-2-hydroxyéthyl)-3-(5-(1-méthyl-1H-pyrazol-4-yl)-1H-pyrrolo[2,3-b]pyridin-3-yl)acrylamide |
| **93** | (R,E)-2-cyano-N-(1-(4-fluorophényl)éthyl)-3-(5-(1-méthyl-1H-pyrazol-4-yl)-1H-pyrrolo[2,3-b]pyridin-3-yl)acrylamide |
| **94** | (R,E)-2-cyano-N-(1-(4-fluoro-3-méthoxyphényl)éthyl)-3-(5-(1-méthyl-1H-pyrazol-4-yl)-1H-pyrrolo[2,3-b]pyridin-3-yl)acrylamide |
| **95** | (R,E)-N-(1-(4-chlorophényl)éthyl)-2-cyano-3-(5-(1-méthyl-1H-pyrazol-4-yl)-1H-pyrrolo[2,3-b]pyridin-3-yl)acrylamide |
| **96** | (R,E)-N-(1-(3-chlorophényl)éthyl)-2-cyano-3-(5-(1-méthyl-1H-pyrazol-4-yl)-1H-pyrrolo[2,3-b]pyridin-3-yl)acrylamide |
| **97** | (R,E)-2-cyano-3-(4-méthyl-5-(1-méthyl-1 H-pyrazol-4-yl)-1 H-pyrrolo[2,3-b]pyridin-3-yl)-N-(1-phényléthyl)acrylamide |
| **98** | (R,E)-2-cyano-N-(1-(4-fluorophényl)éthyl)-3-(4-méthyl-5-(1-méthyl-1H-pyrazol-4-yl)-1H-pyrrolo[2,3-b]pyridin-3-yl)acrylamide |
| **99** | (R,E)-2-cyano-N-(1-(3,4-diméthoxyphényl)éthyl)-3-(4-méthyl-5-(1-méthyl-1H-pyrazol-4-yl)-1H-pyrrolo[2,3-b]pyridin-3-yl)acrylamide |
| **100** | (R,E)-N-(1-(4-chlorophényl)éthyl)-2-cyano-3-(4-méthyl-5-(1-méthyl-1H-pyrazol-4-yl)-1H-pyrrolo[2,3-b]pyridin-3-yl)acrylamide |
| **101** | (R,E)-2-cyano-N-(1-(3,4-diméthoxyphényl)éthyl)-3-(5-((1,3-diméthyl-1H-pyrazol-5-yl)méthoxy)-1H-pyrrolo[2,3-b]pyridin-3-yl)acrylamide |
| **102** | (R,E)-2-cyano-N-(1-(3,4-diméthoxyphényl)éthyl)-3-(5-isopropoxy-1H-pyrrolo[2,3-b]pyridin-3-yl)acrylamide |
| **103** | (E)-2-cyano-N-((R)-1-(3,4-diméthoxyphényl)éthyl)-3-(5-((tétrahydrofuran-3-yl)oxy)-1H-pyrrolo[2,3-b]pyridin-3-yl)acrylamide |
| **104** | (R,E)-2-cyano-N-(1-(3,4-diméthoxyphényl)éthyl)-3-(5-(2-(diméthylamino)éthoxy)-1H-pyrrolo[2,3-b]pyridin-3-yl)acrylamide |
| **105** | (R,E)-2-cyano-N-(1-(3,4-diméthoxyphényl)éthyl)-3-(5-((1,1-dioxidotétrahydro-2H-thiopyran-4-yl)oxy)-1H-pyrrolo[2,3-b]pyridin-3-yl)acrylamide |
| **106** | (R,E)-2-cyano-N-(1-(3,4-diméthoxyphényl)éthyl)-3-(5-((1-méthyl-1H-imidazol-2-yl)méthoxy)-1H-pyrrolo[2,3-b]pyridin-3-yl)acrylamide |
| **107** | (E)-2-cyano-N-((R)-1-(3,4-diméthoxyphényl)éthyl)-3-(5-(((R)-tétrahydrofuran-3-yl)oxy)-1H-pyrrolo[2,3-b]pyridin-3-yl)acrylamide |
| **108** | (E)-2-cyano-N-((R)-1-(3,4-diméthoxyphényl)éthyl)-3-(5-(((S)-tétrahydrofuran-3-yl)oxy)-1H-pyrrolo[2,3-b]pyridin-3-yl)acrylamide |
| **109** | (E)-3-(5-((S)-sec-butoxy)-1H-pyrrolo[2,3-b]pyridin-3-yl)-2-cyano-N-((R)-1-(3,4-diméthoxyphényl)éthyl)acrylamide |
| **110** | (E)-3-(5-((S)-sec-butoxy)-1H-pyrrolo[2,3-b]pyridin-3-yl)-2-cyano-N-((R)-1-(3,4-diméthoxyphényl)éthyl)acrylamide |
| **111** | (R,E)-2-cyano-N-(1-(3,4-diméthoxyphényl)éthyl)-3-(5-((tétrahydro-2H-pyran-4-yl)oxy)-1H-pyrrolo[2,3-b]pyridin-3-yl)acrylamide |
| **112** | (R,E)-2-cyano-N-(1-(3,4-diméthoxyphényl)éthyl)-3-(5-(4-(4-méthylpipérazin-1-yl)phényl)-1H-pyrrolo[2,3-b]pyridin-3-yl)acrylamide |
| **113** | (R,E)-N-(1-(3,4-diméthoxyphényl)éthyl)-3-(5-(4-(4-méthylpipérazin-1-yl)phényl)-1H-pyrrolo[2,3-b]pyridin-3-yl)acrylamide |
| **114** | (R,E)-N-(1-(3,4-diméthoxyphényl)éthyl)-3-(5-(3-(méthylsulfonyl)phényl)-1H-pyrrolo[2,3-b]pyridin-3-yl)acrylamide |
| **115** | (R,E)-3-(5-(3,5-difluorophényl)-1H-pyrrolo[2,3-b]pyridin-3-yl)-N-(1-(3,4-diméthoxyphényl)éthyl)acrylamide |
| **116** | (R,E)-2-cyano-N-(1-(3,4-diméthoxyphényl)éthyl)-3-(5-(4-(4-méthylpipérazin-1-yl)phényl)-1H-pyrrolo[2,3-b]pyridin-3-yl)acrylamide |
| **117** | (R,E)-2-cyano-N-(1-(3,4-diméthoxyphényl)éthyl)-3-(5-(4-((diméthylamino)méthyl)phényl)-1H-pyrrolo[2,3-b]pyridin-3-yl)acrylamide |
| **118** | (R,E)-2-cyano-N-(1-(3,4-diméthoxyphényl)éthyl)-3-(5-(3-((4-méthylpipérazin-1-yl)méthyl)phényl)-1H-pyrrolo[2,3-b]pyridin-3-yl)acrylamide |
| **119** | (R,E)-2-cyano-N-(1-(3,4-diméthoxyphényl)éthyl)-3-(5-(3-((4-méthylpipérazin-1-yl)méthyl)phényl)-1H-pyrrolo[2,3-b]pyridin-3-yl)acrylamide |
| **120** | (R,E)-2-cyano-N-(1-(3,4-diméthoxyphényl)éthyl)-3-(5-(pyrimidin-5-yl)-1H-pyrrolo[2,3-b]pyridin-3-yl)acrylamide |
| **121** | (R,E)-2-cyano-N-(1-(3,4-diméthoxyphényl)éthyl)-3-(5-(4-((4-éthylpipérazin-1-yl)sulfonyl)phényl)-1H-pyrrolo[2,3-b]pyridin-3-yl)acrylamide |
| **122** | (R,E)-2-cyano-N-(1-(3,4-diméthoxyphényl)éthyl)-3-(5-(3-(4-méthylpipérazin-1-yl)phényl)-1H-pyrrolo[2,3-b]pyridin-3-yl)acrylamide |
| **123** | (R,E)-2-cyano-N-(1-(3,4-diméthoxyphényl)éthyl)-3-(5-(3-(4-méthylpipérazin-1-yl)phényl)-1H-pyrrolo[2,3-b]pyridin-3-yl)acrylamide |
| **124** | (R,E)-2-cyano-N-(1-(3,4-diméthoxyphényl)éthyl)-3-(5-(4-fluoro-2-méthoxyphényl)-1H-pyrrolo[2,3-b]pyridin-3-yl)acrylamide |
| **125** | (R,E)-2-cyano-N-(1-(3,4-diméthoxyphényl)éthyl)-3-(5-(2-méthoxyphényl)-1H-pyrrolo[2,3-b]pyridin-3-yl)acrylamide |
| **126** | (R,E)-4-(3-(2-cyano-3-((1-(3,4-diméthoxyphényl)éthyl)amino)-3-oxoprop-1-èn-1-yl)-1H-pyrrolo[2,3-b]pyridin-5-yl)benzamide |
| **127** | (R,E)-2-cyano-N-(1-(3,4-diméthoxyphényl)éthyl)-3-(5-(3-(morpholine-4-carbonyl)phényl)-1H-pyrrolo[2,3-b]pyridin-3-yl)acrylamide |
| **128** | (R,E)-2-cyano-N-(1-(3,4-diméthoxyphényl)éthyl)-3-(5-(3-(diméthylamino)phényl)-1H-pyrrolo[2,3-b]pyridin-3-yl)acrylamide |
| **129** | (R,E)-2-cyano-N-(1-(3,4-diméthoxyphényl)éthyl)-3-(5-(2-(pipéridin-1-ylméthyl)phényl)-1H-pyrrolo[2,3-b]pyridin-3-yl)acrylamide |
| **130** | (R,E)-2-cyano-N-(1-(3,4-diméthoxyphényl)éthyl)-3-(5-(2-(pipéridin-1-ylméthyl)phényl)-1H-pyrrolo[2,3-b]pyridin-3-yl)acrylamide |
| **131** | (R,E)-2-cyano-N-(1-(3,4-diméthoxyphényl)éthyl)-3-(5-(pyridin-3-yl)-1H-pyrrolo[2,3-b]pyridin-3-yl)acrylamide |
| **132** | (R,E)-2-cyano-N-(1-(3,4-diméthoxyphényl)éthyl)-3-(5-(4-(1-méthylpipéridin-4-yl)phényl)-1H-pyrrolo[2,3-b]pyridin-3-yl)acrylamide |
| **133** | (R,E)-2-cyano-N-(1-(3,4-diméthoxyphényl)éthyl)-3-(5-(4-(1-méthylpipéridin-4-yl)phényl)-1H-pyrrolo[2,3-b]pyridin-3-yl)acrylamide |
| **134** | (R,E)-2-cyano-N-(1-(3,4-diméthoxyphényl)éthyl)-3-(5-(3-(3-(diméthylamino)propoxy)phényl)-1H-pyrrolo[2,3-b]pyridin-3-yl)acrylamide |
| **135** | (R,E)-2-cyano-N-(1-(3,4-diméthoxyphényl)éthyl)-3-(5-(4-(2-(diméthylamino)éthyl)phényl)-1H-pyrrolo[2,3-b]pyridin-3-yl)acrylamide |
| **136** | (R,Z)-2-cyano-N-(1-(3,4-diméthoxyphényl)éthyl)-3-(5-(4-(2-(diméthylamino)éthyl)phényl)-1H-pyrrolo[2,3-b]pyridin-3-yl)acrylamide |
| **137** | (R,E)-2-cyano-N-(1-(3,4-diméthoxyphényl)éthyl)-3-(5-(3-méthoxyphényl)-1H-pyrrolo[2,3-b]pyridin-3-yl)acrylamide |
| **138** | (R,E)-2-cyano-N-(1-(3,4-diméthoxyphényl)éthyl)-3-(5-(3-méthoxyphényl)-1H-pyrrolo[2,3-b]pyridin-3-yl)acrylamide |
| **139** | (R,E)-2-cyano-N-(1-(3,4-diméthoxyphényl)éthyl)-3-(5-(2-(morpholinométhyl)phényl)-1H-pyrrolo[2,3-b]pyridin-3-yl)acrylamide |
| **140** | (R,Z)-2-cyano-3-(5-(4-cyanophényl)-1H-pyrrolo[2,3-b]pyridin-3-yl)-N-(1-(3,4-diméthoxyphényl)éthyl)acrylamide |
| **141** | (R,E)-3-(5-(3-((4-acétylpipérazin-1-yl)méthyl)phényl)-1H-pyrrolo[2,3-b]pyridin-3-yl)-2-cyano-N-(1-(3,4-diméthoxyphényl)éthyl)acrylamide |
| **142** | (R,E)-3-(5-(3-((4-acétylpipérazin-1-yl)méthyl)phényl)-1H-pyrrolo[2,3-b]pyridin-3-yl)-2-cyano-N-(1-(3,4-diméthoxyphényl)éthyl)acrylamide |
| **143** | (R,E)-3-(5-(4-acétamidophényl)-1H-pyrrolo[2,3-b]pyridin-3-yl)-2-cyano-N-(1-(3,4-diméthoxyphényl)éthyl)acrylamide |
| **144** | (R,E)-2-cyano-N-(1-(3,4-diméthoxyphényl)éthyl)-3-(5-(pyridin-4-yl)-1H-pyrrolo[2,3-b]pyridin-3-yl)acrylamide |
| **145** | (R,E)-2-cyano-N-(1-(3,4-diméthoxyphényl)éthyl)-3-(5-(naphthalèn-2-yl)-1H-pyrrolo[2,3-b]pyridin-3-yl)acrylamide |
| **146** | (R,Z)-2-cyano-3-(5-(3,4-diméthoxyphényl)-1H-pyrrolo[2,3-b]pyridin-3-yl)-N-(1-(3,4-diméthoxyphényl)éthyl)acrylamide |
| **147** | (R,E)-2-cyano-N-(1-(3,4-diméthoxyphényl)éthyl)-3-(5-(6-(diméthylamino)pyridin-3-yl)-1H-pyrrolo[2,3-b]pyridin-3-yl)acrylamide |
| **148** | (R,E)-2-cyano-N-(1-(3,4-diméthoxyphényl)éthyl)-3-(5-(6-(diméthylamino)pyridin-3-yl)-1H-pyrrolo[2,3-b]pyridin-3-yl)acrylamide |
| **149** | (R,E)-2-cyano-N-(1-(3,4-diméthoxyphényl)éthyl)-3-(5-(3-((diméthylamino)méthyl)phényl)-1H-pyrrolo[2,3-b]pyridin-3-yl)acrylamide |
| **150** | (R,E)-2-cyano-N-(1-(3,4-diméthoxyphényl)éthyl)-3-(5-(3-(N,N-diméthylsulfamoyl)phényl)-1H-pyrrolo[2,3-b]pyridin-3-yl)acrylamide |
| **151** | (R,E)-2-cyano-N-(1-(3,4-diméthoxyphényl)éthyl)-3-(5-(3-(méthylsulfonamido)phényl)-1H-pyrrolo[2,3-b]pyridin-3-yl)acrylamide |
| **152** | (R,E)-2-cyano-N-(1-(3,4-diméthoxyphényl)éthyl)-3-(5-(3-(méthylsulfonamido)phényl)-1H-pyrrolo[2,3-b]pyridin-3-yl)acrylamide |
| **153** | (R,E)-2-cyano-N-(1-(3,4-diméthoxyphényl)éthyl)-3-(5-(3-(2-(diméthylamino)éthoxy)phényl)-1H-pyrrolo[2,3-b]pyridin-3-yl)acrylamide |
| **154** | (R,E)-2-cyano-N-(1-(3,4-diméthoxyphényl)éthyl)-3-(5-(2-méthoxy-4-(4-méthylpipérazin-1-yl)phényl)-1H-pyrrolo[2,3-b]pyridin-3-yl)acrylamide |
| **155** | (R,E)-2-cyano-N-(1-(3,4-diméthoxyphényl)éthyl)-3-(5-(4-(2-(diméthylamino)éthoxy)phényl)-1H-pyrrolo[2,3-b]pyridin-3-yl)acrylamide |
| **156** | (R,E)-2-cyano-3-(5-(cyclohex-1-èn-1-yl)-1H-pyrrolo[2,3-b]pyridin-3-yl)-N-(1-(3,4-diméthoxyphényl)éthyl)acrylamide |
| **157** | (R,E)-2-cyano-3-(5-(cyclohex-1-èn-1-yl)-1H-pyrrolo[2,3-b]pyridin-3-yl)-N-(1-(3,4-diméthoxyphényl)éthyl)acrylamide |
| **158** | (R,E)-2-cyano-3-(5-(cyclopent-1-én-1-yl)-1H-pyrrolo[2,3-b]pyridin-3-yl)-N-(1-(3,4-diméthoxyphényl)éthyl)acrylamide |
| **159** | (R,E)-2-cyano-3-(5-(cyclopent-1-én-1-yl)-1H-pyrrolo[2,3-b]pyridin-3-yl)-N-(1-(3,4-diméthoxyphényl)éthyl)acrylamide |
| **160** | (R,E)-3-(5-(1-benzyl-1H-pyrazol-4-yl)-1H-pyrrolo[2,3-b]pyridin-3-yl)-2-cyano-N-(1-(3,4-diméthoxyphényl)éthyl)acrylamide |
| **161** | (R,E)-2-cyano-N-(1-(3,4-diméthoxyphényl)éthyl)-3-(5-(1-isobutyl-1H-pyrazol-4-yl)-1H-pyrrolo[2,3-b]pyridin-3-yl)acrylamide |
| **162** | (R,E)-2-cyano-N-(1-(3,4-diméthoxyphényl)éthyl)-3-(5-(1-isobutyl-1H-pyrazol-4-yl)-1H-pyrrolo[2,3-b]pyridin-3-yl)acrylamide |
| **163** | (R,E)-2-cyano-N-(1-(3,4-diméthoxyphényl)éthyl)-3-(5-(2-méthylimidazo[1,2-a]pyridin-6-yl)-1H-pyrrolo[2,3-b]pyridin-3-yl)acrylamide |
| **164** | (R,E)-3-(5-(1H-pyrazol-4-yl)-1H-pyrrolo[2,3-b]pyridin-3-yl)-2-cyano-N-(1-(3,4-diméthoxyphényl)éthyl)acrylamide |
| **165** | (R,E)-2-cyano-N-(1-(3,4-diméthoxyphényl)éthyl)-3-(5-(1-méthyl-1H-pyrazol-4-yl)-1H-pyrrolo[2,3-b]pyridin-3-yl)acrylamide |
| **166** | (R,E)-2-cyano-N-(1-(3,4-diméthoxyphényl)éthyl)-3-(5-(1-méthyl-1H-imidazol-4-yl)-1H-pyrrolo[2,3-b]pyridin-3-yl)acrylamide |
| **167** | (R,E)-2-cyano-N-(1-(3,4-diméthoxyphényl)éthyl)-3-(5-(1-méthyl-1H-imidazol-4-yl)-1H-pyrrolo[2,3-b]pyridin-3-yl)acrylamide |
| **168** | (R,E)-2-cyano-N-(1-(3,4-diméthoxyphényl)éthyl)-3-(5-(1,3-diméthyl-1H-pyrazol-4-yl)-1H-pyrrolo[2,3-b]pyridin-3-yl)acrylamide |
| **169** | (R,E)-2-cyano-N-(1-(3,4-diméthoxyphényl)éthyl)-3-(5-(1,3-diméthyl-1H-pyrazol-4-yl)-1H-pyrrolo[2,3-b]pyridin-3-yl)acrylamide |
| **170** | (R,E)-2-cyano-N-(1-(3,4-diméthoxyphényl)éthyl)-3-(5-(4-fluoro-3-(méthylsulfonyl)phényl)-1H-pyrrolo[2,3-b]pyridin-3-yl)acrylamide |
| **171** | (R,E)-2-cyano-N-(1-(3,4-diméthoxyphényl)éthyl)-3-(4-(4-(4-méthylpipérazin-1-yl)phényl)-1H-pyrrolo[2,3-b]pyridin-3-yl)acrylamide |
| **172** | (R,E)-2-cyano-N-(1-(3,4-diméthoxyphényl)éthyl)-3-(4-(3-(4-méthylpipérazin-1-yl)phényl)-1H-pyrrolo[2,3-b]pyridin-3-yl)acrylamide |
| **173** | (R,E)-2-cyano-N-(1-(3,4-diméthoxyphényl)éthyl)-3-(4-(4-((4-méthylpipérazin-1-yl)méthyl)phényl)-1H-pyrrolo[2,3-b]pyridin-3-yl)acrylamide |
| **174** | (R,E)-2-cyano-N-(1-(3,4-diméthoxyphényl)éthyl)-3-(4-(4-((4-méthylpipérazin-1-yl)méthyl)phényl)-1H-pyrrolo[2,3-b]pyridin-3-yl)acrylamide |
| **175** | (R,E)-2-cyano-N-(1-(3,4-diméthoxyphényl)éthyl)-3-(4-(3-((4-méthylpipérazin-1-yl)méthyl)phényl)-1H-pyrrolo[2,3-b]pyridin-3-yl)acrylamide |
| **176** | (R,E)-2-cyano-N-(1-(3,4-diméthoxyphényl)éthyl)-3-(4-(3-((diméthylamino)méthyl)phényl)-1H-pyrrolo[2,3-b]pyridin-3-yl)acrylamide |
| **177** | (R,E)-2-cyano-N-(1-(3,4-diméthoxyphényl)éthyl)-3-(4-(3-((diméthylamino)méthyl)phényl)-1H-pyrrolo[2,3-b]pyridin-3-yl)acrylamide |
| **178** | (R,E)-2-cyano-N-(1-(3,4-diméthoxyphényl)éthyl)-3-(4-(4-((diméthylamino)méthyl)phényl)-1H-pyrrolo[2,3-b]pyridin-3-yl)acrylamide |
| **179** | (R,E)-2-cyano-3-(4-(3,5-difluorophényl)-1H-pyrrolo[2,3-b]pyridin-3-yl)-N-(1-(3,4-diméthoxyphényl)éthyl)acrylamide |
| **180** | (R,E)-2-cyano-N-(1-(4-fluorophényl)éthyl)-3-(5-phényl-1H-pyrrolo[2,3-b]pyridin-3-yl)acrylamide |
| **181** | (R,E)-2-cyano-N-(1-(4-fluorophényl)éthyl)-3-(5-(3-(4-méthylpipérazin-1-yl)phényl)-1H-pyrrolo[2,3-b]pyridin-3-yl)acrylamide |
| **182** | (R,E)-2-cyano-N-(1-(4-fluorophényl)éthyl)-3-(5-(3-(4-méthylpipérazin-1-yl)phényl)-1H-pyrrolo[2,3-b]pyridin-3-yl)acrylamide |
| **183** | (R,E)-3-(5-(4-(4-acétylpipérazin-1-yl)phényl)-1H-pyrrolo[2,3-b]pyridin-3-yl)-N-(1-(3,4-diméthoxyphényl)éthyl)acrylamide |
| **184** | (R, E)-N-(1-(3,4-diméthoxyphényl)éthyl)-3-(5-(3-((4-méthylpipérazin-1-yl)méthyl)phényl)-1H-pyrrolo[2,3-b]pyridin-3-yl)acrylamide |
| **185** | (R, E)-N-(1-(3,4-diméthoxyphényl)éthyl)-3-(5-(4-((diméthylamino)méthyl)phényl)-1H-pyrrolo[2,3-b]pyridin-3-yl)acrylamide |
| **186** | (R,E)-N-(1-(3,4-diméthoxyphényl)éthyl)-3-(5-(pyridin-3-yl)-1H-pyrrolo[2,3-b]pyridin-3-yl)acrylamide |
| **187** | (R,E)-N-(1-(3,4-diméthoxyphényl)éthyl)-3-(5-(pyridin-3-yl)-1H-pyrrolo[2,3-b]pyridin-3-yl)acrylamide |
| **188** | (R,E)-N-(1-(3,4-diméthoxyphényl)éthyl)-3-(5-(4-(méthylsulfonamido)phényl)-1H-pyrrolo[2,3-b]pyridin-3-yl)acrylamide |
| **189** | (R, E)-N-(1-(3,4-diméthoxyphényl)éthyl)-3-(5-(3-(3-(diméthylamino)propoxy)phényl)-1H-pyrrolo[2,3-b]pyridin-3-yl)acrylamide |
| **190** | (R,E)-3-(3-(3-((1-(3,4-diméthoxyphényl)éthyl)amino)-3-oxoprop-1-èn-1-yl)-1H-pyrrolo[2,3-b]pyridin-5-yl)benzamide |
| **191** | (E)-N-((R)-1-(3,4-diméthoxyphényl)éthyl)-3-(5-(3-(éthylsulfinyl)phényl)-1H-pyrrolo[2,3-b]pyridin-3-yl)acrylamide |
| **192** | (E)-N-((R)-1-(3,4-diméthoxyphényl)éthyl)-3-(5-(3-(éthylsulfinyl)phényl)-1H-pyrrolo[2,3-b]pyridin-3-yl)acrylamide |
| **193** | (R, E)-N-(1-(3,4-diméthoxyphényl)éthyl)-3-(5-(3-(2-(diméthylamino)éthyl)phényl)-1H-pyrrolo[2,3-b]pyridin-3-yl)acrylamide |
| **194** | (R,E)-N-(1-(3,4-diméthoxyphényl)éthyl)-3-(5-(3-(méthylsulfonamido)phényl)-1H-pyrrolo[2,3-b]pyridin-3-yl)acrylamide |
| **195** | (R,E)-N-(1-(3,4-diméthoxyphényl)éthyl)-3-(5-(3-(diméthylamino)phényl)-1H-pyrrolo[2,3-b]pyridin-3-yl)acrylamide |
| **196** | (R,E)-N-(1-(3,4-diméthoxyphényl)éthyl)-3-(5-(4-(1-méthylpipéridin-4-yl)phényl)-1H-pyrrolo[2,3-b]pyridin-3-yl)acrylamide |
| **197** | (R, E)-N-(1-(3,4-diméthoxyphényl)éthyl)-3-(5-(4-(2-(diméthylamino)éthoxy)phényl)-1H-pyrrolo[2,3-b]pyridin-3-yl)acrylamide |
| **198** | (R,E)-N-(1-(3,4-diméthoxyphényl)éthyl)-3-(5-(3-(N,N-diméthylsulfamoyl)phényl)-1H-pyrrolo[2,3-b]pyridin-3-yl)acrylamide |
| **199** | (R,E)-3-(5-(3-(1H-imidazol-2-yl)phényl)-1H-pyrrolo[2,3-b]pyridin-3-yl)-N-(1-(3,4-diméthoxyphényl)éthyl)acrylamide |
| **200** | (R,E)-4-(3-(3-((1-(3,4-diméthoxyphényl)éthyl)amino)-3-oxoprop-1-èn-1-yl)-1H-pyrrolo[2,3-b]pyridin-5-yl)benzamide |
| **201** | (R,E)-N-(1-(3,4-diméthoxyphényl)éthyl)-3-(5-(4-fluoro-2-méthoxyphényl)-1H-pyrrolo[2,3-b]pyridin-3-yl)acrylamide |
| **202** | (R,E)-N-(1-(3,4-diméthoxyphényl)éthyl)-3-(5-(1,5-diméthyl-1H-pyrazol-4-yl)-1H-pyrrolo[2,3-b]pyridin-3-yl)acrylamide |
| **203** | (R,E)-3-(5-(3-(acétamidométhyl)phényl)-1H-pyrrolo[2,3-b]pyridin-3-yl)-N-(1-(3,4-diméthoxyphényl)éthyl)acrylamide |
| **204** | (R,E)-N-(1-(3,4-diméthoxyphényl)éthyl)-3-(5-(1-méthyl-1H-pyrazol-4-yl)-1H-pyrrolo[2,3-b]pyridin-3-yl)acrylamide |
| **205** | (R,E)-N-(1-(3,4-diméthoxyphényl)éthyl)-3-(5-(pyrazolo[1,5-a]pyridin-3-yl)-1H-pyrrolo[2,3-b]pyridin-3-yl)acrylamide |
| **206** | (R,E)-N-(1-(3,4-diméthoxyphényl)éthyl)-3-(5-(2-(morpholinométhyl)phényl)-1H-pyrrolo[2,3-b]pyridin-3-yl)acrylamide |
| **207** | (R,E)-N-(1-(3,4-diméthoxyphényl)éthyl)-3-(5-(pyrimidin-5-yl)-1H-pyrrolo[2,3-b]pyridin-3-yl)acrylamide |
| **208** | (R,E)-N-(1-(3,4-diméthoxyphényl)éthyl)-3-(5-(3-sulfamoylphényl)-1H-pyrrolo[2,3-b]pyridin-3-yl)acrylamide |
| **209** | (R,E)-N-(1-(3,4-diméthoxyphényl)éthyl)-3-(5-(1,3,5-triméthyl-1H-pyrazol-4-yl)-1H-pyrrolo[2,3-b]pyridin-3-yl)acrylamide |
| **210** | (R,E)-N-(1-(3,4-diméthoxyphényl)éthyl)-3-(5-(1-(2-morpholinoéthyl)-1H-pyrazol-4-yl)-1H-pyrrolo[2,3-b]pyridin-3-yl)acrylamide |
| **211** | (R,E)-3-(5-(5-cyano-1-méthyl-1H-pyrazol-4-yl)-1H-pyrrolo[2,3-b]pyridin-3-yl)-N-(1-(3,4-diméthoxyphényl)éthyl)acrylamide |
| **212** | (R,E)-N-(1-(3,4-diméthoxyphényl)éthyl)-3-(5-(1,3-diméthyl-1H-pyrazol-4-yl)-1H-pyrrolo[2,3-b]pyridin-3-yl)acrylamide |
| **213** | (R,E)-N-(1-(3,4-diméthoxyphényl)éthyl)-3-(5-(1-(2-(diméthylamino)éthyl)-1H-pyrazol-4-yl)-1H-pyrrolo[2,3-b]pyridin-3-yl)acrylamide |
| **214** | (E)-N-((R)-1-(3,4-diméthoxyphényl)éthyl)-3-(5-(1-(2-hydroxypropyl)-1H-pyrazol-4-yl)-1H-pyrrolo[2,3-b]pyridin-3-yl)acrylamide |
| **215** | (R,E)-N-(1-(3,4-diméthoxyphényl)éthyl)-3-(5-(1-(oxétan-3-yl)-1H-pyrazol-4-yl)-1H-pyrrolo[2,3-b]pyridin-3-yl)acrylamide |
| **216** | (R,E)-3-(5-(3-(cyclopropylsulfonyl)phényl)-1H-pyrrolo[2,3-b]pyridin-3-yl)-N-(1-(3,4-diméthoxyphényl)éthyl)acrylamide |
| **217** | (R,E)-N-(1-(3,4-diméthoxyphényl)éthyl)-3-(5-(3-((méthylsulfonyl)méthyl)phényl)-1H-pyrrolo[2,3-b]pyridin-3-yl)acrylamide |
| **218** | (R,E)-N-(1-(3,4-diméthoxyphényl)éthyl)-3-(5-(5-(méthylsulfonyl)pyridin-3-yl)-1H-pyrrolo[2,3-b]pyridin-3-yl)acrylamide |
| **219** | (R,E)-N-(1-(3,4-diméthoxyphényl)éthyl)-3-(5-(4-(méthylsulfonyl)phényl)-1H-pyrrolo[2,3-b]pyridin-3-yl)acrylamide |
| **220** | (R,E)-N-(1-(3,4-diméthoxyphényl)éthyl)-3-(5-(4-fluoro-3-(méthylsulfonyl)phényl)-1H-pyrrolo[2,3-b]pyridin-3-yl)acrylamide |
| **221** | (R,E)-3-(5-(3-cyanophényl)-1H-pyrrolo[2,b]pyridin-3-yl)-N-(1-(3,4-diméthoxyphényl)éthyl)acrylamide |
| **222** | (R,E)-N-(1-(3,4-diméthoxyphényl)éthyl)-3-(5-(2-(méthylsulfonyl)phényl)-1H-pyrrolo[2,3-b]pyridin-3-yl)acrylamide |
| **223** | (R, E)-N-(1-(3,4-diméthoxyphényl)éthyl)-3-(4-(4-((diméthylamino)méthyl)phényl)-1H-pyrrolo[2,3-b]pyridin-3-yl)acrylamide |
| **224** | (R,E)-N-(1-(3,4-diméthoxyphényl)éthyl)-3-(4-(3-((diméthylamino)méthyl)phényl)-1H-pyrrolo[2,3-b]pyridin-3-yl)acrylamide |
| **225** | (R,E)-N-(1-(3,4-diméthoxyphényl)éthyl)-3-(4-phényl-1H-pyrrolo[2,3-b]pyridin-3-yl)acrylamide |
| **226** | (R,E)-N-(1-(3,4-diméthoxyphényl)éthyl)-3-(4-(4-(méthylsulfonamido)phényl)-1H-pyrrolo[2,3-b]pyridin-3-yl)acrylamide |
| **227** | (R,E)-N-(1-(3,4-diméthoxyphényl)éthyl)-3-(4-(3-(N,N-diméthylsulfamoyl)phényl)-1H-pyrrolo[2,3-b]pyridin-3-yl)acrylamide |
| **228** | (R,E)-N-(1-(3,4-diméthoxyphényl)éthyl)-3-(4-(3-(diméthylamino)phényl)-1H-pyrrolo[2,3-b]pyridin-3-yl)acrylamide |
| **229** | (R,E)-N-(1-(3,4-diméthoxyphényl)éthyl)-3-(4-(4-fluoro-2-méthoxyphényl)-1H-pyrrolo[2,3-b]pyridin-3-yl)acrylamide |
| **230** | (R, E)-N-(1-(3,4-diméthoxyphényl)éthyl)-3-(4-(4-(2-(diméthylamino)éthoxy)phényl)-1H-pyrrolo[2,3-b]pyridin-3-yl)acrylamide |
| **231** | (R,E)-N-(1-(3,4-diméthoxyphényl)éthyl)-3-(4-(3-(méthylsulfonamido)phényl)-1H-pyrrolo[2,3-b]pyridin-3-yl)acrylamide |
| **232** | (R, E)-N-(1-(3,4-diméthoxyphényl)éthyl)-3-(4-(4-(morpholinométhyl)phényl)-1H-pyrrolo[2,3-b]pyridin-3-yl)acrylamide |
| **233** | (R,E)-3-(3-(3-((1-(3,4-diméthoxyphényl)éthyl)amino)-3-oxoprop-1-èn-1-yl)-1H-pyrrolo[2,3-b]pyridin-4-yl)benzamide |
| **234** | (R, E)-N-(1-(3,4-diméthoxyphényl)éthyl)-3-(4-(4-((4-méthylpipérazin-1-yl)méthyl)phényl)-1H-pyrrolo[2,3-b]pyridin-3-yl)acrylamide |
| **235** | (R,E)-3-(4-(4-(4-acétylpipérazin-1-yl)phényl)-1H-pyrrolo[2,3-b]pyridin-3-yl)-N-(1-(3,4-diméthoxyphényl)éthyl)acrylamide |
| **236** | (R,E)-4-(3-(3-((1-(3,4-diméthoxyphényl)éthyl)amino)-3-oxoprop-1-èn-1-yl)-1H-pyrrolo[2,3-b]pyridin-4-yl)benzamide |
| **237** | (R,E)-N-(1-(3,4-diméthoxyphényl)éthyl)-3-(4-(1,5-diméthyl-1H-pyrazol-4-yl)-1H-pyrrolo[2,3-b]pyridin-3-yl)acrylamide |
| **238** | (R,E)-N-(1-(3,4-diméthoxyphényl)éthyl)-3-(4-(1-méthyl-1H-pyrazol-4-yl)-1H-pyrrolo[2,3-b]pyridin-3-yl)acrylamide |
| **239** | (E)-N-((R)-1-(3,4-diméthoxyphényl)éthyl)-3-(4-(3-(éthylsulfinyl)phényl)-1H-pyrrolo[2,3-b]pyridin-3-yl)acrylamide |
| **240** | (R,E)-N-(1-(3,4-diméthoxyphényl)éthyl)-3-(4-(4-(1-méthylpipéridin-4-yl)phényl)-1H-pyrrolo[2,3-b]pyridin-3-yl)acrylamide |
| **241** | (R,E)-N-(1-(3,4-diméthoxyphényl)éthyl)-3-(4-(4-(3-(diméthylamino)propoxy)phényl)-1H-pyrrolo[2,3-b]pyridin-3-yl)acrylamide |
| **242** | (R, E)-N-(1-(3,4-diméthoxyphényl)éthyl)-3-(4-(pyrazolo[1,5-a]pyridin-3-yl)-1H-pyrrolo[2,3-b]pyridin-3-yl)acrylamide |
| **243** | (R,E)-3-(4-(3-(acétamidométhyl)phényl)-1H-pyrrolo[2,3-b]pyridin-3-yl)-N-(1-(3,4-diméthoxyphényl)éthyl)acrylamide |
| **244** | (R,E)-2-cyano-N-(1-(3,4-diméthoxyphényl)éthyl)-3-(4-méthyl-5-(4-(4-méthylpipérazin-1-yl)phényl)-1H-pyrrolo[2,3-b]pyridin-3-yl)acrylamide |
| **245** | (R,E)-2-cyano-N-(1-(3,4-diméthoxyphényl)éthyl)-3-(5-(4-fluoro-3-(méthylsulfonyl)phényl)-4-méthyl-1H-pyrrolo[2,3-b]pyridin-3-yl)acrylamide |
| **246** | (R,E)-N-(1-(benzo[d][1,3]dioxol-5-yl)éthyl)-3-(5-(1-méthyl-1H-pyrazol-4-yl)-1H-pyrrolo[2,3-b]pyridin-3-yl)acrylamide |
| **247** | (R,E)-3-(5-(3-chloro-5-(méthylsulfonyl)phényl)-1H-pyrrolo[2,3-b]pyridin-3-yl)-N-(1-(3,4-diméthoxyphényl)éthyl)acrylamide |
| **248** | (R,E)-N-(1-(3,4-diméthoxyphényl)éthyl)-3-(5-(2-méthyl-5-(méthylsulfonyl)phényl)-1H-pyrrolo[2,3-b]pyridin-3-yl)acrylamide |
| **249** | (R,E)-N-(1-(3,4-diméthoxyphényl)éthyl)-3-(5-(3-fluoro-5-(méthylsulfonyl)phényl)-1H-pyrrolo[2,3-b]pyridin-3-yl)acrylamide |
| **250** | (R,E)-N-(1-(3,4-diméthoxyphényl)éthyl)-3-(5-(2-(méthylsulfonyl)pyridin-4-yl)-1H-pyrrolo[2,3-b]pyridin-3-yl)acrylamide |
| **251** | (E)-N-((R)-1-(3,4-diméthoxyphényl)éthyl)-3-(5-(3-(S-méthylsulfonimidoyl)phényl)-1H-pyrrolo[2,3-b]pyridin-3-yl)acrylamide |
| **252** | (R,E)-N-(1-(benzo[d][1,3]dioxol-5-yl)éthyl)-2-cyano-3-(5-(1-méthyl-1H-pyrazol-4-yl)-1H-pyrrolo[2,3-b]pyridin-3-yl)acrylamide |
| **253** | (E)-N-(1-(3-cyclopropyl-4-méthoxyphényl)éthyl)-3-(5-(1-méthyl-1H-pyrazol-4-yl)-1H-pyrrolo[2,3-b]pyridin-3-yl)acrylamide |
| **254** | (R,E)-N-(1-(3,4-diméthoxyphényl)éthyl)-3-(5-(3-(méthylthio)phényl)-1H-pyrrolo[2,3-b]pyridin-3-yl)acrylamide |
| **255** | (E)-N-(1-(3-méthoxy-4-(méthylsulfinyl)phényl)éthyl)-3-(5-(1-méthyl-1H-pyrazol-4-yl)-1H-pyrrolo[2,3-b]pyridin-3-yl)acrylamide |
| **256** | (E)-2-cyano-N-(1-(3-méthoxy-4-(méthylsulfinyl)phényl)éthyl)-3-(5-(1-méthyl-1H-pyrazol-4-yl)-1H-pyrrolo[2,3-b]pyridin-3-yl)acrylamide |
| **257** | (R,E)-N-(1-(3,4-difluorophényl)éthyl)-3-(5-(1-méthyl-1H-pyrazol-4-yl)-1H-pyrrolo[2,3-b]pyridin-3-yl)acrylamide |
| **258** | (R,E)-2-cyano-N-(1-(3,4-difluorophényl)éthyl)-3-(5-(1-méthyl-1H-pyrazol-4-yl)-1H-pyrrolo[2,3-b]pyridin-3-yl)acrylamide |
| **259** | (R,E)-N-(1-(3,4-diméthoxyphényl)éthyl)-3-(5-(4-(méthylsulfonyl)pyridin-2-yl)-1H-pyrrolo[2,3-b]pyridin-3-yl)acrylamide |
| **260** | (R,E)-3-(5-(1-méthyl-1 H-pyrazol-4-yl)-1 H-pyrrolo[2,3-b]pyridin-3-yl)-N-(1-(3-méthylbenzo[d]isoxazol-5-yl)éthyl)acrylamide |
| **261** | (R,E)-2-cyano-N-(1-(1,3-diméthyl-1H-indazol-6-yl)éthyl)-3-(5-(1-méthyl-1H-pyrazol-4-yl)-1H-pyrrolo[2,3-b]pyridin-3-yl)acrylamide |
| **262** | (R,E)-2-cyano-N-(1-(1,3-diméthyl-1H-indazol-5-yl)éthyl)-3-(5-(1-méthyl-1H-pyrazol-4-yl)-1H-pyrrolo[2,3-b]pyridin-3-yl)acrylamide |
| **263** | (E)-N-((R)-1-(3,4-diméthoxyphényl)éthyl)-3-(5-(3-((trifluorométhyl)sulfinyl)phényl)-1H-pyrrolo[2,3-b]pyridin-3-yl)acrylamide |
| **264** | (R,E)-N-(1-(3,4-diméthoxyphényl)éthyl)-3-(5-(3-((trifluorométhyl)sulfonyl)phényl)-1H-pyrrolo[2,3-b]pyridin-3-yl)acrylamide |
| **265** | (E)-N-(1-(5,6-diméthoxypyridin-3-yl)éthyl)-3-(5-(1-méthyl-1H-pyrazol-4-yl)-1H-pyrrolo[2,3-b]pyridin-3-yl)acrylamide |
ou un sel de formule acceptable dans le domaine pharmaceutique.

8. Une composition pharmaceutique comprenant au moins un composé conforme aux revendications 1 à 7 en tant que principe actif, associé à au moins un vecteur, un excipient et/ou un diluant acceptable dans le domaine pharmaceutique.

9. Un composé conforme aux revendications 1 à 7, destiné à un usage en tant que médicament.

10. Un composé conforme aux revendications 1 à 7, ou une composition pharmaceutique conforme à la revendication 8, pour une utilisation dans la prophylaxie et/ou le traitement d'une maladie causée par ou associée à la kinase 5 des récepteurs couplés aux protéines G (GRK5), choisie parmi les maladies cardiaques, les maladies inflammatoires et immunologiques, les maladies métaboliques et le cancer.

11. Le composé ou la composition pharmaceutique à utiliser conformément à la revendication 10, où la maladie cardiaque est choisie parmi les suivantes : hypertrophie cardiaque, cardiopathie congénitale de l'adulte, anévrisme, angine stable, angine instable, angine de poitrine, œdème angioneurotique, sténose de la valve aortique, anévrisme aortique, arythmie, dysplasie ventriculaire droite arythmogène, artériosclérose, malformations artérioveineuses, fibrillation auriculaire, maladie de Behçet, bradycardie, tamponnade cardiaque, cardiomégalie, cardiomyopathie congestive, cardiomyopathie hypertrophique, cardiomyopathie restrictive, prévention des maladies cardiovasculaires, sténose carotidienne, hémorragie cérébrale, syndrome de Churg et Strauss, diabète, anomalie d'Ebstein, complexe d'Eisenmenger, embolie de cholestérol, endocardite bactérienne, dysplasie fibromusculaire, malformations cardiaques congénitales, maladies cardiaques, insuffisance cardiaque congestive, cardiopathies valvulaires, crise cardiaque, hématome épidural, hématome sous-dural, maladie de von Hippel-Lindau, hyperémie, hypertension, hypertension pulmonaire, croissance hypertrophique, hypertrophie ventriculaire gauche, hypertrophie ventriculaire droite, syndrome d'hypoplasie du cœur gauche, hypotension, claudication intermittente, cardiopathie ischémique, syndrome de Klippel-Trenaunay, syndrome médullaire latéral, syndrome du QT long, prolapsus de la valve mitrale, maladie de Moya Moya, syndrome lympho-cutanéo-muqueux, infarctus du myocarde, ischémie myocardique, myocardite, péricardite, maladies vasculaires périphériques, phlébite, polyartérite noueuse, atrésie pulmonaire, maladie de Raynaud, resténose, syndrome de Sneddon, sténose, syndrome de compression de la veine cave supérieure, syndrome X, tachycardie, artérite de Takayasu, télangiectasie hémorragique héréditaire, télangiectasie, artérite temporale, tétralogie de Fallot, thromboangéite oblitérante, thrombose, thromboembolie, atrésie tricuspide, varices, maladies vasculaires, vascularite, angiospasme, fibrillation ventriculaire, syndrome de Williams, maladie vasculaire périphérique, varices et ulcères de jambe, thrombose veineuse profonde, syndrome de Wolff-Parkinson et White.

12. Le composé ou la composition pharmaceutique à utiliser conformément à la revendication 10, où la maladie inflammatoire et immunologique est choisie parmi une maladie inflammatoire associée à une maladie auto-immune, une maladie inflammatoire du système nerveux central (SNC), une maladie inflammatoire des articulations, une maladie digestive inflammatoire du tube digestif et une maladie inflammatoire de la peau.

13. Le composé ou la composition pharmaceutique à utiliser conformément à la revendication 10, où la maladie métabolique est choisie parmi le diabète, l'obésité et un trouble de l'adipogénèse.

14. Le composé ou la composition pharmaceutique à utiliser conformément à la revendication 10, où le cancer est choisi parmi les suivants : adénocarcinome, mélanome choroïdien, leucémie aiguë, neurinome de l'acoustique, carcinome ampullaire, carcinome épidermoïde du canal anal, astrocytome, carcinome basocellulaire, cancer du pancréas, tumeur desmoïde, cancer de la vessie, carcinome bronchique, cancer du poumon non à petites cellules (CPNPC), cancer du sein, lymphome de Burkitt, cancer du corps de l'utérus, syndrome du carcinome de primitif inconnu (CPI), cancer du côlon-rectum, cancer de l'intestin grêle, tumeurs de l'intestin grêle, cancer de l'ovaire, carcinome endométrioïde, épendymome, cancers de type épithélial, sarcome d'Ewing, tumeurs gastro-intestinales, cancer gastrique, cancer de la vésicule biliaire, carcinomes de la vésicule biliaire, cancer de l'utérus, cancer du col de l'utérus, glioblastomes, tumeurs gynécologiques, tumeurs de la sphère ORL, néoplasies myéloprolifératives, leucémie à tricholeucocytes, cancer de l'urètre, cancer de la peau, cancer du scrotum, tumeurs cérébrales (gliomes), métastases cérébrales, cancer du testicule, tumeur de l'hypophyse, carcinoïdes, sarcome de Kaposi, cancer du larynx, tumeur germinale, cancer des os, carcinome colorectal, tumeurs de la tête et du cou (tumeurs de la sphère ORL), carcinome colorectal, craniopharyngiomes, cancer de la cavité buccale (cancer touchant la bouche et les lèvres), cancer du système nerveux central, cancer du foie, métastases du foie, leucémie, tumeur de la paupière, cancer du poumon, cancer des ganglions lymphatiques (hodgkinien/non hodgkinien), lymphomes, cancer de l'estomac, mélanome malin, néoplasie maligne, tumeurs stromales gastro-intestinales malignes, carcinome mammaire, cancer du rectum, médulloblastomes, mélanome, méningiomes, lymphome de Hodgkin, mycosis fongoïde, cancer des fosses nasales, neurinome, neuroblastome, cancer du rein, carcinomes à cellules rénales, lymphomes non hodgkiniens, oligodendrogliome, carcinome oesophagien, carcinomes ostéolytiques et carcinomes ostéoplastiques, ostéosarcomes, carcinome ovarien, carcinome pancréatique, cancer du pénis, plasmocytome, carcinome épidermoïde de la tête et du cou, cancer de la prostate, cancer du pharynx, carcinome rectal, rétinoblastome, cancer du vagin, carcinome thyroïdien, cancer bronchopulmonaire d'origine professionnelle, cancer de l'œsophage, spinaliome, lymphome T (mycosis fongoïde), thymome, carcinome tubaire, tumeurs de l'oeil, cancer de l'urètre, tumeurs urologiques, carcinome urothélial, cancer de la vulve, lésions verruqueuses, tumeurs des tissus mous, sarcome des tissus mous, tumeur de Wilms, carcinome cervical, cancer de la langue, astrocytomes, cancer bronchique, cancer du larynx, mélanome malin, cancer de l'œsophage et cancer à cellules rénales.

15. ne méthode pour produire le composé de la formule (la) conformément à la revendication 1, comprenant les étapes suivantes :
**Étape** 1 : préparation du composé (2*), 1H-pyrrolo[2,3-b]pyridine-3-carbaldéhyde 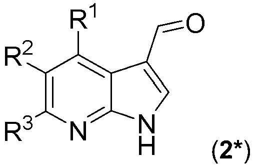
**Étape** 2 : réalisation d'une réaction de condensation du composé (2*) avec un composé (3*) 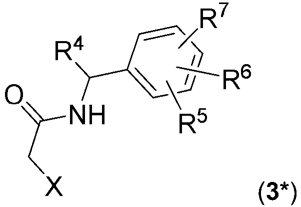
pour obtenir le composé de la formule (la) 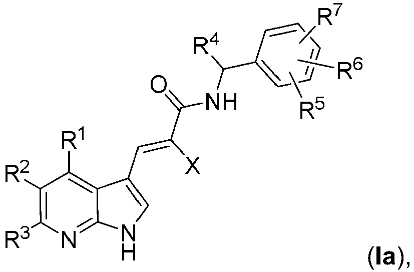
où **R¹, R², R³, R⁴, R⁵, R⁶, R⁷** et X ont les significations définies dans la revendication 1 ;
ou
**Étape** 1 : préparation du composé (2*), 1H-pyrrolo[2,3-b]pyridine-3-carbaldehyde 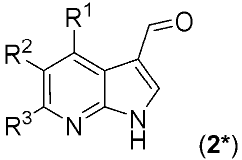
**Étape** 2 : réalisation d'une réaction de condensation du composé (2*) avec de l'acide maléique ou de l'acide cyanoacétique pour obtenir le composé d'acide acrylique (4*) 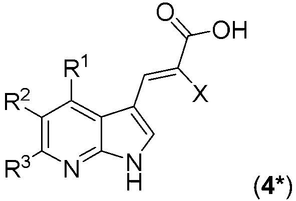
**Étape** 3: réalisation d'une réaction de couplage du composé (4*) avec un composé aminé (5*) 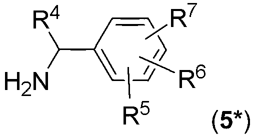
pour obtenir le composé de la formule (la) 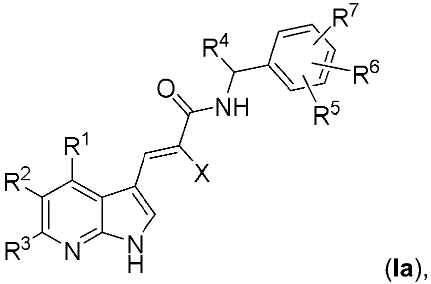
où **R¹, R², R³, R⁴, R⁵, R⁶,** R⁷ et X ont les significations définies dans la revendication 1.
